# EUROPEAN PATENT SPECIFICATION

(11) **EP 2 900 656 B1**
(45) Date of publication and mention of the grant of the patent: **17.05.2017**
(21) Application number: 13842940.2
(22) Date of filing: 29.09.2013
(51) Int. Cl.: C07D 401/14, C07D 403/14, C07D 405/14, C07D 409/14, C07D 413/14, C07D 417/14, C07D 471/08, C07D 491/113, A61K 31/4178, A61K 31/4184, A61K 31/5377, A61K 31/438, A61K 31/4439, A61K 31/4545, A61K 31/427, A61K 31/423, A61K 45/06, A61P 31/14, A61P 1/16

(54) **SPIRO RING COMPOUNDS AS HEPATITIS C VIRUS (HCV) INHIBITORS**
SPIRORINGVERBINDUNGEN ALS HEPATITIS-C-VIRUS (HCV)-INHIBITOREN
COMPOSÉS À CYCLE SPIRO EN TANT QU'INHIBITEURS DU VIRUS DE L'HÉPATITE C (VHC)

(30) Priority: 28.09.2012 CN 201210366536; 04.04.2013 CN 201310117051
(43) Date of publication of application: 05.08.2015
(73) Proprietor: Sunshine Lake Pharma Co., Ltd., Dongguan, Guangdong 523000 (CN)
(72) Inventor: ZHANG, Yingjun, Dongguan Guangdong 523871 (CN); ZHANG, Jiancun, Dongguan Guangdong 523871 (CN); XIE, Hongming, Dongguan Guangdong 523871 (CN); REN, Qingyun, Dongguan Guangdong 523871 (CN); WU, Xiwei, Dongguan Guangdong 523871 (CN); LUO, Huichao, Dongguan Guangdong 523871 (CN); FU, Changping, Dongguan Guangdong 523871 (CN); HU, Bailin, Dongguan Guangdong 523871 (CN); LI, Shifeng, Dongguan Guangdong 523871 (CN); TANG, Changhua, Dongguan Guangdong 523871 (CN); LEI, Yong, Dongguan Guangdong 523871 (CN); YU, Quanxing, Dongguan Guangdong 523871 (CN); FANG, Qinghong, Dongguan Guangdong 523871 (CN); WANG, Chenglin, Dongguan Guangdong 523871 (CN)
(74) Representative: Becker Kurig Straus
(86) International application number: PCT/CN2013/001183
(87) International publication number: WO 2014/048072

(56) References cited:
- WO-A1-2004/002977
- WO-A1-2009/047264
- WO-A1-2012/021591
- WO-A1-2013/007106
- WO-A1-2014/019344

## Description

### CROSS-REFERENCE TO RELATED APPLICATION

This application claims priority to and the benefits of Chinese Patent Application Serial Nos. 201210366536.6, filed with the State Intellectual Property Office of P. R. China on September 28, 2012; and 201310117051.8, filed with the State Intellectual Property Office of P. R. China on April 4,2013.

### FIELD OF THE INVENTION

The present disclosure relates to a field of medicine, and more particularly to compounds for treating Hepatitis C virus (HCV) infection, compositions comprising such compounds, use of the compounds and the compositions thereof, and methods thereof.

### BACKGROUND OF THE INVENTION

HCV is a major human pathogen, infecting an estimated 170 million persons worldwide - roughly five times the number infected by human immunodeficiency virus type I. A substantial fraction of these HCV infected individuals develop serious progressive liver disease, including cirrhosis and hepatocellular carcinoma. Chronic HCV infection is thus a major worldwide cause of liver-related premature mortality.

Presently, the most effective HCV therapy employs a combination of alpha-interferon and ribavirin, leading to sustained efficacy in 40% of patients. Recent clinical results demonstrate that pegylated alpha-interferon is superior to unmodified alpha-interferon as monotherapy. However, even with experimental therapeutic regimens involving combinations of pegylated alpha-interferon and ribavirin, a substantial fraction of patients do not have a sustained reduction in viral load. The treatment has side effects in many patients, so they do not durably respond to treatment. Thus, new and effective methods of treating HCV infection are urgently needed.

HCV is a positive-stranded RNA virus. Based on a comparison of the deduced amino acid sequence and the extensive similarity in the 5'untranslated region, HCV has been classified as a separate genus in the Flaviviridae family. All members of the Flaviviridae family have enveloped virions that contain a positive stranded RNA genome encoding all known virus-specific proteins via translation of a single, uninterrupted, open reading frame (ORF).

Considerable heterogeneity is found within nucleotide and encoded amino acid sequence throughout the HCV genome. At least seven major genotypes have been characterized, and more than 50 subtypes have been described. In HCV infected cells, viral RNA is translated into a polyprotein that is cleaved into ten individual proteins. At the amino terminus are structural proteins, follows El and E2. Additionally, there are six non-structural proteins, NS2, NS3, NS4A, NS4B, NS5A and NS5B, which play a function role in the HCV lifecycle (see, for example, Lindenbach et al., Nature, 2005, 436, 933-938.).

The major genotypes of HCV differ in their distribution worldwide, and the clinical significance of the genetic heterogeneity of HCV remains elusive despite numerous studies of the possible effect of genotypes on pathogenesis and therapy.

The single strand HCV RNA genome is approximately 9500 nucleotides in length and has a single open reading frame (ORF) encoding a single large polyprotein of about 3000 amino acids. In infected cells, this polyprotein is cleaved at multiple sites by cellular and viral proteases to produce the structural and non-structural (NS) proteins. In the case of HCV, the generation of mature non-structural proteins (NS2, NS3, NS4A, NS4B, NS5A and NS5B) is effected by two viral proteases. The first one is believed to be a metalloprotease and cleaves at the NS2-NS3 junction; the second one is a serine protease within the N-terminal region of NS3 (also referred herein as NS3 protease) and mediates all the subsequent cleavages downstream of NS3, both in cis, at the NS3-NS4A cleavage site, and in trans, for the remaining NS4A-NS4B, NS4B-NS5A, NS5A-NS5B sites. The NS4A protein appears to serve multiple functions, acting as a cofactor for the NS3 protease and possibly assisting in the membrane localization of NS3 and other viral replicase components. The complex formation of the NS3 protein with NS4A seems necessary to the processing events, enhancing the proteolytic efficiency at all of the sites. The NS3 protein also exhibits nucleoside triphosphatase and RNA helicase activities. NS5B (also referred to herein as HCV polymerase) is a RNA-dependent RNA polymerase that is involved in the replication of HCV.
Compounds useful for treating HCV-infected patients are desired which selectively inhibit HCV viral replication. In particular, compounds which are effective to inhibit the function of the NS5A protein are desired. The HCV NS5A protein is described, for example, in Tan et al., Virology. 2001, 284, 1-12; and in Park et al., J. Biol. Chem., 2003, 278, 30711-30718. The patent applications WO 2013007106, WO 2014019344 and WO 2009047264 provided a series of compounds, which have an inhibitory activity against HCV NS5A. The patent application WO 2004002977 provided a series of sprio compounds, which have an inhibitory activity against HIV.

### SUMMARY OF THE INVENTION

Provided herein are novel spiro ring compounds and methods of their use to treat HCV infection. Specifically, it has been found that the spiro ring compounds disclosed herein, and compositions thereof, are effective as inhibitors of HCV infection, especially the non-structural 5A (NS5A) protein of HCV.

In one aspect, provided herein are compounds having formula (I) as shown below: 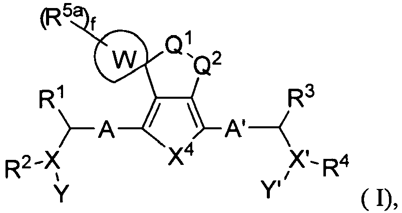 or a stereoisomer, a geometric isomer, a tautomer, an N-oxide, a hydrate, a solvate, a pharmaceutically acceptable salt thereof, wherein:
each of A and A' is independently a bond, alkylene, alkenylene, cycloalkylene, heterocyclylalkylene, -(CR⁸R^{8a})ₙ-O-(CR⁸R^{8a})ₚ-, -(CR⁸R^{8a})ₙ-N(R⁵)-(CR⁸R^{8a})ₚ-, -(CR⁸R^{8a})ₙ-S(=O)ᵣ-N(R⁵)-(CR⁸R^{8a})ₚ-, -(CR⁸R^{8a})ₙ-C(=O)-N(R⁵)-(CR⁸R^{8a})ₚ-, -(CR⁸R^{8a})ₙ-N(R⁵)-C(=O)-N(R⁵)-(CR⁸R^{8a})ₚ-, -(CR⁸R^{8a})ₙ-C(=O)-O-(CR⁸R^{8a})ₚ-, -(CR⁸R^{8a})ₙ-N(R⁵)-S(=O)ᵣ-N(R⁵)-(CR⁸R^{8a})ₚ- or -(CR⁸R^{8a})ₙ-N(R⁵)-C(=O)-O-(CR⁸R^{8a})ₚ-; or each of A and A' is independently 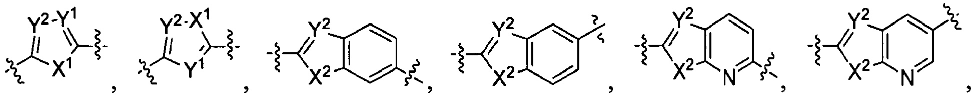 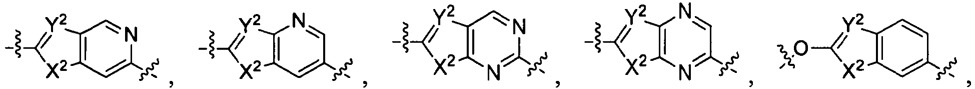 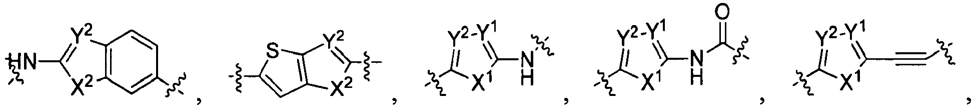 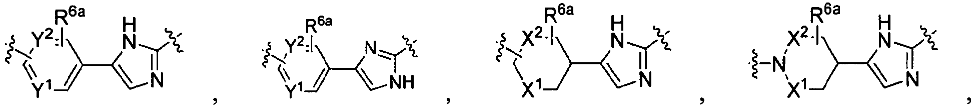 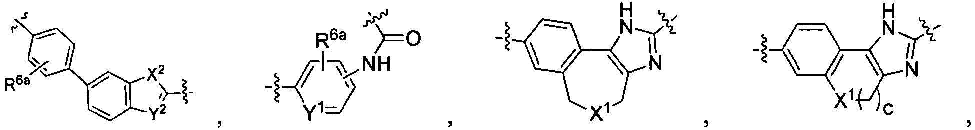 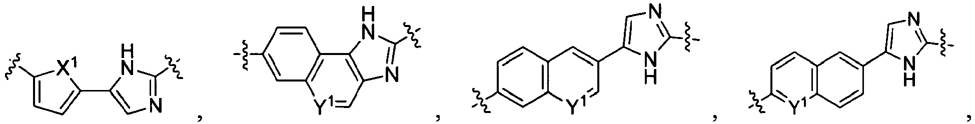 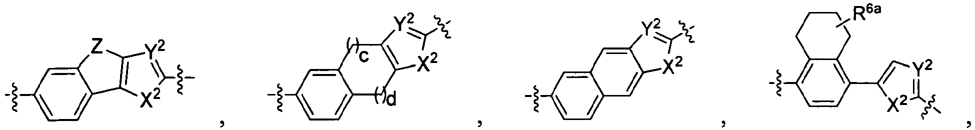 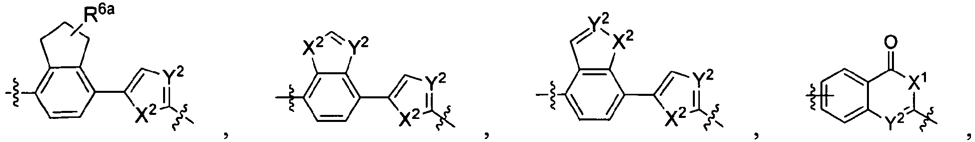 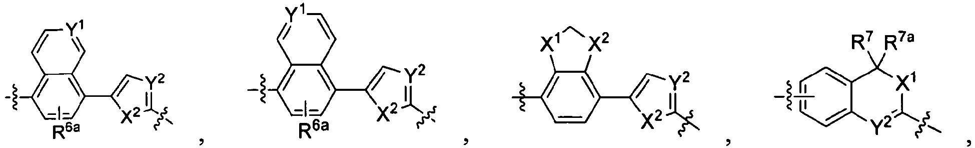 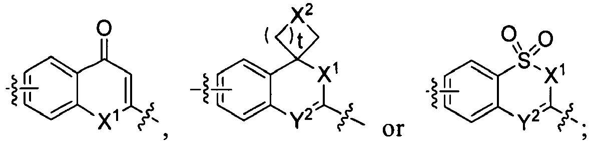
wherein each X¹ is independently O, S, NR⁶ or CR⁷R^{7a};
each X² is independently NR⁶, O or S;
X⁴ is (CR⁷R^{7a})ₙ, -y¹=y²-, O, S or NR⁶;
W is carbocyclyl or heterocyclyl;
each Y¹ and Y² is independently N or CR⁷;
each Z is independently -(CH₂)ₐ-, -CH=CH-, -N=CH-, -(CH₂)ₐ-N(R⁵)-(CH₂)_{b}- or -(CH₂)ₐ-O-(CH₂)_{b}-, wherein each a and b is independently 0, 1, 2 or 3;
each c is independently 1 or 2;
each d is independently 1 or 2;
each n is independently 0, 1, 2 or 3;
each p is independently 0, 1, 2 or 3;
each r is independently 0, 1 or 2;
f is 0, 1, 2, 3 or 4;
each of Q¹ and Q² is independently NR⁶, O, S, C(=O) or (CR⁷R^{7a})ₑ;
e is 0, 1, 2, 3 or 4;
each of X and X' is independently N or CR⁷;
each of Y and Y' is independently H, deuterium, alkyl, heteroalkyl, cycloalkyl, heterocyclyl, aryl, heteroaryl, aralkyl, a group derived from α-amino acid or an optically isomer thereof; or each of Y and Y' is independently -[U-(CR⁹R^{9a})ₜ-N(R¹⁰)-(CR⁹R^{9a})ₜ]ₖ-U-(CR⁹R^{9a})ₜ-N(R¹¹)-(CR⁹R^{9a})ₜ-R¹², -U-(CR⁹R^{9a})ₜ-R¹² or -[U-(CR⁹R^{9a})ₜ-N(R¹⁰)-(CR⁹R^{9a})ₜ]ₖ-U-(CR⁹R^{9a})ₜ-O-(CR⁹R^{9a})ₜ-R¹²;
each U is independently -C(=O)-, -C(=S)-, -S(=O)- or -S(=O)₂-;
each t is independently 0, 1, 2, 3 or 4;
each k is independently 0, 1 or 2;
R¹ and R², together with X-CH they are attached to, optionally form a 3-8 membered heterocycle or carbocycle, C₅₋₂ fused bicycle, C₅₋₁₂ fused heterobicycle, C₅₋₁₂ spiro bicycle or C₅₋₁₂ spiro heterobicycle; R³ and R⁴, together with X'-CH they are attached to, optionally form a 3-8 membered heterocycle or carbocycle, C₅₋₁₂ fused bicycle, C₅₋₁₂ fused heterobicycle, C₅₋₁₂ spiro bicycle or C₅₋₁₂ spiro heterobicycle;
each R⁵ is independently H, deuterium, hydroxy, alkyl, heteroalkyl, cycloalkyl, heterocyclyl, aryl, heteroaryl, aralkyl, alkoxy, alkyl-OC(=O)-, alkyl-C(=O)-, carbamoyl, alkyl-OS(=O)ᵣ-, alkyl-S(=O)ᵣO-, alkyl-S(=O)ᵣ- or aminosulfonyl;
each R^{5a} is independently H, deuterium, oxo (=O), hydroxy, amino, F, Cl, Br, I, cyano, R^{13a}R¹³N-, -C(=O)NR¹³R^{13a}, -OC(=O)NR¹³R^{13a}, -OC(=O)OR¹³, -N(R¹³)C(=O)NR¹³R^{13a}, -N(R¹³)C(=O)OR¹³, -N(R¹³)C(=O)-R¹³, R¹³R^{13a}N-S(=O)₂-, R¹³S(=O)₂-, R¹³S(=O)₂N(R^{13a})-, R^{13a}R¹³N-alkyl, R¹³S(=O)-alkyl, R¹³R^{13a}N-C(=O)-alkyl, R^{13a}R¹³N-alkoxy, R¹³S(=O)-alkoxy, R¹³R^{13a}N-C(=O)-alkoxy, aryl, heteroaryl, alkoxy, alkylamino, alkyl, haloalkyl, alkenyl, alkynyl, heterocyclyl, cycloalkyl, mercapto, nitro, aralkyl, arylamino, heteroarylamino, arylalkylamino, heteroarylalkylamino, heteroaryloxy, heteroarylalkyl, arylalkoxy, heteroarylalkoxy, heterocyclyloxy, heterocyclylalkoxy, heterocyclylamino, heterocyclylalkylamino or aryloxy;
each R⁶ is independently H, deuterium, R¹³R^{13a}NC(=O)-, R¹³OC(=O)-, R¹³C(=O)-, R¹³R^{13a}NS(=O)-, R¹³OS(=O)-, R¹³S(=O)-, R¹³R^{13a}S(=O)₂-, R¹³OS(=O)₂-, R¹³S(=O)₂-, aliphatic, haloaliphatic, hydroxyaliphatic, aminoaliphatic, alkoxyaliphatic, alkylaminoaliphatic, alkylthioaliphatic, arylaliphatic, heteroarylaliphatic, heterocyclylaliphatic, cycloalkylaliphatic, aryloxyaliphatic, heterocyclyloxyaliphatic, cycloalkyloxyaliphatic, arylaminoaliphatic, heterocyclylaminoaliphatic, cycloalkylaminoaliphatic, aryl, heteroaryl, heterocyclyl or carbocyclyl;
each R^{6a} is independently H, deuterium, hydroxy, amino, F, Cl, Br, I, cyano, oxo (=O), R^{13a}R¹³N-, -C(=O)NR¹³R^{13a}, -OC(=O)NR¹³R^{13a}, -OC(=O)OR¹³, -N(R¹³)C(=O)NR¹³R^{13a}, -N(R¹³)C(=O)OR^{13a}, -N(R¹³)C(-O)-R^{13a}, R¹³R^{13a}N-S(=O)₂-, R¹³S(=O)₂-, R¹³S(-O)₂N(R^{13a})-, R^{13a}R¹³N-alkyl, R¹³S(=O)-alkyl, R¹³R^{13a}N-C(=O)-alkyl, R^{13a}R¹³N-alkoxy, R¹³S(=O)-alkoxy, R¹³R^{13a}N-C(=O)-alkoxy, aryl, heteroaryl, alkoxy, alkylamino, alkyl, haloalkyl, alkenyl, alkynyl, heterocyclyl, cycloalkyl, mercapto, nitro, aralkyl, arylamino, heteroarylamino, arylalkylamino, heteroarylalkylamino, heteroaryloxy, heteroarylalkyl, arylalkoxy, heteroarylalkoxy, heterocyclyloxy, heterocyclylalkoxy, heterocyclylamino, heterocyclylalkylamino or aryloxy;
each R⁷ and R^{7a} is independently H, deuterium, F, Cl, Br, I, aliphatic, heteroalkyl, haloaliphatic, hydroxyaliphatic, aminoaliphatic, alkoxyaliphatic, alkylaminoaliphatic, alkylthioaliphatic, arylaliphatic, heteroarylaliphatic, heterocyclylaliphatic, cycloalkylaliphatic, aryloxyaliphatic, heterocyclyloxyaliphatic, cycloalkyloxyaliphatic, arylaminoaliphatic, heterocyclylaminoaliphatic, cycloalkylaminoaliphatic, aryl, heteroaryl, heterocyclyl or carbocyclyl;
each R⁸ and R^{8a} is independently H, deuterium, hydroxy, cyano, nitro, F, Cl, Br, I, alkyl, heteroalkyl, cycloalkyl, heterocyclyl, aryl, heteroaryl, aralkyl, alkoxy, alkyl-OC(=O)-, alkyl-C(=O)-, carbamoyl, alkyl-OS(=O)ᵣ-, alkyl-S(=O)ᵣO-, alkyl-S(=O)ᵣ- or aminosulfonyl;
each R⁹, R^{9a}, R¹⁰ and R¹¹ is independently H, deuterium, alkyl, heteroalkyl, cycloalkyl, heterocyclyl, aryl, heteroaryl, aralkyl, haloalkyl, hydroxyalkyl, heteroarylalkyl, heterocyclylalkyl or cycloalkylalkyl;
each R¹² is independently R^{13a}R¹³N-, -C(=O)R¹³, -C(=S)R¹³, -C(=O)OR^{13,} -C(=O)NR¹³R^{13a}, -OC(=O)NR¹³R^{13a}, -OC(=O)OR¹³, -N(R¹³)C(=O)NR¹³R^{13a}, -N(R¹³)C(=O)OR^{13a}, -N(R¹³)C(=O)-R^{13a}, R¹³R^{13a}N-S(=O)₂-, R¹³S(=O)₂-, R¹³S(=O)₂N(R^{13a})-, R¹³OS(=O)₂-, alkyl, heteroalkyl, cycloalkyl, heterocyclyl, aryl, heteroaryl or aralkyl;
or R¹¹ and R¹² are optionally joined to form a 4-7 membered ring; and
each R¹³ and R^{13a} is independently H, deuterium, alkyl, heteroalkyl, cycloalkyl, heterocyclyl, aryl, heteroaryl or aralkyl; with the proviso that where R¹³ and R^{13a} are bonded to the same nitrogen atom, R¹³ and R^{13a}, together with the nitrogen atom they are attached to, optionally form a substituted or unsubstituted 3-8 membered ring or a substituted or unsubstituted spiro or fused bicyclic ring;
wherein each of alkylene, alkenylene, cycloalkylene, heterocyclylalkylene, -(CR⁸R^{8a})ₙ-O-(CR⁸R^{8a})ₚ, -(CR⁸R^{8a})ₙ-N(R⁵)-(CR⁸R^{8a})ₚ-, -(CR⁸R^{8a})ₙ-S(=O)ᵣ-N(R⁵)-(CR⁸R^{8a})ₚ-, -(CR⁸R^{8a})ₙ-C(=O)-N(R⁵)-(CR⁸R^{8a})ₚ-, -(CR⁸R^{8a})ₙ-N(R⁵)-C(=O)-N(R⁵)-(CR⁸R^{8a})ₚ-, -(CR⁸R^{8a})ₙ-C(=O)-O-(CR⁸R^{8a})ₚ-, -(CR⁸R^{8a})ₙ-N(R⁵)-S(=O)ᵣ-N(R⁵)-(CR⁸R^{8a})ₚ-, -(CR⁸R^{8a})ₙ-N(R⁵)-C(=O)-O-(CR⁸R^{8a})ₚ-, -[U-(CR⁹R^{9a})ₜ-N(R¹⁰)-(CR⁹R^{9a})ₜ]ₖ-U-(CR⁹R^{9a})ₜ-N(R¹¹)-(CR⁹R^{9a})ₜ-R¹², -U-(CR⁹R^{9a})ₜ-R¹², -[U-(CR⁹R^{9a})ₜ-N(R¹⁰)-(CR⁹R^{9a})ₜ]ₖ-U-(CR⁹R^{9a})ₜ-O-(CR⁹R^{9a})ₜ-R¹², NR⁶, CR⁷R^{7a}, CR⁷, -(CH₂)ₐ-, -CH=CH-, -N=CH-, -(CH₂)ₐ-N(R⁵)-(CH₂)_{b}-, -(CH₂)ₐ-O-(CH₂)_{b}-, R^{13a}R¹³N-, -C(=O)R¹³, -C(=S)R¹³, -C(=O)OR¹³, -C(=O)NR¹³R^{13a}, -OC(=O)NR¹³R^{13a}, -OC(=O)OR¹³, -N(R¹³)C(=O)NR¹³R^{13a}, -N(R¹³)C(=O)OR^{13a}, -N(R¹³)C(=O)-R^{13a}, R¹³R^{13a}N-S(=O)₂-, R¹³S(=O)₂-, R¹³S(=O)₂N(R^{13a})-, R¹³OS(=O)₂-, alkyl-OC(=O)-, alkyl-C(=O)-, alkyl-OS(=O)ᵣ-, alkyl-S(=O)ᵣO-, alkyl-S(=O)ᵣ-, R¹³R^{13a}NC(=O)-, R¹³OC(=O)-, R¹³(=O)-, R¹³R^{13a}NS(=O)-, R¹³OS(=O)-, R¹³S(=O)-, R^{13a}R¹³N-alkyl, R¹³S(=O)-alkyl, R¹³R^{13a}N-C(=O)-alkyl, R^{13a}R¹³N-alkoxy, R¹³S(=O)-alkoxy, R¹³R^{13a}N-C(=O)-alkylamino, alkyl, heteroalkyl, carbocyclyl, cycloalkyl, heterocyclyl, aryl, heteroaryl, aralkyl, a group derived from α-amino acid, C₅₋₁₂ fused bicycle, C₅₋₁₂ fused heterobicycle, C₅₋₁₂ spiro bicycle, C₅₋₁₂ spiro heterobicycle, alkoxy, aliphatic, haloaliphatic, hydroxyaliphatic, aminoaliphatic, alkoxyaliphatic, alkylaminoaliphatic, alkylthioaliphatic, arylaliphatic, heteroarylaliphatic, heterocyclylaliphatic, cycloalkylaliphatic, aryloxyaliphatic, heterocyclyloxyaliphatic, cycloalkyloxyaliphatic, arylaminoaliphatic, heterocyclylaminoaliphatic, cycloalkylaminoaliphatic, haloalkyl, alkenyl, alkynyl, arylamino, heteroarylamino, arylalkylamino, heteroarylalkylamino, heteroaryloxy, heteroarylalkyl, arylalkoxy, heteroarylalkoxy, heterocyclyloxy, heterocyclylalkoxy, heterocyclylamino, heterocyclylalkylamino and aryloxy is optionally substituted with one or more substituents, wherein the substituent is deuterium, hydroxy, amino, halo, cyano, aryl, heteroaryl, alkoxy, alkylamino, alkylthio, alkyl, alkenyl, alkynyl, heterocyclyl, mercapto, nitro, aryloxy, heteroaryloxy, oxo (=O), carboxy, hydroxy-substituted alkoxy, hydroxy-substituted alkyl-C(=O)-, alkyl-C(=O)-, alkyl-S(=O)-, alkyl-S(=O)₂-, hydroxy-substituted alkyl-S(=O)-, hydroxy-substituted alkyl-S(=O)₂- or carboxy-substituted alkoxy.

In some embodiments, W is C₃₋₈ carbocyclyl or C₂₋₁₀ heterocyclyl.

In some embodiments, 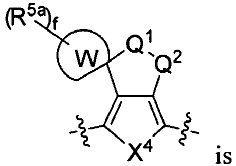 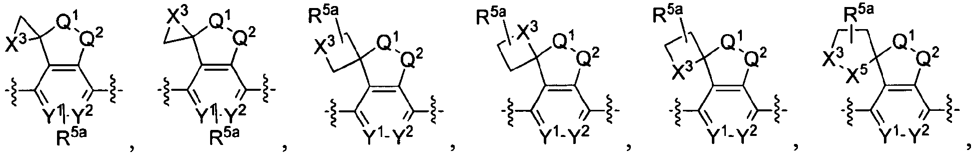 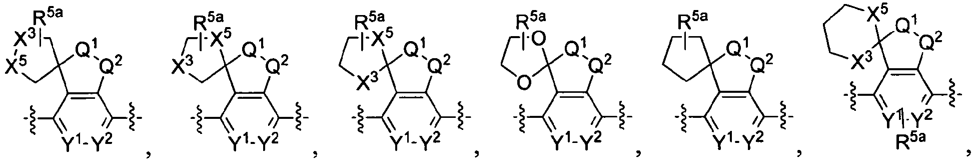 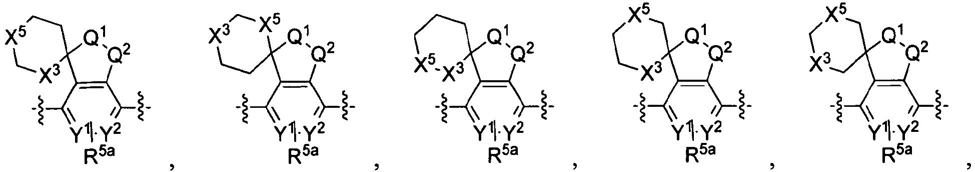 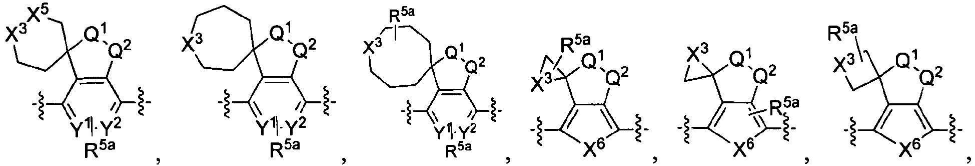 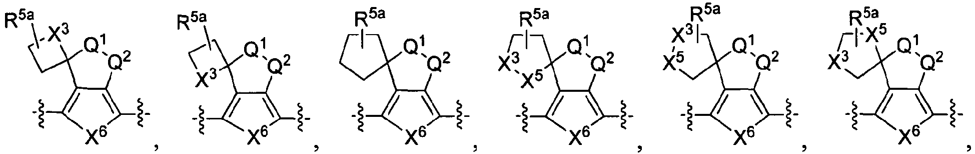 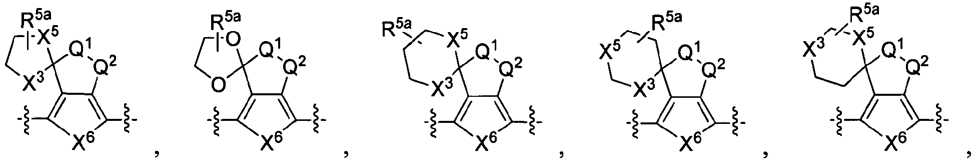 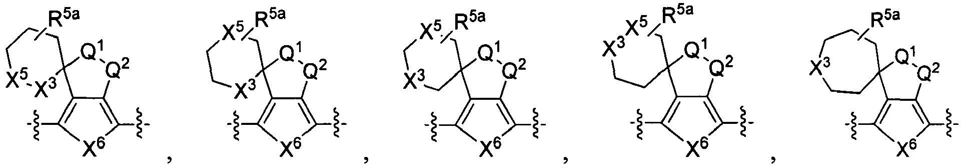 or 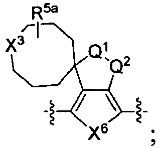
wherein each X³ and X⁵ is independently O, S, NR⁶ or CR⁷R^{7a};
each X⁶ is independently CR⁷R^{7a}, O, S or NR⁶;
each Y¹ and Y² is independently N or CR⁷;
f is 0, 1, 2, 3 or 4;
each Q¹ and Q² is independently NR⁶, O, S, C(=O) or (CR⁷R^{7a})ₑ;
e is 0, 1, 2, 3 or 4;
each R^{5a} is independently H, deuterium, oxo (=O), hydroxy, amino, F, Cl, Br, I, cyano, C₁₋₆ alkylacyl, C₁₋₆ alkylacyloxy, C₁₋₆ alkoxyacyl, C₁₋₆ alkylsulfonyl, C₁₋₆ alkoxysulfonyl, C₁₋₆ alkylsulfinyl, C₁₋₆ alkylsulfonyloxy, C₁₋₆ alkylsulfinyloxy, C₁₋₆ alkoxy, C₁₋₆ alkyl, C₆₋₁₀ aryl, -CF₃, -OCF₃, mercapto, nitro, C₁₋₆ alkylamino, C₃₋₁₀ cycloalkyl or C₆₋₁₀ aryloxy;
each R⁶ is independently H, deuterium, R¹³R^{13a}NC(=O)-, R¹³OC(=O)-, R¹³C(=O)-, R¹³R^{13a}NS(=O)-, R¹³OS(=O)-, R¹³S(=O)-, R¹³R^{13a}NS(=O)₂-, R¹³OS(=O)₂-, R¹³S(=O)₂-, C₁₋₆ alkyl, C₁₋₆ haloalkyl, C₁₋₆ hydroxyalkyl, C₁₋₆ aminoalkyl, C₁₋₆ alkoxy-C₁₋₆-alkyl, C₁₋₆ alkylamino-C₁₋₆-alkyl, C₁₋₆ alkylthio-C₁₋₆-alkyl, C₆₋₁₀ aryl-C₁₋₆-alkyl, C₁₋₉ heteroaryl-C₁₋₆-alkyl, C₂₋₁₀ heterocyclyl-C₁₋₆-alkyl, C₃₋₁₀ cycloalkyl-C₁₋₆-alkyl, C₆₋₁₀ aryloxy-C₁₋₆-alkyl, C₂₋₁₀ heterocyclyloxy-C₁₋₆-alkyl, C₃₋₁₀ cycloalkyloxy-C₁₋₆-alkyl, C₆₋₁₀ arylamino-C₁₋₆-alkyl, C₂₋₁₀ heterocyclylamino-C₁₋₆-alkyl, C₃₋₁₀ cycloalkylamino-C₁₋₆-alkyl, C₆₋₁₀ aryl, C₁₋₉ heteroaryl, C₂₋₁₀ heterocyclyl or C₃₋₁₀ carbocyclyl;
each R⁷ and R^{7a} is independently H, deuterium, F, Cl, Br, I, C₁₋₆ alkyl, C₂₋₆ heteroalkyl, C₁₋₆ haloalkyl, C₁₋₆ hydroxyalkyl, C₁₋₆ aminoalkyl, C₁₋₆ alkoxy-C₁₋₆-alkyl, C₁₋₆ alkylamino-C₁₋₆-alkyl, C₁₋₆ alkylthio-C₁₋₆-alkyl, C₆₋₁₀ aryl-C₁₋₆-alkyl, C₁₋₉ heteroaryl-C₁₋₆-alkyl, C₂₋₁₀ heterocyclyl-C₁₋₆-alkyl, C₃₋₁₀ cycloalkyl-C₁₋₆-alkyl, C₆₋₁₀ aryloxy-C₁₋₆-alkyl, C₂₋₁₀ heterocyclyloxy-C₁₋₆-alkyl, C₃₋₁₀ cycloalkyloxy-C₁₋₆-alkyl, C₆₋₁₀ arylamino-C₁₋₆-alkyl, C₂₋₁₀ heterocyclylamino-C₁₋₆-alkyl, C₃₋₁₀ cycloalkylamino-C₁₋₆-alkyl, C₆₋₁₀ aryl, C₁₋₉ heteroaryl, C₂₋₁₀ heterocyclyl or C₃₋₁₀ carbocyclyl; and
each R¹³ and R^{13a} is independently H, deuterium, C₁₋₆ alkyl, C₂₋₆ heteroalkyl, C₃₋₁₀ cycloalkyl, C₂₋₁₀ heterocyclyl, C₆₋₁₀ aryl, C₁₋₉ heteroaryl or C₆₋₁₀ aryl-C₁₋₆-alkyl; with the proviso that where R¹³ and R^{13a} are bonded to the same nitrogen atom, R¹³ and R^{13a}, together with the nitrogen atom they are attached to, optionally form a substituted or unsubstituted 3-8 membered ring or a substituted or unsubstituted spiro or fused bicyclic ring.

In some embodiments, 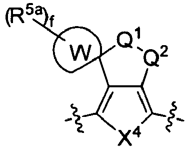 is 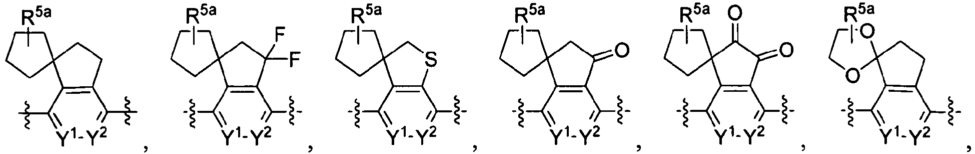 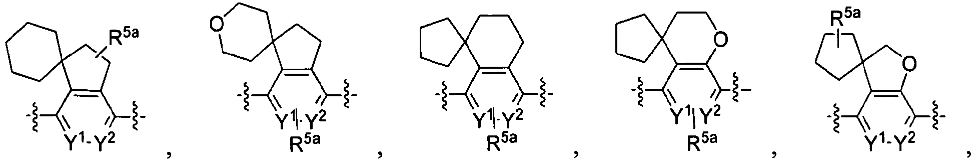 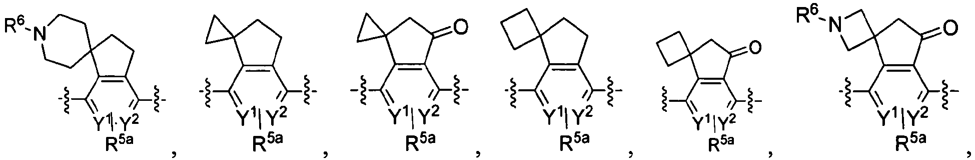 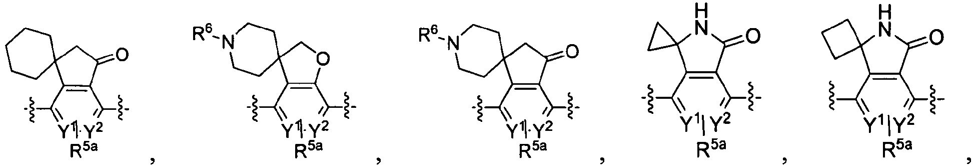 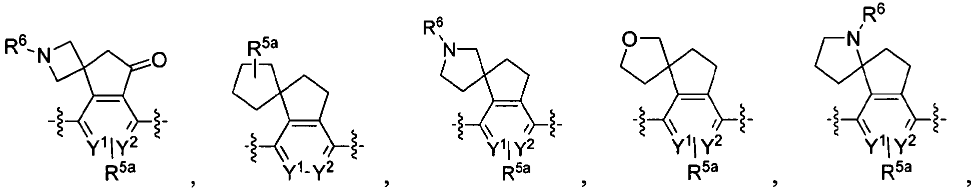 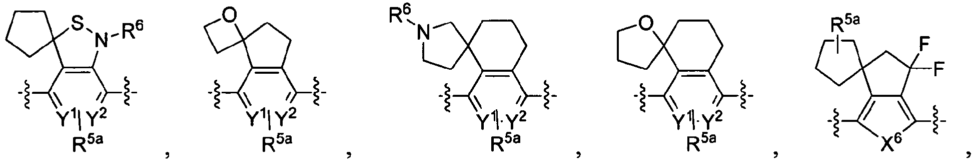 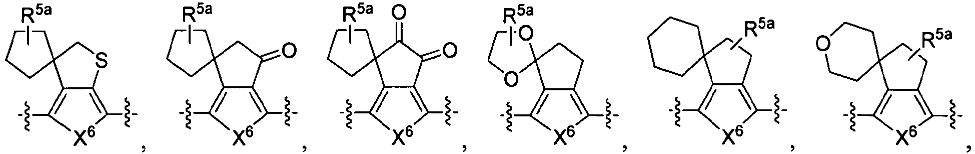 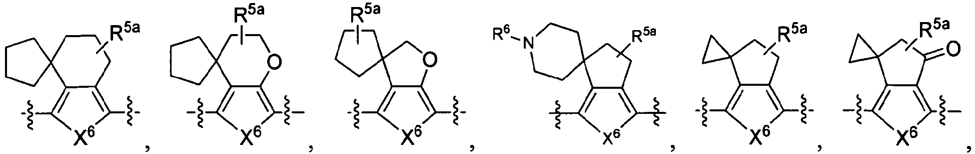 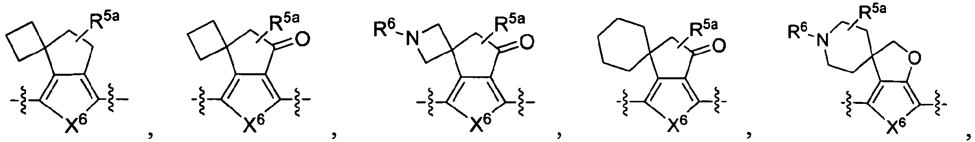 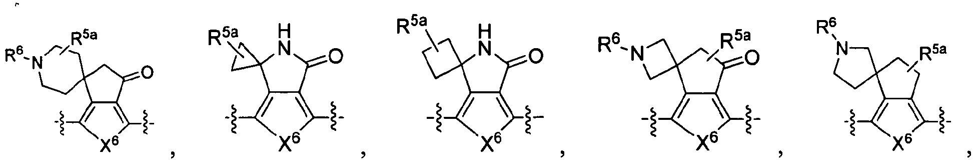 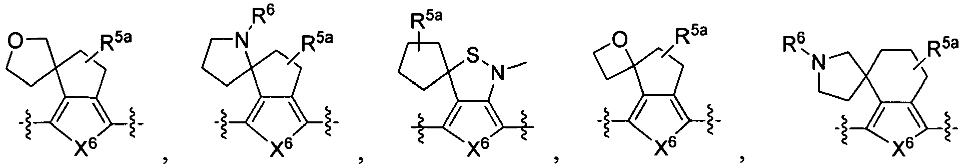 or 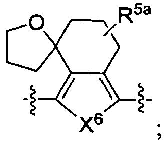
wherein each Y¹ and Y² is independently N or CR⁷;
each X⁶ is independently CR⁷R^{7a}, O, S or NR⁶;
each R^{5a} is independently H, deuterium, oxo (=O), hydroxy, amino, F, Cl, Br, I, cyano, C₁₋₆ alkylacyl, C₁₋₆ alkylacyloxy, C₁₋₆ alkoxyacyl, C₁₋₆ alkylsulfonyl, C₁₋₆ alkoxysulfonyl, C₁₋₆ alkylsulfinyl, C₁₋₆ alkylsulfonyloxy, C₁₋₆ alkylsulfinyloxy, C₁₋₆ alkoxy, C₁₋₆ alkyl, C₆₋₁₀ aryl, -CF₃, -OCF₃, mercapto, nitro or C₁₋₆ alkylamino;
each R⁶ is independently H, deuterium, C₁₋₆ alkyl, C₁₋₆ haloalkyl, C₁₋₆ hydroxyalkyl, C₁₋₆ aminoalkyl, C₁₋₆ alkoxy-C₁₋₆-alkyl, C₁₋₆ alkylamino-C₁₋₆-alkyl, C₁₋₆ alkylthio-C₁₋₆-alkyl, C₆₋₁₀ aryl-C₁₋₆-alkyl, C₁₋₉ heteroaryl-C₁₋₆-alkyl, C₂₋₁₀ heterocyclyl-C₁₋₆-alkyl or C₃₋₈ cycloalkyl-C₁₋₆-alkyl; and
each R⁷ and R^{7a} is independently H, deuterium, F, Cl, Br, I, C₁₋₆ alkyl, C₂₋₆ heteroalkyl, C₁₋₆ haloalkyl, C₁₋₆ hydroxyalkyl, C₁₋₆ aminoalkyl, C₁₋₆ alkoxy-C₁₋₆-alkyl, C₁₋₆ alkylamino-C₁₋₆-alkyl, C₁₋₆ alkylthio-C₁₋₆-alkyl, C₆₋₁₀ aryl-C₁₋₆-alkyl, C₂₋₁₀ heterocyclyl-C₁₋₆-alkyl, C₃₋₁₀ cycloalkyl-C₁₋₆-alkyl, C₆₋₁₀ aryloxy-C₁₋₆-alkyl, C₂₋₁₀ heterocyclyloxy-C₁₋₆-alkyl, C₃₋₁₀ cycloalkyloxy-C₁₋₆-alkyl, C₆₋₁₀ arylamino-C₁₋₆-alkyl, C₂₋₁₀ heterocyclylamino-C₁₋₆-alkyl, C₃₋₁₀ cycloalkylamino-C₁₋₆-alkyl, C₆₋₁₀ aryl, C₁₋₉ heteroaryl, C₂₋₁₀ heterocyclyl or C₃₋₈ carbocyclyl.

In some embodiments, each of A and A' is independently a bond, C₁₋₆ alkylene, C₂₋₆ alkenylene, C₃₋₈ cycloalkylene, C₂₋₁₀ heterocyclylalkylene, -(CR⁸R^{8a})ₙ-O-(CR⁸R^{8a})ₚ-, -(CR⁸R^{8a})ₙ-N(R⁵)-(CR⁸R^{8a})ₚ-, -(CR⁸R^{8a})ₙ-S(=O)ᵣ-N(R⁵)-(CR⁸R^{8a})ₚ-, -(CR⁸R^{8a})ₙ-C(=O)-N(R⁵)-(CR⁸R^{8a})ₚ-, -(CR⁸R^{8a})ₙ-N(R⁵)-C(=O)-N(R⁵)-(CR⁸R^{8a})ₚ-, -(CR⁸R^{8a})ₙ-C(=O)-O-(CR⁸R^{8a})ₚ-, -(CR⁸R^{8a})ₙ-N(R⁵)-S(=O)ᵣ-N(R⁵)-(CR⁸R^{8a})ₚ- or -(CR⁸R^{8a})ₙ-N(R⁵)-C(=O)-O-(CR⁸R^{8a})ₚ-; or each of A and A' is independently 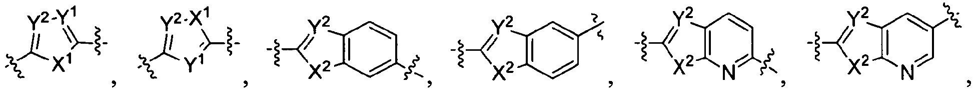 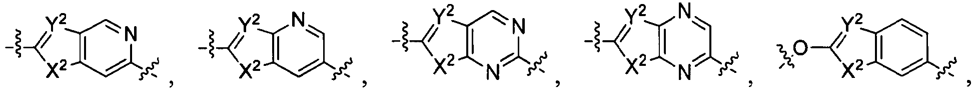 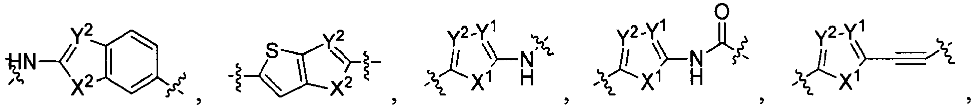 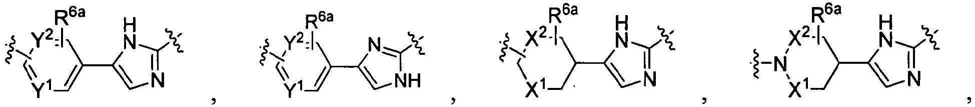 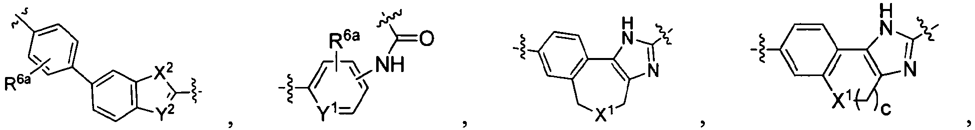 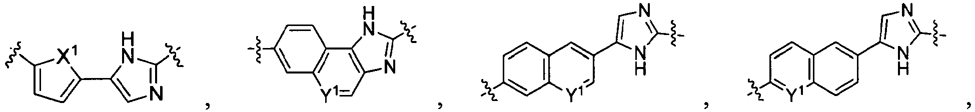 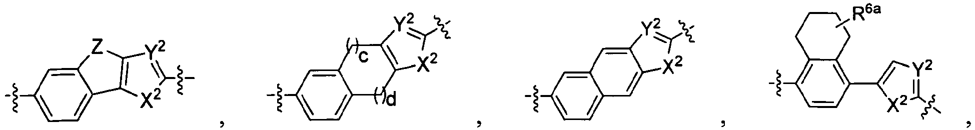 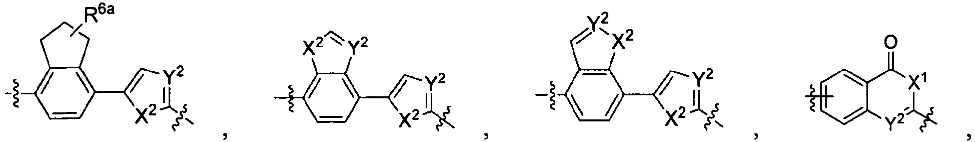 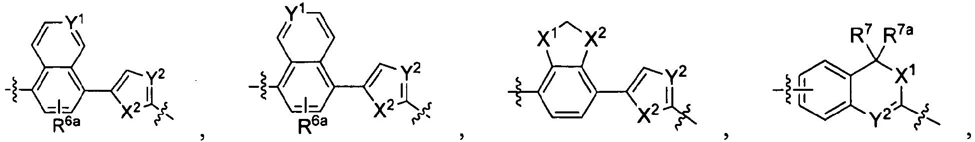 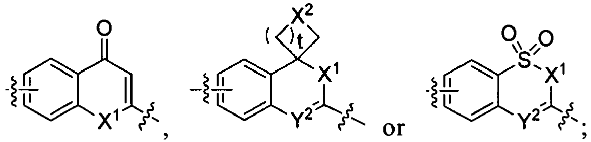
wherein each X¹ is independently O, S, NR⁶ or CR⁷R^{7a};
each X² is independently NR⁶, O or S;
each Y¹ and Y² is independently N or CR⁷;
each c is independently 1 or 2;
each d is independently 1 or 2;
each n is independently 0, 1, 2 or 3;
each p is independently 0, 1, 2 or 3;
each r is independently 0, 1 or 2;
each R⁵ is independently H, deuterium, hydroxy, C₁₋₆ alkyl, C₂₋₆ heteroalkyl, C₃₋₈ cycloalkyl, C₂₋₁₀ heterocyclyl, C₆₋₁₀ aryl, C₁₋₉ heteroaryl, C₆₋₁₀ aryl-C₁₋₆-alkyl, C₁₋₆ alkoxy, C₁₋₆ alkyl-OC(=O)-, C₁₋₆ alk yl-C(=O)-, carbamoyl, C₁₋₆ alk yl-OS(=O)ᵣ-, C₁₋₆ alk yl-S(=O)ᵣO-, C₁₋₆ alk yl-S(=O)ᵣ- or aminosulfonyl;
each R⁶ is independently H, deuterium, R¹³R^{13a}NC(=O)-, R¹³OC(=O)-, R¹³C(=O)-, R¹³R^{13a}NS(=O)-, R¹³OS(=O)-, R¹³S(=O)-, R¹³R^{13a}NS(=O)₂-, R¹³OS(=O)₂-, R¹³S(=O)₂-, C₁₋₆ alkyl, C₁₋₆ haloalkyl, C₁₋₆ hydroxyalkyl, C₁₋₆ aminoalkyl, C₁₋₆ alkoxy-C₁₋₆-alkyl, C₁₋₆ alkylamino-C₁₋₆-alkyl, C₁₋₆ alkylthio-C₁₋₆-alkyl, C₆₋₁₀ aryl-C₁₋₆-alkyl, C₁₋₉ heteroaryl-C₁₋₆-alkyl, C₂₋₁₀ heterocyclyl-C₁₋₆-alkyl, C₃₋₁₀ cycloalkyl-C₁₋₆-alkyl, C₆₋₁₀ aryloxy-C₁₋₆-alkyl, C₂₋₁₀ heterocyclyloxy-C₁₋₆-alkyl, C₃₋₁₀ cycloalkyloxy-C₁₋₆-alkyl, C₆₋₁₀ arylamino-C₁₋₆-alkyl, C₂₋₁₀ heterocyclylamino-C₁₋₆-alkyl, C₃₋₁₀ cycloalkylamino-C₁₋₆-alkyl, C₆₋₁₀ aryl, C₁₋₉ heteroaryl, C₂₋₁₀ heterocyclyl or C₃₋₁₀ carbocyclyl;
each R^{6a} is independently H, deuterium, hydroxy, amino, F, Cl, Br, I, cyano, oxo (=O), R^{13a}R¹³N-, -C(=O)NR¹³R^{13a}, -OC(=O)NR¹³R^{13a}, -OC(=O)OR¹³, -N(R¹³)C(=O)NR¹³R^{13a}, -N(R¹³)C(=O)OR^{13a}, -N(R¹³)C(=O)-R^{13a}, R¹³R^{13a}N-S(=O)₂-, R¹³S(=O)₂-, R¹³S(=O)₂N(R^{13a})-, R^{13a}R¹³N-C₁₋₆ alkyl, R¹³S(=O)-C₁₋₆ alkyl, R¹³R^{13a}N-C(=O)-C₁₋₆ alkyl, R^{13a}R¹³N-C₁₋₆ alkoxy, R¹³S(=O)-C₁₋₆ alkoxy, R¹³R^{13a}N-C(=O)-C₁₋₆ alkoxy, C₆₋₁₀ aryl, C₁₋₉ heteroaryl, C₁₋₆ alkoxy, C₁₋₆ alkylamino, C₁₋₆ alkyl, C₁₋₆ haloalkyl, C₂₋₆ alkenyl, C₂₋₆ alkynyl, C₂₋₁₀ heterocyclyl, C₃₋₈ cycloalkyl, mercapto, nitro, C₆₋₁₀ aryl-C₁₋₆-alkyl, C₆₋₁₀ arylamino, C₁₋₉ heteroarylamino or C₆₋₁₀ aryloxy;
each R⁷ and R^{7a} is independently H, deuterium, F, Cl, Br, I, C₁₋₆ alkyl, C₂₋₆ heteroalkyl, C₁₋₆ haloalkyl, C₁₋₆ hydroxyalkyl, C₁₋₆ aminoalkyl, C₁₋₆ alkoxy-C₁₋₆-alkyl, C₁₋₆ alkylamino-C₁₋₆-alkyl, C₁₋₆ alkylthio-C₁₋₆-alkyl, C₆₋₁₀ aryl-C₁₋₆-alkyl, C₂₋₁₀ heterocyclyl-C₁₋6-alkyl, C₃₋₁₀ cycloalkyl-C₁₋₆-alkyl, C₆₋₁₀ aryloxy-C₁₋₆-alkyl, C₂₋₁₀ heterocyclyloxy-C₁₋₆-alkyl, C₃₋₁₀ cycloalkyloxy-C₁₋₆-alkyl, C₆₋₁₀ arylamino-C₁₋₆-alkyl, C₂₋₁₀ heterocyclylamino-C₁₋₆-alkyl, C₃₋₁₀ cycloalkylamino-C₁₋₆-alkyl, C₆₋₁₀ aryl, C₁₋₉ heteroaryl, C₂₋₁₀ heterocyclyl or C₃₋₈ carbocyclyl;
each R¹³ and R^{13a} is independently H, deuterium, C₁₋₆ alkyl, C₂₋₆ heteroalkyl, C₃₋₁₀ cycloalkyl, C₂₋₁₀ heterocyclyl, C₆₋₁₀ aryl, C₁₋₉ heteroaryl or C₆₋₁₀ aryl-C₁₋₆-alkyl; with the proviso that where R¹³ and R^{13a} are bonded to the same nitrogen atom, R¹³ and R^{13a}, together with the nitrogen atom they are attached to, optionally form a substituted or unsubstituted 3-8 membered ring or a substituted or unsubstituted spiro or fused bicyclic ring; and
each R⁸ and R^{8a} is independently H, deuterium, hydroxy, cyano, nitro, F, Cl, Br, I, C₁₋₆ alkyl, C₂₋₆ heteroalkyl, C₃₋₁₀ cycloalkyl, C₂₋₁₀ heterocyclyl, C₆₋₁₀ aryl, C₁₋₉ heteroaryl, C₆₋₁₀ aryl-C₁₋₆-alkyl, C₁₋₆ alkoxy, C₁₋₆ alkyl-OC(=O)-, C₁₋₆ alkyl-C(=O)-, carbamoyl, C₁₋₆ alkyl-OS(=O)ᵣ-, C₁₋₆ alkyl-S(=O)rO-, C₁₋₆ alkyl-S(=O)ᵣ- or aminosulfonyl.

In some embodiments, each of A and A' is independently a bond, -CH₂-, -(CH₂)₂-, -CH=CH-, -CH=CH-CH₂-, -N(R⁶)-, -C(=O)-, -C(=S)-, -C(=O)-O-, -C(=O)N(R⁶)-, -OC(=O)N(R⁶)-, -OC(=O)O-, -N(R⁶)C(=O)N(R⁶)-, -(R⁶)N-S(=O)₂-, -S(=O)₂-, -OS(=O)₂-, -(R⁶)N-S(=O)-, -S(=O)-, -OS(=O)-; or each of A and A' is independently 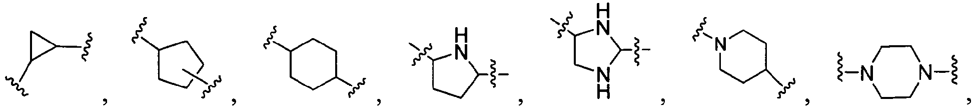 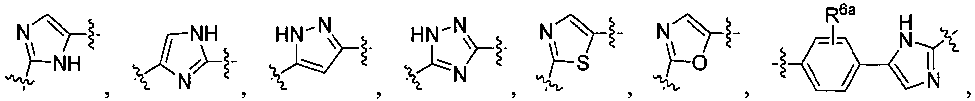 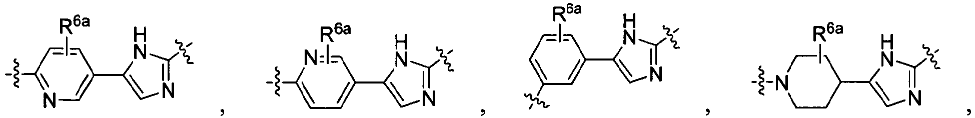 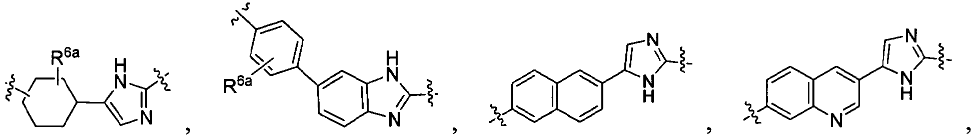 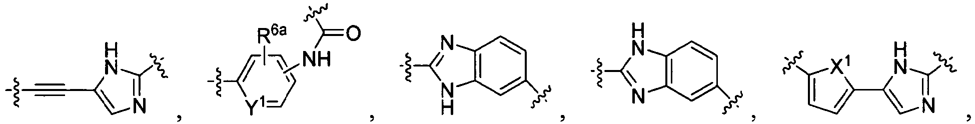 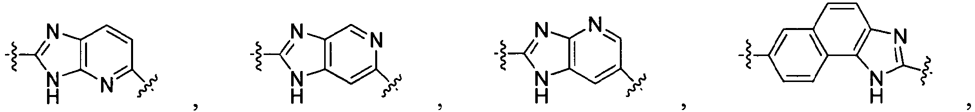 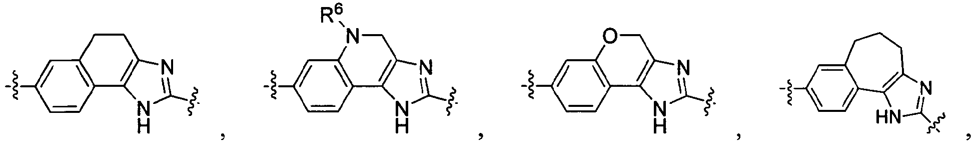 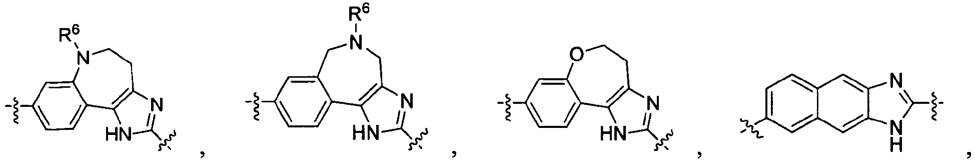 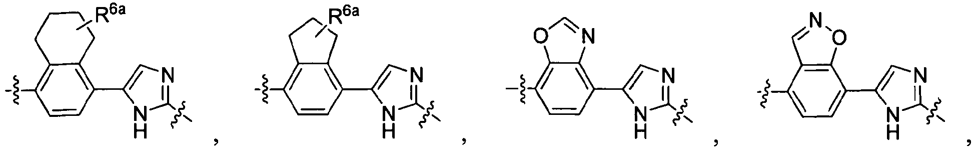 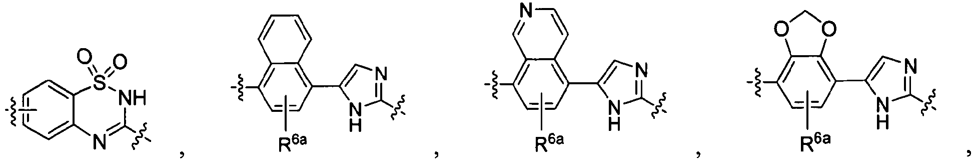 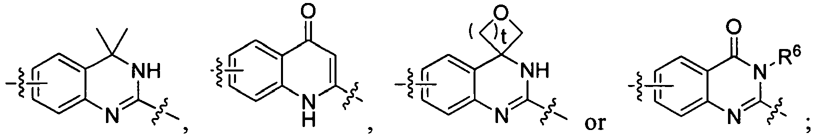
wherein X¹ is O or S;
each Y¹ independently is N or CR⁷;
each R⁶ is independently H, deuterium, C₁₋₄ alkyl, C₁₋₆ haloalkyl, C₁₋₆ hydroxyalkyl, C₁₋₆ aminoalkyl, C₁₋₆ alkoxy-C₁₋₄-alkyl, C₁₋₆ alkylamino-C₁₋₄-alkyl, C₁₋₆ alkylthio-C₁₋₄-alkyl, C₆₋₁₀ aryl-C₁₋₄-alkyl, C₁₋₉ heteroaryl, C₆₋₁₀ aryl, C₂₋₁₀ heterocyclyl or C₃₋₈ carbocyclyl;
each R^{6a} and R⁷ is independently H, deuterium, oxo (=O), hydroxy, amino, F, Cl, Br, I, cyano, R^{13a}R¹³N-, C₁₋₆ alkoxy, C₁₋₆ alkylamino, C₁₋₆ alkyl, C₁₋₆ haloalkyl, C₂₋₆ alkenyl, C₂₋₆ alkynyl, mercapto or nitro; and
each of R¹³ and R^{13a} is independently H, deuterium, C₁₋₆ alkyl, C₂₋₆ heteroalkyl, C₃₋₁₀ cycloalkyl, C₂₋₁₀ heterocyclyl, C₆₋₁₀ aryl, C₁₋₉ heteroaryl or C₆₋₁₀ aryl-C₁₋₆-alkyl; with the proviso that where R¹³ and R^{13a} are bonded to the same nitrogen atom, R¹³ and R^{13a}, together with the nitrogen atom they are attached to, optionally form a substituted or unsubstituted 3-8 membered ring or a substituted or unsubstituted spiro or fused bicyclic ring.

In some embodiments, each of R¹, R², R³ and R⁴ is independently H, deuterium, C₁₋₈ alkyl, C₁₋₈ heteroalkyl, C₆₋₁₀ aryl-C₁₋₆-alkyl, C₃₋₁₀ cycloalkyl, C₂₋₁₀ heterocyclyl, C₁₋₉ heteroaryl or C₆₋₁₀ aryl; or R¹ and R², together with X-CH they are attached to, optionally form a 3-8 membered heterocycle or carbocycle, C₅₋₁₂ fused bicycle, C₅₋₁₂ fused heterobicycle, C₅₋₁₂ spiro bicycle or C₅₋₁₂ spiro heterobicycle; or R³ and R⁴, together with X'-CH they are attached to, optionally form a 3-8 membered heterocycle or carbocycle, C₅₋₁₂ fused bicycle, C₅₋₁₂ fused heterobicycle, C₅₋₁₂ spiro bicycle or C₅₋₁₂ spiro heterobicycle.

In other embodiments, R¹ and R², together with X-CH they are attached to, or R³ and R⁴, together with X'-CH they are attached to, optionally form a 3-8 membered heterocycle, C₅₋₁₂ fused bicycle, C₅₋₁₂ fused heterobicycle, C₅₋₁₂ spiro bicycle or C₅₋₁₂ spiro heterobicycle.

In other embodiments, R¹, R² and X-CH together form one of the following monovalent groups: 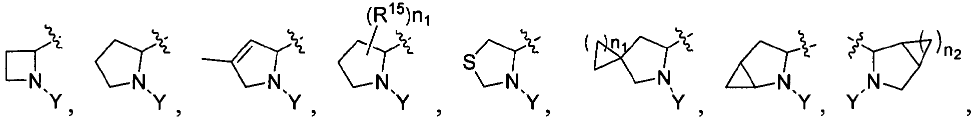 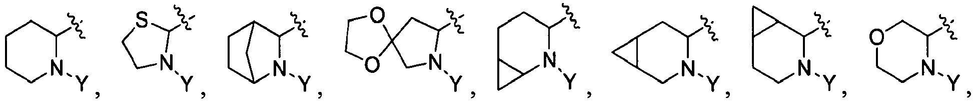 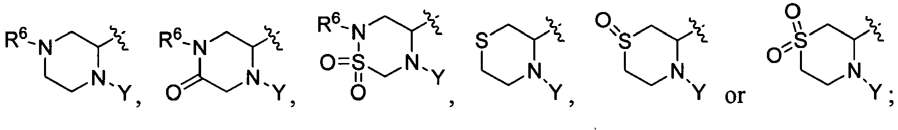
wherein each R¹⁵ is independently H, deuterium, F, Cl, Br, I, cyano, hydroxy, C₁₋₃ alkyl, C₁₋₃ haloalkyl, C₁₋₃ alkoxy, C₁₋₃ alkylamino, C₁₋₃ alkylthio, C₆₋₁₀ arylamino, C₆₋₁₀ aryloxy, C₁₋₉ heteroaryl, C₁₋₉ heteroaryloxy, C₁₋₉ heteroaryl-C₁₋₃-alkyl or C₂₋₁₀ heterocyclyl;
each R⁶ is independently H, deuterium, C₁₋₃ alkyl, C₁₋₃ haloalkyl, C₁₋₃ hydroxyalkyl, C₁₋₃ aminoalkyl, C₁₋₃ alkoxy-C₁₋₃-alkyl, C₁₋₃ alkylamino-C₁₋₃-alkyl, C₁₋₃ alkylthio-C₁₋₃-alkyl, C₆₋₁₀ aryl-C₁₋₃-alkyl, C₁₋₉ heteroaryl, C₆₋₁₀ aryl, C₂₋₁₀ heterocyclyl or C₃₋₈ carbocyclyl; and
each n₁ and n₂ is independently 1, 2, 3 or 4.

In other embodiments, R³, R⁴ and X'-CH together form one of the following monovalent groups: 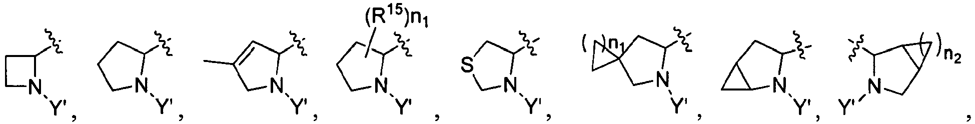 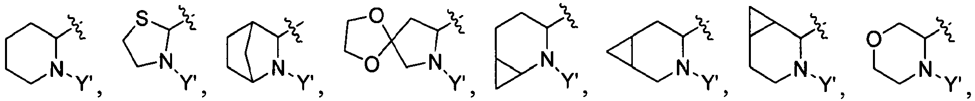 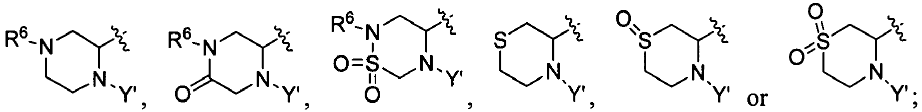
wherein each R¹⁵ is independently H, deuterium, F, Cl, Br, I, cyano, hydroxy, C₁₋₃ alkyl, C₁₋₃ haloalkyl, C₁₋₃ alkoxy, C₁₋₃ alkylamino, C₁₋₃ alkylthio, C₆₋₁₀ arylamino, C₆₋₁₀ aryloxy, C₁₋₉ heteroaryl, C₁₋₉ heteroaryloxy, C₁₋₉ heteroaryl-C₁₋₃-alkyl or C₂₋₁₀ heterocyclyl;
each R⁶ is independently H, deuterium, C₁₋₃ alkyl, C₁₋₃ haloalkyl, C₁₋₃ hydroxyalkyl, C₁₋₃ aminoalkyl, C₁₋₃ alkoxy-C₁₋₃-alkyl, C₁₋₃ alkylamino-C₁₋₃-alkyl, C₁₋₃ alkylthio-C₁₋₃-alkyl, C₆₋₁₀ aryl-C₁₋₃-alkyl, C₁₋₉ heteroaryl, C₆₋₁₀ aryl, C₂₋₁₀ heterocyclyl or C₃₋₈ carbocyclyl; and
each n₁ and n₂ is independently 1, 2, 3 or 4.

In some embodiments, the compound may have formula (II): 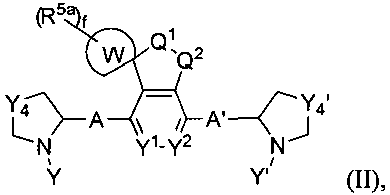 wherein 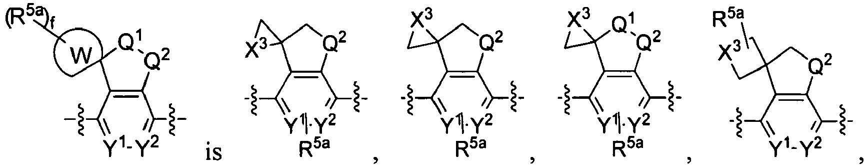 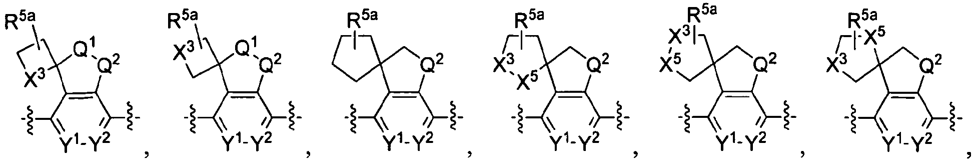 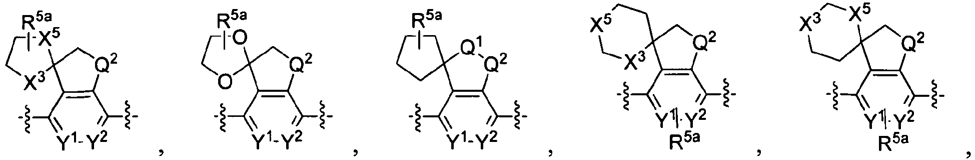 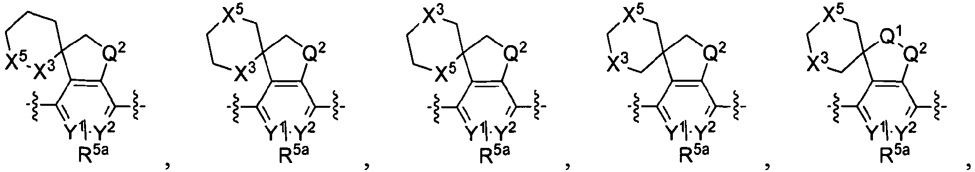 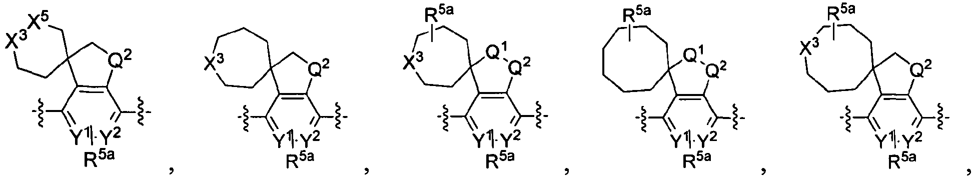 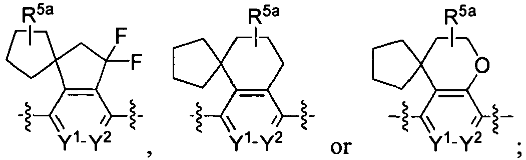
wherein each Q¹ and Q² is independently NR⁶, O, S, C(=O) or (CH₂)ₑ;
e is 0, 1, 2, 3 or 4;
each X¹, X³ and X⁵ is independently O, S, NR⁶ or CR⁷R^{7a};
each Y¹ and Y² is independently N or CR⁷;
each of A and A' is independently a bond, C₁₋₆ alkylene, C₂₋₆ alkenylene, C₃₋₈ cycloalkylene, C₂₋₁₀ heterocyclylalkylene, -(CR⁸R^{8a})ₙ-O-(CR⁸R^{8a})ₚ-, -(CR⁸R^{8a})ₙ-N(R⁵)-(CR⁸R^{8a})ₚ-, -(CR⁸R^{8a})ₙ-S(=O)ᵣ-N(R⁵)-(CR⁸R^{8a})ₚ-, -(CR⁸R^{8a})ₙ-C(=O)-N(R⁵)-(CR⁸R^{8a})ₚ-, -(CR⁸R^{8a})ₙ-N(R⁵)-C(=O)-N(R⁵)-(CR⁸R^{8a})ₚ-, -(CR⁸R^{8a})ₙ-C(=O)-O-(CR⁸R^{8a})ₚ-, -(CR⁸R^{8a})ₙ-N(R⁵)-S(=O)ᵣ-N(R⁵)-(CR⁸R^{8a})ₚ, -(CR⁸R^{8a})ₙ-N(R⁵)-C(=O)-O-(CR⁸R^{8a})ₚ-; or each of A and A' is independently 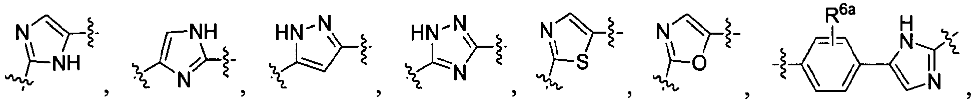 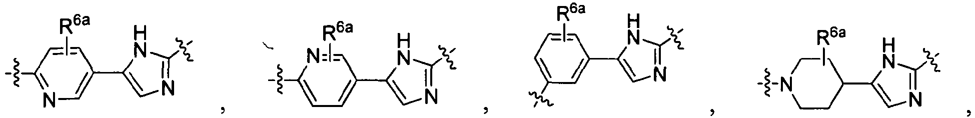 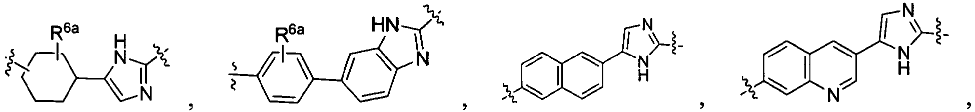 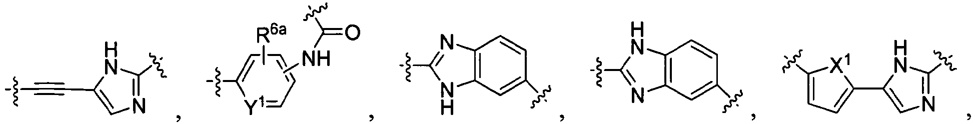 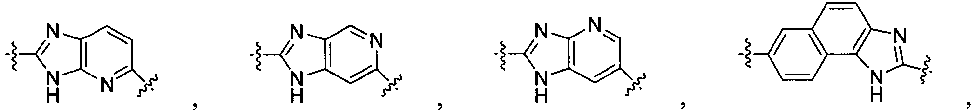 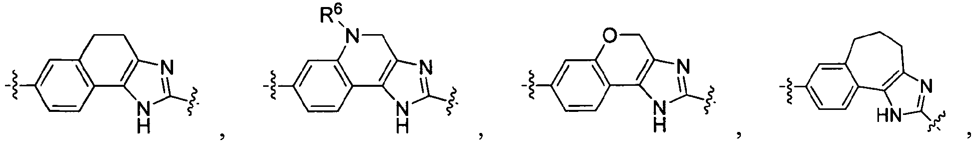 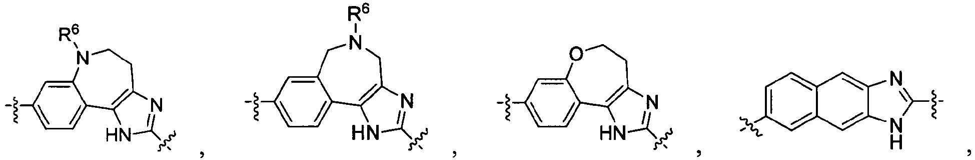 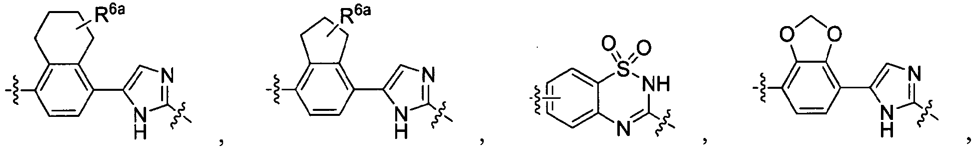 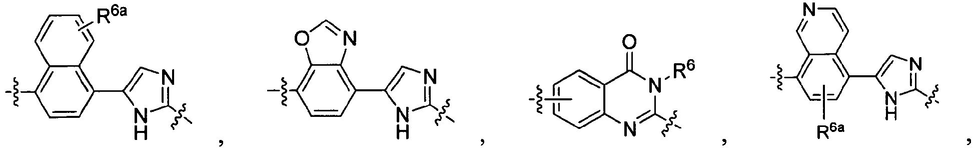 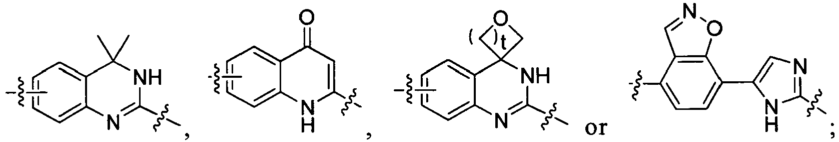
each R⁵ is independently H, deuterium, hydroxy, C₁₋₆ alkyl, C₂₋₆ heteroalkyl, C₃₋₁₀ cycloalkyl, C₂₋₁₀ heterocyclyl, C₆₋₁₀ aryl, C₁₋₉ heteroaryl, C₆₋₁₀ aryl-C₁₋₆-alkyl, C₁₋₆ alkoxy, C₁₋₆ alkyl-OC(=O)-, C_{I}-₆ alk yl-C(=O)-, carbamoyl, C₁₋₆ alk yl-OS(=O)ᵣ-, C₁₋₆ alk yl-S(=O)ᵣO-, C₁₋₆ alk yl-S(=O)ᵣ- or aminosulfonyl;
each R^{5a} and R^{6a} is independently H, deuterium, oxo (=O), hydroxy, amino, F, Cl, Br, I, cyano, C₁₋₆ alkylacyl, C₁₋₆ alkylacyloxy, C₁₋₆ alkoxyacyl, C₁₋₆ alkylsulfonyl, C₁₋₆ alkoxysulfonyl, C₁₋₆ alkylsulfinyl, C₁₋₆ alkylsulfonyloxy, C₁₋₆ alkylsulfinyloxy, C₁₋₆ alkoxy, C₁₋₆ alkyl, C₆₋₁₀ aryl, -CF₃, -OCF₃, mercapto, nitro, C₁₋₆ alkylamino, C₃₋₁₀ cycloalkyl or C₆₋₁₀ aryloxy;
each R⁶ is independently H, deuterium, R¹³R^{13a}NC(=O)-, R¹³OC(=O)-, R¹³C(=O)-, R¹³R^{13a}NS(=O)-, R¹³OS(=O)-, R¹³S(=O)-, R¹³R^{13a}NS(=O)₂-, R¹³OS(=O)₂-, R¹³S(=O)₂-, C₁₋₆ aliphatic, C₁₋₆-alkoxy-C₁₋₆-aliphatic, C₁₋₆ alkylamino-C₁₋₆-aliphatic, C₆₋₁₀ aryl-C₁₋₆-aliphatic, C₁₋₉ heteroaryl-C₁₋₆-aliphatic, C₂₋₁₀ heterocyclyl-C₁₋₆-aliphatic, C₃₋₁₀ cycloalkyl-C₁₋₆-aliphatic, C₆₋₁₀ aryl, C₁₋₉ heteroaryl, C₂₋₁₀ heterocyclyl or C₃₋₁₀ carbocyclyl;
each R⁷ and R^{7a} is independently H, deuterium, F, Cl, Br, I, C₁₋₆ aliphatic, C₂₋₆ heteroalkyl, C₁₋₆ alkoxy-C₁₋₆-aliphatic, C₁₋₆ alkylamino-C₁₋₆-aliphatic, C₆₋₁₀ aryl-C₁₋₆-aliphatic, C₂₋₁₀ heterocyclyl-C₁₋₆-aliphatic, C₃₋₁₀ cycloalkyl-C₁₋₆ aliphatic, C₆₋₁₀ aryl, C₁₋₉ heteroaryl, C₂₋₁₀ heterocyclyl or C₃₋₁₀ carbocyclyl;
each R⁸ and R^{8a} is independently H, deuterium, hydroxy, cyano, nitro, F, Cl, Br, I, C₁₋₆ alkyl, C₂₋₆ heteroalkyl, C₃₋₁₀ cycloalkyl, C₂₋₁₀ heterocyclyl, C₆₋₁₀ aryl, C₁₋₉ heteroaryl, C₆₋₁₀ aryl-C₁₋₆-alkyl, C₁₋₆ alkoxy, C₁₋₆ alkyl-OC(=O)-, C₁₋₆ alkyl-C(=O)-, carbamoyl, C₁₋₆ alkyl-OS(=O)ᵣ-, C₁₋₆ alkyl-S(=O)ᵣO-, C₁₋₆ alkyl-S(=O)ᵣ- or aminosulfonyl;
each R¹³ and R^{13a} is independently H, deuterium, C₁₋₆ alkyl, C₂₋₆ heteroalkyl, C₃₋₁₀ cycloalkyl, C₂₋₁₀ heterocyclyl, C₆₋₁₀ aryl, C₁₋₉ heteroaryl or C₆₋₁₀ aryl-C₁₋₆-alkyl; with the proviso that where R¹³ and R^{13a} are bonded to the same nitrogen atom, R¹³ and R^{13a}, together with the nitrogen atom they are attached to, optionally form a substituted or unsubstituted 3-8 membered ring or a substituted or unsubstituted spiro or fused bicyclic ring;
each n is independently 0, 1, 2 or 3;
each p is independently 0, 1, 2 or 3;
each r is independently 0, 1 or 2; and
each of Y₄ and Y₄' is independently a bond, O, S, -(CH₂)ₙ-, -CH=CH-, -S(=O)ᵣ-, -CH₂O-, -CH₂S-, -CH₂F-, -CHR^{5a}-, -CR^{5a}R^{6a}-, -CH₂S(=O)ᵣ- or -CH₂N(R⁶)-.

In some embodiments, the compound may have formula (II'): 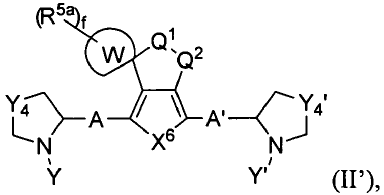 wherein 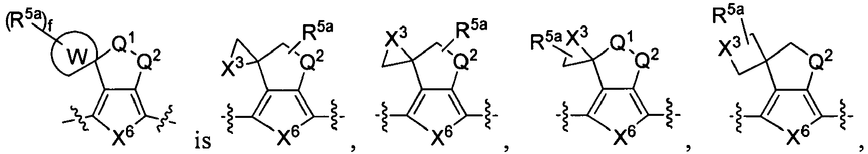 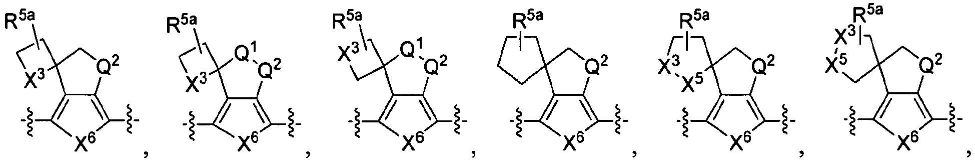 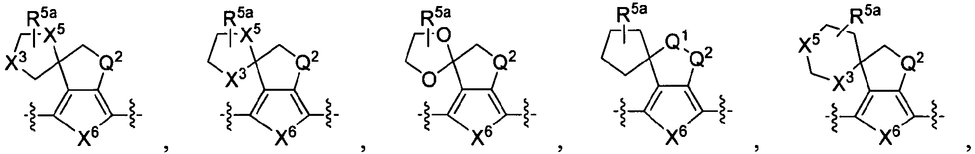 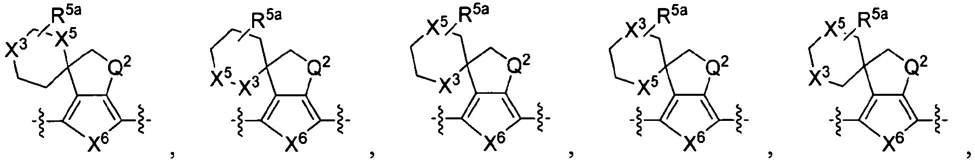 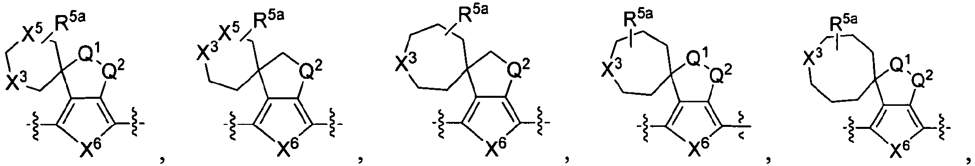 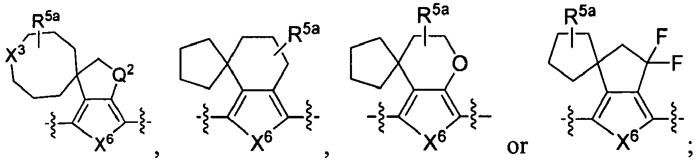
wherein each Q¹ and Q² is independently NR⁶, O, S, C(=O) or (CH₂)ₑ;
e is 0, 1, 2, 3 or 4;
each X¹, X³ and X⁵ is independently O, S, NR⁶ or CR⁷R^{7a};
each X⁶ is independently CR⁷R^{7a}, O, S or NR⁶;
f is 0, 1, 2, 3 or 4;
each of A and A' is independently a bond, C₁₋₆ alkylene, C₂₋₆ alkenylene, C₃₋₈ cycloalkylene, C₂₋₁₀ heterocyclylalkylene, -(CR⁸R^{8a})ₙ-O-(CR⁸R^{8a})ₚ-, -(CR⁸R^{8a})ₙ-N(R⁵)-(CR⁸R^{8a})ₚ-, -(CR⁸R^{8a})ₙ-S(=O)ᵣ-N(R⁵)-(CR⁸R^{8a})ₚ-, -(CR⁸R^{8a})ₙ-C(=O)-N(R⁵)-(CR⁸R^{8a})ₚ-, -(CR⁸R^{8a})ₙ-N(R⁵)-C(=0)-N(R⁵)-(CR⁸R^{8a})ₚ-, -(CR⁸R^{8a})ₙ-C(=O)-O-(CR⁸R^{8a})ₚ-, -(CR⁸R^{8a})ₙ-N(R⁵)-S(=O)ᵣ-N(R⁵)-(CR⁸R^{8a})ₚ-, -(CR⁸R^{8a})ₙ-N(R⁵)-C(=O)-O-(CR⁸R^{8a})ₚ-; or each of A and A' is independently 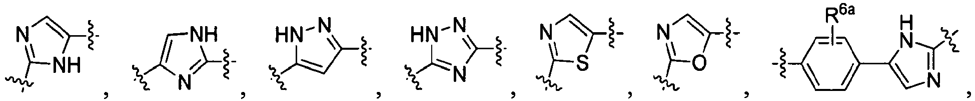 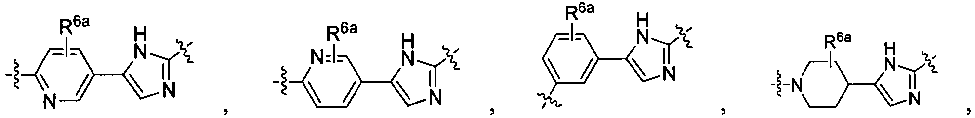 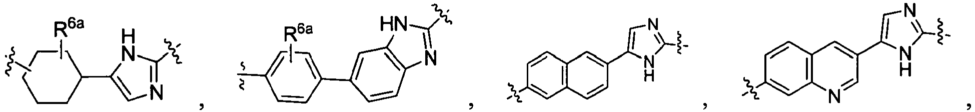 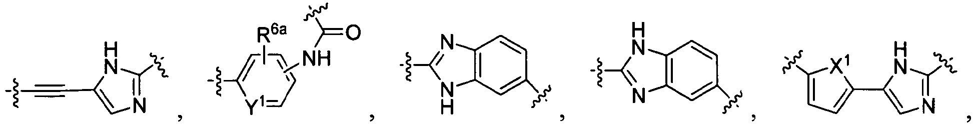 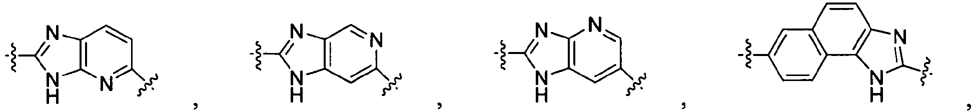 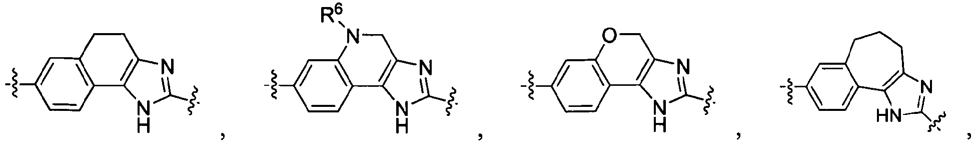 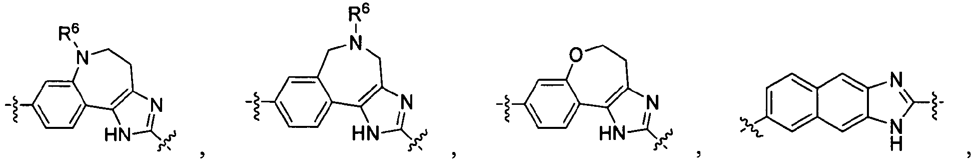 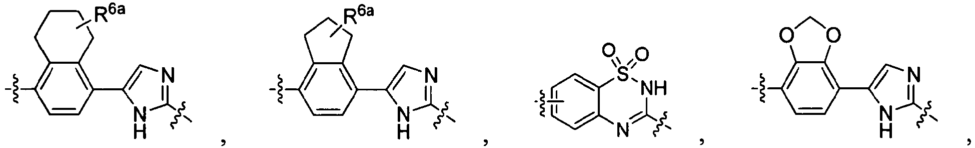 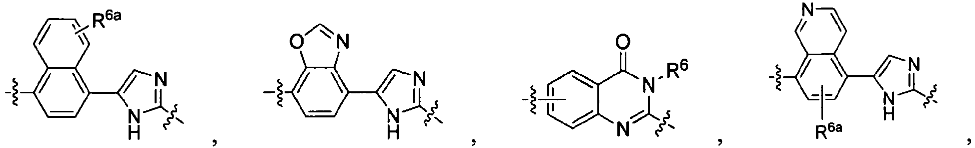 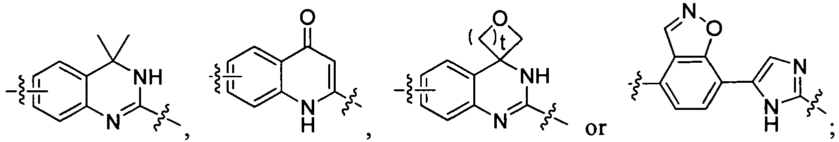
each Y¹ is independently N or CR⁷;
each R⁵ is independently H, deuterium, hydroxy, C₁₋₆ alkyl, C₂₋₆ heteroalkyl, C₃₋₁₀ cycloalkyl, C₂₋₁₀ heterocyclyl, C₆₋₁₀ aryl, C₁₋₉ heteroaryl, C₆₋₁₀ aryl-C₁₋₆-alkyl, C₁₋₆ alkoxy, C₁₋₆ alkyl-OC(=O)-, C₁₋₆ alk yl-C(=O)-, carbamoyl, C₁₋₆ alk yl-OS(=O)ᵣ-, C₁₋₆ alk yl-S(=O)ᵣO-, C₁₋₆ alk yl-S(=O)ᵣ- or aminosulfonyl;
each R^{5a} and R^{6a} is independently H, deuterium, oxo (=O), hydroxy, amino, F, Cl, Br, I, cyano, C₁₋₆ alkylacyl, C₁₋₆ alkylacyloxy, C₁₋₆ alkoxyacyl, C₁₋₆ alkylsulfonyl, C₁₋₆ alkoxysulfonyl, C₁₋₆ alkylsulfinyl, C₁₋₆ alkylsulfonyloxy, C₁₋₆ alkylsulfinyloxy, C₁₋₆ alkoxy, C₁₋₆ alkyl, C₆₋₁₀ aryl, -CF₃, -OCF₃, mercapto, nitro, C₁₋₆ alkylamino, C₃₋₁₀ cycloalkyl or C₆₋₁₀ aryloxy;
each R⁶ is independently H, deuterium, R¹³R^{13a}NC(=O)-, R¹³OC(=O)-, R¹³C(=O)-, R¹³R^{13a}NS(=O)-, R¹³OS(=O)-, R¹³S(=O)-, R¹³R^{13a}NS(=O)₂-, R¹³OS(=O)₂-, R¹³S(=O)₂-, C₁₋₆ aliphatic, C₁₋₆ alkoxy-C₁₋₆-aliphatic, C₁₋₆ alkylamino-C₁₋₆-aliphatic, C₆₋₁₀ aryl-C₁₋₆-aliphatic, C₁₋₉ heteroaryl-C₁₋₆-aliphatic, C₂₋₁₀ heterocyclyl-C₁₋₆-aliphatic, C₃₋₁₀ cycloalkyl-C₁₋₆-aliphatic, C₆₋₁₀ aryl, C₁₋₉ heteroaryl, C₂₋₁₀ heterocyclyl or C₃₋₁₀ carbocyclyl;
each R⁷ and R^{7a} is independently H, deuterium, F, Cl, Br, I, C₁₋₆ aliphatic, C₂₋₆ heteroalkyl, C₁₋₆ alkoxy-C₁₋₆-aliphatic, C₁₋₆ alkylamino-C₁₋₆-aliphatic, C₆₋₁₀ aryl-C₁₋₆-aliphatic, C₂₋₁₀ heterocyclyl-C₁₋₆-aliphatic, C₃₋₁₀ cycloalkyl-C₁₋₆-aliphatic, C₆₋₁₀ aryl, C₁₋₉ heteroaryl, C₂₋₁₀ heterocyclyl or C₃₋₁₀ carbocyclyl;
each R⁸ and R^{8a} is independently H, deuterium, hydroxy, cyano, nitro, F, Cl, Br, I, C₁₋₆ alkyl, C₂₋₆ heteroalkyl, C₃₋₁₀ cycloalkyl, C₂₋₁₀ heterocyclyl, C₆₋₁₀ aryl, C₁₋₉ heteroaryl, C₆₋₁₀ aryl-C₁₋₆-alkyl, C₁₋₆ alkoxy, C₁₋₆ alkyl-OC(=O)-, C₁₋₆ alkyl-C(=O)-, carbamoyl, C₁₋₆ alkyl-OS(=O)ᵣ-, C₁₋₆ alkyl-S(=O)ᵣO-, C₁₋₆ alkyl-S(=O)ᵣ- or aminosulfonyl;
each R¹³ and R^{13a} is independently H, deuterium, C₁₋₆ alkyl, C₂₋₆ heteroalkyl, C₃₋₁₀ cycloalkyl, C₂₋₁₀ heterocyclyl, C₆₋₁₀ aryl, C₁₋₉ heteroaryl or C₆₋₁₀ aryl-C₁₋₆-alkyl; with the proviso that where R¹³ and R^{13a} are bonded to the same nitrogen atom, R¹³ and R^{13a}, together with the nitrogen atom they are attached to, optionally form a substituted or unsubstituted 3-8 membered ring or a substituted or unsubstituted spiro or fused bicyclic ring;
each n is independently 0, 1, 2 or 3;
each p is independently 0, 1, 2 or 3;
each r is independently 0, 1 or 2; and
each of Y₄ and Y₄' is independently a bond, O, S, -(CH₂)ₙ-, -CH=CH-, -S(=O)ᵣ-, -CH₂O-, -CH₂S-, -CH₂F-, -CHR^{5a}-, -CR^{5a}R^{6a}-, -CH₂S(=O)ᵣ- or -CH₂N(R⁶)-.

In other embodiments, the compound may have formula (III):

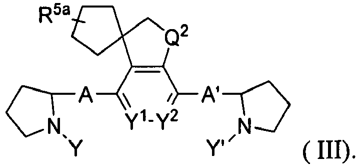

In other embodiments, the compound may have formula (IV): 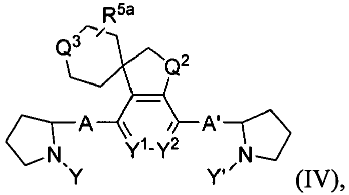 wherein each of Q² and Q³ is independently O, S, C(=O), NR⁶ or CH₂.

In other embodiments, the compound may have formula (V): 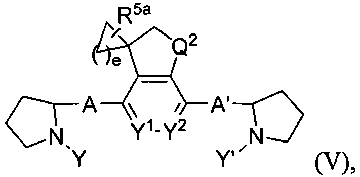 wherein e is 1, 2, 3 or 4.

In other embodiments, the compound may have formula (III'):

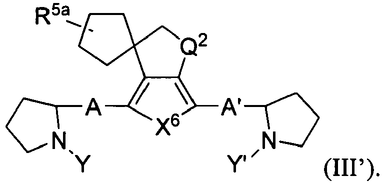

In other embodiments, the compound may have formula (IV'): 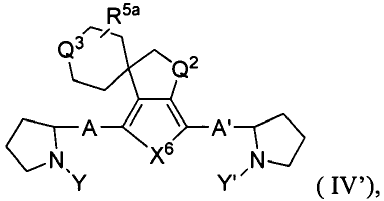 wherein each of Q² and Q³ is independently O, S, C(=O), NR⁶ or CH₂.

In other embodiments, the compound may have formula (V'): 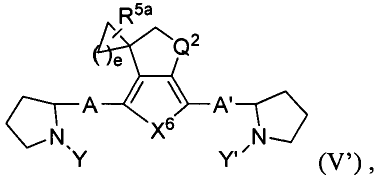 wherein e is 1, 2, 3 or 4.

In some embodiments, each of Y and Y' is independently a group derived from α-amino acid and the group derived from α-amino acid is optionally substituted with one or more substituents, wherein the substituent is deuterium, F, Cl, Br, I, hydroxy, cyano, alkoxycarbonyl, aryloxycarbonyl, heteroaryloxycarbonyl or heterocyclylcarbonyl.

In other embodiments, the α-amino acid is isoleucine, leucine, lysine, methionine, phenylalanine, threonine, tryptophane, valine, alanine, asparagine, aspartic acid, glutamic acid, glutamine, proline, serine, p-tyrosine, arginine, histidine, cysteine, glycine, sarcosine, *N,N*-dimethylglycine, homoserine, norvaline, norleucine, ornithine, homocysteine, homophenylalanine, phenylglycine, o-tyrosine, m-tyrosine or hydroxyproline.

In other embodiments, the α-amino acid is in the D configuration.

In other embodiments, the α-amino acid is in the L configuration.

In some embodiments, each of Y and Y' is independently -[U-(CR⁹R^{9a})ₜ-N(R¹⁰)-(CR⁹R^{9a})ₜ]ₖ-U-(CR⁹R^{9a})ₜ-N(R¹¹)-(CR⁹R^{9a})ₜ-R¹², -U-(CR⁹R^{9a})ₜ-R¹² or -[U-(CR⁹R^{9a})ₜ-N(R¹⁰)-(CR⁹R^{9a})ₜ]ₖ-U-(CR⁹R^{9a})ₜ-O-(CR⁹R^{9a})ₜ-R¹².

In other embodiments, each of Y and Y' is independently -[U-(CR⁹R^{9a})ₜ-N(R¹⁰)-(CR⁹R^{9a})ₜ]ₖ-U-(CR⁹R^{9a})ₜ-N(R¹¹)-(CR⁹R^{9a})ₜ-R¹².

In other embodiments, each of Y and Y' is independently -U-(CR⁹R^{9a})ₜ-N(R¹⁰)-(CR⁹R^{9a})ₜ-U-(CR⁹R^{9a})ₜ-N(R¹¹)-(CR⁹R^{9a})ₜ-R¹².

In other embodiments, each of Y and Y' is independently -U-(CR⁹R^{9a})ₜ-N(R¹¹)-(CR⁹R^{9a})ₜ-R¹².

In other embodiments, each of Y and Y' is independently -[C(=O)-(CR⁹R^{9a})ₜ-N(R¹⁰)-(CR⁹R^{9a})ₜ]ₖ -U-(CR⁹R^{9a})ₜ-N(R¹¹)-(CR⁹R^{9a})ₜ-R¹².

In other embodiments, each of Y and Y' is independently -C(=O)-(CR⁹R^{9a})ₜ-N(R¹⁰)-(CR⁹R^{9a})ₜ-U-(CR⁹R^{9a})ₜ-N(R¹¹)-(CR⁹R^{9a})ₜ-R¹².

In other embodiments, each of Y and Y' is independently -[C(=O)-(CR⁹R^{9a})ₜ-N(R¹⁰)-(CR⁹R^{9a})ₜ]ₖ-C(=O)-(CR⁹R^{9a})ₜ-N(R¹¹)-(CR⁹R^{9a})ₜ-R¹².

In other embodiments, each of Y and Y' is independently -C(=O)-(CR⁹R^{9a})ₜ-N(R¹⁰)-(CR⁹R^{9a})ₜ-C(=O)-(CR⁹R^{9a})ₜ-N(R¹¹)-(CR⁹R^{9a})ₜ-R¹².

In other embodiments, each of Y and Y' is independently -C(=O)-(CR⁹R^{9a})ₜ-N(R¹¹)-(CR⁹R^{9a})ₜ-R¹².

In other embodiments, each of Y and Y' is independently -C(=O)-(CR⁹R^{9a})ₙ-N(R¹¹)-(CR⁹R^{9a})ₙ-C(=O)-R¹³.

In other embodiments, each of Y and Y' is independently -C(=O)-(CR⁹R^{9a})ₙ-N(R¹¹)-C(=O)-R¹³.

In other embodiments, each of Y and Y' is independently -C(=O)-(CR⁹R^{9a})ₙ-N(R¹¹)-(CR⁹R^{9a})ₙ-C(=O)-O-R¹³.

In other embodiments, each of Y and Y' is independently -C(=O)-(CR⁹R^{9a})ₙ-N(R¹¹)-C(=O)-O-R¹³.

In other embodiments, each of Y and Y' is independently -U-(CR⁹R^{9a})ₜ-R¹².

In other embodiments, each of Y and Y' is independently -C(=O)-(CR⁹R^{9a})ₜ-R¹².

In other embodiments, each of Y and Y' is independently -[U-(CR⁹R^{9a})ₜ-N(R¹⁰)-(CR⁹R^{9a})ₜ]ₖ-U-(CR⁹R^{9a})ₜ-O-(CR⁹R^{9a})ₜ-R¹².

In other embodiments, each of Y and Y' is independently -U-(CR⁹R^{9a})ₜ-N(R¹⁰)-(CR⁹R^{9a})ₜ-U-(CR⁹R^{9a})ₜ-O-(CR⁹R^{9a})ₜ-R¹².

In other embodiments, each of Y and Y' is independently -C(=O)-(CR⁹R^{9a})ₜ-N(R¹⁰)-(CR⁹R^{9a})ₜ-C(=O)-(CR⁹R^{9a})ₜ-O-(CR⁹R^{9a})ₜ-R¹².

In other embodiments, each of Y and Y' is independently -U-(CR⁹R^{9a})ₜ-O-(CR⁹R^{9a})ₜ-R¹².

In other embodiments, each of Y and Y' is independently -C(=O)-(CR⁹R^{9a})ₜ-O-(CR⁹R^{9a})ₜ-R¹².

In other embodiments, each of Y and Y' is independently -C(=O)-(CR⁹R^{9a})ₙ-N(R¹¹)-R¹², wherein R¹¹ and R¹², together with the atom they are attached to, form a 4-7 membered ring.

In other embodiments, each R⁹, R^{9a}, R¹⁰ and R¹¹ is independently H, deuterium, C₁₋₆ alkyl, C₂₋₆ heteroalkyl, C₃₋₁₀ cycloalkyl, C₂₋₁₀ heterocyclyl, C₆₋₁₀ aryl, C₁₋₉ heteroaryl, C₁₋₆ haloalkyl, C₁₋₆ hydroxyalkyl, C₆₋₁₀ aryl-C₁₋₆-alkyl, C₁₋₉ heteroaryl-C₁₋₆-alkyl, C₂₋₁₀ heterocyclyl-C₁₋₆-alkyl or C₃₋₈ cycloalkyl-C₁₋₆-alkyl;
each R¹² is independently R^{13a}R¹³N-, -C(=O)R¹³, -C(=S)R¹³, -C(=O)-O-R¹³, -C(=O)NR¹³R^{13a}, -OC(=O)NR¹³R^{13a}, -OC(=O)OR¹³, -N(R¹³)C(=O)NR¹³R^{13a}, -N(R¹³)C(=O)OR^{13a}, -N(R¹³)C(=O)-R^{13a}, R¹³R^{13a}N-S(=O)₂-, R¹³S(=O)₂-, R¹³S(=O)₂N(R^{13a})-, R¹³OS(=O)₂-, C₁₋₆ alkyl, C₂₋₆ heteroalkyl, C₃₋₁₀ cycloalkyl, C₂₋₁₀ heterocyclyl, C₆₋₁₀ aryl, C₁₋₉ heteroaryl or C₆₋₁₀ aryl-C₁₋₆-alkyl; or R¹¹ and R¹², together with the atom they are attached to, form a 4-7 membered ring;
each R¹³ and R^{13a} is independently H, deuterium, C₁₋₆ alkyl, C₂₋₆ heteroalkyl, C₃₋₁₀ cycloalkyl, C₂₋₁₀ heterocyclyl, C₆₋₁₀ aryl, C₁₋₉ heteroaryl or C₆₋₁₀ aryl-C₁₋₆-alkyl; with the proviso that where R¹³ and R^{13a} are bonded to the same nitrogen atom, R¹³ and R^{13a}, together with the nitrogen atom they are attached to, optionally form a substituted or unsubstituted 3-8 membered ring or a substituted or unsubstituted spiro or fused bicyclic ring;
each t is independently 0, 1, 2, 3 or 4; and
each k is independently 0, 1 or 2.

In other embodiments, each R⁹, R^{9a}, R¹⁰ and R¹¹ is independently H, deuterium, methyl, ethyl, isopropyl, cyclohexyl, isobutyl or phenyl;
each R¹² is independently -C(=O)R¹³, -C(=O)-O-R¹³, -C(=O)NR¹³R^{13a}, methyl, ethyl, propyl, phenyl, cyclohexyl, morpholinyl or piperidinyl;
or R¹¹ and R¹², together with the atom they are attached to, form a 4-7 membered ring; and
each R¹³ and R^{13a} is independently H, deuterium, methyl, ethyl, propyl, phenyl, cyclohexyl, morpholinyl or piperidinyl.

In other embodiments, the compound may have formula (VI): 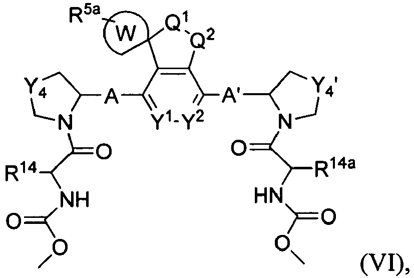
wherein each of R¹⁴ and R^{14a} is independently H, deuterium, C₁₋₆ alkyl, C₁₋₆ haloalkyl, C₁₋₆ hydroxyalkyl, C₂₋₆ heteroalkyl, C₆₋₁₀ aryl, C₁₋₉ heteroaryl, C₂₋₁₀ heterocyclyl, C₃₋₈ cycloalkyl, C₆₋₁₀ aryl-C₁₋₆-alkyl, C₁₋₉ heteroaryl-C₁₋₆-alkyl, C₂₋₁₀ heterocyclyl-C₁₋₆-alkyl, or C₃₋₈ cycloalkyl-C₁₋₆-alkyl; and
wherein each of C₁₋₆ alkyl, C₁₋₆ haloalkyl, C₁₋₆ hydroxyalkyl, C₂₋₆ heteroalkyl, C₆₋₁₀ aryl, C₁₋₉ heteroaryl, C₂₋₁₀ heterocyclyl, C₃₋₈ cycloalkyl, C₆₋₁₀ aryl-C₁₋₆-alkyl, C₁₋₉ heteroaryl-C₁₋₆-alkyl, C₂₋₁₀ heterocyclyl-C₁₋₆-alkyl and C₃₋₈ cycloalkyl-C₁₋₆-alkyl is optionally substituted with one or more substituents, wherein the substituent is deuterium, F, Cl, Br, hydroxy or cyano.

In other embodiments, the compound may have formula (VII): 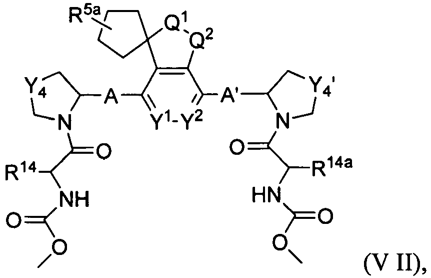
wherein each of R¹⁴ and R^{14a} is independently H, deuterium, C₁₋₃ hydroxyalkyl, methyl, ethyl, isopropyl, isobutyl, *tert*-butyl*,* allyl, propargyl, trifluoroethyl, phenyl, pyranyl, morpholinyl, -NR¹³R^{13a}, benzyl, piperazinyl, cyclopentyl, cyclopropyl, cyclohexyl or C₁₋₉ heteroaryl; and
wherein each of methyl, ethyl, isopropyl, isobutyl, *tert*-butyl, allyl, propargyl, trifluoroethyl, phenyl, pyranyl, morpholinyl, benzyl, piperazinyl, cyclopentyl, cyclopropyl and cyclohexyl is optionally substituted with one or more substituents, wherein the substituent is deuterium, F, Cl, Br, hydroxy or cyano.

In some embodiments, the compound may have formula (VIII): 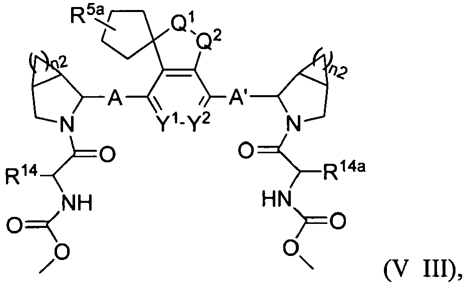
wherein each of R¹⁴ and R^{14a} is independently H, deuterium, C₁₋₆ alkyl, C₁₋₆ haloalkyl, C₁₋₆ hydroxyalkyl, C₂₋₆ heteroalkyl, C₆₋₁₀ aryl, C₁₋₉ heteroaryl, C₂₋₁₀ heterocyclyl, C₃₋₈ cycloalkyl, C₆₋₁₀ aryl-C₁₋₆-alkyl, C₁₋₉ heteroaryl-C₁₋₆-alkyl, C₂₋₁₀ heterocyclyl-C₁₋₆-alkyl or C₃₋₈ cycloalkyl-C₁₋₆-alkyl;
wherein each of C₁₋₆ alkyl, C₁₋₆ haloalkyl, C₁₋₆ hydroxyalkyl, C₂₋₆ heteroalkyl, C₆₋₁₀ aryl, C₁₋₉ heteroaryl, C₂₋₁₀ heterocyclyl, C₃₋₈ cycloalkyl, C₆₋₁₀ aryl-C₁₋₆-alkyl, C₁₋₉ heteroaryl-C₁₋₆₋alkyl, C₂₋₁₀ heterocyclyl-C₁₋₆-alkyl and C₃₋₈ cycloalkyl-C₁₋₆-alkyl is optionally substituted with one or more substituents, wherein the substituent is deuterium, F, Cl, Br, hydroxy or cyano; and
each n₂ is independently 1, 2, 3 or 4.

In some embodiments, the compound may have formula (IX): 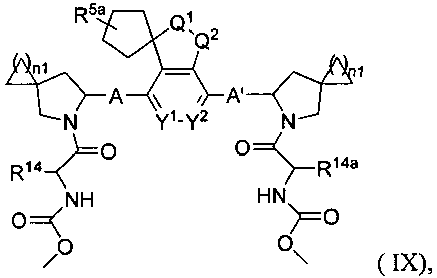
wherein each of R¹⁴ and R^{14a} is independently H, deuterium, C₁₋₆ alkyl, C₁₋₆ haloalkyl, C₁₋₆ hydroxyalkyl, C₂₋₆ heteroalkyl, C₆₋₁₀ aryl, C₁₋₉ heteroaryl, C₂₋₁₀ heterocyclyl, C₃₋₈ cycloalkyl, C₆₋₁₀ aryl-C₁₋₆-alkyl, C₁₋₉ heteroaryl-C₁₋₆-alkyl, C₂₋₁₀ heterocyclyl-C₁₋₆-alkyl or C₃₋₈ cycloalkyl-C₁₋₆-alkyl;
wherein each of ₁₋₆ alkyl, C₁₋₆ haloalkyl, C₁₋₆ hydroxyalkyl, C₂₋₆ heteroalkyl, C₆₋₁₀ aryl, C₁₋₉ heteroaryl, C₂₋₁₀ heterocyclyl, C₃₋₈ cycloalkyl, C₆₋₁₀ aryl-C₁₋₆-alkyl, C₁₋₉ heteroaryl-C₁₋₆-alkyl, C₂₋₁₀ heterocyclyl-C₁₋₆-alkyl and C₃₋₈ cycloalkyl-C₁₋₆-alkyl is optionally substituted with one or more substituents, wherein the substituent is deuterium, F, Cl, Br, hydroxy or cyano; and
each n₁ is independently 1, 2, 3 or 4.

In some embodiments, the compound may have formula (X): 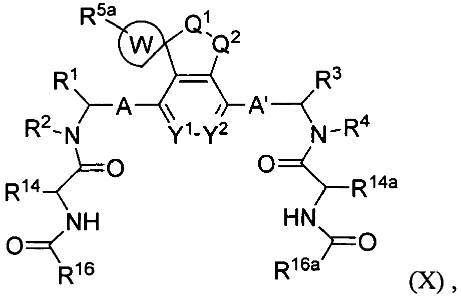
wherein R^{5a} is H, deuterium, methyl, ethyl, F, Cl, Br or I;
Q¹ is CH₂, C(=O), O, S or NR⁶;
Q² is CH₂, C(=O), CF₂, O, NR⁶ or S;
each of Y¹ and Y² is independently N or CR⁷;
each R⁶ and R⁷ is independently H, deuterium, methyl, ethyl, isopropyl, phenyl or cyclohexyl;
each of R¹⁴ and R^{14a} is independently H, deuterium, methyl, ethyl, phenyl, cyclohexyl, 1-methylpropyl, isopropyl or *tert*-butyl;
each of R¹⁶ and R^{16a} is independently hydroxy, methoxy, ethoxy, phenoxy, 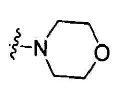 or *tert*-butoxy;
wherein each of methyl, ethyl, phenyl, cyclohexyl, 1-methylpropyl, isopropyl, methoxy, ethoxy, *tert*-butoxy and *tert*-butyl is optionally substituted with one or more substituents, wherein the substituent is deuterium, F, Cl, Br, hydroxy or cyano;
wherein 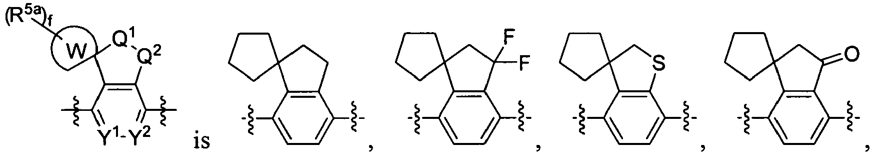 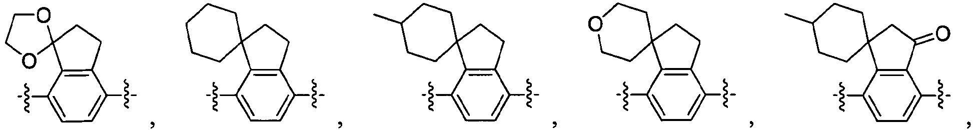 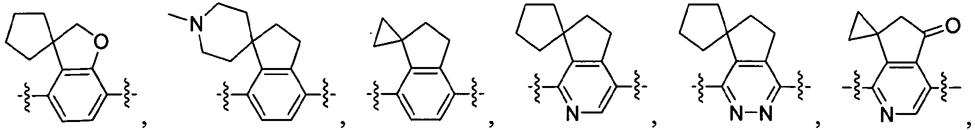 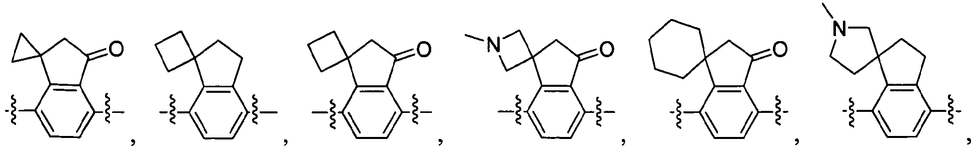 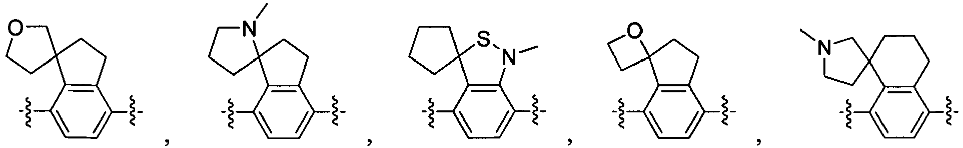 or 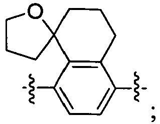
each of A and A' is independently 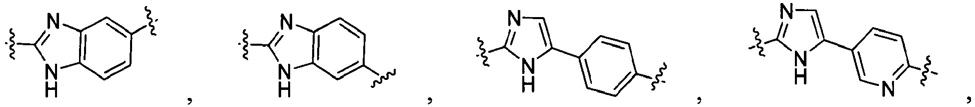 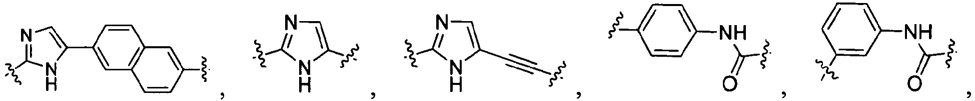 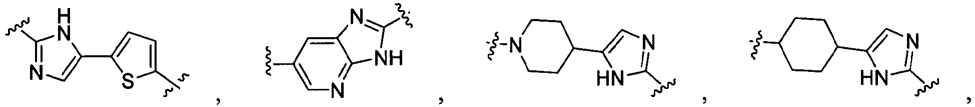 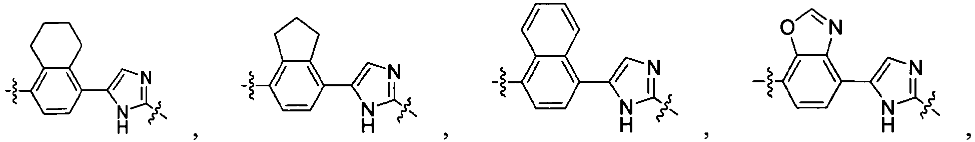 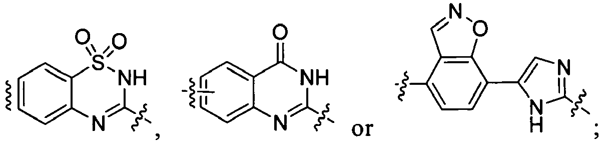
wherein R¹, R² and N-CH together form one of the following divalent groups: 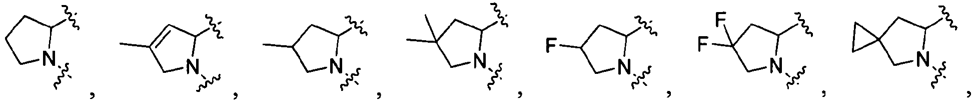 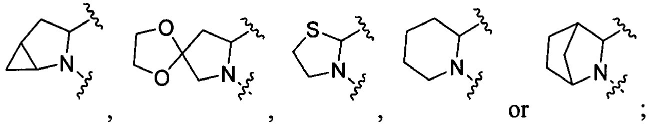 and
wherein R³, R⁴ and N-CH together form one of the following divalent groups:

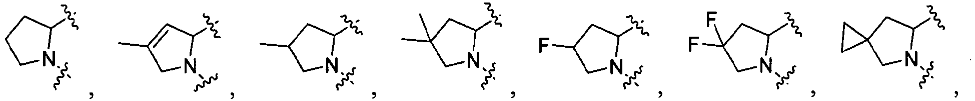

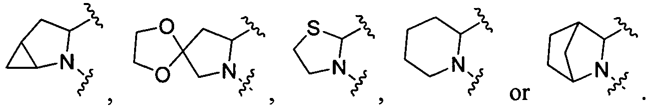

In other embodiments, the compound may have formula (XI): 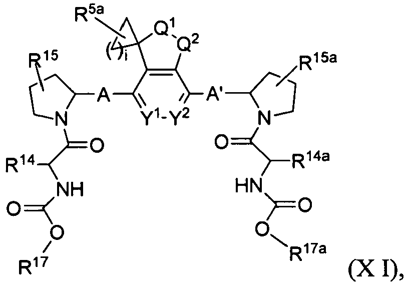
wherein i is 1, 2, 3 or 4;
R^{5a} is H, deuterium or methyl;
each of Q¹ and Q² is independently (CH₂)ₑ, CF₂, S, NR⁶, O or C(=O);
e is 0, 1, 2, 3 or 4;
R⁶ is H, deuterium, methyl, ethyl, isobutyl, cyclohexyl, phenyl or isopropyl;
each of R¹⁴ and R^{14a} is independently H, deuterium, methyl, ethyl, isobutyl, cyclohexyl, phenyl or isopropyl;
each of R¹⁵ and R^{15a} is independently H, deuterium, F, Cl, Br, methyl, ethyl, isopropyl or *tert*-butyl; and
each of R¹⁷ and R^{17a} is independently methyl, phenyl or ethyl;
wherein each of methyl, ethyl, phenyl, cyclohexyl, isopropyl, isobutyl and *tert*-butyl is optionally substituted with one or more substituents, wherein the substituent is deuterium, F, Cl, Br, hydroxy or cyano.

In some embodiments, the compound may have formula (XI'): 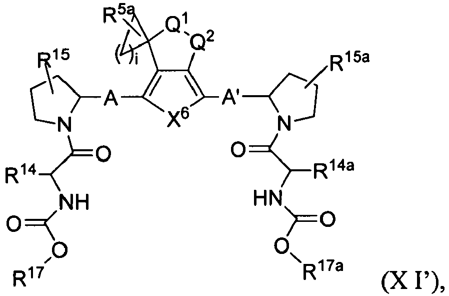
wherein i is 1, 2, 3 or 4;
R^{5a} is H, deuterium or methyl;
each of Q¹ and Q² is independently (CH₂)ₑ, CF₂, S, NR⁶, O or C(=O);
e is 0, 1, 2, 3 or 4;
each R⁶ is independently H, deuterium, methyl, ethyl, isobutyl, cyclohexyl, phenyl or isopropyl;
each of R¹⁴ and R^{14a} is independently H, deuterium, methyl, ethyl, isobutyl, cyclohexyl, phenyl or isopropyl;
each of R¹⁵ and R^{15a} is independently H, deuterium, F, Cl, Br, methyl, ethyl, isopropyl or *tert-*butyl;
each of R¹⁷ and R^{17a} is independently methyl, phenyl or ethyl;
wherein each of methyl, ethyl, phenyl, cyclohexyl, isopropyl, isobutyl and *tert*-butyl is optionally substituted with one or more substituents, wherein the substituent is deuterium, F, Cl, Br, hydroxy or cyano;
X⁶ is independently CH₂, O, S or NR⁶; and
each of A and A' is independently

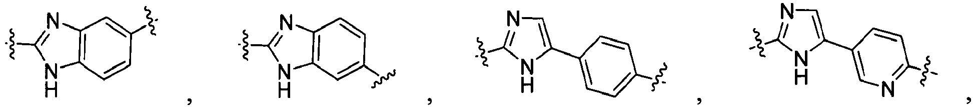

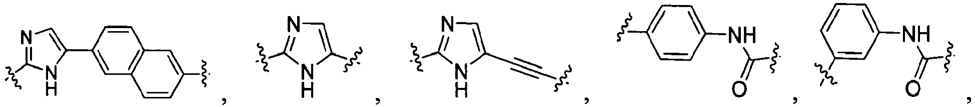

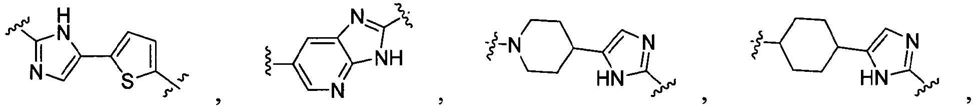

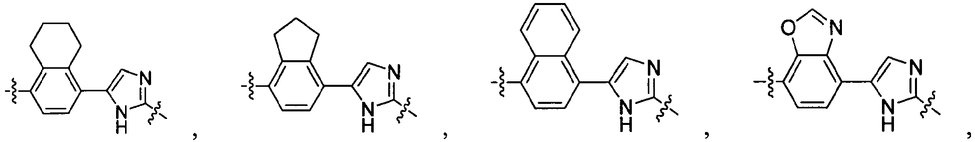

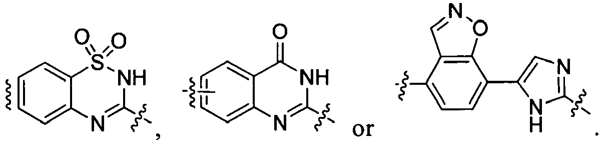

In another aspect, the present disclosure provides a pharmaceutical composition comprising any one of the above compounds.

In some embodiments, the pharmaceutical composition also comprises a pharmaceutically acceptable carrier, excipient, diluent, adjuvant, vehicle or a combination thereof.

In certain embodiments, the pharmaceutical composition disclosed herein further comprises an anti-HCV agent.

In other embodiments, the anti-HCV agent is interferon, ribavirin, IL-2, IL-6, IL-12, a compound that enhances the development of a type 1 helper T cell response, interfering RNA, anti-sense RNA, imiquimod, an inosine5'-monophosphate dehydrogenase inhibitor, amantadine, rimantadine, ribavirin, bavituximab, human hepatitis C immune globulin (CIVACIR™), boceprevir, telaprevir, erlotinib, daclatasvir, simeprevir, asunaprevir, vaniprevir, faldaprevir, ABT-450, danoprevir, sovaprevir, MK-5172, vedroprevir, BZF-961, GS-9256, narlaprevir, ANA975, ABT-267, EDP239, PPI-668, GS-5816, samatasvir (IDX-719), MK-8742, MK-8325, GSK-2336805, PPI-461, TMC-435, MK-7009, BI-2013335, ciluprevir, BMS-650032, ACH-1625, ACH-1095, VX-985, IDX-375, VX-500, VX-813, PHX-1766, PHX-2054, IDX-136, IDX-316, EP-013420, VBY-376, TMC-649128, R-7128, PSI-7977, INX-189, IDX-184, IDX102, R1479, UNX-08189, PSI-6130, PSI-938, PSI-879, HCV-796, HCV-371, VCH-916, VCH-222, ANA-598, MK-3281, ABT-333, ABT-072, PF-00868554, BI-207127, GS-9190, A-837093, JKT-109, Gl-59728, GL-60667, AZD-2795, TMC647055 or a combination thereof.

In other embodiments, the interferon is interferon α-2b, pegylated interferon α, interferon α-2a, pegylated interferon α-2a, consensus interferon-α, interferon γ or a combination thereof.

In other embodiments, the pharmaceutical composition disclosed herein further comprises at least one HCV inhibitor.

In some embodiments, the HCV inhibitor inhibits at least one of HCV replication process and HCV viral protein function.

In some embodiments, the HCV replication process is a whole viral cycle consisting of HCV entry, uncoating, translation, replication, assembly and egress.

In some embodiments, the HCV viral protein is helicase, proteinase, metalloproteinase, serine proteinase, non-structural protein NS4A, non-structural protein NS4B, RNA polymerase of non-structural protein NS5A or non-structural protein NS5B, or an internal ribosome entry site (IRES) or inosine-5'-monophosphate dehydrogenase (IMPDH) required in HCV viral replication.

In another aspect, use of the compound or the pharmaceutical composition in inhibiting at least one of HCV replication process and HCV viral protein function is provided.

In some embodiments, the HCV replication process is a whole viral cycle consisting of HCV entry, uncoating, translation, replication, assembly and egress.

In some embodiments, the HCV viral protein is helicase, proteinase, metalloproteinase, serine proteinase, non-structural protein NS4A, non-structural protein NS4B, RNA polymerase of non-structural protein NS5A or non-structural protein NS5B, or an internal ribosome entry site (IRES) or inosine-5'-monophosphate dehydrogenase (IMPDH) required in HCV viral replication.

In another aspect, use of the compound or the pharmaceutical composition disclosed herein for preventing, managing, treating or lessening the severity of HCV infection and a HCV disorder in a patient is provided, which comprises administering a therapeutically effective amount of the (a) compound or pharmaceutical composition disclosed herein to the patient.

In another aspect, the compound or the pharmaceutical composition disclosed herein for use in inhibiting at least one of HCV replication process and HCV viral protein function is provided. In some embodiments, the HCV replication process is a whole viral cycle consisting of HCV entry, uncoating, translation, replication, assembly and egress; In some embodiments, the HCV viral protein is helicase, proteinase, metalloproteinase, serine proteinase, non-structural protein NS4A, non-structural protein NS4B, RNA polymerase of non-structural protein NS5A or non-structural protein NS5B, or an internal ribosome entry site (IRES) or inosine-5'-monophosphate dehydrogenase (IMPDH) required in HCV viral replication.

In another aspect, the compound or the pharmaceutical composition disclosed herein for use in preventing, managing, treating or lessening the severity of HCV infection or a HCV disorder in a patient is provided.

In another aspect, provided herein include methods of preparing, methods of separating, and methods of purifying compounds of formula (I), (II), (III), (IV), (V), (VI), (VII), (VIII), (IX), (X), (XI), (II'), (III'), (IV'), (V') or (XI').

The foregoing merely summarizes certain aspects disclosed herein and is not intended to be limiting in nature. These aspects and other aspects and embodiments are described more fully below.

### DETAILED DESCRIPTION OF THE INVENTION

### DEFINITIONS AND GENERAL TERMINOLOGY

Reference will now be made in detail to certain embodiments disclosed herein, examples of which are illustrated in the accompanying structures and formulas. The invention is intended to cover all alternatives, modifications, and equivalents that may be included within the scope disclosed herein as defined by the claims. One skilled in the art will recognize many methods and materials similar or equivalent to those described herein, which could be used in the practice disclosed herein. Described herein is in no way limited to the methods and materials. In the event that one or more of the incorporated literature, patents, and similar materials differ from or contradict this application, including but not limited to defined terms, term usage, described techniques, or the like, this application controls.

As used herein, the following definitions shall be applied unless otherwise indicated. For purposes disclosed herein, the chemical elements are identified in accordance with the Periodic Table of the Elements, CAS version, and *the* Handbook of Chemistry and Physics, 75th Ed. 1994**.** Additionally, general principles of organic chemistry are described in Sorrell et al., "Organic Chemistry", University Science Books, Sausalito: 1999**,** and Smith et al., "March's Advanced Organic Chemistry", John Wiley & Sons, New York: 2007**.**

As described herein, compounds may optionally be substituted with one or more substituents, such as those illustrated above, or as exemplified by particular classes, subclasses, and species disclosed herein. It will be appreciated that the phrase "optionally substituted" is used interchangeably with the phrase "substituted or unsubstituted". In general, the term "substituted" whether preceded by the term "optionally" or not, refers to the replacement of one or more hydrogen radicals in a given structure with the radical of a specified substituent. Unless otherwise indicated, an optionally substituted group may have a substituent at each substitutable position of the group. When more than one position in a given structure can be substituted with more than one substituent selected from a specified group, the substituent may be either the same or different at each position. Wherein the substituents described herein include, but are not limited to, deuterium, hydroxy, amino, halo, cyano, aryl, heteroaryl, alkoxy, alkylamino, alkylthio, alkyl, alkenyl, alkynyl, heterocyclyl, mercapto, nitro, aryloxy, heteroaryloxy, oxo (=O), carboxy, hydroxy-substituted alkoxy, hydroxy-substituted alkyl-C(=O)-, alkyl-C(=O)-, alkyl-S(=O)-, alkyl-S(=O)₂-, hydroxy-substituted alkyl-S(=O)-, hydroxy-substituted alkyl-S(=O)₂-, carboxyalkoxy, and the like.

The term "aliphatic" or "aliphatic group" refers to a straight-chain (i.e., unbranched) or branched, substituted or unsubstituted hydrocarbon chain that is completely saturated or that contains one or more units of unsaturation. Unless otherwise specified, aliphatic groups contain 1-20 carbon atoms. In some embodiments, aliphatic groups contain 1-10 carbon atoms. In other embodiments, aliphatic groups contain 1-8 carbon atoms. In still other embodiments, aliphatic groups contain 1-6 carbon atoms, and in yet other embodiments, aliphatic groups contain 1-4 carbon atoms. In other embodiments, aliphatic groups contain 1-3 carbon atoms. Some non-limiting examples of aliphatic groups include linear or branched, substituted or unsubstituted alkyl, alkenyl, or alkynyl groups, as methyl, ethyl, propyl, isopropyl, butyl, *tert*-butyl, hexyl, isobutyl, *sec*-butyl, vinyl, and the like.

The term "haloaliphatic" refers to an aliphatic group substituted with one or more of the same or different halogen atoms (i.e., F, Cl, Br or I,), wherein the aliphatic group is as defined herein. Some non-limiting examples include trifluoromethyl, trifluoroethyl, chloromethyl, 2-chlorovinyl, and the like.

The term "hydroxyaliphatic" refers to an aliphatic group substituted with one or more hydroxy groups, wherein the aliphatic group is as defined herein. Some non-limiting examples include hydroxyethyl, 2-hydroxypropyl, hydroxymethyl, and the like.

The term "aminoaliphatic" refers to an aliphatic group substituted with one or more amino groups, wherein the aliphatic group is as defined herein. Some non-limiting examples include aminomethyl, 2-aminoethyl, 2-amino isopropyl, and the like.

The term "alkyl" refers to a saturated linear or branched chain monovalent hydrocarbon radical of one to twenty carbon atoms, or one to ten carbon atoms, or one to eight carbon atoms, or one to six carbon atoms, or one to four carbon atoms, or one to three carbon atoms, wherein the alkyl radical may be optionally substituted independently with one or more substituents described herein. The examples of alkyl groups include, but are not limited to, methyl (Me, -CH₃), ethyl (Et, -CH₂CH₃), 1-propyl (*n*-Pr, *n*-propyl, -CH₂CH₂CH₃), 2-propyl (*i*-Pr, *i*-propyl, -CH(CH₃)₂), 1-butyl (*n*-Bu, *n*-butyl, -CH₂CH₂CH₂CH₃), 2-methyl-propyl (*i*-Bu, *i*-butyl, -CH₂CH(CH₃)₂), 2-butyl (*s*-Bu, *s*-butyl, -CH(CH₃)CH₂CH₃), 2-methyl-2-propyl (*t*-Bu, *t*-butyl, -C(CH₃)₃), 1-pentyl (*n*-pentyl, -CH₂CH₂CH₂CH₂CH₃), 2-pentyl (-CH(CH₃)CH₂CH₂CH₃), 3-pentyl (-CH(CH₂CH₃)₂), 2-methyl-2-butyl (-C(CH₃)₂CH₂CH₃), 3-methyl-2-butyl (-CH(CH₃)CH(CH₃)₂), 3-methyl-1-butyl (-CH₂CH₂CH(CH₃)₂), 2-methyl-1-butyl (-CH₂CH(CH₃)CH₂CH₃), 1-hexyl (-CH₂CH₂CH₂CH₂CH₂CH₃), 2-hexyl (-CH(CH₃)CH₂CH₂CH₂CH₃), 3-hexyl (-CH(CH₂CH₃)(CH₂CH₂CH₃)), 2-methyl-2-pentyl (-C(CH₃)₂CH₂CH₂CH₃), 3-methyl-2-pentyl (-CH(CH₃)CH(CH₃)CH₂CH₃), 4-methyl-2-pentyl (-CH(CH₃)CH₂CH(CH₃)₂), 3-methyl-3-pentyl (-C(CH₃)(CH₂CH₃)₂), 2-methyl-3-pentyl (-CH(CH₂CH₃)CH(CH₃)₂), 2,3-dimethyl-2-butyl (-C(CH₃)₂CH(CH₃)₂), 3,3-dimethyl-2-butyl (-CH(CH₃)C(CH₃)₃, 1-heptyl, 1-octyl, and the like. The terms "alkyl" and the prefix "alk-" are inclusive of both straight chain and branched saturated carbon chain. The term "alkylene", as used herein, represents a saturated divalent hydrocarbon group derived from a straight or branched chain saturated hydrocarbon by the removal of two hydrogen atoms, and is exemplified by methylene, ethylene, isopropylene, and the like.

The term "alkenyl" refers to a linear or branched chain monovalent hydrocarbon radical of two to twelve carbon atoms, or two to eight carbon atoms, or two to six carbon atoms, or two to four carbon atoms, with at least one site of unsaturation, i.e., a carbon-carbon, sp² double bond, wherein the alkenyl radical may be optionally substituted independently with one or more substituents described herein, and includes radicals having "cis" and "trans" orientations, or alternatively, *"E"* and *"Z"* orientations. Some non-limiting examples include ethenyl or vinyl (-CH=CH₂), allyl (-CH₂CH=CH₂), and the like.

The term "alkynyl" refers to a linear or branched chain monovalent hydrocarbon radical of two to twelve carbon atoms, or two to eight carbon atoms, or two to six carbon atoms, or two to four carbon atoms, with at least one site of unsaturation, i.e., a carbon-carbon, sp triple bond, wherein the alkynyl radical may be optionally substituted independently with one or more substituents described herein. Some non-limiting examples include ethynyl (-C≡CH), 2-propynyl or propargyl (-CH₂C≡CH), and the like.

The term "hydroxy-substituted alkyl" refers to an alkyl group substituted with one or more hydroxy groups, wherein the alkyl group is as defined herein. Some non-limiting examples include hydroxymethyl, hydroxyethyl, 1,2-dihydroxyethyl, and the like.

The term "haloalkyl" refers to an alkyl group substituted with one or more of the same or different halogen atoms (i.e., F, Cl, Br or I,), wherein the alkyl group is as defined herein. Some non-limiting examples include trifluoromethyl, trifluoroethyl, chloromethyl, fluoromethyl, and the like.

The term "hydroxyalkyl" refers to an alkyl group substituted with one or more hydroxy groups, wherein the alkyl group is as defined herein. Some non-limiting examples include hydroxyethyl, 2-hydroxypropyl, hydroxymethyl, and the like.

The term "aminoalkyl" refers to an alkyl group substituted with one or more amino groups, wherein the alkyl group is as defined herein. Some non-limiting examples include aminomethyl, 2-aminoethyl, 2-amino isopropyl, and the like.

The term "alkylene" refers to a saturated divalent hydrocarbon group derived from a straight or branched chain saturated hydrocarbon by the removal of two hydrogen atoms. The alkylene group is optionally substituted with one or more substituents. The substituents include, but are not limited to, deuterium, hydroxy, amino, halo, cyano, aryl, heteroaryl, alkoxy, alkyl, alkenyl, alkynyl, heterocyclyl, mercapto, nitro, or aryloxy. Some non-limiting examples include methylene (-CH₂-), ethylene (-CH₂-CH₂-), isopropylene (-CH₂-CH(CH₃)-), ethylidene, 2-methoxy-1,1-propylidene, 2-hydroxy-1,1-propylidene, 2-methyl-2-hydroxy-1,1-propylidene, and the like.

The term "alkenylene" refers to an unsaturated divalent hydrocarbon group derived from a straight or branched chain unsaturated hydrocarbon alkene by the removal of two hydrogen atoms. The alkenylene group is optionally substituted with one or more substituents. The substituents include, but are not limited to, deuterium, hydroxy, amino, halo, cyano, aryl, heteroaryl, alkoxy, alkyl, alkenyl, alkynyl, heterocyclyl, mercapto, nitro, or aryloxy. Some non-limiting examples include ethenylene (-CH=CH-), isopropenylene (-C(CH₃)=CH-), 3-methoxy-1,1-propenylidene, 2-methyl-1,1-butenylidene, and the like.

The term "carbocyclylene" or "cycloalkylene" refers to a saturated divalent hydrocarbon ring derived from a monocyclic ring having 3 to 12 carbon atoms or a bicyclic ring having 7 to 12 carbon atoms by the removal of two hydrogen atoms, wherein the carbocyclyl group or the cycloalkyl group is as defined herein. Some non-limiting examples include cyclopropylene, cyclobutylene, cyclopentylene, 1-cyclopent-1-enylene, 1-cyclopent-2-enylene, and the like.

The term "heterocyclylene" refers to a monocyclic, bicyclic, or tricyclic ring system in which one or more ring members are an independently selected heteroatom and that is completely saturated or that contains one or more units of unsaturation, but which is not aromatic, that has two points of attachment to the rest of the molecule, wherein the heterocyclyl group is as defined herein. Some non-limiting examples include piperidin-1,4-ylene, piperazin-1,4-ylene, tetrahydrofuran-2,4-ylene, tetrahydrofuran-3,4-ylene, azetidin-1,3-ylene, pyrrolidin-1,3-ylene, and the like.

The term "heterocyclylalkylene" refers to a divalent group derived from a heterocyclylalkyl by the removal of two hydrogen atoms, wherein the heterocyclylalkyl group is as defined herein. Some non-limiting examples include morpholin-4-methylmethylene, piperidin-*N*-methylmethylene, piperazin-4-ethyl-1-yl, piperidin-4-methyl-1-yl, piperidin-4-ethyl-1-yl, pyrrolidon-2-methyl-1-yl, and the like.

The term "haloalkylene" refers to haloalkyl system having two points connected to the rest of the molecule, wherein the haloalkyl group is as defined herein. Some non-limiting examples include difluoromethylene (-CF₂-).

The term "arylene" refers to aryl system having two connection points connected to the rest of the molecule, wherein the aryl radical is as defined herein. Some non-limiting examples include phenylene, *p*-fluorophenylene, and the like.

The term "aralkylene" refers to aralkyl system having two connection points connected to the rest of the molecule, wherein the aralkyl radical is as defined herein. Some non-limiting examples include benzylene, phenylethylene, and the like.

The term "heteroarylene" refers to heteroaryl system having two connection points connected to the rest of the molecule, wherein the heteroaryl radical is as defined herein. Some non-limiting examples include pyridylene, pyrrylene, thiazolylene, imidazolylene, and the like.

The term "heteroarylalkylene" refers to heteroarylalkyl system having two connection points connected to the rest of the molecule, wherein the heteroarylalkyl group is as defined herein. Some non-limiting examples include pyridin-2-ethylene, thiazol-2-methylene, imidazol-2-ethylene, pyrimidin-2-methylene, and the like.

The term "fused bicyclylene" refers to fused bicyclyl system having two connection points connected to the rest of the molecule, wherein the fused bicyclyl group is as defined herein. Some non-limiting examples include bicyclo[3.1.0]hexane-3,6-ylene.

The term "fused heterobicyclylene" refers to fused heterobicyclyl system having two connection points connected to the rest of the molecule, wherein the fused heterobicyclyl group is as defined herein. Some non-limiting examples include 3-azabicyclo[3.1.0]hexane-3,6-ylene.

The term "fused bicyclylalkylene" refers to fused bicyclylalkyl system having two connection points connected to the rest of the molecule, wherein the fused bicyclylalkyl group is as defined herein.

The term "fused heterobicyclylalkylene" refers to fused heterobicyclylalkyl system having two connection points connected to the rest of the molecule, wherein the fused heterobicyclylalkyl group is as defined herein.

The term "spiro bicyclylene" refers to spiro bicyclyl system having two connection points connected to the rest of the molecule, wherein the fused spiro bicyclyl group is as defined herein. Some non-limiting examples include 5-spiro[2,4]heptane-5,7-ylene, spiro[4,4]nonane-2,7-ylene, and the like.

The term "spiro heterobicyclylene" refers to spiro heterobicyclyl system having two connection points connected to the rest of the molecule, wherein the fused spiro heterobicyclyl group is as defined herein. Some non-limiting examples include 5-azaspiro[2,4]heptane-5,7-ylene, 2-azaspiro[4,4]nonane-2,7-ylene, and the like.

The term "spiro bicyclylalkylene" refers to spiro bicyclylalkyl system having two connection points connected to the rest of the molecule, wherein the fused spiro bicyclylalkyl group is as defined herein.

The term "spiro heterobicyclylalkylene" refers to spiro heterobicyclylalkyl system having two connection points connected to the rest of the molecule, wherein the fused spiro heterobicyclylalkyl group is as defined herein.

The term "heteroalkyl" refers to alkyl chain inserted with one or more heteroatoms, wherein the alkyl and heteroatom are as defined herein. Unless otherwise specified, heteroalkyl groups contain 1-10 carbon atoms. In other embodiments, heteroalkyl groups contain 1-8 carbon atoms. In still other embodiments, heteroalkyl groups contain 1-6 carbon atoms, and in yet other embodiments, heteroalkyl groups contain 1-4 carbon atoms. In other embodiments, heteroalkyl groups contain 1-3 carbon atoms. Some non-limiting examples include CH₃OCH₂-, CH₃CH₂OCH₂-, CH₃SCH₂-, (CH₃)₂NCH₂-, (CH₃)₂CH₂OCH₂-, CH₃OCH₂CH₂-, CH₃CH₂OCH₂CH₂-, and the like.

The term "cycloaliphatic", "carbocycle", "carbocyclyl", or "cycloalkyl" refers to a monovalent or multivalent non-aromatic, saturated or partially unsaturated ring exclusive of heteroatoms, having 3 to 12 carbon atoms as a monocyclic ring or 7 to 12 carbon atoms as a bicyclic ring. Bicyclic carbocycles having 7 to 12 atoms can be arranged, for example, as a bicyclo [4,5], [5,5], [5,6] or [6,6] system, and bicyclic carbocycles having 9 or 10 ring atoms can be arranged as a bicyclo [5,6] or [6,6] system. Some non-limiting examples of cycloaliphatic groups include cycloalkyl, cycloalkenyl, and cycloalkynyl. Further examples of cycloaliphatic groups include cyclopropyl, cyclobutyl, cyclopentyl, 1-cyclopentyl-1-enyl, 1-cyclopentyl-2-enyl, 1-cyclopentyl-3-enyl, cyclohexyl, 1-cyclohexyl-1-enyl, 1-cyclohexyl-2-enyl, 1-cyclohexyl-3-enyl, cyclohexadienyl, cycloheptyl, cyclooctyl, cyclononyl, cyclodecyl, cycloundecyl, cyclododecyl, and the like. The term "cycloaliphatic", "carbocycle", "carbocyclyl", or "cycloalkyl" may be substituted or unsubstituted, wherein the substituent may be, but is not limited to, deuterium, hydroxy, amino, halo, cyano, aryl, heteroaryl, alkoxy, alkylamino, alkyl, alkenyl, alkynyl, heterocyclyl, mercapto, nitro, aryloxy, hydroxy-substituted alkoxy, hydroxy-substituted alkyl-C(=O)-, alkyl-C(=O)-, alkyl-S(=O)-, alkyl-S(=O)₂-, hydroxy-substituted alkyl-S(=O)-, hydroxy-substituted alkyl-S(=O)₂-, carboxyalkoxy, and the like.

The term "cycloalkyloxy" or "carbocyclyloxy" refers to an optionally substituted cycloalkyl or carbocyclyl radical, as defined herein, attached to an oxygen atom, wherein the oxygen atom serves as the attaching point to the rest of the molecule. Some non-limiting examples include cyclopropyloxy, cyclopentyloxy, cyclohexyloxy, hydroxy-substituted cyclopropyloxy, and the like.

The term "cycloalkylamino" refers to an amino group substituted with one or two optionally substituted cycloalkyl radicals, wherein the cycloalkyl group is as defined herein. Some non-limiting examples include cyclopropylamino, cyclopentylamino, cyclohexylamino, hydroxy-substituted cyclopropylamino, dicyclohexylamino, dicyclopropylamino, and the like.

The term "carbocyclyloxyalkoxy" refers to an alkoxy group substituted with one or more carbocyclyloxy groups, wherein the alkoxy group and carbocyclyloxy group are as defined herein. Some non-limiting examples include cyclopropyloxymethoxy, cyclopropyloxyethoxy, cyclopentyloxyethoxy, cyclohexyloxyethoxy, cyclohexenyl-3-oxyethoxy, and the like.

The term "cycloalkyloxyaliphatic" refers to an aliphatic group substituted with one or more optionally substituted cycloalkyloxy groups, wherein the aliphatic group and cycloalkyloxy group are as defined herein. Some non-limiting examples include cyclopropyloxymethyl, cyclopropyloxyethyl, cyclopentyloxymethyl, cyclopentyloxyethyl, cyclohexyloxyethyl, halocyclopropyloxyethyl, and the like.

The term "cycloalkylaminoaliphatic" refers to an aliphatic group substituted with one or more optionally substituted cycloalkylamino groups, wherein the aliphatic group and cycloalkylamino group are as defined herein. Some non-limiting examples include cyclopropylaminomethyl, cyclopropylaminoethyl, cyclopentylaminomethyl, cyclopentylaminoethyl, cyclohexylaminoethyl, halocyclopropylaminoethyl, and the like.

The term "cycloalkylaliphatic" refers to an aliphatic group substituted with one or more cycloalkyl groups, wherein the cycloalkyl group and aliphatic group are as defined herein. Some non-limiting examples include cyclopropylmethyl, cyclopropylethyl, cyclopropylpropyl, cyclopentylmethyl, cyclohexylethyl, and the like.

The term "cycloalkylalkoxy" ("carbocyclylalkoxy") refers to an alkoxy group substituted with one or more cycloalkyl (carbocyclyl) groups, wherein the cycloalkyl (carbocyclyl) group and alkoxy group are as defined herein. Some non-limiting examples include cyclopropylmethoxy, cyclopropylethoxy, cyclopentylethoxy, cyclohexylethoxy, cyclohexylmethoxy, cyclopropylpropoxy, and the like.

The term "heterocycle", "heterocyclyl", "heterocycloaliphatic", or "heterocyclic" as used interchangeably herein refers to a monocyclic, bicyclic, or tricyclic ring system in which one or more ring members are an independently selected heteroatom and that is completely saturated or that contains one or more units of unsaturation, but not aromatic having a single point of attachment to the rest of the molecule. One or more ring atoms are optionally substituted independently with one or more substituents described herein. In some embodiments, the "heterocycle", "heterocyclyl", "heterocycloaliphatic" or "heterocyclic" group is a monocycle having 3 to 7 ring members (e.g., 1 to 6 carbon atoms and 1 to 3 heteroatoms selected from N, O, P or S, wherein the S or P is optionally substituted with one or more oxo to provide the group SO or SO₂ PO or PO₂, with the proviso that when the ring is a 3-membered ring, there is only one heteroatom) or a bicycle having 7 to 10 ring members (e.g., 4 to 9 carbon atoms and 1 to 3 heteroatoms selected from N, O, P or S, wherein the S or P is optionally substituted with one or more oxo to provide the group SO or SO₂, PO or PO₂).

The heterocyclyl may be a carbon radical or heteroatom radical. "Heterocyclyl" also includes radicals where heterocycle radicals are fused with a saturated, partially unsaturated ring, or heterocyclic ring. Some non-limiting examples of heterocyclic rings include pyrrolidinyl, tetrahydrofuranyl, dihydrofuranyl, tetrahydrothienyl, tetrahydropyranyl, dihydropyranyl, tetrahydrothiopyranyl, piperidyl, morpholinyl, thiomorpholinyl, thioxanyl, thiazolidinyl, oxazolidinyl, piperazinyl, homopiperazinyl, azetidinyl, oxetanyl, thietanyl, homopiperidinyl, epoxypropyl (oxiranyl), azepanyl, oxepanyl, thiepanyl, 4-methoxy-piperidin-1-yl, 1,2,3,6-tetrahydropyridine-1-yl, oxazepinyl, diazepinyl, thiazepinyl, pyrrolidin-1-yl, 2-pyrrolinyl, 3-pyrrolinyl, 2H-indolinyl, 2H-pyranyl, 4H-pyranyl, dioxolan-2-yl, 1,3-dioxopenyl, pyrazolinyl, dithianyl, ditholanyl, dihydrothienyl, pyrazolidinylimidazolinyl, imidazolidinyl, 1,2,3,4-tetrahydroisoquinolinyl, 1,2,6-dithiazinyl, 1,1-dioxo-2-yl, 4-hydroxy-1,4-azaphosphine-4-oxide-1-yl, 2-hydroxy-1-(piperazin-1-yl)ethanone-4-yl, 2-hydroxy-1-(5,6-dihydro-1,2,4-triazin-1(4H)-yl)ethanone-4-yl, 5,6-dihydro-4H-1,2,4-oxadiazine-4-yl, 2-hydroxy-1-(5,6-diludine-1(2H)-yl)ethanone-4-yl, 3-azabicyclo[3,1,0]hexyl, 3-azabicyclo[4,1,0]heptyl, azabicyclo[2,2,2]hexyl, 2-methyl-5,6,7,8-tetrahydro-[1,2,4]triazole[1,5-c]pyrimidine-6-yl, 4,5,6,7-teterhydro-isoxazolo[4,3-c]pyridine-5-yl, 3H-indoxyl-2-oxo-5-azabicyclo[2,2,1]heptane-5-yl, 2-oxo-5-azabicyclo[2,2,2]octane-5-yl, quinolizinyl and N-pyridyl urea. Some non-limiting examples of a heterocyclic ring include 1,1-dioxo-thiomorpholinyl and heterocyclic group wherein 2 carbon atoms on the ring are substituted with oxo (=O) moieties are pyrimidindionyl. The heterocyclic groups herein may be substituted or unsubstituted, wherein the substituent may be, but is not limited to, deuterium, oxo (=O), hydroxy, amino, halo, cyano, heteroaryl, alkoxy, alkylamino, alkyl, alkenyl, alkynyl, heterocyclyl, mercapto, nitro, aryloxy, hydroxy-substituted alkoxy, hydroxy-substituted alkyl-C(=O)-, alkyl-C(=O)-, alkyl-S(=O)-, alkyl-S(=O)₂-, hydroxy-substituted alkyl-S(=O)-, hydroxy-substituted alkyl-S(=O)₂-, carboxyalkoxy, and the like.

The term "heterocyclylalkyl" refers to heterocyclic-substituted alkyl radical. The term "heterocyclylalkoxy" refers to heterocyclic-substituted alkoxy radical wherein oxygen atom serves as the attaching point to the rest of the molecule. The term "heterocyclylalkylamino" refers to heterocyclic-substituted alkylamino radical wherein nitrogen atom serves as the attaching point to the rest of the molecule. Wherein the heterocyclyl, alkyl, alkoxy and alkylamino group are as defined herein. Some non-limiting examples include pyrrol-2-ylmethyl, morpholin-4-ylethyl, morpholin-4-ylethoxy, piperazin-4-ylethoxy, piperidin-4-ylethylamino, and the like.

The term "heterocyclylaliphatic" refers to heterocyclic-substituted aliphatic group, wherein the heterocyclic group and aliphatic group are as defined herein. Some non-limiting examples include pyrrol-2-ylmethyl, piperidin-2-ylethyl, piperazin-2-ylethyl, piperidin-2-ylmethyl, and the like.

The term "heterocyclyloxy" refers to optionally substituted heterocyclyl radical, as defined herein, connected to an oxygen atom, and the oxygen atom serves as the attaching point to the rest of the molecule. Some non-limiting examples include pyrrol-2-yloxy, pyrrol-3-yloxy, piperidin-2-yloxy, piperidin-3-yloxy, piperazin-2-yloxy, piperidin-4-yloxy, and the like.

The term "heterocyclylamino" refers to an amino group substituted with one or two heterocyclyl groups, wherein nitrogen atom serves as the attaching point to the rest of the molecule and the heterocyclyl group is as defined herein. Some non-limiting examples include pyrrol-2-ylamino, pyrrol-3-ylamino, piperidin-2-ylamino, piperidin-3-ylamino, piperidin-4-ylamino, piperazin-2-ylamino, dipyrrol-2-ylamino, and the like.

The term "heterocyclyloxyalkoxy" refers to an alkoxy radical substituted with one or more heterocyclyloxy groups, wherein the alkoxy group and heterocyclyloxy group are as defined herein. Some non-limiting examples include pyrrol-2-yloxymethoxy, pyrrol-3-yloxyethoxy, piperidin-2-yloxyethoxy, piperidin-3-yloxyethoxy, piperazin-2-yloxymethoxy, piperidin-4-yloxyethoxy, and the like.

The term "heterocyclyloxyaliphatic" refers to an aliphatic group substituted with one or more heterocyclyloxy groups, wherein the aliphatic group and heterocyclyloxy group are as defined herein. Some non-limiting examples include pyrrol-2-yloxymethyl, piperazin-3-yloxyethyl, piperazin-2-yloxyethyl, morpholin-2-yloxymethyl, piperidin-2-yloxyethyl, and the like.

The term "heterocyclylaminoaliphatic" refers to an aliphatic group substituted with one or more heterocyclylamino groups, wherein the aliphatic group and heterocyclylamino group are as defined herein. Some non-limiting examples include pyrrol-2-ylaminomethyl, piperazin-3-lyaminoethyl, piperazin-2-lyaminoethyl, piperidin-2-lyaminoethyl, morpholin-2-lyaminomethyl, and the like.

The term "heteroatom" refers to one or more of oxygen, sulfur, nitrogen, phosphorus, or silicon, including any oxidized form of nitrogen, sulfur, or phosphorus; the quaternized form of any basic nitrogen; or a substitutable nitrogen of a heterocyclic ring, for example, N (as in 3,4-dihydro-2*H*-pyrrolyl), NH (as in pyrrolidinyl) or NR (as in N-substituted pyrrolidinyl).

The term "halogen" or "halo" refers to F, Cl, Br or I.

The term "unsaturated" as used herein, refers to a moiety having one or more units of unsaturation.

The term "alkoxy" refers to an alkyl group, as previously defined, attached to the principal carbon chain through an oxygen ("alkoxy") atom. Some non-limiting examples include methoxy, ethoxy, propoxy, butoxy, and the like. And the alkoxy defined above may be substituted or unsubstituted, wherein the substituent may be, but is not limited to, deuterium, hydroxy, amino, halo, cyano, alkoxy, alkyl, alkenyl, alkynyl, thiol, nitro, and the like.

The term "hydroxy-substituted alkoxy" or "hydroxyalkoxy" refers to an alkoxy group substituted with one or more hydroxy groups, wherein the alkoxy group is as defined above. Some non-limiting examples include hydroxymethoxy, 2-hydroxyethoxy, 2-hydroxypropoxy, 2-hydroxyisopropoxy, and the like.

The term "aminoalkoxy" refers to an alkoxy group substituted with one or more amino groups, wherein the alkoxy group is as defined above. Some non-limiting examples include aminomethoxy, 2-aminoethoxy, 2-aminopropoxy, 2-aminoisopropoxy, and the like.

The term "azidoalkoxy" refers to an alkoxy group substituted with one or more azido groups, wherein the alkoxy group is as defined above. Some non-limiting examples include 2-azidoethoxy, 3-azidopropoxy, 2-azidopropoxy, and the like.

The term "alkoxyalkoxy" refers to an alkoxy group substituted with one or more alkoxy groups, wherein the alkoxy group is as defined above. Some non-limiting examples include methoxymethoxy, methoxyethoxy, ethoxymethoxy, ethoxyethoxy, ethoxypropoxy, and the like.

The term "alkoxyaliphatic" refers to an aliphatic group substituted with one or more alkoxy groups, wherein the aliphatic group and alkoxy group are as defined herein. Some non-limiting examples include methoxymethyl, ethoxymethyl, ethoxyethyl, ethoxypropenyl, and the like.

The term "alkylaminoaliphatic" refers to an aliphatic group substituted with one or more alkylamino groups, wherein the aliphatic group and alkylamino group are as defined herein. Some non-limiting examples include dimethylaminoethyl, methylaminoethyl, diethylaminomethyl, diethylaminoethyl, and the like.

The term "alkylthioaliphatic" refers to an aliphatic group substituted with one or more alkylthio groups, wherein the aliphatic group and alkylthio group are as defined herein. Some non-limiting examples include methylthioethyl, methylthiopropyl, ethylthioethyl, methylthiopropenyl, and the like.

The term "haloalkyl", "haloalkenyl" or "haloalkoxy" refers to an alkyl group, alkenyl group or alkoxy group substituted with one or more halogen atoms. Some non-limiting examples include trifluoromethyl, 2-chloro-ethenyl, trifluoromethoxy, and the like.

The term "aryl" used alone or as part of a larger moiety as in "aralkyl", "arylalkoxy" or "aryloxyalkyl" refers to monocyclic, bicyclic, and tricyclic carbocyclic ring systems having a total of six to fourteen ring members, wherein at least one ring in the system is aromatic, wherein each ring in the system contains 3 to 7 ring members and that has a single point of attachment to the rest of the molecule. The term "aryl" may be used interchangeably with the term "aryl ring". Some non-limiting examples of aryl rings include phenyl, naphthyl, and anthryl. The aryl may be substituted or unsubstituted, wherein the substituents include, but are not limited to, deuterium, hydroxy, amino, halo, cyano, aryl, heteroaryl, alkoxy, alkylamino, alkyl, alkenyl, alkynyl, heterocyclyl, mercapto, nitro, aryloxy, hydroxy-substituted alkoxy, hydroxy-substituted alkyl-C(=O)-, alkyl-C(=O)-, alkyl-S(=O)-, alkyl-S(=O)₂-, hydroxy-substituted alkyl-S(=O)-, hydroxy-substituted alkyl-S(=O)₂-, carboxyalkoxy, and the like.

The term "arylaliphatic" refers to an aliphatic group substituted with one or more optionally substituted aryl groups, wherein the aliphatic group and the aryl group are as defined herein. Some non-limiting examples include phenylethyl, benzyl, (*p*-tolyl)ethyl, styryl, and the like.

The term "aryloxy" refers to optionally substituted aryl radicals, as defined herein, attached to an oxygen atom, wherein the oxygen atom serves as the attaching point to the rest of the molecule. Wherein the aryl radical is as defined herein. Some non-limiting examples include phenyloxy, methylphenyloxy, ethylphenyloxy, and the like.

The term "arylamino" refers to an amino group substituted with one or two optionally substituted aryl groups, wherein the aryl group is as defined herein. Some non-limiting examples include phenylamino, (*p*-fluorophenyl)amino, diphenylamino, ditolylamino, (di-*p*-tolyl)amino, and the like.

The term "aryloxyalkoxy" refers to an alkoxy group substituted with one or more optionally substituted aryloxy groups, wherein the alkoxy group and the aryloxy group are as defined herein.

Some non-limiting examples include phenyloxymethoxy, phenyloxyethoxy, phenyloxypropoxy, and the like.

The term "aryloxyaliphatic" refers to an aliphatic group substituted with one or more optionally substituted aryloxy groups, wherein the aryloxy group and the aliphatic group are as defined herein. Some non-limiting examples include phenyloxymethyl, phenyloxyethyl, phenyloxypropyl, and the like.

The term "arylaminoaliphatic" refers to an aliphatic group substituted with one or more optionally substituted arylamino groups, wherein the arylamino group and the aliphatic group are as defined herein. Some non-limiting examples include phenylaminomethyl, phenylaminoethyl, tolylaminoethyl, phenylaminopropyl, phenylaminoallyl, and the like.

The term "arylalkoxy" refers to an alkoxy group substituted with one or more optionally substituted aryl groups, wherein the aryl group and the alkoxy group are as defined herein. Some non-limiting examples include phenylmethoxy, phenylethoxy, (*p*-tolyl)methoxy, phenylpropoxy, and the like.

The term "arylalkylamino" refers to an alkylamino group substituted with one or more optionally substituted aryl groups, wherein the aryl group and the alkylamino group are as defined herein. Some non-limiting examples include phenylmethylamino, phenylethylamino, phenylpropylamino, (*p*-tolyl)methylamino, and the like.

The term "heteroaryl" used alone or as part of a larger moiety as in "heteroarylalkyl" or "heteroarylalkoxy" refers to monocyclic, bicyclic, and tricyclic ring systems having a total of five to fourteen ring members, wherein at least one ring in the system is aromatic, and at least one ring in the system is inclusive of one or more heteroatoms, wherein each ring in the system contains 3 to 7 ring members and that has a single point of attachment to the rest of the molecule. The term "heteroaryl" may be used interchangeably with the term "heteroaryl ring" or "heteroaromatic compound". The heteroaryl defined herein may be substituted or unsubstituted, wherein the substituents include, but are not limited to, deuterium, hydroxy, amino, halo, cyano, aryl, heteroaryl, alkoxy, alkylamino, alkyl, alkenyl, alkynyl, heterocyclyl, mercapto, nitro, aryloxy, hydroxy-substituted alkoxy, hydroxy-substituted alkyl-C(=O)-, alkyl-C(=O)-, alkyl-S(=O)-, alkyl-S(=O)₂-, hydroxy-substituted alkyl-S(=O)-, hydroxy-substituted alkyl-S(=O)₂-, carboxyalkoxy, and the like.

Some non-limiting examples of suitable heteroaryl rings include the following monocycles: 2-furanyl, 3-furanyl, *N*-imidazolyl, 2-imidazolyl, 4-imidazolyl, 5-imidazolyl, 3-isoxazolyl, 4-isoxazolyl, 5-isoxazolyl, 2-oxazolyl, 4-oxazolyl, 5-oxazolyl, 4-methylisoxazolyl-5-yl, N-pyrrolyl, 2-pyrrolyl, 3-pyrrolyl, 2-pyridyl, 3-pyridyl, 4-pyridyl, 2-pyrimidinyl, 4-pyrimidinyl, pyrimidin-5-yl, pyridazinyl (e.g., 3-pyridazinyl), 2-thiazolyl, 4-thiazolyl, 5-thiazolyl, tetrazolyl (e.g., 5-tetrazolyl), triazolyl (e.g., 2-triazolyl and 5-triazolyl), 2-thienyl, 3-thienyl, pyrazolyl (e.g., 2-pyrazolyl), isothiazolyl, 1,2,3-oxadiazolyl, 1,2,5-oxadiazolyl, 1,2,4-oxadiazolyl, 1,2,3-triazolyl, 1,2,3-thiadiazolyl, 1,3,4-thiadiazolyl, 1,2,5-thiadiazolyl, 1,3,4-thiadiazol-2-yl, pyrazinyl, pyrazin-2-yl, 1,3,5-triazinyl, benzo[*d*]thiazol-2-yl, imidazo[1,5-a]pyridin-6-yl, and the following bicycles: benzimidazolyl, benzofuryl, benzothiophenyl, indolyl (e.g., 2-indolyl), purinyl, quinolinyl (e.g., 2-quinolinyl, 3-quinolinyl, 4-quinolinyl), or isoquinolinyl (e.g., 1-isoquinolinyl, 3-isoquinolinyl, or 4-isoquinolinyl).

The term "heteroaryloxy" refers to an optionally substituted heteroaryl radical, as defined herein, attached to an oxygen atom, wherein the oxygen atom serves as the attaching point to the rest of the molecule. Some non-limiting examples include pyrid-2-yloxy, thiazol-2-yloxy, imidazol-2-yloxy, pyrimidin-2-yloxy, and the like.

The term "heteroaryloxyaliphatic" refers to an aliphatic group substituted with one or more optionally substituted heteroaryloxy groups, wherein the aliphatic group and the heteroaryloxy group are as defined herein. Some non-limiting examples include pyrid-2-yloxyethyl, thiazol-2-yloxymethyl, imidazol-2-yloxyethyl, pyrimidin-2-yloxypropyl, and the like.

The term "sulfonyl", whether used alone or linked to other terms such as "alkylsulfonyl", refers to respectively divalent radicals -SO₂-.

The term "alkylsulfonyl", refers to a sulfonyl radical substituted with an alkyl radical, forming an alkylsulfonyl (-SO₂-alkyl, such as -SO₂CH₃).

The term "sulfamyl", "aminosulfonyl" or "sulfonamidyl" refer to a sulfonyl radical substituted with an amine radical, forming a sulfonamide (-SO₂NH₂).

The term "carboxy" or "carboxyl", whether used alone or with other terms, such as "carboxyalkyl", refers to -CO₂H.

The term "carbonyl", whether used alone or with other terms, such as "aminocarbonyl" or "carbonyloxy", refers to -(C=O)-.

The term "carboxyalkoxy" refers to an alkoxy group substituted with one or more carboxy groups, wherein the alkoxy group and the carboxy group are as defined herein. Some non-limiting examples include carboxymethoxy, carboxyethoxy, and the like.

The term "aralkyl" or "arylalkyl" refers to aryl-substituted alkyl radicals. In some embodiments, aralkyl radicals are "lower aralkyl" radicals having aryl radicals attached to alkyl radicals having one to six carbon atoms. In other embodiments, aralkyl radicals are "phenylalkylenyl" attached to alkyl portions having one to three carbon atoms. Some non-limiting examples of such radicals include benzyl, diphenylmethyl and phenylethyl. The aryl in said aralkyl can be additionally substituted with halo, alkyl, alkoxy, haloalkyl or haloalkoxy.

The term "alkylthio" refers to radicals containing a linear or branched alkyl radical, of one to ten carbon atoms, attached to a divalent sulfur atom. In other embodiments, alkylthio radicals are lower alkylthio radicals having one to three carbon atoms. Some non-limiting examples of "alkylthio" include methylthio (CH₃S-).

The term "haloalkylthio" refers to radicals containing a haloalkyl radical, of one to ten carbon atoms, attached to a divalent sulfur atom. In other embodiments, haloalkylthio radicals are lower haloalkylthio radicals having one to three carbon atoms. Some non-limiting examples of "haloalkylthio" include trifluoromethylthio.

The term "alkylamino" refers to *"N-*alkylamino" and *"N,N-*dialkylamino", wherein amino groups are independently substituted with one alkyl radical or with two alkyl radicals, respectively. In other embidiments, alkylamino radicals are lower alkylamino radicals having one or two alkyl radicals of one to six carbon atoms, attached to a nitrogen atom. In still other embodiments, alkylamino radicals are lower alkylamino radicals having one to three carbon atoms. Some non-limiting examples of suitable alkylamino radicals include mono or dialkylamino such as *N*-methylamino, *N-*ethylamino, *N,N*-dimethylamino, *N,N*-diethylamino, and the like.

The term "alkylaminohaloalkoxy" refers to a haloalkoxy group substituted with one or more alkylamino groups, wherein the haloalkoxy group and the alkylamino group are as defined herein. Some non-limiting examples include methylaminodifluoromethoxy, ethylaminotrifluoromethoxy, and the like.

The term "heteroarylamino" refers to amino groups substituted with one or two heteroaryl radicals, wherein the heteroaryl radical is as defined herein. Some non-limiting examples of heteroarylamino include *N*-thienylamino. In other embodiments, the "heteroarylamino" radicals include substituted on the heteroaryl ring portion of the radical.

The term "heteroarylaliphatic" refers to aliphatic groups substituted with one or more heteroaryl radicals, wherein the heteroaryl group and the aliphatic group are as defined herein. Some non-limiting examples of heteroarylaliphatic include thiophen-2-ylpropenyl, pyridin-4-ylethyl, imidazol-2-ylmethyl, furan-2-ylethyl, indol-3-ylmethyl, and the like.

The term "heteroarylalkyl" refers to alkyl groups substituted with one or more heteroaryl radicals, wherein the heteroaryl group and the alkyl group are as defined herein. Some non-limiting examples of heteroarylalkyl include imidazol-2-ylmethyl, furan-2-ylethyl, indol-3-ylmethyl, and the like.

The term "heteroarylalkylamino" refers to nitrogen-containing heteroarylalkyl radicals attached through a nitrogen atom to other radicals, wherein the heteroarylalkyl radical is as defined herein. Some non-limiting examples of heteroarylalkylamino include pyridin-2-ylmethylamino, thiazol-2-ylethylamino, imidazol-2-ylethylamino, pyrimidin-2-ylpropylamino, pyrimidin-2-ylmethylamino, and the like.

The term "aminoalkyl" refers to a linear or branched alkyl radical having one to ten carbon atoms, substituted with one or more amino radicals. In some embodiments, aminoalkyl radicals are "lower aminoalkyl" radicals having one to six carbon atoms and one or more amino radicals. Some non-limiting examples of such radicals include aminomethyl, aminoethyl, aminopropyl, aminobutyl and aminohexyl.

The term "alkylaminoalkyl" refers to alkyl radicals substituted with alkylamino radicals. In some embodiments, alkylaminoalkyl radicals are "lower alkylaminoalkyl" radicals having alkyl radicals of one to six carbon atoms. In other embodiments, alkylaminoalkyl radicals are lower alkylaminoalkyl radicals having alkyl radicals of one to three carbon atoms. Some non-limiting examples of suitable alkylaminoalkyl radicals include mono and dialkyl substituted, such as *N*-methylaminomethyl, *N,N*-dimethylaminoethyl, *N,N*-diethylaminomethyl, and the like.

The term "carboxyalkyl" refers to a linear or branched alkyl radical having one to ten carbon atoms substituted with one or more carboxy radicals. Some non-limiting examples of such radicals include carboxymethyl, carboxypropyl, and the like.

The term "aryloxy" refers to optionally substituted aryl radicals, as defined above, attached to an oxygen atom, wherein the oxygen atom serves as the attaching point to the rest of the molecule. Some non-limiting examples of such radicals include phenoxy.

The term "heteroarylalkoxy" refers to oxy-containing heteroarylalkyl radicals attached through an oxygen atom to other radicals, wherein the heteroarylalkyl radical is as defined herein. Some non-limiting examples of such radicals include pyridin-2-ylmethoxy, thiazol-2-ylethoxy, imidazol-2-ylethoxy, pyrimidin-2-ylpropoxy, pyrimidin-2-ylmethoxy, and the like.

The term "cycloalkylalkyl" refers to cycloalkyl-substituted alkyl radicals. Some non-limiting examples of such radicals include cyclohexylmethyl. The cycloalkyl in the radicals may be additionally substituted with halo, alkyl, alkoxy or hydroxy.

The term "fused bicyclic", "fused cyclic", "fused bicyclyl" or "fused cyclyl" refers to unsaturated or saturated fused cyclic system and bridged ring system that is not aromatic. For example, as depicted below (Formula (al)), ring A1 and ring A2 share a bond that is a alkyl or heteroalkyl chain, wherein j is 0, 1, 2, 3 or 4. Such a system may contain isolated or conjugated unsaturation, but not aromatic or heteroaromatic rings in its core structure (but may have aromatic substitution thereon). Each cyclic ring in a fused bicyclyl can be either a carbocyclic or a heteroalicyclic. Some non-limiting examples of fused bicyclic ring system or bridged ring system include hexahydro-furo[3,2-*b*]furan, 2,3,3a,4,7,7*a*-hexahydro-1*H-*indene, 7-azabicyclo[2.3.0]heptane, fused bicyclo[3.3.0]octane, fused bicyclo[3.1.0]hexane, bicyclo[2.2.1]heptane, 2-azabicyclo[2.2.1]heptane, and 1,2,3,4,4a,5,8,8*a*-octahydro- naphthalene. The fused bicyclyl defined herein may be substituted or unsubstituted, wherein the substituents include, but are not limited to, deuterium, oxo (=O), hydroxy, amino, halo, cyano, aryl, heteroaryl, alkoxy, alkylamino, alkyl, alkenyl, alkynyl, heterocyclyl, mercapto, nitro, aryloxy, hydroxy-substituted alkoxy, hydroxy-substituted alkyl-C(=O)-, alkyl-C(=O)-, alkyl-S(=O)-, alkyl-S(=O)₂-, hydroxy-substituted alkyl-S(=O)-, hydroxy-substituted alkyl-S(=O)₂-, carboxyalkoxy, and the like.

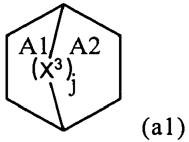

The term "fused heterobicyclyl" refers to unsaturated or saturated fused cyclic system and bridged ring system that is not aromatic. Such a system may contain isolated or conjugated unsaturation, but not aromatic or heteroaromatic rings in its core structure (but may have aromatic substitution thereon). And at least one ring in the system is inclusive of one or more heteroatoms, wherein each ring in the system contains 3 to 7 ring members, e.g., 1 to 6 carbon atoms and 1 to 3 heteroatoms selected from N, O, P or S, wherein the S or P is optionally substituted with one or more oxo to provide the group SO or SO₂, PO or PO₂. Some non-limiting examples of fused heterobicyclic ring system include hexahydro-furo[3,2-*b*]furan, 6-azabicyclo[3.2.0]heptane, 2-azabicyclo[3.1.0]heptane, 3-azabicyclo[3.1.0]heptane, 7-azabicyclo[2.3.0]heptane, 2-azabicyclo[2.2.1]heptane, and the like. The fused heterobicyclyl defined herein may be substituted or unsubstituted, wherein the substituents include, but are not limited to, deuterium, oxo (=O), hydroxy, amino, halo, cyano, aryl, heteroaryl, alkoxy, alkylamino, alkyl, alkenyl, alkynyl, heterocyclyl, mercapto, nitro, aryloxy, hydroxy-substituted alkoxy, hydroxy-substituted alkyl-C(=O)-, alkyl-C(=O)-, alkyl-S(=O)-, alkyl-S(=O)₂-, hydroxy-substituted alkyl-S(=O)-, hydroxy-substituted alkyl-S(=O)₂-, carboxyalkoxy, and the like.

The term "spirocyclyl", "spirocyclic", "spiro bicyclyl" or "spiro bicyclic" refers to a ring originating from a particular annular carbon of another ring. For example, as depicted below, ring A and ring B share a carbon atom between the two saturated ring system, which terms as a "spirocyclyl" or "spiro bicyclyl". Each cyclic ring in the spirocyclyl or spiro bicyclyl can be either a carbocyclic or a heteroalicyclic. Some non-limiting examples of such radicals include 2,7-diaza-spiro[4.4]non-2-yl, 7-oxo-2-azaspiro[4.5]dec-2-yl, 4-azaspiro[2.4]hept-5-yl, 4-oxaspiro[2.4]hept-5-yl, 5-azaspiro[2.4]hept-5-yl, spiro[2.4]heptyl, spiro[4.4]nonyl, 7-hydroxy-5-azaspiro[2.4]hept-5-yl, and the like. The spirocyclyl or spiro bicyclyl may be substituted or unsubstituted, wherein the substituents include, but are not limited to, deuterium, oxo (=O), hydroxy, amino, halo, cyano, aryl, heteroaryl, alkoxy, alkylamino, alkyl, alkenyl, alkynyl, heterocyclyl, mercapto, nitro, aryloxy, hydroxy-substituted alkoxy, hydroxy-substituted alkyl-C(=O)-, alkyl-C(=O)-, alkyl-S(=O)-, alkyl-S(=O)₂-, hydroxy-substituted alkyl-S(=O)-, hydroxy-substituted alkyl-S(=O)₂-, carboxyalkoxy, and the like.

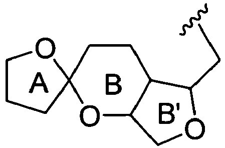

The term "spiro bicyclylene" refers to spiro bicyclyl system having two connection points connected to the rest of the molecule, wherein the spiro bicyclyl radical is as defined herein.

The terms "spiro heterobicyclyl" refers to a ring originating from a particular annular carbon of another ring. For example, as depicted above, ring A and ring B share a carbon atom between the two saturated ring system, which terms as a "spirocyclyl". And at least one ring in the system is inclusive of one or more heteroatoms, wherein each ring in the system contains 3 to 7 ring members, e.g., 1 to 6 carbon atoms and 1 to 3 heteroatoms selected from N, O, P or S, wherein the S or P is optionally substituted with one or more oxo to provide the group SO or SO₂, PO or PO₂. Some non-limiting examples of such radicals include 4-azaspiro[2,4]hept-5-yl, 4-oxaspiro[2,4]hept-5-yl, 5-azaspiro[2,4]hept-5-yl, 7-hydroxy-5-azaspiro[2,4]hept-5-yl, 5-azaspiro[2,4]hept-6-yl, 1,4-dioxo-7-azaspiro [4,4]non-8-yl, and the like. The spiro heterobicyclyl defined herein may be substituted or unsubstituted, wherein the substituents include, but are not limited to, deuterium, oxo (=O), hydroxy, amino, halo, cyano, aryl, heteroaryl, alkoxy, alkylamino, alkyl, alkenyl, alkynyl, heterocyclyl, mercapto, nitro, aryloxy, hydroxy-substituted alkoxy, hydroxy-substituted alkyl-C(=O)-, alkyl-C(=O)-, alkyl-S(=O)-, alkyl-S(=O)₂-, hydroxy-substituted alkyl-S(=O)-, hydroxy-substituted alkyl-S(=O)₂-, carboxyalkoxy, and the like.

As described herein, the group derived from α-amino acid refers to an α-amino acid radical derived from an α-amino acid by the removal of one hydroxy from the carboxy group, which attached to X or X', and the group derived from α-amino acid is optionally substituted with one or more substituents, wherein the substituent is deuterium, F, Cl, Br, I, hydroxy, cyano, alkoxycarbonyl, aryloxycarbonyl, heteroaryloxycarbonyl or heterocycylcarbonyl. For example,

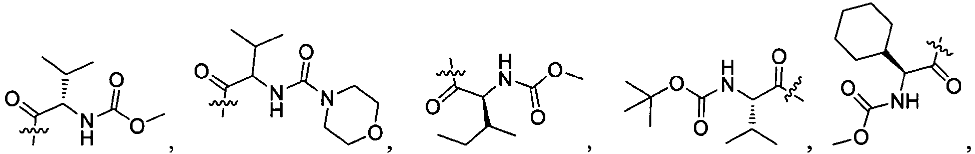

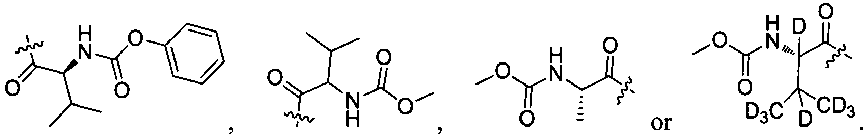

As described herein, a bond drawn from a substituent to the center of one ring within a ring system (as shown in Formula (a)) represents substitution of the substituent (R^{5a})_{f} at any substitutable position on the rings (W1, W2, and W). For example, Formula (a) represents possible substitution in any of the positions on the W1, W2, and W ring.

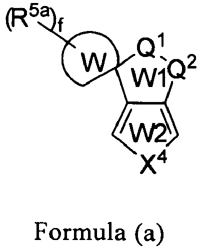

As described herein, two attaching points either E or E', within a ring system (as shown in Formula (b)), attach to the rest of the molecule, e.g., E and E' may be used interchangeably with each other.

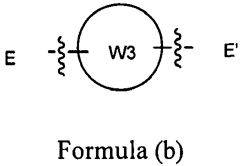

As described herein, a dot line drawn together with a bond within a ring system (as shown in Formula (c)) represents either a double bond or a single bond. For example, structure in Formula (c) represents any structures selected from Formula (d).

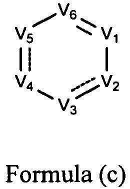

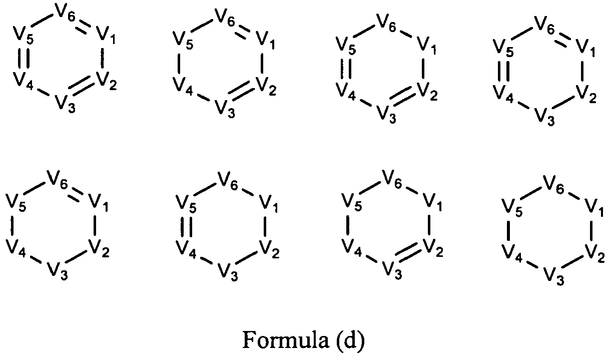

Unless otherwise stated, structures depicted herein are also meant to include all isomeric (e.g., enantiomeric, diastereomeric, and geometric (or conformational)) forms of the structure; for example, the R and S configurations for each asymmetric center, (Z) and (E) double bond isomers, and (Z) and (E) conformational isomers. Therefore, single stereochemical isomers as well as enantiomeric, diastereomeric, or geometric (or conformational) mixtures of the present compounds are within the scope disclosed herein.

Furthermore, what need to be explained is that the phrase "each... is independently" is used interchangeably with the phrase "each (of)...and...is independently", unless otherwise stated. It should be broadly understood that the specific options expressed by the same symbol are independently of each other in different radicals; or the specific options expressed by the same symbol are independently of each other in same radicals. For example, R⁹ of the structure formulas "-U -(CR⁹R^{9a})ₜ-R¹²" and "-[U-(CR⁹R^{9a})^{t}-N(R¹⁰)-(CR⁹R^{9a})ₜ]ₖ-U-(CR⁹R^{9a)}ₜ-N(R¹¹)-(CR⁹R^{9a})ₜ-R¹²", whose specific options are independently of each other; meanwhile, multiple R⁹ of the structure formula "-[U-(CR⁹R^{9a})ₜ-N(R¹⁰)-(CR⁹R^{9a})ₜ]ₖ-U-(CR⁹R^{9a})ₜ-N(R¹¹)-(CR⁹R^{9a})ₜ-R¹²", whose specific options are independently of each other.

The term "prodrug" refers to a compound that is transformed *in vivo* into a compound of formula (I). Such a transformation can be affected, for example, by hydrolysis in blood or enzymatic transformation of the prodrug form to the parent form in blood or tissue. Prodrugs of the compounds disclosed herein may be, for example, esters. Esters that may be utilized as prodrugs in the present invention are phenyl esters, aliphatic (C₁-C₂₄) esters, acyloxymethyl esters, carbonates, carbamates, and amino acid esters. For example, a compound disclosed herein that contains an OH group may be acylated at this position in its prodrug form. Other prodrug forms include phosphates, such as, for example those phosphates resulting from the phosphonation of an OH group on the parent compound. A thorough discussion of prodrugs is provided in Higuchi et al., Pro-drugs as Novel Delivery Systems, Vol. 14, A.C.S. Symposium Series; Roche, et al. ed., Bioreversible Carriers in Drug Design, American Pharmaceutical Association and Pergamon Press, 1987**;** Rautio et al., Prodrugs: Design and Clinical Applications, Nature Reviews Drug Discovery, 2008, 7, 255-270, and Hecker et al, Prodrugs of Phosphates and Phosphonates, J. Med. Chem., 2008, 51, 2328-2345.

Unless otherwise stated, all tautomeric forms of the compounds disclosed herein are within the scope of the invention. Additionally, unless otherwise stated, structures depicted herein are also meant to include compounds that differ only in the presence of one or more isotopically enriched atoms.

A "metabolite" is a product produced through metabolism in the body of a specified compound or salt thereof. Metabolites of a compound may be identified using routine techniques known in the art and their activities determined using tests such as those described herein. Such products may result for example from the oxidation, reduction, hydrolysis, amidation, deamidation, esterification, deesterification, enzyme cleavage, and the like, of the administered compound. Accordingly, the invention includes metabolites of compounds disclosed herein, including compounds produced by a process comprising contacting a compound disclosed herein with a mammal for a period of time sufficient to yield a metabolic product thereof.

Stereochemical definitions and conventions used herein generally follow Parker et al., McGraw-Hill Dictionary of Chemical Terms (1984) McGraw-Hill Book Company, New York and Eliel et al., "Stereochemistry of Organic Compounds", John Wiley & Sons, Inc., New York, 1994**.** The compounds disclosed herein may contain asymmetric or chiral centers, and therefore exist in different stereoisomeric forms. It is intended that all stereoisomeric forms of the compounds disclosed herein, including but not limited to, diastereomers, enantiomers and atropisomers, as well as mixtures thereof such as racemic mixtures, form part of the present invention. Many organic compounds exist in optically active forms, i.e., they have the ability to rotate the plane of plane-polarized light. In describing an optically active compound, the prefixes D and L, or R and S, are used to denote the absolute configuration of the molecule about its chiral center(s). The prefixes d and 1 or (+) and (-) are employed to designate the sign of rotation of plane-polarized light by the compound, with (-) or 1 meaning that the compound is levorotatory. A compound prefixed with (+) or d is dextrorotatory. For a given chemical structure, these stereoisomers are identical except that they are mirror images of one another. A specific stereoisomer may also be referred to as an enantiomer, and a mixture of such isomers is often called an enantiomeric mixture. A 50:50 mixture of enantiomers is referred to as a racemic mixture or a racemate, which may occur where there has been no stereoselection or stereospecificity in a chemical reaction or process. The term "racemic mixture" or "racemate" refers to an equimolar mixture of two enantiomeric species, devoid of optical activity.

The term "tautomer" or "tautomeric form" refers to structural isomers of different energies which are interconvertible via a low energy barrier. Some non-limiting examples of proton tautomers (also known as prototropic tautomers) include interconversions via migration of a proton, such as keto-enol and imine-enamine isomerizations. Valence tautomers include interconversions by reorganization of some of the bonding electrons.

A "pharmaceutically acceptable salts" refers to organic or inorganic salts of a compound disclosed herein. Pharmaceutically acceptable salts are well known in the art. For example, Berge et al., describe pharmaceutically acceptable salts in detail in J. Pharmacol Sci, 1977, 66: 1-19. Some non-limiting examples of pharmaceutically salts include salts of an amino group formed with inorganic acids such as hydrochloric acid, hydrobromic acid, phosphoric acid, sulfuric acid and perchloric acid or with organic acids such as acetic acid, oxalic acid, maleic acid, tartaric acid, citric acid, succinic acid or malonic acid or by using other methods used in the art such as ion exchange. Other pharmaceutically acceptable salts include adipate, alginate, ascorbate, aspartate, benzenesulfonate, benzoate, bisulfate, borate, butyrate, camphorate, camphorsulfonate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxy-ethanesulfonate, lactobionate, lactate, laurate, laurylsulfate, malate, malonate, methanesulfonate, 2-naphthalenesulfonate, nicotinate, nitrate, oleate, palmitate, pamoate, pectinate, persulfate, 3-phenylpropionate, picrate, pivalate, propionate, stearate, thiocyanate, *p*-toluenesulfonate, undecanoate, valerate salts, and the like. Salts derived from appropriate bases include alkali metal, alkaline earth metal, ammonium and N⁺(C₁₋₄ alkyl)₄ salts. This invention also envisions the quaternization of any basic nitrogen-containing groups of the compounds disclosed herein. Water or oilsoluble or dispersable products may be obtained by such quaternization. Representative alkali or alkaline earth metal salts include sodium, lithium, potassium, calcium, magnesium, and the like. Further pharmaceutically acceptable salts include, when appropriate, nontoxic ammonium, quaternary ammonium, and amine cations formed using counterions such as halide, hydroxide, carboxylate, sulfate, phosphate, nitrate, C₁₋₈ sulfonate or aryl sulfonate.

A "solvate" refers to an association or complex of one or more solvent molecules and a compound disclosed herein. Some non-limiting examples of solvents that form solvates include water, isopropanol, ethanol, methanol, DMSO, ethyl acetate, acetic acid, and ethanolamine. The term "hydrate" refers to the complex where the solvent molecule is water.

The term "protecting group" or "Pg" refers to a substituent that is commonly employed to block or protect a particular functionality while reacting with other functional groups on the compound. For example, an "amino-protecting group" is a substituent attached to an amino group that blocks or protects the amino functionality in the compound. Some non-limiting examples of suitable amino-protecting groups include acetyl, trifluoroacetyl, *t*-butoxycarbonyl (BOC), benzyloxycarbonyl (CBZ) and 9-fluorenylmethylenoxycarbonyl (Fmoc). Similarly, a "hydroxy-protecting group" refers to a substituent of a hydroxy group that blocks or protects the hydroxy functionality. Some non-limiting examples of suitable hydroxy-protecting groups include acetyl and silyl. A "carboxy-protecting group" refers to a substituent of the carboxy group that blocks or protects the carboxy functionality. Some non-limiting examples of common carboxy-protecting groups include -CH₂CH₂SO₂Ph, cyanoethyl, 2-(trimethylsilyl)ethyl, 2-(trimethylsilyl)ethoxymethyl, 2-(*p*-toluenesulfonyl)ethyl, 2-(*p*-nitrophenylsulfonyl)ethyl, 2-(diphenylphosphino)-ethyl, nitroethyl, and the like. For a general description of protecting groups and their use, see Greene et al., Protective Groups in Organic Synthesis, John Wiley & Sons, New York, 1991 and Kocienski et al., Protecting Groups, Thieme, Stuttgart, 2005.

It should be noted that the term of "inhibiting HCV viral protein" should be broadly understood, which comprises inhibiting the expression level of HCV viral protein, inhibiting activity level of HCV viral protein, viral assembly and egress level. The expression level of HCV protein includes but not limited to translation level of the viral protein, posttranslational modification level of the viral protein, replication level of genetic material in offsprings and so on.

### DESCRIPTION OF COMPOUNDS OF THE INVENTION

Provided herein are spiro ring compounds, and pharmaceutical formulations thereof, that are useful in inhibiting HCV infection, especially inhibiting the activity of the non-structural 5A ("NS5A") protein.

In one aspect, provided herein are compounds having formula (I) as shown below: 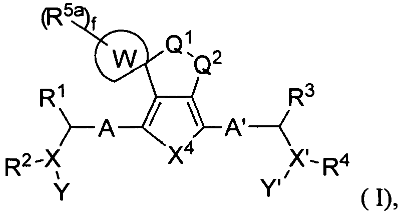 or a stereoisomer, a geometric isomer, a tautomer, an N-oxide, a hydrate, a solvate, a metabolite, a pharmaceutically acceptable salt or a prodrug thereof, wherein:
each of A and A' is independently a bond, alkylene, alkenylene, cycloalkylene, heterocyclylalkylene, -(CR⁸R^{8a})ₙ-O-(CR⁸R^{8a})ₚ-, -(CR⁸R^{8a})ₙ-N(R⁵)-(CR⁸R^{8a})ₚ-, -(CR⁸R^{8a})ₙ-S(=O)ᵣ-N(R⁵)-(CR⁸R^{8a})ₚ-, -(CR⁸R^{8a})ₙ-C(=O)-N(R⁵)-(CR⁸R^{8a})ₚ-, -(CR⁸R^{8a})ₙ-N(R⁵)-C(=O)-N(R⁵)-(CR⁸R^{8a})ₚ-, -(CR⁸R^{8a})ₙ-C(=O)-O-(CR⁸R^{8a})ₚ-, -(CR⁸R^{8a})ₙ-N(R⁵)-S(=O)ᵣ-N(R⁵)-(CR⁸R^{8a})ₚ- or -(CR⁸R^{8a})ₙ-N(R⁵)-C(=O)-O-(CR⁸R^{8a})ₚ-; or each of A and A' is independently 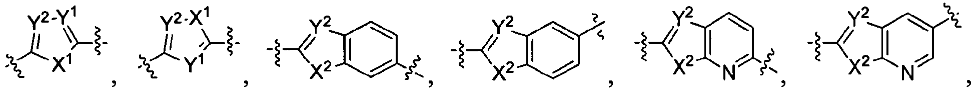 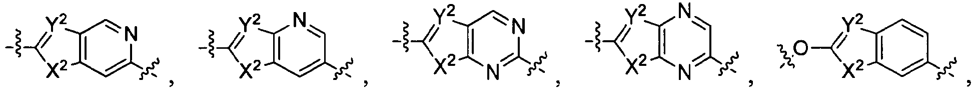 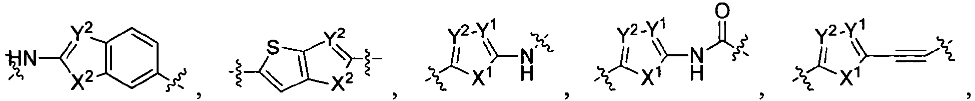 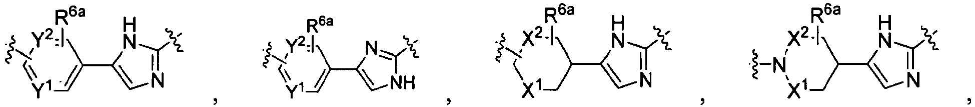 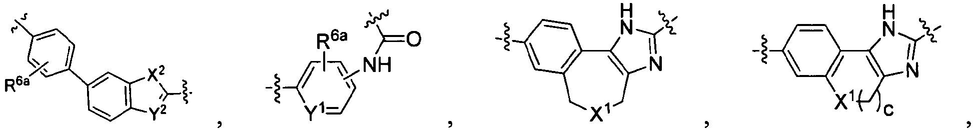 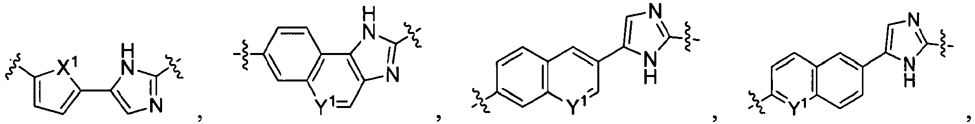 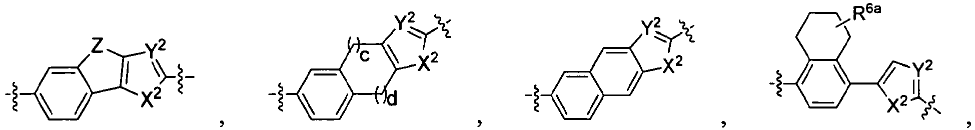 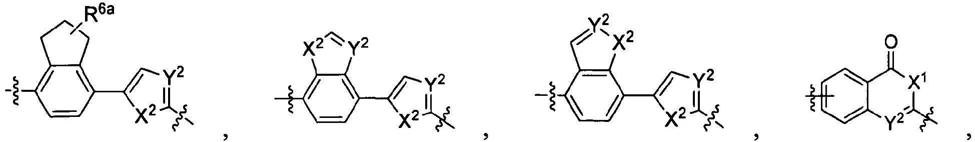 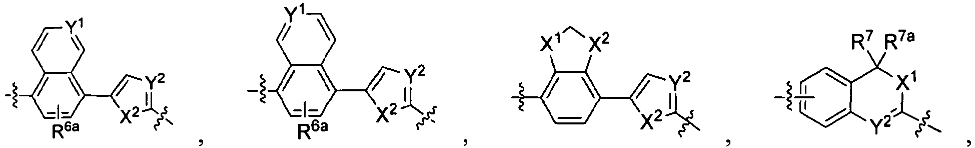 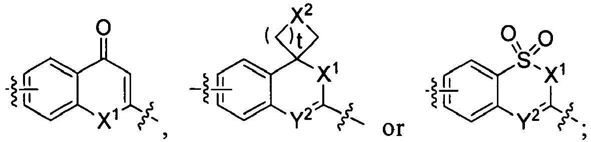
wherein each X¹ is independently O, S, NR⁶ or CR⁷R^{7a};
each X² is independently NR⁶, O or S;
X⁴ is (CR⁷R^{7a})ₙ, -Y¹=Y²-, O, S or NR⁶;
W is carbocyclyl or heterocyclyl;
each Y¹ and Y² is independently N or CR⁷;
each Z is independently -(CH₂)ₐ-, -CH=CH-, -N=CH-, -(CH₂)ₐ-N(R⁵)-(CH₂)_{b}- or -(CH₂)ₐ-O-(CH₂)_{b}-, wherein each a and b is independently 0, 1, 2 or 3;
each c is independently 1 or 2;
each d is independently 1 or 2;
each n is independently 0, 1, 2 or 3;
each p is independently 0, 1, 2 or 3;
each r is independently 0, 1 or 2;
f is 0, 1, 2, 3 or 4;
each of Q¹ and Q² is independently NR⁶, O, S, C(=O) or (CR⁷R^{7a})ₑ;
e is 0, 1, 2, 3 or 4;
each of X and X' is independently N or CR⁷;
each of Y and Y' is independently H, deuterium, alkyl, heteroalkyl, cycloalkyl, heterocyclyl, aryl, heteroaryl, aralkyl, a group derived from α-amino acid or an optically isomer thereof; or each of Y and Y' is independently -[U-(CR⁹R^{9a})ₜ-N(R¹⁰)-(CR⁹R^{9a})ₜ]ₖ-U-(CR⁹R^{9a})ₜ-N(R¹¹)-(CR⁹R^{9a})ₜ-R¹², -U-(CR⁹R^{9a})ₜ-R¹² or -[U-(CR⁹R^{9a})ₜ-N(R¹⁰)-(CR⁹R^{9a})ₜ]ₖ-U-(CR⁹R^{9a})ₜ-O-(CR⁹R^{9a})ₜ-R¹²;
each U is independently -C(=O)-, -C(=S)-, -S(=O)- or -S(=O)₂-;
each t is independently 0, 1, 2, 3 or 4;
each k is independently 0, 1 or 2;
each of R¹, R², R³ and R⁴ is independently H, deuterium, alkyl, heteroalkyl, aralkyl, cycloalkyl, heterocyclyl, heteroaryl or aryl; or R¹ and R², together with X-CH they are attached to, optionally form a 3-8 membered heterocycle or carbocycle, C₅₋₁₂ fused bicycle, C₅₋₁₂ fused heterobicycle, C₅₋₁₂ spiro bicycle or C₅₋₁₂ spiro heterobicycle; or R³ and R⁴, together with X'-CH they are attached to, optionally form a 3-8 membered heterocycle or carbocycle, C₅₋₁₂ fused bicycle, C₅₋₁₂ fused heterobicycle, C₅₋₁₂ spiro bicycle or C₅₋₁₂ spiro heterobicycle;
each R⁵ is independently H, deuterium, hydroxy, alkyl, heteroalkyl, cycloalkyl, heterocyclyl, aryl, heteroaryl, aralkyl, alkoxy, alkyl-OC(=O)-, alkyl-C(=O)-, carbamoyl, alkyl-OS(=O)ᵣ-, alkyl-S(=O)ᵣO-, alkyl-S(=O)ᵣ- or aminosulfonyl;
each R^{5a} is independently H, deuterium, oxo (=O), hydroxy, amino, F, Cl, Br, I, cyano, R^{13a}R¹³N-, -C(=O)NR¹³R^{13a}, -OC(=O)NR¹³R^{13a}, -OC(=O)OR¹³, -N(R¹³)C(=O)NR¹³R^{13a}, -N(R¹³)C(=O)OR¹³, -N(R¹³)C(=O)-R¹³, R¹³R^{13a}N-S(=O)₂-, R¹³S(=O)₂-, R¹³S(=O)₂N(R^{13a})-, R^{13a}R¹³N-alkyl, R¹³S(=O)-alkyl, R¹³R^{13a}N-C(=O)-alkyl, R^{13a}R¹³N-alkoxy, R¹³S(=O)-alkoxy, R¹³R^{13a}N-C(=O)-alkoxy, aryl, heteroaryl, alkoxy, alkylamino, alkyl, haloalkyl, alkenyl, alkynyl, heterocyclyl, cycloalkyl, mercapto, nitro, aralkyl, arylamino, heteroarylamino, arylalkylamino, heteroarylalkylamino, heteroaryloxy, heteroarylalkyl, arylalkoxy, heteroarylalkoxy, heterocyclyloxy, heterocyclylalkoxy, heterocyclylamino, heterocyclylalkylamino or aryloxy;
each R⁶ is independently H, deuterium, R¹³R^{13a}NC(=O)-, R¹³OC(=O)-, R¹³C(=O)-, R¹³R^{13a}NS(=O)-, R¹³OS(=O)-, R¹³S(=O)-, R¹³R^{13a}NS(=O)₂-, R¹³OS(=O)₂-, R¹³S(=O)₂-, aliphatic, haloaliphatic, hydroxyaliphatic, aminoaliphatic, alkoxyaliphatic, alkylaminoaliphatic, alkylthioaliphatic, arylaliphatic, heteroarylaliphatic, heterocyclylaliphatic, cycloalkylaliphatic, aryloxyaliphatic, heterocyclyloxyaliphatic, cycloalkyloxyaliphatic, arylaminoaliphatic, heterocyclylaminoaliphatic, cycloalkylaminoaliphatic, aryl, heteroaryl, heterocyclyl or carbocyclyl;
each R^{6a} is independently H, deuterium, hydroxy, amino, F, Cl, Br, I, cyano, oxo (=O), R^{13a}R¹³N-, -C(=O)NR¹³R^{13a}, -OC(=O)NR¹³R^{13a}, -OC(=O)OR¹³, -N(R¹³)C(=O)NR¹³R^{13a}, -N(R¹³)C(=O)OR^{13a}, -N(R¹³)C(=O)-R^{13a}, R¹³R^{13a}N-S(=O)₂-, R¹³S(=O)₂-, R¹³S(=O)₂N(R^{13a})-, R^{13a}R¹³N-alkyl, R¹³S(=O)-alkyl, R¹³R^{13a}N-C(=O)-alkyl, R^{13a}R¹³N-alkoxy, R¹³S(=O)-alkoxy, R¹³R^{13a}N-C(=O)-alkoxy, aryl, heteroaryl, alkoxy, alkylamino, alkyl, haloalkyl, alkenyl, alkynyl, heterocyclyl, cycloalkyl, mercapto, nitro, aralkyl, arylamino, heteroarylamino, arylalkylamino, heteroarylalkylamino, heteroaryloxy, heteroarylalkyl, arylalkoxy, heteroarylalkoxy, heterocyclyloxy, heterocyclylalkoxy, heterocyclylamino, heterocyclylalkylamino or aryloxy;
each R⁷ and R^{7a} is independently H, deuterium, F, Cl, Br, I, aliphatic, heteroalkyl, haloaliphatic, hydroxyaliphatic, aminoaliphatic, alkoxyaliphatic, alkylaminoaliphatic, alkylthioaliphatic, arylaliphatic, heteroarylaliphatic, heterocyclylaliphatic, cycloalkylaliphatic, aryloxyaliphatic, heterocyclyloxyaliphatic, cycloalkyloxyaliphatic, arylaminoaliphatic, heterocyclylaminoaliphatic, cycloalkylaminoaliphatic, aryl, heteroaryl, heterocyclyl or carbocyclyl;
each R⁸ and R^{8a} is independently H, deuterium, hydroxy, cyano, nitro, F, Cl, Br, I, alkyl, heteroalkyl, cycloalkyl, heterocyclyl, aryl, heteroaryl, aralkyl, alkoxy, alkyl-OC(=O)-, alkyl-C(=O)-, carbamoyl, alkyl-OS(=O)ᵣ-, alkyl-S(=O)ᵣO-, alkyl-S(=O)ᵣ- or aminosulfonyl;
each R⁹, R^{9a}, R¹⁰ and R¹¹ is independently H, deuterium, alkyl, heteroalkyl, cycloalkyl, heterocyclyl, aryl, heteroaryl, aralkyl, haloalkyl, hydroxyalkyl, heteroarylalkyl, heterocyclylalkyl or cycloalkylalkyl;
each R¹² is independently R^{13a}R¹³N-, -C(=O)R¹³, -C(=S)R¹³, -C(=O)OR¹³, -C(=O)NR¹³R^{13a}, -OC(=O)NR¹³R^{13a},-OC(=O)OR¹³, -N(R¹³)C(=O)NR¹³R^{13a}, -N(R¹³)C(=O)OR^{13a}, -N(R¹³)C(=O)-R^{13a}, R¹³R^{13a}N-S(=O)₂-, R¹³S(=O)₂-, R¹³S(=O)₂N(R^{13a})-, R¹³OS(=O)₂-, alkyl, heteroalkyl, cycloalkyl, heterocyclyl, aryl, heteroaryl or aralkyl;
or R¹¹ and R¹² are optionally joined to form a 4-7 membered ring; and
each R¹³ and R^{13a} is independently H, deuterium, alkyl, heteroalkyl, cycloalkyl, heterocyclyl, aryl, heteroaryl or aralkyl, with the proviso that where R¹³ and R^{13a} are bonded to the same nitrogen atom, R¹³ and R^{13a}, together with the nitrogen atom they are attached to, optionally form a substituted or unsubstituted 3-8 membered ring or a substituted or unsubstituted spiro or fused bicyclic ring;
wherein each of alkylene, alkenylene, cycloalkylene, heterocyclylalkylene, -(CR⁸R^{8a})ₙ-O-(CR⁸R^{8a})ₚ-, -(CR⁸R^{8a})ₙ-N(R⁵)-(CR⁸R^{8a})ₚ-, -(CR⁸R^{8a})ₙ-S(=O)ᵣ-N(R⁵)-(CR⁸R^{8a})ₚ-, -(CR⁸R^{8a})ₙ-C(=O)-N(R⁵)-(CR⁸R^{8a})ₚ-, -(CR⁸R^{8a})ₙ-N(R⁵)-C(=O)-N(R⁵)-(CR⁸R^{8a})ₚ-, -(CR⁸R^{8a})ₙ-C(=O)-O-(CR⁸R^{8a})ₚ-, -(CR⁸R^{8a})ₙ-N(R⁵)-S(=O)ᵣ-N(R⁵)-(CR⁸R^{8a})ₚ-, -(CR⁸R^{8a})ₙ-N(R⁵)-C(=O)-O-(CR⁸R^{8a})ₚ-, -[U-CR⁹R^{9a})ₜ-N(R¹⁰)-(CR⁹R^{9a})ₜ]ₖ-U-(CR⁹R^{9a})ₜ-N(R¹¹)-(CR⁹R^{9a})ₜ-R¹², -U-(CR⁹R^{9a})ₜ-R¹², -[U-(CR⁹R^{9a})ₜ-N(R¹⁰)-(CR⁹R^{9a})ₜ]ₖ-U-(CR⁹R^{9a})ₜ-O-(CR⁹R^{9a})ₜ-R¹², NR⁶, CR⁷R^{7a}, CR⁷, -(CH₂)ₐ-, -CH=CH-, -N=CH-, -(CH₂)ₐ-N(R⁵)-(CH₂)_{b}-, -(CH₂)ₐ-O-₍CH₂)_{b}-, R^{13a}R¹³N-, -C(=O)R¹³, -C(=S)R¹³, -C(=O)OR¹³, -C(=O)NR¹³R^{13a}, -OC(=O)NR¹³R^{13a}, -OC(=O)OR¹³, -N(R¹³)C(=O)NR¹³R^{13a}, -N(R¹³)C(=O)OR^{13a}, -N(R¹³)C(=O)-R^{13a}, R¹³R^{13a}N-S(=O)₂-, R¹³S(=O)₂-, R¹³S(=O)₂N(R^{13a})-, R¹³OS(=O)₂-, alkyl-OC(=O)-, alkyl-C(=O)-, alkyl-OS(=O)ᵣ-, alkyl-S(=O)ᵣO-, alkyl-S(=O)ᵣ-, R¹³R¹³aNC(=O)-, R¹³OC(=O)-, R¹³C(=O)-, R¹³R^{13a}NS(=O)-, R¹³OS(=O)-, R¹³S(=O)-, R^{13a}R¹³N-alkyl, R¹³S(=O)-alkyl, R¹³R^{13a}N-C(=O)-alkyl, R^{13a}R¹³N-alkoxy, R¹³S(=O)-alkoxy, R¹³R^{13a}N-C(=O)-alkylamino, alkyl, heteroalkyl, carbocyclyl, cycloalkyl, heterocyclyl, aryl, heteroaryl, aralkyl, a group derived from α-amino acid, C₅₋₁₂ fused bicycle, C₅₋₁₂ fused heterobicycle, C₅₋₁₂ spiro bicycle, C₅₋₁₂ spiro heterobicycle, alkoxy, aliphatic, haloaliphatic, hydroxyaliphatic, aminoaliphatic, alkoxyaliphatic, alkylaminoaliphatic, alkylthioaliphatic, arylaliphatic, heteroarylaliphatic, heterocyclylaliphatic, cycloalkylaliphatic, aryloxyaliphatic, heterocyclyloxyaliphatic, cycloalkyloxyaliphatic, arylaminoaliphatic, heterocyclylaminoaliphatic, cycloalkylaminoaliphatic, haloalkyl, alkenyl, alkynyl, arylamino, heteroarylamino, arylalkylamino, heteroarylalkylamino, heteroaryloxy, heteroarylalkyl, arylalkoxy, heteroarylalkoxy, heterocyclyloxy, heterocyclylalkoxy, heterocyclylamino, heterocyclylalkylamino and aryloxy is optionally substituted with one or more substituents, wherein the substituent is deuterium, hydroxy, amino, halo, cyano, aryl, heteroaryl, alkoxy, alkylamino, alkylthio, alkyl, alkenyl, alkynyl, heterocyclyl, mercapto, nitro, aryloxy, heteroaryloxy, oxo (=O), carboxy, hydroxy-substituted alkoxy, hydroxy-substituted alkyl-C(=O)-, alkyl-C(=O)-, alkyl-S(=O)-, alkyl-S(=O)₂-, hydroxy-substituted alkyl-S(=O)-, hydroxy-substituted alkyl-S(=O)₂- or carboxy-substituted alkoxy.

In some embodiments, W is C₃₋₈ carbocyclyl or C₂₋₁₀ heterocyclyl.

In some embodiments, 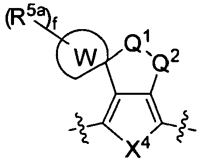 is 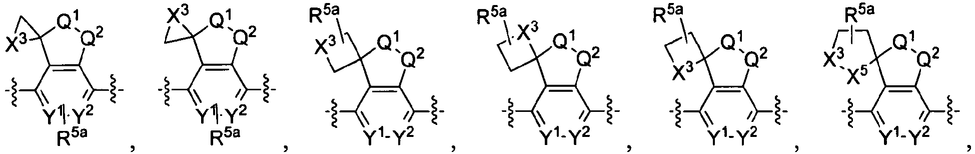 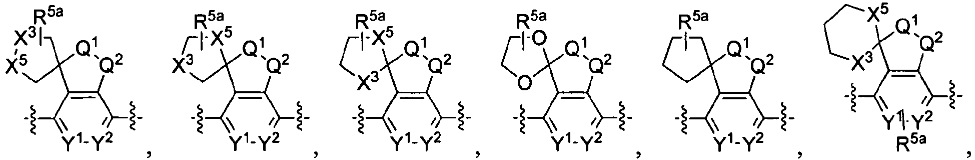 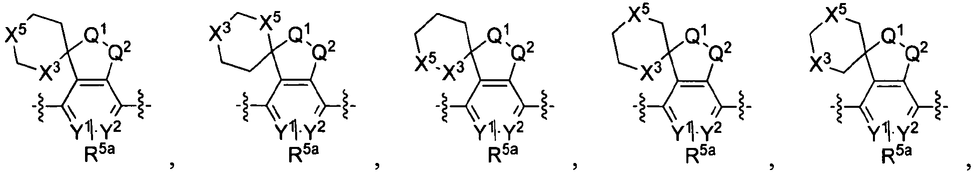 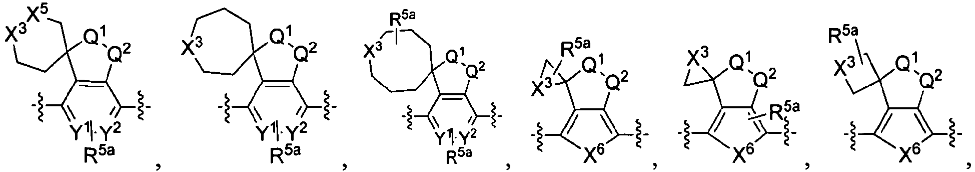 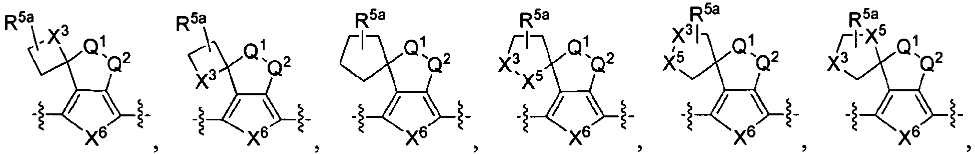 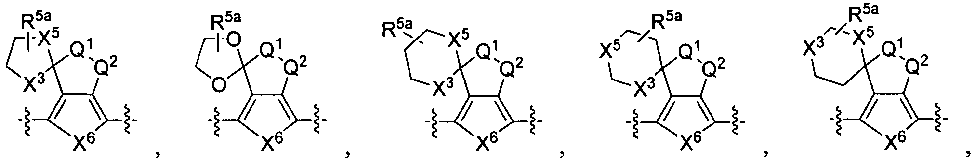 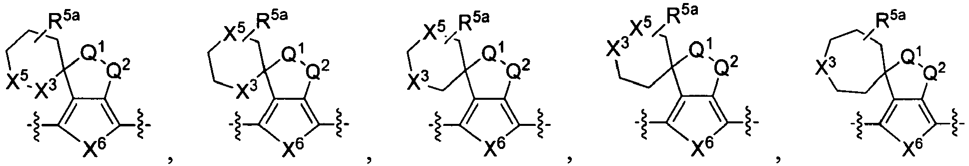 or 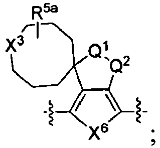
wherein each X³ and X⁵ is independently O, S, NR⁶ or CR⁷R^{7a};
each X⁶ is independently CR⁷R^{7a}, O, S or NR⁶;
each Y¹ and Y² is independently N or CR⁷;
f is 0, 1, 2, 3 or 4;
each Q¹ and Q² is independently NR⁶, O, S, C(=O) or (CR⁷R^{7a})ₑ;
e is 0, 1, 2, 3 or 4;
each R^{5a} is independently H, deuterium, oxo (=O), hydroxy, amino, F, Cl, Br, I, cyano, C₁₋₆ alkylacyl, C₁₋₆ alkylacyloxy, C₁₋₆ alkoxyacyl, C₁₋₆ alkylsulfonyl, C₁₋₆ alkoxysulfonyl, C₁₋₆ alkylsulfinyl, C₁₋₆ alkylsulfonyloxy, C₁₋₆ alkylsulfinyloxy, C₁₋₆ alkoxy, C₁₋₆ alkyl, C₆₋₁₀ aryl, -CF₃, -OCF₃, mercapto, nitro, C₁₋₆ alkylamino, C₃₋₁₀ cycloalkyl or C₆₋₁₀ aryloxy;
each R⁶ is independently H, deuterium, R¹³R^{13a}NC(=O)-, R¹³OC(=O)-, R¹³C(=O)-, R¹³R^{13a}NS(=O)-, R¹³OS(=O)-, R¹³S(=O)-, R¹³R^{13a}NS(=O)₂-, R¹³OS(=O)₂-, R¹³S(=O)₂-, C₁₋₆ alkyl, C₁₋₆ haloalkyl, C₁₋₆ hydroxyalkyl, C₁₋₆ aminoalkyl, C₁₋₆ alkoxy-C₁₋₆-alkyl, C₁₋₆ alkylainino-C₁₋₆-alkyl, C₁₋₆ alkylthio-C₁₋₆-alkyl, C₆₋₁₀ aryl-C₁₋₆-alkyl, C₁₋₉ heteroaryl-C₁₋₆-alkyl, C₂₋₁₀ heterocyclyl-C₁₋₆-alkyl, C₃₋₁₀ cycloalkyl-C₁₋₆-alkyl, C₆₋₁₀ aryloxy-C₁₋₆-alkyl, C₂₋₁₀ heterocyclyloxy-C₁₋₆-alkyl, C₃₋₁₀ cycloalkyloxy-C₁₋₆-alkyl, C₆₋₁₀ arylamino-C₁₋₆-alkyl, C₂₋₁₀ heterocyclylamino-C₁₋₆-alkyl, C₃₋₁₀ cycloalkylamino-C₁₋₆-alkyl, C₆₋₁₀ aryl, C₁₋₉ heteroaryl, C₂₋₁₀ heterocyclyl or C₃₋₁₀ carbocyclyl;
each R⁷ and R^{7a} is independently H, deuterium, F, Cl, Br, I, C₁₋₆ alkyl, C₂₋₆ heteroalkyl, C₁₋₆ haloalkyl, C₁₋₆ hydroxyalkyl, C₁₋₆ aminoalkyl, C₁₋₆ alkoxy-C₁₋₆-alkyl, C₁₋₆ alkylamino-C₁₋₆-alkyl, C₁₋₆ alkylthio-C₁₋₆-alkyl, C₆₋₁₀ aryl-C₁₋₆-alkyl, C₁₋₉ heteroaryl-C₁₋₆-alkyl, C₂₋₁₀ heterocyclyl-C₁₋₆-alkyl, C₃₋₁₀ cycloalkyl-C₁₋₆-alkyl, C₆₋₁₀ aryloxy-C₁₋₆-alkyl, C₂₋₁₀ heterocyclyloxy-C₁₋₆-alkyl, C₃₋₁₀ cycloalkyloxy-C₁₋₆-alkyl, C₆₋₁₀ arylamino-C₁₋₆-alkyl, C₂₋₁₀ heterocyclylamino-C₁₋₆-alkyl, C₃₋₁₀ cycloalkylamino-C₁₋₆-alkyl, C₆₋₁₀ aryl, C₁₋₉ heteroaryl, C₂₋₁₀ heterocyclyl or C₃₋₁₀ carbocyclyl; and
each R¹³ and R^{13a} is independently H, deuterium, C₁₋₆ alkyl, C₂₋₆ heteroalkyl, C₃₋₁₀ cycloalkyl, C₂₋₁₀ heterocyclyl, C₆₋₁₀ aryl, C₁₋₉ heteroaryl or C₆₋₁₀ aryl-C₁₋₆-alkyl; with the proviso that where R¹³ and R^{13a} are bonded to the same nitrogen atom, R¹³ and R^{13a}, together with the nitrogen atom they are attached to, optionally form a substituted or unsubstituted 3-8 membered ring or a substituted or unsubstituted spiro or fused bicyclic ring.

In some embodiments, 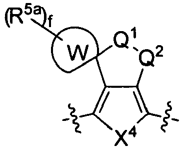 is 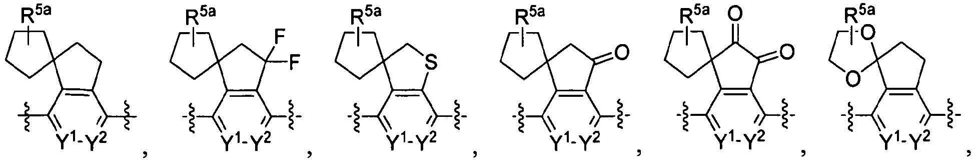 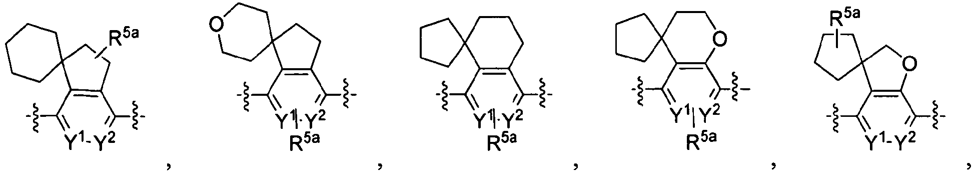 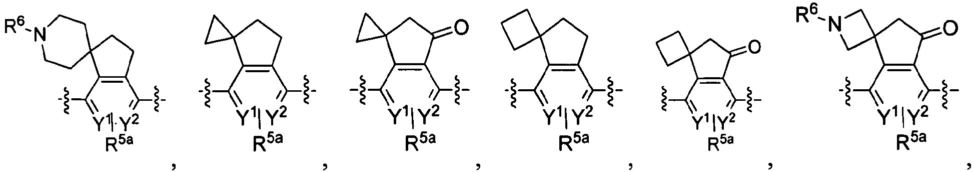 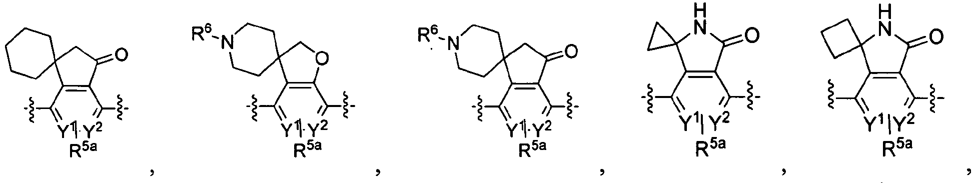 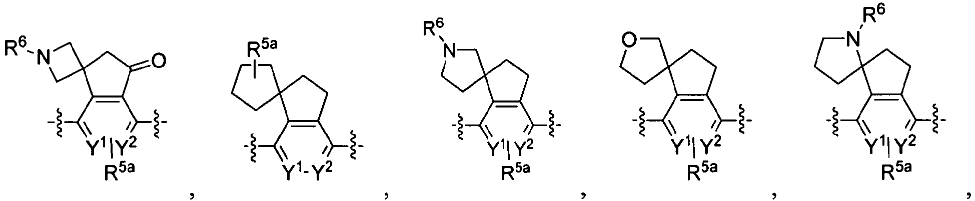 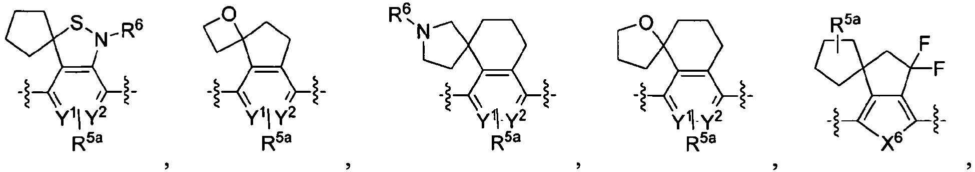 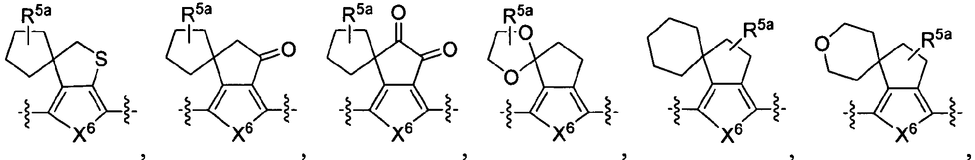 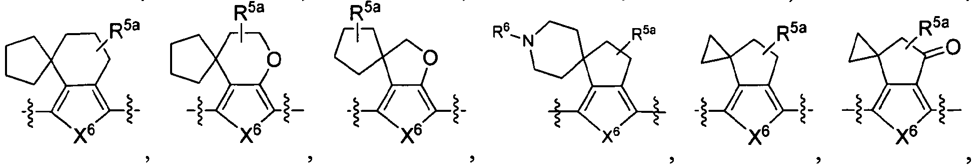 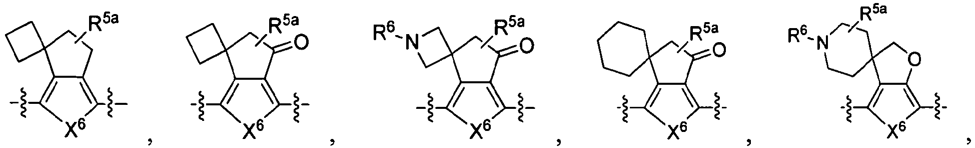 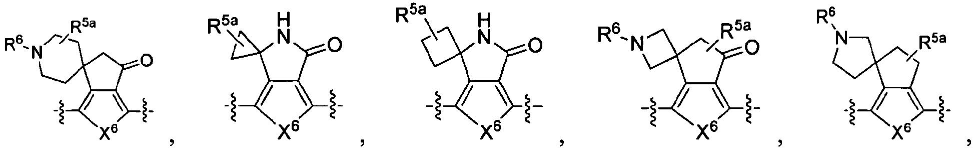 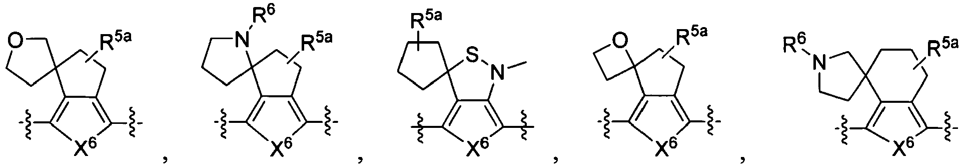 or 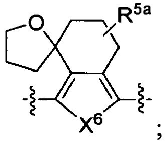
wherein each Y¹ and Y² is independently N or CR⁷;
each X⁶ is independently CR⁷R^{7a}, O, S or NR⁶;
each R^{5a} is independently H, deuterium, oxo (=O), hydroxy, amino, F, Cl, Br, I, cyano, C₁₋₆ alkylacyl, C₁₋₆ alkylacyloxy, C₁₋₆ alkoxyacyl, C₁₋₆ alkylsulfonyl, C₁₋₆ alkoxysulfonyl, C₁₋₆ alkylsulfinyl, C₁₋₆ alkylsulfonyloxy, C₁₋₆ alkylsulfinyloxy, C₁₋₆ alkoxy, C₁₋₆ alkyl, C₆₋₁₀ aryl, -CF₃, -OCF₃, mercapto, nitro or C₁₋₆ alkylamino;
each R⁶ is independently H, deuterium, C₁₋₆ alkyl, C₁₋₆ haloalkyl, C₁₋₆ hydroxyalkyl, C₁₋₆ aminoalkyl, C₁₋₆ alkoxy-C₁₋₆-alkyl, C₁₋₆ alkylamino-C₁₋₆-alkyl, C₁₋₆ alkylthio-C₁₋₆-alkyl, C₆₋₁₀ aryl-C₁₋₆-alkyl, C₁₋₉ heteroaryl-C₁₋₆-alkyl, C₂₋₁₀ heterocyclyl-C₁₋₆-alkyl or C₃₋₈ cycloalkyl-C₁₋₆-alkyl; and
each R⁷ and R^{7a} is independently H, deuterium, F, Cl, Br, I, C₁₋₆ alkyl, C₂₋₆ heteroalkyl, C₁₋₆ haloalkyl, C₁₋₆ hydroxyalkyl, C₁₋₆ aminoalkyl, C₁₋₆ alkoxy-C₁₋₆-alkyl, C₁₋₆ alkylamino-C₁₋₆-alkyl, C₁₋₆ alkylthio-C₁₋₆-alkyl, C₆₋₁₀ aryl-C₁₋₆-alkyl, C₂₋₁₀ heterocyclyl-C₁₋₆-alkyl, C₃₋₁₀ cycloalkyl-C₁₋₆-alkyl, C₆₋₁₀ aryloxy-C₁₋₆-alkyl, C₂₋₁₀ heterocyclyloxy-C₁₋₆-alkyl, C₃₋₁₀ cycloalkyloxy-C₁₋₆-alkyl, C₆₋₁₀ arylamino-C₁₋₆-alkyl, C₂₋₁₀ heterocyclylamino-C₁₋₆-alkyl, C₃₋₁₀ cycloalkylamino-C₁₋₆-alkyl, C₆₋₁₀ aryl, C₁₋₉ heteroaryl, C₂₋₁₀ heterocyclyl or C₃₋₈ carbocyclyl.

In some embodiments, each of A and A' is independently a bond, C₁₋₆ alkylene, C₂₋₆ alkenylene, C₃₋₈ cycloalkylene, C₂₋₁₀ heterocyclylalkylene, -(CR⁸R^{8a})ₙ-O-(CR⁸R^{8a})ₚ-, -(CR⁸R^{8a})ₙ-N(R⁵)-(CR⁸R^{8a})ₚ-, -(CR⁸R^{8a})ₙ-S(=O)ᵣ-N(R⁵)-(CR⁸R^{8a})ₚ-, -(CR⁸R^{8a})ₙ-C(=O)-N(R⁵)-(CR⁸R^{8a})ₚ-, -(CR⁸R^{8a})ₙ-N(R⁵)-C(=O)-N(R⁵)-(CR⁸R^{8a})ₚ-, -(CR⁸R^{8a})ₙ-C(=O)-O-(CR⁸R^{8a})ₚ-, -(CR⁸R^{8a})ₙ-N(R⁵)-S(=O)ᵣ-N(R⁵)-(CR⁸R^{8a})ₚ- or -(CR⁸R^{8a})ₙ-N(R⁵)-C(=O)-O-(CR⁸R^{8a})ₚ-; or each of A and A' is independently 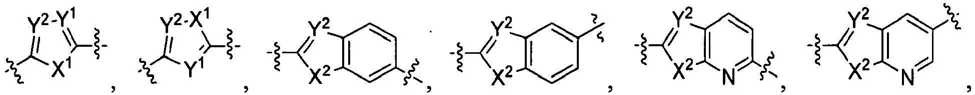 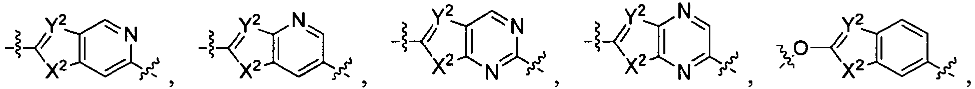 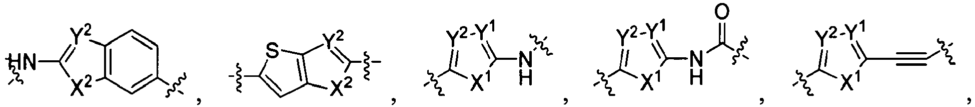 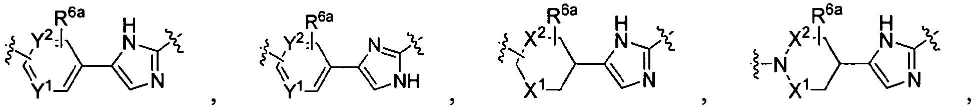 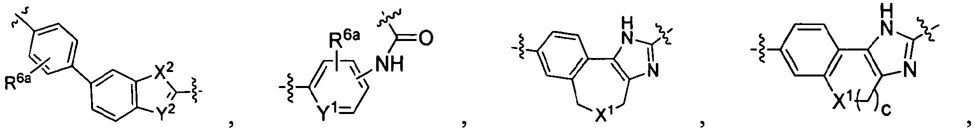 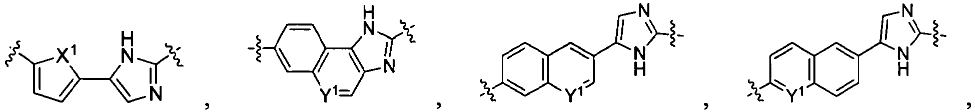 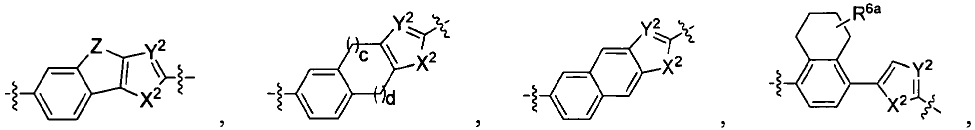 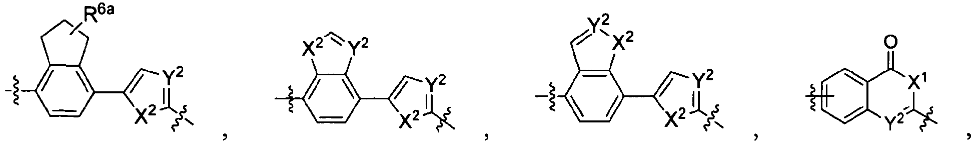 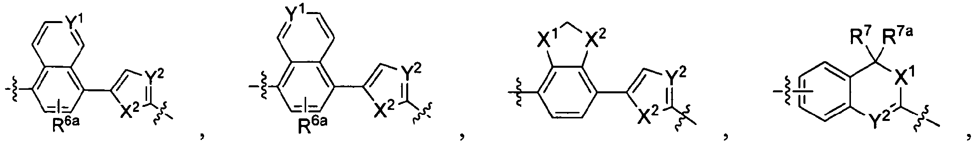 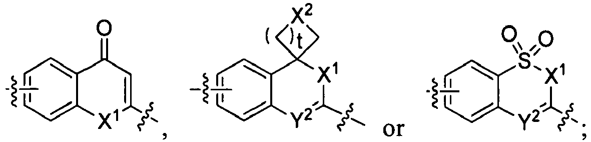
wherein each X¹ is independently O, S, NR⁶ or CR⁷R^{7a};
each X² is independently NR⁶, O or S;
each Y¹ and Y² is independently N or CR⁷;
each c is independently 1 or 2;
each d is independently 1 or 2;
each n is independently 0, 1, 2 or 3;
each p is independently 0, 1, 2 or 3;
each r is independently 0, 1 or 2;
each R⁵ is independently H, deuterium, hydroxy, C₁₋₆ alkyl, C₂₋₆ heteroalkyl, C₃₋₈ cycloalkyl, C₂₋₁₀ heterocyclyl, C₆₋₁₀ aryl, C₁₋₉ heteroaryl, C₆₋₁₀ aryl-C₁₋₆-alkyl, C₁₋₆ alkoxy, C₁₋₆ alkyl-OC(=O)-, C₁₋₆ alk yl-C(=O)-, carbamoyl, C₁₋₆ alk yl-OS(=O)ᵣ-, C₁₋₆ alk yl-S(=O)ᵣO-, C₁₋₆ alk yl-S(=O)ᵣ- o r aminosulfonyl;
each R⁶ is independently H, deuterium, R¹³R^{13a}NC(=O)-, R¹³OC(=O)-, R¹³C(=O)-, R¹³R^{13a}NS(=O)-, R¹³OS(=O)-, R¹³S(=O)-, R¹³R^{13a}NS(=O)₂-, R¹³OS(=O)₂-, R¹³S(=O)₂-, C₁₋₆ alkyl, C₁₋₆ haloalkyl, C₁₋₆ hydroxyalkyl, C₁₋₆ aminoalkyl, C₁₋₆ alkoxy-C₁₋₆-alkyl, C₁₋₆ alkylamino-C₁₋₆-alkyl, C₁₋₆ alkylthio-C₁₋₆-alkyl, C₆₋₁₀ aryl-C₁₋₆-alkyl, C₁₋₉ heteroaryl-C₁₋₆-alkyl, C₂₋₁₀ heterocyclyl-C₁₋₆-alkyl, C₃₋₁₀ cycloalkyl-C₁₋₆-alkyl, C₆₋₁₀ aryloxy-C₁₋₆-alkyl, C₂₋₁₀ heterocyclyloxy-C₁₋₆-alkyl, C₃₋₁₀ cycloalkyloxy-C₁₋₆-alkyl, C₆₋₁₀ arylamino-C₁₋₆-alkyl, C₂₋₁₀ heterocyclylamino-C₁₋₆-alkyl, C₃₋₁₀ cycloalkylamino-C₁₋₆-alkyl, C₆₋₁₀ aryl, C₁₋₉ heteroaryl, C₂₋₁₀ heterocyclyl or C₃₋₁₀ carbocyclyl;
each R^{6a} is independently H, deuterium, hydroxy, amino, F, Cl, Br, I, cyano, oxo (=O), R^{13a}R¹³N-, -C(=O)NR¹³R^{13a}, -OC(=O)NR¹³R^{13a}, -OC(=O)OR¹³, -N(R¹³)C(=O)NR¹³R^{13a}, -N(R¹³)C(=O)OR^{13a}, -N(R¹³)C(=O)-R^{13a}, R¹³R^{13a}N-S(=O)₂-, R¹³S(=O)₂-, R¹³S(=O)₂N(R^{13a})-, R^{13a}R¹³N-C₁₋₆ alkyl, R¹³S(=O)-C₁₋₆ alkyl, R¹³R^{13a}N-C(=O)-C₁₋₆ alkyl, R^{13a}R¹³N-C₁₋₆ alkoxy, R¹³S(=O)-C₁₋₆ alkoxy, R¹³R^{13a}N-C(=O)-C₁₋₆ alkoxy, C₆₋₁₀ aryl, C₁₋₉ heteroaryl, C₁₋₆ alkoxy, C₁₋₆ alkylamino, C₁₋₆ alkyl, C₁₋₆ haloalkyl, C₂₋₆ alkenyl, C₂₋₆ alkynyl, C₂₋₁₀ heterocyclyl, C₃₋₈ cycloalkyl, mercapto, nitro, C₆₋₁₀ aryl-C₁₋₆-alkyl, C₆₋₁₀ arylamino, C₁₋₉ heteroarylamino or C₆₋₁₀ aryloxy;
each R⁷ and R^{7a} is independently H, deuterium, F, Cl, Br, I, C₁₋₆ alkyl, C₂₋₆ heteroalkyl, C₁₋₆ haloalkyl, C₁₋₆ hydroxyalkyl, C₁₋₆ aminoalkyl, C₁₋₆ alkoxy-C₁₋₆-alkyl, C₁₋₆ alkylamino-C₁₋₆-alkyl, C₁₋₆ alkylthio-C₁₋₆-alkyl, C₆₋₁₀ aryl-C₁₋₆-alkyl, C₂₋₁₀ heterocyclyl-C₁₋₆-alkyl, C₃₋₁₀ cycloalkyl-C₁₋₆-alkyl, C₆₋₁₀ aryloxy-C₁₋₆-alkyl, C₂₋₁₀ heterocyclyloxy-C₁₋₆-alkyl, C₃₋₁₀ cycloalkyloxy-C₁₋₆-alkyl, C₆₋₁₀ arylamino-C₁₋₆-alkyl, C₂₋₁₀ heterocyclylamino-C₁₋₆-alkyl, C₃₋₁₀ cycloalkylamino-C₁₋₆-alkyl, C₆₋₁₀ aryl, C₁₋₉ heteroaryl, C₂₋₁₀ heterocyclyl or C₃₋₈ carbocyclyl;
each R¹³ and R^{13a} is independently H, deuterium, C₁₋₆ alkyl, C₂₋₆ heteroalkyl, C₃₋₁₀ cycloalkyl, C₂₋₁₀ heterocyclyl, C₆₋₁₀ aryl, C₁₋₉ heteroaryl or C₆₋₁₀ aryl-C₁₋₆-alkyl; with the proviso that where R¹³ and R^{13a} are bonded to the same nitrogen atom, R¹³ and R^{13a}, together with the nitrogen atom they are attached to, optionally form a substituted or unsubstituted 3-8 membered ring or a substituted or unsubstituted spiro or fused bicyclic ring; and
each R⁸ and R^{8a} is independently H, deuterium, hydroxy, cyano, nitro, F, Cl, Br, I, C₁₋₆ alkyl, C₂₋₆ heteroalkyl, C₃₋₁₀ cycloalkyl, C₂₋₁₀ heterocyclyl, C₆₋₁₀ aryl, C₁₋₉ heteroaryl, C₆₋₁₀ aryl-C₁₋₆-alkyl, C₁₋₆ alkoxy, C₁₋₆ alkyl-OC(=O)-, C₁₋₆ alkyl-C(=O)-, carbamoyl, C₁₋₆ alkyl-OS(=O)ᵣ-, C₁₋₆ alkyl-S(=O)ᵣO-, C₁₋₆ alkyl-S(=O)ᵣ- or aminosulfonyl.

In some embodiments, each of A and A' is independently a bond, -CH₂-, -(CH₂)₂-, -CH=CH-, -CH=CH-CH₂-, -N(R⁶)-, -C(=O)-, -C(=S)-, -C(=O)-O-, -C(=O)N(R⁶)-, -OC(=O)N(R⁶)-, -OC(=O)O-, -N(R⁶)C(=O)N(R⁶)-, -(R⁶)N-S(=O)₂-, -S(=O)₂-, -OS(=O)₂-, -(R⁶)N-S(=O)-, -S(=O)-, -OS(=O)-; or each of A and A' is independently 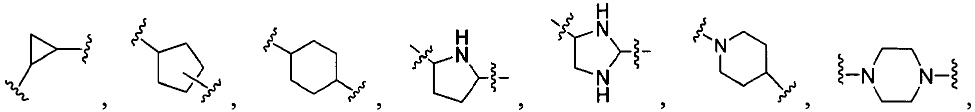 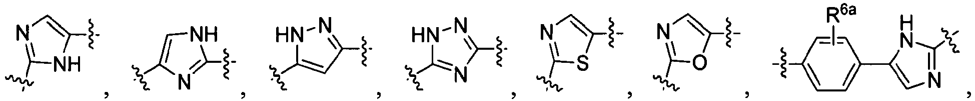 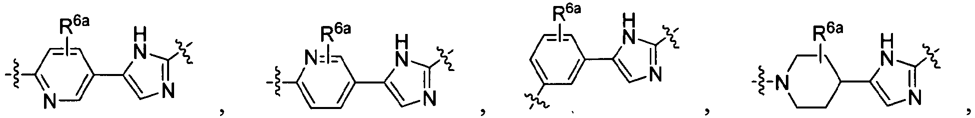 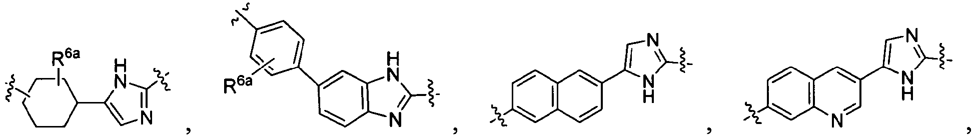 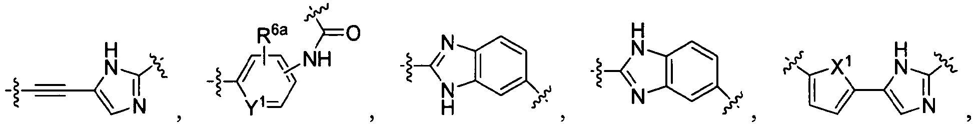 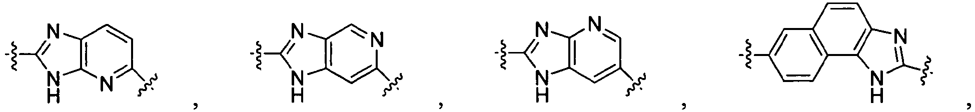 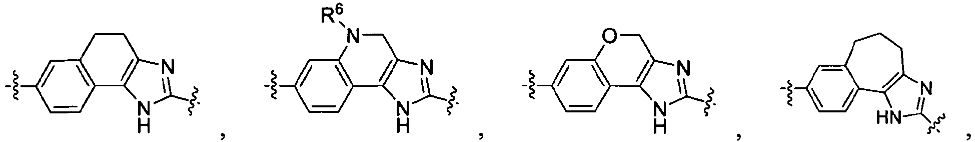 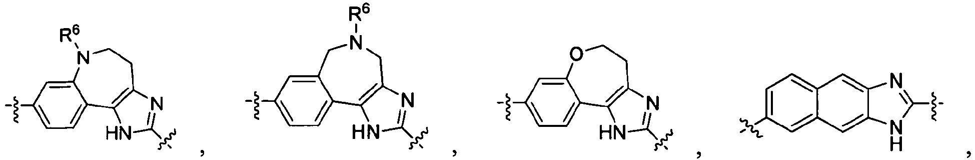 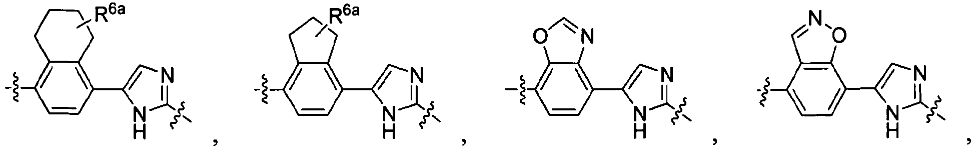 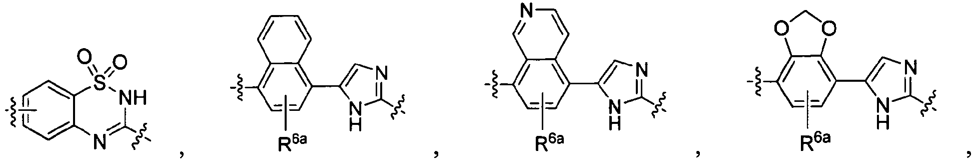 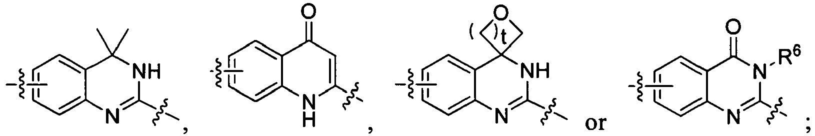
wherein X¹ is O or S;
each Y¹ independently is N or CR⁷;
each R⁶ is independently H, deuterium, C₁₋₄ alkyl, C₁₋₆ haloalkyl, C₁₋₆ hydroxyalkyl, C₁₋₆ aminoalkyl, C₁₋₆ alkoxy-C₁₋₄-alkyl, C₁₋₆ alkylamino-C₁₋₄-alkyl, C₁₋₆ alkylthio-C₁₋₄-alkyl, C₆₋₁₀ aryl-C₁₋₄-alkyl, C₁₋₉ heteroaryl, C₆₋₁₀ aryl, C₂₋₁₀ heterocyclyl or C₃₋₈ carbocyclyl;
each R^{6a} and R⁷ is independently H, deuterium, oxo (=O), hydroxy, amino, F, Cl, Br, I, cyano, R^{13a}R¹³N-, C₁₋₆ alkoxy, C₁₋₆ alkylamino, C₁₋₆ alkyl, C₁₋₆ haloalkyl, C₂₋₆ alkenyl, C₂₋₆ alkynyl, mercapto or nitro; and
each of R¹³ and R^{13a} is independently H, deuterium, C₁₋₆ alkyl, C₂₋₆ heteroalkyl, C₃₋₁₀ cycloalkyl, C₂₋₁₀ heterocyclyl, C₆₋₁₀ aryl, C₁₋₉ heteroaryl or C₆₋₁₀ aryl-C₁₋₆-alkyl; with the proviso that where R¹³ and R^{13a} are bonded to the same nitrogen atom, R¹³ and R^{13a}, together with the nitrogen atom they are attached to, optionally form a substituted or unsubstituted 3-8 membered ring or a substituted or unsubstituted spiro or fused bicyclic ring.

In some embodiments, each of R¹, R², R³ and R⁴ is independently H, deuterium, C₁₋₈ alkyl, C₁₋₈ heteroalkyl, C₆₋₁₀ aryl-C₁₋₆-alkyl, C₃₋₁₀ cycloalkyl, C₂₋₁₀ heterocyclyl, C₁₋₉ heteroaryl or C₆₋₁₀ aryl; or R¹ and R², together with X-CH they are attached to, optionally form a 3-8 membered heterocycle or carbocycle, C₅₋₁₂ fused bicycle, C₅₋₁₂ fused heterobicycle, C₅₋₁₂ spiro bicycle or C₅₋₁₂ spiro heterobicycle; or R³ and R⁴, together with X'-CH they are attached to, optionally form a 3-8 membered heterocycle or carbocycle, C₅₋₁₂ fused bicycle, C₅₋₁₂ fused heterobicycle, C₅₋₁₂ spiro bicycle or C₅₋₁₂ spiro heterobicycle.

In other embodiments, R¹ and R², together with X-CH they are attached to, or R³ and R⁴, together with X'-CH they are attached to, optionally form a 3-8 membered heterocycle, C₅₋₁₂ fused bicycle, C₅₋₁₂ fused heterobicycle, C₅₋₁₂ spiro bicycle or C₅₋₁₂ spiro heterobicycle.

In other embodiments, R¹, R² and X-CH together form one of the following monovalent groups: 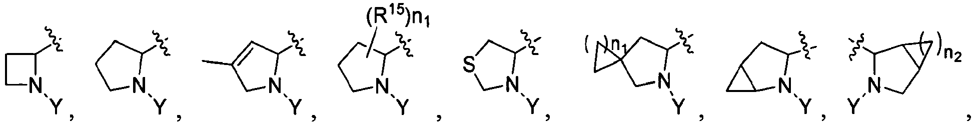 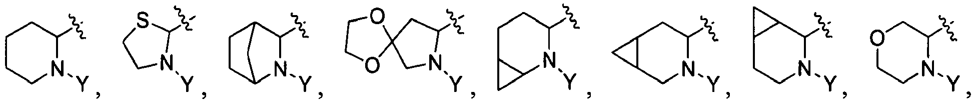 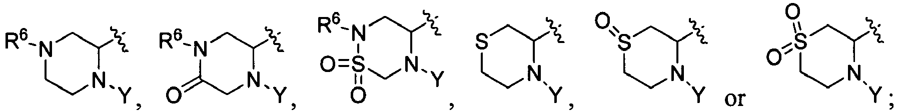
wherein each R¹⁵ is independently H, deuterium, F, Cl, Br, I, cyano, hydroxy, C₁₋₃ alkyl, C₁₋₃ haloalkyl, C₁₋₃ alkoxy, C₁₋₃ alkylamino, C₁₋₃ alkylthio, C₆₋₁₀ arylamino, C₆₋₁₀ aryloxy, C₁₋₉ heteroaryl, C₁₋₉ heteroaryloxy, C₁₋₉ heteroaryl-C₁₋₃-alkyl or C₂₋₁₀ heterocyclyl;
each R⁶ is independently H, deuterium, C₁₋₃ alkyl, C₁₋₃ haloalkyl, C₁₋₃ hydroxyalkyl, C₁₋₃ aminoalkyl, C₁₋₃ alkoxy-C₁₋₃-alkyl, C₁₋₃ alkylamino-C₁₋₃-alkyl, C₁₋₃ alkylthio-C₁₋₃-alkyl, C₆₋₁₀ aryl-C₁₋₃-alkyl, C₁₋₉ heteroaryl, C₆₋₁₀ aryl, C₂₋₁₀ heterocyclyl or C₃₋₈ carbocyclyl; and
each n₁ and n₂ is independently 1, 2, 3 or 4.

In other embodiments, R³, R⁴ and X'-CH together form one of the following monovalent groups: 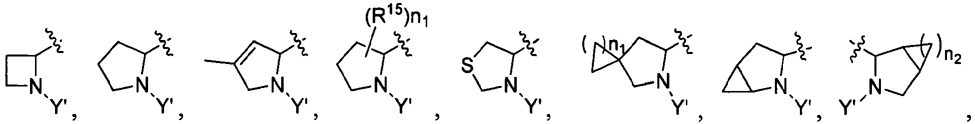 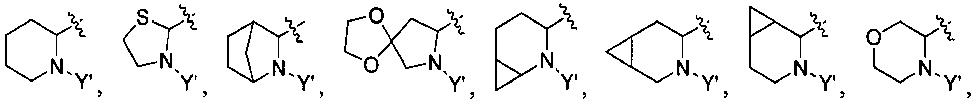 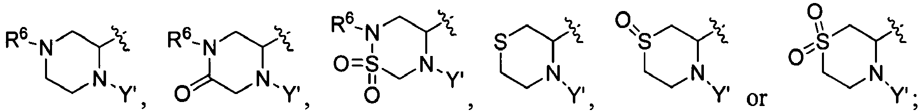
wherein each R¹⁵ is independently H, deuterium, F, Cl, Br, I, cyano, hydroxy, C₁₋₃ alkyl, C₁₋₃ haloalkyl, C₁₋₃ alkoxy, C₁₋₃ alkylamino, C₁₋₃ alkylthio, C₆₋₁₀ arylamino, C₆₋₁₀ aryloxy, C₁₋₉ heteroaryl, C₁₋₉ heteroaryloxy, C₁₋₉ heteroaryl-C₁₋₃-alkyl or C₂₋₁₀ heterocyclyl;
each R⁶ is independently H, deuterium, C₁₋₃ alkyl, C₁₋₃ haloalkyl, C₁₋₃ hydroxyalkyl, C₁₋₃ aminoalkyl, C₁₋₃ alkoxy-C₁₋₃-alkyl, C₁₋₃ alkylamino-C₁₋₃-alkyl, C₁₋₃ alkylthio-C₁₋₃-alkyl, C₆₋₁₀ aryl-C₁₋₃-alkyl, C₁₋₉ heteroaryl, C₆₋₁₀ aryl, C₂₋₁₀ heterocyclyl or C₃₋₈ carbocyclyl; and
each n₁ and n₂ is independently 1, 2, 3 or 4.

In some embodiments, the compound may have formula (II): 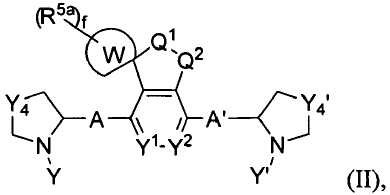 wherein 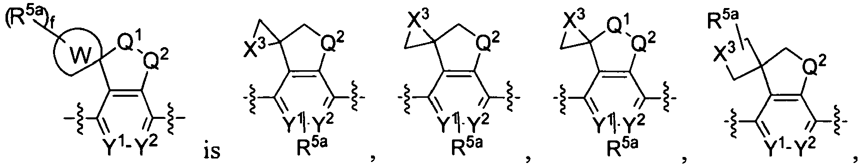 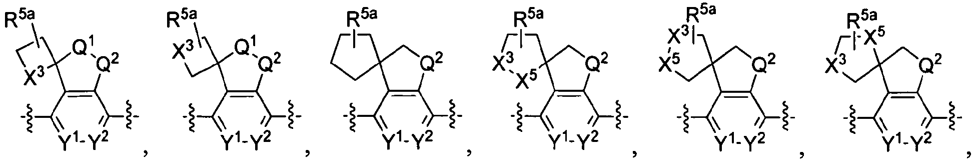 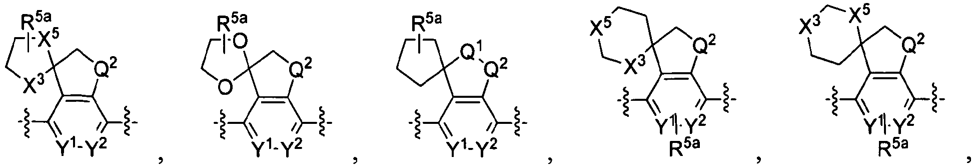 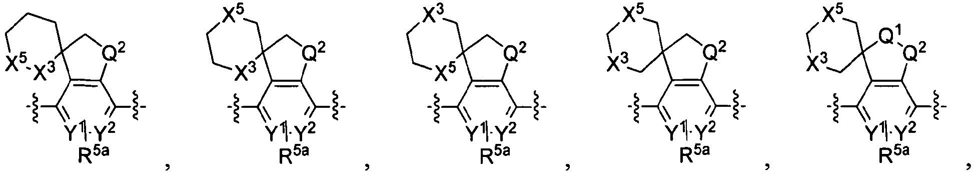 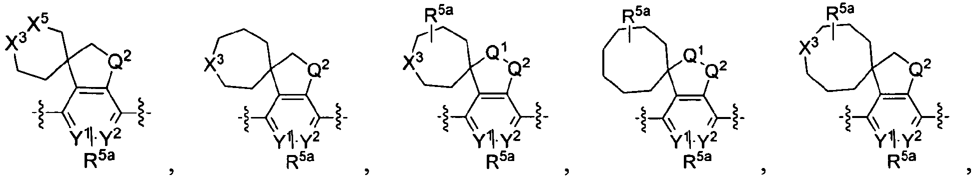 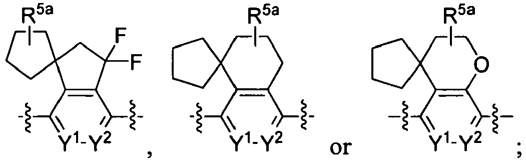
wherein each Q¹ and Q² is independently NR⁶, O, S, C(=O) or (CH₂)ₑ;
e is 0, 1, 2, 3 or 4;
each X¹, X³ and X⁵ is independently O, S, NR⁶ or CR⁷R^{7a};
each Y¹ and Y² is independently N or CR⁷;
each of A and A' is independently a bond, C₁₋₆ alkylene, C₂₋₆ alkenylene, C₃₋₈ cycloalkylene, C₂₋₁₀ heterocyclylalkylene, -(CR⁸R^{8a})ₙ-O-(CR⁸R^{8a})ₚ-, -(CR⁸R^{8a})ₙ-N(R⁵)-(CR⁸R^{8a})ₚ-, -(CR⁸R^{8a})ₙ-S(=O)ᵣ-N(R⁵)-(CR⁸R^{8a})ₚ-, -(CR⁸R^{8a})ₙ-C(=O)-N(R⁵)-(CR⁸R^{8a})ₚ-, -(CR⁸R^{8a})ₙ-N(R⁵)-C(=O)-N(R⁵)-(CR⁸R^{8a})ₚ-, -(CR⁸R^{8a})ₙ-C(=O)-O-(CR⁸R^{8a})ₚ-, -(CR⁸R^{8a})ₙ-N(R⁵)-S(=O)ᵣ-N(R⁵)-(CR⁸R^{8a})ₚ-, -(CR⁸R^{8a})ₙ-N(R⁵)-C(=O)-O-(CR⁸R^{8a})ₚ-; or each of A and A' is independently 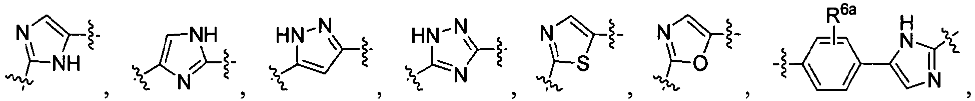 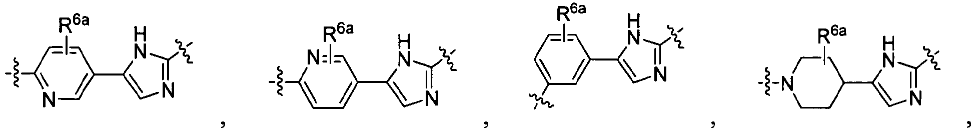 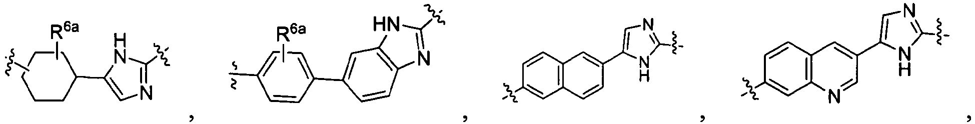 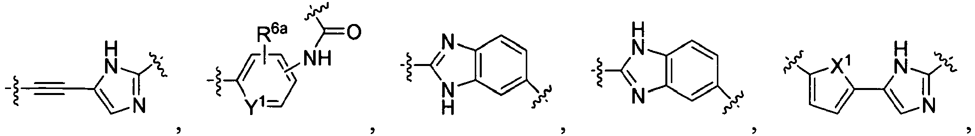 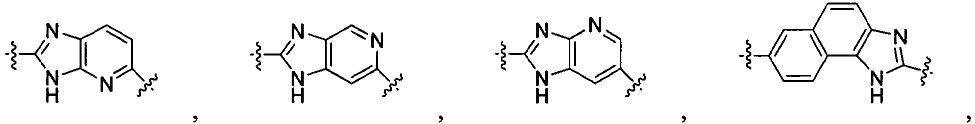 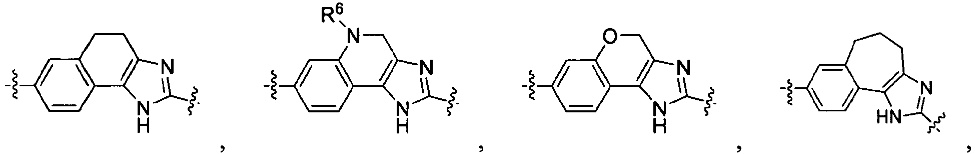 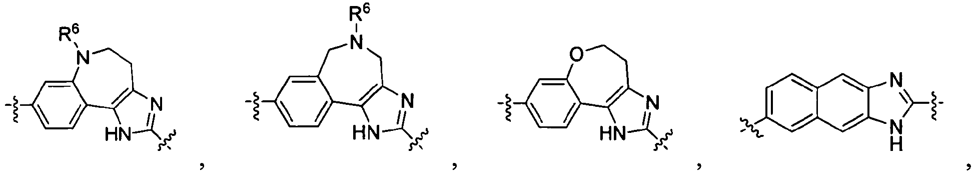 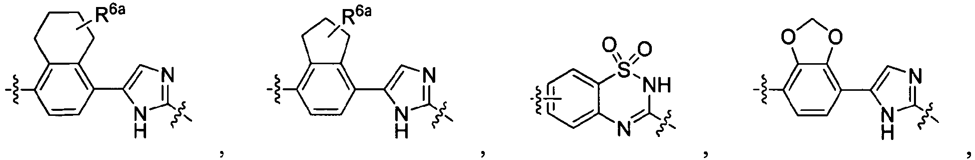 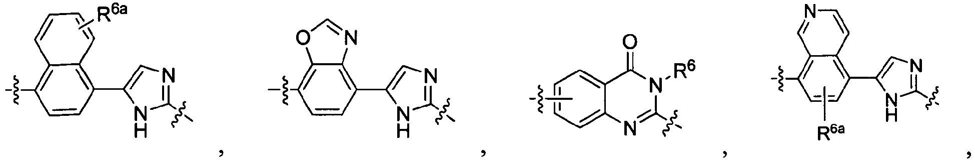 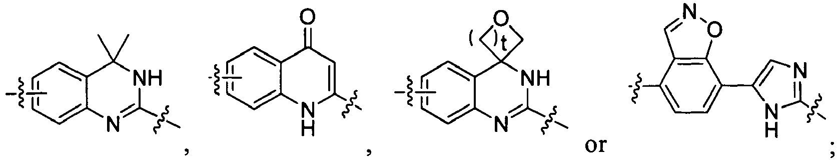
each R⁵ is independently H, deuterium, hydroxy, C₁₋₆ alkyl, C₂₋₆ heteroalkyl, C₃₋₁₀ cycloalkyl, C₂₋₁₀ heterocyclyl, C₆₋₁₀ aryl, C₁₋₉ heteroaryl, C₆₋₁₀ aryl-C₁₋₆-alkyl, C₁₋₆ alkoxy, C₁₋₆ alkyl-OC(=O)-, C₁₋₆ alk yl-C(=O)-, carbamoyl, C₁₋₆ alk yl-OS(=O)ᵣ-, C₁₋₆ alk yl-S(=O)ᵣO-, C₁₋₆ alk yl-S(=O)ᵣ- or aminosulfonyl;
each R^{5a} and R^{6a} is independently H, deuterium, oxo (=O), hydroxy, amino, F, Cl, Br, I, cyano, C₁₋₆ alkylacyl, C₁₋₆ alkylacyloxy, C₁₋₆ alkoxyacyl, C₁₋₆ alkylsulfonyl, C₁₋₆ alkoxysulfonyl, C₁₋₆ alkylsulfinyl, C₁₋₆ alkylsulfonyloxy, C₁₋₆ alkylsulfinyloxy, C₁₋₆ alkoxy, C₁₋₆ alkyl, C₆₋₁₀ aryl, -CF₃, -OCF₃, mercapto, nitro, C₁₋₆ alkylamino, C₃₋₁₀ cycloalkyl or C₆₋₁₀ aryloxy;
each R⁶ is independently H, deuterium, R¹³R^{13a}NC(=O)-, R¹³OC(=O)-, R¹³C(=O)-, R¹³R^{13a}NS(=O)-, R¹³OS(=O)-, R¹³S(=O)-, R¹³R^{13a}NS(=O)₂-, R¹³OS(=O)₂-, R¹³S(=O)₂-, C₁₋₆ aliphatic, C₁₋₆-alkoxy-C₁₋₆-aliphatic, C₁₋₆ alkylamino-C₁₋₆-aliphatic, C₆₋₁₀ aryl-C₁₋₆-aliphatic, C₁₋₉ heteroaryl-C₁₋₆-aliphatic, C₂₋₁₀ heterocyclyl-C₁₋₆-aliphatic, C₃₋₁₀ cycloalkyl-C₁₋₆-aliphatic, C₆₋₁₀ aryl, C₁₋₉ heteroaryl, C₂₋₁₀ heterocyclyl or C₃₋₁₀ carbocyclyl;
each R⁷ and R^{7a} is independently H, deuterium, F, Cl, Br, I, C₁₋₆ aliphatic, C₂₋₆ heteroalkyl, C₁₋₆ alkoxy-C₁₋₆-aliphatic, C₁₋₆ alkylamino-C₁₋₆-aliphatic, C₆₋₁₀ aryl-C₁₋₆-aliphatic, C₂₋₁₀ heterocyclyl-C₁₋₆-aliphatic, C₃₋₁₀ cycloalkyl-C₁₋₆ aliphatic, C₆₋₁₀ aryl, C₁₋₉ heteroaryl, C₂₋₁₀ heterocyclyl or C₃₋₁₀ carbocyclyl;
each R⁸ and R^{8a} is independently H, deuterium, hydroxy, cyano, nitro, F, Cl, Br, I, C₁₋₆ alkyl, C₂₋₆ heteroalkyl, C₃₋₁₀ cycloalkyl, C₂₋₁₀ heterocyclyl, C₆₋₁₀ aryl, C₁₋₉ heteroaryl, C₆₋₁₀ aryl-C₁₋₆-alkyl, C₁₋₆ alkoxy, C₁₋₆ alkyl-OC(=O)-, C₁₋₆ alkyl-C(=O)-, carbamoyl, C₁₋₆ alkyl-OS(=O)ᵣ-, C₁₋₆ alkyl-S(=O)ᵣO-, C₁₋₆ alkyl-S(=O)ᵣ- or aminosulfonyl;
each R¹³ and R^{13a} is independently H, deuterium, C₁₋₆ alkyl, C₂₋₆ heteroalkyl, C₃₋₁₀ cycloalkyl, C₂₋₁₀ heterocyclyl, C₆₋₁₀ aryl, C₁₋₉ heteroaryl or C₆₋₁₀ aryl-C₁₋₆-alkyl; with the proviso that where R¹³ and R^{13a} are bonded to the same nitrogen atom, R¹³ and R^{13a}, together with the nitrogen atom they are attached to, optionally form a substituted or unsubstituted 3-8 membered ring or a substituted or unsubstituted spiro or fused bicyclic ring;
each n is independently 0, 1, 2 or 3;
each p is independently 0, 1, 2 or 3;
each r is independently 0, 1 or 2; and
each of Y₄ and Y₄' is independently a bond, O, S, -(CH₂)ₙ-, -CH=CH-, -S(=O)ᵣ-, -CH₂O-, -CH₂S-, -CH₂F-, -CHR^{5a}-_{,} -CR^{5a}R^{6a}-, -CH₂S(=O)ᵣ- or -CH₂N(R⁶)-.

In some embodiments, the compound may have formula (II'): 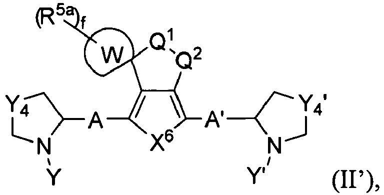 wherein 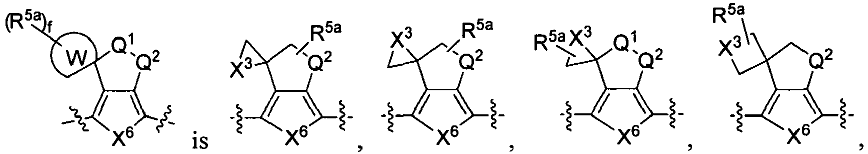 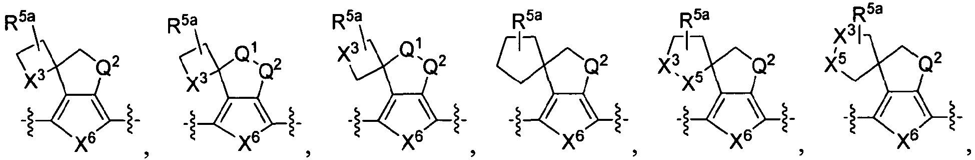 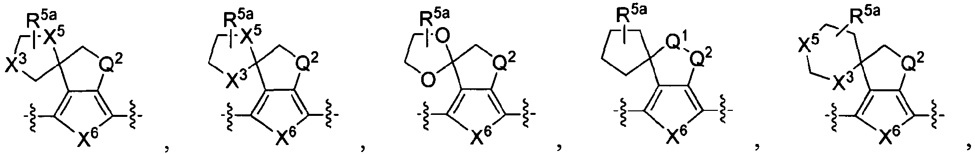 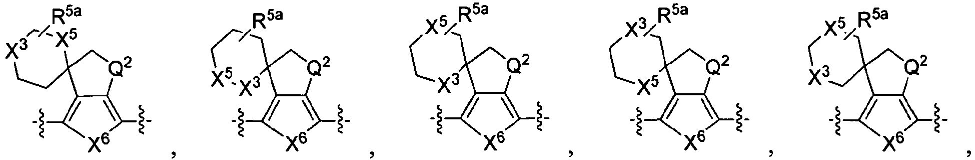 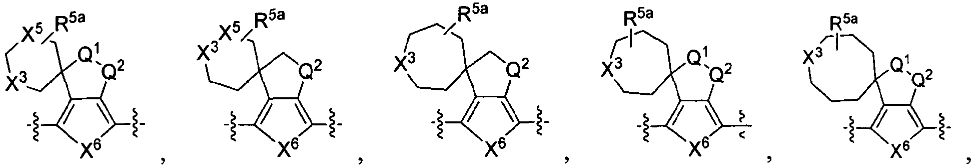 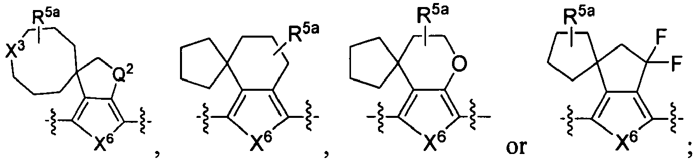
wherein each Q¹ and Q² is independently NR⁶, O, S, C(=O) or (CH₂)ₑ;
e is 0, 1, 2, 3 or 4;
each X¹, X³ and X⁵ is independently O, S, NR⁶ or CR⁷R^{7a};
each X⁶ is independently CR⁷R^{7a}, O, S or NR⁶;
f is 0, 1, 2, 3 or 4;
each of A and A' is independently a bond, C₁₋₆ alkylene, C₂₋₆ alkenylene, C₃₋₈ cycloalkylene, C₂₋₁₀ heterocyclylalkylene, -(CR⁸R^{8a})ₙ-O-(CR⁸R^{8a})ₚ-, -(CR⁸R^{8a})ₙ-N(R⁵)-(CR⁸R^{8a})ₚ-, -(CR⁸R^{8a})ₙ-S(=O)ᵣ-N(R⁵)-(CR⁸R^{8a})ₚ-, -(CR⁸R^{8a})ₙ-C(=O)-N(R⁵)-(CR⁸R^{8a})ₚ-, -(CR⁸R^{8a})ₙ-N(R⁵)-C(=O)-N(R⁵)-(CR⁸R^{8a})ₚ-, -(CR⁸R^{8a})ₙ-C(=O)-O-(CR⁸R^{8a})ₚ-, -(CR⁸R^{8a})ₙ-N(R⁵)-S(=O)ᵣ-N(R⁵)-(CR⁸R^{8a})ₚ-, -(CR⁸R^{8a})ₙ-N(R⁵)-C(=O)-O-(CR⁸R^{8a})ₚ-; or each of A and A' is independently 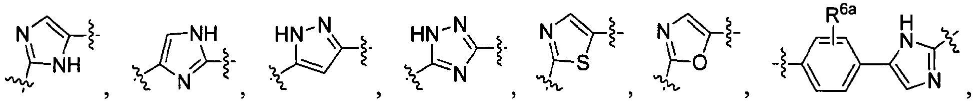 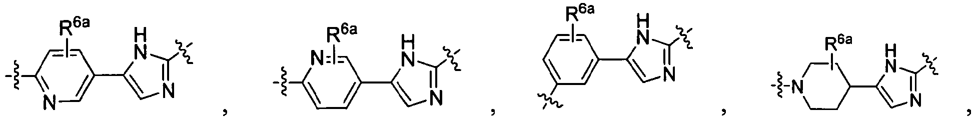 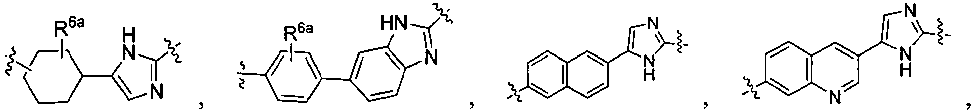 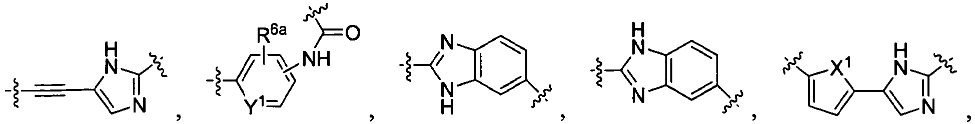 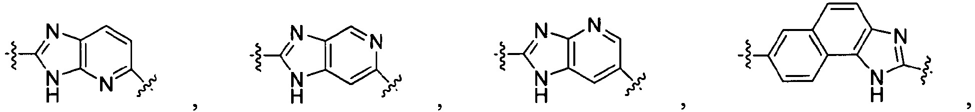 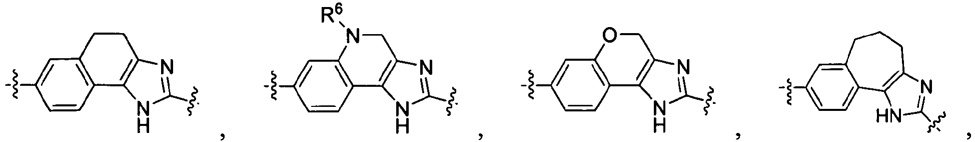 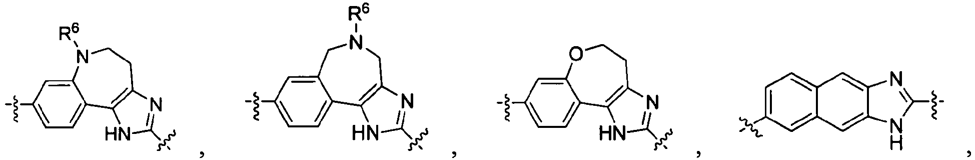 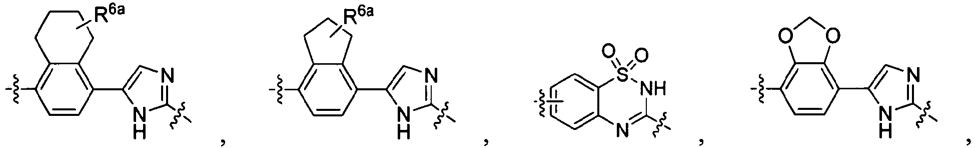 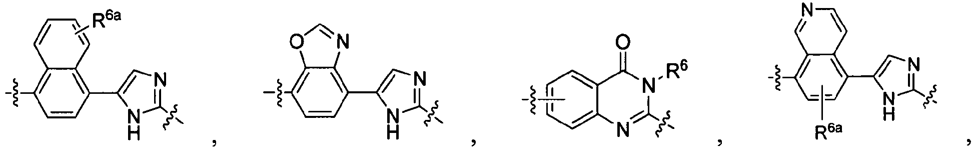 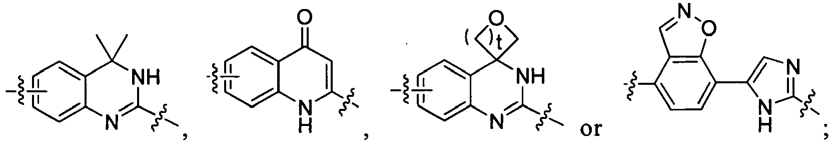
each Y¹ is independently N or CR⁷;
each R⁵ is independently H, deuterium, hydroxy, C₁₋₆ alkyl, C₂₋₆ heteroalkyl, C₃₋₁₀ cycloalkyl, C₂₋₁₀ heterocyclyl, C₆₋₁₀ aryl, C₁₋₉ heteroaryl, C₆₋₁₀ aryl-C₁₋₆-alkyl, C₁₋₆ alkoxy, C₁₋₆ alkyl-OC(=O)-, C₁₋₆ alk yl-C(=O)-, carbamoyl, C₁₋₆ alk yl-OS(=O)ᵣ-, C₁₋₆ alk yl-S(=O)ᵣO-, C₁₋₆ alk yl-S(=O)ᵣ- or aminosulfonyl;
each R^{5a} and R^{6a} is independently H, deuterium, oxo (=O), hydroxy, amino, F, Cl, Br, I, cyano, C₁₋₆ alkylacyl, C₁₋₆ alkylacyloxy, C₁₋₆ alkoxyacyl, C₁₋₆ alkylsulfonyl, C₁₋₆ alkoxysulfonyl, C₁₋₆ alkylsulfinyl, C₁₋₆ alkylsulfonyloxy, C₁₋₆ alkylsulfinyloxy, C₁₋₆ alkoxy, C₁₋₆ alkyl, C₆₋₁₀ aryl, -CF₃, -OCF₃, mercapto, nitro, C₁₋₆ alkylamino, C₃₋₁₀ cycloalkyl or C₆₋₁₀ aryloxy;
each R⁶ is independently H, deuterium, R¹³R^{13a}NC(=O)-, R¹³OC(=O)-, R¹³C(=O)-, R¹³R^{13a}NS(=O)-, R¹³OS(=O)-, R¹³S(=O)-, R¹³R^{13a}NS(=O)₂-, R¹³OS(=O)₂-, R¹³S(=O)₂-, C₁₋₆ aliphatic, C₁₋₆ alkoxy-C₁₋₆-aliphatic, C₁₋₆ alkylamino-C₁₋₆-aliphatic, C₆₋₁₀ aryl-C₁₋₆-aliphatic, C₁₋₉ heteroaryl-C₁₋₆-aliphatic, C₂₋₁₀ heterocyclyl-C₁₋₆-aliphatic, C₃₋₁₀ cycloalkyl-C₁₋₆-aliphatic, C₆₋₁₀ aryl, C₁₋₉ heteroaryl, C₂₋₁₀ heterocyclyl or C₃₋₁₀ carbocyclyl;
each R⁷ and R^{7a} is independently H, deuterium, F, Cl, Br, I, C₁₋₆ aliphatic, C₂₋₆ heteroalkyl, C₁₋₆ alkoxy-C₁₋₆-aliphatic, C₁₋₆ alkylamino-C₁₋₆-aliphatic, C₆₋₁₀ aryl-C₁₋₆-aliphatic, C₂₋₁₀ heterocyclyl-C₁₋₆-aliphatic, C₃₋₁₀ cycloalkyl-C₁₋₆-aliphatic, C₆₋₁₀ aryl, C₁₋₉ heteroaryl, C₂₋₁₀ heterocyclyl or C₃₋₁₀ carbocyclyl;
each R⁸ and R^{8a} is independently H, deuterium, hydroxy, cyano, nitro, F, Cl, Br, I, C₁₋₆ alkyl, C₂₋₆ heteroalkyl, C₃₋₁₀ cycloalkyl, C₂₋₁₀ heterocyclyl, C₆₋₁₀ aryl, C₁₋₉ heteroaryl, C₆₋₁₀ aryl-C₁₋₆-alkyl, C₁₋₆ alkoxy, C₁₋₆ alkyl-OC(=O)-, C₁₋₆ alkyl-C(=O)-, carbamoyl, C₁₋₆ alkyl-OS(=O)ᵣ-, C₁₋₆ alkyl-S(=O)ᵣO-, C₁₋₆ alkyl-S(=O)ᵣ- or aminosulfonyl;
each R¹³ and R^{13a} is independently H, deuterium, C₁₋₆ alkyl, C₂₋₆ heteroalkyl, C₃₋₁₀ cycloalkyl, C₂₋₁₀ heterocyclyl, C₆₋₁₀ aryl, C₁₋₉ heteroaryl or C₆₋₁₀ aryl-C₁₋₆-alkyl; with the proviso that where R¹³ and R^{13a} are bonded to the same nitrogen atom, R¹³ and R^{13a}, together with the nitrogen atom they are attached to, optionally form a substituted or unsubstituted 3-8 membered ring or a substituted or unsubstituted spiro or fused bicyclic ring;
each n is independently 0, 1, 2 or 3;
each p is independently 0, 1, 2 or 3;
each r is independently 0, 1 or 2; and
each of Y₄ and Y₄' is independently a bond, O, S, -(CH₂)ₙ-, -CH=CH-, -S(=O)ᵣ-, -CH₂O-, -CH₂S-,-CH₂F-, -CHR^{5a}-, -CR^{5a}R^{6a}-, -CH₂S(=O)ᵣ- or -CH₂N(R⁶)-.

In other embodiments, the compound may have formula (III):

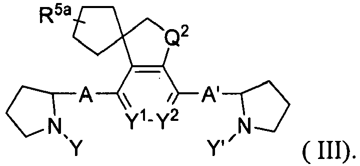

In other embodiments, the compound may have formula (IV): 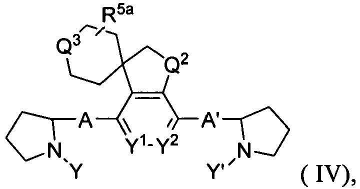 wherein each of Q² and Q³ is independently O, S, C(=O), NR⁶ or CH₂.

In other embodiments, the compound may have formula (V): 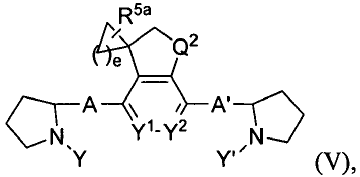 wherein e is 1, 2, 3 or 4.

In other embodiments, the compound may have formula (III'):

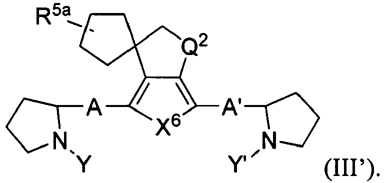

In other embodiments, the compound may have formula (IV'): 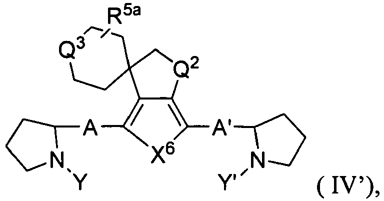 wherein each of Q² and Q³ is independently O, S, C(=O), NR⁶ or CH₂.

In other embodiments, the compound may have formula (V'): 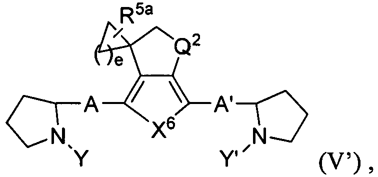 wherein e is 1, 2, 3 or 4.

In some embodiments, each of Y and Y' is independently a group derived from α-amino acid and the group derived from α-amino acid is optionally substituted with one or more substituents, wherein the substituent is deuterium, F, Cl, Br, I, hydroxy, cyano, alkoxycarbonyl, aryloxycarbonyl, heteroaryloxycarbonyl or heterocyclylcarbonyl.

In other embodiments, the α-amino acid is isoleucine, leucine, lysine, methionine, phenylalanine, threonine, tryptophane, valine, alanine, asparagine, aspartic acid, glutamic acid, glutamine, proline, serine, p-tyrosine, arginine, histidine, cysteine, glycine, sarcosine, *N*,*N*-dimethylglycine, homoserine, norvaline, norleucine, ornithine, homocysteine, homophenylalanine, phenylglycine, o-tyrosine, m-tyrosine or hydroxyproline.

In other embodiments, the α-amino acid is in the D configuration.

In other embodiments, the α-amino acid is in the L configuration.

In some embodiments, each of Y and Y' is independently -[U-(CR⁹R^{9a})ₜ-N(R¹⁰)-(CR⁹R^{9a})ₜ]ₖ-U-(CR⁹R^{9a})ₜ-N(R¹¹)-(CR⁹R^{9a})ₜ-R¹², -U-(CR⁹R^{9a})ₜ-R¹² or -[U-(CR⁹R^{9a})ₜ-N(R¹⁰)-(CR⁹R^{9a})ₜ]ₖ-U-(CR⁹R^{9a})ₜ-O-(CR⁹R^{9a})ₜ-R¹².

In other embodiments, each of Y and Y' is independently -[U-(CR⁹R^{9a})ₜ-N(R¹⁰)-(CR⁹R^{9a})ₜ]ₖ-U-(CR⁹R^{9a})ₜ-N(R¹¹)-(CR⁹R^{9a})ₜ-R¹².

In other embodiments, each of Y and Y' is independently -U-(CR⁹R^{9a})ₜ-N(R¹⁰)-(CR⁹R^{9a})ₜ-U-(CR⁹R^{9a})ₜ-N(R¹¹)-(CR⁹R^{9a})ₜ-R¹².

In other embodiments, each of Y and Y' is independently -U-(CR⁹R^{9a})ₜ-N(R¹¹)-(CR⁹R^{9a})ₜ-R¹².

In other embodiments, each of Y and Y' is independently -[C(=O)-(CR⁹R^{9a})ₜ-N(R¹⁰)-(CR⁹R^{9a})ₜ]ₖ -U-(CR⁹R^{9a})ₜ-N(R¹¹)-(CR⁹R^{9a})ₜ-R¹².

In other embodiments, each of Y and Y' is independently -C(=O)-(CR⁹R^{9a})ₜ-N(R¹⁰)-(CR⁹R^{9a})ₜ-U-(CR⁹R^{9a})ₜ-N(R¹¹)-(CR⁹R^{9a})ₜ-R¹².

In other embodiments, each of Y and Y' is independently -[C(=O)-(CR⁹R^{9a})ₜ-N(R¹⁰)-(CR⁹R^{9a})ₜ]ₖ-C(=O)-(CR⁹R^{9a})ₜ-N(R¹¹)-(CR⁹R^{9a})ₜ-R¹².

In other embodiments, each of Y and Y' is independently -C(=O)-(CR⁹R^{9a})ₜ-N(R¹⁰)-(CR⁹R^{9a})ₜ-C(=O)-(CR⁹R^{9a})ₜ-N(R¹¹)-(CR⁹R^{9a})ₜ-R¹².

In other embodiments, each of Y and Y' is independently -C(=O)-(CR⁹R^{9a})ₜ-N(R¹¹)-(CR⁹R^{9a})ₜ-R¹².

In other embodiments, each of Y and Y' is independently -C(=O)-(CR⁹R^{9a})ₙ-N(R¹¹)-(CR⁹R^{9a})ₙ-C(=O)-R¹³.

In other embodiments, each of Y and Y' is independently -C(=O)-(CR⁹R^{9a})ₙ-N(R¹¹)-C(=O)-R¹³.

In other embodiments, each of Y and Y' is independently -C(=O)-(CR⁹R^{9a})ₙ-N(R¹¹)-(CR⁹R^{9a})ₙ-C(=O)-O-R¹³.

In other embodiments, each of Y and Y' is independently -C(=O)-(CR⁹R^{9a})ₙ-N(R¹¹)-C(=O)-O-R¹³.

In other embodiments, each of Y and Y' is independently -U-(CR⁹R^{9a})ₜ-R¹².

In other embodiments, each of Y and Y' is independently -C(=O)-(CR⁹R^{9a})ₜ-R¹².

In other embodiments, each of Y and Y' is independently -[U-(CR⁹R^{9a})ₜ-N(R¹⁰)-(CR⁹R^{9a})ₜ]ₖ-U-(CR⁹R^{9a})ₜ-O-(CR⁹R^{9a})ₜ-R¹².

In other embodiments, each of Y and Y' is independently -U-(CR⁹R^{9a})ₜ-N(R¹⁰)-(CR⁹R^{9a})ₜ-U-(CR⁹R^{9a})ₜ-O-(CR⁹R^{9a})ₜ-R¹².

In other embodiments, each of Y and Y' is independently -C(=O)-(CR⁹R^{9a})ₜ-N(R¹⁰)-(CR⁹R^{9a})ₜ-C(=O)-(CR⁹R^{9a})ₜ-O-(CR⁹R^{9a})ₜ-R¹².

In other embodiments, each of Y and Y' is independently -U-(CR⁹R^{9a})ₜ-O-(CR⁹R^{9a})ₜ-R¹².

In other embodiments, each of Y and Y' is independently -C(=O)-(CR⁹R^{9a})ₜ-O-(CR⁹R^{9a})ₜ-R¹².

In other embodiments, each of Y and Y' is independently -C(=O)-(CR⁹R^{9a})ₙ-N(R¹¹)-R¹², wherein R¹¹ and R¹², together with the atom they are attached to, form a 4-7 membered ring.

In other embodiments, each R⁹, R^{9a}, R¹⁰ and R¹¹ is independently H, deuterium, C₁₋₆ alkyl, C₂₋₆ heteroalkyl, C₃₋₁₀ cycloalkyl, C₂₋₁₀ heterocyclyl, C₆₋₁₀ aryl, C₁₋₉ heteroaryl, C₁₋₆ haloalkyl, C₁₋₆ hydroxyalkyl, C₆₋₁₀ aryl-C₁₋₆-alkyl, C₁₋₉ heteroaryl-C₁₋₆-alkyl, C₂₋₁₀ heterocyclyl-C₁₋₆-alkyl or C₃₋₈ cycloalkyl-C₁₋₆-alkyl;
each R¹² is independently R^{13a}R¹³N-, -C(=O)R¹³, -C(=S)R¹³, -C(=O)-O-R¹³, -C(=O)NR¹³R^{13a}, -OC(=O)NR¹³R^{13a}, -OC(=O)OR¹³, -N(R¹³)C(=O)NR¹³R^{13a}, -N(R¹³)C(=O)OR^{13a}, -N(R¹³)C(=O)-R^{13a}, R¹³R^{13a}N-S(=O)₂-, R¹³S(=O)₂-, R¹³S(=O)₂N(R^{13a})-, R¹³OS(=O)₂-, C₁₋₆ alkyl, C₂₋₆ heteroalkyl, C₃₋₁₀ cycloalkyl, C₂₋₁₀ heterocyclyl, C₆₋₁₀ aryl, C₁₋₉ heteroaryl or C₆₋₁₀ aryl-C₁₋₆-alkyl; or R¹¹ and R¹², together with the atom they are attached to, form a 4-7 membered ring;
each R¹³ and R^{13a} is independently H, deuterium, C₁₋₆ alkyl, C₂₋₆ heteroalkyl, C₃₋₁₀ cycloalkyl, C₂₋₁₀ heterocyclyl, C₆₋₁₀ aryl, C₁₋₉ heteroaryl or C₆₋₁₀ aryl-C₁₋₆-alkyl; with the proviso that where R¹³ and R^{13a} are bonded to the same nitrogen atom, R¹³ and R^{13a}, together with the nitrogen atom they are attached to, optionally form a substituted or unsubstituted 3-8 membe red ring or a substituted or unsubstituted spiro or fused bicyclic ring;
each t is independently 0, 1, 2, 3 or 4; and
each k is independently 0, 1 or 2.

In other embodiments, each R⁹, R^{9a}, R¹⁰ and R¹¹ is independently H, deuterium, methyl, ethyl, isopropyl, cyclohexyl, isobutyl or phenyl;
each R¹² is independently -C(=O)R¹³, -C(=O)-O-R¹³, -C(=O)NR¹³R^{13a}, methyl, ethyl, propyl, phenyl, cyclohexyl, morpholinyl or piperidinyl;
or R¹¹ and R¹², together with the atom they are attached to, form a 4-7 membered ring; and
each R¹³ and R^{13a} is independently H, deuterium, methyl, ethyl, propyl, phenyl, cyclohexyl, morpholinyl or piperidinyl.

In other embodiments, the compound may have formula (VI): 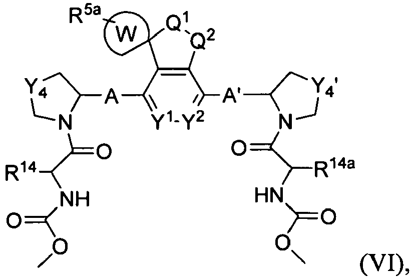
wherein each of R¹⁴ and R^{14a} is independently H, deuterium, C₁₋₆ alkyl, C₁₋₆ haloalkyl, C₁₋₆ hydroxyalkyl, C₂₋₆ heteroalkyl, C₆₋₁₀ aryl, C₁₋₉ heteroaryl, C₂₋₁₀ heterocyclyl, C₃₋₈ cycloalkyl, C₆₋₁₀ aryl-C₁₋₆-alkyl, C₁₋₉ heteroaryl-C₁₋₆-alkyl, C₂₋₁₀ heterocyclyl-C₁₋₆-alkyl, or C₃₋₈ cycloalkyl-C₁₋₆-alkyl; and
wherein each of C₁₋₆ alkyl, C₁₋₆ haloalkyl, C₁₋₆ hydroxyalkyl, C₂₋₆ heteroalkyl, C₆₋₁₀ aryl, C₁₋₉ heteroaryl, C₂₋₁₀ heterocyclyl, C₃₋₈ cycloalkyl, C₆₋₁₀ aryl-C₁₋₆-alkyl, C₁₋₉ heteroaryl-C₁₋₆-alkyl, C₂₋₁₀ heterocyclyl-C₁₋₆-alkyl and C₃₋₈ cycloalkyl-C₁₋₆-alkyl is optionally substituted with one or more substituents, wherein the substituent is deuterium, F, Cl, Br, hydroxy or cyano.

In other embodiments, the compound may have formula (VII): 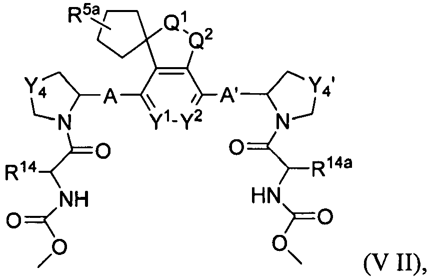
wherein each of R¹⁴ and R^{14a} is independently H, deuterium, C₁₋₃ hydroxyalkyl, methyl, ethyl, isopropyl, isobutyl, *tert-*butyl*,* allyl, propargyl, trifluoroethyl, phenyl, pyranyl, morpholinyl, -NR¹³R^{13a}, benzyl, piperazinyl, cyclopentyl, cyclopropyl, cyclohexyl or C₁₋₉ heteroaryl; and
wherein each of methyl, ethyl, isopropyl, isobutyl, *tert*-butyl, allyl, propargyl, trifluoroethyl, phenyl, pyranyl, morpholinyl, benzyl, piperazinyl, cyclopentyl, cyclopropyl and cyclohexyl is optionally substituted with one or more substituents, wherein the substituent is deuterium, F, Cl, Br, hydroxy or cyano.

In some embodiments, the compound may have formula (VIII): 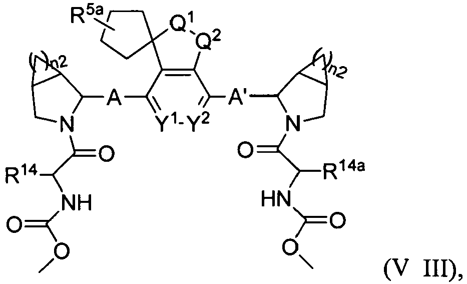
wherein each of R¹⁴ and R^{14a} is independently H, deuterium, C₁₋₆ alkyl, C₁₋₆ haloalkyl, C₁₋₆ hydroxyalkyl, C₂₋₆ heteroalkyl, C₆₋₁₀ aryl, C₁₋₉ heteroaryl, C₂₋₁₀ heterocyclyl, C₃₋₈ cycloalkyl, C₆₋₁₀ aryl-C₁₋₆-alkyl, C₁₋₉ heteroaryl-C₁₋₆-alkyl, C₂₋₁₀ heterocyclyl-C₁₋₆-alkyl or C₃₋₈ cycloalkyl-C₁₋₆-alkyl;
wherein each of C₁₋₆ alkyl, C₁₋₆ haloalkyl, C₁₋₆ hydroxyalkyl, C₂₋₆ heteroalkyl, C₆₋₁₀ aryl, C₁₋₉ heteroaryl, C₂₋₁₀ heterocyclyl, C₃₋₈ cycloalkyl, C₆₋₁₀ aryl-C₁₋₆-alkyl, C₁₋₉ heteroaryl-C₁₋₆-alkyl, C₂₋₁₀ heterocyclyl-C₁₋₆-alkyl and C₃₋₈ cycloalkyl-C₁₋₆-alkyl is optionally substituted with one or more substituents, wherein the substituent is deuterium, F, Cl, Br, hydroxy or cyano; and
each n₂ is independently 1, 2, 3 or 4.

In some embodiments, the compound may have formula (IX): 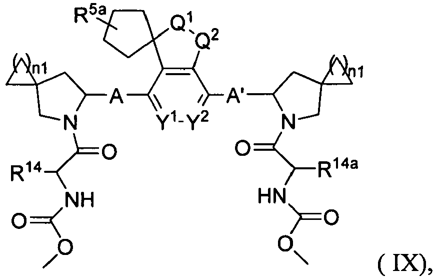
wherein each of R¹⁴ and R^{14a} is independently H, deuterium, C₁₋₆ alkyl, C₁₋₆ haloalkyl, C₁₋₆ hydroxyalkyl, C₂₋₆ heteroalkyl, C₆₋₁₀ aryl, C₁₋₉ heteroaryl, C₂₋₁₀ heterocyclyl, C₃₋₈ cycloalkyl, C₆₋₁₀ aryl-C₁₋₆-alkyl, C₁₋₉ heteroaryl-C₁₋₆-alkyl, C₂₋₁₀ heterocyclyl-C₁₋₆-alkyl or C₃₋₈ cycloalkyl-C₁₋₆-alkyl;
wherein each of ₁₋₆ alkyl, C₁₋₆ haloalkyl, C₁₋₆ hydroxyalkyl, C₂₋₆ heteroalkyl, C₆₋₁₀ aryl, C₁₋₉ heteroaryl, C₂₋₁₀ heterocyclyl, C₃₋₈ cycloalkyl, C₆₋₁₀ aryl-C₁₋₆-alkyl, C₁₋₉ heteroaryl-C₁₋₆-alkyl, C₂₋₁₀ heterocyclyl-C₁₋₆-alkyl and C₃₋₈ cycloalkyl-C₁₋₆-alkyl is optionally substituted with one or more substituents, wherein the substituent is deuterium, F, Cl, Br, hydroxy or cyano; and
each n₁ is independently 1, 2, 3 or 4.

In some embodiments, the compound may have formula (X): 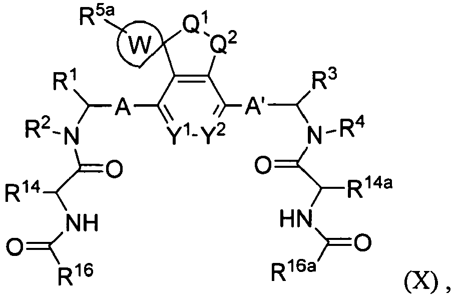
wherein R^{5a} is H, deuterium, methyl, ethyl, F, Cl, Br or I;
Q¹ is CH₂, C(=O), O, S or NR⁶;
Q² is CH₂, C(=O), CF₂, O, NR⁶ or S;
each of Y¹ and Y² is independently N or CR⁷;
each R⁶ and R⁷ is independently H, deuterium, methyl, ethyl, isopropyl, phenyl or cyclohexyl;
each of R¹⁴ and R^{14a} is independently H, deuterium, methyl, ethyl, phenyl, cyclohexyl, 1-methylpropyl, isopropyl or *tert*-butyl;
each of R¹⁶ and R^{16a} is independently hydroxy, methoxy, ethoxy, phenoxy, 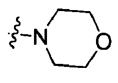 or *tert*-butoxy;
wherein each of methyl, ethyl, phenyl, cyclohexyl, 1-methylpropyl, isopropyl, methoxy, ethoxy, *tert*-butoxy and *tert*-butyl is optionally substituted with one or more substituents, wherein the substituent is deuterium, F, Cl, Br, hydroxy or cyano;
wherein 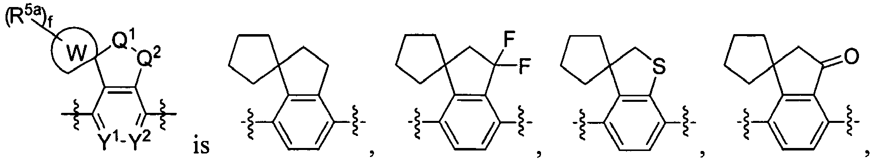 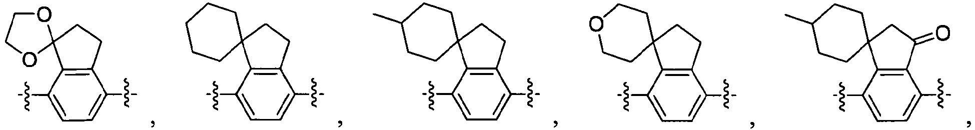 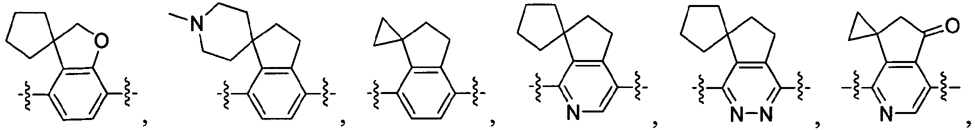 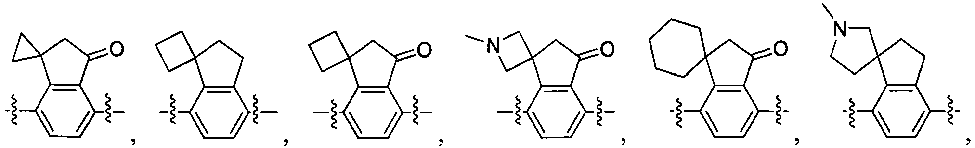 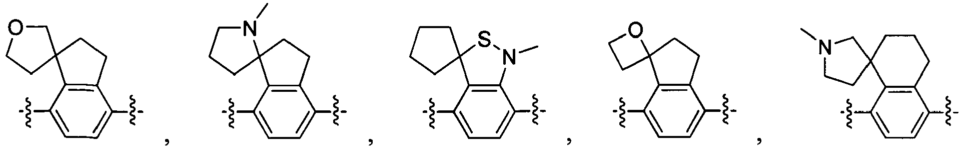 or 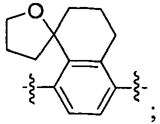
each of A and A' is independently 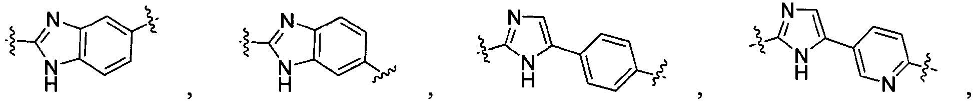 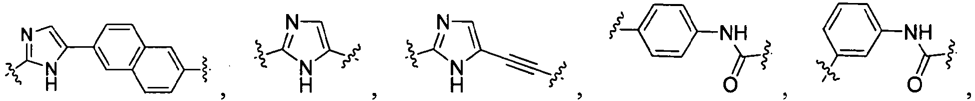 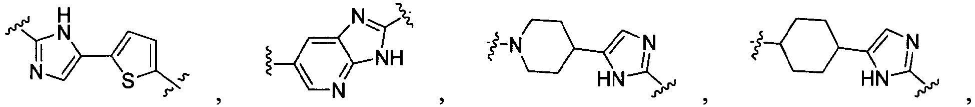 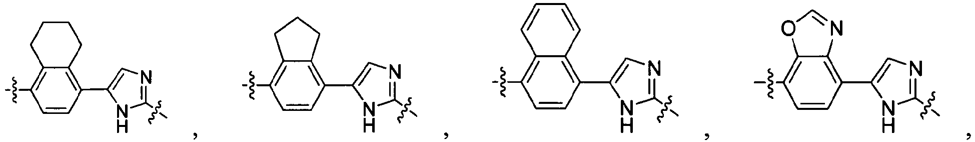 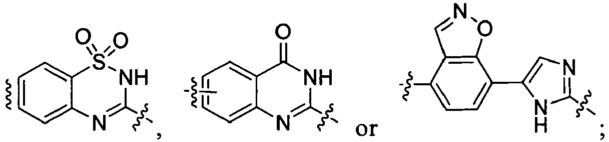
wherein R¹, R² and N-CH together form one of the following divalent groups: 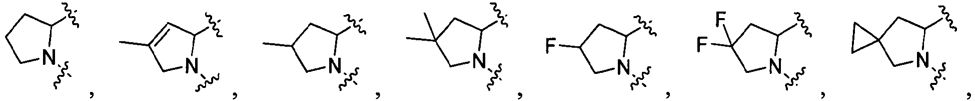 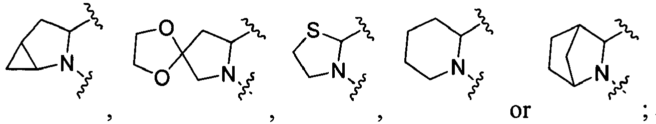 and
wherein R³, R⁴ and N-CH together form one of the following divalent groups:

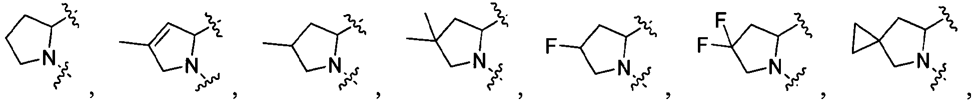

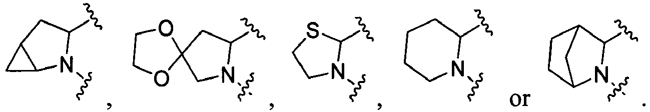

In other embodiments, the compound may have formula (XI): 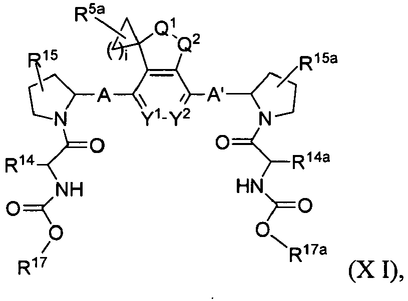
wherein i is 1, 2, 3 or 4;
R^{5a} is H, deuterium or methyl;
each of Q¹ and Q² is independently (CH₂)ₑ, CF₂, S, NR⁶, O or C(=O);
e is 0, 1, 2, 3 or 4;
R⁶ is H, deuterium, methyl, ethyl, isobutyl, cyclohexyl, phenyl or isopropyl;
each of R¹⁴ and R^{14a} is independently H, deuterium, methyl, ethyl, isobutyl, cyclohexyl, phenyl or isopropyl;
each of R¹⁵ and R^{15a} is independently H, deuterium, F, Cl, Br, methyl, ethyl, isopropyl or *tert*-butyl; and
each of R¹⁷ and R^{17a} is independently methyl, phenyl or ethyl;
wherein each of methyl, ethyl, phenyl, cyclohexyl, isopropyl, isobutyl and *tert*-butyl is optionally substituted with one or more substituents, wherein the substituent is deuterium, F, Cl, Br, hydroxy or cyano.

In some embodiments, the compound may have formula (XI'): 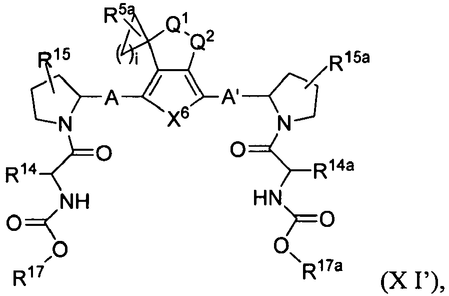
wherein i is 1, 2, 3 or 4;
R^{5a} is H, deuterium or methyl;
each of Q¹ and Q² is independently (CH₂)ₑ, CF₂, S, NR⁶, O or C(=O);
e is 0, 1, 2, 3 or 4;
each R⁶ is independently H, deuterium, methyl, ethyl, isobutyl, cyclohexyl, phenyl or isopropyl;
each of R¹⁴ and R^{14a} is independently H, deuterium, methyl, ethyl, isobutyl, cyclohexyl, phenyl or isopropyl;
each of R¹⁵ and R^{15a} is independently H, deuterium, F, Cl, Br, methyl, ethyl, isopropyl or *tert-*butyl;
each of R¹⁷ and R^{17a} is independently methyl, phenyl or ethyl;
wherein each of methyl, ethyl, phenyl, cyclohexyl, isopropyl, isobutyl and *tert*-butyl is optionally substituted with one or more substituents, wherein the substituent is deuterium, F, Cl, Br, hydroxy or cyano;
X⁶ is independently CH₂, O, S or NR⁶; and
each of A and A' is independently

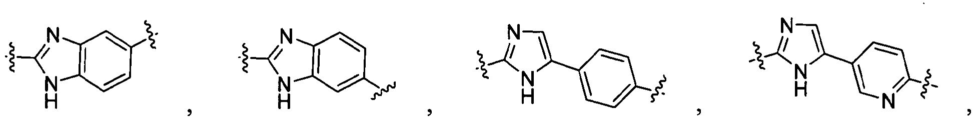

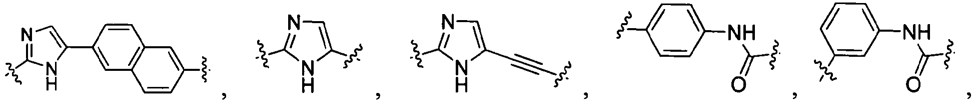

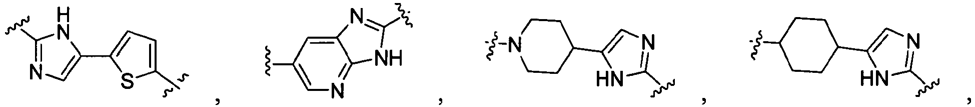

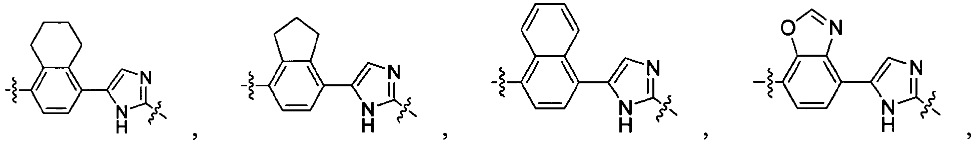

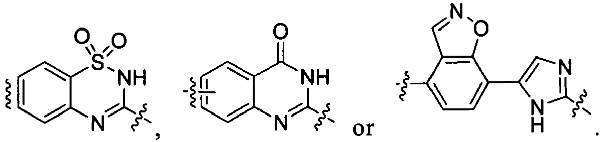

In some embodiments, non-limiting examples of compounds disclosed herein, or a stereoisomer, a geometric isomer, a tautomer, an N-oxide, a hydrate, a solvate, or a pharmaceutically acceptable salt thereof, are shown in the following:

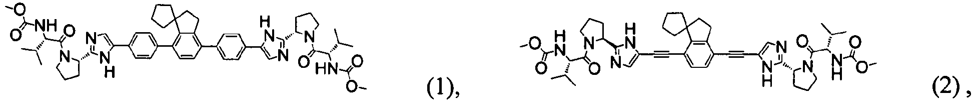

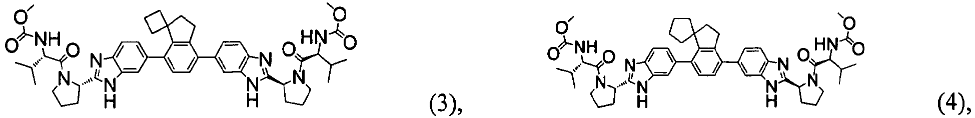

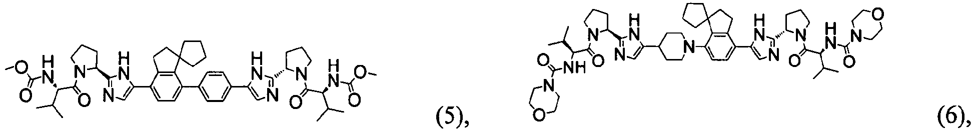

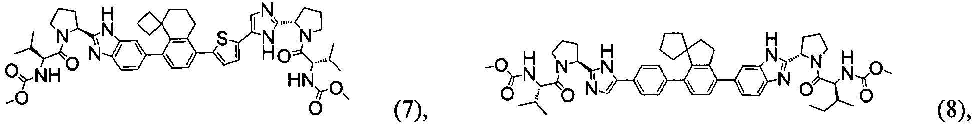

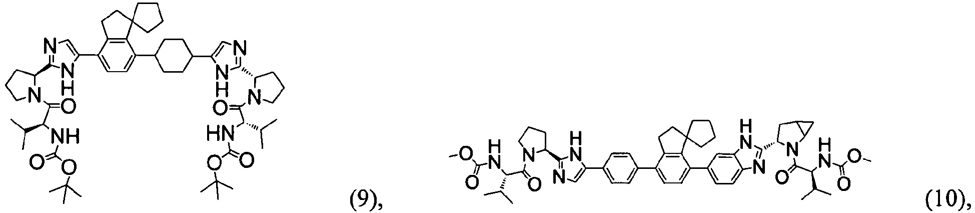

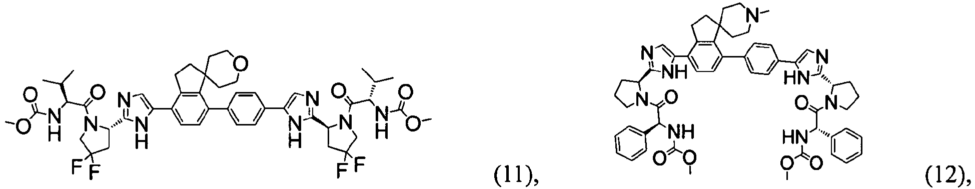

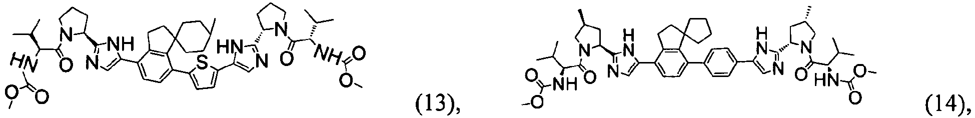

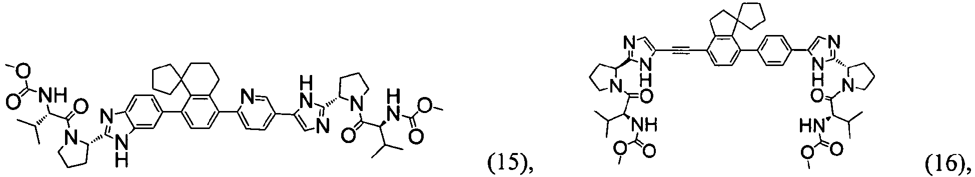

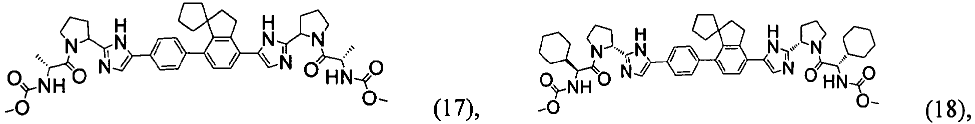

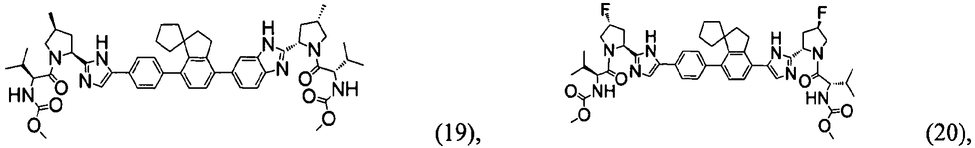

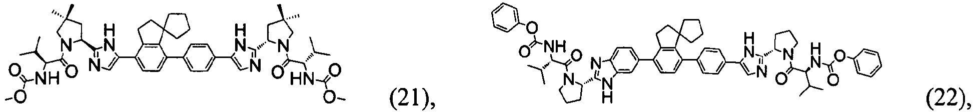

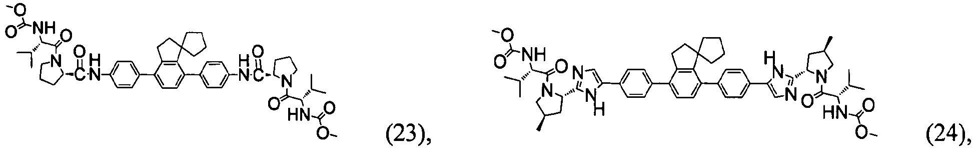

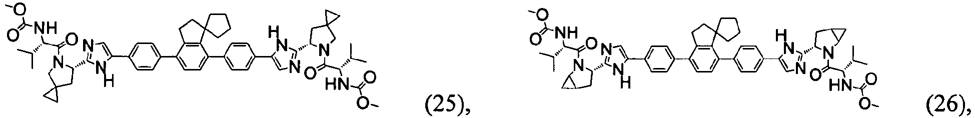

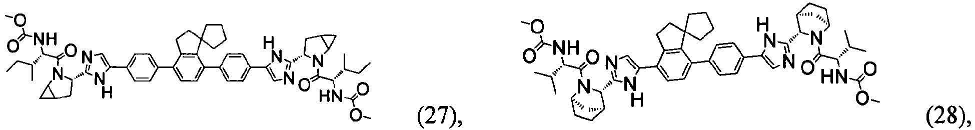

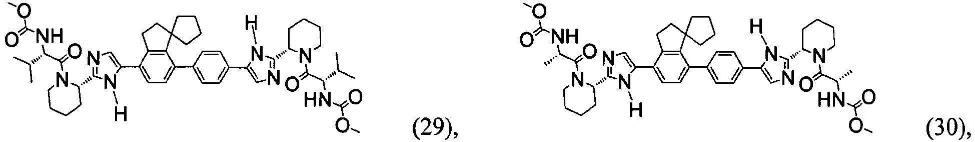

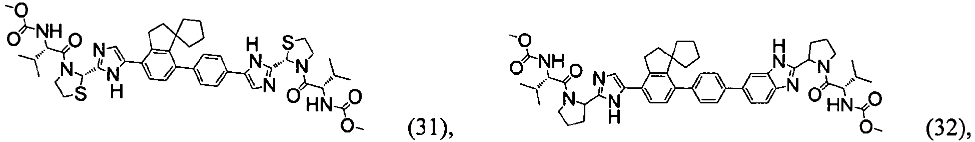

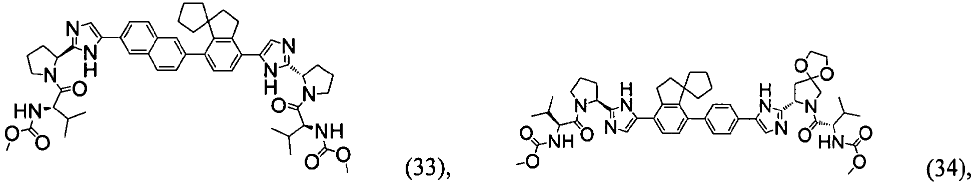

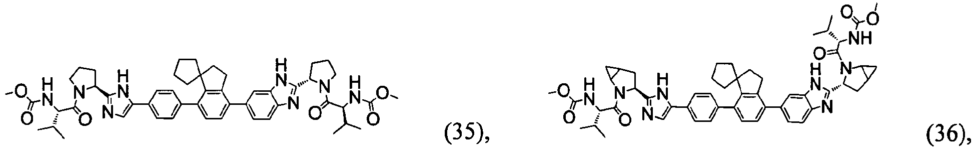

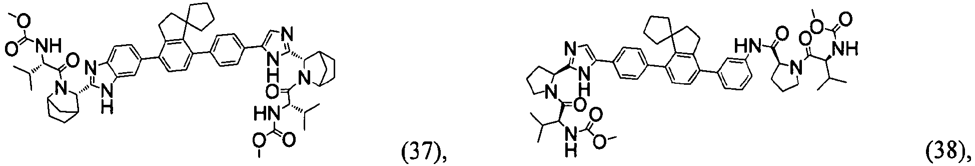

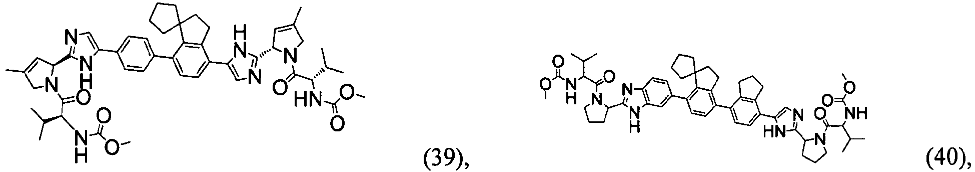

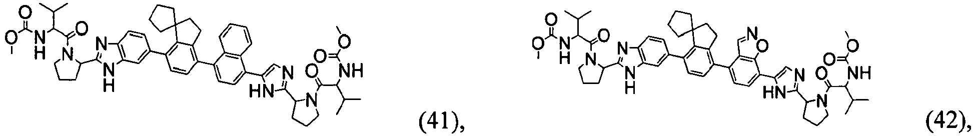

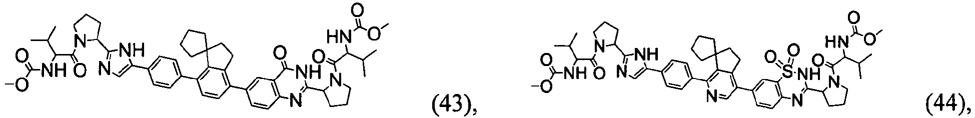

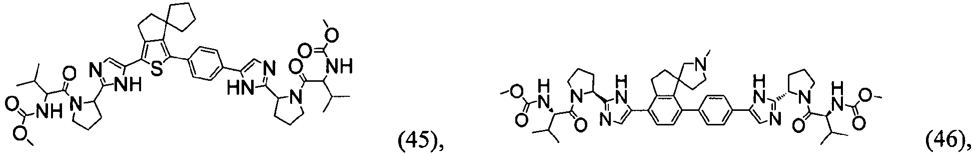

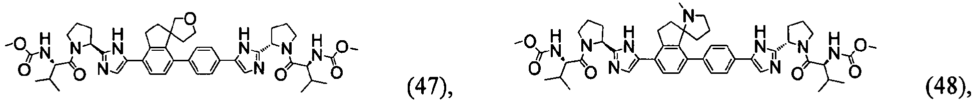

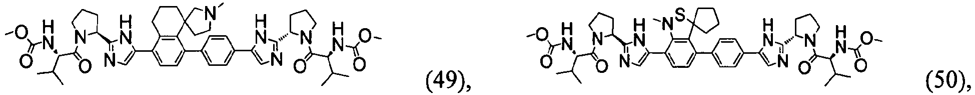

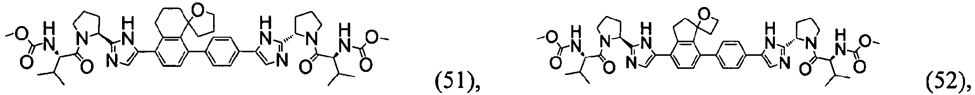

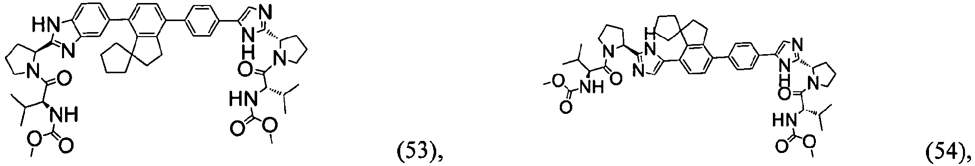

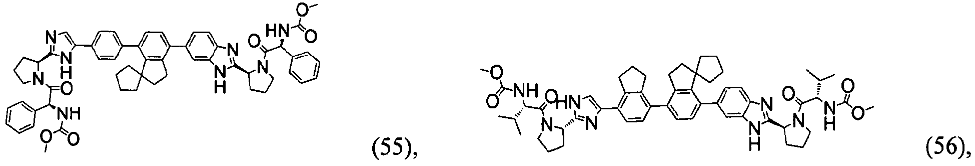

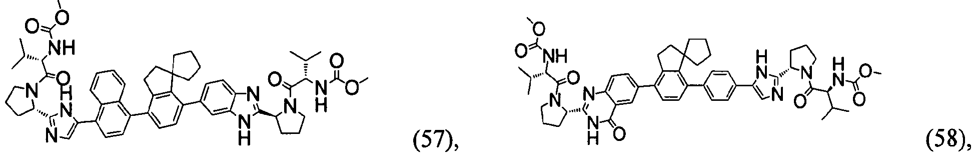

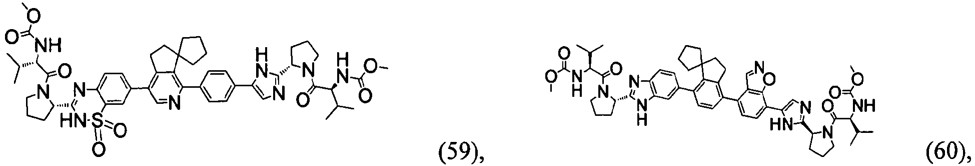

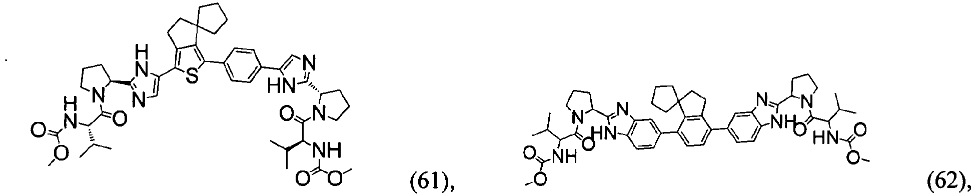

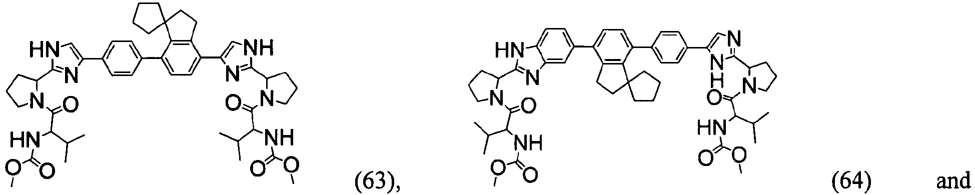

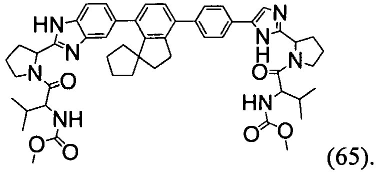

Provided herein includes the use of a compound disclosed herein (In present disclosure, "a compound disclosed herein" comprises a compound of formula (I), a stereoisomer, a geometric isomer, a tautomer, an N-oxide, a hydrate, a solvate and a pharmaceutically acceptable salt thereof.), in the manufacture of a medicament for the treatment either acutely or chronically of HCV infection in a patient, including those described herein. Also provided herein is the use of the compound in the manufacture of an anti-HCV medicament. Provided herein is the use of the compound disclosed herein, in the manufacture of a medicament to attenuate, prevent, manage or treat HCV-mediated disease, especially HCV's NS5A protein. Also provided herein is a pharmaceutical composition comprising a therapeutically effective amount of a compound of formula (I) in association with at least one pharmaceutically acceptable carrier, adjuvant or diluent.

In certain embodiments, the salt is a pharmaceutically acceptable salt. The phrase "pharmaceutically acceptable" refers to that the substance or composition must be compatible chemically and/or toxicologically, with the other ingredients comprising a Formulation and/or the mammal being treated therewith. Persons skilled in the art can choose the "pharmaceutically acceptable" substance or composition specifically according to other components and the object being treated with (such as man).

The compounds disclosed herein also include salts of such compounds which are not necessarily pharmaceutically acceptable salts, and which may be useful as intermediates for preparing and/or purifying compounds of formula (I) and/or for separating enantiomers of compounds of formula (I).

If the compound disclosed herein is a base, the desired salt may be prepared by any suitable method available in the art, for example, treatment of the free base with an inorganic acid, such as hydrochloric acid, hydrobromic acid, sulfuric acid, nitric acid, phosphoric acid, and the like. Or with an organic acid, such as acetic acid, maleic acid, succinic acid, mandelic acid, fumaric acid, malonic acid, pyruvic acid, oxalic acid, glycolic acid, salicylic acid; a pyranosidyl acid, such as glucuronic acid or galacturonic acid; an alpha hydroxy acid, such as citric acid or tartaric acid; an amino acid, such as aspartic acid or glutamic acid; an aromatic acid, such as benzoic acid or cinnamic acid, a sulfonic acid, such as *p*-toluenesulfonic acid or ethanesulfonic acid, and the like.

If the compound disclosed herein is an acid, the desired salt may be prepared by any suitable method, for example, treatment of the free acid with an inorganic or organic base, such as an amine (primary, secondary or tertiary), an alkali metal hydroxide or alkaline earth metal hydroxide, and the like. Some non-limiting examples of suitable salts include organic salts derived from amino acids, such as glycine and arginine, ammonia, primary, secondary, and tertiary amines, and cyclic amines, such as piperidine, morpholine and piperazine, and inorganic salts derived from sodium, calcium, potassium, magnesium, manganese, iron, copper, zinc, aluminum, lithium, and the like.

### COMPOSITION, FORMULATIONS AND ADMINSTRATION OF COMPOUNDS OF THE INVENTION

The pharmaceutical composition disclosed herein comprises any one of the compounds in the present disclosure. The pharmaceutical composition further comprises a pharmaceutically acceptable carrier, excipient, diluent, adjuvant, vehicle or a combination thereof. And the pharmaceutical composition can be used for treating HCV infection or a HCV disorder, especially it is effective as inhibitor of the non-structural 5A (NS5A) protein of HCV.

The pharmaceutical composition disclosed herein further comprises an additional anti-HCV agent. The anti-HCV agent refers to any known agents for anti-HCV and they are different from the compounds disclosed herein. For example, the anti-HCV agent is interferon, ribavirin, IL-2, IL-6, IL-12, a compound that enhances the development of a type 1 helper T cell response, interfering RNA, anti-sense RNA, imiquimod, an inosine5'-monophosphate dehydrogenase inhibitor, amantadine, rimantadine, ribavirin, bavituximab, human hepatitis C immune globulin (CIVACIR™), boceprevir, telaprevir, erlotinib, daclatasvir, simeprevir, asunaprevir, vaniprevir, faldaprevir, ABT-450, danoprevir, sovaprevir, MK-5172, vedroprevir, BZF-961, GS-9256, narlaprevir, ANA975, ABT-267, EDP239, PPI-668, GS-5816, samatasvir (IDX-719), MK-8742, MK-8325, GSK-2336805, PPI-461, TMC-435, MK-7009, BI-2013335, ciluprevir, BMS-650032, ACH-1625, ACH-1095, VX-985, IDX-375, VX-500, VX-813, PHX-1766, PHX-2054, IDX-136, IDX-316, EP-013420, VBY-376, TMC-649128, R-7128, PSI-7977, INX-189, IDX-184, IDX102, R1479, UNX-08189, PSI-6130, PSI-938, PSI-879, HCV-796, HCV-371, VCH-916, VCH-222, ANA-598, MK-3281, ABT-333, ABT-072, PF-00868554, BI-207127, GS-9190, A-837093, JKT-109, G1-59728, GL-60667, AZD-2795, TMC647055 or a combination thereof. The interferon is interferon α-2b, pegylated interferon α, interferon α-2a, pegylated interferon α-2a, consensus interferon-α, interferon γ or a combination thereof. The pharmaceutical composition disclosed herein further comprises at least one HCV inhibitor. In some embodiments, the HCV inhibitor inhibits at least one of HCV replication process and HCV viral protein function. In some embodiments, the HCV replication process is a whole viral cycle consisting of HCV entry, uncoating, translation, replication, assembly and egress. In some embodiments, the HCV viral protein is helicase, proteinase, metalloproteinase, serine proteinase, non-structural protein NS4A, non-structural protein NS4B, RNA polymerase of non-structural protein NS5A or non-structural protein NS5B, or an internal ribosome entry site (IRES) or inosine-5'-monophosphate dehydrogenase (IMPDH) required in HCV viral replication.

When it is possible that, for use in therapy, therapeutically effective amounts of a compound of formula (I), as well as pharmaceutically acceptable salts thereof, may be administered as the raw chemical, it is possible to present the active ingredient as a pharmaceutical compositions, which include therapeutically effective amounts of compounds of formula (I) or pharmaceutically acceptable salts thereof, and one or more pharmaceutically acceptable carriers, diluents, or excipients. The term "therapeutically effective amount" refers to the total amount of each active component that is sufficient to show a meaningful patient benefit (e.g., a reduction in viral load). When applied to individual active ingredient, administered alone, the term refers to that ingredient alone. When applied to a combination, the term refers to combined amounts of the active ingredients that result in the therapeutic effect, whether administered in combination, serially, or simultaneously. The compounds of formula (I) and pharmaceutically acceptable salts thereof, are as described above. The carrier(s), diluents(s), or excipient(s) must be acceptable in the sense of being compatible with the other ingredients of the formulation and not deleterious to recipient thereof. In accordance with another aspect of the present disclosure there is also provided a process for the preparation of a pharmaceutical formulation including admixing a compound of formula (I), or a pharmaceutically acceptable salt thereof, with one or more pharmaceutically acceptable carriers, diluents, or excipients. The term "pharmaceutically acceptable" refers to those compounds, materials, composition, and/or dosage forms which are, within the scope of sound medical judgment, suitable for use in contact with the tissues of patients without excessive toxicity, irritation, allergic response, or other problem or complication commensurate with a reasonable benefit/risk ratio, and are effective for their intended use.

Pharmaceutical formulations may be presented in unit dose forms containing a predetermined amount of active ingredient per unit dose. Dosage levels of between about 0.01 and about 250 milligram per kilogram ("mg/kg") body weight per day, preferably between about 0.05 and about 100 mg/kg body weight per day of the compounds of the present disclosure are typical in a monotherapy for the prevention and treatment of HCV mediated disease. Typically, the pharmaceutical compositions of this disclosure will be administered from about 1 to about 5 times per day or alternatively, as a continuous infusion. Such administration can be used as a chronic or acute therapy. The amount of active ingredient that may be combined with the carrier materials to produce a single dosage form will vary depending on the condition being treated, the severity of the condition, the time of administration, the route of administration, the rate of excretion of the compound employed, the duration of treatment, and the age, gender, weight, and condition of the patient. Preferred unit dosage formulations are those containing a daily dose or sub-dose, as herein above recited, or an appropriate fraction thereof, of an active ingredient. Treatment may be initiated with small dosages substantially less than the optimum dose of the compound. Thereafter, the dosage is increased by small increments until the optimum effect under the circumstances is reached. In general, the compound is most desirably administered at a concentration level that will generally afford antivirally effective results without causing any harmful or deleterious side effects.

When the compositions of this disclosure comprise a combination of a compound of the present disclosure and one or more additional therapeutic or prophylactic agent, both the compound and the additional agent are usually present at dosage levels of between about 10 to 150%, and more preferably between about 10 to 80% of the dosage normally administered in a monotherapy regimen. Pharmaceutical formulations may be adapted for administration by any appropriate route, for example by the oral (including buccal or sublingual), rectal, nasal, topical (including buccal, sublingual, or transdermal), vaginal, or parenteral (including subcutaneous, intracutaneous, intramuscular, intra-articular, intrasynovial, intrasternal, intrathecal, intralesional, intravenous, or intradermal injections or infusions) route. Such formulations may be prepared by any method known in the art of pharmacy, for example by bringing into association the active ingredient with the carrier(s) or excipient(s). Oral administration or administration by injection is preferred.

Pharmaceutical formulations adapted for oral administration may be presented as discrete units such as capsules or tablets; powders or granules; solution or suspensions in aqueous or non-aqueous liquids; edible foams or whips; or oil-in-water liquid emulsions or water-in-oil emulsions.

For instance, for oral administration in the form of a tablet or capsule, the active drug component can be combined with an oral, non-toxic pharmaceutically acceptable inert carrier such as ethanol, glycerol, water, and the like. Powders are prepared by comminuting the compound to a suitable fine size and mixing with a similarly comminuted pharmaceutical carrier such as an edible carbohydrate, as, for example, starch or mannitol. Flavoring, preservative, dispersing, and coloring agent can also be present.

Capsules are made by preparing a powder mixture, as described above, and filling formed gelatin sheaths. Glidants and lubricants such as colloidal silica, talc, magnesium stearate, calcium stearate, or solid polyethylene glycol can be added to the powder mixture before the filling operation. A disintegrating or solubilizing agent such as agar-agar, calcium carbonate, or sodium carbonate can also be added to improve the availability of the medicament when the capsule is ingested.

Moreover, when desired or necessary, suitable binders, lubricants, disintegrating agents, and coloring agents can also be incorporated into the mixture. Suitable binders include starch, gelatin, natural sugars such as glucose, β-lactose, corn sweetener, natural gum and synthetic resin, such as Arabic gum, tragacanth or sodium alginate, carboxymethylcellulose, polyethylene glycol, and the like. Lubricants used in these dosage forms include sodium oleate, sodium chloride, and the like. Disintegrators include, without limitation, starch, methyl cellulose, agar, betonite, xanthan gum, and the like. Tablets are formulated, for example, by preparing a powder mixture, granulating or slugging, adding a lubricant and disintegrant, and pressing into tablets. A powder mixture is prepared by mixing the compound, suitable comminuted, with a diluents or base as described above, and optionally, with a binder such as carboxymethylcellulose, an alginate, gelatin, or polyvinyl pyrrolidone, a solution retardant such as paraffin, a resorption accelerator such as a quaternary salt and/or and absorption agent such as betonite, kaolin, or dicalcium phosphate. The powder mixture can be granulated by wetting with a binder such as syrup, starch paste, acadiamucilage, or solution of cellulosic or polymeric materials and forcing through a screen. As an alternative to granulation, the powder mixture can be run through the tablet machine and the result is imperfectly formed slugs broken into granules. The granules can be lubricated to prevent sticking to the tablet forming dies by means of the addition of stearic acid, a stearate salt, talc, or mineral oil. The lubricated mixture is then compressed into tablets. The compounds of the present disclosure can also be combined with a free flowing inert carrier and compressed into tablets directly without going through the granulating or slugging steps. A clear or opaque protective coating consisting of a sealing coat of shellac, a coating of sugar or polymeric material, and a polish coating of wax can be provided. Dyestuffs can be added to these coatings to distinguish different unit dosages.

Oral fluids such as solution, syrups, and elixirs can be prepared in dosage unit form so that a given quantity contains a predetermined amount of the compound. Syrups can be prepared by dissolving the compound in a suitably flavored aqueous solution, while elixirs are prepared through the use of a non-toxic vehicle. Solubilizers and emulsifiers such as ethoxylated isostearyl alcohols and polyoxyethylene sorbitol ethers, preservatives, flavor additives such as peppermint oil or natural sweeteners, or saccharin or other artificial sweeteners, and the like can also be added.

Where appropriate, dosage unit formulations for oral administration can be microencapsulated. The formulation can also be prepared to prolong or sustain the release as for example by coating of embedding particulate material in polymers, wax, or the like.

The compounds of formula (I), and pharmaceutically acceptable salts thereof, can also be administered in the form of liposome delivery systems, such as small unilamellar vesicles, large unilamellar vesicles, and multilamellar vesicles. Liposomes can be formed from a variety of phospholipids, such as cholesterol, stearylamine, or phosphatidylcholines.

The compounds of formula (I) and pharmaceutically acceptable salts thereof may also be delivered by the use of monoclonal antibodies as individual carrier to which the compound molecules are coupled. The compounds may also be coupled with soluble polymers as targetable drug carriers. Such polymers can include polyvinylpyrrolidone, pyran copolymer, polyhydroxypropylmethacrylamidephenol, polyhydroxyethylaspartamidephenol, or polyethyleneoxidepolylysine substituted with palmitoyl residues. Furthermore, the compounds may be coupled to a class of biodegradable polymers useful in achieving controlled release of a drug, for example, polylactic acid, poly(ε-caprolactone), polyhydroxy butyric acid, polyorthoesters, polyacetals, polydihydropyrans, polycyanoacrylates, and cross-linked or amphipathic block copolymers of hydrogels.

Pharmaceutical formulations adapted for transdermal administration may be presented as discrete patches intended to remain in intimate contact with the epidermis of the recipient for a prolonged period of time. For example, the active ingredient may be delivered from the patch by iontophoresis as generally described in Pharmaceutical Research, 1986, 3(6), 318.

Pharmaceutical formulations adapted for topical administration may be formulated as ointments, creams, suspensions, lotions, powders, solutions, pastes, gels, sprays, aerosols, oils or transdermal patch.

Pharmaceutical formulations adapted for rectal administration may be presented as suppositories or as enemas.

Pharmaceutical formulations adapted for nasal administration wherein the carrier is a solid include a course powder having a particle size for example in the range 20 to 500 microns which is administered in the manner in which snuff is taken, *i.e.,* by rapid inhalation through the nasal passage from a container of the powder held close up to the nose. Suitable formulations wherein the carrier is a liquid, for administration as a nasal spray or nasal drops, include aqueous or oil solutions of the active ingredient.

Pharmaceutical formulations adapted for administration by inhalation include fine particle dusts or mists, which may be generated by means of various types of metered, dose pressurized aerosols, nebulizers, or insufflators.

Pharmaceutical formulations adapted for vaginal administration may be presented as pessaries, tampons, creams, gels, pastes, foams, or spray formulations.

Pharmaceutical formulations adapted for parenteral administration include aqueous and non-aqueous sterile injection solutions which may contain anti oxidants, buffers, bacteriostats, and solutes which render the formulation isotonic with the blood of the intended recipient; and aqueous and non-aqueous sterile suspensions which may include suspending agents and thickening agents. The formulations may be presented in unit-dose or multi-dose containers, for example sealed ampoules and vials, and may be stored in freeze-dried (lyophilized) condition requiring only the addition of the sterile liquid carrier, for example water for injections, immediately prior to use. Extemporaneous injection solutions and suspensions may be prepared from sterile powders, granules, and tablets.

It should be understood that in addition to ingredients particularly mentioned above, the formulations may include other agents conventional in the art having regard to the type of formulation in question, for example those suitable for oral administration may include flavoring agents.

### USE OF THE COMPOUNDS AND COMPOSITIONS OF THE INVENTION

Provided herein is use of the compound or the pharmaceutical composition in the manufacture of a medicament for inhibiting at least one of HCV replication process and HCV viral protein function. In some embodiments, the HCV replication process is a whole viral cycle consisting of HCV entry, uncoating, translation, replication, assembly and egress. In some embodiments, the HCV viral protein is helicase, proteinase, metalloproteinase, serine proteinase, non-structural protein NS4A, non-structural protein NS4B, RNA polymerase of non-structural protein NS5A or non-structural protein NS5B, or an internal ribosome entry site (IRES) or inosine-5'-monophosphate dehydrogenase (IMPDH) required in HCV viral replication. And any one of the compounds or the pharmaceutical compositions disclosed herein can be used for treating HCV infection or a HCV disorder, especially it is effective as inhibitor of the non-structural 5A (NS5A) protein of HCV.

Also provided herein is a method, which comprises the compound or the pharmaceutical composition disclosed herein, further comprising administering to the patient additional anti-HCV agents (combination therapy), wherein the anti-HCV agent is interferon, ribavirin, IL-2, IL-6, IL-12, a compound that enhances the development of a type 1 helper T cell response, interfering RNA, anti-sense RNA, imiquimod, an inosine-5'-monophosphate dehydrogenase inhibitor, amantadine, rimantadine, ribavirin, bavituximab, human hepatitis C immune globulin (CIVACIR™), boceprevir, telaprevir, erlotinib, daclatasvir, simeprevir, asunaprevir, vaniprevir, faldaprevir, A BT-450, danoprevir, sovaprevir, MK-5172, v edroprevir, B ZF-961, GS-9256, narlaprevir, ANA975, ABT-267, EDP239, PPI-668, GS-5816, samatasvir (IDX-719), MK-8742, MK-8325, GSK-2336805, PPI-461, TMC-435, MK-7009, BI-2013335, ciluprevir, BMS-650032, ACH-1625, ACH-1095, VX-985, IDX-375, VX-500, VX-813, PHX-1766, PHX-2054, IDX-136, IDX-316, EP-013420, VBY-376, TMC-649128, R-7128, PSI-7977, INX-189, IDX-184, IDX102, R1479, UNX-08189, PSI-6130, PSI-938, PSI-879, HCV-796, HCV-371, VCH-916, VCH-222, ANA-598, MK-3281, ABT-333, ABT-072, PF-00868554, BI-207127, GS-9190, A-837093, JKT-109, Gl-59728, GL-60667, AZD-2795, TMC647055 or a combination thereof; and the interferon is interferon α-2b, pegylated interferon α, interferon α-2a, pegylated interferon α-2a, consensus interferon-α, or interferon γ.

The treatment method that includes administering a compound or composition disclosed herein can further include administering to the patient an additional anti-HCV agent, wherein the additional anti-HCV drug is administered together with a compound or composition disclosed herein as a single dosage form or separately from the compound or composition as part of a multiple dosage form. The additional anti-HCV agent may be administered at the same time as a compound disclosed herein or at a different time. In the latter case, administration may be staggered by, for example, 6 hours, 12 hours, 1 day, 2 days, 3 days, 1 week, 2 weeks, 3 weeks, 1 month, or 2 months.

In certain embodiments disclosed herein, an "effective amount" or "effective dose" of the compound or pharmaceutically acceptable composition is that amount effective for treating or lessening the severity of one or more of the aforementioned disorders. The compounds and compositions, according to the method disclosed herein, may be administered using any amount and any route of administration effective for treating or lessening the severity of the disorder or disease. The exact amount required will vary from subject to subject, depending on the species, age, and general condition of the subject, the severity of the infection, the particular agent, its mode of administration, and the like. A compound or composition can also be administered with one or more other therapeutic agents, as discussed above.

### GENERAL SYNTHETIC PROCEDURES

Generally, the compounds disclosed herein may be prepared by methods described herein, wherein the substituents are as defined for formula (I), above, except where further noted. The following non-limiting schemes and examples are presented to further exemplify the invention.

Persons skilled in the art will recognize that the chemical reactions described may be readily adapted to prepare a number of other compounds disclosed herein, and alternative methods for preparing the compounds disclosed herein are deemed to be within the scope disclosed herein. For example, the synthesis of non-exemplified compounds according to the invention may be successfully performed by modifications apparent to those skilled in the art, *e.g.,* by appropriately protecting interfering groups, by utilizing other suitable reagents known in the art other than those described, and/or by making routine modifications of reaction conditions. Alternatively, other reactions disclosed herein or known in the art will be recognized as having applicability for preparing other compounds disclosed herein.

In the examples described below, unless otherwise indicated all temperatures are set forth in degrees Celsius. Reagents were purchased from commercial suppliers such as Aldrich Chemical Company, Arco Chemical Company and Alfa Chemical Company, and were used without further purification unless otherwise indicated. Common solvents were purchased from commercial suppliers such as Shantou XiLong Chemical Factory, Guangdong Guanghua Reagent Chemical Factory Co. Ltd., Guangzhou Reagent Chemical Factory, Tianjin YuYu Fine Chemical Ltd., Qingdao Tenglong Reagent Chemical Ltd., and Qingdao Ocean Chemical Factory.

Anhydrous THF, dioxane, toluene, and ether were obtained by refluxing the solvent with sodium. Anhydrous CH₂Cl₂ and CHCl₃ were obtained by refluxing the solvent with CaH₂. EtOAc, PE, hexane, DMAC and DMF were treated with anhydrous Na₂SO₄ prior to use.

The reactions set forth below were done generally under a positive pressure of nitrogen or argon or with a drying tube (unless otherwise stated) in anhydrous solvents, and the reaction flasks were typically fitted with rubber septa for the introduction of substrates and reagents via syringe. Glassware was oven dried and/or heat dried.

Column chromatography was conducted using a silica gel column. Silica gel (300 - 400 mesh) was purchased from Qingdao Ocean Chemical Factory. ¹H NMR spectra were obtained as CDCl₃, *d₆*-DMSO, CD₃OD or *d₆*-acetone solutions (reported in ppm), using TMS (0 ppm) or chloroform (7.25 ppm) as the reference standard. When peak multiplicities are reported, the following abbreviations are used: s (singlet), d (doublet), t (triplet), m (multiplet), br (broadened), dd (doublet of doublets), dt (doublet of triplets). Coupling constants, when given, are reported in Hertz (Hz).

Low-resolution mass spectral (MS) data were also determined on an Agilent 6320 series LC-MS spectrometer equipped with G1312A binary pumps, a G1316A TCC (Temperature Control of Column, maintained at 30 °C), a G1329A autosampler and a G1315B DAD detector were used in the analysis. An ESI source was used on the LC-MS spectrometer.

Low-resolution mass spectral (MS) data were also determined on an Agilent 6120 series LC-MS spectrometer equipped with G 1311 A Quaternary pump, a G1316A TCC (Temperature Control of Column, maintained at 30 °C), a G1329A autosampler and a G1315D DAD detector were used in the analysis. An ESI source was used on the LC-MS spectrometer.

Both LC-MS spectrometers were equipped with an Agilent Zorbax SB-C18, 2.1 x 30 mm, 5 µm column. Injection volume was decided by the sample concentration. The flow rate was 0.6 mL/min. The HPLC peaks were recorded by UV-Vis wavelength at 210 nm and 254 nm. The mobile phase was 0.1% formic acid in acetonitrile (phase A) and 0.1% formic acid in ultrapure water (phase B). The gradient condition is shown in Table 1:

**Table 1**

| Time (min) | A (CH₃CN, 0.1% HCOOH) | B (H₂O, 0.1% HCOOH) |
|---|---|---|
| 0 - 3 | 5 - 100 | 95 - 0 |
| 3 - 6 | 100 | 0 |
| 6 - 6.1 | 100 - 5 | 0 - 95 |
| 6.1 - 8 | 5 | 95 |

Purities of compounds were also assessed by Agilent 1100 Series high performance liquid chromatography (HPLC) with UV detection at 210 nm and 254 nm (Zorbax SB-C18, 2.1 x 30 mm, 4 micron, 10 min, 0.6 mL/min flow rate, 5 to 95% (0.1% formic acid in CH₃CN) in (0.1% formic acid in H₂O). Column was operated at 40 °C.

The following abbreviations are used throughout the specification:
hr(s) hour(s)
min(s) minute(s)
M mol/L
HOAc acetic acid
MeCN, CH₃CN acetonitrile
NH₃ ammonia
NH₄Cl ammonium chloride
BBr₃ boron tribromide
BSA bovine serum albumin
Br₂ bromine
BOC, Boc *tert*-butyloxycarbonyl
Cs₂CO₃ cesium carbonate
CHCl₃ chloroform
CDCl₃ chloroform deuterated
Cu copper
CuI copper (I) iodide
Et₂O diethyl ether
DMF dimethylformamide
DMAP 4-dimethylaminopyridine
DMSO dimethylsulfoxide
EDC, EDCI 1-(3-dimethylaminopropyl)-3-ethylcarbodiimide hydrochloride
Dppa diphenylphosphoryl azide
EtOAc ethyl acetate
EA ethyl acetate
HBr hydrobromic acid
HCl hydrochloric acid
HOAt, HOAT 1-hydroxy-7-azabenzotriazole
HOBT 1-hydroxybenzotriazole hydrate
H₂ hydrogen
H₂O₂ hydrogen peroxide
Fe iron
LDA lithium diisopropylamide
MCPBA meta-chloroperbenzoic acid
MgSO₄ magnesium sulfate
MeOH, CH₃OH methanol
MeI methyl iodide
CH₂Cl₂, DCM methylene chloride
NMP *N*-methylpyrrolidinone
mL, m milliliter
N₂ nitrogen
Pd/C palladium on carbon
PE petroleum ether (60-90 °C)
PBS phosphate buffered saline
POCl₃ phosphorous oxychloride
Pd(PPh₃)₄ palladium tetrakis triphenylphosphine
Pd(dppf)Cl₂ 1,1-*bis*(diphenylphosphino)ferrocene palladium chloride
K₂CO₃ potassium carbonate
KOH potassium hydroxide
RT, rt room temperature
Rt retention time
NaHCO₃ sodium bicarbonate
NaBH₄ sodium borohydride
NaBH₃CN sodium cyanoborohydride
NaOtBu sodium *tert*-butoxide
NaOH sodium hydroxide
NaClO₂ sodium chlorite
NaCl sodium chloride
NaH₂PO₄ sodium dihydric phosphate
NaH sodium hydride
NaI sodium iodide
Na₂SO₄ sodium sulfate
TBTU O-benzotriazol-1-yl-*N,N,N',N'*-tetramethyluronium tetrafluoroborate
THF tetrahydrofuran
Et₃N, TEA triethylamine
TFA trifluoroacetic acid
P(t-bu)₃ tri(*tert*-butyl)phosphine
NBS *N*-bromosuccinimide
TBAI tetrabutylammonium iodide
H₂O water
TEAF formic acid triethylamine complex 5:2
PPA polyphosphoric acid
Tf₂O Trifluoromethanesulfonic anhydride
HCl.EA a solution of HCl in ethyl acetate
DIPEA *N,N*-diisopropylethylamine
DME 1,2-dimethoxyethane
HATU 2-(7-aza-1*H*-benzotriazole-1-yl)-1,1,3,3-tetramethyluronium hexafluorophosphate
NIS *N*-iodosuccinimide
TFAA trifluoroaceticanhydride
SEMCl 2-(Trimethylsilyl)ethoxymethyl chloride
Dess-Martin(Dess-Martin periodinane)
(1,1,1-Triacetoxy)-1,1-dihydro-1,2-benziodoxol-3(1*H*)-one
*p*-TSA(TsOH) *p*-toluenesulfonic acid
TMSA Trimethyl silyl acetylene
Meldrum's acid 2,2-Dimethyl-1,3-dioxane-4,6-dione
BAST *Bis*(2-methoxyethyl)aminosulphurtrifluoride Deoxo-fluor
SbCl₃ antimony trichloride
SmCl₃ samarium chloride
LiHMDS lithium hexamethyldisilazide
TMSCl trimethyl chlorosilane
PhNTf₂ *N,N*-Bis(trifluoromethylsulfonyl)aniline
TBDMSOTf *tert*-butyldimethylsilyl triflate
Et₂NSF₃ diethylaminosulfur trifluoride
MTBE methyl *tert*-butyl ether
LiN(SiMe₃)₂ Lithium *bis*(trimethylsilyl)amide
PPh₃MeBr Methyltriphenylphosphonium bromide
Lawesson's Reagent 2,4-*bis*(4-methoxyphenyl)-1,3-dithia-2,4-diphosphetane 2,4-disulfide
TEBAC benzyltriethylammonium chloride
I₂ iodine
DAST Diethylaminosulfur trifluoride
IPA isopropanol
TCCA trichloroisocyanuric acid
TEMPO 2,2,6,6-tetramethylpiperidinooxy

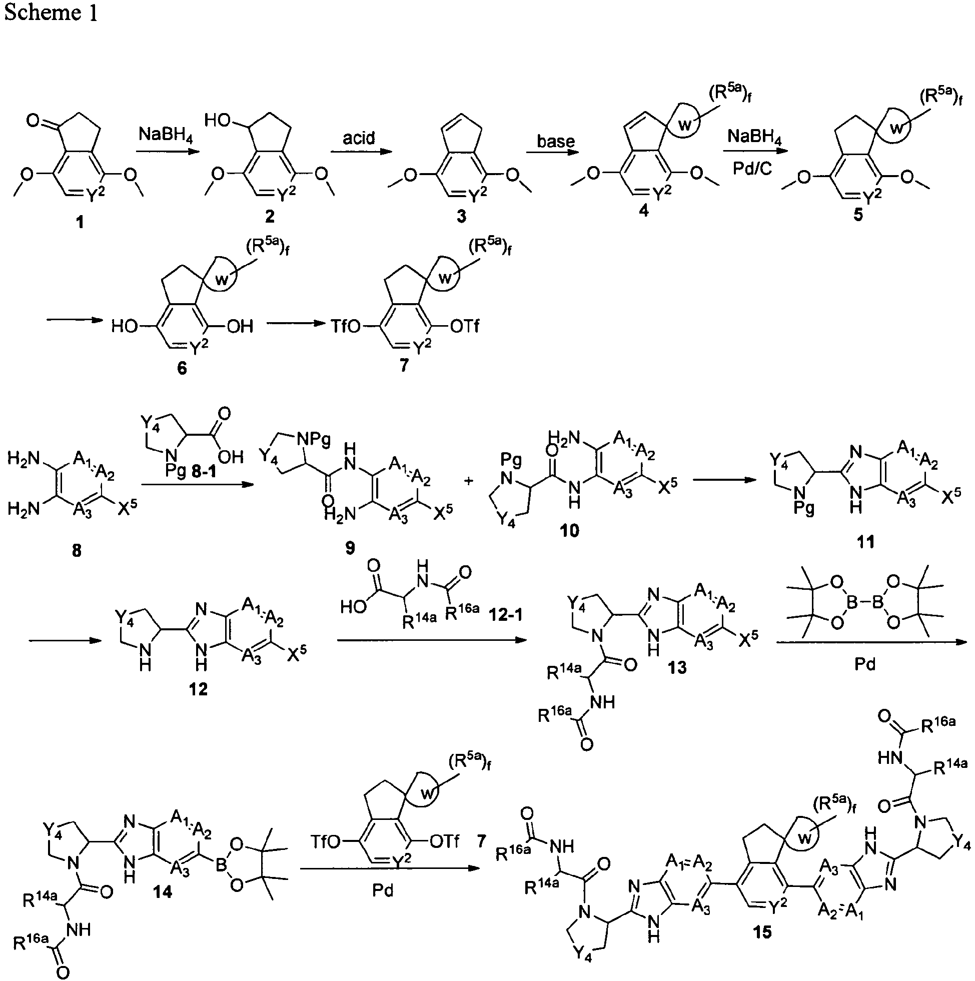

Compound 15 can be prepared by a general synthetic procedure illustrated in Scheme 1, wherein each of A¹, A² and A³ is independently N or CR⁷; X⁵ is F, Cl, Br or I; each of W, Y², Y₄, R^{5a}, f, R^{14a} and R^{16a} is as defined herein; and Pg is an amino-protecting group such as Boc, Fmoc or Cbz. Reduction of compound **1** with a reducing agent such as NaBH₄ can give compound **2,** which is converted to compound **3** in the presence of acid. Compound **4** can be prepared through cyclization at elevated temperature in the presence of base from compound **3.** Compound 4 can react with a reducing agent such as NaBH₄ in the presence of Pd/C to give compound **5.** The methyl group in compound **5** can then be removed in the presence of boron tribromide to provide compound **6.** Compound **6** can react with trifluoromethanesulfonic anhydride to afford compound **7** by base catalysis. Condensation of compound **8** with compound **8-1** can give a mixture of compound **9** and compound **10.** Then compound **9** and compound **10** can be cyclized at elevated temperature in acetic acid system to give compound **11.** The protecting group Pg in compound **11** can be removed to provide compound **12.** Compound **12** can be condensed with compound **12-1** to afford compound **13.** Compound **13** can further react with *bis*(pinacolato)diboron in the presence of Pd catalyst to afford compound **14.** Coupling reaction of compound **14** with compound **7** in the presence of Pd catalyst can give compound **15.**

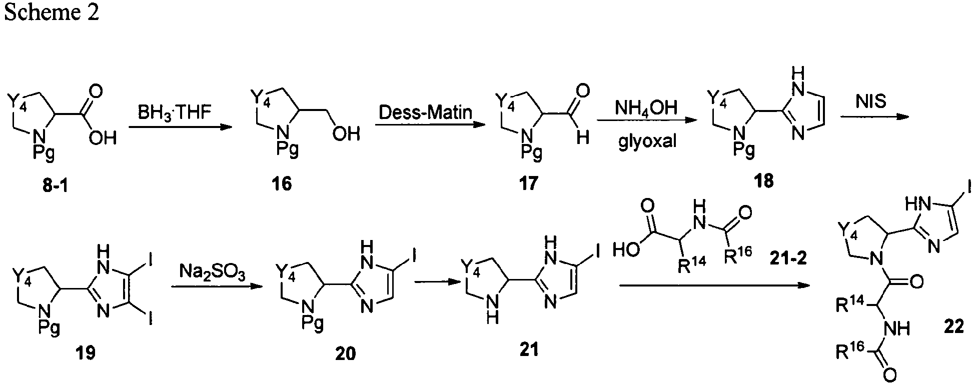

Compound **22** can be prepared by a general synthetic procedure illustrated in Scheme 2, wherein each of Y₄, R¹⁴ and R¹⁶ is as defined herein; and Pg is an amino-protecting group such as Boc, Fmoc or Cbz. Reduction of compound **8-1** with borane-tetrahydrofuran can give compound **16.** Compound **16** can be oxidized to give compound **17** with an oxidant such as Dess-Martin Periodinane. Compound **17** can be cyclized in the presence of ammonium hydroxide and glyoxal to give compound **18.** Compound **18** can react with NIS to afford di-iodo compound **19.** One iodine in di-iodo compound **19** can then be removed in the presence of sodium sulfite to provide compound **20.** The protecting group Pg in compound **20** can be removed to provide compound **21,** which in turn can be condensed with compound **21-2** to afford compound **22.**

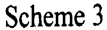

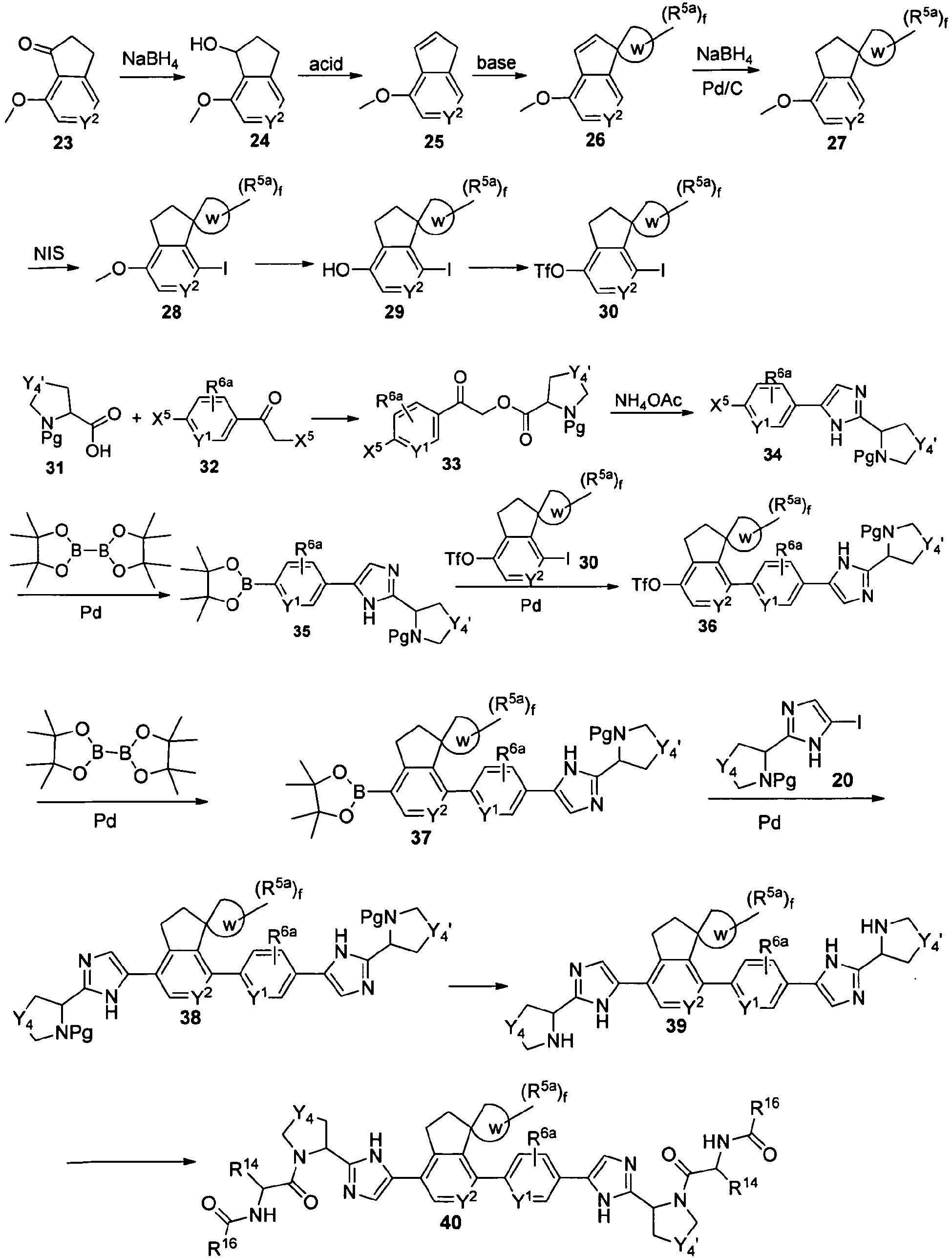

Compound **40** can be prepared by a general synthetic procedure illustrated in Scheme 3, wherein X⁵ is F, Cl, Br or I; each of W, Y₄, Y₄', R^{5a}, R^{6a}, Y¹, Y², f, R¹⁴ and R¹⁶ is as defined herein; and Pg is an amino-protecting group such as Boc, Fmoc or Cbz. Reduction of compound **23** with a reducing agent such as NaBH₄ can give compound **24,** which can be converted to compound **25** in the presence of acid. Compound **25** can give compound **26** at elevated temperature in the presence of base by cyclization. Compound **26** can react with a reducing agent such NaBH₄ in the presence of Pd/C to give compound **27.** Compound **27** can be transformed to compound **28** by reacting with NIS. The methyl group in compound **28** can then be removed in the presence of boron tribromide to provide compound **29.** Compound **29** can react with trifluoromethanesulfonic anhydride to afford compound **30** by base catalysis. Condensation of compound **31** with compound **32** can give compound **33.** Compound **33** can be cyclized in the presence of ammonium acetate to afford compound **34.** Compound **34** can further react with *bis*(pinacolato)diboron to afford compound **35** by Pd catalysis. Coupling reaction of compound **35** with compound **30** in the presence of Pd catalyst can give compound **36.** Compound **36** can further react with *bis*(pinacolato)diboron in the presence of Pd catalyst to afford compound **37.** Coupling reaction of compound **37** with compound **20** in the presence of Pd catalyst can give compound **38.** The protecting group Pg in compound **38** can be removed to afford compound **39.** Compound **39** can be condensed with compound **21-2** to provide compound **40.**

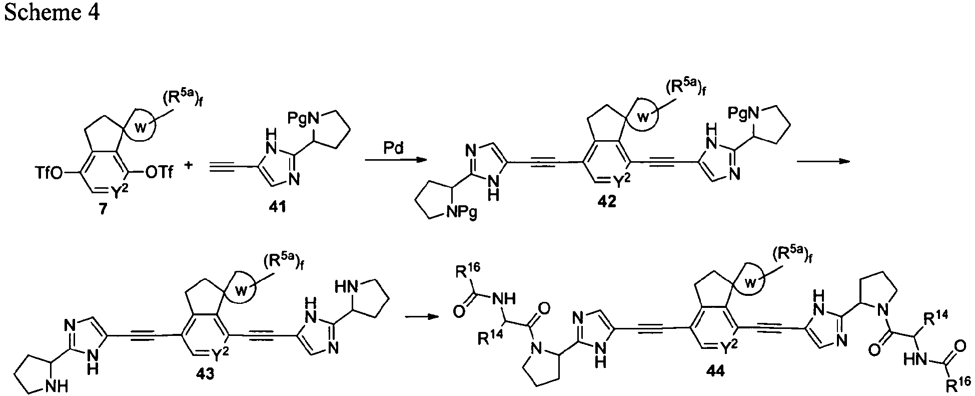

Compound **44** can be synthesized through a general synthetic procedure depicted in Scheme 4, wherein each of R^{5a}, Y², f, R¹⁴ and R¹⁶ is as defined herein; and Pg is an amino-protecting group such as Boc, Fmoc or Cbz. Coupling reaction of compound **41** with compound **7** in the presence of Pd catalyst can give compound **42,** then the protecting group Pg in compound **42** can be removed to afford compound **43.** Compound **43** can be condensed with compound 21-2 to provide compound **44.**

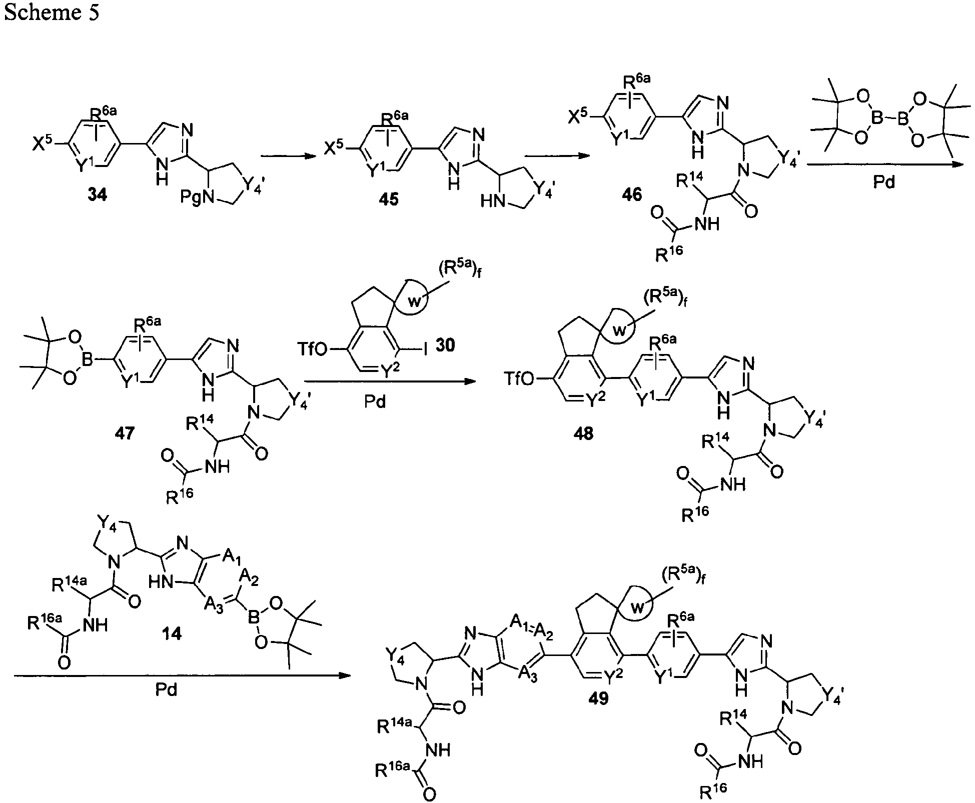

Compound 49 can be synthesized through a general synthetic procedure depicted in Scheme 5, wherein each of W, Y₄', Y₄, R^{5a}, R^{6a}, Y¹, Y², f, R¹⁴, R^{14a}, R¹⁶ and R^{16a} is as defined herein; X⁵ is F, Cl, Br or I; and Pg is an amino-protecting group such as Boc, Fmoc or Cbz. The protecting group Pg in compound **34** can be removed to provide compound **45.** Compound **45** can be condensed with compound **21-2** to afford compound 46. Compound **46** can further react with *bis*(pinacolato)diboron in the presence of Pd catalyst to afford compound **47.** Coupling reaction of compound **47** with compound **30** in the presence of Pd catalyst can give compound **48.** Compound **48** can further react with compound **14** in the presence of Pd catalyst to afford compound **49.**

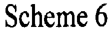

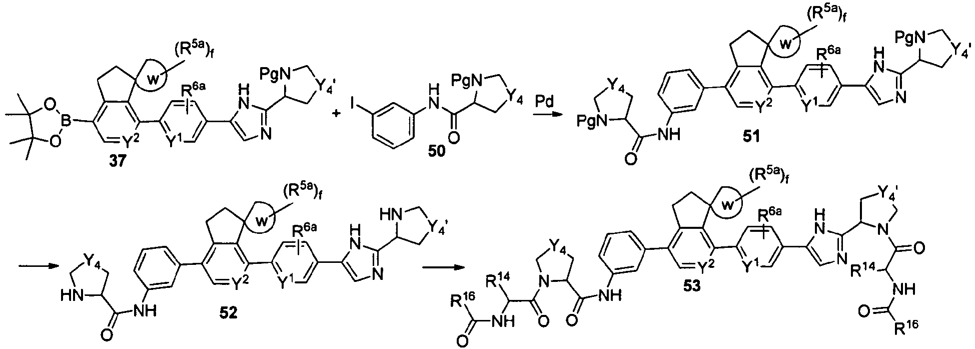

Compound **53** can be prepared by a general synthetic procedure illustrated in Scheme 6, wherein each of W, Y₄', Y₄, R^{5a}, R^{6a}, Y¹, Y², f, R¹⁴ and R¹⁶ is as defined herein; and Pg is an amino-protecting group such as Boc, Fmoc or Cbz. Coupling reaction of compound **37** with compound **50** in the presence of Pd catalyst can give compound **51.** The protecting group Pg in compound **51** can be removed to afford compound **52,** which can be condensed with compound **21-2** to provide compound **53.**

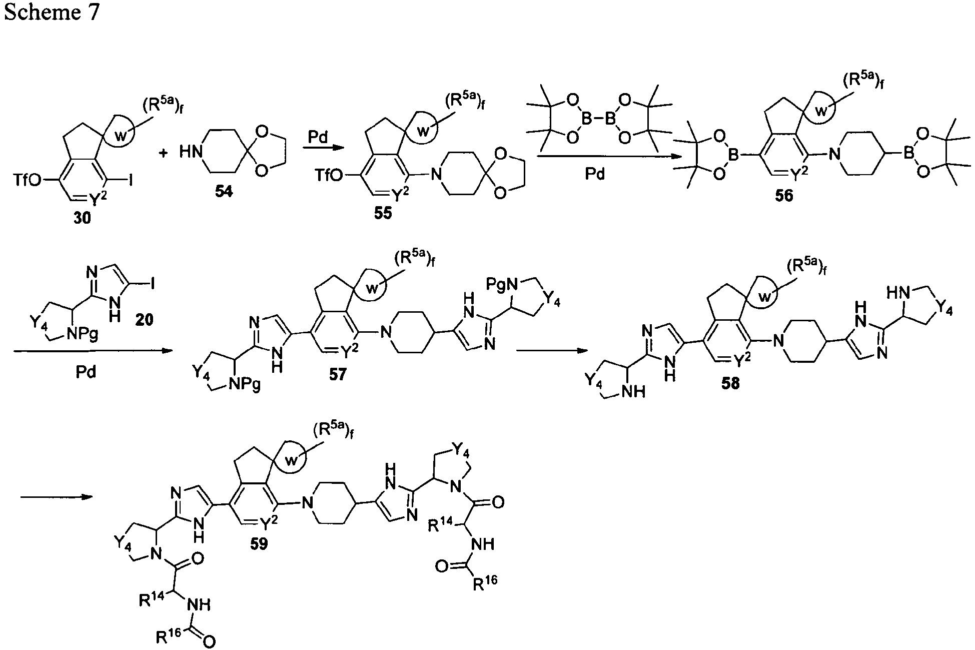

Compound **59** can be synthesized through a general synthetic procedure depicted in Scheme 7, wherein each of W, Y₄, Y², R^{5a}, f, R¹⁴ and R¹⁶ is as defined herein; and Pg is an amino-protecting group such as Boc, Fmoc or Cbz. Coupling reaction of compound **30** with compound **54** in the presence of Pd catalyst can give compound **55.** Compound **55** can react with SmCl₃/TMSCl and LiHMDS/PhNTf₂ to afford an intermediate in a polar solvent such as THF, then the intermediate can further react with *bis*(pinacolato)diboron in the presence of Pd catalyst to afford compound **56.** Compound **56** can be converted to compound **57** by reacting with compound **20** in the presence of Pd catalyst. The protecting group Pg in compound **57** can be removed to afford compound **58,** which can be condensed with compound **21-2** to provide compound **59.**

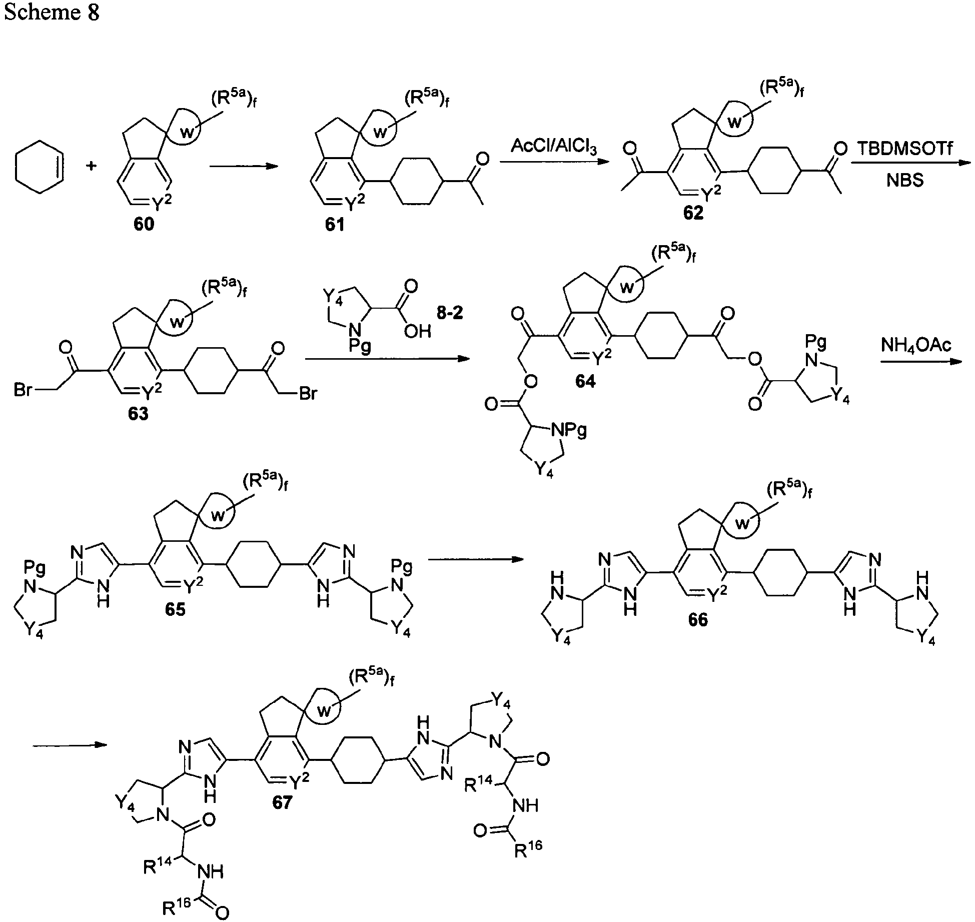

Compound **67** can be synthesized through a general synthetic procedure depicted in Scheme **8,** wherein each of W, Y₄, Y², R^{5a}, f, R¹⁴ and R¹⁶ is as defined herein; and Pg is an amino-protecting group such as Boc, Fmoc or Cbz. Compound **60** can be converted to compound **61** by reacting with cyclohexene in the presence of acyl chloride and aluminium chloride in a polar solvent such as CS₂. Compound **61** can react with acetyl chloride to afford compound **62** in the presence of aluminium chloride. Compound **62** can be converted to compound **63** in the presence of a brominating agent such as TBDMSOTf/NBS. Condensation of compound **63** with compound **8-2** can give compound **64.** Compound **64** can be cyclized in the presence of ammonium acetate to afford compound **65.** The protecting group Pg in compound **65** can be removed to afford compound **66,** which can be condensed with compound **21-2** to provide compound **67.**

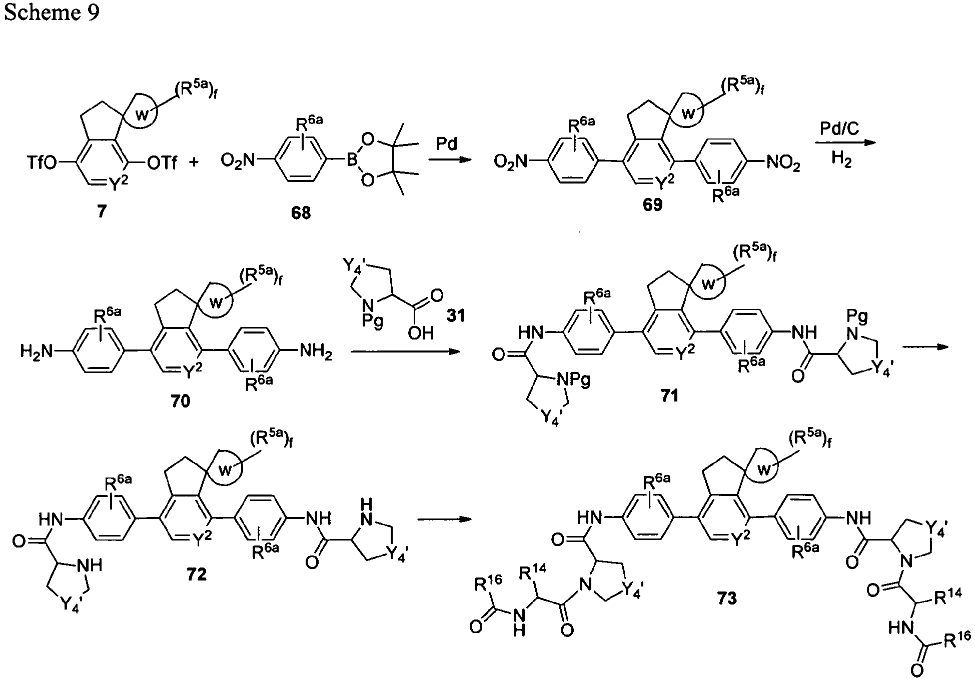

Compound **73** can be synthesized through a general synthetic procedure depicted in Scheme **9,** wherein each of W, Y₄', Y², R^{5a}, R^{6a}, f, R¹⁴ and R¹⁶ is as defined herein; and Pg is an amino-protecting group such as Boc, Fmoc or Cbz. Coupling reaction of compound **68** with compound **7** in the presence of Pd catalyst can give compound **69.** Reduction of compound **69** with a reducing agent such as Pd/C by catalytic hydrogenation can afford compound 70. Condensation of compound **70** with compound **31** can give compound **71** by base catalysis. The protecting group Pg in compound **71** can be removed to afford compound **72,** which can be condensed with compound **21-2** to provide compound **73.**

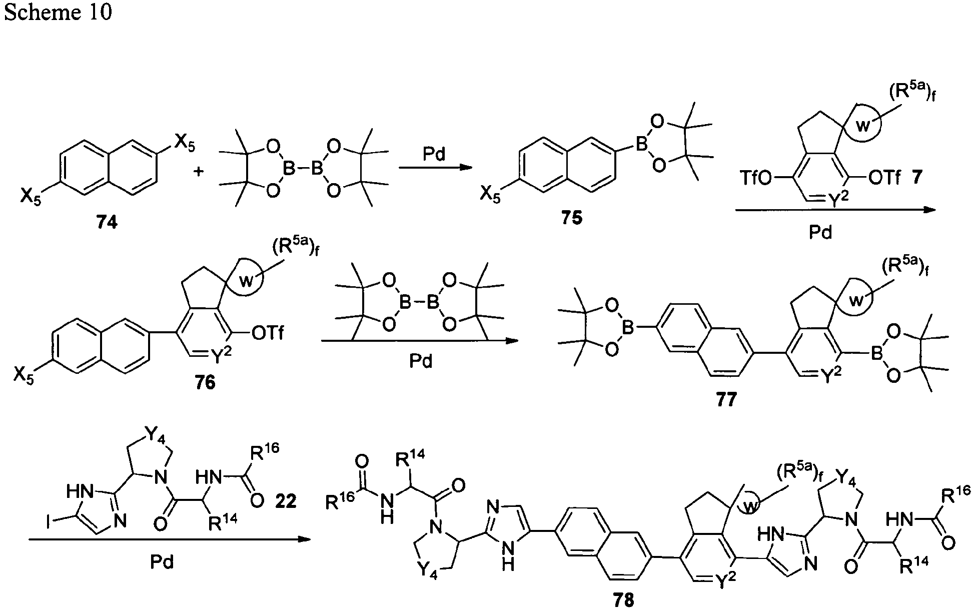

Compound **78** can be prepared by a general synthetic procedure illustrated in Scheme 10, wherein X⁵ is F, Cl, Br or I; and each of W, Y², Y₄, R^{5a}, f, R¹⁴ and R¹⁶ is as defined herein. Reaction of compound **74** with *bis*(pinacolato)diboron can afford compound **75** by Pd catalysis. Coupling reaction of compound **75** with compound **7** in the presence of Pd catalyst can give compound **76.** Compound **76** can further react with *bis*(pinacolato)diboron in the presence of Pd catalyst to afford compound **77.** Compound **77** can be converted to compound **78** by reacting with compound **22** in the presence of Pd catalyst.

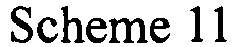

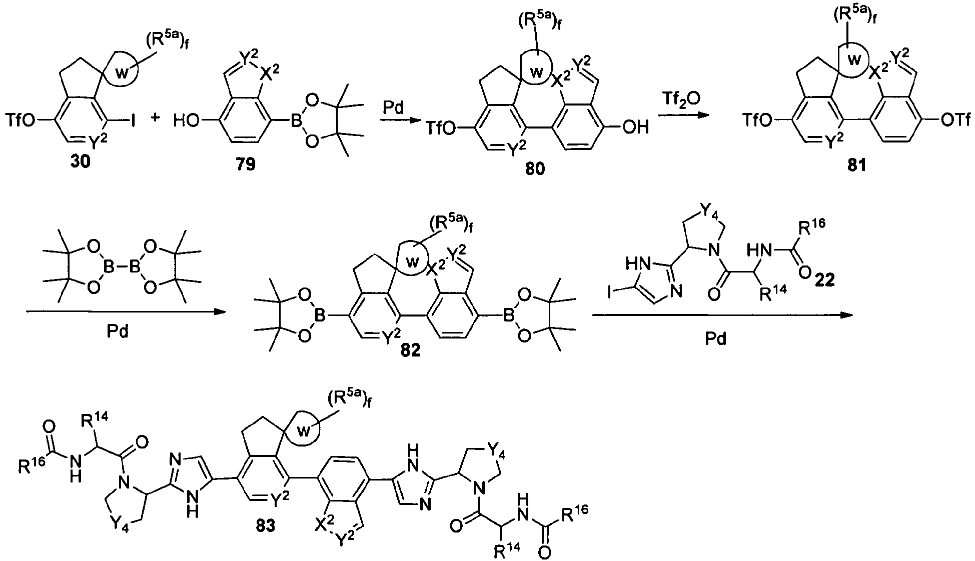

Compound **83** can be prepared by a general synthetic procedure illustrated in Scheme 11, wherein each of W, Y², Y₄, R^{5a}, f, R¹⁴ and R¹⁶ is as defined herein. Coupling reaction of compound **79** with compound **30** in the presence of Pd catalyst can give compound **80.** Compound **80** can react with trifluoromethanesulfonic anhydride to afford compound **81** by base catalysis. Reaction of compound **81** with *bis*(pinacolato)diboron can afford compound **82** by Pd catalysis. Coupling reaction of compound **82** with compound **22** in the presence of Pd catalyst can give compound 83.

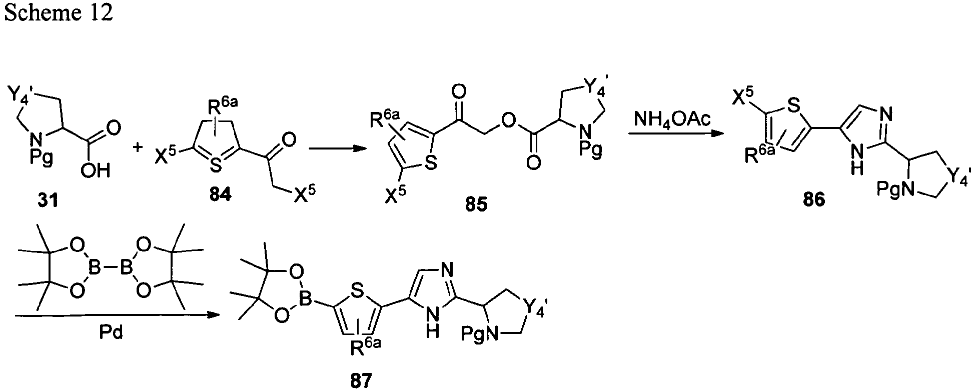

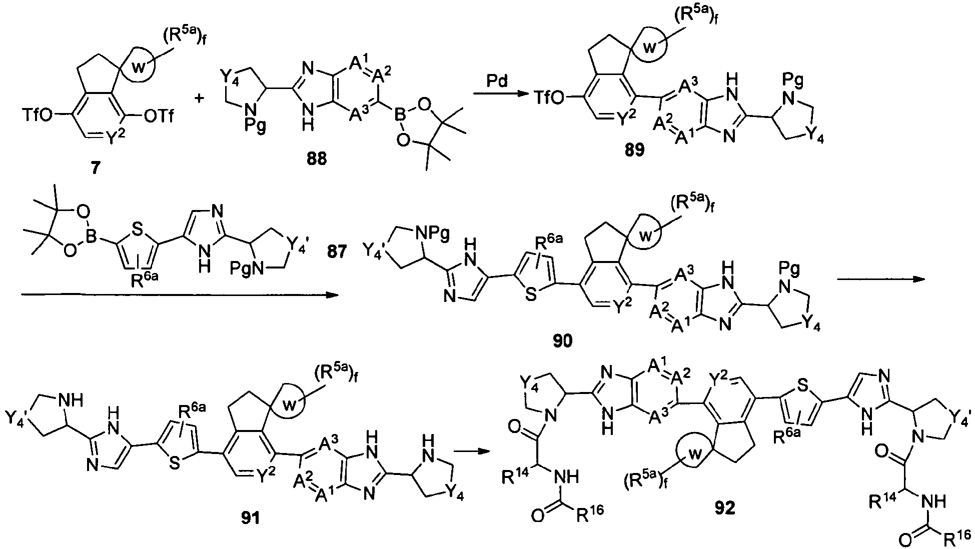

Compound **92** can be synthesized through a general synthetic procedure depicted in Scheme 12, wherein each of A¹, A² and A³ is independently N or CR⁷; X⁵ is F, Cl, Br or I; each of W, Y₄', Y₄, Y², R^{5a}, R^{6a}, f, R¹⁴ and R¹⁶ is as defined herein; and Pg is an amino-protecting group such as Boc, Fmoc or Cbz. Condensation of compound **84** with compound **31** can give compound **85** by base catalysis. Compound **85** can be cyclized at elevated temperature in the presence of ammonium acetate to afford compound **86.** Reaction of compound **86** with *bis*(pinacolato)diboron can afford compound 87 by Pd catalysis. Coupling reaction of compound **88** with compound **7** in the presence of Pd catalyst can give compound **89.** Compound **89** can further react with compound **87** in the presence of Pd catalyst to afford compound **90.** The protecting group Pg in compound **90** can be removed to afford compound **91,** which can be condensed with compound **21-2** to provide compound **92.**

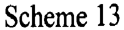

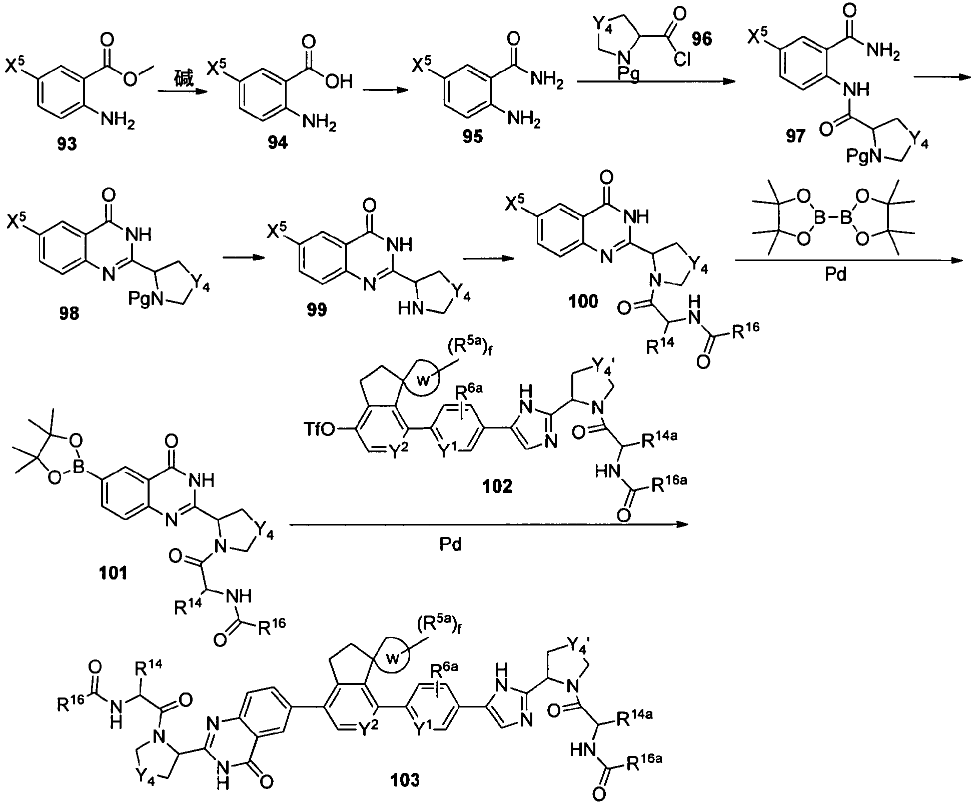

Compound **103** can be synthesized through a general synthetic procedure depicted in Scheme 13, wherein X⁵ is F, Cl, Br or I; each of W, Y₄', Y₄, Y¹, Y², R^{5a}, R^{6a}, f, R¹⁴, R^{14a}, R¹⁶ and R^{16a} is as defined herein; and Pg is an amino-protecting group such as Boc, Fmoc or Cbz. Compound **93** can be converted to compound **94** by base catalysis. Compound **94** can be converted to compound **95** in the presence of HOBT/EDCI and ammonium hydroxide. Condensation of compound **95** with compound **96** can give compound **97** by base catalysis. Compound **97** can be cyclized in the presence of base to afford compound **98.** The protecting group Pg in compound **98** can be removed to afford compound **99,** which can be condensed with compound **21-2** to provide compound **100.** Reaction of compound **100** with *bis*(pinacolato)diboron can afford compound **101** by Pd catalysis. Coupling reaction of compound **101** with compound **102** in the presence of Pd catalyst can give compound **103.**

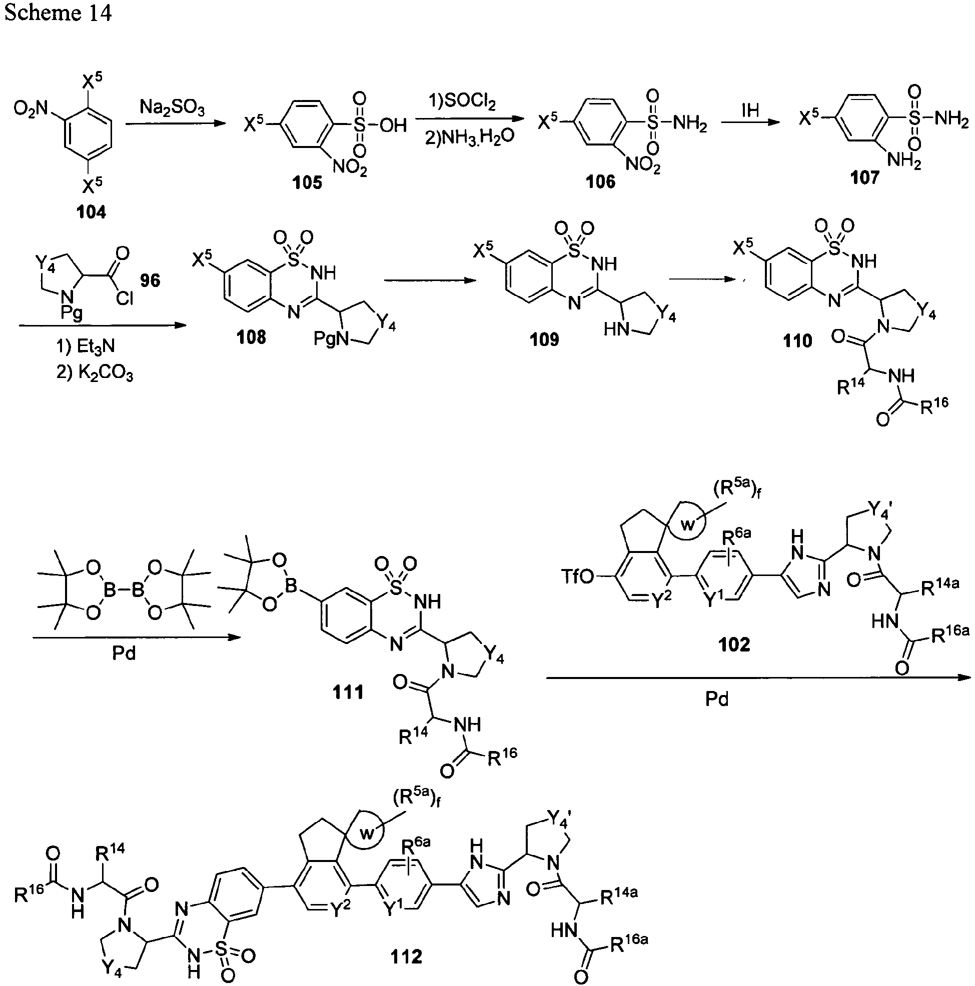

Compound **112** can be synthesized through a general synthetic procedure depicted in Scheme 14, wherein X⁵ is F, Cl, Br or I; each of W, Y₄', Y₄, Y¹, Y², R^{5a}, R^{6a}, f, R¹⁴, R^{14a}, R¹⁶ and R^{16a} is as defined herein; and Pg is an amino-protecting group such as Boc, Fmoc or Cbz. Compound **104** can be transformed to compound **105** by reacting with sodium sulfite. Compound **105** can be converted to compound **106** in the presence of thionyl chloride and ammonium hydroxide. Reduction of compound **106** with a reducing agent such as HI can afford compound **107.** Condensation of compound **107** with compound **96** can afford compound **108** in the presence of base. The protecting group Pg in compound **108** can be removed to afford compound **109,** which can be condensed with compound **21-2** to provide compound **110.** Reaction of compound **110** with *bis*(pinacolato)diboron can afford compound **111** by Pd catalysis. Coupling reaction of compound **111** with compound **102** in the presence of Pd catalyst can give compound **112.**

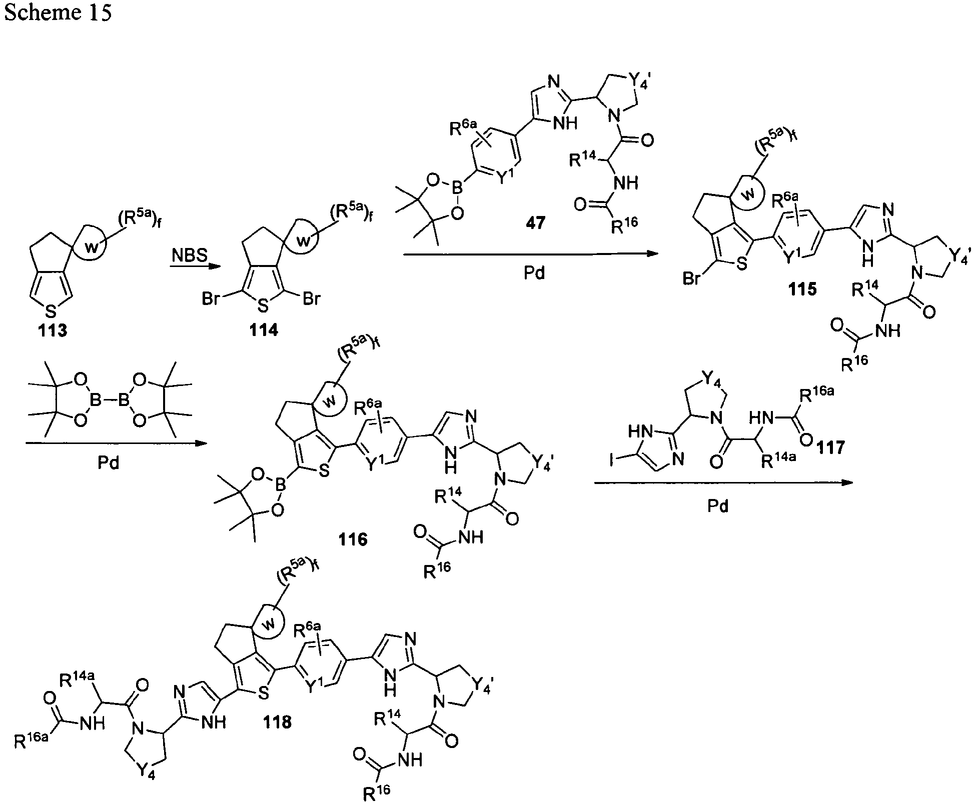

Compound **118** can be prepared by a general synthetic procedure illustrated in Scheme 15, wherein each of W, Y₄', Y₄, Y¹, R^{5a}, R^{6a}, f, R¹⁴, R^{14a}, R¹⁶ and R^{16a} is as defined herein. Compound **113** can be converted to compound **114** in the presence of a brominating agent such as NBS. Coupling reaction of compound **114** with compound **47** in the presence of Pd catalyst can give compound **115.** Reaction of compound **115** with *bis*(pinacolato)diboron can afford compound **116** by Pd catalysis. Coupling reaction of compound **116** with compound **117** in the presence of Pd catalyst can give compound **118.**

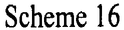

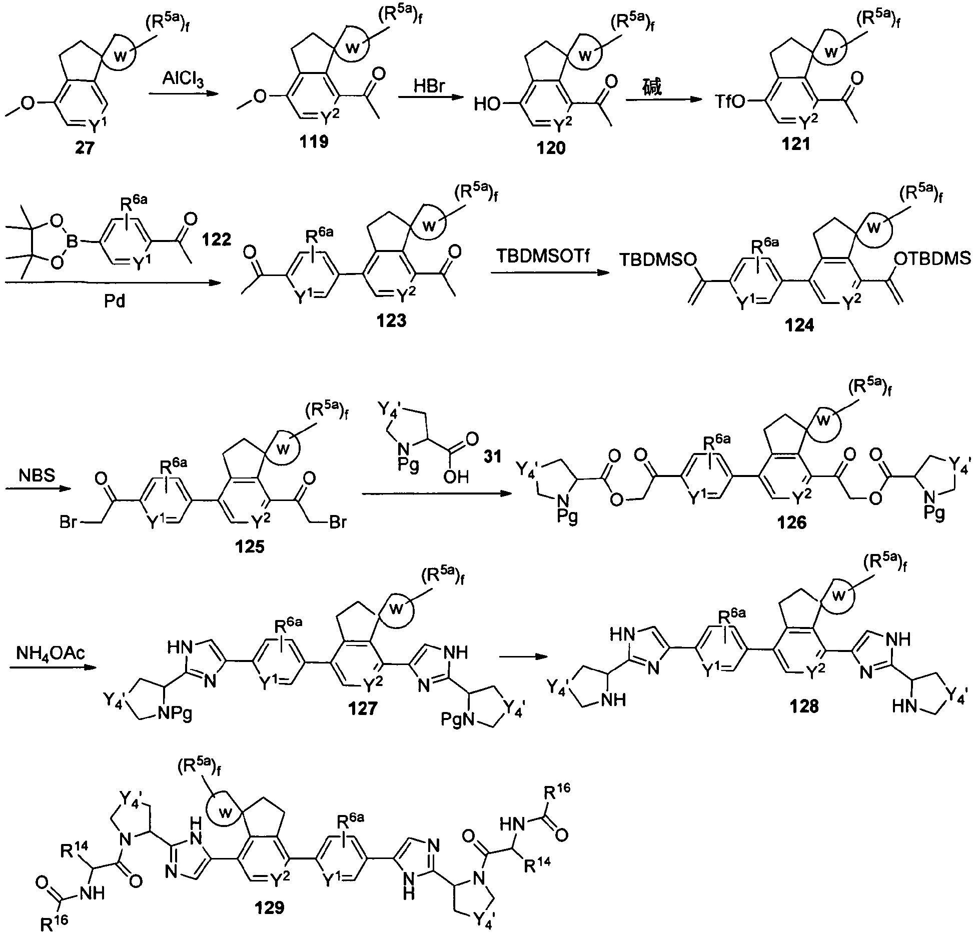

Compound **129** can be synthesized through a general synthetic procedure depicted in Scheme 16, wherein each of W, Y₄', Y¹, Y², R^{5a}, R^{6a}, f, R¹⁴ and R¹⁶ is as defined herein; and Pg is an amino-protecting group such as Boc, Fmoc or Cbz. Compound **27** can react with acyl chloride to afford compound **119** in the presence of aluminium chloride. The methyl group in compound **119** can then be removed in the presence of hydrogen bromide to provide compound **120.** Compound **120** can react with trifluoromethanesulfonic anhydride to afford compound **121** by base catalysis. Coupling reaction of compound **121** with compound **122** in the presence of Pd catalyst can give compound **123.** Compound **123** can be converted to compound **124** in the presence of TBDMSOTf. Compound **124** can react with NBS to give compound **125.** Condensation of compound **125** with compound **31** can give compound **126.** Compound **126** can be cyclized at elevated temperature in the presence of ammonium acetate to afford compound **127.** The protecting group Pg in compound **127** can be removed to afford compound **128,** which can be condensed with compound **21-2** to provide compound **129.**

### EXAMPLES

### Example 1

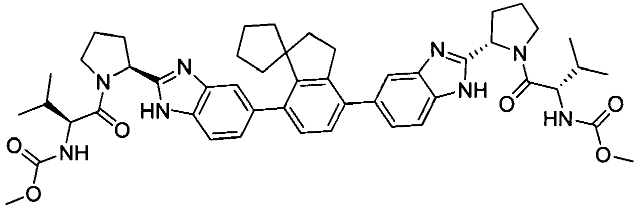

### Synthetic route:

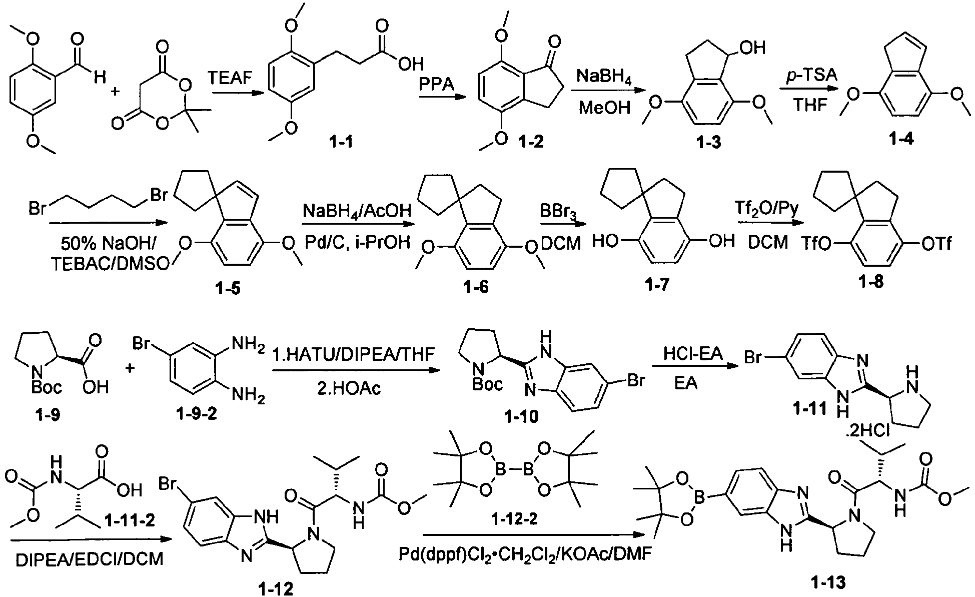

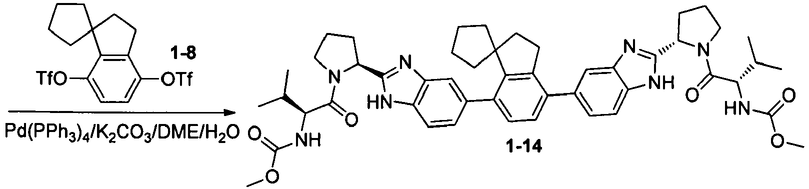

### Step 1) the preparation of compound 1-1

To formic acid (3.7 mL) was added TEA (5.4 mL) at 0 °C followed by 2,5-dimethoxybenzaldehyde (2.0 g, 12 mmol) and 2,2-dimethyl-1,3-dioxane-4,6- dione (1.73 g, 12 mmol) in a portionwise manner. At the end of the addition, the mixture was stirred at 100 °C for 2.0 hrs. After the reaction was completed, the mixture was quenched with ice water (50 mL). The resulting mixture was adjusted to pH 1 with hydrochloric acid (2 M), and the aqueous layer was extracted with EtOAc (50 mL x 3). The combined organic layers were washed with brine, dried over anhydrous Na₂SO₄ and concentrated *in vacuo.* The residue was purified by silica gel column chromatography (PE/EtOAc (v/v) = 3/1) to give the title compound as a white solid (2.1 g, 83%). The compound was characterized by the following spectroscopic data:
MS (ESI, pos.ion) *m*/*z:* 211.1 [M+H]⁺;
¹H NMR (400 MHz, CDCl₃) *δ* (ppm): 6.70-6.78 (m, 6H), 3.78 (s, 3H), 3.75 (s, 3H), 2.91 (t, 2H, *J* = 7.8 Hz), 2.65 (t, 2H, *J* = 7.8 Hz).

### Step 2) the preparation of compound 1-2

A mixture of compound **1-1** (4.68 g, 22.3 mmol) and PPA (50.87 g, 24.8 mmol) was stirred at 80 °C for 4.0 hrs. After the reaction was completed, ice water (250 mL) was added to the mixture, and the resulting mixture was extracted with EtOAc (100 mL x 5). The combined organic layers were washed with NaHCO₃ aqueous solution and brine, dried over anhydrous Na₂SO₄ and concentrated *in vacuo.* The residue was purified by silica gel column chromatography (PE/EtOAc (v/v) = 3/1) to give the title compound as a pale yellow solid (3.0 g, 70.0%). The compound was characterized by the following spectroscopic data:
MS (ESI, pos.ion) *m*/*z*: 193.2 [M+H]⁺;
¹H NMR (400 MHz, CDCl₃) *δ* (ppm): 6.98 (d, 1H, *J* = 8.7 Hz), 6.73 (d, 1H, *J* = 8.7 Hz), 3.90 (s, 3H), 3.85 (s, 3H), 2.97-3.00 (m, 2H), 2.65-2.68 (m, 2H).

### Step 3) the preparation of compound 1-3

To a solution of compound **1-2** (11.52 g, 60 mmol) in methanol (150 mL) was added NaBH₄ (1.3 g, 40 mmol). At the end of the addition, the mixture was stirred at rt for 1.0 hr. After the reaction was completed, the mixture was quenched with water (50 mL), and the MeOH solvent was removed. The resulting mixture was extracted with EtOAc (50 mL x 3). The combined organic layers were washed with brine, dried over anhydrous Na₂SO₄ and concentrated *in vacuo.* The residue was purified by silica gel column chromatography (PE/EtOAc (v/v) = 4/1) to give the title compound (9.78 g, 84%). The compound was characterized by the following spectroscopic data:
MS (ESI, pos.ion) *m*/*z:* 195.1 [M+H]⁺;
¹H NMR (400 MHz, CDCl₃) *δ* (ppm): 6.63-6.70 (m, 2H), 5.45-5.48 (m, 1H), 3.82 (s, 3H), 3.78 (s, 3H), 2.99-3.07 (m, 1H), 2.68-2.78 (m, 2H), 2.44-2.46 (m, 1H), 1.97-2.06 (m, 1H).

### Step 4) the preparation of compound 1-4

To a solution of compound **1-3** (3.18 g, 16.4 mmol) in THF (100 mL) was added *p*-TSA (1.4 g, 8.2 mmol) at 0 °C. The mixture was refluxed for 2.0 hrs. After the reaction was completed, the mixture was cooled to rt, quenched with NaHCO₃ aqueous solution (50 mL), and the THF solvent was removed. The resulting mixture was extracted with EtOAc (50 mL x 3). The combined organic layers were washed with brine, dried over anhydrous Na₂SO₄ and concentrated *in vacuo.* The residue was purified by silica gel column chromatography (PE) to give the title compound as a white solid (1.2 g, 42%). The compound was characterized by the following spectroscopic data:
MS (ESI, pos.ion) *m*/*z*: 177.2 [M+H]⁺;
¹H NMR (400 MHz, CDCl₃) *δ* (ppm): 6.99-7.01 (m, 1H), 6.74 (d, 1H, *J* = 4.32 Hz), 6.66 (d, 1H, *J* = 4.32 Hz), 6.47-6.49 (m, 1H), 3.86 (s, 3H), 3.85 (s, 3H), 3.37 (s, 3H).

### Step 5) the preparation of compound 1-5

To a solution of compound **1-4** (1.20 g, 6.82 mmol), 1, 4-dibromobutane (1.54 g, 7.15 mmol) and TEBAC (0.3 g, 1.36 mmol) in DMSO (30 mL) was added sodium hydroxide aqueous solution (50%, 2 mL) at 0 °C. At the end of the addition, the mixture was stirred at 50 °C for 2.0 hrs. After the reaction was completed, the mixture was quenched with water (50 mL). The aqueous layer was extracted with EtOAc (50 mL x 3). The combined organic layers were washed with brine, dried over anhydrous Na₂SO₄ and concentrated *in vacuo.* The residue was purified by silica gel column chromatography (PE) to give the title compound as yellow oil (1.08 g, 69%). The compound was characterized by the following spectroscopic data:
MS (ESI, pos.ion) *m*/*z:* 231.1 [M+H]⁺;
¹H NMR (400 MHz, *d*₆-DMSO) *δ* (ppm): 6.67-6.69 (m, 2H), 6.61 (s, 1H, *J* = 4.36 Hz), 6.43 (s, 1H, *J* = 4.36 Hz), 3.81 (s, 3H), 3.79 (s, 3H), 2.38-2.43 (m, 2H), 1.96-2.03 (m, 2H), 1.82-1.86 (m, 2H), 1.54-1.60 (m, 2H).

### Step 6) the preparation of compound 1-6

To a solution of compound **1-5** (1.08 g, 4.69 mmol) in isopropanol (15 mL) were added a catalytic amount of Pd/C (100 mg), acetic acid (0.56 g, 9.38 mmol) and NaB H₄ (0.7 g, 18.76 mmol) in turn. At the end of the addition, the mixture was stirred at rt for 1.0 hr. After the reaction was completed, the mixture was filtered through a celite pad. The filtrate was concentrated *in vacuo.* The residue was purified by silica gel column chromatography (PE) to give the title compound as yellow oil (0.89 g, 82%). The compound was characterized by the following spectroscopic data:
MS (ESI, pos.ion) *m*/*z:* 233.1 [M+H]⁺;
¹H NMR (400 MHz, CDCl₃) *δ* (ppm): 6.59-6.64 (m, 2H), 3.77 (s, 3H), 3.75 (s, 3H), 2.77-2.81 (m, 2H), 2.03-2.18 (m, 2H), 1.94-1.91 (m, 2H), 1.82-1.78 (m, 2H), 1.68-1.64 (m, 2H), 1.58-1.56 (m, 2H).

### Step 7) the preparation of compound 1-7

To a solution of compound **1-6** (0.89 g, 3.84 mmol) in DCM (15 mL) was added boron tribromide (2.2 mL, 23.04 mmol) dropwise at -78 °C. At the end of the addition, the mixture was stirred at rt for 1.0 hr. After the reaction was completed, the mixture was quenched with ice water (20 mL) and the organic phase was separated. The aqueous layer was extracted with DCM (20 mL x 3). The combined organic layers were washed with brine, dried over anhydrous Na₂SO₄ and concentrated *in vacuo.* The residue was purified by silica gel column chromatography (PE/EtOAc (v/v) = 4/1) to give the title compound as a white solid (0.64 g, 80%). The compound was characterized by the following spectroscopic data:
MS (ESI, pos.ion) *m*/*z:* 205.1 [M+H]⁺;
¹H NMR (400 MHz, CDCl₃) *δ* (ppm): 8.37 (s, 1H), 8.31 (s, 1H), 6.35 (s, 2H), 2.61-2.65 (m, 2H), 2.18-2.11 (m, 2H), 1.81-1.77 (m, 2H), 1.75-1.69 (m, 2H), 1.63-1.59 (m, 2H), 1.47-1.45 (m, 2H).

### Step 8) the preparation of compound 1-8

To a solution of compound **1-7** (0.62 g, 3.04 mmol) in DCM (15 mL) was added pyridine (1.95 mL, 24.32 mmol) dropwise at 0 °C. After the mixture was stirred for 10 mins, trifluoromethanesulfonic anhydride (2.04 mL, 12.16 mmol) was added. At the end of the addition, the mixture was stirred at rt for 2.0 hrs. After the reaction was completed, the mixture was diluted with DCM (50 mL). The resulting mixture was washed with brine, dried over anhydrous Na₂SO₄ and concentrated *in vacuo.* The residue was purified by silica gel column chromatography (PE) to give the title compound as yellow oil (1.32 g, 93%). The compound was characterized by the following spectroscopic data:
¹H NMR (400 MHz, CDCl₃) *δ* (ppm): 7.21 (d, 1H, *J* = 4.52 Hz), 7.14 (d, 1H, *J* = 4.52 Hz), 3.03-2.98 (m, 2H), 2.13-2.04 (m, 4H), 1.90-1.85 (m, 2H), 1.79-1.71 (m, 4H).

### Step 9) the preparation of compound 1-10

To a solution of compound **1-9** (20 g, 107 mmol) and compound HATU (48.82 g, 128.4 mmol) in THF (250 mL) was added DIPEA (19.5 mL, 118 mmol) at 0 °C. After stirring at 0 °C for 0.5 hr, compound **1-9-2** (22.2 g, 119 mmol) was added in a portionwise manner. At the end of the addition, the mixture was stirred at rt for 4.0 hrs. After the reaction was completed, the mixture was quenched with water (100 mL), the THF solvent was removed, and the resulting mixture was extracted with EtOAc (200 mL x 3). The combined organic layers were washed with brine, dried over anhydrous Na₂SO₄ and concentrated *in vacuo.* The residue was dissolved in glacial acetic acid (100 mL), and the solution was stirred at 40 °C overnight. After the reaction was completed, the mixture was concentrated *in vacuo.* The residue was dissolved in EtOAc (400 mL). The mixture was washed with Na₂CO₃ aqueous solution (150 mL x 3), dried over anhydrous Na₂SO₄ and concentrated *in vacuo.* The residue was purified by silica gel column chromatography (PE/EtOAc (v/v) = 1/2) to give the title compound (35 g, 81%). The compound was characterized by the following spectroscopic data:
MS (ESI, pos.ion) *m*/*z*: 367.3 [M+H]⁺;
¹H NMR (400 MHz, CDCl₃) *δ* (ppm): 7.68 (s, 1H), 7.42-7.40 (m, 1H), 7.30-7.28 (m, 1H), 5.11-5.09 (m, 1H), 3.45-3.43 (m, 2H), 2.94-2.93 (m, 1H), 2.21-2.18 (m, 2H), 2.01-1.91 (m, 1H), 1.49 (s, 9H).

### Step 10) the preparation of compound 1-11

To a solution of compound **1-10** (10.0 g, 27.39 mmol) in EtOAc (50.0 mL) was added a solution of HCl in EtOAc (60.0 mL, 4 M) dropwise at 0 °C. The mixture was stirred at rt for 8.0 hrs. After the reaction was completed, the mixture was filtered, and the filter cake was washed with EtOAc to give the title compound as a pale yellow solid (8.0 g, 86.49%). The compound was characterized by the following spectroscopic data:
MS (ESI, pos.ion) *m*/*z*: 313.2 [M+H]⁺;
¹H NMR (400 MHz, CDCl₃) *δ* (ppm): 8.01 (s, 1H), 7.70-7.76 (m, 2H), 5.27-5.25 (m, 1H), 3.31-3.30 (m, 2H), 2.77-2.74 (m, 1H), 2.54-2.52 (m, 1H), 2.40-2.37 (m, 1H), 2.30-2.10 (m, 1H).

### Step 11) the preparation of compound 1-12

To a solution of compound **1-11** (6.0 g, 18.8 mmol), compound **1-11-2** (4.9 g, 28.2 mmol) and EDCI (5.4 g, 28.2 mmol) in DCM (100.0 mL) was added DIPEA (18.64 mL, 112.8 mmol) dropwise at 0 °C. At the end of the addition, the mixture was stirred at rt for 3.0 hrs. After the reaction was completed, the mixture was quenched with water (100 mL) and extracted with DCM (150 mL x 3). The combined organic layers were washed with brine, dried over anhydrous Na₂SO₄ and concentrated *in vacuo.* The residue was purified by silica gel column chromatography (PE/EtOAc (v/v) = 1/2) to give the title compound as a solid (5.77 g, 85%). The compound was characterized by the following spectroscopic data:
MS (ESI, pos.ion) *m*/*z*: 423.3 [M+H]⁺;
¹H NMR (400 MHz, CDCl₃) *δ* (ppm): 7.59-7.51 (m, 1H), 7.34-7.21 (m, 2H), 5.42-5.38 (m, 2H), 4.34-4.30 (m, 1H), 3.87-3.76 (m, 1H), 3.70 (s, 3H), 3.66-3.62 (m, 1H), 3.04-2.98 (m, 1H), 2.25-2.21 (m, 1H), 2.20-2.13 (m, 2H), 1.96-1.94 (m, 1H), 0.88-0.84 (m, 6H).

### Step 12) the preparation of compound 1-13

To a mixture of compound **1-12** (3.0 g, 7.1 mmol), compound **1-12-2** (2.72 g, 10.7 mmol), Pd(dppf)Cl₂·CH₂Cl₂ (653 mg, 0.8 mmol) and KOAc (2.09 g, 21.3 mmol) was added DMF (30 mL) via syringe under N₂, and the mixture was stirred at 90 °C for 3.0 hrs. After the reaction was completed, the mixture was cooled to rt and diluted with EtOAc (200 mL). The resulting mixture was filtered through a celite pad. The filtrate was washed with water (50 mL x 3) and brine, dried over anhydrous Na₂SO₄ and concentrated *in vacuo.* The residue was purified by silica gel column chromatography (PE/EtOAc (v/v) = 1/2) to give the title compound as a beige solid (2.1 g, 62.87%). The compound was characterized by the following spectroscopic data:
MS (ESI, pos.ion) *m*/*z*: 471.3 [M+H]⁺;
¹H NMR (400 MHz, CDCl₃) *δ* (ppm): 7.87-7.80 (m, 1H), 7.71-7.66 (m, 2H), 5.47-5.42 (m, 2H), 4.34-4.30 (m, 1H), 3.86-3.84 (m, 1H), 3.70 (s, 3H), 3.64-3.62 (m, 1H), 3.04-2.98 (m, 1H), 2.25-2.21 (m, 1H), 2.20-2.13 (m, 2H), 1.96-1.94 (m, 1H), 1.35 (s, 12H), 0.88-0.84 (m, 6H).

### Step 13) the preparation of compound 1-14

To a suspension of compound **1-8** (50 mg, 0.11 mmol), compound **1-13** (131.68 mg, 0.28 mmol), Pd(PPh₃)₄ (25 mg, 0.02 mmol) and K₂CO₃ (91 mg, 0.66 mmol) were added DME (2.0 mL) and pure water (0.4 mL) via syringe, and the mixture was stirred at 90 °C under N₂ for 3.0 hrs. After the reaction was completed, the mixture was cooled to rt and diluted with EtOAc (20 mL). The resulting mixture was washed with water and brine, dried over anhydrous Na₂SO₄ and concentrated *in vacuo.* The residue was purified by silica gel column chromatography (DCM/MeOH (v/v) = 50/1) to give the title compound as a pale yellow solid (75.37 mg, 80%). The compound was characterized by the following spectroscopic data:
MS (ESI, pos.ion) *m*/*z*: 857.5 [M+H]⁺;
¹H NMR (400 MHz, CDCl₃) *δ* (ppm): 7.78-7.69 (m, 2H), 7.48-7.47 (m, 1H), 7.38-7.32 (m, 2H), 7.22-7.18 (m, 2H), 7.06-7.01 (m, 1H), 5.54-5.53 (m, 2H), 5.48-5.46 (m, 2H), 4.37-4.33 (m, 2H), 3.89-3.87 (m, 2H), 3.71 (s, 6H), 3.13-3.11 (m, 2H), 2.94-2.92 (m, 2H), 2.48-2.32 (m, 2H), 2.29-2.11 (m, 4H), 2.09-1.92 (m, 4H), 1.58-1.36 (m, 4H), 1.82-1.72 (m, 8H), 1.11-1.03 (m, 2H), 0.92-0.86 (m, 12H).

### Example 2

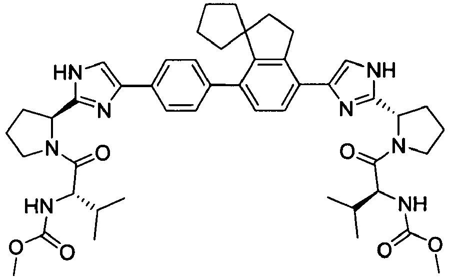

### Synthetic route:

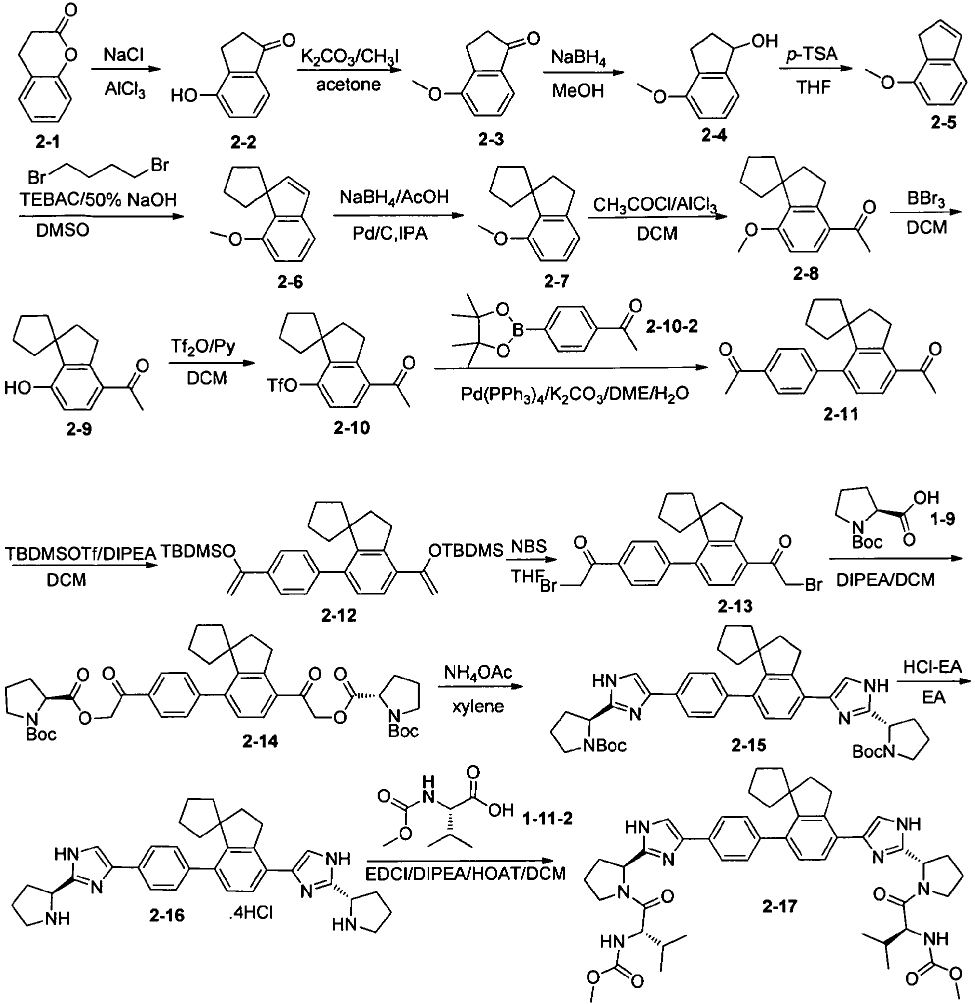

### Step 1) the preparation of compound 2-2

A mixture of aluminium chloride (90.0 g, 676 mmol) and sodium chloride (25 g, 432 mmol) was stirred at 150 °C until the mixture was in molten state, and then compound **2-1** (20.0 g, 135 mmol) was added dropwise. At the end of the addition, the mixture was stirred at 200 °C for 1.0 hr. After the reaction was completed, the mixture was cooled to rt, poured slowly into ice water (500 mL) and filtered to get the crude product. The crude product was purified by beating in MeOH to give the title compound as a gray solid (19 g, 95%). The compound was characterized by the following spectroscopic data:
MS (ESI, pos.ion) *m*/*z*: 149.5 [M+H]⁺;
¹H NMR (400 MHz, CDCl₃) *δ* (ppm): 7.41-7.38 (m, 1H), 7.24-7.19 (m, 1H), 6.80-6.79, 6.78-6.77 (d, d, 1H, *J* = 4.0 Hz), 5.46 (br, 1H), 3.06-3.03 (m, 2H), 2.69-2.66 (m, 2H).

### Step 2) the preparation of compound 2-3

To a mixture of compound **2-2** (5.0 g, 33.7 mmol) and K₂CO₃ (23.4 g, 168.5 mmol) in acetone (50.0 mL) was added iodomethane (3.15 mL, 50.55 mmol) dropwise. The mixture was stirred at 60 °C for 5.0 hrs. After the reaction was completed, the mixture was concentrated *in vacuo.* To the residue was added EtOAc (150 mL) and water (150 mL), and the mixture was filtered through a celite pad. The aqueous layer was extracted with EtOAc (150 mL x 2). The combine organic layers were washed with brine, dried over anhydrous Na₂SO₄ and concentrated *in vacuo.* The residue was purified by silica gel column chromatography (PE/EtOAc (v/v) = 10/1) to give the title compound as a yellow solid (2.5 g, 45%). The compound was characterized by the following spectroscopic data:
MS (ESI, pos.ion) *m*/*z*: 163.5 [M+H]⁺;
¹H NMR (400 MHz, CDCl₃) *δ* (ppm): 7.51-7.48 (m, 1H), 7.30-7.26 (m, 1H), 6.91-6.87 (m, 1H), 3.90 (s, 3H), 3.08-3.05 (m, 2H), 2.70-2.67 (m, 2H).

### Step 3) the preparation of compound 2-4

To a solution of compound **2-3** (20 g, 123.3 mmol) in MeOH (250 mL) was added NaBH₄ (2.8 g, 74.0 mmol) in a portionwise manner. At the end of the addition, the mixture was stirred at rt for 1.0 hr. After the reaction was completed, the MeOH solvent was removed and the residue was dissolved in EtOAc (400 mL). The resulting mixture was washed with water (50 mL x 2) and brine (50 mL), dried over anhydrous Na₂SO₄ and concentrated *in vacuo.* The residue was purified by silica gel column chromatography (PE/EtOAc (v/v) = 5/1) to give the title compound as a pale yellow solid (17.6 g, 87%). The compound was characterized by the following spectroscopic data:
MS (ESI, pos.ion) *m*/*z:* 165.5 [M+H]⁺;
¹H NMR (400 MHz, CDCl₃) δ (ppm): 7.13-7.09 (m, 1H), 7.08-7.05 (m, 1H), 6.75-6.72 (m, 1H), 5.29-5.25 (m, 1H), 3.84 (d, 3H), 3.70 (brs, 1H), 2.84-2.80 (m, 2H), 2.49-2.40 (m, 1H), 1.96-1.88 (m, 1H).

### Step 4) the preparation of compound 2-5

To a solution of compound 2-4 (2.0 g, 12.2 mmol) in THF (20 mL) was added *p*-TSA (1.0 g, 6.1 mmol) at 0 °C. At the end of the addition, the mixture was refluxed for 3.0 hrs. After the reaction was completed, the THF solvent was removed. To the residue was added EtOAc (100 mL). The resulting mixture was washed with water (50 mL x 3) and brine, dried over anhydrous Na₂SO₄ and concentrated *in vacuo.* The residue was purified by silica gel column chromatography (hexane) to give the title compound as colorless oil (1.23 g, 69%). The compound was characterized by the following spectroscopic data:
MS (ESI, pos.ion) *m*/*z:* 147.1 [M+H]⁺;
¹H NMR (400 MHz, CDCl₃) δ (ppm): 7.29 (t, 1H, *J=* 7.8 Hz), 7.09 (d, 1H, *J =* 7.4 Hz), 6.90-6.86 (m, 1H), 6.77 (d, 1H, *J =* 8.0 Hz), 6.61-6.57 (m, 1H), 3.92 (s, 3H), 3.39 (s, 2H).

### Step 5) the preparation of compound 2-6

To a suspension of compound **2-5** (12.4 g, 84.8 mmol), 1,4-dibromobutane (19.2 g, 89.1 mmol) and TEBAC (3.9 g, 17.0 mmol) in DMSO (60 mL) was added sodium hydroxide aqueous solution (50%, 13.6 mL) at 0 °C. At the end of the addition, the mixture was stirred at 50 °C for 2.0 hrs. After the reaction was completed, the mixture was diluted with PE (500 mL). The resulting mixture was washed with water (100 mL x 3) and brine, dried over anhydrous Na₂SO₄ and concentrated *in vacuo.* The residue was purified by silica gel column chromatography (PE) to give the title compound as colorless liquid (6.0 g, 35%). The compound was characterized by the following spectroscopic data:
MS (ESI, pos.ion) *m*/*z:* 201.2 [M+H]⁺;
¹H NMR (400 MHz, CDCl₃) *δ* (ppm): 7.21 (t, 1H, *J =* 7.8 Hz), 6.94 (dd, 1H), 6.72 (d, 1H, *J =* 8.2 Hz), 6.56-6.52 (m, 2H), 2.50-2.40 (m, 2H), 2.08-1.98 (m, 2H), 1.93-1.82 (m, 2H), 1.62-1.54 (m, 2H).

### Step 6) the preparation of compound 2-7

To a solution of compound **2-6** (4.0 g, 20.0 mmol) in isopropanol (30.0 mL) were added a catalytic amount of Pd/C (400 mg), glacial acetic acid (2.4 g, 40.0 mmol) and Na BH₄ (3.0 g, 80.0 mmol) in turn at 0 °C. At the end of the addition, the mixture was stirred at rt for 1.0 hr. After the reaction was completed, the mixture was filtered through a celite pad. The filtrate was concentrated *in vacuo.* The residue was purified by silica gel column chromatography (PE) to give the title compound as colorless liquid (3.9 g, 97%). The compound was characterized by the following spectroscopic data:
MS (ESI, pos.ion) m/z: 203.1 [M+H]⁺;
¹H NMR (400 MHz, CDCl₃) *δ* (ppm): 7.11 (t, 1H, *J =* 7.7 Hz), 6.80 (dd, 1H), 6.67 (d, 1H, *J =* 8.1 Hz), 3.79 (s, 3H), 2.84 (t, 2H, *J=* 7.3 Hz), 2.22-2.12 (m, 2H), 1.92 (t, 2H, *J=* 7.4 Hz), 1.86-1.75 (m, 2H), 1.73-1.60 (m, 2H), 1.60-1.52 (m, 2H).

### Step 7) the preparation of compound 2-8

To a suspension of aluminium chloride (3.9 g, 28.9 mmol) in DCM (15 mL) was added acetyl chloride (1.8 mL, 25.1 mmol) dropwise at 0 °C. After the mixture was stirred for 10 mins, a solution of compound **2-7** (3.9 g, 19.3 mmol) in DCM (25 mL) was added dropwise to the reaction mixture. At the end of the addition, the mixture was stirred at rt for 1.0 hr. After the reaction was completed, the mixture was quenched with ice water (100 mL) and the organic phase was separated. The aqueous layer was extracted with DCM (50 mL x 3). The combined organic layers were washed with brine, dried over anhydrous Na₂SO₄ and concentrated *in vacuo.* The residue was purified by silica gel column chromatography (PE/EtOAc (v/v) = 40/1) to give the title compound as pale yellow oil (4.5 g, 96%). The compound was characterized by the following spectroscopic data:
MS (ESI, pos.ion) *m*/*z*: 245.1 [M+H]⁺;
¹H NMR (400 MHz, CDCl₃) *δ* (ppm): 7.72 (d, 1H, *J =* 8.6 Hz), 6.73 (d, 1H, *J =* 8.6 Hz), 3.87 (s, 3H), 3.21 (t, 2H, *J=* 7.4 Hz), 2.54 (s, 3H), 2.19-2.08 (m, 2H), 1.93 (t, 2H, *J=* 7.4 Hz), 1.85-1.74 (m, 2H), 1.73-1.62 (m, 2H), 1.59-1.50 (m, 2H).

### Step 8) the preparation of compound 2-9

To a solution of compound **2-8** (4.5 g, 18.4 mmol) in DCM (30 mL) was added boron tribromide (8.8 mL, 92.1 mmol) dropwise at -78 °C. At the end of the addition, the mixture was stirred at -78 °C for 10 mins and at rt for another 1.0 hr. After the reaction was completed, the mixture was quenched with ice water (100 mL) and the organic phase was separated. The aqueous layer was extracted with DCM (100 mL x 3). The combined organic layers were washed with brine, dried over anhydrous Na₂SO₄ and concentrated *in vacuo.* The residue was purified by silica gel column chromatography (PE/EtOAc (v/v) = 10/1) to give the title compound as a white solid (3.4 g, 80%). The compound was characterized by the following spectroscopic data:
MS (ESI, pos.ion) m/z: 231.2 [M+H]⁺;
¹H NMR (400 MHz, CDCl₃) *δ* (ppm): 7.62 (d, 1H, *J =* 8.4 Hz), 6.64 (d, 1H, *J =* 8.4 Hz), 6.04 (s, 1H), 3.23 (t, 2H, *J=* 7.4 Hz), 2.54 (s, 3H), 2.22-2.13 (m, 2H), 1.96 (t, 2H, *J =* 7.4 Hz), 1.88-1.78 (m, 2H), 1.77-1.69 (m, 2H), 1.66-1.59 (m, 2H).

### Step 9) the preparation of compound 2-10

To a solution of compound **2-9** (3.4 g, 14.8 mmol) in DCM (40 mL) was added pyridine (3.6 mL, 44.3 mmol) dropwise at 0 °C. After the mixture was stirred for 10 mins, trifluoromethanesulfonic anhydride (5.0 mL, 29.5 mmol) was added dropwise. At the end of the addition, the mixture was stirred at rt for 1.0 hr. After the reaction was completed, the mixture was quenched with ice water (50 mL). The aqueous layer was extracted with DCM (50 mL x 3). The combined organic layers were washed with brine, dried over anhydrous Na₂SO₄ and concentrated *in vacuo.* The residue was purified by silica gel column chromatography (PE/EtOAc (v/v) = 40/1) to give the title compound as pale yellow liquid (4.5 g, 84%). The compound was characterized by the following spectroscopic data:
MS (ESI, pos.ion) *m*/*z*: 362.1 [M+H]⁺;
¹H NMR (400 MHz, CDCl₃) *δ* (ppm): 7.72 (d, 1H, *J =* 8.7 Hz), 7.23 (d, 1H*, J =* 8.7 Hz), 3.23 (t, 2H, *J =* 7.4 Hz), 2.58 (s, 3H), 2.13-2.02 (m, 2H), 2.00 (t, 2H, *J =* 7.4 Hz), 1.94-1.80 (m, 2H), 1.80-1.68 (m, 4H).

### Step 10) the preparation of compound 2-11

To a mixture of compound **2-10** (4.5 g, 12.4 mmol), compound **2-10-2** (3.2 g, 13.0 mmol), Pd(PPh₃)₄ (720 mg, 0.62 mmol) and K₂CO₃ (3.4 g, 24.8 mmol) were added DME (25.0 mL) and pure water (5.0 mL) via syringe, and the mixture was stirred at 90 °C under N₂ for 3.0 hrs. After the reaction was completed, the mixture was cooled to rt and diluted with EtOAc (100 mL). The resulting mixture was washed with water and brine, dried over anhydrous Na₂SO₄ and concentrated *in vacuo.* The residue was purified by silica gel column chromatography (PE/EtOAc (v/v) = 10/1) to give the title compound as a pale yellow solid (3.0 g, 73%). The compound was characterized by the following spectroscopic data:
MS (ESI, pos.ion) m/z: 333.1 [M+H]⁺;
¹H NMR (400 MHz, CDCl₃) *δ* (ppm): 7.98 (d, 2H, *J =* 8.3 Hz), 7.67 (d, 1H, *J =* 7.9 Hz), 7.38 (d, 2H*, J =* 8.3 Hz), 6.99 (d, 1H, *J =* 7.9 Hz), 3.22 (t, 2H, *J =* 7.1 Hz), 2.66 (s, 3H), 2.63 (s, 3H), 1.89 (t, 2H, *J* = 7.1 Hz), 1.61-1.53 (m, 4H), 1.52-1.42 (m, 2H), 1.34-1.22 (m, 2H).

### Step 11) the preparation of compound 2-12

To a solution of compound **2-11** (2.2 g, 6.62 mmol) in DCM (20 mL) were added DIPEA (4.0 mL, 23.16 mmol) and TBDMSOTf (3.5 mL, 15.22 mmol) dropwise at 0 °C separately. At the end of the addition, the mixture was stirred at 0 °C for 30 mins. After the reaction was completed, the mixture was quenched with water (50 mL). The resulting mixture was extracted with DCM (50 mL x 3). The combined organic layers were washed with brine, dried over anhydrous Na₂SO₄ and concentrated *in vacuo.* To the residue was added MTBE (10 mL). The mixture was fully shake, and then filtered. The filtrate was concentrated *in vacuo* to give the title compound as tawny oil, which was used for the next step without further purification.

### Step 12) the preparation of compound 2-13

To a suspension of NBS (2.6 g, 14.56 mmol) in THF (20 mL) was added a solution of compound **2-12** (3.7 g, 6.62 mmol) in THF (30 mL) dropwise at -20 °C. At the end of the addition, the mixture was stirred at -20 °C for 15 mins. After the reaction was completed, the mixture was quenched with saturated NaHCO₃ aqueous solution (50 mL). The resulting mixture was extracted with EtOAc (40 mL x 3). The combined organic layers were washed with brine, dried over anhydrous Na₂SO₄ and concentrated *in vacuo.* The residue was purified by silica gel column chromatography (PE/EtOAc (v/v) = 20/1) to give the title compound as pale yellow oil (2.0 g, 62%). The compound was characterized by the following spectroscopic data:
MS (ESI, pos.ion) *m*/*z*: 491 [M+H]⁺;
¹H NMR (400 MHz, CDCl₃) *δ* (ppm): 8.02 (d, 2H, *J =* 8.3 Hz), 7.68 (d, 1H, *J =* 8.0 Hz), 7.41 (d, 2H, *J =* 8.3 Hz), 7.02 (d, 1H, *J =* 8.0 Hz), 4.50 (s, 2H), 4.48 (s, 2H), 3.22 (t, 2H, *J =* 7.1 Hz), 1.91 (t, *2H, J=* 7.1 Hz), 1.65-1.43 (m, 5H), 1.33-1.22 (m, 3H).

### Step 13) the preparation of compound 2-14

To a solution of compound **2-13** (988 mg, 2.02 mmol) and compound **1-9** (911 mg, 4.23 mmol) in DCM (8.0 mL) was added DIPEA (0.732 mL, 4.43 mmol) dropwise at 0 °C. At the end of the addition, the mixture was stirred at rt for 2.0 hrs. After the reaction was completed, the mixture was quenched with water (20 mL). The aqueous layer was extracted with DCM (30 mL x 3). The combined organic layers were washed with brine, dried over anhydrous Na₂SO₄ and concentrated *in vacuo.* The residue was purified by silica gel column chromatography (PE/EtOAc (v/v) = 3/1) to give the title compound as white foam (1.2 g, 79%). The compound was characterized by the following spectroscopic data:
MS (ESI, pos.ion) *m*/*z:* 759.5 [M+H]⁺;
¹H NMR (400 MHz, CDCl₃) *δ* (ppm): 7.93 (t, 2H, *J =* 6.4 Hz), 7.58 (d, 1H, *J =* 7.8 Hz), 7.42-7.35 (m, 2H), 7.00 (d, 1H, *J =* 7.8 Hz), 5.66-5.10 (m, 4H), 4.53-4.38 (m, 2H), 3.65-3.51 (m, 2H), 3.51-3.36 (m, 2H), 3.21-3.14 (m, 2H), 2.40-2.25 (m, 4H), 2.15-2.05 (m, 2H), 2.00-1.85 (m, 4H), 1.60-1.40 (m, 24H), 1.36-1.20 (m, 4H).

### Step 14) the preparation of compound 2-15

A suspension of compound **2-14** (1.20 g, 1.58 mmol) and ammonium acetate (1.80 g, 23.72 mmol) in xylene (10 mL) was stirred at 110 °C for 3.0 hrs. The mixture was cooled to rt, quenched with water (20 mL) and the organic phase was separated. The aqueous layer was extracted with EtOAc (30 mL x 3). The combined organic layers were washed with brine, dried over anhydrous Na₂SO₄ and concentrated *in vacuo.* The residue was purified by silica gel column chromatography (PE/EtOAc (v/v) = 1/2) to give the title compound as yellow foam (790 mg, 69%). The compound was characterized by the following spectroscopic data:
MS (ESI, pos.ion) *m*/*z*: 719.5 [M+H]⁺;
¹H NMR (400 MHz, CDCl₃) *δ* (ppm): 7.77-7.53 (br, 2H), 7.32-7.24 (m, 4H), 7.15 (s, 1H), 7.02-6.97 (m, 1H), 5.05-4.95 (m, 2H), 3.60-3.34 (m, 4H), 3.10-2.90 (m, 4H), 2.25-2.10 (m, 4H), 2.02-1.94 (m, 2H), 1.90 (t, 2H*, J =* 7.0 Hz), 1.80-1.69 (m, 2H), 1.63-1.40 (m, 22H), 1.40-1.30 (m, 2H).

### Step 15) the preparation of compound 2-16

To a solution of compound **2-15** (775 mg, 1.08 mmol) in EtOAc (10.0 mL) was added a solution of HCl in EtOAc (5.0 mL, 4 M) dropwise. At the end of the addition, the mixture was stirred at rt for 8.0 hrs. After the reaction was completed, the mixture was concentrated *in vacuo.* The residue was purified by beating in EtOAc (10 mL) and filtered to give the title compound as a pale yellow solid (664 mg, 93%). The compound was characterized by the following spectroscopic data:
MS (ESI, pos.ion) *m*/*z*: 519.4 [M+H]⁺.

### Step 16) the preparation of compound 2-17

To a suspension of compound **2-16** (650 mg, 0.98 mmol), compound **1-11-2** (360 mg, 2.05 mmol), EDCI (394 mg, 2.05 mmol) and HOAT (200 mg, 1.47 mmol) in DCM (10.0 mL) was added DIPEA (1.30 mL, 7.82 mmol) dropwise at 0 °C. At the end of the addition, the mixture was stirred at rt for 3.0 hrs. After the reaction was completed, the mixture was diluted with DCM (20 mL). The resulting mixture was washed with NH₄Cl aqueous solution and brine, dried over Na₂SO₄ and *concentrated in vacuo.* The residue was purified by silica gel column chromatography (PE/EtOAc (v/v) = 10/1) to give the title compound as white powder (382 mg, 47%). The compound was characterized by the following spectroscopic data:
MS (ESI, pos.ion) *m*/*z*: 833.5 [M+H]⁺;
¹H NMR (400 MHz, CDCl₃) *δ* (ppm): 10.80 (brs, 1H), 10.45 (brs, 1H), 7.80-7.67 (m, 2H), 7.45-7.10 (m, 5H), 6.96 (d, 1H, *J =* 7.7 Hz), 5.68-5.55 (m, 2H), 5.33-5.20 (m, 2H), 4.40-4.30 (m, 2H), 3.90-3.80 (m, 2H), 3.77-3.58 (m, 8H), 3.15-2.88 (m, 4H), 2.46-1.80 (m, 16H), 1.80-1.63 (m, 2H), 1.63-1.40 (m, 4H), 1.40-1.28 (m, 2H), 0.95-0.80 (m, 12H).

### Example 3

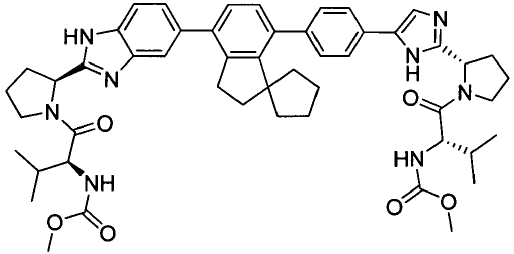

### Synthetic route:

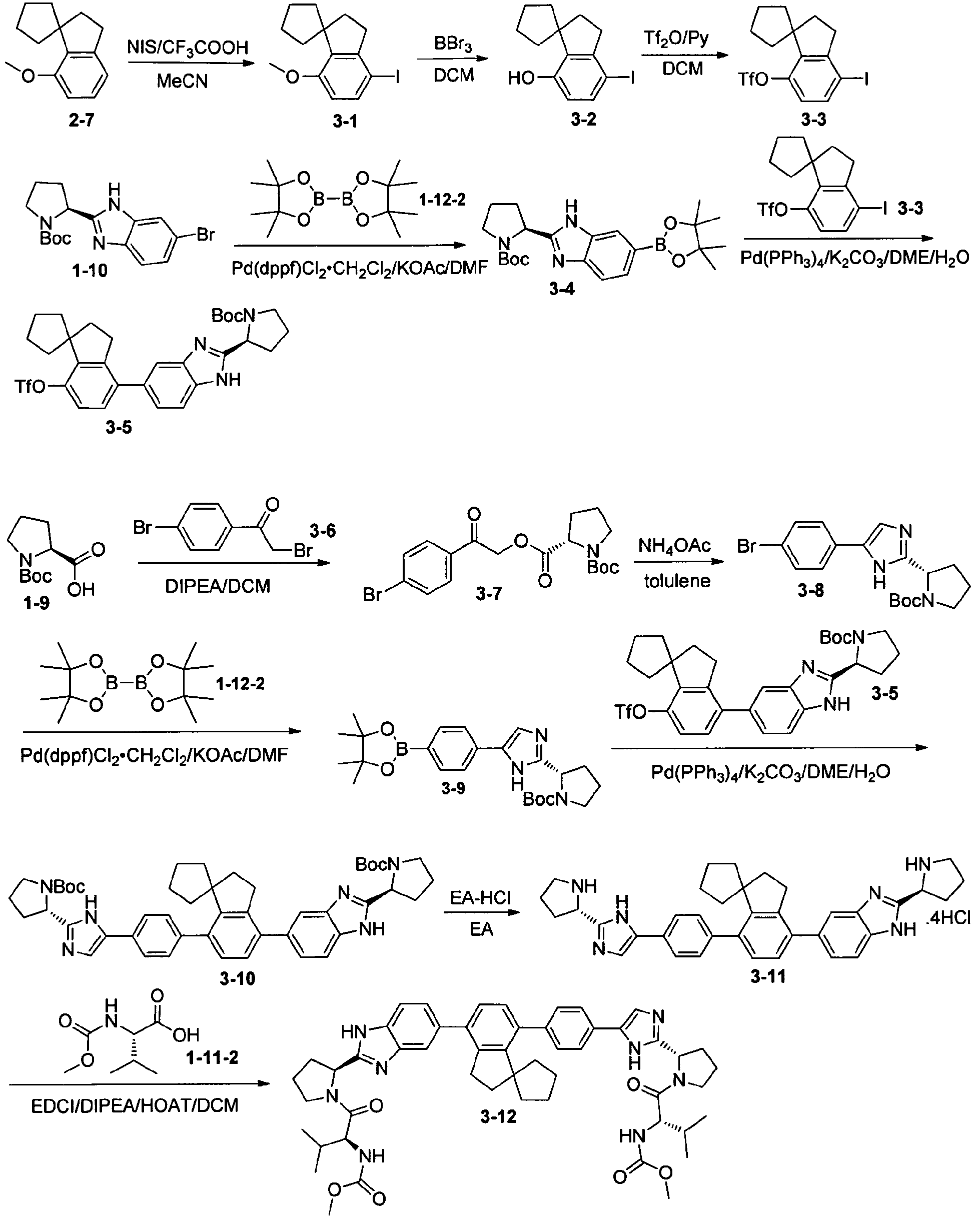

### Step 1) the preparation of compound 3-1

To a mixture of compound **2-7** (5.3 g, 26.2 mmol) and NIS (6.5 g, 28.8 mmol) in MeCN (60 mL) was added CF₃COOH (0.59 mL, 7.9 mmol) dropwise at 0 °C. At the end of the addition, the mixture was stirred at 45 °C overnight. After the reaction was completed, the mixture was concentrated *in vacuo.* To the residue was added water (50 mL). The resulting mixture was extracted with EtOAc (60 mL x 3). The combined organic layers were washed with saturated sodium sulfite aqueous solution (30 mL x 2), dried over anhydrous Na₂SO₄ and concentrated *in vacuo.* The residue was purified by silica gel column chromatography (PE) to give the title compound as colorless liquid (8.6 g, 100%). The compound was characterized by the following spectroscopic data:
MS (ESI, pos.ion) *m*/*z:* 329.1 [M+H]⁺;
¹H NMR (400 MHz, CDCl₃) *δ* (ppm): 7.47 (d, 1H, *J =* 8.5 Hz), 6.48 (d, 1H, *J=* 8.5 Hz), 3.78 (s, 3H), 2.83 (t, 2H, *J*=7.4 Hz), 2.20-2.10 (m, 2H), 1.93 (t, 2H, *J=* 7.4Hz), 1.86-1.75 (m, 2H).

### Step 2) the preparation of compound 3-2

To a solution of compound **3-1** (1.55 g, 4.72 mmol) in DCM (10 mL) was added boron tribromide (1.79 mL, 18.89 mmol) dropwise at -78 °C. At the end of the addition, the mixture was stirred at -78 °C for 10 mins and at rt for another 1.0 hr. After the reaction was completed, the mixture was quenched with ice water (25 mL) and the organic phase was separated. The aqueous layer was extracted with DCM (30 mL x 3). The combined organic layers were washed with brine, dried over anhydrous Na₂SO₄ and concentrated *in vacuo.* The residue was purified by silica gel column chromatography (PE/EtOAc (v/v) = 40/1) to give the title compound as colorless oil (1.36 g, 92%). The compound was characterized by the following spectroscopic data:
¹H NMR (400 MHz, CDCl₃) *δ* (ppm): 7.36 (d, 1H, *J =* 8.3 Hz), 6.34 (d, 1H, *J =* 8.3 Hz), 4.72 (s, 1H), 2.83 (t, 2H, *J =* 7.4 Hz), 2.21-2.08 (m, 2H), 1.95 (t, 2H, *J =* 7.4 Hz), 1.86-1.79 (m, 2H), 1.74-1.60 (m, 4H).

### Step 3) the preparation of compound 3-3

To a solution of compound **3-2** (1.33 g, 4.24 mmol) in DCM (10 mL) was added pyridine (0.85 mL, 10.58 mmol) dropwise at 0 °C. After the mixture was stirred for 10 mins, trifluoromethanesulfonic anhydride (1.07 mL, 6.36 mmol) was added dropwise. At the end of the addition, the mixture was stirred at rt for 1.0 hr. After the reaction was completed, the mixture was quenched with ice water (25 mL). The aqueous layer was extracted with DCM (30 mL x 3). The combined organic layers were washed with brine, dried over anhydrous Na₂SO₄ and concentrated *in vacuo.* The residue was purified by silica gel column chromatography (PE) to give the title compound as colorless oil (1.8 g, 95%). The compound was characterized by the following spectroscopic data:
¹H NMR (400 MHz, CDCl₃) *δ* (ppm): 7.58 (d, 1H, *J=* 8.6 Hz), 6.88 (d, 1H, *J=* 8.7 Hz), 2.88 (t, 2H, *J =* 7.4 Hz), 2.13-2.03 (m, 2H), 1.99 (t, *2H, J=* 7.4 Hz), 1.94-1.82 (m, 2H), 1.78-1.65 (m, 4H).

### Step 4) the preparation of compound 3-4

To a mixture of compound **1-10** (4.114 g, 11.27 mmol), compound **1-12-2** (4.29 g, 16.9 mmol), Pd(dppf)Cl₂·CH₂Cl₂ (653 mg, 0.8 mmol) and KOAc (2.09 g, 21.3 mmol) was added DMF (30 mL) via syringe, and the mixture was stirred at 90 °C under N₂ for 3.0 hrs. After cooling to rt, the mixture was diluted with EtOAc (200 mL) and filtered through a celite pad. The filtrate was washed with water (60 mL x 3) and brine, dried over anhydrous Na₂SO₄ and concentrated *in vacuo.* The residue was purified by silica gel column chromatography (PE/EtOAc (v/v) = 1/2) to give the title compound as a beige solid (2.2 g, 65%). The compound was characterized by the following spectroscopic data:
MS (ESI, pos.ion) *m*/*z:* 414.3 [M+H]⁺;
¹H NMR (400 MHz, CDCl₃) *δ* (ppm): 7.69 (s, 1H), 7.45-7.43 (m, 1H), 7.32-7.30 (m, 1H), 5.12-5.10 (m, 1H), 3.45-3.43 (m, 2H), 2.95-2.94 (m, 1H), 2.25-2.22 (m, 2H), 2.01-1.91 (m, 1H), 1.49 (s, 9H), 1.35 (s, 12H).

### Step 5) the preparation of compound 3-5

To a mixture of compound **3-3** (446 mg, 1.0 mmol), compound **3-4** (413 mg, 1.0 mmol), Pd(PPh₃)₄ (116 mg, 0.1 mmol) and K₂CO₃ (346 mg, 2.5 mmol) were added DME (5.0 mL) and pure water (1.0 mL) via syringe, and the mixture was stirred at 90 °C under N₂ for 3.0 hrs. After the reaction was completed, the mixture was cooled to rt and diluted with EtOAc (30 mL). The resulting mixture was washed with brine, dried over anhydrous Na₂SO₄ and concentrated *in vacuo.* The residue was purified by silica gel column chromatography (PE/EtOAc (v/v) = 2/1) to give the title compound as a pale yellow solid (450 mg, 74%). The compound was characterized by the following spectroscopic data:
MS (ESI, pos.ion) *m*/*z*: 606.5 [M+H]⁺;
¹H NMR (400 MHz, CDCl₃) *δ* (ppm): 7.61-7.51 (m, 2H), 7.25-7.16 (m, 3H), 5.15-5.13 (br, 1H), 3.44 (br, 2H), 2.93-2.89 (t, 2H, *J=* 8.0 Hz), 2.21-2.14 (m, 4H), 2.03-2.02 (m, 2H), 1.95-1.88 (m, 4H), 1.86-1.67 (m, 4H), 1.49 (s, 9H).

### Step 6) the preparation of compound 3-7

To a solution of compound **3-6** (30 g, 107.9 mmol) and compound **1-9** (25.6 g, 118.7 mmol) in DCM (250 mL) was added DIPEA (21.4 mL, 129.5 mmol) dropwise at 0 °C. At the end of the addition, the mixture was stirred at rt for 3.0 hrs. After the reaction was completed, the mixture was quenched with ice water (100 mL). The aqueous layer was extracted with EtOAc (100 mL x 3). The combined organic layers were washed with brine, dried over anhydrous Na₂SO₄ and concentrated *in vacuo.* The residue was purified by silica gel column chromatography (PE/EtOAc (v/v) = 10/1) to give the title compound as a white solid (40 g, 91%). The compound was characterized by the following spectroscopic data:
MS (ESI, pos.ion) *m*/*z*: 412.7 [M+H]⁺;
¹H NMR (400 MHz, CDCl₃) *δ* (ppm): 7.78-7.75 (m, 2H), 7.65-7.63 (m, 2H), 5.53-5.15 (m, 2H), 4.49-4.39 (m, 1H), 3.59-3.54 (m, 1H), 3.48-3.38 (m, 1H), 2.31-2.21 (m, 2H), 2.12-2.01 (m, 1H), 1.98-1.85 (m, 1H), 1.45 (d, 9H).

### Step 7) the preparation of compound 3-8

A suspension of compound **3-7** (15 g, 36.4 mmol) and ammonium acetate (42 g, 54.6 mmol) in toluene (150 mL) was stirred at 110 °C for 5.0 hrs. After cooling to rt, the reaction was quenched with water (100 mL). The resulting mixture was extracted with EtOAc (100 mL x 3). The combined organic layers were washed with brine, dried over anhydrous Na₂SO₄ and concentrated *in vacuo.* The residue was purified by silica gel column chromatography (PE/EtOAc (v/v) = 5/1) to give the title compound (12.12 g, 85%). The compound was characterized by the following spectroscopic data:
MS (ESI, pos.ion) *m*/*z*: 392.2 [M+H]⁺;
¹H NMR (400 MHz, CDCl₃) *δ* (ppm): 7.78-7.75 (m, 2H), 7.65-7.63 (m, 2H), 7.21-7.20 (m, 1H), 5.53-5.15 (m, 2H), 4.49-4.39 (m, 1H), 3.59-3.54 (m, 1H), 3.48-3.38 (m, 1H), 2.31-2.21 (m, 2H), 2.12-2.01 (m, 1H), 1.98-1.85 (m, 1H), 1.45 (d, 9H).

### Step 8) the preparation of compound 3-9

A mixture of compound **3-8** (4.0 g, 10.23 mmol), compound **1-12-2** (2.86 g, 11.25 mmol), Pd(dppf)Cl₂·CH₂Cl₂ (418 mg, 0.51 mmol) and KOAc (2.51 g, 25.57 mmol) in DMF (40 mL) was stirred at 90 °C under N₂ for 3.0 hrs. After the reaction was completed, the mixture was cooled to rt, diluted with EtOAc (250 mL) and filtered through a celite pad. The filtrate was washed with water (80 mL x 3) and brine, dried over anhydrous Na₂SO₄ and concentrated *in vacuo.* The residue was purified by silica gel column chromatography (PE/EtOAc (v/v) = 2/1) to give the title compound (3.6 g, 80%). The compound was characterized by the following spectroscopic data:
¹H NMR (400 MHz, CDCl₃) *δ* (ppm): 7.35 (m, 4H), 7.10 (s, 1H), 4.93 (t, 1H, *J* = 8.2 Hz), 3.88-3.66 (m, 2H), 2.90 (t, 1 H, *J =* 8.0 Hz), 2.50-2.47 (m, 2H), 2.27-2.25 (m, 1H), 1.48 (s, 9H), 1.26 (s, 12H).

### Step 9) the preparation of compound 3-10

To a mixture of compound **3-5** (920.6 mg, 1.52 mmol), compound **3-9** (667.8 mg, 1.52 mmol), Pd(PPh₃)₄ (176 mg, 0.15 mmol) and K₂CO₃ (527 mg, 3.81 mmol) were added DME (30.0 mL) and H₂O (5.0 mL) via syringe, and the mixture was stirred at 90 °C under N₂ for 3.0 hrs. After the reaction was completed, the mixture was cooled to rt and quenched with water (50 mL). The aqueous layer was extracted with EtOAc (60 mL x 3). The combined organic layers were washed with brine, dried over anhydrous Na₂SO₄ and concentrated *in vacuo.* The residue was purified by silica gel column chromatography (DCM/MeOH (v/v) = 50/1) to give the title compound as pale yellow foam (335 mg, 30%). The compound was characterized by the following spectroscopic data:
MS (ESI, pos.ion) *m*/*z*: 769.5 [M+H]⁺;
¹H NMR (400 MHz, CDCl₃) *δ* (ppm): 7.73-7.68 (m, 3H), 7.40-7.35 (m, 3H), 7.29-7.27 (m, 3H), 7.07-7.05 (d, 3H, *J =* 8.0 Hz), 5.19-5.17 (br, 1H), 5.03-5.02 (br, 1H), 3.46 (br, 4H), 2.97-2.94 (m, 4H), 2.24-2.20 (m, 4H), 2.06-1.99 (m, 2H),1.88-1.79 (m, 4H), 1.64-1.61 (m, 2H), 1.53-1.51 (m, 18H), 1.41-1.36 (m, 4H).

### Step 10) the preparation of compound 3-11

To a solution of compound **3-10** (355 mg, 0.46 mmol) in EtOAc (5.0 mL) was added a solution of HCl in EtOAc (3.0 mL, 4 M) dropwise. At the end of the addition, the mixture was stirred at rt for 8.0 hrs. After the reaction was completed, the mixture was concentrated *in vacuo.* The residue was purified by beating in EtOAc (10 mL) and filtered to give the title compound as a pale yellow solid (208 mg, 63%). The compound was characterized by the following spectroscopic data:
MS (ESI, pos.ion) *m*/*z:* 569.3 [M+H]⁺.

### Step 11) the preparation of compound 3-12

To a mixture of compound **3-11** (208 mg, 0.29 mmol), compound **1-11-2** (107 mg, 0.61 mmol), EDCI (117 mg, 0.61 mmol) and HOAT (79 mg, 0.58 mmol) in DCM (20.0 mL) was added DIPEA (0.406 mL, 2.33 mmol) dropwise at 0 °C. At the end of the addition, the mixture was stirred at rt for 3.0 hrs. After the reaction was completed, the mixture was quenched with saturated NH₄Cl aqueous solution (20 mL). The aqueous layer was extracted with DCM (20 mL x 3). The combined organic layers were washed with brine, dried over anhydrous Na₂SO₄ and concentrated *in vacuo.* The residue was purified by silica gel column chromatography (DCM/MeOH (v/v) = 60/1) to give the title compound as a white solid (157 mg, 61%). The compound was characterized by the following spectroscopic data:
MS (ESI, pos.ion) *m*/*z*: 883.5 [M+H]⁺;
¹ H NMR (400 MHz, CDCl₃) *δ* (ppm): 7.77 (m, 1H), 7.73-7.69 (m, 1H), 7.54-7.52 (m, 1H), 7.37-7.26 (m, 4H), 7.26-7.16 (m, 2H), 6.98-6.96 (d, 1H, *J =* 8.0 Hz), 5.73-5.69 (m, 2H), 5.45-5.42 (br, 1H), 5.29-5.26 (br, 1H), 3.92-3.81 (m, 2H), 3.68 (s, 6H), 3.67-3.65 (m, 2H), 3.11-2.99 (m, 2H), 2.89-2.88 (m, 2H), 2.49-2.32 (m, 2H), 2.32-2.12 (m, 4H), 2.12-1.98 (m, 2H), 1.85-1.69 (m, 4H), 1.63-1.52 (br, 2H), 1.51-1.42 (m, 2H), 1.41-1.32 (m, 2H), 0.87-0.83 (m, 12H).

### Example 4

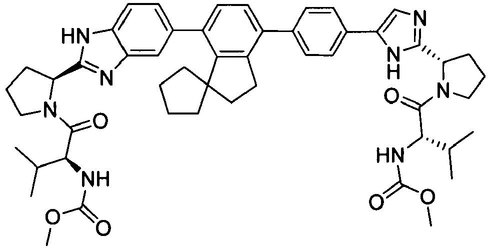

### Synthetic route:

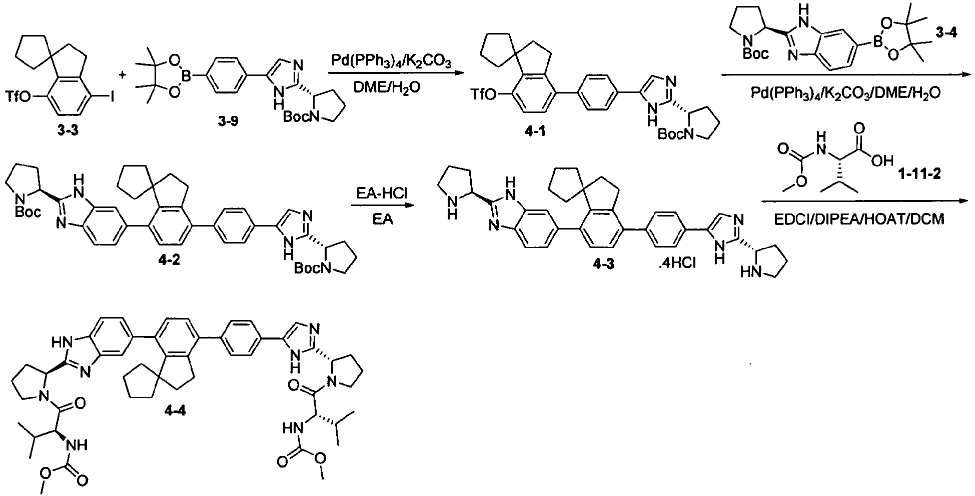

### Step 1) the preparation of compound 4-1

To a mixture of compound **3-3** (4.10 g, 9.19 mmol), compound **3-9** (4.04 g, 9.19 mmol), Pd(PPh₃)₄ (849 mg, 0.08 mmol) and K₂CO₃ (3.17 g, 22.97 mmol) were added DME (20.0 mL) and H₂O (4.0 mL) via syringe, and the mixture was stirred at 90 °C under N₂ for 3.0 hrs. After the reaction was completed, the mixture was cooled to rt and quenched with water (50 mL). The resulting mixture was extracted with EtOAc (60 mL x 3). The combined organic layers were washed with brine, dried over anhydrous Na₂SO₄ and concentrated *in vacuo.* The residue was purified by silica gel column chromatography (PE/EtOAc (v/v) = 2/1) to give the title compound as a pale yellow solid (1.46 g, 50%). The compound was characterized by the following spectroscopic data:
MS (ESI, pos.ion) *m*/*z*: 632.3 [M+H]⁺;
¹H NMR (400 MHz, CDCl₃) *δ* (ppm): 7.79-7.61 (m, 3H), 7.40-7.38 (d, 2H, *J =* 8.0 Hz), 7.23-7.18 (m, 2H), 5.01-4.99 (br, 1H), 3.44 (br, 2H), 2.95-2.92 (t, 2H, *J =* 8.0 Hz), 2.22-2.15 (m, 6H), 1.99-1.91 (m, 6H), 1.77-1.73 (m, 3H), 1.51 (s, 9H).

### Step 2) the preparation of compound 4-2

To a mixture of compound **4-1** (1.47 g, 2.33 mmol), compound **3-4** (962.9 mg, 2.33 mmol), Pd(PPh₃)₄ (269 mg, 0.23 mmol) and K₂CO₃ (804 mg, 5.82 mmol) were added DME (10.0 mL) and H₂O (2.0 mL) via syringe, and the mixture was stirred at 90 °C under N₂ for 3.0 hrs. After the reaction was completed, the mixture was cooled to rt and quenched with water (50 mL). The aqueous layer was extracted with EtOAc (60 mL x 3). The combined organic layers were washed with brine, dried over anhydrous Na₂SO₄ and concentrated *in vacuo.* The residue was purified by silica gel column chromatography (DCM/MeOH (v/v) = 50/1) to give the title compound as pale yellow foam (887 mg, 50%). The compound was characterized by the following spectroscopic data:
MS (ESI, neg.ion) *m*/*z*: 767.5 [M-H]⁻;
¹H NMR (400 MHz, CDCl₃) *δ* (ppm): 7.71-7.70 (m, 3H), 7.53-7.51 (m, 3H), 7.28-7.27 (m, 1H), 7.22-7.20 (t, 2H, *J =* 8.0 Hz), 7.07-7.05 (m, 1H), 5.17-5.16 (br, 1H), 5.02-5.01 (br, 1H), 3.46 (br, 4H), 3.11-3.01 (br, 2H), 2.97-2.93 (t, 4H, *J =* 8.0 Hz), 2.23-2.21 (m, 4H), 2.06-1.99 (m, 2H), 1.87-1.84 (m, 2H), 1.61-1.60 (m, 2H), 1.57-1.55 (m, 2H), 1.53-1.51 (m, 18H), 1.41-1.36 (m, 4H).

### Step 3) the preparation of compound 4-3

To a solution of compound **4-2** (883.7 mg, 1.15 mmol) in EtOAc (5.0 mL) was added a solution of HCl in EtOAc (5.0 mL, 4 M) dropwise. At the end of the addition, the mixture was stirred at rt for 8.0 hrs. After the reaction was completed, the mixture was concentrated *in vacuo.* The residue was purified by beating in EtOAc (10.0 mL) and filtered to give the title compound as pale yellow powder (559 mg, 68%). The compound was characterized by the following spectroscopic data:
MS (ESI, pos.ion) *m*/*z:* 569.3 [M+H]⁺.

### Step 4) the preparation of compound 4-4

To a mixture of compound **4-3** (559 mg, 0.78 mmol), compound **1-11-2** (288 mg, 1.64 mmol), EDCI (315 mg, 1.64 mmol) and HOAT (213 mg, 1.56 mmol) in DCM (20.0 mL) at 0 °C was added DIPEA (1.09 mL, 6.26 mmol) dropwise. At the end of the addition, the mixture was stirred at rt for 3.0 hrs. After the reaction was completed, the mixture was diluted with DCM (20 mL). The organic layer was washed with NH₄Cl aqueous solution and brine, dried over anhydrous Na₂SO₄ and concentrated *in vacuo.* The residue was purified by silica gel column chromatography (DCM/MeOH (v/v) = 60/1) to give the title compound as a white solid (340 mg, 49%). The compound was characterized by the following spectroscopic data:
MS (ESI, pos.ion) *m*/*z*: 883.5 [M+H]⁺;
¹H NMR (400 MHz, CDCl₃) *δ* (ppm): 7.80-7.68 (m, 2H), 7.45-7.43 (m, 3H), 7.19-7.17 (m, 1H), 7.16-7.15 (m, 3H), 7.00-6.98 (d, 1H*, J =* 8.0 Hz), 5.86-5.82 (m, 2H), 5.45-5.43 (br, 1H), 5.28-5.27 (br, 1H), 3.95-3.82 (m, 2H), 3.68 (s, 6H), 3.67-3.65 (m, 2H), 3.11-2.99 (m, 2H), 2.91-2.88 (m, 2H), 2.49-2.32 (m, 2H), 2.31-2.12 (m, 4H), 2.11-1.98 (m, 2H), 1.85-1.69 (m, 4H), 1.63-1.52 (br, 2H), 1.51-1.42 (m, 2H), 1.41-1.32 (m, 2H), 0.88-0.84 (m, 12H).

### Example 5

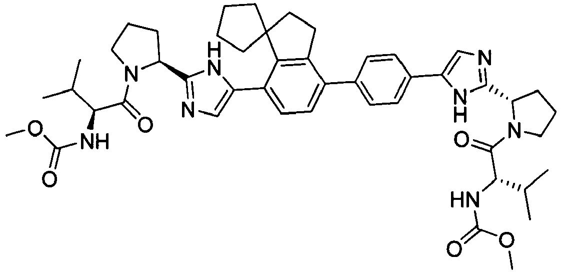

### Synthetic route:

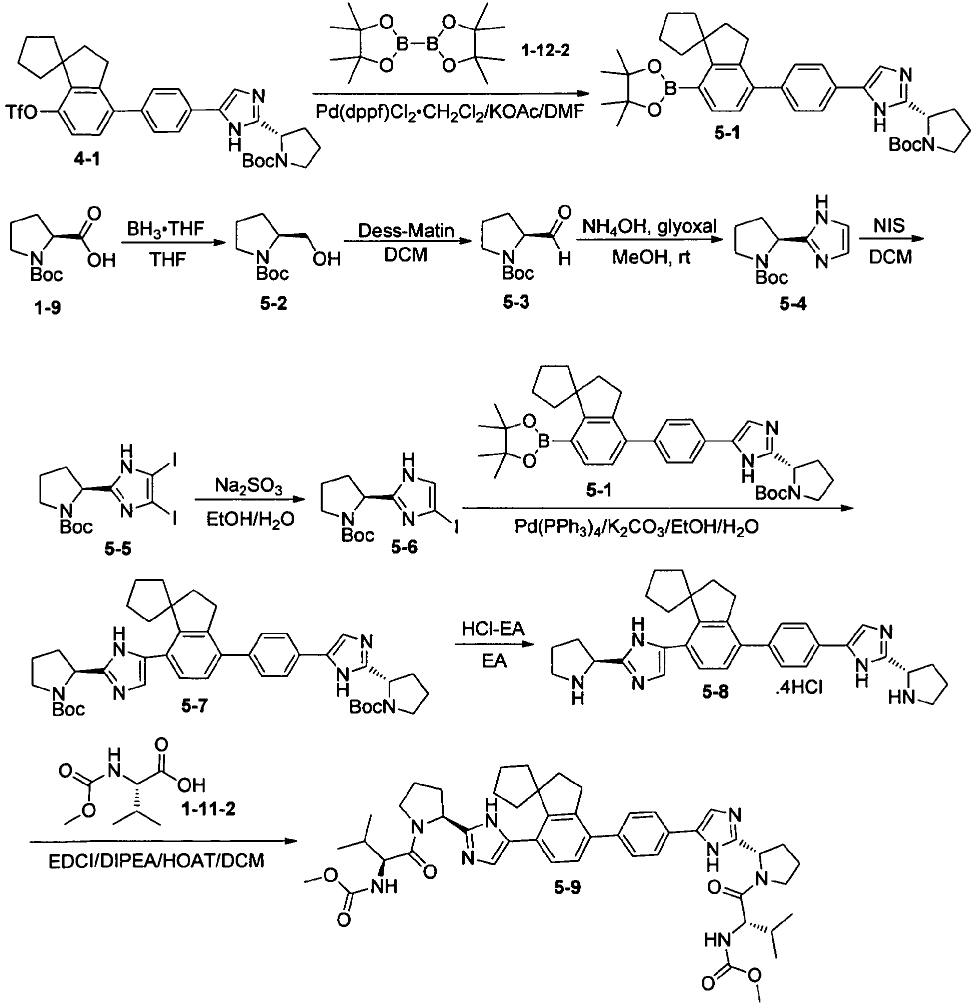

### Step 1) the preparation of compound 5-1

A mixture of compound **4-1** (801.7 mg, 1.27 mmol), compound **1-12-2** (353 mg, 1.39 mmol), Pd(dppf)Cl₂·CH₂Cl₂ (103 mg, 0.13 mmol) and KOAc (311 mg, 3.17 mmol) in DMF (5.0 mL) was stirred at 90 °C under N₂ for 3.0 hrs. After the reaction was completed, the mixture was cooled to rt, diluted with EtOAc (50 mL) and filtered through a celite pad. The filtrate was washed with water (30 mL x 3) and brine, dried over anhydrous Na₂SO₄ and concentrated *in vacuo.* The residue was purified by silica gel column chromatography (PE/EtOAc (v/v) = 2/1) to give the title compound as a pale yellow solid (603.6 mg, 78%). The compound was characterized by the following spectroscopic data:
MS (ESI, pos.ion) *m*/*z*: 610.3 [M+H]⁺;
¹H NMR (400 MHz, CDCl₃) *δ* (ppm): 7.65-7.63 (m, 2H), 7.40-7.38 (d, 2H, *J =* 8.0 Hz), 7.20-7.15 (m, 2H), 4.98-4.96 (br, 1H), 3.42 (br, 2H), 2.87-2.84 (m, 2H), 2.22-2.15 (m, 6H), 1.99-1.91 (m, 6H),1.77-1.73 (m, 3H), 1.50 (s, 9H), 1.32 (s, 12H).

### Step 2) the preparation of compound 5-2

To a solution of compound **1-9** (10.0 g, 46.6 mmol) in THF (100 mL) was added diborane (100 mL, 1M in THF) dropwise at 0 °C. At the end of the addition, the mixture was stirred at 0 °C for 3.0 hrs. After the reaction was completed, the mixture was quenched with MeOH (80 mL) and concentrated *in vacuo.* The residue was purified by silica gel column chromatography (PE/EtOAc (v/v) = 3/2) to give the title compound as colorless oil (7.0 g, 75.2%). The compound was characterized by the following spectroscopic data:
¹H NMR (400 MHz, CDCl₃) *δ* (ppm): 3.99-3.87 (br, 1H), 3.68-3.51 (m, 2H), 3.48-3.39 (m, 1H), 3.34-3.25 (m, 1H), 2.05-1.92 (m, 2H), 1.88-1.71 (m, 2H), 1.45 (s, 9H).

### Step 3) the preparation of compound 5-3

To a solution of compound **5-2** (7.0 g, 34.8 mmol) in DCM (250 mL) was added Dess-Martin periodinane (20.7 g, 48.8 mmol) in a portionwise manner at 0 °C. At the end of the addition, the mixture was stirred at rt for 2.0 hrs. After the reaction was completed, the mixture was diluted with water (250 mL), and the resulting mixture was filtered. After the layers were partitioned, the organic layer was washed with brine, dried over anhydrous Na₂SO₄ and concentrated *in vacuo.* The residue was purified by silica gel column chromatography (PE/EtOAc (v/v) = 3/2) to give the title compound as colorless oil (3.5 g, 50.7%). The compound was characterized by the following spectroscopic data:
¹H NMR (400 MHz, CDCl₃) *δ* (ppm): 9.46 (d, 1H, *J =* 2.8 Hz), 4.08-4.03 (m, 1H), 3.51-3.42 (m, 2H), 2.01-1.93 (m, 2H), 1.91-1.84 (m, 2H), 1.43 (s, 9H).

### Step 4) the preparation of compound 5-4

To a solution of compound **5-3** (3.5 g, 17.6 mmol) and ammonia (13 mL) in MeOH (30 mL) was added glyoxal (8 mL, 40% in H₂O) dropwise at 0 °C. At the end of the addition, the mixture was stirred at rt overnight. After the reaction was completed, the mixture was concentrated *in vacuo.* The residue was purified by silica gel column chromatography (PE/EtOAc (v/v) = 3/2) to give the title compound as a white solid (2.0 g, 47.6%). The compound was characterized by the following spectroscopic data:
MS (ESI, pos.ion) *m*/*z*: 238.2 [M+H]⁺;
¹H NMR (400 MHz, CDCl₃) *δ* (ppm): 6.96 (s, 1H), 4.94 (dd, 1H, *J =* 7.68 Hz, 2.40 Hz), 3.38 (t, 2H, *J=* 6.24 Hz), 2.17-2.03 (m, 2H), 1.99-1.91 (m, 2H), 1.48 (s, 9H).

### Step 5) the preparation of compound 5-5

To a solution of compound **5-4** (2.0 g, 8.4 mmol) in DCM (60 mL) was added *N-*iodosuccinimide (3.8 g, 16.8 mmol) at 0 °C in a portionwise manner. At the end of the addition, the mixture was stirred at 0 °C for 1.5 hrs. After the reaction was completed, the mixture was washed with brine, dried over anhydrous Na₂SO₄ and concentrated *in vacuo.* The residue was purified by silica gel column chromatography (PE/EtOAc (v/v) = 3/2) to give the title compound as a white solid (2.6 g, 63.1%). The compound was characterized by the following spectroscopic data:
MS (ESI, pos.ion) *m*/*z*: 490.0 [M+H]⁺;
¹H NMR (400 MHz, CDCl₃) *δ* (ppm): 4.89 (dd, 1H, *J=* 7.64 Hz, 2.52 Hz), 3.36 (t, 2H), 2.14-2.02 (m, 2H), 1.97-1.85 (m, 2H), 1.49 (s, 9H).

### Step 6) the preparation of compound 5-6

To a suspension of compound **5-5** (1.6 g, 3.27 mmol) in mixed solvents of ethanol and water (50 mL, v/v = 3/7) was added Na₂SO₃ (3.7 g, 29 mmol), and the mixture was refluxed for 17 hrs. After the reaction was completed, the ethanol solvent was removed *in vacuo,* and to the residue was added water (50 mL). The resulting mixture was extracted with EtOAc (50 mL x 3). The combined organic layers were washed with brine, dried over anhydrous Na₂SO₄ and concentrated *in vacuo.* The residue was purified by silica gel column chromatography (PE/EtOAc (v/v) = 3/2) to give the title compound as a white solid (1.0 g, 84%). The compound was characterized by the following spectroscopic data:
MS (ESI, pos.ion) *m*/*z*: 364.1 [M+H]⁺;
¹H NMR (400 MHz, CDCl₃) *δ* (ppm): 7.04 (d, 1H, *J=* 1.84 Hz), 4.89 (dd, 1H, *J =* 7.72 Hz, 2.56 Hz), 3.36 (t, 2H), 2.18-2.03 (m, 2H), 1.97-1.82 (m, 2H), 1.47 (s, 9H).

### Step 7) the preparation of compound 5-7

To a mixture of compound **5-1** (609 mg, 1.0 mmol), compound **5-6** (370 mg, 1.02 mmol), Pd(PPh₃)₄ (116 mg, 0.1 mmol) and K₂CO₃ (804 mg, 5.82 mmol) were added EtOH (10.0 mL) and H₂O (2.0 mL) via syringe, and the mixture was stirred at 90 °C under N₂ for 3.0 hrs. After the reaction was completed, the mixture was cooled to rt, and diluted with water (50 mL). The aqueous layer was extracted with EtOAc (60 mL x 3). The combined organic layers were washed with brine, dried over anhydrous Na₂SO₄ and concentrated *in vacuo.* The residue was purified by silica gel column chromatography (DCM/MeOH (v/v) = 100/1) to give the title compound as pale yellow foam (251 mg, 35%). The compound was characterized by the following spectroscopic data:
MS (ESI, pos.ion) *m*/*z*: 719.3 [M+H]⁺;
¹H NMR (400 MHz, CDCl₃) *δ* (ppm): 7.66-7.61 (m, 2H), 7.54-7.49 (m, 1H), 7.45-7.41 (m, 2H), 7.21-7.15 (m, 2H), 6.89 (s, 1H), 4.98-4.96 (br, 2H), 3.40 (br, 4H), 2.93-2.87 (br, 4H), 2.14-2.12 (m, 4H), 1.93 (br, 3H), 1.86-1.82 (br, 3H), 1.56-1.52 (m, 4H), 1.48 (m, 18H), 1.21-1.25 (m, 2H).

### Step 8) the preparation of compound 5-8

To a solution of compound **5-7** (251 mg, 0.35 mmol) in EtOAc (5.0 mL) was added a solution of HCl in EtOAc (3.0 mL, 4 M) dropwise. At the end of the addition, the mixture was stirred at rt for 8.0 hrs. After the reaction was completed, the mixture was concentrated *in vacuo.* The residue was washed with EtOAc (10 mL) and filtered to give the title compound as pale yellow powder (168 mg, 72%). The compound was characterized by the following spectroscopic data:
MS (ESI, pos.ion) *m*/*z:* 519.3 [M+H]⁺.

### Step 9) the preparation of compound 5-9

To a mixture of compound **5-8** (168 mg, 0.25 mmol), compound **1-11-2** (93 mg, 0.53 mmol), EDCI (102 mg, 0.53 mmol) and HOAT (69 mg, 0.51 mmol) in DCM (20 mL) at 0 °C was added DIPEA (0.352 mL, 2.02 mmol) dropwise. At the end of the addition, the mixture was stirred at rt for 3.0 hrs. After the reaction was completed, the mixture was diluted with DCM (20 mL). The organic layer was washed with saturated NH₄Cl aqueous solution and brine, dried over anhydrous Na₂SO₄ and concentrated *in vacuo.* The residue was purified by silica gel column chromatography (DCM/MeOH (v/v) = 60/1) to give the title compound as a white solid (130 mg, 62%). The compound was characterized by the following spectroscopic data:
MS (ESI, pos.ion) *m*/*z*: 834.5 [M+H]⁺;
¹H NMR (400 MHz, CDCl₃) *δ* (ppm): 7.64-7.54 (m, 1H), 7.40-7.38 (m, 2H), 7.20-7.09 (m, 3H), 6.91-6.88 (m, 2H), 5.84-5.78 (m, 2H), 5.25-5.18 (m, 2H), 4.31-4.27 (m, 2H), 3.83-3.79 (m, 2H), 3.68 (s, 6H), 2.95-2.86 (m, 4H), 2.37-2.30 (m, 2H), 2.16-2.07 (m, 4H), 1.94-1.80 (m, 4H), 1.56-1.50 (br, 6H), 1.05-1.02 (m, 2H), 0.87-0.83 (m, 12H).

### Example 6

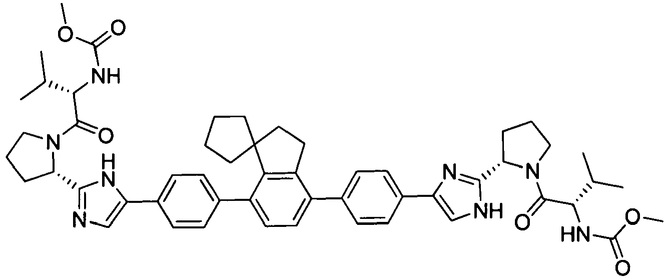

### Synthetic route:

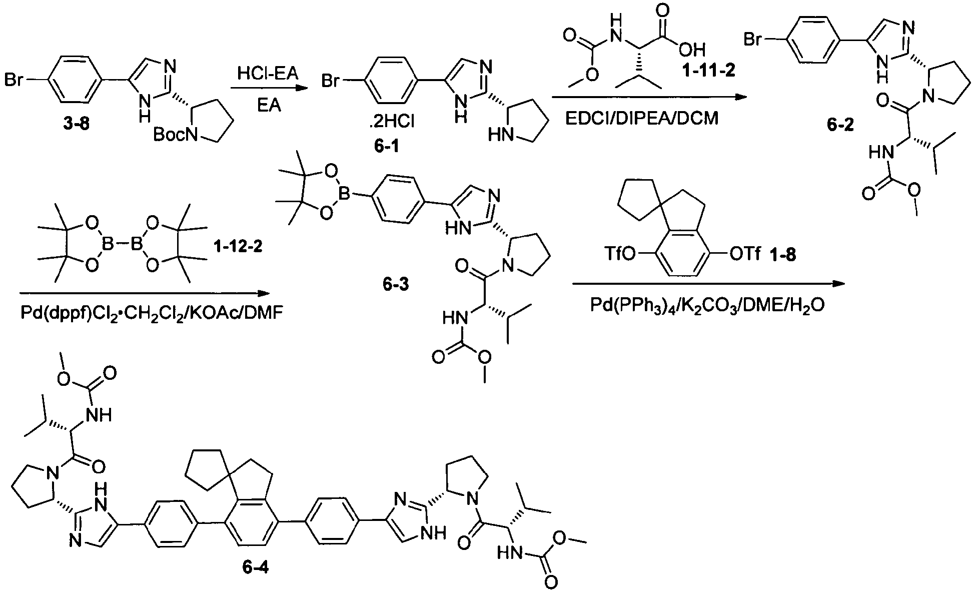

### Step 1) the preparation of compound 6-1

To a solution of compound **3-8** (10.0 g, 25.5 mmol) in EtOAc (50.0 mL) was added a solution of HCl in EtOAc (50.0 mL, 4 M) dropwise. At the end of the addition, the mixture was stirred at rt for 8.0 hrs. After the reaction was completed, the mixture was concentrated *in vacuo.* The residue was purified by beating in EtOAc (40 mL) and filtered to give the title compound as a pale yellow solid (8.0 g, 86.2%). The compound was characterized by the following spectroscopic data:
MS (ESI, pos.ion) *m*/*z*: 292.3 [M+H]⁺;
¹H NMR (400 MHz, CDCl₃) *δ* (ppm): 7.76-7.73 (m, 2H), 7.66-7.63 (m, 2H), 7.21-7.20 (m, 1H), 5.50-5.22 (m, 2H), 4.49-4.39 (m, 1H), 3.61-3.56 (m, 1H), 3.49-3.39 (m, 1H), 2.31-2.21 (m, 2H), 2.12-2.01 (m, 1 H), 1.98-1.85 (m, 1H).

### Step 2) the preparation of compound 6-2

To a solution of compound 6-1 (7.03 g, 19.26 mmol), compound **1-11-2** (5.06 g, 28.88 mmol) and EDCI (5.56 g, 28.88 mmol) in DCM (100 mL) was added DIPEA (21 mL, 127 mmol) dropwise at 0 °C. At the end of the addition, the mixture was stirred at rt for 2.0 hrs. After the reaction was completed, the mixture was diluted with water (100 mL) and extracted with CH₂Cl₂ (150 mL x 3). The combined organic layers were washed with brine, dried over anhydrous Na₂SO₄ and concentrated *in vacuo.* The residue was purified by silica gel column chromatography (PE/EtOAc (v/v) = 1/2) to give the title compound as a solid (7.6 g, 88%). The compound was characterized by the following spectroscopic data:
¹H NMR (400 MHz, CDCl₃) *δ* (ppm): 7.65-7.60 (m, 2H), 7.47-7.43 (m, 2H), 7.22-7.20 (m, 1H), 5.67-5.65 (m, 1H), 5.24-5.22 (m, 1H), 4.34-4.30 (m, 1H), 3.85-3.81 (m, 1H), 3.72 (s, 3H), 3.71-3.64 (m, 1H), 3.00 (s, 1H), 2.34-2.31 (m, 1H), 2.21-1.95 (m, 5H), 1.04-1.02 (m, 1H), 0.88-0.86 (d, 6H).

### Step 3) the preparation of compound 6-3

To a mixture of compound **6-2** (5.0 g, 11.13 mmol), compound **1-12-2** (4.3 g, 16.7 mmol), Pd(dppf)Cl₂·CH₂Cl₂ (0.91 g, 1.11 mmol) and KOAc (3.3 g, 33.4 mmol) was added DMF (50 mL) via syringe under N₂, and the mixture was stirred at 90 °C for 3.0 hrs. After the reaction was completed, the mixture was cooled to rt and diluted with water (80 mL). The aqueous layer was extracted with EtOAc (40 mL x 3). The combined organic layers were washed with brine, dried over anhydrous Na₂SO₄ and concentrated *in vacuo.* The residue was purified by silica gel column chromatography (PE/EtOAc (v/v) = 1/2) to give the title compound as a beige solid (4.0 g, 72.4%). The compound was characterized by the following spectroscopic data:
¹H NMR (400 MHz, CDCl₃) *δ* (ppm): 7.65-7.60 (m, 2H), 7.47-7.43 (m, 2H), 7.22-7.20 (m, 1H), 5.67-5.65 (m, 1H), 5.24-5.22 (m, 1H), 4.34-4.30 (m, 1H), 3.85-3.81 (m, 1H), 3.72 (s, 3H), 3.71-3.64 (m, 1H), 3.00 (s, 1H), 2.34-2.31 (m, 1H), 2.21-1.95 (m, 5H),1.45-1.32 (m, 12H), 1.04-1.02 (m, 1H), 0.88-0.86 (d, 6H).

### Step 4) the preparation of compound 6-4

A suspension of compound 1-8 (514 mg, 1.1 mmol), compound 6-3 (1.39 g, 2.8 mmol), Pd(PPh₃)₄ (250 mg, 0.2 mmol) and K₂CO₃ (910 mg, 6.6 mmol) in mixed solvents of DME (20.0 mL) and H₂O (4.0 mL) was stirred at 90 °C under N₂ for 3.0 hrs. After the reaction was completed, the mixture was cooled to rt and diluted with EtOAc (200 mL). The resulting mixture was washed with water (50 mL x 3) and brine, dried over anhydrous Na₂SO₄ and concentrated *in vacuo.* The residue was purified by silica gel column chromatography (DCM/MeOH (v/v) = 50/1) to give the title compound as a pale yellow solid (750 mg, 75%). The compound was characterized by the following spectroscopic data:
MS (ESI, pos.ion) *m*/*z*: 455.5 [M+2H]²⁺;
¹H NMR (400 MHz, CDCl₃) *δ* (ppm): 7.76-7.68 (m, 4H), 7.65, 7.63 (s, s, 1H), 7.59 (s, 1H), 7.55-7.46 (m, 5H), 7.39 (s, 1H), 5.56, 5.55 (d, d, 2H), 5.23-5.19 (m, 1H), 5.07-5.03 (m, 1H), 4.34-4.30 (m, 2H), 3.85-3.78 (m, 2H), 3.68-3.67 (m, 2H), 3.66 (s, 6H), 2.84-2.80 (m, 2H), 2.43-1.92 (m, 16H), 1.76-1.58 (m, 4H), 1.02, 1.00 (m, m, 6H), 0.93, 0.91 (m, m, 6H).

### Example 7

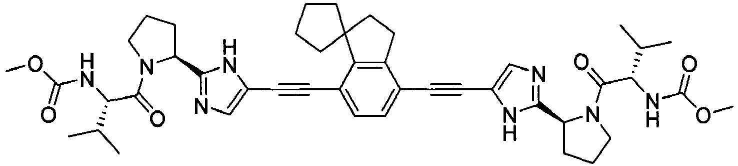

### Synthetic route:

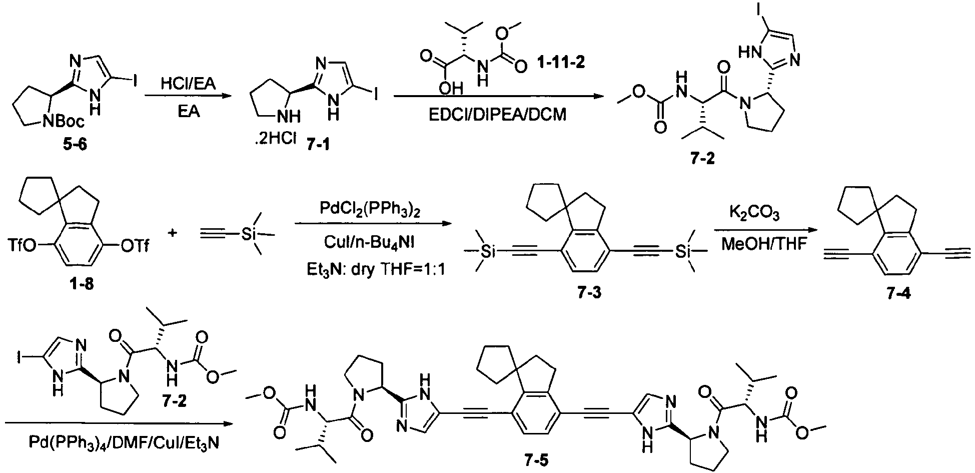

### Step 1) the preparation of compound 7-1

To a solution of compound **5-6** (1.50 g, 4.13 mmol) in EtOAc (10 mL) was added a solution of HCl in EtOAc (5.0 mL, 4 M) dropwise. At the end of the addition, the mixture was stirred at rt overnight. After the reaction was completed, the mixture was filtered, and the filter cake (1.2 g, 86.45%) was used for the next step without further purification. The compound was characterized by the following spectroscopic data:
MS (ESI, pos.ion) *m*/*z*: 264 [M+H]⁺.

### Step 2) the preparation of compound 7-2

A suspension of compound **7-1** (1.2 g, 3.6 mmol), compound **1-11-2** (0.69 g, 3.9 mmol) and EDCI (0.75 g, 3.9 mmol) in DCM (20 mL) was stirred at 0 °C for 5 mins, then DIPEA (2.38 mL, 14.4 mmol) was added dropwise. At the end of the addition, the mixture was stirred at rt for 2.0 hrs. After the reaction was completed, the mixture was diluted with DCM (40 mL). The organic layer was washed with saturated NH₄Cl aqueous solution, dried over anhydrous Na₂SO₄ and concentrated *in vacuo.* The residue was purified by silica gel column chromatography (PE/EtOAc (v/v) = 1/2) to give the title compound as a pale yellow solid (1.31 g, 86.8%). The compound was characterized by the following spectroscopic data:
MS (ESI, pos.ion) *m*/*z*: 421.1 [M+H]⁺;
¹H NMR (400 MHz, CDCl₃) *δ* (ppm): 7.35 (s, 1H), 5.32, 5.29 (brs, brs, 1H), 5.20-5.15 (m, 1H), 4.41-4.37 (m, 1H), 3.85-3.78 (m, 1H), 3.69-3.65 (m, 1H), 3.63 (s, 3H), 2.28-2.17 (m, 3H), 2.11-1.96 (m, 2H), 0.97-0.95 (m, 3H), 0.91-0.89 (m, 3H).

### Step 3) the preparation of compound 7-3

To a mixture of compound **1-8** (1.59 g, 3.4 mmol), tetrabutylammonium iodide (3.77 g, 10.2 mmol), CuI (195 mg, 1.02 mmol) and PdCl₂(PPh₃)₂ (239 mg, 0.34 mmol) were added anhydrous THF (8.0 mL) and Et₃N (8.0 mL) separately under N₂. The mixture was stirred at rt for 10 mins, then trimethylsilylacetylene (2.4 mL) was added. At the end of the addition, the mixture was stirred at 50 °C for 2.0 hrs. After the reaction was completed, the mixture was concentrated *in vacuo.* The residue was purified by silica gel column chromatography (PE) to give the title compound as pale yellow liquid (681 mg, 55%). The compound was characterized by the following spectroscopic data:
MS (ESI, pos.ion) *m*/*z*: 365.5 [M+H]⁺;
¹H NMR (400 MHz, CDCl₃) *δ* (ppm): 7.02, 7.00 (s, s, 1H), 6.92-6.91, 6.90-6.89 (dd, dd, 1H), 3.05-3.02 (m, 2H), 2.41-2.18 (m, 6H), 1.77-1.59 (m, 4H), 0.29-0.27 (m, 18H).

### Step 4) the preparation of compound 7-4

To a solution of compound **7-3** (324 mg, 0.89 mmol) in mixed solvents of MeOH (4.0 mL) and THF (4.0 mL) was added K₂CO₃ (492 mg, 3.56 mmol), and the mixture was stirred at rt for 5.0 hrs. After the reaction was completed, the mixture was diluted with EtOAc (20 mL) and filtered. The filtrate was concentrated *in vacuo.* The residue was purified by silica gel column chromatography (PE) to give the title compound as a gray solid (160.65 mg, 82%). The compound was characterized by the following spectroscopic data:
MS (ESI, pos.ion) *m*/*z*: 221.5 [M+H]⁺;
¹H NMR (400 MHz, CD_{3O}D) *δ* (ppm): 6.82, 6.80 (s, s, 1H), 6.66, 6.64 (m, m, 1H), 3.26-3.25 (m, 1H), 3.06-3.05 (m, 1H), 2.98-2.94 (m, 2H), 2.43-2.21 (m, 6H), 1.77-1.60 (m, 4H).

### Step 5) the preparation of compound 7-5

To a mixture of compound **7-4** (160.69 mg, 0.73mmol), compound **7-2** (672.4 mg, 1.6 mmol), CuI (28 mg, 0.147 mmol) and Pd(PPh₃)₄ (84 mg, 0.073 mmol) were added anhydrous DMF (6.0 mL) and Et₃N (0.2 mL) separately under N₂. At the end of the addition, the mixture was stirred at rt for 20 hrs. After the reaction was completed, the mixture was concentrated *in vacuo.* The residue was purified by silica gel column chromatography (EtOAc) to give the title compound as a yellow solid (381.7 mg, 65%). The compound was characterized by the following spectroscopic data:
MS (ESI, pos.ion) *m*/*z*: 805.5 [M+H]⁺;
¹H NMR (400 MHz, CD₃OD) *δ* (ppm): 7.66 (s, 1H), 7.48 (s, 1H), 7.27, 7.25 (s, s, 1H), 7.24, 7.22 (m, m, 1H), 5.51-5.47 (m, 2H), 5.32, 5.30 (d, d, 2H), 4.41-4.36 (m, 2H), 3.89-3.83 (m, 2H), 3.73-3.66 (m, 2H), 3.63 (s, 6H), 3.06-3.02 (m, 2H), 2.40-1.92 (m, 16H), 1.77-1.59 (m, 4H), 0.97, 0.95 (m, m, 6H), 0.91, 0.89 (m, m, 6H).

### Example 8

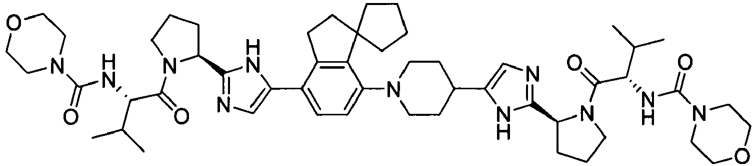

### Synthetic routes:

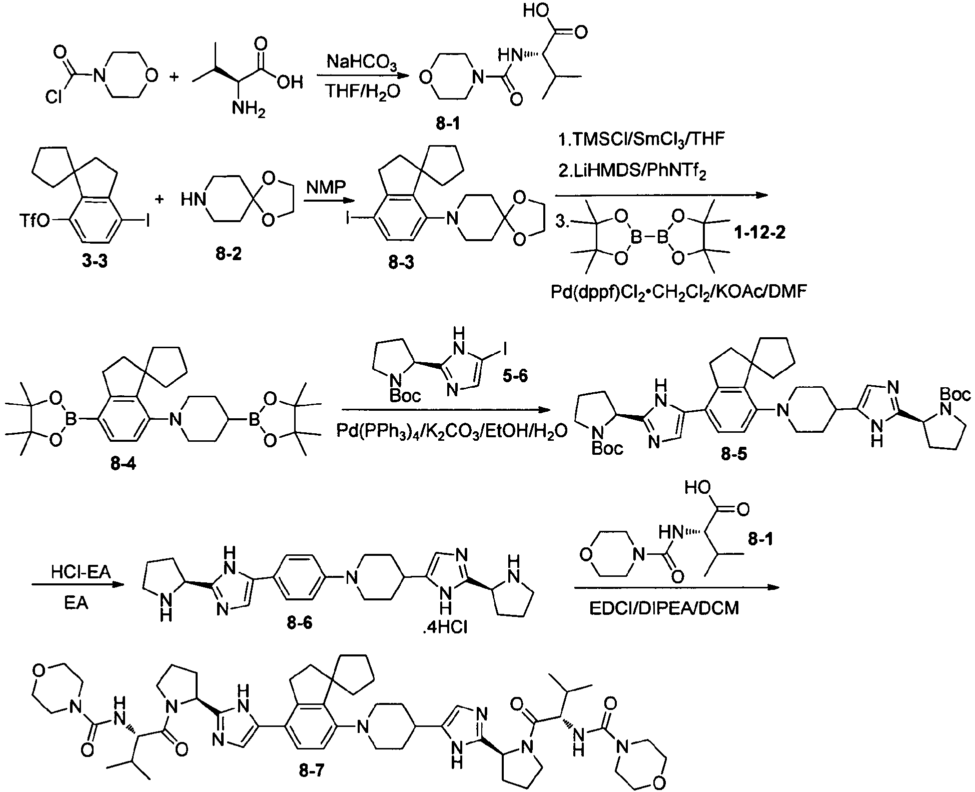

### Step 1) the preparation of compound 8-1

To a solution of L-Valine (2.49 g, 21.3 mmol) in THF (64.5 mL) was added NaHCO₃ aqueous solution (5.37 g, 64 mmol, 64.5 mL). The mixture was stirred at rt for 10 mins, and morpholine-4-carbonyl chloride (2.8 mL, 23.5 mmol) was added dropwise. At the end of the addition, the mixture was stirred at rt overnight. After the reaction was completed, the mixture was concentrated *in vacuo.* The resulting mixture was adjusted to pH 3 with hydrochloric acid (1 M) and extracted with EtOAc (50 mL x 3). The combined organic layers were washed with brine, dried over anhydrous Na₂SO₄ and concentrated *in vacuo* to give the title compound as a white solid (2.94 g, 60%). The compound was characterized by the following spectroscopic data:
MS (ESI, pos.ion) *m*/*z*: 231.3 [M+H]⁺;
¹H NMR (400 MHz, CDCl₃) *δ* (ppm): 9.31 (br, 1H), 5.75, 5.73 (br, br, 1H), 4.27-4.22 (m, 1H), 3.73-3.65 (m, 4H), 3.34-3.28 (m, 4H), 2.35-2.24 (m, 1H), 0.97-0.95 (m, 3H), 0.88-0.85 (m, 3H).

### Step 2) the preparation of compound 8-3

A mixture of compound **3-3** (892 mg, 2.0 mmol), compound **8-2** (715.45 mg, 5.0 mmol) and NMP (10 mL) was placed inside the center of a microwave oven and exposed to microwave irradiation at 250 W to reflux for 60 mins at 200 °C. After the reaction was completed, the mixture was cooled to rt, and water (50 mL) was added. The aqueous layer was extracted with Et₂O (50 mL x 3). The combined organic layers were washed with brine, dried over anhydrous Na₂SO₄ and concentrated *in vacuo.* The residue was purified by silica gel column chromatography (PE/EtOAc (v/v) = 20/1) to give the title compound (492 mg, 56%). The compound was characterized by the following spectroscopic data:
MS (ESI, pos.ion) *m*/*z*: 440.5 [M+H]⁺;
¹H NMR (400 MHz, CDCl₃) *δ* (ppm): 7.67, 7.65 (s, s, 1H), 6.34-6.33, 6.32-6.31 (dd, dd, 1H), 4.01-3.87 (m, 4H), 3.15-3.10 (m, 4H), 2.75-2.70 (m, 2H), 2.33-2.21 (m, 6H), 1.91-1.82 (m, 4H), 1.80-1.64 (m, 4H).

### Step 3) the preparation of compound 8-4

To a mixture of compound **8-3** (2.24 g, 5.1 mmol) and SmCl₃ (131 mg, 0.51 mmol) was added THF (20 mL) under N₂. The mixture was stirred at rt for 15 mins, and then TMSCl (610 mg, 5.61 mmol) was added dropwise. At the end of the addition, the mixture was stirred at rt for 10 hrs. After the reaction was completed, the mixture was filtered through a celite pad. The filtrate was concentrated *in vacuo* to give compound (a) (1.8 g), which was used for the next step without further purification.

To a solution of compound (a) in THF (20 mL) was added LiHMDS (6.5 mL, 6.46 mmol, 1 M in THF) dropwise at -78 °C. The mixture was stirred at -78 °C for 30 mins, and then PhNTf₂ (2.77 g, 7.76 mmol) was added dropwise. At the end of the addition, the mixture was stirred at -78 °C for 30 mins and at rt for another 10 hrs. After the reaction was completed, the mixture was quenched with ice water (50 mL) and the aqueous layer was extracted with EtOAc (50 mL x 3). The combined organic layers were washed with brine, dried over anhydrous Na₂SO₄ and concentrated *in vacuo* to give compound (b) (1.0 g), which was used for the next step without further purification.

A mixture of compound (b) (1.0 g, 2.0 mmol), compound **1-12-2** (1.27 g, 5.0 mmol), Pd(dppf)Cl₂·CH₂Cl₂ (0.16 g, 0.2 mmol) and KOAc (0.78 g, 8.0 mmol) in DMF (20 mL) was stirred at 90 °C under N₂ overnight. After the reaction was completed, the mixture was cooled to rt and diluted with EtOAc (200 mL). The resulting mixture was filtered through a celite pad. The filtrate was washed with water (40 mL x 3) and brine, dried over anhydrous Na₂SO₄ and concentrated *in vacuo.* The residue was purified by silica gel column chromatography (PE/EtOAc (v/v) = 1/2) to give the title compound as a beige solid (932 mg, 36%). The compound was characterized by the following spectroscopic data:
MS (ESI, pos.ion) *m*/*z*: 508.5 [M+H]⁺;
¹H NMR (400 MHz, CDCl₃) *δ* (ppm): 7.49, 7.47 (s, s, 1H), 6.78, 6.76 (d, d, 1H), 2.91-2.86 (m, 4H), 2.80-2.76 (m, 2H), 2.28-2.20 (m, 4H), 2.00-1.92 (m, 2H), 1.87-1.60 (m, 8H), 1.32 (m, 6H), 1.29 (m, 6H), 1.25 (m, 6H), 1.22 (m, 6H), 0.88-0.80 (m, 1H).

### Step 4) the preparation of compound 8-5

To a mixture of compound **8-4** (3.0 g, 5.91 mmol), compound **5-6** (4.94 g, 13.6 mmol), Pd(PPh₃)₄ (342 mg, 0.296 mmol) and K₂CO₃ (2.47 g, 17.73 mmol) were added EtOH (60.0 mL) and pure water (12 mL) via syringe, and the mixture was stirred at 90 °C under N₂ for 3.0 hrs. After the reaction was completed, the mixture was *concentrated in vacuo.* The residue was dissolved in EtOAc (200 mL). The resulting mixture was washed with water (50 mL x 3) and brine, dried over anhydrous Na₂SO₄ and concentrated *in vacuo.* The residue was purified by silica gel column chromatography (DCM/MeOH (v/v) = 200/1) to give the title compound as a beige solid (2.14 g, 50%). The compound was characterized by the following spectroscopic data:
MS (ESI, pos.ion) *m*/*z*: 726.5 [M+H]⁺;
¹H NMR (400 MHz, CDCl₃) *δ* (ppm): 7.50 (s, 1H), 7.16, 7.14 (s, s, 1H), 6.81, 6.80 (d, 1H, *J =* 4.0 Hz), 6.46, 6.44 (dd, dd, 1H), 5.14-5.08 (m, 1 H), 4.87-4.80 (m, 1 H), 3.87-3.85 (m, 1H), 3.73-3.67 (m, 1H), 3.65-3.58 (m, 1H), 3.44-3.36 (m, 1H), 3.31-3.21 (m, 5H), 2.77-2.72 (m, 2H), 2.62-2.52 (m, 1H), 2.47-1.96 (m, 16H), 1.83-1.58 (m, 6H), 1.53 (s, 18H).

### Step 5) the preparation of compound 8-6

To a solution of compound **8-5** (1.02 g, 1.4 mmol) in EtOAc (10 mL) was added a solution of HCl in EtOAc (10.0 mL, 4 M) dropwise. At the end of the addition, the mixture was stirred at rt for 8.0 hrs. After the reaction was completed, the mixture was concentrated *in vacuo.* The residue was purified by beating in EtOAc (10.0 mL) and filtered to give the title compound as a pale yellow solid (727.4 mg, 90%). The compound was characterized by the following spectroscopic data:
MS (ESI, pos.ion) *m*/*z*: 432.5 [M+H]⁺.

### Step 6) the preparation of compound 8-7

To a mixture of compound **8-6** (384 mg, 0.6654 mmol), EDCI (192 mg, 0.998 mmol) and compound 8-1 (230 mg, 0.998 mmol) in DCM (10 mL) was added DIPEA (0.74 mL, 4.5 mmol) dropwise at 0 °C. At the end of the addition, the mixture was stirred at rt for 10.0 hrs. After the reaction was completed, the mixture was diluted with DCM (50 mL), washed with saturated NH₄Cl aqueous solution and brine, dried over anhydrous Na₂SO₄ and concentrated *in vacuo.* The residue was purified by silica gel column chromatography (DCM/MeOH (v/v) = 50/1) to give the title compound as a pale yellow solid (253 mg, 40%). The compound was characterized by the following spectroscopic data:
MS (ESI, pos.ion) *m*/*z*: 476.5 [M+2H]²⁺;
¹H NMR (400 MHz, CDCl₃) *δ* (ppm): 7.44 (s, 1H), 7.16, 7.14 (s, s, 1H), 6.82 (d, 1H), 6.46, 6.44 (dd, dd, 1H), 5.67, 5.65 (d, d, 2H), 5.36-5.32 (m, 1H), 5.12-5.08 (m, 1H), 4.51-4.47 (m, 2H), 3.87-3.79 (m, 2H), 3.74-3.62 (m, 10H), 3.35-3.28 (m, 8H), 3.26-3.21 (m, 4H), 2.77-2.72 (m, 2H), 2.62-2.52 (m, 1H), 2.45-1.90 (m, 18H), 1.83-1.58 (m, 6H), 1.02, 1.01 (m, m, 6H), 0.92, 0.91 (m, m, 6H).

### Example 9

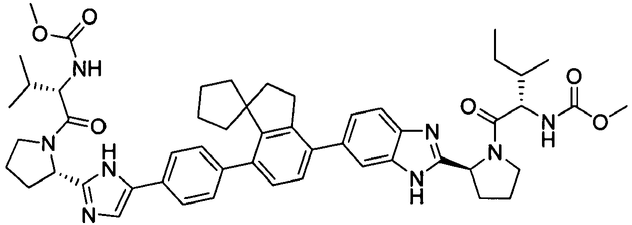

### Synthetic route:

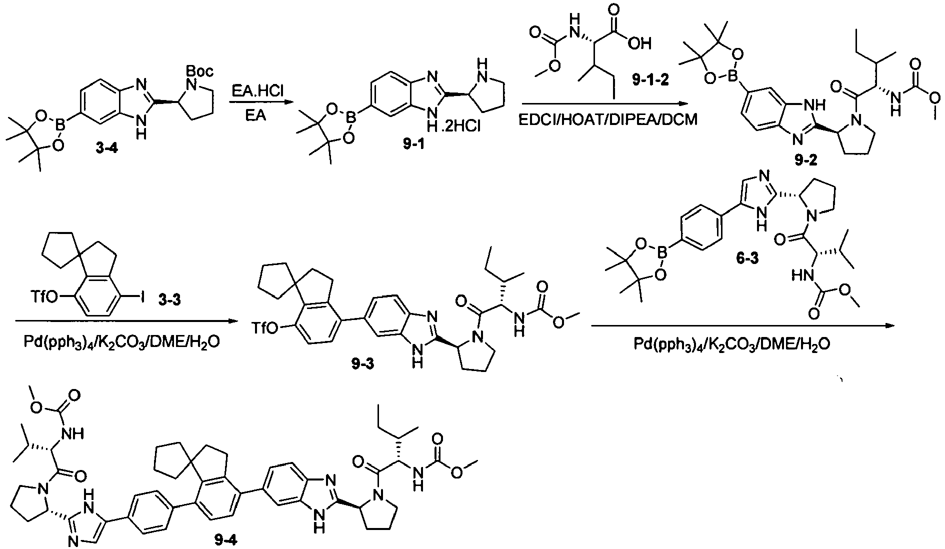

### Step 1) the preparation of compound 9-1

To a solution of compound **3-4** (578.55 mg, 1.4 mmol) in EtOAc (10 mL) was added a solution of HCl in EtOAc (5.0 mL, 4 M) dropwise. At the end of the addition, the mixture was stirred at rt for 8.0 hrs. After the reaction was completed, the mixture was concentrated *in vacuo.* The residue was purified by beating in EtOAc (10 mL) and filtered to give the title compound as a pale yellow solid (347.58 mg, 90%). The compound was characterized by the following spectroscopic data:
MS (ESI, pos.ion) *m*/*z*: 314.5 [M+H]⁺.

### Step 2) the preparation of compound 9-2

To a suspension of compound **9-1** (1.0 g, 2.6 mmol), compound **9-1-2** (0.589 g, 3.1 mmol), EDCI (0.55 g, 2.86 mmol) and HOAT (0.35 g, 2.6 mmol) in DCM (15 mL) was added DIPEA (1.72 mL, 10.4 mmol) dropwise at 0 °C. At the end of the addition, the mixture was stirred at rt for 3.0 hrs. After the reaction was completed, the mixture was diluted with DCM (40 mL). The resulting mixture was washed with saturated NH₄Cl aqueous solution and brine, dried over anhydrous Na₂SO₄ and concentrated *in vacuo.* The residue was purified by silica gel column chromatography (PE/EtOAc (v/v) = 1/2) to give the title compound as a white solid (1.17 g, 93%). The compound was characterized by the following spectroscopic data:
MS (ESI, pos.ion) *m*/*z*: 485.4 [M+H]⁺;
¹H NMR (400 MHz, CDCl₃) *δ* (ppm): 10.62 (brs, 1H), 8.22 (m, 1H), 7.73-7.65 (m, 2H), 5.72 (d, 1H, *J* = 8.0 Hz), 5.43 (d, 1H, *J* = 8.0 Hz), 4.35-4.31 (m, 1H), 3.95-3.88 (m, 1H), 3.78-3.75 (m, 1H), 3.69-3.67 (m, 4H), 3.08-3.04 (m, 1H), 2.43-2.37 (m, 1H), 2.25-2.15 (m, 2H), 1.91 (s, 1H), 1.74-1.72 (m, 1H), 1.52-1.50 (m, 1H), 1.35 (s, 12H), 1.24 (t, 2H, *J=* 8.0 Hz), 1.12-1.10 (m, 1H), 0.93-0.88 (m, 1H).

### Step 3) the preparation of compound 9-3

To a mixture of compound **3-3** (453.14 mg, 1.016 mmol), compound **9-2** (492 mg, 1.016 mmol), Pd(PPh₃)₄ (117 mg, 0.1016 mmol) and K₂CO₃ (420.7 mg, 3.048 mmol) were added DME (10.0 mL) and pure water (2.5 mL) via syringe, and the mixture was stirred at 90 °C under N₂ for 3.0 hrs. After the reaction was completed, the mixture was cooled to rt and diluted with EtOAc (20 mL). The resulting mixture was washed with water (10 mL x 3) and brine, dried over anhydrous Na₂SO₄ and concentrated *in vacuo.* The residue was purified by silica gel column chromatography (PE/EtOAc (v/v) = 1/3) to give the title compound as a pale yellow solid (412 mg, 60%). The compound was characterized by the following spectroscopic data:
MS (ESI, pos.ion) *m*/*z*: 677.3 [M+H]⁺;
¹H NMR (400 MHz, CDCl₃) *δ* (ppm): 7.55-7.54 (m, 1H), 7.52, 7.50 (d, d, 1H), 7.44, 7.42 (d, d, 1H), 7.41-7.40, 7.39-7.38 (d, d, 1H, *J =* 4.0 Hz), 7.06, 7.04 (s, s, 1H), 5.31-5.30, 5.29-5.28 (d, d, 1H, *J=* 4.0 Hz), 5.13-5.09 (m, 1H), 4.46-4.41 (m, 1H), 3.84-3.77 (m, 1H), 3.67-3.65 (m, 1H), 3.63 (s, 3H), 2.82-2.78 (m, 2H), 2.43-1.49 (m, 15H), 1.24-1.11 (m, 2H), 0.92-0.88 (m, 6H).

### Step 4) the preparation of compound 9-4

To a mixture of compound **9-3** (353 mg, 0.522 mmol), compound **6-3** (284.87 mg, 0.574 mmol), Pd(PPh₃)₄ (60.29 mg, 0.0522 mmol) and K₂CO₃ (216 mg, 1.566 mmol) were added DME (6.0 mL) and pure water (1.5 mL) via syringe, and the mixture was stirred at 90 °C under N₂ for 3.0 hrs. After the reaction was cooled to rt, the mixture was diluted with EtOAc (40 mL). The resulting mixture was washed with water (20 mL x 3) and brine, dried over anhydrous Na₂SO₄ and *concentrated in vacuo.* The residue was purified by silica gel column chromatography (DCM/EtOH (v/v) = 80/1) to give the title compound as a pale yellow solid (233.98 mg, 50%). The compound was characterized by the following spectroscopic data:
MS (ESI, pos.ion) *m*/*z*: 499.5 [M+2H]²⁺;
¹H NMR (400 MHz, CDCl₃) *δ* (ppm): 7.60-7.42 (m, 10H), 5.56, 5.55 (d, d, 1H), 5.30, 5.29 (d, d, 1H), 5.23-5.19 (m, 1H), 5.13-5.09 (m, 1H), 4.46-4.41 (m, 1H), 4.34-4.30 (m, 1H), 3.85-3.77 (m, 2H), 3.68-3.67 (m, 2H), 3.66 (s, 3H), 3.63 (s, 3H), 3.10-3.06 (m, 2H), 2.43-1.86 (m, 16H), 1.76-1.48 (m, 5H), 1.24-1.11 (m, 1H), 1.02, 1.00 (m, m, 3H), 0.93, 0.91 (m, m, 3H), 0.90-0.88 (m, 6H).

### Example 10

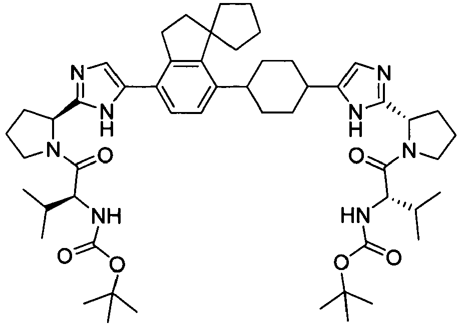

### Synthetic route:

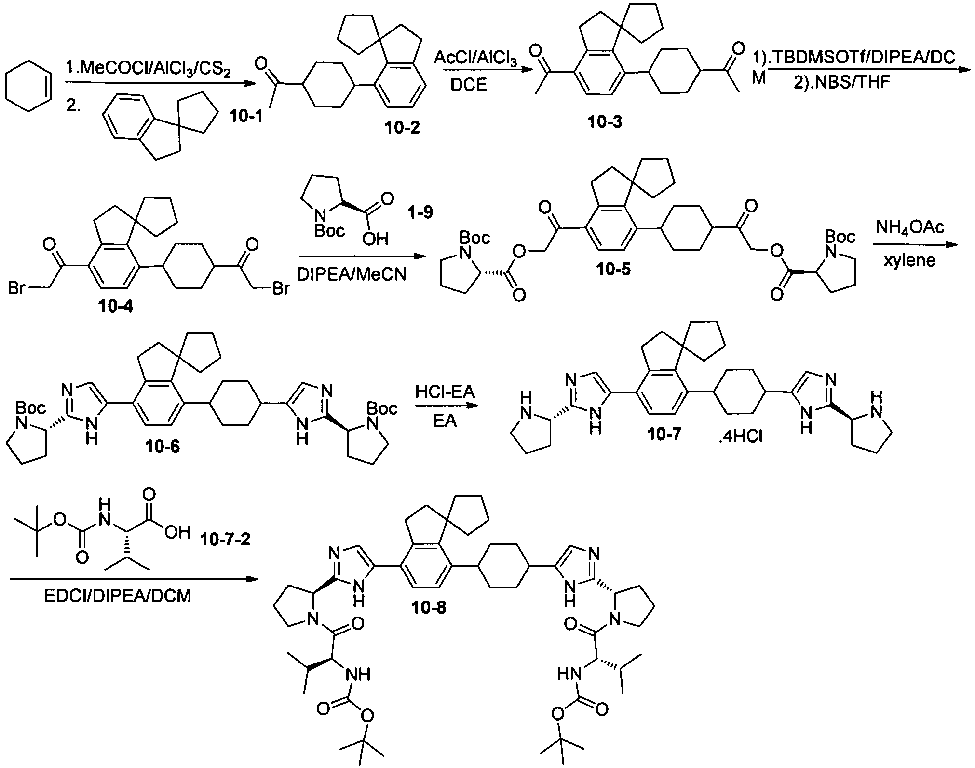

### Step 1) the preparation of compound 10-2

To a suspension of aluminium chloride (2.15 g, 16.2 mmol) in carbon disulphide (40 mL) was added acetyl chloride (1.4 mL, 19.7 mmol) dropwise at 0 °C. After the mixture was stirred for 10 mins, a solution of cyclohexene (1.0 mL, 10 mmol) in CS₂ (20 mL) was added. At the end of the addition, the mixture was stirred at rt for 2.0 hrs. After the reaction was completed, the mixture was concentrated *in vacuo.* To the residue was added compound 10-1 (2.58 g, 15 mmol), and the mixture was stirred at 50 °C for 4.0 hrs. After the reaction was completed, the mixture was quenched with ice water (40 mL). The aqueous layer was extracted with EtOAc (100 mL x 3). The combined organic lays were washed with brine, dried over anhydrous Na₂SO₄ and concentrated *in vacuo.* The residue was purified by silica gel column chromatography (PE/DCM (v/v) = 10/1) to give the title compound as colorless oil (889 mg, 30%). The compound was characterized by the following spectroscopic data:
MS (ESI, pos.ion) *m*/*z*: 297.3 [M+H]⁺;
¹H NMR (400 MHz, CDCl₃) *δ* (ppm): 7.23, 7.21, 7.19 (m, m, m, 1H), 7.09-7.05 (m, 1H), 6.75-6.72 (m, 1H), 2.89-2.80 (m, 1H), 2.79-2.77 (m, 2H), 2.31-2.18 (m, 3H), 2.11 (br, 3H), 2.10-2.05 (m, 2H), 1.84-1.50 (m, 14H).

### Step 2) the preparation of compound 10-3

To a suspension of aluminium chloride (2.12 g, 15.9 mmol) in 1,2-dichloroethane (40 mL) was added acetyl chloride (1.2 mL, 16.8 mmol) dropwise at 0 °C followed by a solution of compound **10-2** (3.85 g, 13 mmol) in 1,2-dichloroethane (20 mL). At the end of the addition, the mixture was stirred at rt for 2.0 hrs. After the reaction was completed, the mixture was quenched with ice water (40 mL). The aqueous layer was extracted with EtOAc (100 mL x 3). The combined organic layers were washed with brine, dried over anhydrous Na₂SO₄ and concentrated *in vacuo.* The residue was purified by silica gel column chromatography (PE/EtOAc (v/v) = 10/1) to give the title compound as a white solid (1.67 g, 38%). The compound was characterized by the following spectroscopic data:
MS (ESI, pos.ion) *m*/*z*: 339.3 [M+H]⁺;
¹H NMR (400 MHz, CDCl₃) *δ* (ppm): 7.50, 7.48 (d, d, 1H), 6.89, 6.87 (m, m, 1H), 3.15-3.06 (m, 1H), 2.93-2.89 (m, 2H), 2.55 (s, 3H), 2.31-2.15 (m, 5H), 2.11 (s, 3H), 1.85-1.50 (m, 14H).

### Step 3) the preparation of compound 10-4

To a solution of compound **10-3** (1.601 g, 4.73 mmol) in DCM (30 mL) was added DIPEA (2.33 mL, 14.1 mmol) at 0 °C. After the mixture was stirred for 10 mins, TBDMSOTf (3.5 mL, 11.5 mmol) was added dropwise. At the end of the addition, the mixture was stirred at rt for 2.0 hrs. After the reaction was completed, the mixture was quenched with water (20 mL). The aqueous layer was extracted with DCM (40 mL x 3). The combined organic layers were washed with brine, dried over anhydrous Na₂SO₄ and concentrated *in vacuo* to give the compound as yellow gel-like substance. To the solution of yellow gel-like substance in THF (20 mL) was added NBS (1.56 g, 8.76 mmol) at 0 °C. At the end of the addition, the mixture was stirred at 0 °C for 4.0 hrs. After the reaction was completed, the THF solvent was removed, and water (20 mL) was added. The aqueous layer was extracted with EtOAc (50 mL x 3). The combined organic layers were washed with brine, dried over anhydrous Na₂SO₄ and *concentrated in vacuo.* The residue was purified by silica gel column chromatography (PE/EtOAc (v/v) = 10/1) to give the title compound as white slurry (1.168 g, 50%). The compound was characterized by the following spectroscopic data:
MS (ESI, pos.ion) *m*/*z*: 495.3 [M+H]⁺;
¹H NMR (400 MHz, CDCl₃) *δ* (ppm): 7.59, 7.57 (d, d, 1H), 6.88, 6.85 (m, m, 1H), 4.36 (s, 2H), 3.93 (s, 2H), 3.14-3.05 (m, 1H), 2.99-2.94 (m, 2H), 2.86-2.77 (m, 1H), 2.27-2.15 (m, 4H), 1.84-1.56 (m, 14H).

### Step 4) the preparation of compound 10-5

To a solution of compound **10-4** (1.07 g, 2.18 mmol) in MeCN (22 mL) were added DIPEA (1.02 mL, 6.2 mmol) and compound 1-9 (1.08 g, 5.014 mmol) at 0 °C separately. At the end of the addition, the mixture was stirred at rt for 1.0 hr. After the reaction was completed, the mixture was concentrated *in vacuo.* The residue was purified by silica gel column chromatography (PE/EtOAc (v/v) = 2/3) to give the title compound as pale yellow slurry (1.33 g, 80%). The compound was characterized by the following spectroscopic data:
MS (ESI, pos.ion) *m*/*z*: 765.3 [M+H]⁺.

### Step 5) the preparation of compound 10-6

A suspension of compound **10-5** (1.4 g, 1.83 mmol) and NH₄OAc (2.82 g, 36.6 mmol) in xylene (20 mL) was stirred at 140 °C for 5.0 hrs. After the reaction was completed, the mixture was cooled to rt, and 40 mL of water was added. The aqueous layer was extracted with EtOAc (50 mL x 3). The combined organic layers were washed with brine, dried over anhydrous Na₂SO₄ and concentrated *in vacuo.* The residue was purified by silica gel column chromatography (DCM/MeOH (v/v) = 100/1) to give the title compound as a pale yellow solid (862 mg, 65%). The compound was characterized by the following spectroscopic data:
MS (ESI, pos.ion) *m*/*z*: 725.5 [M+H]⁺;
¹H NMR (400 MHz, CDCl₃) *δ* (ppm): 7.16 (s, 1H), 7.10, 7.09 (d, d, 1H), 6.83-6.82 (m, 0.5H), 6.81-6.80 (m, 1.5 H), 5.14-5.08 (m, 1H), 4.87-4.80 (m, 1H), 3.74-3.66 (m, 1H), 3.65-3.58 (m, 1H), 3.44-3.36 (m, 1H), 3.31-3.24 (m, 1H), 3.01-2.92 (m, 1H), 2.75-2.65 (m, 3H), 2.47-1.57 (m, 26H), 1.53 (s, 9H), 1.41 (s, 9H).

### Step 6) the preparation of compound 10-7

To a solution of compound **10-6** (963.55 mg, 1.33 mmol) in EtOAc (10 mL) was added a solution of HCl in EtOAc (10 mL, 4 M) dropwise. At the end of the addition, the mixture was stirred at rt for 8.0 hrs. After the reaction was completed, the mixture was *concentrated in vacuo.* The residue was purified by beating in EtOAc (20 mL) and filtered to give the title compound as pale yellow powder (713 mg, 80%). The compound was characterized by the following spectroscopic data:
MS (ESI, pos.ion) *m*/*z:* 525.7 [M+H]⁺.

### Step 7) the preparation of compound 10-8

To a suspension of compound **10-7** (402.2 mg, 0.6 mmol), EDCI (300.56 mg, 1.56 mmol) and compound **10-7-2** (341 mg, 1.56 mmol) in DCM (10.0 mL) was added DIPEA (1.02 mL, 6.14 mmol) dropwise at 0 °C. At the end of the addition, the mixture was stirred at rt for 10 hrs. After the reaction was completed, the mixture was diluted with DCM (20 mL), washed with saturated NH₄Cl aqueous solution and brine, dried over anhydrous Na₂SO₄ and concentrated *in vacuo.* The residue was purified by silica gel column chromatography (DCM/MeOH (v/v) = 50/1) to give the title compound as a pale yellow solid (332 mg, 60%). The compound was characterized by the following spectroscopic data:
MS (ESI, pos.ion) *m*/*z:* 463.5 [M+2H]²⁺;
¹H NMR (400 MHz, CDCl₃) *δ* (ppm): 7.56 (s, 1H), 7.11, 7.09 (d, d, 1H), 6.83-6.80 (m, 2H), 5.32-5.28 (m, 1H), 5.20, 5.18 (d, d, 2H), 5.12-5.07 (m, 1H), 4.40-4.35 (m, 2H), 3.86-3.78 (m, 2H), 3.70-3.61 (m, 2H), 3.01-2.92 (m, 1H), 2.75-2.65 (m, 3H), 2.30-1.57 (m, 28H), 1.43 (s, 18H), 0.97, 0.95 (m, m, 6H), 0.91, 0.89 (m, m, 6H).

### Example 11

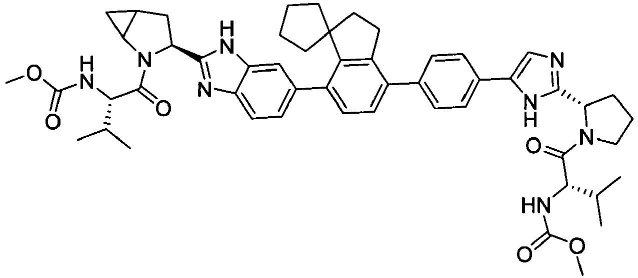

### Synthetic route:

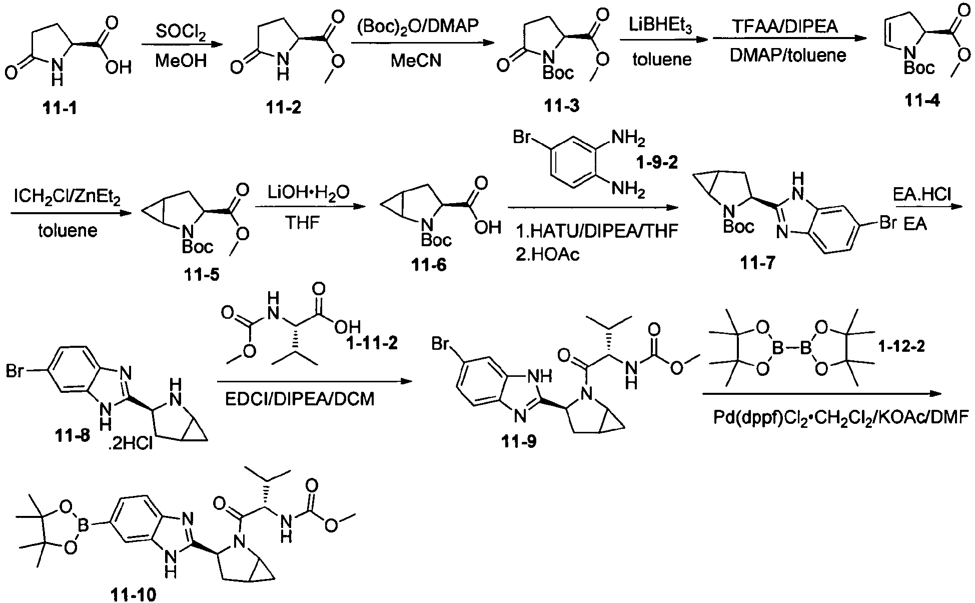

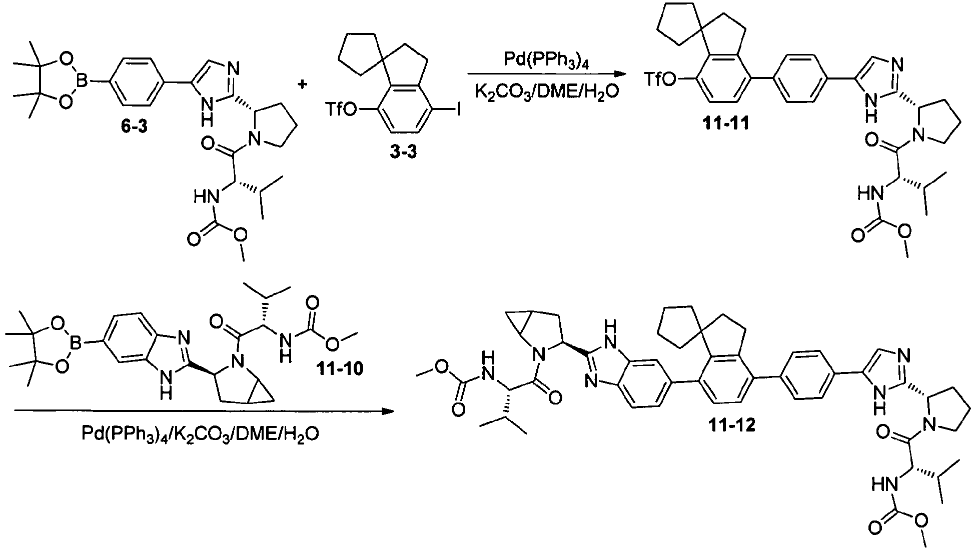

### Step 1) the preparation of compound 11-2

To a solution of compound **11-1** (10 g, 77.5 mmol) in MeOH (50 mL) was added thionyl chloride (5.5 mL, 75.8 mmol) dropwise at 0 °C. At the end of the addition, the mixture was stirred at 0 °C for 1.0 hr and at rt for another 2.0 hrs. After the reaction was completed, the mixture was quenched with NaHCO₃ aqueous solution (50 mL), and the MeOH solvent was removed. The resulting mixture was extracted with CH₂Cl₂ (35 mL x 3). The combined organic layers were washed with brine, dried over anhydrous Na₂SO₄ and concentrated *in vacuo.* The residue was purified by silica gel column chromatography (EtOAc) to give the title compound as colorless liquid (7.5 g, 67.6%). The compound was characterized by the following spectroscopic data:
MS (ESI, pos.ion) *m*/*z*: 144.2 [M-Boc]⁺;
¹H NMR (400 MHz, CDCl₃) *δ* (ppm): 7.38 (br, 1H), 4.20-4.16 (m, 1H), 3.67 (s, 3H), 2.39-2.23 (m, 3H), 2.14-2.07 (m, 1H).

### Step 2) the preparation of compound 11-3

To a solution of compound **11-2** (6.45 g, 45.06 mmol) in MeCN (30 mL) was added DMAP (0.5503 g, 4.5 mmol) at 0 °C. After the mixture was stirred for 10 mins, di-*tert*-butyl dicarbonate (10.816 g, 49.56 mmol) was added dropwise. At the end of the addition, the mixture was stirred at 0 °C for 30 mins and at rt for another 2.0 hrs. After the reaction was completed, the mixture was *concentrated in vacuo.* The residue was purified by silica gel column chromatography (PE/EtOAc (v/v) = 1/1) to give the title compound as colorless liquid (5.0 g, 45.6%). The compound was characterized by the following spectroscopic data:
MS (ESI, pos.ion) *m*/*z*: 144.2 [M+H]⁺;
¹H NMR (400 MHz, CDCl₃) *δ* (ppm): 4. 60-4.57 (m, 1H), 3.75 (s, 3H), 2.65-2.55 (m, 1H), 2.50-2.42 (m, 1H), 2.36-2.24 (m, 1H), 2.04-1.96 (m, 1H), 1.45 (s, 9H).

### Step 3) the preparation of compound 11-4

To a solution of compound **11-3** (3.74 g, 15.4 mmol) in toluene (50 mL) was added lithium triethylborohydride (1.793 g, 16.9 mmol) dropwise at -78 °C. After the mixture was stirred at -78 °C for 70 mins, DIPEA (3.2 mL, 19.4 mmol), DMAP (0.1877 g, 1.54 mmol) and TFAA (3.0 mL, 40.4 mmol) were added separately, and the mixture was stirred at rt for 2.0 hrs. After the reaction was completed, the mixture was concentrated *in vacuo.* The residue was purified by silica gel column chromatography (PE/EtOAc (v/v) = 10/1) to give the title compound as yellow liquid (2.26 g, 64.8%). The compound was characterized by the following spectroscopic data:
MS (ESI, pos.ion) *m*/*z*: 128.2 [M-Boc]⁺;
¹H NMR (400 MHz, CDCl₃) *δ* (ppm): 6.65-6.52 (br, 1H), 4.96-4.91 (br, 1H), 4.68-4.57 (m, 1H), 3.76 (s, 3H), 3.12-3.00 (m, 1H), 2.71-2.61 (m, 1H), 1.49-1.44 (br, 9H).

### Step 4) the preparation of compound 11-5

To a solution of diethylzinc (0.4871 g, 3.94 mmol) in toluene (6.0 mL) was added chloroiodomethane (1.394 g, 7.9 mmol) dropwise at 0 °C. After the mixture was stirred at 0 °C for 45 mins, a solution of compound **11-4** (300 mg, 1.32 mmol) in toluene (4.0 mL) was added dropwise. At the end of the addition, the mixture was stirred at 0 °C for 18 hrs. After the reaction was completed, the mixture was quenched with saturated NH₄Cl aqueous solution (15 mL). The aqueous layer was extracted with EtOAc (25 mL x 3). The combined organic layers were dried over anhydrous Na₂SO₄ and concentrated *in vacuo.* The residue was purified by silica gel column chromatography (PE/EtOAc (v/v) = 10/1) to give the title compound as yellow liquid (0.19 g, 59.7%). The compound was characterized by the following spectroscopic data:
MS (ESI, pos.ion) *m*/*z*: 142.2 [M-Boc]⁺;
¹H NMR (400 MHz, CDCl₃) *δ* (ppm): 4.64 -4.51 (m, 1H), 3.70 (s, 3H), 3.56-3.45 (m, 1H), 2.64-2.54 (m, 1H), 2.05-2.01 (m, 1H), 1.50, 1.41 (s, s, 9H), 0.75-0.65 (m, 3H).

### Step 5) the preparation of compound 11-6

To a solution of compound **11-5** (1.02 g, 4.23 mmol) in THF (20 mL) was added lithium hydroxide monohydrate aqueous solution (0.8888 g, 21.2 mmol, 10 mL) dropwise at 0 °C. At the end of the addition, the mixture was stirred at 40 °C for 12 hrs. After the reaction was completed, the THF solvent was removed and 20 mL of water was added to the mixture. The resulting mixture was washed with EtOAc (15 mL x 3). The aqueous phase was adjusted to pH 1 with hydrochloric acid (10%) and extracted with EtOAc (25 mL x 3). The combined organic layers were washed with brine, dried over anhydrous Na₂SO₄ and concentrated *in vacuo* to give the title compound as a white solid (0.8371 g, 87%). The compound was characterized by the following spectroscopic data:
MS (ESI, neg.ion) *m*/*z*: 226.2 [M-H]⁻;
¹H NMR (400 MHz, CD₃OD) *δ* (ppm): 4.53-4.46 (m, 1H), 3.48-3.42 (m, 1H), 2.70-2.57 (m, 1H), 2.05-2.01 (m, 1H), 1.60-1.54 (m, 1H), 1.48, 1.41 (s, s, 9H), 0.89-0.80 (m, 1H), 0.73-0.66 (m, 1H).

### Step 6) the preparation of compound 11-7

To a solution of compound **11-6** (2.43 g, 10.7 mmol) and HATU (4.88 g, 12.84 mmol) in THF (25 mL) was added DIPEA (1.95 mL, 11.8 mmol) at 0 °C. After stirring at 0 °C for 0.5 hr, compound 1-9-2 (2.22 g, 11.9 mmol) was added in a portionwise manner. At the end of the addition, the reaction mixture was stirred at rt for 4.0 hrs. After the reaction was completed, the mixture was quenched with water (50 mL), and the THF solvent was removed. The resulting mixture was extracted with EtOAc (50 mL x 3). The combined organic layers were washed with brine, dried over anhydrous Na₂SO₄ and concentrated *in vacuo.* The residue was dissolved in glacial acetic acid (20 mL), and the solution was stirred at 40 °C overnight. After the reaction was completed, the solvent was removed under reduced pressure. The resulting mixture was dissolved in EtOAc (100 mL), washed with Na₂CO₃ aqueous solution (50 mL x 3), dried over anhydrous Na₂SO₄ and concentrated *in vacuo.* The residue was purified by silica gel column chromatography (PE/EtOAc (v/v) = 1/2) to give the title compound (2.02 g, 50%). The compound was characterized by the following spectroscopic data:
MS (ESI, pos.ion) *m*/*z*: 378.3 [M+H]⁺;
¹H NMR (400 MHz, CDCl₃) *δ* (ppm): 7.67 (dd, 1H), 7.22, 7.20 (d, d, 1H), 7.19, 7.17 (d, d, 1H), 5.03-5.00 (m, 1H), 3.31-3.24 (m, 1H), 2.56-2.49 (m, 1H), 2.12-2.07 (m, 1H), 1.53-1.48 (m, 1H), 1.46 (s, 9H), 1.42-1.38 (m, 1H), 1.00-0.97 (m, 1H).

### Step 7) the preparation of compound 11-8

To a solution of compound **11-7** (1.03 g, 2.74 mmol) in EtOAc (5.0 mL) was added a solution of HCl in EtOAc (6.0 mL, 4 M) dropwise at 0 °C. At the end the of addition, the mixture was stirred at rt for 8.0 hrs. After the reaction was completed, the mixture was *concentrated in vacuo.* The residue was purified by beating in EtOAc (10 mL) and filtered to give the title compound as a pale yellow solid (815.2 mg, 85%). The compound was characterized by the following spectroscopic data:
MS (ESI, pos.ion) *m*/*z*: 278.2 [M+H]⁺.

### Step 8) the preparation of compound 11-9

To a suspension of compound **11-8** (658 mg, 1.88 mmol), compound **1-11-2** (493.7 mg, 2.82 mmol) and EDCI (540 mg, 2.82 mmol) in DCM (10 mL) was added DIPEA (1.864 mL, 11.28 mmol) dropwise at 0 °C. At the end of the addition, the mixture was stirred at rt for 3.0 hrs. After the reaction was completed, the mixture was diluted with DCM (50 mL). The resulting mixture was washed with water (50 mL x 3) and brine, dried over anhydrous Na₂SO₄ and concentrated *in vacuo.* The residue was purified by silica gel column chromatography (PE/EtOAc (v/v) = 1/2) to give the title compound as a solid (694 mg, 85%). The compound was characterized by the following spectroscopic data:
MS (ESI, pos.ion) *m*/*z:* 435.5 [M+H]⁺;
¹H NMR (400 MHz, CDCl₃) *δ* (ppm): 7.67 (dd, 1 H), 7.22, 7.20 (d, d, 1H), 7.19, 7.17 (d, d, 1H), 5.32, 5.30 (d, d, 1H), 5.16-5.12 (m, 1H), 4.13-4.08 (m, 1H), 3.63 (s, 3H), 3.42-3.36 (m, 1H), 2.62-2.55 (m,1H), 2.21-2.09 (m, 2H), 1.53-1.45 (m, 1H), 0.97-0.89 (m, 7H), 0.50-0.46 (m, 1H).

### Step 9) the preparation of compound 11-10

To a mixture of compound **11-9** (3.08 g, 7.1 mmol), compound **1-12-2** (2.72 g, 10.7 mmol), Pd(dppf)Cl₂·CH₂Cl₂ (653 mg, 0.8 mmol) and KOAc (2.09 g, 21.3 mmol) was added DMF (30 mL) via syringe under N₂, and the mixture was stirred at 90 °C for 3.0 hrs. After the reaction was completed, the mixture was cooled to rt and diluted with EtOAc (200 mL). The resulting mixture was filtered through a celite pad. The filtrate was washed with water (50 mL x 3) and brine, dried over anhydrous Na₂SO₄ and concentrated *in vacuo.* The residue was purified by silica gel column chromatography (PE/EtOAc (v/v) = 1/2) to give the title compound as a beige solid (2.225 g, 65%). The compound was characterized by the following spectroscopic data:
MS (ESI, pos.ion) *m*/*z*: 483.5 [M+H]⁺;
¹H NMR (400 MHz, CDCl₃) *δ* (ppm): 7.82 (dd, 1H), 7.65, 7.63 (d, d, 1H), 7.45, 7.42 (d, d, 1H), 5.32, 5.30 (d, d, 1H), 5.16-5.12 (m, 1H), 4.13-4.08 (m, 1H), 3.63 (s, 3H), 3.42-3.36 (m, 1H), 2.62-2.55 (m, 1H), 2.22-2.09 (m, 2H), 1.53-1.45 (m, 1H), 1.32, 1.29 (m, 12H), 0.97-0.89 (m, 7H), 0.50-0.46 (m, 1H).

### Step 10) the preparation of compound 11-11

A mixture of compound **3-3** (232.93 mg, 0.522 mmol), compound **6-3** (284.87 mg, 0.574 mmol), Pd(PPh₃)₄ (60.29 mg, 0.0522 mmol) and K₂CO₃ (216 mg, 1.566 mmol) in mixed solvents of DME and H₂O (8 mL, v/v = 3/1) was stirred at 90 °C under N₂ for 3.0 hrs, cooled to rt and diluted with EtOAc (40 mL). The resulting mixture was washed with water (20 mL x 3) and brine, dried over anhydrous Na₂SO₄ and concentrated *in vacuo.* The residue was purified by silica gel column chromatography (DCM/EtOH (v/v) = 150/1) to give the title compound as a pale yellow solid (179.63 mg, 50%). The compound was characterized by the following spectroscopic data:
MS (ESI, pos.ion) *m*/*z*: 689.5 [M+H]⁺;
¹H NMR (400 MHz, CDCl₃) *δ* (ppm): 7.65-7.62 (m, 2H), 7.59 (s, 1H), 7.46-7.43 (m, 2H), 7.25-7.23 (d, d, 1H), 7.09, 7.07 (s, s, 1H), 5.56, 5.55 (d, d, 1H), 5.23-5.19 (m, 1H), 4.34-4.30 (m, 1H), 3.85-3.78 (m, 1H), 3.66 (s, 3H), 3.65-3.61 (m, 1H), 2.83-2.79 (m, 2H), 2.44-2.36 (m, 2H), 2.31-1.92 (m, 9H), 1.75-1.57 (m, 4H), 1.02, 1.00 (m, m, 3H), 0.93, 0.91 (m, m, 3H).

### Step 11) the preparation of compound 11-12

A suspension of compound **11-11** (359.26 mg, 0.522 mmol), compound **11-10** (276.82 mg, 0.574 mmol), Pd(PPh₃)₄ (60.29 mg, 0.0522 mmol) and K₂CO₃ (216 mg, 1.566 mmol) in mixed solvents of DME and H₂O (8 mL, v/v = 3/1) was stirred at 90 °C under N₂ for 3.0 hrs. After the reaction was cooled to rt, the mixture was diluted with EtOAc (40 mL). The resulting mixture was washed with water (20 mL x 3) and brine, dried over anhydrous Na₂SO₄ and concentrated *in vacuo.* The residue was purified by silica gel column chromatography (DCM/EtOH (v/v) = 50/1) to give the title compound as a pale yellow solid (256.8 mg, 55%). The compound was characterized by the following spectroscopic data:
MS (ESI, pos.ion) *m*/*z*: 448.3 [M+2H]²⁺;
¹H NMR (400 MHz, CDCl₃) *δ* (ppm): 7.90, 7.88 (d, d, 1H), 7.68, 7.66 (s, s, 1H), 7.65-7.62 (m, 2H), 7.60-7.59 (m, 2H), 7.50-7.46 (m, 3H), 7.45, 7.43 (d, d, 1H), 5.56-5.55 (d, d, 1H), 5.32, 5.30 (d, d, 1H), 5.23-5.19 (m, 1H), 5.14-5.10 (m, 1H), 4.34-4.30 (m, 1H), 4.13-4.08 (m, 1H), 3.85-3.78 (m, 1H), 3.68-3.67 (m, 1H), 3.66 (s, 3H), 3.63 (s, 3H), 3.42-3.36 (m, 1H), 2.84-2.80 (m, 2H), 2.62-2.55 (m, 1H), 2.44-2.35 (m, 2H), 2.30-1.92 (m, 11H), 1.76-1.58 (m, 4H), 1.53-1.45 (m, 1H), 1.02-0.89 (m, 13H), 0.50-0.46 (m, 1H).

### Example 12

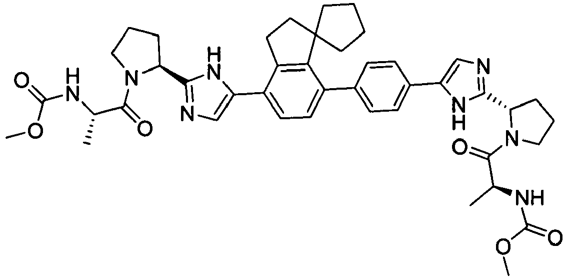

### Synthetic route:

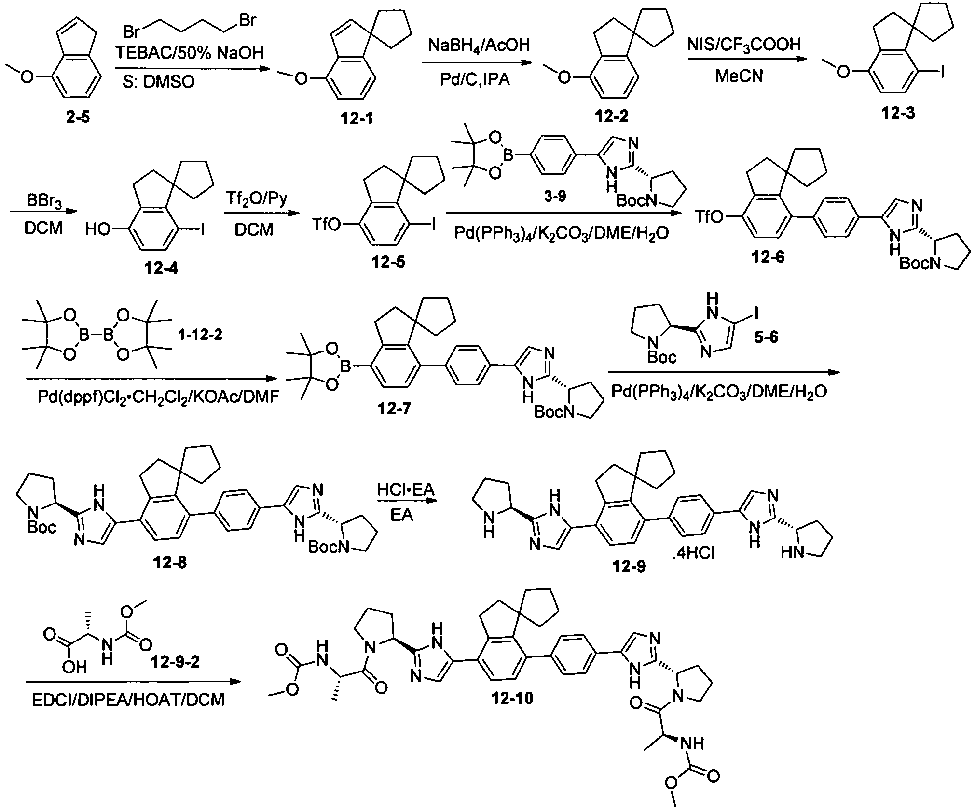

### Step 1) the preparation of compound 12-1

To a solution of compound **2-5** (12.4 g, 84.8 mmol), 1,4-dibromobutane (19.2 g, 89.1 mmol) and TEBAC (3.9 g, 17.0 mmol) in DMSO (60 mL) was added sodium hydroxide aqueous solution (50%, 13.6 g, 339.3 mmol) dropwise at 0 °C. At the end of the addition, the mixture was stirred at 50 °C for 2.0 hrs. After the reaction was completed, PE (500 mL) was added to the mixture. The resulting mixture was washed with water (100 mL x 3) and brine, dried over anhydrous Na₂SO₄ and concentrated *in vacuo.* The residue was purified by silica gel column chromatography (PE) to give the title compound as colorless liquid (6.0 g, 35%). The compound was characterized by the following spectroscopic data:
MS (ESI, pos.ion) *m*/*z*: 201.2 [M+H]⁺;
¹H NMR (400 MHz, CDCl₃) *δ* (ppm): 6.90-6.85 (m, 1H), 6.70-6.67 (m, 1H), 6.62-6.59 (m, 1H), 3.82 (s, 3H), 2.86-2.81 (m, 2H), 2.30-2.22 (m, 2H), 2.16-2.10 (m, 2H), 1.88-1.79 (m, 2H), 1.75-1.58 (m, 4H).

### Step 2) the preparation of compound 12-2

To a solution of compound **12-1** (4.0 g, 20.0 mmol) in isopropanol (15 mL) were added a catalytic amount of Pd/C (400 mg), glacial acetic acid (2.4 g, 40.0 mmol) and Na BH₄ (3.0 g, 80.0 mmol) separately. At the end of the addition, the mixture was stirred at rt for 1.0 hr. After the reaction was completed, the mixture was filtered. The filtrate was concentrated *in vacuo.* The residue was purified by silica gel column chromatography (PE) to give the title compound as colorless liquid (3.9 g, 97%). The compound was characterized by the following spectroscopic data:
MS (ESI, pos.ion) *m*/*z*: 203.1 [M+H]⁺;
¹H NMR (400 MHz, CDCl₃) *δ* (ppm): 6.85-6.80 (m, 2H), 6.65-6.62 (m, 1H), 6.56-6.53 (m, 1H), 6.45, 6.43 (m, m, 1H), 3.80 (s, 3H), 2.14-2.01 (m, 2H), 1.83-1.63 (m, 4H), 1.47-1.35 (m, 2H).

### Step 3) the preparation of compound 12-3

To a mixture of compound **12-2** (5.3 g, 26.2 mmol) and NIS (6.5 g, 28.8 mmol) in MeCN (60 mL) was added CF₃COOH (0.59 mL, 7.9 mmol) dropwise at 0 °C. At the end of the addition, the mixture was stirred at 45 °C overnight. After the reaction was completed, the mixture was concentrated *in vacuo.* To the residue was added EtOAc (200 mL). The resulting mixture was washed with saturated sodium sulfite aqueous solution (30 mL x 2), dried over anhydrous Na₂SO₄ and concentrated *in vacuo.* The residue was purified by silica gel column chromatography (PE) to give the title compound as colorless liquid (8.6 g, 100%). The compound was characterized by the following spectroscopic data:
MS (ESI, pos.ion) *m*/*z*: 328.1 [M+H]⁺;
¹H NMR (400 MHz, CDCl₃) *δ* (ppm): 7.48, 7.46 (dd, dd, 1H), 6.44, 6.41 (m, m, 1H), 3.83 (s, 3H), 2.87-2.83 (m, 2H), 2.47-2.39 (m, 2H), 2.34-2.23 (m, 4H), 1.75-1.58 (m, 4H).

### Step 4) the preparation of compound 12-4

To a solution of compound **12-3** (1.55 g, 4.72 mmol) in DCM (10 mL) was added boron tribromide (1.79 mL, 18.89 mmol) dropwise at -78 °C. At the end of the addition, the mixture was stirred at -78 °C for 10 mins and at rt for another 1.0 hr. After the reaction was completed, the mixture was quenched with ice water (25 mL) and the organic phase was separated. The aqueous layer was extracted with DCM (30 mL x 3). The combined organic layers were washed with brine, dried over anhydrous Na₂SO₄ and concentrated *in vacuo.* The residue was purified by silica gel column chromatography (PE/EtOAc (v/v) = 40/1) to give the title compound as colorless oil (1.36 g, 92%). The compound was characterized by the following spectroscopic data:
¹H NMR (400 MHz, CDCl₃) *δ* (ppm): 7.39, 7.37 (dd, dd, 1H), 6.34, 6.32 (s, s, 1H), 4.81 (br, 1H), 2.81-2.77 (m, 2H), 2.41-2.26 (m, 4H), 1.98-1.90 (m, 2H), 1.74-1.58 (m, 4H).

### Step 5) the preparation of compound 12-5

To a solution of compound **12-4** (1.33 g, 4.24 mmol) in DCM (10 mL) was added pyridine (0.85 mL, 10.58 mmol) dropwise at 0 °C. After the mixture was stirred for 10 mins, trifluoromethanesulfonic anhydride (1.07 mL, 6.36 mmol) was added. At the end of the addition, the mixture was stirred at rt for 1.0 hr. After the reaction was completed, the mixture was quenched with ice water (25 mL). The aqueous layer was extracted with DCM (30 mL x 3). The combined organic layers were washed with brine, dried over anhydrous Na₂SO₄ and concentrated *in vacuo.* The residue was purified by silica gel column chromatography (PE) to give the title compound as white oil (1.8 g, 95%). The compound was characterized by the following spectroscopic data:
¹H NMR (400 MHz, CDCl₃) *δ* (ppm): 7.60, 7.58 (dd, dd, 1H), 6.87, 6.85 (s, s, 1H), 3.01-2.97 (m, 2H), 2.42-2.34 (m, 2H), 2.31-2.25 (m, 2H), 2.00-1.91 (m, 2H), 1.75-1.58 (m, 4H).

### Step 6) the preparation of compound 12-6

A suspension of compound **12-5** (4.10 g, 9.19 mmol), compound **3-9** (4.04 g, 9.19 mmol), Pd(PPh₃)₄ (849 mg, 0.08 mmol) and K₂CO₃ (3.17 g, 22.97 mmol) in mixed solvents of DME and H₂O (24.0 mL, v/v = 5/1) was stirred at 90 °C under N₂ for 3.0 hrs. After the reaction was completed, the mixture was diluted with EtOAc (200 mL). The resulting mixture was washed with water (50 mL x 3) and brine, dried over anhydrous Na₂SO₄ and concentrated *in vacuo.* The residue was purified by silica gel column chromatography (PE/EtOAc (v/v) = 2/1) to give the title compound as a pale yellow solid (1.46 g, 50%). The compound was characterized by the following spectroscopic data:
MS (ESI, pos.ion) *m*/*z*: 632.3 [M+H]⁺;
¹H NMR (400 MHz, CDCl₃) *δ* (ppm): 7.59 (s, 1H), 7.54-7.51 (m, 2 H), 7.45-7.42 (m, 2H), 7.27-7.25 (s, s, 1H), 6.97-6.95 (dd, dd, 1H), 4.97-4.92 (m, 1H), 3.85-3.78 (m, 1H), 3.60-3.52 (m, 1H), 3.00-2.96 (m, 2H), 2.80-2.72 (m, 1H), 2.42-2.34 (m, 2H), 2.29-2.12 (m, 5H), 1.94-1.83 (m, 1H), 1.76-1.59 (m, 5H), 1.43 (s, 9H).

### Step 7) the preparation of compound 12-7

To a mixture of compound **12-6** (800 mg, 1.27 mmol), compound **1-12-2** (354 mg, 1.39 mmol), Pd(dppf)Cl₂CH₂Cl₂ (103 mg, 0.13 mmol) and KOAc (311 mg, 3.17 mmol) was added DMF (5.0 mL) via syringe under N₂, and the mixture was stirred at 90 °C for 3.0 hrs. After the reaction was completed, the mixture was cooled to rt, diluted with EtOAc (50 mL) and filtered through a celite pad. The filtrate was washed with water (30 mL x 3) and brine, dried over anhydrous Na₂SO₄ and concentrated *in vacuo.* The residue was purified by silica gel column chromatography (PE/EtOAc (v/v) = 3/1) to give the title compound as a pale yellow solid (603.9 mg, 78%). The compound was characterized by the following spectroscopic data:
MS (ESI, pos.ion) *m*/*z:* 610.5 [M+H]⁺;
¹H NMR (400 MHz, CDCl₃) *δ* (ppm): 7.59-7.57 (m, 2H), 7.54-7.51 (m, 2H), 7.50-7.46 (m, 2H), 7.46, 7.44 (s, s, 1H), 4.98-4.92 (m, 1H), 3.64-3.58 (m, 1H), 3.31-3.23 (m, 1H), 2.94-2.90 (m, 2H), 2.47-1.90 (m, 10H), 1.77-1.60 (m, 4H), 1.41 (s, 9H), 1.32 (m, 6H), 1.29 (m, 6H).

### Step 8) the preparation of compound 12-8

A suspension of compound **12-7** (609 mg, 1.0 mmol), compound **5-6** (370 mg, 1.02 mmol), Pd(PPh₃)₄ (116 mg, 0.1 mmol) and K₂CO₃ (804 mg, 5.82 mmol) in mixed solvents of EtOH and H₂O (12 mL, v/v = 5/1) was stirred at 90°C under N₂ for 3.0 hrs. After the reaction was cooled to rt, the mixture was diluted with water (20 mL). The aqueous layer was extracted with EtOAc (30 mL x 3). The combined organic layers were washed with brine, dried over anhydrous Na₂SO₄ and concentrated *in vacuo.* The residue was purified by silica gel column chromatography (DCM/MeOH (v/v) = 100/1) to give the title compound as pale yellow foam (251 mg, 35%). The compound was characterized by the following spectroscopic data:
MS (ESI, pos.ion) *m*/*z*: 719.3 [M+H]⁺;
¹H NMR (400 MHz, CDCl₃) *δ* (ppm): 7.78 (s, 1H), 7.59 (s, 1H), 7.54-7.51 (m, 3H), 7.49-7.46 (m, 2H), 7.18, 7.16 (s, s, 1H), 5.14-5.08 (m, 1H), 4.98-4.92 (m, 1H), 3.64-3.58 (m, 2H), 3.31-3.24 (m, 2H), 2.85-2.81 (m, 2H), 2.47-2.38 (m, 2H), 2.37-1.86 (m, 12H), 1.76-1.58 (m, 4H), 1.53 (s, 9H), 1.41 (s, 9H).

### Step 9) the preparation of compound 12-9

To a solution of compound **12-8** (166 mg, 0.35 mmol) in EtOAc (5.0 mL) was added a solution of HCl in EtOAc (3.0 mL, 4 M) dropwise. At the end of the addition, the mixture was stirred at rt for 8.0 hrs. After the reaction was completed, the mixture was concentrated *in vacuo.* The residue was purified by beating in EtOAc (10 mL) and filtered to give the title compound as pale yellow powder (120.3 mg, 72%). The compound was characterized by the following spectroscopic data:
MS (ESI, pos.ion) *m*/*z*: 777.5 [M+H]⁺.

### Step 10) the preparation of compound 12-10

To a suspension of compound **12-9** (168 mg, 0.25 mmol), compound **12-9-2** (77.94 mg, 0.53 mmol), EDCI (102 mg, 0.53 mmol) and HOAT (69 mg, 0.51 mmol) in DCM (20.0 mL) was added DIPEA (0.352 mL, 2.02 mmol) dropwise at 0 °C. At the end of the addition, the mixture was stirred at rt for 3.0 hrs. After the reaction was completed, the mixture was diluted with DCM (20 mL), washed with saturated NH₄Cl aqueous solution and brine, dried over anhydrous Na₂SO₄ and concentrated *in vacuo.* The residue was purified by silica gel column chromatography (DCM/MeOH (v/v) = 60/1) to give the title compound as a white solid (130 mg, 62%). The compound was characterized by the following spectroscopic data:
MS (ESI, pos.ion) *m*/*z*: 776.4 [M+H]⁺;
¹H NMR (400 MHz, CDCl₃) *δ* (ppm): 7.71 (s, 1H), 7.59 (s, 1H), 7.54-7.51 (m, 3H), 7.49-7.46 (m, 2H), 7.18, 7.16 (s, s, 1H), 5.64, 5.61 (m, m, 1H), 5.34, 5.32 (m, m, 1H), 5.18-5.13 (m, 1H), 5.10-5.05 (m, 1H), 4.67-4.54 (m, 2H), 3.88-3.82 (m, 2H), 3.72-3.65 (m, 2H), 3.64 (s, 6H), 2.85-2.81 (m, 2H), 2.37-1.86 (m, 14H), 1.76-1.58 (m, 4H), 1.48, 1.46 (m, m, 3H), 1.40, 1.38 (m, m, 3H).

### Example 13

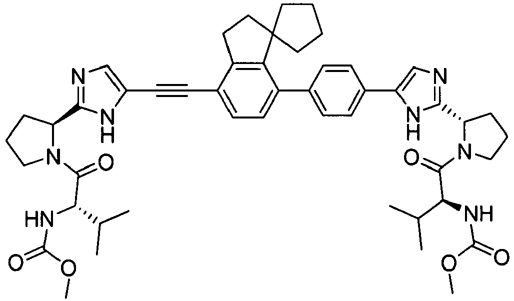

### Synthetic route:

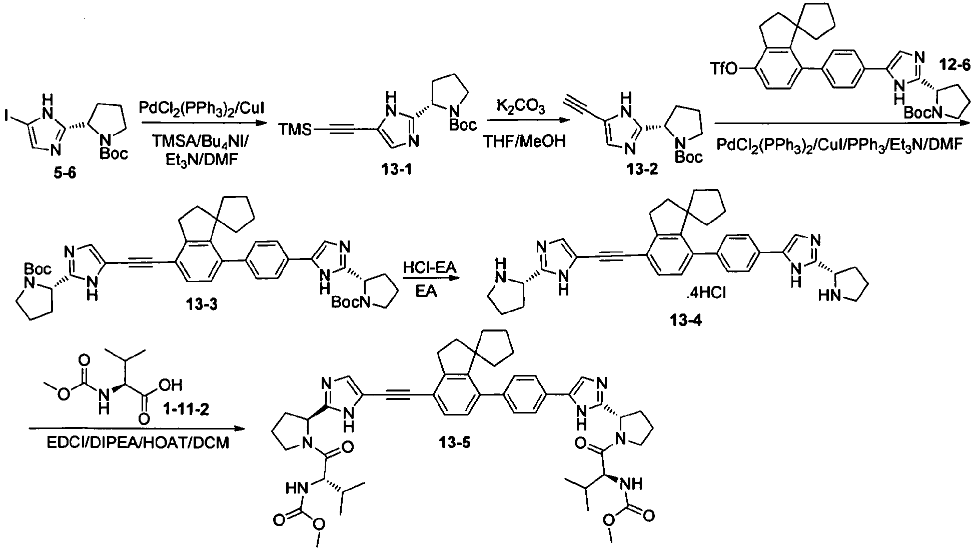

### Step 1) the preparation of compound 13-1

To a mixture of compound **5-6** (501 mg, 1.38 mmol), PdCl₂(PPh₃)₂ (98 mg, 0.14 mmol), tetrabutylammonium iodide (1.53 g, 4.14 mmol) and CuI (78 mg, 0.41 mmol) in DMF (8.0 mL) was added Et₃N (2.0 mL) dropwise under N₂. After the mixture was stirred at rt for 10 mins, TMSA (0.98 mL, 6.89 mmol) was added. At the end of the addition, the reaction mixture was stirred at rt for 10 mins and at 70 °C overnight. After the reaction was completed, the mixture was filtered through a celite pad. To the filtrate was added water (30 mL) and the mixture was extracted with EtOAc (20 mL x 3). The combined organic layers were washed with brine, dried over anhydrous Na₂SO₄ and concentrated *in vacuo.* The residue was purified by silica gel column chromatography (PE/EtOAc (v/v) = 5/1) to give the title compound (290 mg, 63%). The compound was characterized by the following spectroscopic data:
MS (ESI, pos.ion) *m*/*z*: 334.2 [M+H]⁺;
¹H NMR (400 MHz, CDCl₃) *δ* (ppm): 7.24 (s, 1H), 4.95-4.90 (m, 1H), 3.72-3.66 (m, 1H), 3.38-3.30 (m, 1H), 2.56-2.48 (m, 1H), 2.40-2.30 (m, 1H), 2.38-2.19 (m, 1H), 2.07-1.97 (m, 1H), 1.41 (s, 9H), 0.32 (s, 9H).

### Step 2) the preparation of compound 13-2

A mixture of compound **13-1** (290 mg, 0.87 mmol) and K₂CO₃ (601 mg, 4.35 mmol) in mixed solvents of MeOH (2.0 mL) and THF (2.0 mL) was stirred at rt for 6.0 hrs. After the reaction was completed, the mixture was diluted with EtOAc (20 mL) and filtered. The filtrate was concentrated *in vacuo*, and the residue was purified by silica gel column chromatography (PE/EtOAc (v/v) = 2/1) to give the title compound (208 mg, 91%). The compound was characterized by the following spectroscopic data:
MS (ESI, pos.ion) *m*/*z*: 262.2 [M+H]⁺;
¹H NMR (400 MHz, CDCl₃) *δ* (ppm): 7.25 (s, 1H), 4.99-4.94 (m, 1H), 3.72-3.66 (m, 1H), 3.36 (s, 1H), 3.35-3.30 (m, 1H), 2.56-2.48 (m, 1H), 2.40-2.30 (m, 1H), 2.28-2.19 (m, 1H), 2.07-1.97 (m, 1H), 1.41 (s, 9H).

### Step 3) the preparation of compound 13-3

To a mixture of compound **12-6** (246.18 mg, 0.39 mmol), compound **13-2** (113 mg, 0.43 mmol), CuI (33 mg, 0.172 mmol), PdCl₂(PPh₃)₂ (14.1 mg, 0.02 mmol) and PPh₃ (226 mg, 0.86 mmol) were added anhydrous DMF (10.0 mL) and Et₃N (5.0 mL) separately under N₂. At the end of the addition, the mixture was stirred at rt for 10 mins and at 90 °C for another 6.0 hrs. After the reaction was completed, the mixture was diluted with EtOAc (50 mL) and filtered through a celite pad. The filtrate was washed with water (20 mL x 3) and brine, dried over anhydrous Na₂SO₄ and concentrated *in vacuo.* The residue was purified by silica gel column chromatography (PE/EtOAc (v/v) = 1/2) to give the title compound (159 mg, 55%). The compound was characterized by the following spectroscopic data:
MS (ESI, pos.ion) *m*/*z*: 743.5 [M+H]⁺;
¹H NMR (400 MHz, CDCl₃) *δ* (ppm): 7.59 (s, 1H), 7.57-7.54 (m, 2H), 7.53-7.50 (m, 3H), 7.48, 7.45 (m, m, 1H), 7.44, 7.42 (s, s, 1H), 5.18-5.14 (m, 1H), 4.98-4.92 (m, 1H), 3.72-3.66 (m, 1H), 3.64-3.58 (m, 1H), 3.38-3.24 (m, 2H), 2.95-2.90 (m, 2H), 2.56-1.97 (m, 14H), 1.78-1.61 (m, 4H), 1.53 (s, 9H), 1.41 (s, 9H).

### Step 4) the preparation of compound 13-4

To a solution of compound **13-3** (170.76 mg, 0.23 mmol) in EtOAc (2.0 mL) was added a solution of HCl in EtOAc (2.0 mL, 4 M) dropwise. At the end of the addition, the mixture was stirred at rt for 8.0 hrs. After the reaction was completed, the mixture was concentrated *in vacuo.* The residue was purified by beating in EtOAc (5.0 mL) and filtered to give the title compound as a white solid (143 mg, 90%). The compound was characterized by the following spectroscopic data:
MS (ESI, pos.ion) *m*/*z*: 543.5 [M+H]⁺.

### Step 5) the preparation of compound 13-5

A suspension of compound **13-4** (206.5 mg, 0.3 mmol), compound **1-11-2** (110.3 mg, 0.63 mmol), HOAT (81.67 mg, 0.6 mmol) and EDCI (120.8 mg, 0.63 mmol) in DCM (5.0 mL) was added DIPEA (0.397 mL, 2.4 mmol) dropwise at 0 °C. At the end of the addition, the mixture was stirred at rt for 3.0 hrs. After the reaction was completed, the mixture was diluted with DCM (20 mL). The resulting mixture was washed with saturated NH₄Cl aqueous solution, dried over anhydrous Na₂SO₄ and concentrated *in vacuo.* The residue was purified by silica gel column chromatography (DCM/MeOH (v/v) = 60/1) to give the title compound as a white solid (167 mg, 65%). The compound was characterized by the following spectroscopic data:
MS (ESI, pos.ion) *m*/*z*: 429.4 [M+2H]²⁺;
¹H NMR (400 MHz, CDCl₃) *δ* (ppm): 7.59 (s, 1H), 7.57-7.54 (m, 2H), 7.53-7.50 (m, 2H), 7.48 (s, 1H), 7.47, 7.45 (dd, dd, 1H), 7.44, 7.42 (s, s, 1H), 5.56, 5.55 (d, d, 1H), 5.51-5.47 (m, 1H), 5.46, 5.44 (d, d, 1H), 5.23-5.19 (m, 1H), 4.41-4.30 (m, 2H), 3.89-3.78 (m, 2H), 3.66 (s, 6H), 3.73-3.67 (m, 2H), 2.95-2.90 (m, 2H), 2.42-1.92 (m, 16H), 1.78-1.61 (m, 4H), 1.02-0.89 (m, 12H).

### Example 14

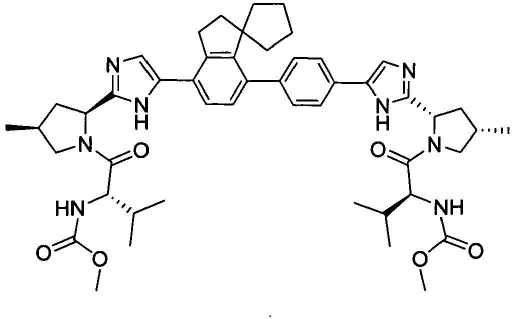

### Synthetic route:

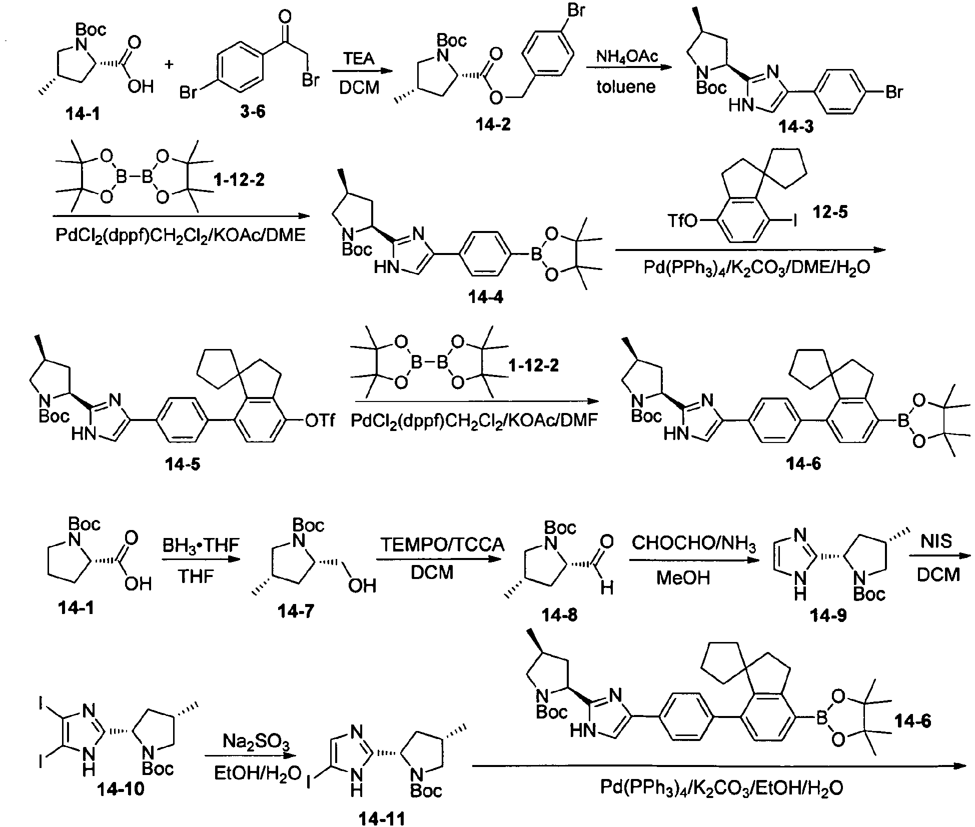

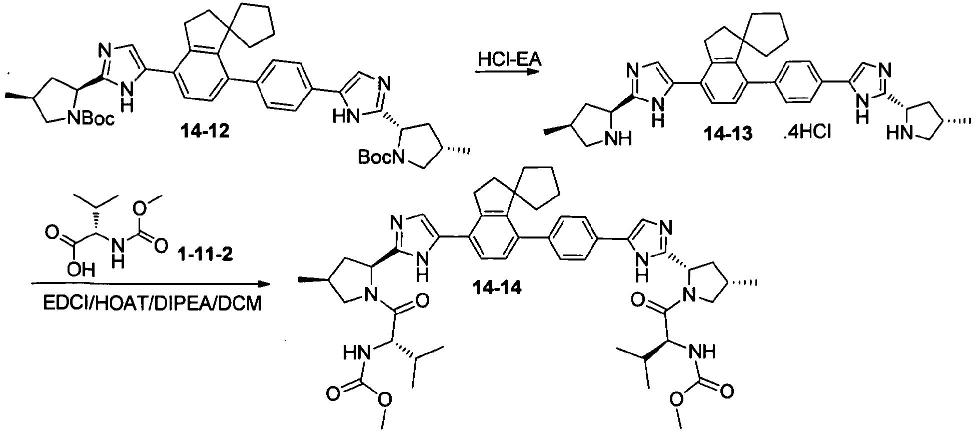

### Step 1) the preparation of compound 14-2

To a solution of compound **14-1** (3.0 g, 13.1 mmol) and compound **3-6** (3.63 g, 13.1 mmol) in DCM (40 mL) was added TEA (3.9 mL, 26.3 mmol) dropwise at 0 °C. At the end of the addition, the mixture was stirred at rt for 2.0 hrs. After the reaction was completed, the mixture was quenched with water (50 mL). The aqueous layer was extracted with DCM (50 mL x 3). The combined organic layers were washed with brine, dried over anhydrous Na₂SO₄ and concentrated *in vacuo* to give the crude product (3.27 g), which was used for the next step without further purification. The compound was characterized by the following spectroscopic data:
MS (ESI, pos.ion) *m*/*z*: 399.29 [M+H]⁺.

### Step 2) the preparation of compound 14-3

A mixture of compound **14-2** (3.27 g, 8.2 mmol) and ammonium acetate (5.1 g, 66 mmol) in toluene (30 mL) was stirred at 110 °C for 5.0 hrs. After the reaction was cooled to rt, the mixture was quenched with water (50 mL). The aqueous layer was extracted with EtOAc (80 mL x 3). The combined organic layers were washed with brine, dried over anhydrous Na₂SO₄ and concentrated *in vacuo.* The residue was purified by silica gel column chromatography (PE/EtOAc (v/v) = 4/l) to give the title compound (2.8 g, 86%). The compound was characterized by the following spectroscopic data:
MS (ESI, pos.ion) *m*/*z*: 407.32 [M+H]⁺;
¹H NMR (400 MHz, CDCl₃) *δ* (ppm): 7.45 (m, 4H), 7.20 (s, 1H), 4.93 (t, 1H, *J* = 8.2 Hz), 3.88-3.66 (m, 1H), 2.90 (t, 1H, *J* = 8.0 Hz), 2.50-2.47 (m, 2H), 2.27-2.25 (m, 1H), 1.48 (s, 7H), 1.26 (s, 2H), 1.12 (d, 3H, *J* = 6.2 Hz).

### Step 3) the preparation of compound 14-4

A mixture of compound **14-3** (2.8 g, 6.9 mmol), compound **1-12-2** (1.93 g, 7.6 mmol), Pd(dppf)Cl₂CH₂Cl₂ (0.28 g, 0.34 mmol) and KOAc (1.7 g, 17.25 mmol) in DME (30 mL) was stirred at 90 °C under N₂ for 2.0 hrs. After the reaction was completed, the mixture was cooled to rt, diluted with EtOAc (200 mL) and filtered through a celite pad. The filtrate was washed with water (20 mL x 3) and brine, dried over anhydrous Na₂SO₄ and concentrated *in vacuo.* The residue was purified by silica gel column chromatography (PE/EtOAc (v/v) = 1/2) to give the title compound as a pale yellow solid (3.0 g, 88.2%). The compound was characterized by the following spectroscopic data:
MS (ESI, pos.ion) *m*/*z*: 454.38 [M+H]⁺;
¹H NMR (400 MHz, CDCl₃ *δ* (ppm): 7.35 (m, 4H), 7.10 (s, 1H), 4.93 (t, 1H, *J* = 8.2 Hz), 3.88-3.66 (m, 1H), 2.90 (t, 1H, *J* = 8.0 Hz), 2.50-2.47 (m, 2H), 2.27-2.25 (m, 1H), 1.48 (s, 9H), 1.26 (s, 12H), 1.02 (d, 3H, *J* = 6.2 Hz).

### Step 4) the preparation of compound 14-5

To a mixture of compound **14-4** (3.4 g, 7.7 mmol), compound **12-5** (3.43 g, 7.7 mmol), Pd(PPh₃)₄ (450 mg, 0.38 mmol) and K₂CO₃ (2.1 g, 15.4 mmol) were added DME (32 mL) and pure water (8.0 mL) via syringe. The mixture was stirred at 90 °C for 3.0 hrs. After the reaction was completed, the mixture was cooled to rt and diluted with EtOAc (250 mL). The resulting mixture was washed with water (50 mL x 3) and brine, dried over anhydrous Na₂SO₄ and concentrated *in vacuo.* The residue was purified by silica gel column chromatography (PE/EtOAc (v/v) = 2/1) to give the title compound as a pale yellow solid (2.98 g, 60%). The compound was characterized by the following spectroscopic data:
MS (ESI, pos.ion) *m*/*z*: 646.5 [M+H]⁺;
¹H NMR (400 MHz, CDCl₃) *δ* (ppm): 7.67-7.61 (m, 4H), 7.36 (s, 1H), 7.27, 7.25 (s, s, 1H), 6.97, 6.95 (dd, dd, 1H), 4.81-4.76 (m, 1H), 3.80-3.73 (m, 1H), 3.09-3.02 (m, 1H), 3.00-2.96 (m, 2H), 2.42-2.18 (m, 9H), 1.76-1.59 (m, 4H), 1.41 (s, 9H), 0.96-0.93 (s, 3H).

### Step 5) the preparation of compound 14-6

A mixture of compound **14-5** (1.01 g, 1.62 mmol), compound **1-12-2** (0.42 g, 1.7 mmol), PdCl₂(dppf)CH₂Cl₂ (67 mg, 0.08 mmol) and KOAc (0.4 g, 4.05 mmol) in DMF (10 mL) was stirred at 90 °C under N₂ for 3.0 hrs. After the reaction was completed, the mixture was cooled to rt, diluted with EtOAc (50 mL) and filtered through a celite pad. The filtrate was washed with water (30 mL x 3) and brine, dried over anhydrous Na₂SO₄ and concentrated *in vacuo.* The residue was purified by silica gel column chromatography (PE/EtOAc (v/v) = 3/1) to give the title compound as a pale yellow solid (706.9 mg, 70%). The compound was characterized by the following spectroscopic data:
MS (ESI, pos.ion) *m*/*z*: 624.5 [M+H]⁺;
¹H NMR (400 MHz, CDCl₃) *δ* (ppm): 7.71-7.68 (m, 2H), 7.65-7.61 (m, 2H), 7.59, 7.57 (dd, dd, 1H), 7.46, 7.44 (s, s, 1H), 7.36 (s, 1H), 4.81-4.76 (m, 1H), 3.80-3.73 (m, 1H), 3.09-3.02 (m, 1H), 2.94-2.90 (m, 2H), 2.41-2.18 (m, 7H), 1.99-1.90 (m, 2H), 1.77-1.60 (m, 4H), 1.41 (s, 9H), 1.32, 1.29 (m, m, 12H), 0.96-0.93 (m, 3H).

### Step 6) the preparation of compound 14-7

To a solution of compound **14-1** (1.12 g, 4.88 mmol) in THF (10 mL) was added borane (7.3 mL, 1 M in THF) dropwise at 0 °C. At the end of the addition, the mixture was stirred at rt for 2.0 hrs. After the reaction was completed, the mixture was quenched with MeOH (4.0 mL). The THF solvent was removed, and the residue was dissolved with DCM (50 mL). The mixture was washed with (20 mL x 3) and brine, dried over anhydrous Na₂SO₄ and concentrated *in vacuo.* The residue was purified by silica gel column chromatography (PE/EtOAc (v/v) = 3/1) to give the title compound as colorless slurry (1.03 g, 100%). The compound was characterized by the following spectroscopic data:
MS (ESI, pos.ion) *m*/*z*: 216.29 [M+H]⁺;
¹H NMR (400 MHz, CDCl₃) *δ* (ppm): 4.02 (s, 1H), 3.99-3.87 (m, 1H), 3.75-3.68 (m, 1H), 3.66 (dd, 1H*, J* = 11.6 Hz, 2.0 Hz), 3.57 (dd, 1H, *J* = 11.6 Hz, 7.4 Hz), 2.76 (t, 1H, *J* = 10.5 Hz), 2.19-2.06 (m, 2H), 1.46 (s, 9H), 1.01 (d, 3H, *J* = 6.2 Hz).

### Step 7) the preparation of compound 14-9

To a solution of compound **14-7** (1.0 g, 4.64 mmol) in DCM (12 mL) was added TCCA (1.08 g, 4.64 mmol) dropwise at 0 °C followed by a solution of TEMPO in DCM (64 mg, 0.46 mmol, 5.0 mL). At the end of the addition, the mixture was stirred at 0 °C for 1.0 hr and at rt for another 1.0 hr. After the reaction was completed, the mixture was filtered, and the filtrate was washed with saturated Na₂SO₃ aqueous solution (30 mL x 3). The combined organic layers were dried over anhydrous Na₂SO₄ and concentrated *in vacuo* to give compound 14-8 as colorless slurry.

Compound **14-8** was dissolved in a solution of NH₃ in MeOH (10 mL, 7 M). The solution was stirred at 0 °C for 0.5 hr and at rt for another 1.0 hr. To the mixture was added glyoxal (1.2 mL, 40%) dropwise at 0 °C, and solid precipitated. At the end of the addition, the mixture was stirred at rt for 24 hrs and concentrated *in vacuo.* The residue was dissolved in DCM (100 mL). The solution was washed with water (30 mL x 3) and brine, dried over anhydrous Na₂SO₄ and concentrated *in vacuo.* The residue was purified by silica gel column chromatography (DCM/MeOH (v/v) = 100/1) to give the title compound as a pale yellow solid (0.51 g, 44%). The compound was characterized by the following spectroscopic data:
MS (ESI, pos.ion) *m*/*z*: 252.32 [M+H]⁺;
¹H NMR (400 MHz, CDCl₃ *δ* (ppm): 6.97 (s, 2H), 4.90 (t, 1H, *J* = 8.0 Hz), 3.76 (dd, 1H, *J* = 10 Hz, 7.2 Hz), 2.83 (t, 1H, *J* = 8.0 Hz), 2.64-2.33 (m, 2H), 2.32-2.12 (m, 1H), 1.47 (s, 9H), 1.09 (d, 3H, *J* = 6.4 Hz).

### Step 8) the preparation of compound 14-10

To a solution of compound **14-9** (0.51 g, 2.03 mmol) in DCM (10 mL) was added NIS (1.0 g, 4.46 mmol) at 0 °C. The mixture was stirred at 0 °C for 2.0 hrs and filtered. The filtrate was washed with saturated Na₂SO₃ aqueous solution (30 mL x 3), dried over anhydrous Na₂SO₄ and concentrated *in vacuo* to give the title compound as a yellow solid (0.9 g, 90%), which was used for the next step directly. The compound was characterized by the following spectroscopic data:
MS (ESI, pos.ion) *m*/*z*: 504.12 [M+H]⁺;
¹H NMR (400 MHz, CDCl₃) *δ* (ppm): 4.85 (t, 1H, *J* = 8.0 Hz), 3.75 (dd, 1H, *J* = 10 Hz, 7.2 Hz), 2.84 (t, 1H, *J* = 10 Hz), 2.52-2.29 (m, 2H), 2.21 (d, 1H, *J* = 6.6 Hz), 1.48 (s, 9H), 1.08 (d, 3H, *J* = 6.4 Hz).

### Step 9) the preparation of compound 14-11

To a solution of compound **14-10** (0.9 g, 1.8 mmol) in ethanol (10.0 mL) were added Na₂SO₃ (2.0 g, 16 mmol) and water (10 mL). The mixture was stirred at 90 °C for 30 hrs and filtered. The filtrate was concentrated in *vacuo.* The residue was dissolved in DCM (50 mL). The mixture was washed with water (20 mL x 2) and brine, dried over anhydrous Na₂SO₄ and concentrated *in vacuo.* The residue was purified by silica gel column chromatography (PE/EtOAc (v/v) = 6/l) to give the title compound as a white solid (0.38 g, 60%). The compound was characterized by the following spectroscopic data:
MS (ESI, pos.ion) *m*/*z*: 378.22 [M+H]⁺;
¹H NMR (400 MHz, CDCl₃) *δ* (ppm): 7.04 (s, 1H), 4.85 (t, 1H, *J* = 8.4 Hz), 3.75 (dd, 1H, *J* = 10.3 Hz, 7.3 Hz), 2.82 (t, 1H, *J* = 10.4 Hz), 2.58-2.36 (m, 2H), 2.29-2.11 (m, 1H), 1.08 (d, 3H, *J* = 6.4 Hz).

### Step 10) the preparation of compound 14-12

A suspension of compound **14-6** (0.36 g, 0.58 mmol), compound **14-11** (0.24 g, 0.63 mmol), Pd(PPh₃)₄ (35 mg, 0.03 mmol) and K₂CO₃ (0.08 g, 1.4 mmol) in mixed solvents of EtOH and H₂O (10 mL, v/v = 4/1) was stirred at 90°C under N₂ for 4.0 hrs. After the reaction was completed, the mixture was cooled to rt and diluted with EtOAc (40 mL). The resulting mixture was washed with water (30 mL x 3) and brine, dried over anhydrous Na₂SO₄ and concentrated *in vacuo.* The residue was purified by silica gel column chromatography (DCM/MeOH (v/v) = 60/1) to give the title compound as a pale yellow solid (280 mg, 65%). The compound was characterized by the following spectroscopic data:
MS (ESI, pos.ion) *m*/*z*: 747.5 [M+H]⁺;
¹H NMR (400 MHz, CDCl₃) *δ* (ppm): 7.72 (s, 1H), 7.59 (s, 1H), 7.54-7.51 (m, 3H), 7.49-7.46 (m, 2H), 7.18, 7.16 (s, s, 1H), 5.06-4.97 (m, 2H), 3.80-3.72 (m, 2H), 3.32-3.25 (m, 1H), 3.09-3.02 (m, 1H), 2.85-2.81 (m, 2H), 2.37-2.16 (m, 8H), 1.94-1.86 (m, 2H), 1.82-1.58 (m, 6H), 1.43-1.42 (m, 18H), 0.96-0.93 (m, 6H).

### Step 11) the preparation of compound 14-13

To a solution of compound **14-12** (280 mg, 0.375 mmol) in EtOAc (4.0 mL) was added a solution of HCl in EtOAc (3.0 mL, 4 M) dropwise. At the end of the addition, the mixture was stirred at rt for 8.0 hrs. After the reaction was completed, the reaction mixture was concentrated *in vacuo.* The residue was purified by beating in EtOAc (10 mL) and filtered to give the title compound as a pale yellow solid (233.6 mg, 90%), which was used for the next step without further purification. The compound was characterized by the following spectroscopic data:
MS (ESI, pos.ion) *m*/*z*: 547.5 [M+H]⁺.

### Step 12) the preparation of compound 14-14

To a suspension of compound **14-13** (214.62 mg, 0.31 mmol), compound **1-11-2** (120 mg, 0.68 mmol), EDCI (130 mg, 0.68 mmol) and HOAT (85 mg, 0.62 mmol) in DCM (20.0 mL) was added DIPEA (0.51 mL, 3.1 mmol) at 0 °C. At the end of the addition, the mixture was stirred at rt for 3.0 hrs. After the reaction was completed, the mixture was diluted with DCM (20.0 mL), washed with NH₄Cl aqueous solution and brine, dried over anhydrous Na₂SO₄ and concentrated *in vacuo.* The residue was purified by silica gel column chromatography (DCM/MeOH (v/v) = 50/1) to give the title compound as a white solid (173.4 mg, 65%). The compound was characterized by the following spectroscopic data:
MS (ESI, pos.ion) *m*/*z*: 861.49 [M+H]⁺;
¹H NMR (400 MHz, CDCl₃) *δ* (ppm): 7.64 (s, 1H), 7.59 (s, 1H), 7.54-7.51 (m, 3H), 7.49-7.46 (m, 2H), 7.18, 7.16 (s, s, 1H), 5.56, 5.55 (d, d, 2H), 5.39-5.31 (m, 1H), 5.07-5.02 (m, 1H), 4.31-4.27 (m, 2H), 4.02-3.85 (m, 3H), 3.66 (s, 6H), 3.61-3.55 (m, 1H), 2.85-2.81 (m, 2H), 2.37-2.14 (m, 10H), 1.94-1.58 (m, 8H), 1.02, 1.00 (m, m, 6H), 0.94-0.89 (m, 12 H).

### Example 15

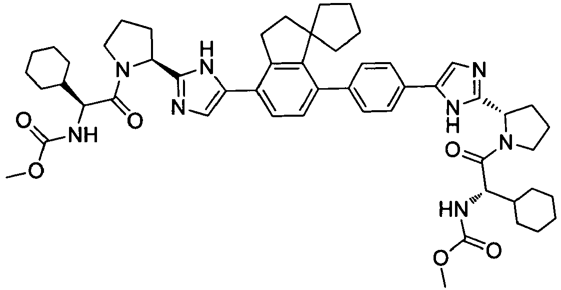

### Synthetic route:

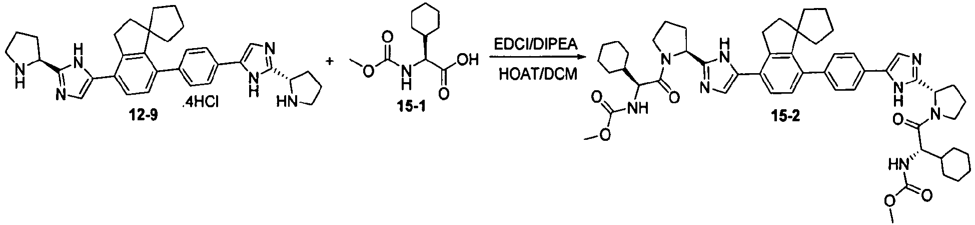

### Step 1) the preparation of compound 15-2

To a suspension of compound **12-9** (168 mg, 0.25 mmol), compound **15-1** (114 mg, 0.53 mmol), EDCI (102 mg, 0.53 mmol) and HOAT (69 mg, 0.51 mmol) in DCM (20.0 mL) was added DIPEA (0.352 mL, 2.02 mmol) dropwise at 0 °C. At the end of the addition, the mixture was stirred at rt for 3.0 hrs. After the reaction was completed, the mixture was diluted with DCM (20.0 mL), washed with NH₄Cl aqueous solution and brine, dried over anhydrous Na₂SO₄ and concentrated *in vacuo.* The residue was purified by silica gel column chromatography (DCM/MeOH (v/v) = 60/1) to give the title compound as a white solid (141.44 mg, 62%). The compound was characterized by the following spectroscopic data:
MS (ESI, pos.ion) *m*/*z*: 457.3 [M+2H]²⁺;
¹H NMR (400 MHz, CDCl₃) *δ* (ppm): 8.33-8.27 (m, 2H), 7.39 (s, 2H), 7.36 (d, 2H, *J* = 5.5 Hz), 7.28-7.21 (m, 2H), 5.45 (t, 1H, *J* = 7.8 Hz), 5.17 (dd, 2H, *J* = 12.0 Hz, 8.7 Hz), 4.90-4.85 (m, 1H), 4.53 (s, 2H), 3.85-3.73 (m, 1H), 3.63 (s, 6H), 3.44 (dt, 2H, *J* = 24.8 Hz, 13.2 Hz), 2.80 (t, 2H, *J* = 13.4 Hz), 2.52-2.36 (m, 2H), 2.23-1.60 (m, 25H), 1.51- 0.97 (m, 14H).

### Example 16

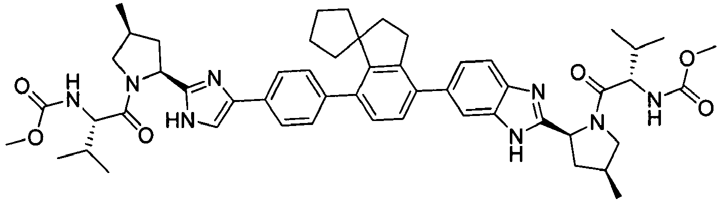

### Synthetic route:

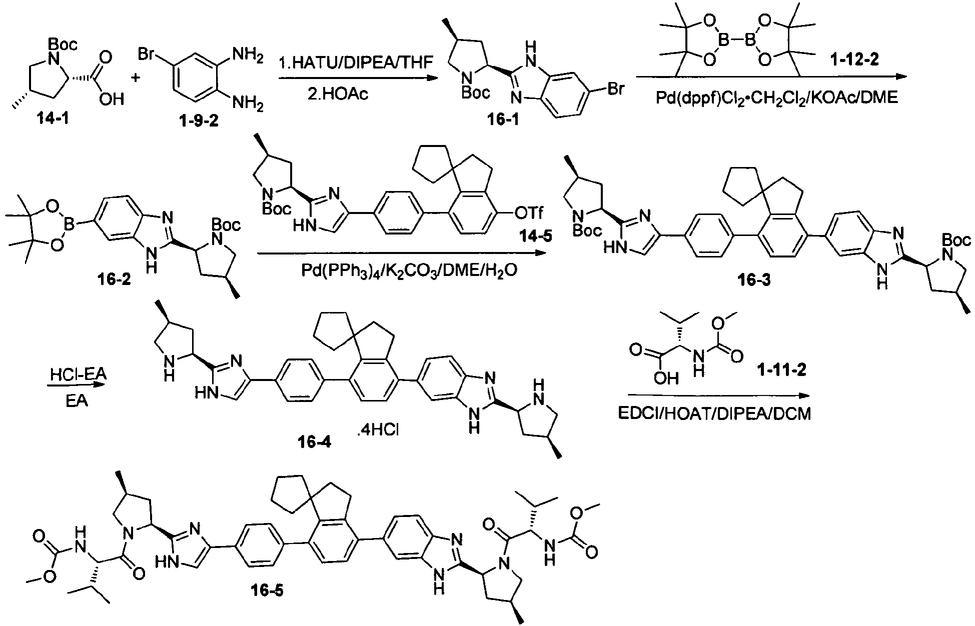

### Step 1) the preparation of compound 16-1

To a solution of compound **14-1** (2.45 g, 10.7 mmol) and HATU (4.88 g, 12.84 mmol) in THF (30.0 mL) was added DIPEA (1.95 mL, 11.8 mmol) at 0°C. After stirring at 0°C for 0.5 hr, compound **1-9-2** (2.22 g, 11.9 mmol) was added in a portionwise manner, and the mixture was stirred at rt for 4.0 hrs. After the reaction was completed, the mixture was quenched with water (50 mL), and the THF solvent was removed. The resulting mixture was extracted with EtOAc (50 mL x 3). The combined organic layers were washed with brine, dried over anhydrous Na₂SO₄ and concentrated *in vacuo.* The residue was dissolved in glacial acetic acid (20.0 mL), and the mixture was stirred at 40 °C overnight. After the reaction was completed, the solvent was removed. The resulting mixture was dissolved in EtOAc (100 mL), washed with Na₂CO₃ aqueous solution (50.0 mL x 3), dried over anhydrous Na₂SO₄ and *concentrated in vacuo.* The residue was purified by silica gel column chromatography (PE/EtOAc (v/v) = 1/2) to give the title compound (3.245 g, 80%). The compound was characterized by the following spectroscopic data:
MS (ESI, pos.ion) *m*/*z*: 380.5 [M+H]⁺;
¹H NMR (400 MHz, CDCl₃) *δ* (ppm): 7.84 (d, 1H, *J* = 2.9 Hz), 7.44 (d, 1H, *J* = 15.0 Hz), 7.33 (dd, 1H, *J* = 15.0 Hz, 2.9 Hz), 4.88 (t, 1H, *J* = 16.9 Hz), 4.27 (dd, 1H, *J* = 24.8 Hz, 17.3 Hz), 3.14 (dd, 1H, *J* = 24.7 Hz, 17.3 Hz), 2.53 (dt, 1H, *J* = 24.4 Hz, 17.2 Hz), 2.21-2.03 (m, 1H), 1.81 (dt, 1H, *J* = 24.4 Hz, 17.2 Hz), 1.41 (s, 9H), 0.95 (d, 3H, *J* = 12.7 Hz).

### Step 2) the preparation of compound 16-2

A mixture of compound **16-1** (4.27 g, 11.27 mmol), compound **1-12-2** (4.29 g, 16.9 mmol), Pd(dppf)Cl₂CH₂Cl₂ (653 mg, 0.8 mmol) and KOAc (2.09 g, 21.3 mmol) in DME (30.0 mL) was stirred at 90 °C under N₂ for 3.0 hrs. After the reaction was completed, DME was removed and then water (50.0 mL) was added. The mixture was extracted with EtOAc (50.0 mL x 3). The combined organic layers were washed with brine, dried over anhydrous Na₂SO₄ and concentrated *in vacuo.* The residue was purified by silica gel column chromatography (PE/EtOAc (v/v) = 3/1) to give the title compound as a beige solid (2.889 g, 60%). The compound was characterized by the following spectroscopic data:
MS (ESI, pos.ion) *m*/*z*: 428.5 [M+H]⁺;
¹H NMR (400 MHz, CDCl₃) *δ* (ppm): 7.82 (dd, 1H), 7.65,7.63 (d, d, 1H), 7.27, 7.25 (d, d, 1H), 5.07-5.02 (m, 1H), 3.85-3.78 (m, 1H), 3.14-3.07 (m, 1H), 2.51-2.42 (m, 1H), 2.30-2.16 (m, 1H), 1.86-1.78 (m, 1H), 1.41 (s, 9H), 1.32, 1.29 (m, m, 12H), 0.96-0.93 (m, 3H).

### Step 3) the preparation of compound 16-3

A mixture of compound **14-5** (0.374 g, 0.58 mmol), compound **16-2** (0.269 g, 0.63 mmol), Pd(PPh₃)₄ (35 mg, 0.03 mmol) and K₂CO₃ (0.08 g, 1.4 mmol) in mixed solvents of DME/H₂O (10.0 mL, v/v = 4/1) was stirred at 90 °C under N₂ for 4.0 hrs, cooled to rt, and diluted with EtOAc (50.0 mL). The resulting mixture was washed with water (30.0 mL x 3) and brine, dried over anhydrous Na₂SO₄ and concentrated *in vacuo.* The residue was purified by silica gel column chromatography (DCM/MeOH (v/v) = 100/1) to give the title compound as a pale yellow solid (300.3 mg, 65%). The compound was characterized by the following spectroscopic data:
MS (ESI, pos.ion) *m*/*z*: 797.5 [M+H]⁺;
¹H NMR (400 MHz, CDCl₃) *δ* (ppm): 8.33-8.27 (m, 2H), 8.19 (dd, 1H, *J* = 15.0 Hz, 2.9 Hz), 8.03 (d, 1H, *J* = 3.1 Hz), 7.76 (d, 1H, *J* = 15.0 Hz), 7.56-7.47 (m, 2H), 7.28-7.22 (m, 3H), 5.46 (dt, 2H, *J* = 17.7 Hz, 17.0 Hz), 4.37 (dt, 2H, *J* = 24.7 Hz, 17.2 Hz), 3.02-2.51 (m, 6H), 2.16-2.00 (m, 2H), 1.98-1.90 (m, 2H), 1.80-1.59 (m, 10H), 1.42 (s, 18H), 0.97-0.92 (m, 6H).

### Step 4) the preparation of compound 16-4

To a solution of compound **16-3** (298.7 mg, 0.375 mmol) in EtOAc (4.0 mL) was added a solution of HCl in EtOAc (3.0 mL, 4 M) dropwise. At the end of the addition, the mixture was stirred at rt for 8.0 hrs. After the reaction was completed, the mixture was concentrated *in vacuo.* The residue was purified by beating in EtOAc (5.0 mL) and filtered to give the title compound as a pale yellow solid (250.5 mg, 90%), which was used for the next step without further purification. The compound was characterized by the following spectroscopic data:
MS (ESI, pos.ion) *m*/*z*: 597.3 [M+H]⁺.

### Step 5) the preparation of compound 16-5

To a suspension of compound **16-4** (230.1 mg, 0.31 mmol), compound **1-11-2** (120 mg, 0.68 mmol), EDCI (130 mg, 0.68 mmol) and HOAT (85 mg, 0.62 mmol) in DCM (20.0 mL) was added DIPEA (0.51 mL, 3.1 mmol) dropwise at 0 °C. At the end of the addition, the mixture was stirred at rt for 3.0 hrs. After the reaction was completed, the mixture was diluted with DCM (20.0 mL). The resulting mixture was washed with NH₄Cl aqueous solution and brine, dried over anhydrous Na₂SO₄ and concentrated *in vacuo.* The residue was purified by silica gel column chromatography (DCM/MeOH (v/v) = 50/1) to give the title compound as a white solid (158 mg, 56%). The compound was characterized by the following spectroscopic data:
MS (ESI, pos.ion) *m*/*z*: 456.3 [M+2H]²⁺;
¹H NMR (400 MHz, EDCl₃) *δ* (ppm): 7.70-7.67 (m, 2H), 7.65-7.61 (m, 2H), 7.60-7.53 (m, 4H), 7.37, 7.35 (d, d, 1H), 7.32 (s, 1H), 5.56, 5.55 (d, d, 2H), 5.31-5.26 (m, 1H), 5.04-4.99 (m, 1H), 4.35-4.27 (m, 2H), 3.92-3.84 (m, 2H), 3.66 (s, 6H), 3.61-3.54 (m, 2H), 3.10-3.06 (m, 2H), 2.56-2.47 (m, 1H), 2.43-2.14 (m, 12H), 1.83-1.74 (m, 1H), 1.73-1.58 (m, 4H), 1.02, 1.00 (m, m, 6H), 0.94-0.90 (m, 12H).

### Example 17

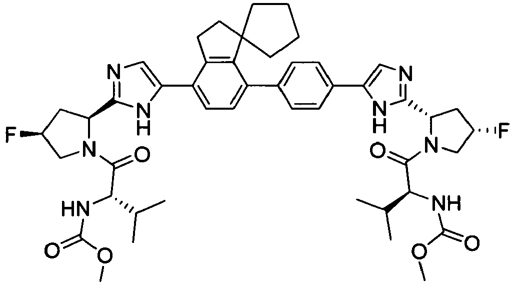

### Synthetic route:

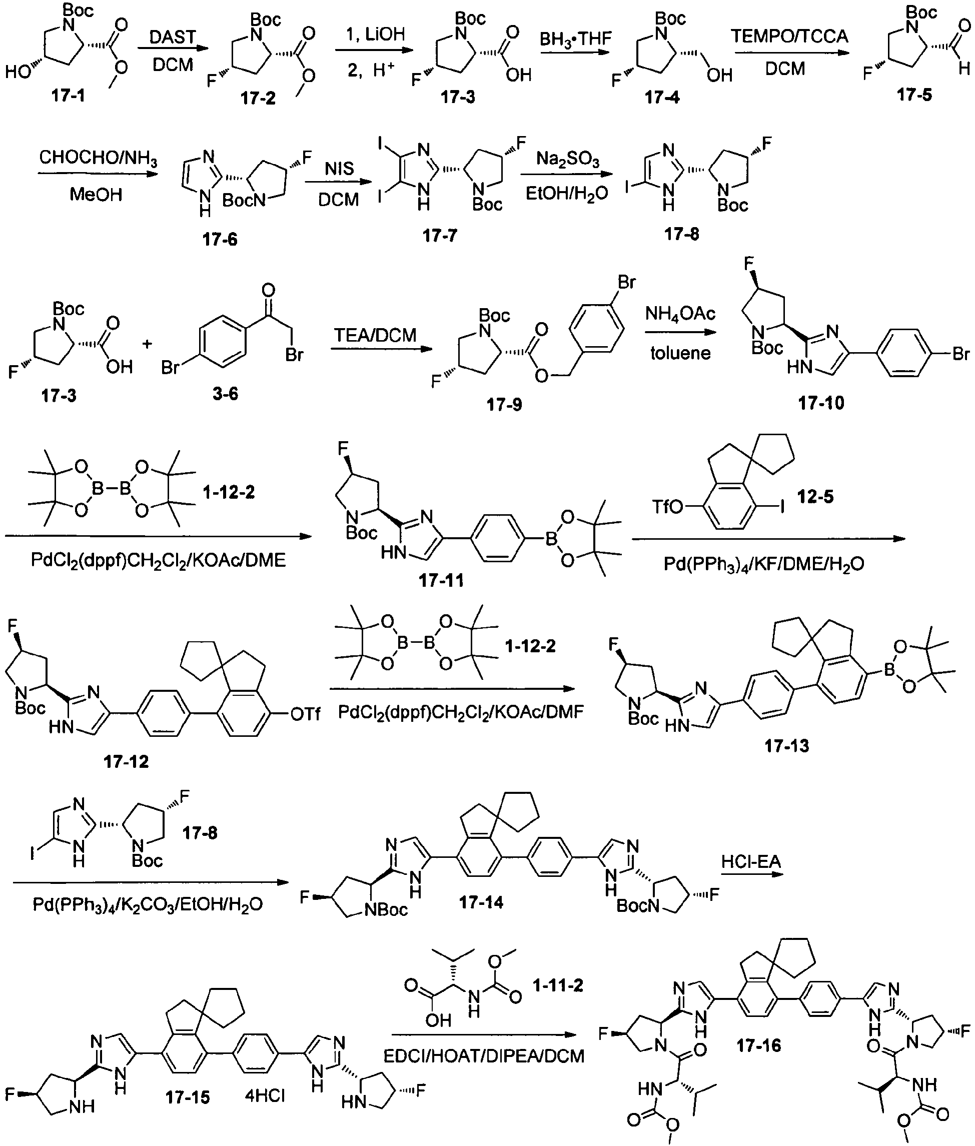

### Step 1) the preparation of compound 17-2

To a solution of compound **17-1** (11.0 g, 44.84 mmol) in DCM (200 mL) was added Et₂NSF₃ (8.85 mL, 67.3 mmol) dropwise at -78 °C. At the end of the addition, the mixture was stirred at -78 °C for 2.0 hrs and at rt for another 19 hrs. After the reaction was completed, the mixture was quenched with NH₄Cl aqueous solution (100 mL). The resulting mixture was extracted with DCM (100 mL x 3). The combined organic layers were washed with brine, dried over anhydrous Na₂SO₄ and concentrated *in vacuo.* The residue was purified by silica gel column chromatography (PE/EtOAc (v/v) = 20/1) to give the title compound as pale yellow liquid (5.0 g, 70%). The compound was characterized by the following spectroscopic data:
MS (ESI, pos.ion) *m*/*z*: 248.26 [M+H]⁺;
¹H NMR (400 MHz, CDCl₃) *δ* (ppm): 5.26, 5.13 (ds, ds, 1H), 4.55-4.41 (m, 1H), 3.88-3.74 (m, 1H), 3.73 (s, 3H), 3.64-3.58 (m, 1H), 2.52-2.44 (m, 1H), 2.40-2.32 (m, 1H), 1.42-1.47 (d, 9H, *J* = 20 Hz).

### Step 2) the preparation of compound 17-3

To a solution of compound **17-2** (5.83 g, 23.58 mmol) in THF (30 mL) was added LiOH aqueous solution (1.98 g, 30 mL) at 0 °C. At the end of the addition, the mixture was stirred at rt for 2.0 hrs. After the reaction was completed, the mixture was adjusted to pH 5 with diluted hydrochloric acid (1 M) and the THF solvent was removed *in vacuo.* The aqueous layer was adjusted to pH 2 with diluted hydrochloric acid (1 M) and extracted with EtOAc (80 mL x 3). The combined organic layers were washed with brine, dried over Na₂SO₄ and concentrated *in vacuo* to give the title compound as a white solid (5.3 g, 96%). The compound was characterized by the following spectroscopic data:
MS (ESI, pos.ion) *m*/*z*: 234.24 [M+H]⁺;
¹H NMR (400 MHz, CDCl₃) *δ* (ppm): 8.76 (brs, 1H), 5.28-5.12 (m, 1H), 4.56-4.44 (m, 1H), 3.86-3.58 (m, 2H), 2.77-2.01 (m, 2H), 1.48-1.44 (d, 9H, *J* = 16 Hz).

### Step 3) the preparation of compound 17-4

To a solution of compound **17-3** (1.3 g, 5.57 mmol) in THF (20 mL) was added borane (8.3 mL, 1 M in THF) at 0 °C. At the end of the addition, the mixture was stirred at rt for 2.0 hrs. After the reaction was completed, the mixture was quenched with MeOH (4.0 mL) and concentrated *in vacuo.* The residue was dissolved in DCM (50 mL). The resulting mixture was washed with water (20 mL x 3) and brine, dried over anhydrous Na₂SO₄ and concentrated *in vacuo* to give the title compound as colorless slurry (1.15 g, 88%). The compound was characterized by the following spectroscopic data:
MS (ESI, pos.ion) *m*/*z*: 220.24 [M+H]⁺;
¹H NMR (400 MHz, CDCl₃) *δ* (ppm): 5.19-5.06 (m, 1H), 4.12-4.04 (m, 1H), 3.99-3.79 (m, 1H), 3.69-3.63 (m, 1H), 3.60-3.46 (m, 2H), 2.25-2.00 (m, 2H), 1.44 (s, 9H).

### Step 4) the preparation of compound 17-6

To a solution of compound **17-4** (1.15 g, 5.24 mmol) in DCM (20 mL) was added TCCA (1.22 g, 5.24 mmol) at 0 °C followed by a solution of TEMPO (82 mg, 0.52 mmol) in DCM (3.0 mL) dropwise. The mixture was stirred at 0 °C for 1.0 hr and at rt for another 1.0 hr. After the reaction was completed, the mixture was filtered. The filtrate was washed with saturated Na₂SO₃ aqueous solution (20 mL x 3), dried over anhydrous Na₂SO₄ and concentrated *in vacuo.* The residue was dissolved in a solution of NH₃ in MeOH (7 mL, 7 M). The solution was stirred at 0 °C for 0.5 hr and at rt for another 1.0 hr. Glyoxal (1.1 mL, 40%) was added to the mixture dropwise at 0 °C. At the end of the addition, the mixture was stirred at rt for 24 hrs. After the reaction was completed, the mixture was concentrated *in vacuo.* The residue was dissolved in DCM (50 mL). The solution was washed with water (30 mL x 3) and brine, dried over anhydrous Na₂SO₄ and concentrated *in vacuo.* The residue was purified by silica gel column chromatography (DCM/MeOH (v/v) = 100/1) to give the title compound as a pale yellow solid (0.63 g, 50%). The compound was characterized by the following spectroscopic data:
MS (ESI, pos.ion) *m*/*z*: 256.29 [M+H]⁺;
¹H NMR (400 MHz, CDCl₃) *δ* (ppm): 6.98 (s, 2H), 5.36-5.13 (m, 2H), 3.72-3.31 (m, 2H), 2.58-2.32 (m, 2H), 1.48 (s, 9H).

### Step 5) the preparation of compound 17-7

To a solution of compound **17-6** (0.63 g, 2.47 mmol) in DCM (8.0 mL) was added NIS (1.23 g, 5.43 mmol) at 0 °C. At the end of the addition, the mixture was stirred at 0 °C for 2.0 hrs. After the reaction was completed, the mixture was diluted with DCM (50 mL) and filtered. The filtrate was washed with saturated Na₂SO₃ aqueous solution (20 mL x 3), dried over anhydrous Na₂SO₄ and concentrated *in vacuo* to give the title compound as a yellow solid (1.07 g), which was used for the next step directly. The compound was characterized by the following spectroscopic data:
MS (ESI, pos.ion) *m*/*z*: 508.08 [M+H]⁺;
¹H NMR (400 MHz, CDCl₃) *δ* (ppm): 5.34-5.08 (m, 2H), 3.72-3.28 (m, 2H), 2.58-2.33 (m, 2H), 1.48 (s, 9H).

### Step 6) the preparation of compound 17-8

To a solution of compound **17-7** (1.07 g, 2.12 mmol) in ethanol (6.0 mL) were added Na₂SO₃ (2.14 g, 17 mmol) and water (6.0 mL), and the mixture was stirred at 90 °C for 30 hrs. After the reaction was completed, the mixture was filtered, and the filtrate was concentrated *in vacuo.* The residue was dissolved in DCM (40 mL). The solution was washed with water (20 mL x 3) and brine, dried over anhydrous Na₂SO₄ and concentrated *in vacuo.* The residue was purified by silica gel column chromatography (PE/EtOAc (v/v) = 1/1) to give the title compound as a white solid (0.58 g, 73%). The compound was characterized by the following spectroscopic data:
MS (ESI, pos.ion) *m*/*z*: 382.19 [M+H]⁺;
¹H NMR (400 MHz, CDCl₃) *δ* (ppm): 7.04 (s, 1H), 5.35-5.09 (m, 2H), 3.98-3.63 (m, 1H), 3.58-3.29 (m, 1H), 2.55-2.34 (m, 2H), 1.48 (s, 9H).

### Step 7) the preparation of compound 17-9

To a solution of compound **17-3** (5.0 g, 21.45 mmol) and compound **3-6** (4.93 g, 17.87 mmol) in DCM (100 mL) was added TEA (4.34 g, 42.9 mmol) dropwise at 0 °C. At the end of the addition, the mixture was stirred at rt for 3.0 hrs. After the reaction was completed, the mixture was quenched with water (50 mL). The resulting mixture was extracted with DCM (50 mL x 3). The combined organic layers were washed with brine, dried over anhydrous Na₂SO₄ and concentrated *in vacuo.* The residue was purified by silica gel column chromatography (PE/EtOAc (v/v) = 8/1) to give the title compound (4.8 g, 52.2%). The compound was characterized by the following spectroscopic data:
MS (ESI, pos.ion) *m*/*z*: 403.26 [M+H]⁺.

### Step 8) the preparation of compound 17-10

A mixture of compound **17-9** (4.5 g, 11.19 mmol) and ammonium acetate (12.5 g, 162 mmol) in toluene (50 mL) was stirred at 110 °C for 5.0 hrs. After the reaction was completed, the mixture was cooled to rt and quenched with water (50 mL). The aqueous layer was extracted with EtOAc (80 mL x 3). The combined organic layers were washed with brine, dried over anhydrous Na₂SO₄ and concentrated *in vacuo.* The residue was purified by silica gel column chromatography (PE/EtOAc (v/v) = 3/1) to give the title compound (4.2 g, 92%). The compound was characterized by the following spectroscopic data:
MS (ESI, pos.ion) *m*/*z*: 411.2 [M+H]⁺;
¹H NMR (400 MHz, CDCl₃ *δ* (ppm): 7.56-7.51 (m, 2H), 7.47-7.45 (m, 2H), 7.22 (s, 1H), 5.38-5.29 (m, 1H), 5.25-5.17 (m, 1H), 4.13-4.07, 3.62-3.39 (m, m, 1H), 3.68-3.58 (m, 1H), 2.68-2.38 (m, 2H), 1.38 (s, 9H).

### Step 9) the preparation of compound 17-11

A mixture of compound **17-10** (2.0 g, 4.87 mmol), compound **1-12-2** (1.26 g, 4.97 mmol), Pd(dppf)Cl₂·CH₂Cl₂ (0.07 g, 0.097 mmol) and KOAc (1.19 g, 12.2 mmol) in DME (20 mL) was stirred at 90 °C under N₂ for 2.0 hrs. After the reaction was completed, the mixture was cooled to rt, diluted with EtOAc (100 mL) and filtered through a celite pad. The filtrate was washed with water (30 mL x 3) and brine, dried over anhydrous Na₂SO₄ and concentrated *in vacuo.* The residue was purified by silica gel column chromatography (PE/EtOAc (v/v) = 1/2) to give the title compound (1.425 g, 64%). The compound was characterized by the following spectroscopic data:
MS (ESI, pos.ion) *m*/*z*: 458.35 [M+H]⁺;
¹H NMR (400 MHz, CDCl₃) *δ* (ppm): 7.81-7.79 (m, 2H), 7.65-7.60 (m, 2H), 7.28 (s, 1H), 5.39-5.26 (m, 1H), 5.20-5.12 (m, 1H), 4.07-3.99, 3.59-3.41 (m, 1H), 3.69-3.62 (m, 1H), 2.62-2.51 (m, 2H), 1.34 (s, 12H), 1.28 (s, 9H).

### Step 10) the preparation of compound 17-12

To a mixture of compound **17-11** (1.198 g, 2.62 mmol), compound **12-5** (1.17 g, 2.62 mmol), Pd(PPh₃)₄ (120 mg, 0.1 mmol) and KF (0.30 g, 5.24 mmol) were added DME (12 mL) and pure water (3.0 mL) via syringe. The mixture was stirred at 90 °C for 2.0 hrs. After the reaction was completed, the mixture was cooled to rt and diluted with EtOAc (50 mL). The resulting mixture was washed with water (20 mL x 3) and brine, dried over anhydrous Na₂SO₄ and concentrated *in vacuo.* The residue was purified by silica gel column chromatography (DCM/MeOH (v/v) = 100/1) to give the title compound as a white solid (1.02 g, 60%). The compound was characterized by the following spectroscopic data:
MS (ESI, pos.ion) *m*/*z*: 650.3 [M+H]⁺;
¹H NMR (400 MHz, CDCl₃ *δ* (ppm): 7.66-7.61 (m, 4H), 7.44 (s, 1H), 7.27, 7.25 (s, s, 1H), 6.97, 6.95 (d, d, 1H), 5.39-5.31, 5.26-5.19 (m, m, 1H), 4.83-4.78 (m, 1H), 4.04-3.92 (m, 1H), 3.79-3.66 (m, 1H), 3.00-2.96 (m, 2H), 2.77-2.61 (m, 1H), 2.42-2.20 (m, 7H), 1.76-1.59 (m, 4H), 1.41 (s, 9H).

### Step 11) the preparation of compound 17-13

A mixture of compound **17-12** (1.05 g, 1.61 mmol), compound **1-12-2** (0.45 g, 10.7 mmol), Pd(dppf)Cl₂CH₂Cl₂ (80 mg, 0.096 mmol) and KOAc (0.4 g, 4.02 mmol) in DMF (10 mL) was stirred at 120°C under N₂ for 4.0 hrs. After the reaction was completed, the mixture was cooled to rt, diluted with EtOAc (80 mL) and filtered through a celite pad. The filtrate was washed with water (30 mL x 3) and brine, dried over anhydrous Na₂SO₄ and concentrated *in vacuo.* The residue was purified by silica gel column chromatography (PE/EtOAc (v/v) = 3/1) to give the title compound as a white solid (707 mg, 70%). The compound was characterized by the following spectroscopic data:
MS (ESI, pos.ion) *m*/*z*: 628.4 [M+H]⁺;
¹H NMR (400 MHz, CDCl₃) *δ* (ppm): 7.71-7.61 (m, 4H), 7.59, 7.57 (d, d, 1H), 7.46 (s, 0.5H), 7.44 (s, 1.5H), 5.39-5.31, 5.26-5.19 (m, m, 1H), 4.83-4.78 (m, 1H), 4.04-3.92 (m, 1H), 3.79-3.66 (m, 1H), 2.94-2.90 (m, 2H), 2.77-2.61 (m, 1H), 2.41-2.24 (m, 5H), 1.99-1.90 (m, 2H), 1.76-1.60 (m, 4H), 1.41 (s, 9H), 1.32, 1.29 (m, m, 12H).

### Step 12) the preparation of compound 17-14

A suspension of compound **17-8** (0.17 g, 0.446 mmol), compound **17-13** (0.264 g, 0.42 mmol), Pd(PPh₃)₄ (25 mg, 0.02 mmol) and K₂CO₃ (0.17 g, 1.27 mmol) in mixed solvents of EtOH and H₂O (8.0 mL, v/v = 3/1) was stirred at 90 °C under N₂ for 2.0 hrs. After the reaction was completed, the mixture was concentrated *in vacuo.* The residue was dissolved in EtOAc (50 mL). The resulting mixture was washed with brine, dried over anhydrous Na₂SO₄ and concentrated *in vacuo.* The residue was purified by silica gel column chromatography (DCM/EtOH (v/v) = 100/1) to give the title compound as a pale yellow solid (206 mg, 65%). The compound was characterized by the following spectroscopic data:
MS (ESI, pos.ion) *m*/*z*: 755.5 [M+H]⁺;
¹H NMR (400 MHz, CDCl₃) *δ* (ppm): 7.92 (s, 1H), 7.70-7.67 (m, 2H), 7.65-7.61 (m, 2H), 7.53, 7.51 (d, d, 1H), 7.44 (s, 1H), 7.18, 7.16 (s, s, 1H), 5.39-5.29 (m, 1H), 5.26-5.19 (m, 1H), 5.07-5.02 (m, 1H), 4.83-4.78 (m, 1H), 4.01-3.91 (m, 2H), 3.80-3.66 (m, 2H), 2.84-2.80 (m, 2H), 2.79-2.61 (m, 2H), 2.42-2.24 (m, 6H), 1.94-1.86 (m, 2H), 1.76-1.58 (m, 4H), 1.41 (s, 18H).

### Step 13) the preparation of compound 17-15

To a solution of compound **17-14** (384.7 mg, 0.51 mmol) in EtOAc (4.0 mL) was added a solution of HCl in EtOAc (3.0 mL, 4 M) dropwise. At the end of the addition, the mixture was stirred at rt for 8.0 hrs. After the reaction was completed, the mixture was concentrated *in vacuo.* The residue was purified by beating in EtOAc (4.0 mL) and filtered to give the title compound as a pale yellow solid (321.4 mg, 90%), which was used for the next step without further purification. The compound was characterized by the following spectroscopic data:
MS (ESI, pos.ion) *m*/*z*: 555.3 [M+H]⁺.

### Step 14) the preparation of compound 17-16

To a suspension of compound **17-15** (203.1 mg, 0.29 mmol), compound **1-11-2** (110 mg, 0.65 mmol), EDCI (120 mg, 0.65 mmol) and HOAT (80 mg, 0.59 mmol) in DCM (5.0 mL) was added DIPEA (0.49 mL, 2.97 mmol) dropwise at 0 °C. At the end of the addition, the mixture was stirred at rt for 3.0 hrs. After the reaction was completed, the mixture was diluted with DCM (20.0 mL), washed with NH₄Cl aqueous solution and brine, dried over anhydrous Na₂SO₄ and concentrated *in vacuo.* The residue was purified by silica gel column chromatography (DCM/MeOH (v/v) = 40/1) to give the title compound as a white solid (163.7 mg, 65%). The compound was characterized by the following spectroscopic data:
MS (ESI, pos.ion) *m*/*z*: 869.5 [M+H]⁺;
¹H NMR (400 MHz, CDCl₃) *δ* (ppm): 7.83 (s, 1H), 7.54-7.46 (m, 5H), 7.38 (s, 1H), 7.18, 7.16 (s, s, 1H), 5.56, 5.55 (d, d, 2H), 5.35-5.27 (m, 1H), 5.22-5.17 (m, 1H), 5.16-5.12 (m, 1H), 5.09-5.04 (m, 1H), 4.30-4.25 (m, 2H), 4.06-3.94 (m, 2H), 3.82-3.69 (m, 2H), 3.66 (s, 6H), 2.85-2.63 (m, 4H), 2.44-2.17 (m, 8H), 1.94-1.86 (m, 2H), 1.76-1.58 (m, 4H), 1.02, 1.00 (m, 6H), 0.93, 0.91 (m, 6H).

### Example 18

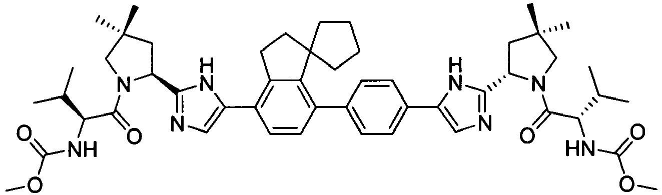

### Synthetic route:

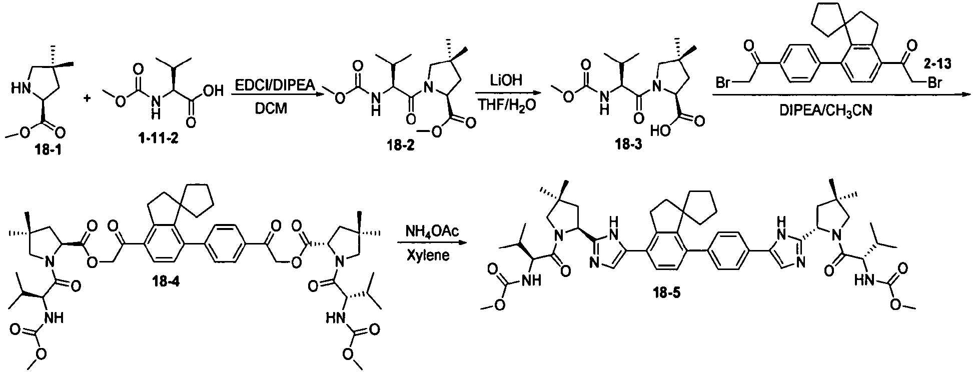

### Step 1) the preparation of compound 18-2

To a suspension of compound **18-1** (817 mg, 5.2 mmol), compound **1-11-2** (1.5 g, 8.6 mmol) and EDCI (1.95 g, 10 mmol) in DCM (5.0 mL) was added DIPEA (5.3 mL, 32 mmol) dropwise at 0 °C. At the end of the addition, the mixture was stirred at rt for 3.0 hrs. After the reaction was completed, the mixture was diluted with DCM (50 mL). The resulting mixture was washed with water (20 mL x 2) and brine, dried over anhydrous Na₂SO₄ and concentrated *in vacuo.* The residue was purified by silica gel column chromatography (PE/EtOAc (v/v) = 1/1) to give the title compound (980.24 mg, 60%). The compound was characterized by the following spectroscopic data:
MS (ESI, pos.ion) *m*/*z:* 315.3 [M+H]⁺;
¹H NMR (400 MHz, CDCl₃) *δ* (ppm): 5.32, 5.29 (d, d, 1H), 4.65-4.60 (m, 1H), 4.31-4.27 (m, 1H), 3.71 (s, 3H), 3.63 (s, 3H), 3.54-3.47 (m, 1H), 3.43-3.36 (m, 1H), 2.31-2.25 (m, 1H), 2.14-2.04 (m, 1H), 1.94-1.88 (m, 1H), 0.97, 0.95 (m, m, 3H), 0.92-0.89 (m, 9H).

### Step 2) the preparation of compound 18-3

To a solution of compound **18-2** (1.45 g, 4.6 mmol) in THF (10 mL) was added LiOH aqueous solution (2.1 g, 50 mmol, 8.0 mL) at 0 °C, and the mixture was stirred at rt overnight. After the reaction was completed, the THF solvent was removed. To the residue was added water (40 mL). The aqueous layer was extracted with EtOAc (30 mL x 3) and the organic layers were separated. The aqueous layer was adjusted to pH 2 with diluted hydrochloric acid (10%) and extracted with EtOAc (50 mL x 3). The combined organic layers were washed with brine, dried over anhydrous Na₂SO₄ and concentrated *in vacuo* to give the title compound as colorless slurry (1.17 g, 85%). The compound was characterized by the following spectroscopic data:
¹H NMR (400 MHz, CDCl₃) *δ* (ppm): 5.32, 5.29 (d, d, 1H), 4.81-4.76 (m, 1H), 4.35-4.30 (m, 1H), 3.63 (s, 3H), 3.57-3.49 (m, 1H), 3.44-3.37 (m, 1H), 2.24-2.18 (m, 1H), 2.14-2.04 (m, 1H), 1.89-1.83 (m, 1H), 0.97, 0.95 (m, m, 3H), 0.93-0.89 (m, 9H).

### Step 3) the preparation of compound 18-4

To a solution of compound **18-3** (1.14 g, 3.8 mmol) and compound **2-13** (878.4 mg, 1.8 mmol) in MeCN (15 mL) was added DIPEA (1.586 mL, 9.6 mmol) dropwise at 0 °C. At the end of the addition, the mixture was stirred at rt for 3.0 hrs. After the reaction was completed, the mixture was concentrated *in vacuo.* The residue was purified by silica gel column chromatography (PE/EtOAc (v/v) = 1/5) to give the title compound (0.83 g, 49.8%). The compound was characterized by the following spectroscopic data:
MS (ESI, pos.ion) *m*/*z*: 466.3 [M+2H]²⁺;
¹H NMR (400 MHz, CDCl₃) *δ* (ppm): 8.06-8.02 (m, 2H), 7.85, 7.83 (s, s, 1H), 7.68, 7.66 (d, d, 1H), 7.58-7.54 (m, 2H), 5.32, 5.29 (d, d, 2H), 5.21 (s, 2H), 5.19 (s, 2H), 4.76-4.71 (m, 2H), 4.34-4.29 (m, 2H), 3.63 (s, 3H), 3.54-3.47 (m, 2H), 3.42-3.35 (m, 2H), 3.07-3.03 (m, 2H), 2.42-2.33 (m, 2H), 2.28-2.19 (m, 4H), 2.16-2.04 (m, 2H), 1.99-1.85 (m, 4H), 1.77-1.60 (m, 4H), 0.97, 0.95 (m, m, 6H), 0.92-0.89 (m, 18H).

### Step 4) the preparation of compound 18-5

A suspension of compound **18-4** (826.4 mg, 0.89 mmol) and ammonium acetate (2.3 g, 29.8 mmol) in xylene (15 mL) was stirred at 130 °C for 4.0 hrs. After the reaction was completed, the mixture was cooled to rt and diluted with EtOAc (40 mL). The resulting mixture was washed with water (30 mL x 3) and brine, dried over anhydrous Na₂SO₄ and concentrated *in vacuo.* The residue was purified by silica gel column chromatography (DCM/MeOH (v/v) = 40/1) to give the title compound as a white solid (395.4 mg, 50%). The compound was characterized by the following spectroscopic data:
MS (ESI, pos.ion) *m*/*z*: 446.3 [M+2H]²⁺;
¹H NMR (400 MHz, CDCl₃) *δ* (ppm): 7.66 (s, 1H), 7.57 (s, 1H), 7.54-7.51 (m, 3H), 7.49-7.46 (m, 2H), 7.18, 7.16 (s, s, 1H), 5.48-5.43 (m, 1H), 5.41-5.36 (m, 1H), 5.32, 5.29 (d, d, 2H), 4.40-4.35 (m, 2H), 3.63 (s, 6H), 3.61-3.53 (m, 2H), 3.47-3.40 (m, 2H), 2.85-2.81 (m, 2H), 2.37-2.23 (m, 6H), 2.20-2.08 (m, 2H), 1.94-1.86 (m, 4H), 1.76-1.58 (m, 4H), 0.97, 0.96-0.94 (m, m, 18H), 0.91, 0.89 (m, m, 6H).

### Example 19

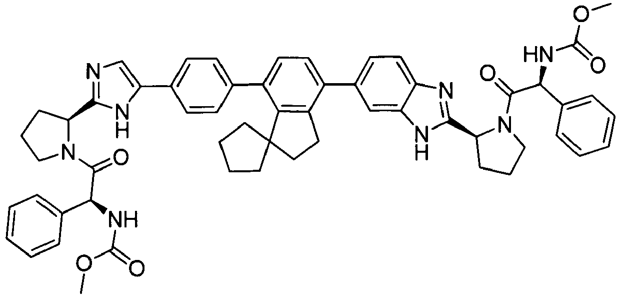

### Synthetic route:

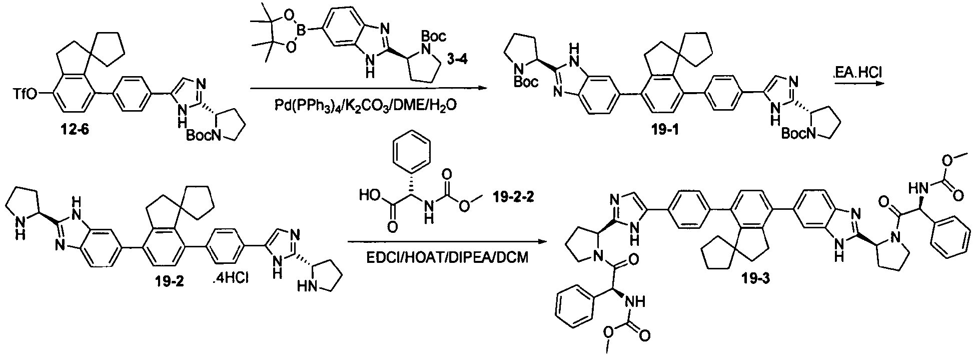

### Step 1) the preparation of compound 19-1

A suspension of compound **12-6** (366 mg, 0.58 mmol), compound **3-4** (260.35 mg, 0.63 mmol), Pd(PPh₃)₄ (35 mg, 0.03 mmol) and K₂CO₃ (80 mg, 1.4 mmol) in mixed solvents of DME and H₂O (10 mL, v/v = 4/1) was stirred at 90 °C under N₂ for 4.0 hrs. After the reaction was completed, the mixture was cooled to rt and diluted with EtOAc (40 mL). The resulting mixture was washed with water (20 mL x 3) and brine, dried over anhydrous Na₂SO₄ and concentrated *in vacuo.* The residue was purified by silica gel column chromatography (PE/EtOAc (v/v) = 1/1) to give the title compound as a pale yellow solid (267.4 mg, 60%). The compound was characterized by the following spectroscopic data:
MS (ESI, pos.ion) *m*/*z*: 769.5 [M+H]⁺;
¹H NMR (400 MHz, CDCl₃) *δ* (ppm): 7.60-7.51 (m, 7H), 7.49-7.46 (m, 2H), 7.44, 7.42 (d, d, 1H), 5.04-4.99 (m, 1H), 4.97-4.93 (m, 1H), 3.82-3.76 (m, 1H), 3.64-3.57 (m, 2H), 3.31-3.24 (m, 1H), 3.10-3.06 (m, 2H), 2.62-1.94 (m, 14H), 1.76-1.58 (m, 4H), 1.53 (s, 9H), 1.41 (s, 9H).

### Step 2) the preparation of compound 19-2

To a solution of compound **19-1** (288.2 mg, 0.375 mmol) in EtOAc (4.0 mL) was added a solution of HCl in EtOAc (3.0 mL, 4 M) dropwise. At the end of the addition, the mixture was stirred at rt for 8.0 hrs. After the reaction was completed, the mixture was concentrated *in vacuo.* The residue was purified by beating in EtOAc (5.0 mL) and filtered to give the title compound as a pale yellow solid (241 mg, 90%), which was used for the next step directly. The compound was characterized by the following spectroscopic data:
MS (ESI, pos.ion) *m*/*z*: 569.3 [M+H]⁺.

### Step 3) the preparation of compound 19-3

To a suspension of compound **19-2** (242.9 mg, 0.34 mmol), compound **19-2-2** (150 mg, 0.714 mmol), EDCI (73 mg, 0.38 mmol) and HOAT (47 mg, 0.34 mmol) in DCM (8.0 mL) was added DIPEA (0.4 mL, 2.42 mmol) dropwise at 0 °C. At the end of the addition, the mixture was stirred at rt for 3.0 hrs. After the reaction was completed, the mixture was diluted with DCM (20 mL), washed with NH₄Cl aqueous solution and brine, dried over anhydrous Na₂SO₄ and concentrated *in vacuo.* The residue was purified by silica gel column chromatography (DCM/MeOH (v/v) = 50/1) to give the title compound as a white solid (226.2 mg, 70%). The compound was characterized by the following spectroscopic data:
MS (ESI, pos.ion) *m*/*z*: 476.3 [M+2H]²⁺;
¹H NMR (400 MHz, CDCl₃) *δ* (ppm): 7.60-7.51 (m, 7H), 7.49-7.46 (m, 2H), 7.44, 7.42 (d, d, 1H), 7.35-7.27 (m, 5H), 7.20-7.15 (m, 5H), 6.12, 6.11 (s, s, 1H), 5.91, 5.89 (s, s, 1H), 5.35-5.32 (m, 2H), 5.19-5.13 (m, 2H), 3.91-3.84 (m, 2H), 3.75-3.66 (m, 2H), 3.64 (s, 3H), 3.62 (s, 3H), 3.10-3.06 (m, 2H), 2.43-2.08 (m, 14H), 2.02-1.91 (m, 2H), 1.76-1.58 (m, 2H).

### Example 20

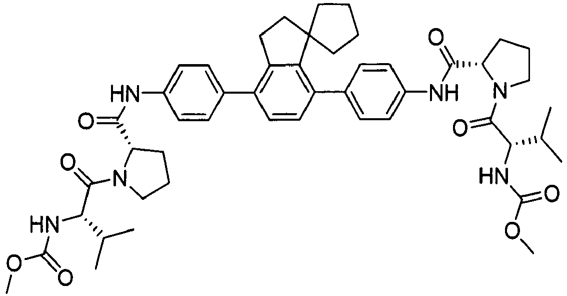

### Synthetic route:

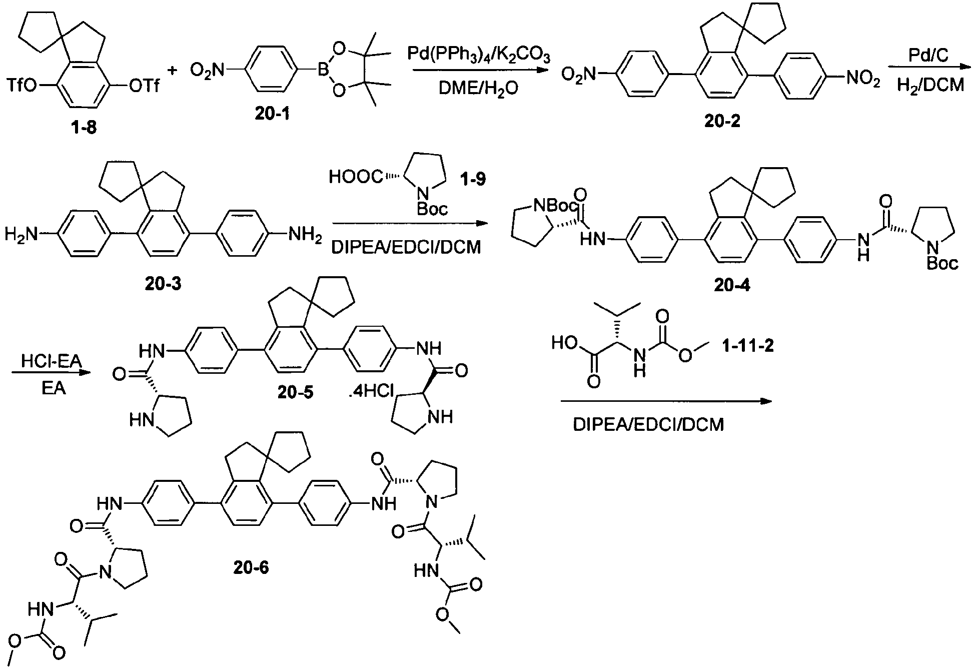

### Step 1) the preparation of compound 20-2

To a mixture of compound **20-1** (380 mg, 1.526 mmol), compound **1-8** (324.3 mg, 0.693 mmol), Pd(PPh₃)₄ (80.1 mg, 0.0693 mmol) and K₂CO₃ (478.6 mg, 3.463 mmol) were added DME (8.0 mL) and water (2.0 mL) via syringe, and the mixture was stirred at 90°C under N₂ for 3.0 hrs. After the reaction was completed, the mixture was cooled to rt, and 15 mL of water was added. The aqueous layer was extracted with DCM (25.0 mL x 3). The combined organic layers were washed with brine, dried over anhydrous Na₂SO₄ and concentrated *in vacuo.* The residue was purified by silica gel column chromatography (PE/EtOAc (v/v) = 5/1) to give the title compound as a yellow solid (272.67 mg, 95%). The compound was characterized by the following spectroscopic data:
MS (ESI, pos.ion) *m*/*z:* 415.3 [M+H]⁺;
¹H NMR (400 MHz, CDCl₃) *δ* (ppm): 8.41-8.37 (m, 2H), 8.30-8.26 (m, 2H), 7.60-7.56 (m, 3H), 7.52-7.48 (m, 3H), 2.84-2.80 (m, 2H), 2.43-2.35 (m, 2H), 2.30-2.20 (m, 6H), 1.76-1.58 (m, 2H).

### Step 2) the preparation of compound 20-3

A suspension of compound **20-2** (269.62 mg, 0.651 mmol) and a catalytic amount of Pd/C (27 mg) in DCM (15.0 mL) was stirred at rt under H₂ for 4.0 hrs. After the reaction was completed, the mixture was filtered, and the filtrate was concentrated *in vacuo* to give the title compound as a white solid (207.5 mg, 90%). The compound was characterized by the following spectroscopic data:
MS (ESI, pos.ion) *m*/*z*: 355.3 [M+H]⁺;
¹H NMR (400 MHz, CDCl₃ *δ* (ppm): 7.56, 7.54 (s, s, 1H), 7.44, 7.41 (d, d, 1H), 7.33-7.28 (m, 4H), 6.62-6.59 (m, 2H), 6.48-6.45 (m, 2H), 3.47 (br, 4H), 2.84-2.80 (m, 2H), 2.84-2.80 (m, 2H), 2.43-2.35 (m, 2H), 2.30-2.20 (m, 4H), 1.76-1.58 (m, 4H).

### Step 3) the preparation of compound 20-4

To a solution of compound **20-3** (120 mg, 0.339 mmol), compound **1-9** (218.6 mg, 1.016 mmol) and EDCI (259.9 mg, 1.356 mmol) in DCM (10.0 mL) was added DIPEA (0.336 mL, 2.033 mmol) dropwise at 0 °C. At the end of the addition, the mixture was stirred at rt for 12.0 hrs. After the reaction was completed, the mixture was quenched with water (20 mL). The aqueous layer was extracted with DCM (25.0 mL x 3). The combined organic layers were washed with NH₄Cl aqueous solution and brine, dried over anhydrous Na₂SO₄ and concentrated *in vacuo.* The residue was purified by silica gel column chromatography (PE/EtOAc (v/v) = 1/1) to give the title compound as a white solid (203 mg, 80%). The compound was characterized by the following spectroscopic data:
MS (ESI, pos.ion) *m*/*z*: 749.5 [M+H]⁺;
¹H NMR (400 MHz, CDCl₃) *δ* (ppm): 8.96 (m, 2H), 7.64-7.60 (m, 2H), 7.53, 7.51 (s, s, 1H), 7.50-7.46 (m, 2H), 7.42, 7.40 (dd, dd, 1H), 7.34-7.30 (m, 2H), 7.26-7.22 (m, 2H), 4.40-4.36 (m, 2H), 3.56-3.49 (m, 2H), 3.44-3.36 (m, 2H), 2.84-2.80 (m, 2H), 2.43-2.07 (m, 12H), 1.95-1.58 (m, 6H), 1.40 (s, 18H).

### Step 4) the preparation of compound 20-5

To a solution of compound **20-4** (94.9 mg, 0.1268 mmol) in EtOAc (5.0 mL) was added a solution of HCl in EtOAc (5.0 mL, 4 M) dropwise. At the end of the addition, the mixture was stirred at rt for 8.0 hrs. After the reaction was completed, the mixture was concentrated *in vacuo.* The residue was purified by beating in EtOAc (5.0 mL) and filtered to give the title compound as a white solid (83.63 mg, 95%). The compound was characterized by the following spectroscopic data:
MS (ESI, pos.ion) *m*/*z*: 549.5 [M+H]⁺.

### Step 5) the preparation of compound 20-6

To a suspension of compound **20-5** (125.67 mg, 0.181 mmol), compound **1-11-2** (95.2 mg, 0.543 mmol) and EDCI (139 mg, 0.725 mmol) in DCM (5.0 mL) was added DIPEA (0.3 mL, 1.815 mmol) dropwise at 0 °C. At the end of the addition, the mixture was stirred at rt for 3.0 hrs. After the reaction was completed, the mixture was diluted with DCM (20.0 mL). The resulting mixture was washed with NH₄Cl aqueous solution and brine, dried over anhydrous Na₂SO₄ and concentrated *in vacuo.* The residue was purified by silica gel column chromatography (EtOAc) to give the title compound as a white solid (132.69 mg, 85%). The compound was characterized by the following spectroscopic data:
MS (ESI, pos.ion) *m*/*z*: 863.5 [M+H]⁺;
¹H NMR (400 MHz, CDCl₃) *δ* (ppm): 8.96 (m, 1H), 8.90 (m, 1H), 7.77-7.73 (m, 2H), 7.67-7.63 (m, 2H), 7.53-7.48 (m, 3H), 7.42, 7.40 (dd, dd, 1H), 7.25-7.21 (m, 2H), 5.32, 5.30 (d, d, 2H), 4.31-4.28 (m, 2H), 4.27-4.22 (m, 2H), 3.63 (s, 6H), 3.61-3.55 (m, 2H), 3.44-3.36 (m, 2H), 2.84-2.80 (m, 2H), 2.43-2.35 (m, 2H), 2.30-2.20 (m, 4H), 2.18-2.02 (m, 8H), 1.76-1.58 (m, 6H), 0.97, 0.95 (m, m, 6H), 0.91, 0.89 (m, m, 6H).

### Example 21

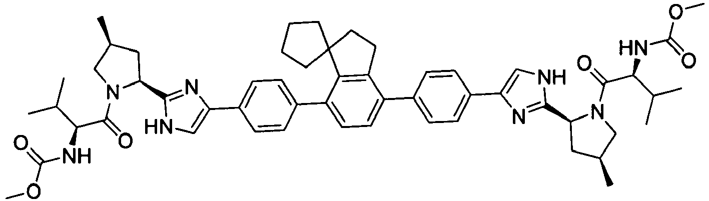

### Synthetic route:

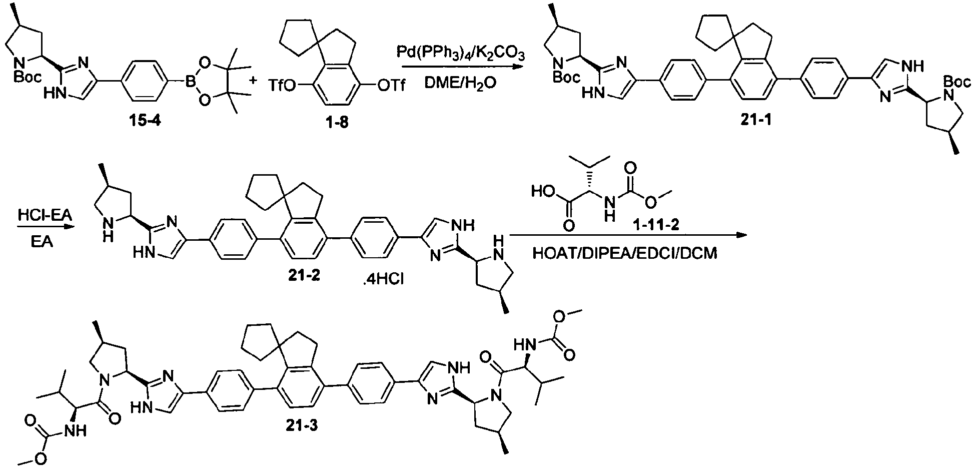

### Step 1) the preparation of compound 21-1

A suspension of compound **15-4** (552.1 mg, 1.218 mmol), compound **1-8** (271.45 mg, 0.58 mmol), Pd(PPh₃)₄ (35 mg, 0.03 mmol) and K₂CO₃ (80 mg, 1.4 mmol) in mixed solvents of DME and H₂O (10 mL, v/v = 4/1) was stirred at 90 °C under N₂ for 4.0 hrs. After the reaction was completed, the mixture was cooled to rt and diluted with EtOAc (40 mL). The resulting mixture was washed with water (20 mL x 3) and brine, dried over anhydrous Na₂SO₄ and concentrated *in vacuo.* The residue was purified by silica gel column chromatography (PE/EtOAc (v/v) = 1/3) to give the title compound as a pale yellow solid (238.52 mg, 50%). The compound was characterized by the following spectroscopic data:
MS (ESI, pos.ion) *m*/*z*: 823.5 [M+H]⁺;
¹H NMR (400 MHz, CDCl₃) *δ* (ppm): 7.76-7.73 (m, 2H), 7.71-7.67 (m, 4H), 7.65-7.61 (m, 3H), 7.55-7.53 (d, d, 1H), 7.36 (s, 2H), 4.81-4.76 (m, 2H), 3.80-3.73 (m, 2H), 3.09-3.02 (m, 2H), 2.84-2.80 (m, 2H), 2.43-2.35 (m, 2H), 2.23-2.16 (m, 8H), 1.76-1.58 (m, 6H), 1.41 (s, 18H), 0.96-0.93 (m, 6H).

### Step 2) the preparation of compound 21-2

To a solution of compound **21-1** (308.43 mg, 0.375 mmol) in EtOAc (4.0 mL) was added a solution of HCl in EtOAc (3.0 mL, 4 M) dropwise. At the end of the addition, the mixture was stirred at rt for 8.0 hrs. After the reaction was completed, the mixture was concentrated *in vacuo.* The residue was purified by beating in EtOAc (5.0 mL) and filtered to give the title compound as a pale yellow solid (259.3 mg, 90%), which was used for the next step directly. The compound was characterized by the following spectroscopic data:
MS (ESI, pos.ion) *m*/*z*: 623.5 [M+H]⁺.

### Step 3) the preparation of compound 21-3

To a suspension of compound **21-2** (261.25 mg, 0.34 mmol), compound **1-11-2** (125 mg, 0.714 mmol), EDCI (73 mg, 0.38 mmol) and HOAT (47 mg, 0.34 mmol) in DCM (8.0 mL) was added DIPEA (0.4 mL, 2.42 mmol) dropwise at 0 °C. At the end of the addition, the mixture was stirred at rt for 3.0 hrs. After the reaction was completed, the mixture was diluted with DCM (20 mL), washed with saturated NH₄Cl aqueous solution and brine, dried over anhydrous Na₂SO₄ and concentrated *in vacuo.* The residue was purified by silica gel column chromatography (DCM/MeOH (v/v) = 50/1) to give the title compound as a white solid (229.2 mg, 70%). The compound was characterized by the following spectroscopic data:
MS (ESI, pos.ion) *m*/*z*: 470.3 [M+2H]²⁺;
¹H NMR (400 MHz, CDCl₃) *δ* (ppm): 7.76-7.73 (m, 2H), 7.72-7.71 (m, 4H), 7.65-7.61 (m, 3H), 7.55-7.53 (dd, dd, 1H), 7.32 (s, 2H), 5.32, 5.29 (d, d, 2H), 5.04-4.99 (m, 2H), 4.42-4.37 (m, 2H), 3.92-3.85 (m, 2H), 3.63 (s, 6H), 3.61-3.54 (m, 2H), 2.84-2.80 (m, 2H), 2.43-2.09 (m, 12H), 1.76-1.58 (m, 6H), 0.97, 0.95 (m, m, 6H), 0.93-0.89 (m, 12H).

### Example 22

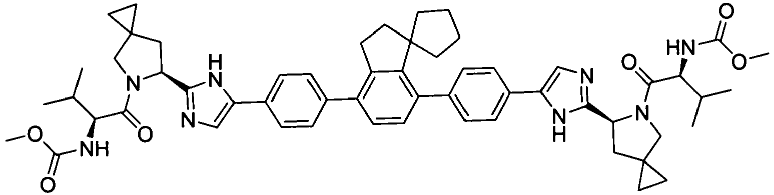

### Synthetic route:

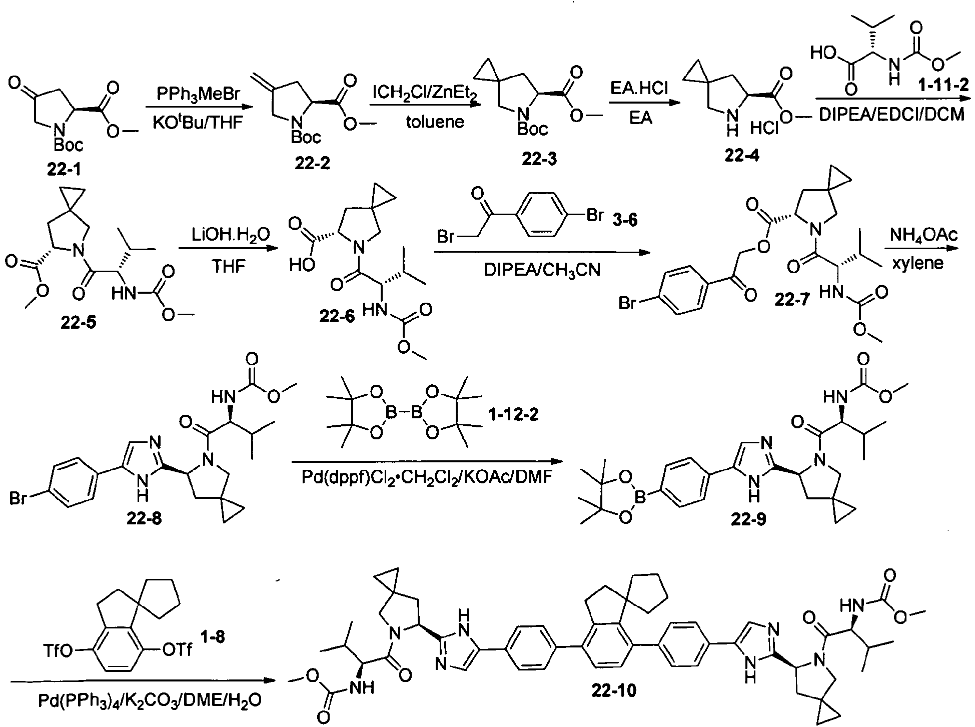

### Step 1) the preparation of compound 22-2

To a suspension of PPh₃MeBr (5.05 g, 14.2 mmol) in THF (50.0 mL) was added potassium *tert*-butanolate (14.9 mL, 14.9 mmol, 1.0 M in THF) dropwise at -20 °C. At the end of the addition, the mixture was heated to -5 °C and stirred for 30 mins, and then compound **22-1** (1.72 g, 7.07 mmol) was added. The mixture was stirred at rt for 1.0 hrs. After the reaction was completed, the mixture was quenched with ice water (50.0 mL), and the THF solvent was removed. The aqueous layer was extracted with EtOAc (50 mL x 3). The combined organic layers were washed with brine, dried over anhydrous Na₂SO₄ and concentrated *in vacuo.* The residue was purified by silica gel column chromatography (PE/EtOAc (v/v) = 5/1) to give the title compound as pale yellow oil (1.07 g, 62.9%). The compound was characterized by the following spectroscopic data:
MS (ESI, pos.ion) *m*/*z*: 242.12 [M+H]⁺;
¹H NMR (400 MHz, DMSO-*d₆*) *δ* (ppm): 5.01 (d, 2H, *J* = 10.8 Hz), 4.36 (t, 1H, *J* = 11.2 Hz), 3.95 (s, 2H), 3.64 (s, 3H), 3.01 (q, 1H, *J* = 14.6 Hz), 2.57-2.50 (m, 1H), 1.38 (s, 9H).

### Step 2) the preparation of compound 22-3

To a solution of diethylzinc (2.297 g, 18.60 mmol) in toluene (30.0 mL) was added chloroiodomethane (6.569 g, 37.24 mmol) dropwise at 0 °C. At the end of the addition, the mixture was stirred at 0 °C for 45 mins, and then a solution of compound 22-2 (1.5 g, 6.22 mmol) in toluene (15.0 mL) was added. The mixture was stirred at 0 °C for 18.0 hrs. After the reaction was completed, the mixture was quenched with saturated NH₄Cl aqueous solution (20.0 mL). The aqueous layer was extracted with EtOAc (25 mL x 3). The combined organic layers were dried over anhydrous Na₂SO₄ and concentrated *in vacuo.* The residue was purified by silica gel column chromatography (PE/EtOAc (v/v) = 10/1) to give the title compound as white liquid (0.58 g, 36.5%). The compound was characterized by the following spectroscopic data:
MS (ESI, pos.ion) *m*/*z:* 156.2 [M-Boc]⁺;
¹H NMR (400 MHz, CDCl₃) *δ* (ppm): 4.47 -4.33 (m, 1H), 3.71 (s, 3H), 3.37-3.29 (m, 2H), 2.25-2.17 (m, 1H), 1.86-1.75 (m, 1H), 1.44, 1.40 (s, s, 9H), 0.62-0.50 (m, 4H).

### Step 3) the preparation of compound 22-4

To a solution of compound **22-3** (0.69 g, 2.7 mmol) in EtOAc (6.0 mL) was added a solution of HCl in EtOAc (5.0 mL, 4 M) dropwise. At the end of the addition, the mixture was stirred at rt for 8.0 hrs. After the reaction was completed, the mixture was concentrated *in vacuo* to give the title compound as colorless oil (0.5 g, 96.5%). The compound was characterized by the following spectroscopic data:
MS (ESI, pos.ion) *m*/*z*: 156.2 [M+H]⁺;
¹H NMR (400 MHz, CD₃OD *δ* (ppm): 4.66-4.62 (m, 1H), 4.45-4.44 (m, 1H), 3.86 (s, 3H), 3.61-3.60 (m, 1H), 2.39-2.34 (m, 1H), 2.19-2.14 (m, 1H), 1.49-1.46 (m, 1H), 1.19-1.16 (m, 1H), 0.88-0.87 (m, 1H),0.81-0.79 (m, 1H).

### Step 4) the preparation of compound 22-5

To a suspension of compound **22-4** (0.53 g, 2.77 mmol), compound **1-11-2** (0.729 g, 4.16 mmol) and EDCI (1.063 g, 5.55 mmol) in DCM (10.0 mL) was added DIPEA (2.4 mL, 14.52 mmol) dropwise at 0 °C. At the end of the addition, the mixture was stirred at rt for 3.0 hrs. After the reaction was completed, the mixture was diluted with DCM (20.0 mL), washed with NH₄Cl aqueous solution and brine, dried over anhydrous Na₂SO₄ and concentrated *in vacuo.* The residue was purified by silica gel column chromatography (PE/EtOAc (v/v) = 1/1) to give the title compound as white liquid (0.6067 g, 70.2%). The compound was characterized by the following spectroscopic data:
MS (ESI, pos.ion) *m*/*z*: 313.2 [M+H]⁺;
¹H NMR (400 MHz, CDCl₃ *δ* (ppm): 5.44-5.42 (br, 1H), 4.71-4.68 (m, 1H), 4.29-4.20 (m, 1H), 3.73 (s, 3H), 3.72-3.69 (m, 1H), 3.67 (s, 3H), 3.59-3.54 (m, 1H), 2.20-2.15 (m, 1H), 2.06-2.01 (m, 1H), 1.95-1.90 (m, 1H), 1.05-0.93 (m, 6H), 0.66-0.61 (m, 4H).

### Step 5) the preparation of compound 22-6

To a solution of compound **22-5** (0.20 g, 0.64 mmol) in THF (5.0 mL) was added lithium hydroxide aqueous solution (0.1346 g, 3.2 mmol, 5.0 mL) dropwise at 0 °C. At the end of the addition, the mixture was stirred at 40 °C for 12.0 hrs. After the reaction was completed, the THF solvent was removed and 30 mL of water was added. The resulting mixture was extracted with EtOAc (10 mL x 3). The aqueous layer was adjusted to pH 1 with hydrochloric acid (10%), and extracted with EtOAc (25 mL x 3). The combined organic layers were dried over anhydrous Na₂SO₄ and concentrated *in vacuo* to give the title compound as a white solid (0.1581 g, 82.8%). The compound was characterized by the following spectroscopic data:
MS (ESI, pos.ion) *m*/*z*: 299.2 [M+H]⁺;
¹H NMR (400 MHz, CDCl₃) *δ* (ppm): 7.06 (br, 1H), 5.76 (br, 1H), 4.73-4.69 (m, 1H), 4.23-4.18 (m, 1H), 3.79 (d, 1H, *J* = 9.7 Hz), 3.66 (s, 3H), 3.49 (d, 1H, *J* = 9.7 Hz), 2.26-2.18 (m, 1H), 2.07-1.93 (m, 2H), 1.00-0.94 (m, 6H), 0.68-0.64 (m, 4H).

### Step 6) the preparation of compound 22-7

To a solution of compound **3-6** (308 mg, 1.1074 mmol), compound **22-6** (300 mg, 1.0067 mmol) in MeCN (30.0 mL) was added DIPEA (0.21 mL, 1.27 mmol) dropwise at 0 °C. At the end of the addition, the mixture was stirred at rt for 2.0 hrs. After the reaction was completed, 20 mL of water was added and the MeCN solvent was removed. The residue was dissolved in EtOAc (30.0 mL), washed with NH₄Cl aqueous solution and brine, dried over anhydrous Na₂SO₄ and concentrated *in vacuo.* The residue was purified by silica gel column chromatography (PE/EtOAc (v/v) = 1/1) to give the title compound as a pale yellow solid (331.7 mg, 66.7%). The compound was characterized by the following spectroscopic data:
MS (ESI, pos.ion) *m*/z: 495.3 [M+H]⁺;
¹H NMR (400 MHz, CDCl₃) *δ* (ppm): 7.82-7.78 (m, 2H), 7.67-7.64 (m, 2H), 5.32, 5.29 (br, br, 1H), 5.31 (s, 2H), 4.72-4.70 (m, 1H), 4.35-4.30 (m, 1H), 3.67 (s, 3H), 3.61-3.59 (m, 1H), 3.55-3.49 (m, 1H), 2.20-2.07 (m, 2H), 1.83-1.76 (m, 1H), 0.97, 0.96 (m, m, 3H), 0.91, 0.89 (m, m, 3H), 0.52-0.39 (m, 4H).

### Step 7) the preparation of compound 22-8

A mixture of compound **22-7** (331.7 mg, 0.6714 mmol) and NH₄OAc (1.035 g, 13.43 mmol) in xylene (10.0 mL) was stirred at 120 °C for 5.0 hrs. After the reaction was completed, the mixture was cooled to rt and quenched with water (20 mL). The aqueous layer was extracted with EtOAc (20.0 mL x 3). The combined organic layers were washed with brine, dried over anhydrous Na₂SO₄ and concentrated *in vacuo.* The residue was purified by silica gel column chromatography (PE/EtOAc (v/v) = 1/2) to give the title compound as a yellow solid (187.6 mg, 58.94%). The compound was characterized by the following spectroscopic data:
MS (ESI, pos.ion) *m*/*z:* 475.3 [M+H]⁺;
¹H NMR (400 MHz, CDCl₃) *δ* (ppm): 7.58 (s, 1H), 7.45-7.41 (m, 2H), 7.29-7.26 (m, 2H), 5.46, 5.44 (br, br, 1H), 4.93-4.89 (m, 1H), 4.41-4.37 (m, 1H), 3.71-3.67 (m, 1H), 3.67 (s, 3H), 3.50-3.44 (m, 1H), 2.39-2.32 (m, 1H), 2.23-2.11 (m, 1H), 2.05-1.97 (m, 1H), 0.97, 0.95 (m, m, 3H), 0.91, 0.89 (m, m, 3H), 0.52-0.39 (m, 4H).

### Step 8) the preparation of compound 22-9

To a mixture of compound **22-8** (187.6 mg, 0.3957 mmol), compound **1-12-2** (150.75 mg, 0.5935 mmol), Pd(dppf)Cl₂CH₂Cl₂ (33 mg, 0.03956 mmol) and KOAc (116.45 mg, 1.187 mmol) was added DMF (5.0 mL) via syringe under N₂, and the mixture was stirred at 90°C for 3.0 hrs. After the reaction was completed, the mixture was cooled to rt, diluted with EtOAc (50 mL) and filtered through a celite pad. The filtrate was washed with water (20.0 mL x 3) and brine, dried over anhydrous Na₂SO₄ and concentrated *in vacuo.* The residue was purified by silica gel column chromatography (PE/EtOAc (v/v) = 1/2) to give the title compound as a beige solid (165.34 mg, 80%). The compound was characterized by the following spectroscopic data:
MS (ESI, pos.ion) *m*/*z*: 523.3 [M+H]⁺;
¹H NMR (400 MHz, CDCl₃) *δ* (ppm): 7.64-7.57 (m, 4H), 7.21 (s, 1H), 5.46, 5.44 (br, br, 1H), 4.93-4.89 (m, 1 H), 4.42-4.37 (m, 1H), 3.71-3.67 (m, 1H), 3.66 (s, 3H), 3.50-3.44 (m, 1H), 2.39-2.32 (m, 1H), 2.23-2.11 (m, 1H), 2.05-1.97 (m, 1H), 1.35 (m, 6H), 1.32 (m, 6H), 0.97, 0.95 (m, m, 3H), 0.91, 0.89 (m, m, 3H), 0.55-0.42 (m, 4H).

### Step 9) the preparation of compound 22-10

To a mixture of compound **1-8** (244.3 mg, 0.522 mmol), compound **22-9** (572.5 mg, 1.096 mmol), Pd(PPh₃)₄ (60.29 mg, 0.0522 mmol) and K₂CO₃ (216 mg, 1.566 mmol) were added DME (6.0 mL) and water (1.5 mL) under N₂ via syringe, and the mixture was stirred at 90 °C for 3.0 hrs. After the reaction was completed, the mixture was cooled to rt and diluted with EtOAc (30.0 mL). The resulting mixture was washed with water (10 mL x 3) and brine, dried over anhydrous Na₂SO₄ and concentrated *in vacuo.* The residue was purified by silica gel column chromatography (DCM/EtOH (v/v) = 50/1) to give the title compound as a pale yellow solid (250.7 mg, 50%). The compound was characterized by the following spectroscopic data:
MS (ESI, pos.ion) *m*/*z*: 481.5 [M+2H]²⁺;
¹H NMR (400 MHz, CDCl₃) *δ* (ppm): 7.65-7.62 (m, 2H), 7.57 (s, 2H), 7.55-7.46 (m, 8H), 5.46, 5.44 (dd, dd, 2H), 4.93-4.89 (m, 2H), 4.42-4.37 (m, 2H), 3.71-3.67 (m, 2H), 3.66 (s, 6H), 3.50-3.44 (m, 2H), 2.84-2.80 (m, 2H), 2.43-2.32 (m, 4H), 2.30-2.11 (m, 6H), 2.05-1.97 (m, 2H), 1.76-1.58 (m, 4H), 0.97, 0.95 (m, m, 6H), 0.91, 0.89 (m, m, 6H), 0.55-0.42 (m, 8H).

### Example 23

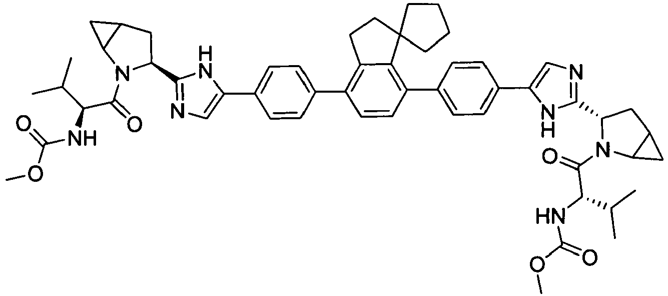

### Synthetic route:

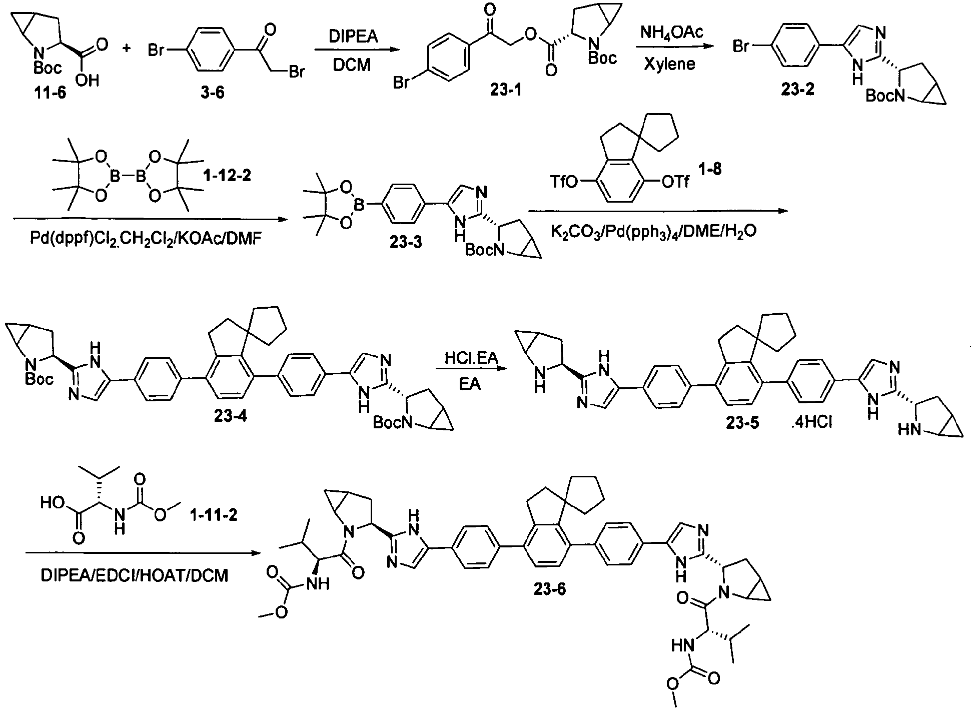

### Step 1) the preparation of compound 23-1

To a solution of compound **11-6** (3.911 g, 17.22 mmol) and compound **3-6** (5.465 g, 19.81 mmol) in DCM (60 mL) was added DIPEA (3.4 mL, 20.67 mmol) dropwise at 0 °C. At the end of the addition, the mixture was stirred at rt for 3.0 hrs. After the reaction was completed, the mixture was quenched with water (50 mL). The aqueous layer was extracted with DCM (100 mL x 3). The combined organic layers were washed with brine, dried over anhydrous Na₂SO₄ and concentrated *in vacuo.* The residue was purified by silica gel column chromatography (PE/EtOAc (v/v) = 5/1) to give the title compound as a white solid (4.5 g, 61.73%). The compound was characterized by the following spectroscopic data:
MS (ESI, pos.ion) *m*/*z*: 425.3 [M+H]⁺;
¹H NMR (400 MHz, CDCl₃) *δ* (ppm): 7.77-7.73 (m, 2H), 7.64-7.62 (m, 2H), 5.53-5.09 (m, 2H), 4.78-4.67 (m, 1H), 3.59-3.46 (m, 1H), 2.69-2.62 (m, 1H), 2.43-2.40 (m, 1H), 1.42 (s, 9H), 1.00-0.96 (m, 1H), 0.76-0.69 (m, 2H).

### Step 2) the preparation of compound 23-2

A suspension of compound **23-1** (4.5 g, 10.64 mmol) and ammonium acetate_(16.4 g, 212.73 mmol) in xylene (50 mL) was stirred at 120 °C for 5.0 hrs. After the reaction was completed, the mixture was cooled to rt, and quenched with water (50 mL). The aqueous layer was extracted with EtOAc (100 mL x 3). The combined organic layers were washed with brine, dried over anhydrous Na₂SO₄ and concentrated *in vacuo.* The residue was purified by silica gel column chromatography (PE/EtOAc (v/v) = 8/1) to give the title compound (2.144 g, 50%). The compound was characterized by the following spectroscopic data:
MS (ESI, pos.ion) *m*/*z:* 404.3 [M+H]⁺;
¹H NMR (400 MHz, CDCl₃) *δ* (ppm): 7.62-7.52 (br, 2H), 7.49-7.46 (d, 2H, *J =* 12 Hz), 7.21 (s, 1H), 5.27-5.24 (d, 1H, *J=* 10.0 Hz), 3.31-3.27 (m, 1H), 1.71-1.67 (m, 2H), 1.52 (s, 9H), 0.89-0.86 (m, 1H), 0.64-0.69 (m, 2H).

### Step 3) the preparation of compound 23-3

A mixture of compound **23-2** (2.1 g, 5.2 mmol), compound **1-12-2** (1.59 g, 6.25 mmol), Pd(dppf)Cl₂CH₂Cl₂ (425 mg, 0.52 mmol) and KOAc (1.54 g, 15.63 mmol) in DMF (30 mL) was stirred at 90 °C under N₂ for 3.0 hrs. After the reaction was completed, the mixture was cooled to rt, diluted with EtOAc (100 mL) and filtered through a celite pad. The filtrate was washed with water (60 mL x 3) and brine, dried over anhydrous Na₂SO₄ and *concentrated in vacuo.* The residue was purified by silica gel column chromatography (PE/EtOAc (v/v) = 2/1) to give the title compound (2.27 g, 97%). The compound was characterized by the following spectroscopic data:
MS (ESI, pos.ion) *m*/*z*: 452.3 [M+H]⁺;
¹H NMR (400 MHz, CDCl₃) *δ* (ppm): 7.81-7.79 (d, 2H, *J* = 8.04 Hz), 7.60 (br, 2H), 7.26 (s, 1H), 5.28-5.26 (d, 1H, *J* = 8.0 Hz), 3.53 (br, 1H), 3.30-3.27 (br, 1H), 1.67-1.66 (m, 2H), 1.52 (s, 9H), 1.34 (s, 12H), 0.89-0.86 (m, 1H), 0.69-0.64 (m, 2H).

### Step 4) the preparation of compound 23-4

A suspension of compound **1-8** (1.59 g, 3.4 mmol), compound **23-3** (3.33 g, 7.38 mmol), Pd(PPh₃)₄ (196.7 mg, 0.17 mmol) and K₂CO₃ (1.412 g, 10.22 mmol) in mixed solvents of DME and H₂O (15.0 mL, v/v = 4/1) was stirred at 90 °C under N₂ for 3.0 hrs. After the reaction was completed, the mixture was cooled to rt and diluted with EtOAc (100 mL). The resulting mixture was washed with water (50.0 mL x 3) and brine, dried over anhydrous Na₂SO₄ and concentrated *in vacuo.* The residue was purified by silica gel column chromatography (DCM/MeOH (v/v) = 100/1) to give the title compound as a pale yellow solid (1.67 g, 60%). The compound was characterized by the following spectroscopic data:
MS (ESI, pos.ion) *m*/*z*: 819.5 [M+H]⁺;
¹H NMR (400 MHz, CDCl₃) *δ* (ppm): 7.65-7.62 (m, 3H), 7.55-7.46 (m, 9H), 4.75-4.72 (m, 2H), 3.29-3.23 (m, 2H), 2.84-2.80 (m, 2H), 2.43-2.34 (m, 4H), 2.30-2.20 (m, 4H), 1.97-1.92 (m, 4H), 1.76-1.58 (m, 4H), 1.46 (s, 9H), 1.43 (s, 9H), 1.42-1.34 (m, 4H), 1.02-0.95 (m, 2H).

### Step 5) the preparation of compound 23-5

To a solution of compound **23-4** (800.4 mg, 0.978 mmol) in EtOAc (10.0 mL) was added a solution of HCl in EtOAc (5.0 mL, 4 M) dropwise. At the end of the addition, the mixture was stirred at rt for 8.0 hrs. After the reaction was completed, the mixture was concentrated *in vacuo.* The residue was purified by beating in EtOAc (10.0 mL) and filtered to give the title compound as a pale yellow solid (710 mg, 95%). The compound was characterized by the following spectroscopic data:
MS (ESI, pos.ion) *m*/*z*: 619.5 [M+H]⁺.

### Step 6) the preparation of compound 23-6

To a suspension of compound **23-5** (515.2 mg, 0.674 mmol), compound **1-11-2** (235.8 mg, 1.35 mmol), EDCI (271.3 mg, 1.415 mmol) and HOAT (137.58 mg, 1.01 mmol) in DCM (20.0 mL) was added DIPEA (0.93 mL, 5.63 mmol) dropwise at 0 °C. At the end of the addition, the mixture was stirred at rt for 3.0 hrs. After the reaction was completed, the mixture was diluted with DCM (30.0 mL), washed with NH₄Cl aqueous solution and brine, dried over anhydrous Na₂SO₄ and concentrated *in vacuo.* The residue was purified by silica gel column chromatography (DCM/MeOH (v/v) = 60/1) to give the title compound as a white solid (314.2 mg, 50%). The compound was characterized by the following spectroscopic data:
MS (ESI, pos.ion) *m*/*z*: 467.5 [M+2H]²⁺;
¹H NMR (400 MHz, CDCl₃) *δ* (ppm): 7.65-7.62 (m, 5H), 7.55-7.46 (m, 7H), 5.32, 5.29 (d, d, 2H), 4.89-4.85 (m, 2H), 4.09-4.04 (m, 2H), 3.63 (s, 6H), 3.45-3.38 (m, 2H), 2.84-2.80 (m, 2H), 2.46-2.35 (m, 4H), 2.30-2.09 (m, 6H), 2.00-1.94 (m, 2H), 1.76-1.58 (m, 4H), 1.43-1.36 (m, 2H), 0.97, 0.95 (m, m, 6H), 0.94-0.89 (m, 8H), 0.50-0.46 (m, 2H).

### Example 24

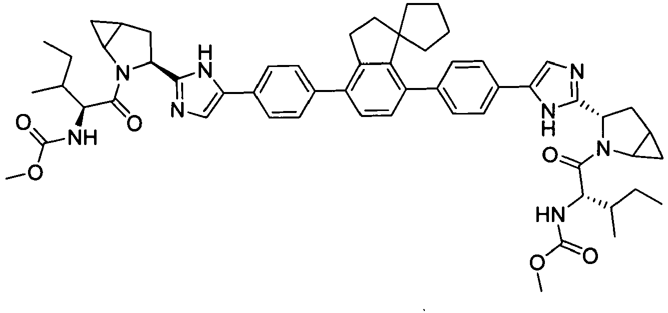

### Synthetic route:

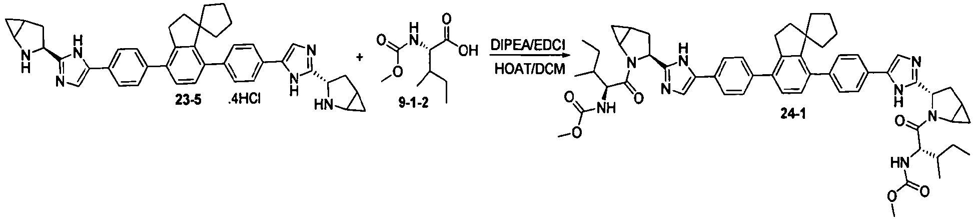

### Step 1) the preparation of compound 24-1

To a suspension of compound **23-5** (515.2 mg, 0.674 mmol), compound **9-1-2** (255.3 mg, 1.35 mmol), EDCI (271.3 mg, 1.415 mmol) and HOAT (137.58 mg, 1.01 mmol) in DCM (20.0 mL) was added DIPEA (0.93 mL, 5.63 mmol) dropwise at 0 °C. At the end of the addition, the mixture was stirred at rt for 3.0 hrs. After the reaction was completed, the mixture was diluted with DCM (30.0 mL), washed with NH₄Cl aqueous solution and brine, dried over anhydrous Na₂SO₄ and *concentrated in vacuo.* The residue was purified by silica gel column chromatography (DCM/MeOH (v/v) = 50/1) to give the title compound as a white solid (323.7 mg, 50%). The compound was characterized by the following spectroscopic data:
MS (ESI, pos.ion) *m*/*z*: 481.3 [M+2H]²⁺;
¹H NMR (400 MHz, CDCl₃) *δ* (ppm): 7.65-7.62 (m, 4H), 7.55-7.46 (m, 8H), 5.39, 5.36 (d, d, 1H), 5.28, 5.25 (d, d, 1H), 4.85-4.82 (m, 2H), 4.09-4.04 (m, 2H), 3.63 (s, 6H), 3.37-3.31 (m, 2H), 2.84-2.80 (m, 2H), 2.44-2.35 (m, 4H), 2.30-2.10 (m, 5H), 1.97-1.91 (m, 2H), 1.83-1.52 (m, 7H), 1.47-1.40 (m, 2H), 1.26-1.13 (m, 2H), 1.00-0.82 (m, 14H), 0.50-0.47 (m, 2H).

### Example 25

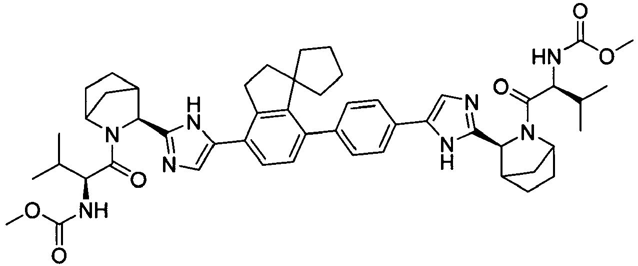

### Synthetic route:

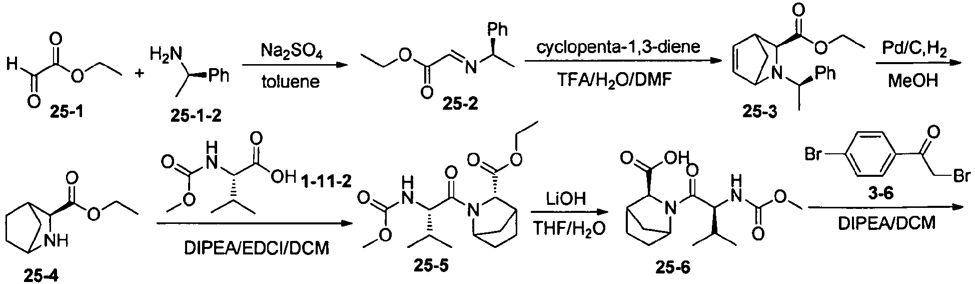

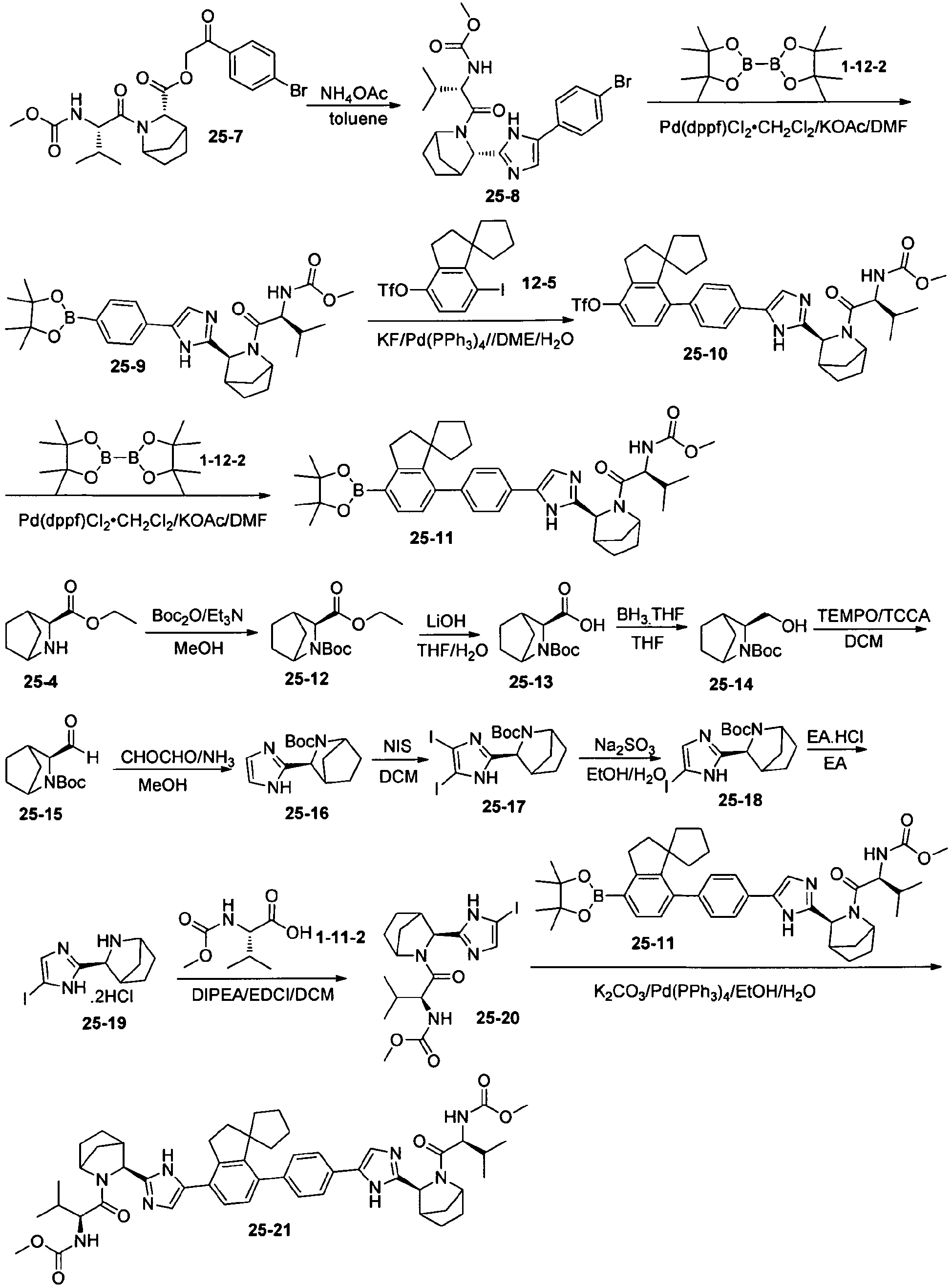

### Step 1) the preparation of compound 25-2

To a solution of (*R*)-1-phenylethylamine (1.3 mL, 10.1 mmol) in toluene (15 mL) was added anhydrous Na₂SO₄ (3.48 g, 24.5 mmol) dropwise at rt followed by ethyl glyoxalate (1.0 mL, 10.1 mmol). At the end of the addition, the mixture was stirred at rt for 1.0 hr. After the reaction was completed, the mixture was filtered. The filtrate was *concentrated in vacuo* to give the title compound as yellow liquid (1.9 g, 91.8%), which was used for the next step without further purification.

### Step 2) the preparation of compound 25-3

To a solution of compound **25-2** (2.0 g, 9.7 mmol) in DMF (15 mL) was added TFA (0.75 mL, 10.1 mmol). After stirring for 10 mins, to the mixture were added fresh 1,3-cyclopentadiene (1.29 g, 19.5 mmol) and two drops of water in turn. The reaction mixture was stirred at rt for 12 hrs. After the reaction was completed, the DMF solvent was removed and to the residue was added NaHCO₃ aqueous solution (20 mL, 10%). The mixture was adjusted to pH 8 with Na₂CO₃ and extracted with PE (25 mL x 3). The combined organic layers were washed with brine, dried over anhydrous Na₂SO₄ and *concentrated in vacuo.* The residue was purified by silica gel column chromatography (PE/EtOAc (v/v) = 10/1) to give the title compound as pale yellow liquid (2.38 g, 90.0%). The compound was characterized by the following spectroscopic data:
¹H NMR (400 MHz, CDCl₃) *δ* (ppm): 7.35-7.17 (m, 5H), 6.42 (br, 1H), 6.28-6.26 (br, 1H), 4.34-4.30 (m, 2H), 3.82-3.78 (m, 2H), 3.04-3.02 (m, 1H), 2.90 (br, 1H), 2.20 (br, 1H), 2.13 (m, 1H), 1.41 (d, 3H, *J=* 6.6 Hz), 0.95 (t, 3H, *J=* 7.2 Hz).

### Step 3) the preparation of compound 25-4

To a solution of compound **25-3** (2.0 g, 7.37 mmol) in MeOH (60 mL) was added Pd/C (200 mg). The mixture was stirred at rt under 20 atm of H₂ gas for 24 hrs. After the reaction was completed, the mixture was filtered. The filtrate was concentrated *in vacuo* to give the title compound as yellow liquid (1.2 g, 96.2%). The compound was characterized by the following spectroscopic data:
MS (ESI, pos.ion) *m*/*z*: 170.2 [M+H]⁺;
¹H NMR (400 MHz, CDCl₃) *δ* (ppm): 4.21-4.15 (m, 2H), 3.55 (br, 1H), 3.33 (br, 1H), 2.63 (br, 1H), 2.32 (br, 1H), 1.64-1.60 (m, 2H), 1.53-1.47 (m, 2H), 1.42-1.36 (m, 2H), 1.28 (t, 3H, *J* = 7.1 Hz).

### Step 4) the preparation of compound 25-5

To a suspension of compound **25-4** (0.68 g, 4.02 mmol), compound **1-11-2** (1.057 g, 6.03 mmol) and EDCI (1.543 g, 8.05 mmol) in DCM (25 mL) was added DIPEA (2.1 mL, 12.7 mmol) dropwise at 0 °C. At the end of the addition, the mixture was stirred at rt overnight. After the reaction was completed, the mixture was quenched with water (30 mL). The aqueous layer was extracted with DCM (35 mL x 3). The combined organic layers were washed with brine, dried over anhydrous Na₂SO₄ and concentrated *in vacuo.* The residue was purified by silica gel column chromatography (PE/EtOAc (v/v) = 2/1) to give the title compound as a white solid (0.74 g, 56.4%). The compound was characterized by the following spectroscopic data:
MS (ESI, pos.ion) *m*/*z*: 170.2 [M+H]⁺;
¹H NMR (400 MHz, CDCl₃) *δ* (ppm): 5.44 (br, 1H), 4.40 (br, 1H), 4.33-4.30 (m, 1H), 4.19-4.14 (m, 2H), 4.02 (br, 1H), 3.66 (s, 3H), 2.74 (br, 1H), 2.04 (br, 1H), 1.91-1.88 (m, 2H), 1.80-1.74 (m, 2H), 1.56-1.54 (m, 1H), 1.43-1.38 (m, 1H), 1.26 (t, 3H, *J* = 7.1 Hz), 1.07 (d, 3H, *J =* 6.8 Hz), 0.97 (d, 3H, *J* = 6.8 Hz).

### Step 5) the preparation of compound 25-6

To a solution of compound **25-5** (0.74 g, 2.27 mmol) in THF (25 mL) was added lithium hydroxide monohydrate aqueous solution (0.4767 g, 11.35 mmol, 10 mL) at 0 °C. At the end of the addition, the mixture was stirred at 40 °C for 12 hrs. After the reaction was completed, the THF solvent was removed and to the residue was added water (20 mL). The aqueous layer was washed with EtOAc (15 mL x 3) and separated. The aqueous phase was adjusted to pH 1 with hydrochloric acid (10%) and extracted with EtOAc (25 mL x 3). The combined organic layers were washed with brine, dried over anhydrous Na₂SO₄ and concentrated *in vacuo* to give the title compound as a white solid (0.55 g, 81.3%). The compound was characterized by the following spectroscopic data:
MS (ESI, pos.ion) *m*/*z*: 299.2 [M+H]⁺;
¹H NMR (400 MHz, CD₃OD) *δ* (ppm): 4.52 (br, 1H), 4.20 (d, 1H, *J =* 7.8 Hz), 3.93 (br, 1H), 3.63 (s, 3H), 2.73 (br, 1H), 2.01-1.98 (m, 4H), 1.85-1.75 (m, 2H), 1.54-1.46 (m, 2H), 1.05 (d, 3H, *J* = 6.8 Hz), 0.98 (d, 3H, *J=* 6.8 Hz).

### Step 6) the preparation of compound 25-7

To a mixture of compound **3-6** (308 mg, 1.1074 mmol) and compound **25-6** (300 mg, 1.0067 mmol) in DCM (30.0 mL) was added DIPEA (0.20 mL, 1.2081 mmol) dropwise at 0 °C. At the end of the addition, the mixture was stirred at rt for 3.0 hrs. After the reaction was completed, the DCM solvent was removed *in vacuo.* To the residue was added water (20 mL). The resulting mixture was extracted with EtOAc (30 mL x 3). The combined organic layers were washed with brine, dried over anhydrous Na₂SO₄ and *concentrated in vacuo.* The residue was purified by silica gel column chromatography (PE/EtOAc (v/v) = 1/2) to give the title compound as a pale yellow solid (332.6 mg, 66.7%). The compound was characterized by the following spectroscopic data:
MS (ESI, pos.ion) *m*/*z*: 495.3 [M+H]⁺;
¹H NMR (400 MHz, CDCl₃) *δ* (ppm): 7.75 (d, 2H, *J* = 8.52 Hz), 7.68 (d, 2H, *J* = 8.56 Hz), 5.45 (d, 1H, *J* = 9.4 Hz), 5.24 (d, 1H, *J* = 16.56 Hz), 4.55-4.59 (m, 1H), 3.67 (s, 3H), 3.57 (m, 1H), 2.73-2.65 (m, 2H), 2.27-2.19 (m, 1H), 2.04 (s, 1H), 1.84-1.77 (m, 2H), 1.49-1.46 (m, 1H), 1.27-1.24 (m, 1H), 1.08-1.07 (br, 1H), 1.05-1.03 (m, 1H), 0.91-0.89 (m, 6H).

### Step 7) the preparation of compound 25-8

To a suspension of compound **25-7** (332.6 mg, 0.6714 mmol) and NH₄OAc (1.035 g, 13.43 mmol) in toluene (8.0 mL) was stirred at 110 °C for 5.0 hrs. After the reaction was completed, the mixture was cooled to rt and quenched with water (20 mL). The aqueous layer was extracted with EtOAc (20 mL x 3). The combined organic layers were washed with brine, dried over anhydrous Na₂SO₄ and concentrated *in vacuo.* The residue was purified by silica gel column chromatography (PE/EtOAc (v/v) = 1/2) to give the title compound as a yellow solid (188 mg, 58.94%). The compound was characterized by the following spectroscopic data:
MS (ESI, pos.ion) *m*/*z*: 476.3 [M+H]⁺;
¹H NMR (400 MHz, CDCl₃) *δ* (ppm): 10.35 (s, 1H), 7.64-7.62 (d, 2H, *J* = 8.52 Hz,), 7.55-7.45 (d, 2H, *J =* 1.84 Hz), 7.16 (s, 1H), 5.54-5.46 (br, 2H), 4.57-4.53 (m, 1H), 3.70 (s, 3H), 3.58 (m, 1H), 2.69 (m, 1H), 2.54-2.48 (m, 1H), 1.87-1.76 (m, 4H), 1.47-1.45 (m, 2H), 0.85-0.81 (m, 6H).

### Step 8) the preparation of compound 25-9

To a mixture of compound **25-8** (188.1 mg, 0.3957 mmol), compound **1-12-2** (150.75 mg, 0.5935 mmol), Pd(dppf)Cl₂·CH₂Cl₂ (33 mg, 0.03956 mmol) and KOAc (116.45 mg, 1.187 mmol) was added DMF (5.0 mL) via syringe under N₂, and the mixture was stirred at 90 °C for 3.0 hrs. After the reaction was completed, the mixture was cooled to rt, diluted with EtOAc (50 mL) and filtered through a celite pad. The filtrate was washed with water (20 mL x 3) and brine, dried over anhydrous Na₂SO₄ and concentrated *in vacuo.* The residue was purified by silica gel column chromatography (PE/EtOAc (v/v) = 1/2) to give the title compound as a beige solid (165.38 mg, 80%). The compound was characterized by the following spectroscopic data:
MS (ESI, pos.ion) *m*/*z*: 523.3 [M+H]⁺;
¹H NMR (400 MHz, CDCl₃) *δ* (ppm): 10.48 (s, 1H), 7.81-7.75 (m, 4H), 7.43-7.41 (d, 1H, *J=* 8.0 Hz), 5.49-5.39 (m, 2H), 4.58-4.53 (m, 2H), 3.67 (s, 3H), 3.57 (m, 1H), 2.65 (m, 1H), 2.54-2.47 (m, 1H), 2.10-2.04 (m, 2H), 1.83-1.79 (m, 1H), 1.49-1.46 (m, 2H), 1.38 (s, 12H), 0.85-0.81 (m, 6H).

### Step 9) the preparation of compound 25-10

To a mixture of compound **25-9** (1.37 g, 2.62 mmol), compound **12-5** (1.17 g, 2.62 mmol), Pd(PPh₃)₄ (120 mg, 0.1 mmol) and KF (0.30 g, 5.24 mmol) were added DME (12.0 mL) and pure water (3.0 mL) via syringe under N₂, and the mixture was stirred at 90 °C for 2.0 hrs. After the reaction was completed, the mixture was cooled to rt and diluted with EtOAc (100 mL). The resulting mixture was washed with water (30 mL x 3) and brine, dried over anhydrous Na₂SO₄ and concentrated *in vacuo.* The residue was purified by silica gel column chromatography (DCM/MeOH (v/v) = 100/1) to give the title compound as a white solid (1.12 g, 60%). The compound was characterized by the following spectroscopic data:
MS (ESI, pos.ion) *m*/*z:* 715.3 [M+H]⁺;
¹H NMR (400 MHz, CDCl₃) *δ* (ppm): 7.60 (s, 1H), 7.55-7.51 (m, 2H), 7.45-7.42 (m, 2H), 7.27, 7.25 (s, s, 1H), 6.97, 6.94 (dd, dd, 1H), 5.56, 5.55 (d, d, 1H), 5.06-5.02 (m, 1H), 4.80-4.75 (m, 1H), 4.21-4.17 (m, 1H), 3.66 (s, 3H), 3.00-2.96 (m, 2H), 2.54-2.50 (m, 1H), 2.42-2.34 (m, 2H), 2.29-2.21 (m, 4H), 2.20-2.15 (m, 1H), 2.08-2.00 (m, 1H), 1.83-1.78 (m, 1H), 1.76-1.55 (m, 7H), 1.47-1.37 (m, 1H), 1.02, 1.00 (m, m, 3H), 0.94, 0.92 (m, m, 3H).

### Step 10) the preparation of compound 25-11

A mixture of compound **25-10** (1.15 g, 1.61 mmol), compound **1-12-2** (0.45 g, 1.77 mmol), Pd(dppf)Cl₂·CH₂Cl₂ (80 mg, 0.096 mmol) and KOAc (0.4 g, 4.02 mmol) in DMF (10 mL) was stirred at 90 °C under N₂ for 4.0 hrs. After the reaction was completed, the mixture was cooled to rt, diluted with EtOAc (80 mL) and filtered through a celite pad. The filtrate was washed with water (30 mL x 3) and brine, dried over anhydrous Na₂SO₄ and concentrated *in vacuo.* The residue was purified by silica gel column chromatography (PE/EtOAc (v/v) = 3/1) to give the title compound as a white solid (780 mg, 70%). The compound was characterized by the following spectroscopic data:
MS (ESI, pos.ion) *m*/*z*: 693.5 [M+H]⁺;
¹H NMR (400 MHz, CDCl₃) *δ* (ppm): 7.60 (s, 1H), 7.59, 7.57 (dd, dd, 1H), 7.55-7.52 (m, 2H), 7.50-7.46 (m, 2H), 7.45, 7.44 (s, s, 1H), 5.56, 5.55 (d, d, 1H), 5.06-5.02 (m, 1H), 4.80-4.75 (m, 1H), 4.21-4.17 (m, 1H), 3.66 (s, 3H), 2.94-2.90 (m, 2H), 2.55-2.50 (m, 1H), 2.41-2.33 (m, 2H), 2.30-2.24 (m, 2H), 2.23-2.16 (m, 1H), 2.08-2.00 (m, 1H), 1.99-1.90 (m, 2H), 1.84-1.55 (m, 8H), 1.47-1.37 (m, 1H), 1.32 (m, 6H), 1.29 (m, 6H), 1.02, 1.01 (m, m, 3H), 0.93, 0.92 (m, m, 3H).

### Step 11) the preparation of compound 25-12

To a solution of compound **25-4** (1.69 g, 10 mmol) in MeOH (20 mL) was added Et₃N (1.67 mL, 12 mmol) dropwise at 0 °C followed by Boc₂O (2.41 mL, 10.5 mmol). At the end of the addition, the mixture was stirred at rt overnight. After the reaction was completed, the MeOH solvent was removed *in vacuo.* The residue was dissolved in DCM (50 mL). The solution was washed with water (20 mL x 3) and brine, dried over anhydrous Na₂SO₄ and concentrated *in vacuo* to give the title compound as colorless slurry (2.37 g, 88%).

### Step 12) the preparation of compound 25-13

To a solution of compound **25-12** (611 mg, 2.27 mmol) in THF (25 mL) was added lithium hydroxide monohydrate aqueous solution (476.7 mg, 11.35 mmol, 10 mL) at 0 °C, and the mixture was stirred at 40 °C for 12 hrs. After the reaction was completed, the THF solvent was removed and to the residue was added water (50 mL). The aqueous layer was washed with EtOAc (25 mL x 3) and separated. Then the aqueous phase was adjusted to pH 1 with hydrochloric acid (10%) and extracted with EtOAc (25 mL x 3). The combined organic layers were washed with brine, dried over anhydrous Na₂SO₄ and concentrated *in vacuo* to give the title compound as a white solid (438 mg, 80%). The compound was characterized by the following spectroscopic data:
MS (ESI, pos.ion) *m*/*z*: 242.3 [M+H]⁺.

### Step 13) the preparation of compound 25-14

To a solution of compound **25-13** (1.34 g, 5.57 mmol) in THF (20 mL) was added diborane (8.3 mL, 1M in THF) dropwise at 0 °C. At the end of the addition, the mixture was stirred at rt for 2.0 hrs. After the reaction was completed, the mixture was quenched with MeOH (4.0 mL), and concentrated *in vacuo.* The residue was dissolved in DCM (50 mL). The solution was washed with water (20 mL x 3) and brine, dried over anhydrous Na₂SO₄ and concentrated *in vacuo* to give the title compound as colorless slurry (1.01 g, 80%). The compound was characterized by the following spectroscopic data:
MS (ESI, pos.ion) *m*/*z*: 228.3 [M+H]⁺.

### Step 14) the preparation of compound 25-16

To a solution of compound **25-14** (1.19 g, 5.24 mmol) in DCM (20 mL) at 0 °C was added TCCA (1.22 g, 5.24 mmol) dropwise followed by a solution of TEMPO (82 mg, 0.52 mmol) in DCM (3.0 mL). The mixture was stirred at 0 °C for 1.0 hr and at rt for another 1.0 hr. After the reaction was completed, the mixture was filtered. The filtrate was washed with saturated Na₂SO₃ aqueous solution (20 mL x 3), dried over anhydrous Na₂SO₄ and concentrated *in vacuo.* The residue was dissolved in a solution of NH₃ in MeOH (7.0 mL, 7 M). The solution was stirred at 0 °C for 0.5 hr and at rt for another 1.0 hr. Glyoxal (1.1 mL, 40%) was added to the mixture dropwise at 0 °C. At the end of the addition, the mixture was stirred at rt for 24 hrs. After the reaction was completed, the mixture was concentrated *in vacuo.* The residue was dissolved in DCM (60 mL). The solution was washed with water (20 mL x 3) and brine, dried over anhydrous Na₂SO₄ and concentrated *in vacuo.* The residue was purified by silica gel column chromatography (PE/EtOAc (v/v) = 1/1) to give the title compound as a pale yellow solid (590 mg, 50%). The compound was characterized by the following spectroscopic data:
MS (ESI, pos.ion) *m*/*z:* 264.3 [M+H]⁺;
¹H NMR (400 MHz, CDCl₃) *δ*(ppm): 6.99 (s, 2H), 4.88-4.85 (m, 1H), 4.49-4.44 (m, 1H), 2.57-2.53 (m, 1H), 2.04-1.96 (m, 1H), 1.89-1.84 (m, 1H), 1.74-1.62 (m, 2H), 1.59-1.46 (m, 2H), 1.44 (s, 9H).

### Step 15) the preparation of compound 25-17

To a solution of compound **25-16** (650 mg, 2.47 mmol) in DCM (8.0 mL) was added *N*-iodosuccinimide (1.23 g, 5.43 mmol) dropwise at 0 °C. At the end of the addition, the mixture was stirred at 0 °C for 2.0 hrs. After the reaction was completed, the mixture was diluted with DCM (30 mL) and filtered. The filtrate was washed with saturated Na₂SO₃ aqueous solution (20 mL x 3), dried over anhydrous Na₂SO₄ and concentrated *in vacuo* to give the title compound as a yellow solid (1.27 g), which was used for the next step with further purification. The compound was characterized by the following spectroscopic data:
MS (ESI, pos.ion) *m*/*z*: 516.3 [M+H]⁺;
¹H NMR (400 MHz, CDCl₃) *δ* (ppm): 5.33-5.30 (m, 1H), 4.39-4.35 (m, 1H), 2.76-2.72 (m, 1H), 2.04-1.92 (m, 2H), 1.83-1.71 (m, 2H), 1.61-1.51 (m, 2H), 1.44 (s, 9H).

### Step 16) the preparation of compound 25-18

To a solution of compound **25-17** (1.12g, 2.12 mmol) in mixed solvents of ethanol and water (12 mL, v/v = 1/1) was added Na₂SO₃ (2.14 g, 17 mmol), and the mixture was stirred at 90 °C for 30 hrs. After the reaction was completed, the mixture was filtered. The filtrate was concentrated *in vacuo.* The residue was dissolved in DCM (80 mL). The solution was washed with water (20 mL x 3) and brine, dried over anhydrous Na₂SO₄ and concentrated *in vacuo.* The residue was purified by silica gel column chromatography (PE/EtOAc (v/v) = 1/1) to give the title compound as a white solid (577.36 mg, 70%). The compound was characterized by the following spectroscopic data:
MS (ESI, pos.ion) *m*/*z*: 390.3 [M+H]⁺;
¹H NMR (400 MHz, CDCl₃) *δ* (ppm): 7. 36 (s, 1H), 4.81-4.78 (m, 1H), 4.47-4.43 (m, 1H), 2.65-2.61 (m, 1H), 2.04-1.96 (m, 1H), 1.93-1.88 (m, 1H), 1.79-1.67 (m, 2H), 1.59-1.48 (m, 2H), 1.44 (s, 9H).

### Step 17) the preparation of compound 25-19

To a solution of compound **25-18** (1.59 g, 4.1 mmol) in EtOAc (10 mL) was added a solution of HCl in EtOAc (10.0 mL, 4 M) dropwise. At the end of the addition, the mixture was stirred at rt overnight. After the reaction was completed, the mixture was concentrated *in vacuo.* The residue was purified by beating in EtOAc (10 mL) and filtered to give the title compound as a solid (1.276 g, 86%), which was used for the next step without further purification. The compound was characterized by the following spectroscopic data:
MS (ESI, pos.ion) *m*/*z:* 290.5 [M+H]⁺.

### Step 18) the preparation of compound 25-20

To a suspension of compound **25-19** (1.3 g, 3.6 mmol), compound **1-11-2** (0.69 g, 3.9 mmol) and EDCI (0.75 g, 3.9 mmol) in DCM (20 mL) was added DIPEA (2.38 mL, 14.4 mmol) dropwise at 0 °C. At the end of the addition, the mixture was stirred at rt for 2.0 hrs. After the reaction was completed, the mixture was diluted with DCM (40 mL). The resulting mixture was washed with saturated NH₄Cl aqueous solution, dried over anhydrous Na₂SO₄ and concentrated *in vacuo.* The residue was purified by silica gel column chromatography (PE/EtOAc (v/v) = 1/2) to give the title compound as a pale yellow solid (1.365 g, 85%). The compound was characterized by the following spectroscopic data:
MS (ESI, pos.ion) *m*/*z*: 447.3 [M+H]⁺;
¹H NMR (400 MHz, CDCl₃) *δ* (ppm): 7.33 (s, 1H), 5.46, 5.44 (d, d, 1H), 5.09-5.05 (m, 1H), 4.74-4.69 (m, 1H), 4.45-4.41 (m, 1H), 3.66 (s, 3H), 2.61-2.57 (m, 1H), 2.22-2.11 (m, 1H), 2.08-2.00 (m, 1H), 1.88-1.83 (m, 1H), 1.72-1.55 (m, 3H), 1.50-1.42 (m, 1H), 0.97-0.95 (m, m, 3H), 0.91, 0.89 (m, m, 3H).

### Step 19) the preparation of compound 25-21

A suspension of compound **25-11** (970 mg, 1.4 mmol), compound **25-20** (720 mg, 1.7 mmol), Pd(PPh₃)₄ (160 mg, 0.14 mmol) and K₂CO₃ (580 mg, 4.2 mmol) in mixed solvents of EtOH and H₂O (16 mL, v/v = 3/1) was stirred at 90 °C under N₂ for 3.0 hrs. After the reaction was completed, the mixture was diluted with EtOAc (40 mL). The resulting mixture was washed with brine, dried over anhydrous Na₂SO₄ and concentrated *in vacuo.* The residue was purified by recrystallization to give the title compound as a white solid (495.3 mg, 40%). The compound was characterized by the following spectroscopic data:
MS (ESI, pos.ion) *m*/*z*: 885.5 [M+H]⁺;
¹H NMR (400 MHz, CDCl₃) *δ* (ppm): 7.71 (s, 1H), 7.60 (s, 1H), 7.55-7.51 (m, 3H), 7.49-7.46 (m, 2H), 7.19, 7.16 (s, s, 1H), 5.56, 5.55 (d, d, 1H), 5.46, 5.44 (d, d, 1H), 5.06-5.02 (m, 1H), 5.01-4.97 (m, 1H), 4.80-4.75 (m, 1H), 4.45-4.41 (m, 1H), 4.21-4.17 (m, 1H), 3.66 (s, 6H), 2.85-2.81 (m, 2H), 2.55-2.45 (m, 2H), 2.37-2.23 (m, 5H), 2.22-2.10 (m, 2H), 2.08-2.00 (m, 2H), 1.94-1.86 (m, 4H), 1.83-1.78 (m, 2H), 1.76-1.55 (m, 8H), 1.47-1.36 (m, 2H), 1.02-0.89 (m, 12H).

### Example 26

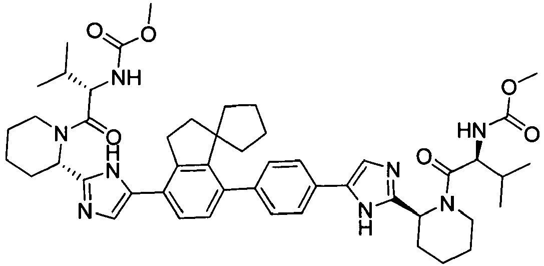

### Synthetic route:

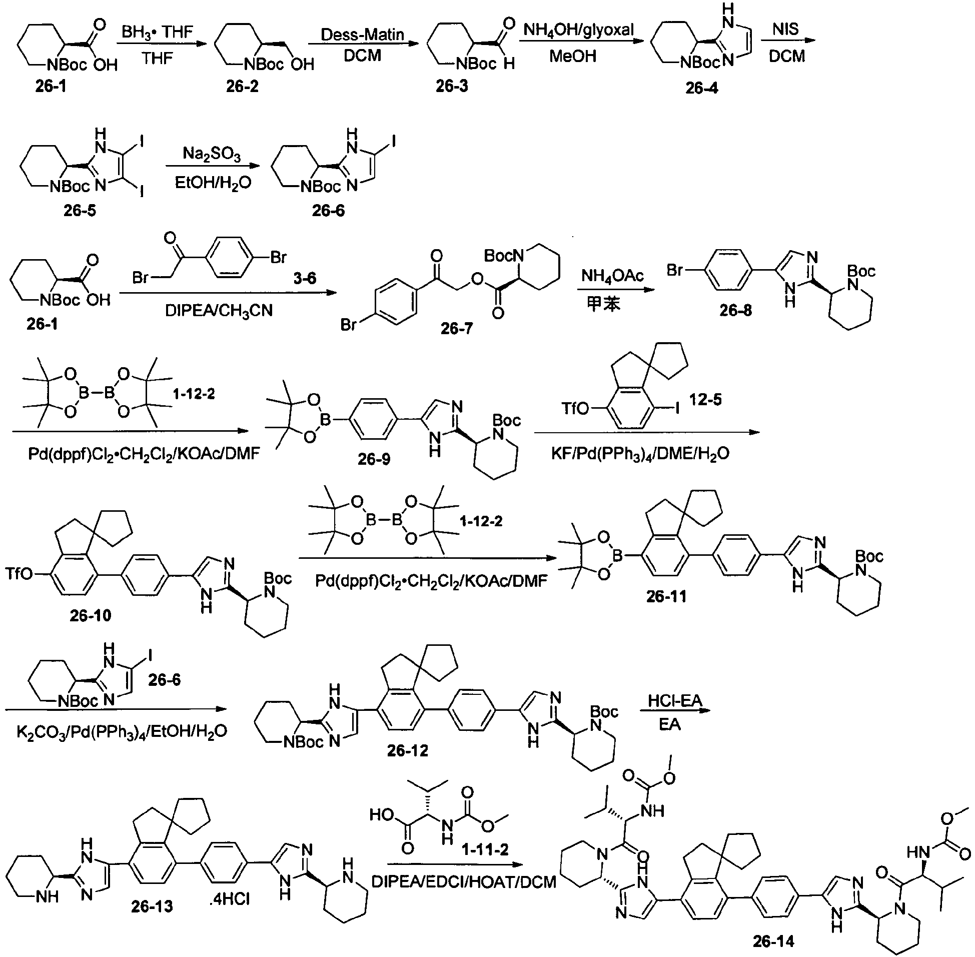

### Step 1) the preparation of compound 26-2

To a solution of compound **26-1** (10.68 g, 46.6 mmol) in THF (100 mL) was added diborane (100 mL, 1M in THF) dropwise at 0 °C under N₂. At the end of the addition, the mixture was stirred at 0 °C for 3.0 hrs. After the reaction was completed, the mixture was quenched with MeOH (80 mL) and concentrated *in vacuo.* The residue was purified by silica gel column chromatography (PE/EtOAc (v/v) = 4/1) to give the title compound as colorless oil (7.52 g, 75%). The compound was characterized by the following spectroscopic data:
¹H NMR (400 MHz, CDCl₃) *δ* (ppm): 4.20-4.11 (m, 1H), 3.91-3.85 (m, 2H), 3.72-3.65 (m, 1H), 3.34-3.26 (m, 1H), 2.69 (brs, 1H), 2.43-2.33 (m, 1H), 1.52-1.48 (m, 1H), 1.45 (s, 9H), 1.35-1.04 (m, 3H), 0.94-0.80 (m, 1H).

### Step 2) the preparation of compound 26-3

To a solution of compound **26-2** (7.49 g, 34.8 mmol) in DCM (250 mL) was added Dess-Martin periodinane (20.7 g, 48.8 mmol) in a portionwise manner at 0 °C. At the end of the addition, the mixture was stirred at rt for 2.0 hrs. After the reaction was completed, water (250 mL) was added to the mixture, and the resulting mixture was filtered. After the layers were partitioned, the organic layer was washed with brine, dried over anhydrous Na₂SO₄ and concentrated *in vacuo.* The residue was purified by silica gel column chromatography (PE/EtOAc (v/v) = 3/1) to give the title compound as colorless oil (3.71 g, 50%). The compound was characterized by the following spectroscopic data:
¹H NMR (400 MHz, CDCl₃) *δ* (ppm): 9.69-9.66 (m, 1H), 4.07-4.02 (m, 1H), 3.90-3.83 (m, 1H), 3.04-2.94 (m, 1H), 1.93-1.71 (m, 2H), 1.44 (s, 9H), 1.29-1.05 (m, 4H).

### Step 3) the preparation of compound 26-4

To a solution of compound **26-3** (3.75 g, 17.6 mmol) and ammonia (13.0 mL) in MeOH (30 mL) was added glyoxal (8.0 mL, 40% in H₂O) dropwise at 0 °C. At the end of the addition, the mixture was stirred at rt overnight. After the reaction was completed, the mixture was concentrated *in vacuo.* The residue was purified by silica gel column chromatography (PE/EtOAc (v/v) = 3/1) to give the title compound as a white solid (1.99 g, 45%). The compound was characterized by the following spectroscopic data:
MS (ESI, pos.ion) *m*/*z*: 252.5 [M+H]⁺;
¹H NMR (400 MHz, CDCl₃) *δ* (ppm): 6.9 9 (s, 2H), 4.91-4.84 (m, 1H), 4.06-3.98 (m, 1H), 2.97-2.88 (m, 1H), 2.11-2.02 (m, 1H), 1.86-1.74 (m, 1H), 1.73-1.65 (m, 1H), 1.63-1.52 (m, 1H), 1.50 (s, 9H), 1.25-1.03 (m, 2H).

### Step 4) the preparation of compound 26-5

To a solution of compound **26-4** (2.11 g, 8.4 mmol) in DCM (60 mL) was added *N*-iodosuccinimide (3.8 g, 16.8 mmol) at 0 °C in a portionwise manner, and the mixture was stirred at 0 °C for 1.5 hrs. After the reaction was completed, the mixture was washed with brine, dried over anhydrous Na₂SO₄ and concentrated *in vacuo.* The residue was purified by silica gel column chromatography (PE/EtOAc (v/v) = 3/1) to give the title compound as a white solid (2.66 g, 63%). The compound was characterized by the following spectroscopic data:
MS (ESI, pos.ion) *m*/*z*: 504.3 [M+H]⁺;
¹H NMR (400 MHz, CDCl₃) *δ* (ppm): 4.69-4.63 (m, 1H), 4.62-4.18 (m, 1H), 2.97-2.87 (m, 1H), 2.21-2.12 (m, 1H), 1.85-1.72 (m, 2H), 1.64-1.52 (m, 1H), 1.50 (s, 9H), 1.25-1.06 (m, 2H).

### Step 5) the preparation of compound 26-6

To a suspension of compound **26-5** (1.64 g, 3.27 mmol) in mixed solvents of ethanol and water (50 mL, v/v = 3/7) was added Na₂SO₃ (3.7 g, 29 mmol), and the mixture was refluxed for 17 hrs. After the reaction was completed, the EtOH solvent was removed *in vacuo* and to the residue was added water (50 mL). The resulting mixture was extracted with EtOAc (50 mL x 3). The combined organic layers were dried over anhydrous Na₂SO₄ and concentrated *in vacuo.* The residue was purified by silica gel column chromatography (PE/EtOAc (v/v) = 3/2) to give the title compound as a white solid (986 mg, 80%). The compound was characterized by the following spectroscopic data:
MS (ESI, pos.ion) *m*/*z*: 378.1 [M+H]⁺;
¹H NMR (400 MHz, CDCl₃) *δ* (ppm): 7. 34 (s, 1H), 4.75-4.68 (m, 1H), 4.06-3.99 (m, 1H), 2.97-2.87 (m, 1H), 2.16-2.07 (m, 1H), 1.86-1.69 (m, 2H), 1.64-1.53 (m, 1H), 1.50 (s, 9H), 1.25-1.02 (m, 2H).

### Step 6) the preparation of compound 26-7

To a solution of compound **26-1** (3.96 g, 17.28 mmol) and compound **3-6** (5.465 g, 19.81 mmol) in CH₃CN (60 mL) was added DIPEA (3.4 mL, 20.67 mmol) dropwise at 0 °C. At the end of the addition, the mixture was stirred at rt for 3.0 hrs. After the reaction was completed, the mixture was quenched with ice water (50 mL). The aqueous layer was extracted with DCM (50 mL x 3). The combined organic layers were washed with brine, dried over anhydrous Na₂SO₄ and concentrated *in vacuo.* The residue was purified by silica gel column chromatography (PE/EtOAc (v/v) = 5/1) to give the title compound as a white solid (4.48 g, 61%). The compound was characterized by the following spectroscopic data:
MS (ESI, pos.ion) *m*/*z*: 426.3 [M+H]⁺;
¹H NMR (400 MHz, CDCl₃) *δ* (ppm): 7.82-7.78 (m, 2H), 7.67-7.64 (m, 2H), 5.27 (m, 2H), 4.79-4.74 (m, 1H), 3.96-3.89 (m, 1H), 3.09-3.00 (m, 1H), 2.15-2.06 (m, 2H), 1.42 (s, 9H), 1.27-1.02 (m, 4H).

### Step 7) the preparation of compound 26-8

A suspension of compound **26-7** (4.523 g, 10.64 mmol) and ammonium acetate (16.4 g, 212.73 mmol) in toluene (50 mL) was stirred at 110 °C for 5.0 hrs. After the reaction was completed, the mixture was cooled to rt and quenched with water (50 mL). The aqueous layer was extracted with EtOAc (100 mL x 3). The combined organic layers were washed with brine, dried over anhydrous Na₂SO₄ and concentrated *in vacuo.* The residue was purified by silica gel column chromatography (PE/EtOAc (v/v) = 8/1) to give the title compound (2.15 g, 50%). The compound was characterized by the following spectroscopic data:
MS (ESI, pos.ion) *m*/*z*: 406.3 [M+H]⁺;
¹H NMR (400 MHz, CDCl₃) *δ* (ppm): 7.58 (s, 1H), 7.45-7.41 (m, 2H), 7.29-7.26 (m, 2H), 4.78-4.73 (m, 1H), 3.80-3.73 (m, 1H), 3.15-3.05 (m, 1H), 2.11-2.03 (m, 1H), 1.86-1.74 (m, 1H), 1.72-1.51 (m, 2H), 1.39 (s, 9H), 1.25-1.02 (m, 2H).

### Step 8) the preparation of compound 26-9

A mixture of compound **26-8** (2.11 g, 5.2 mmol), compound **1-12-2** (1.59 g, 6.25 mmol), Pd(dppf)Cl₂·CH₂Cl₂ (425 mg, 0.52 mmol) and KOAc (1.54 g, 15.63 mmol) in DMF (30 mL) was stirred at 90 °C under N₂ for 3.0 hrs. After the reaction was completed, the mixture was cooled to rt, diluted with EtOAc (250 mL) and filtered through a celite pad. The filtrate was washed with water (80 mL x 3) and brine, dried over anhydrous Na₂SO₄ and concentrated *in vacuo.* The residue was purified by silica gel column chromatography (PE/EtOAc (v/v) = 2/1) to give the title compound (2.0 g, 85%). The compound was characterized by the following spectroscopic data:
MS (ESI, pos.ion) *m*/*z*: 454.3 [M+H]⁺;
¹H NMR (400 MHz, CDCl₃) *δ* (ppm): 7.64-7.61 (m, 2H), 7.60-7.57 (m, 2H), 7.21 (s, 1H), 4.78-4.73 (m, 1H), 3.80-3.73 (m, 1H), 3.15-3.05 (m, 1H), 2.11-2.03 (m, 1H), 1.86-1.74 (m, 1H), 1.72-1.51 (m, 2H), 1.39 (s, 9H), 1.35 (q, 6H), 1.32 (q, 6H), 1.25-1.02 (m, 2H).

### Step 9) the preparation of compound 26-10

To a mixture of compound **26-9** (1.19 g, 2.62 mmol), compound **12-5** (1.17 g, 2.62 mmol), Pd(PPh₃)₄ (120 mg, 0.1 mmol) and KF (0.30 g, 5.24 mmol) were added DME (12.0 mL) and H₂O (3.0 mL) via syringe under N₂, and the mixture was stirred at 90 °C for 2.0 hrs. After the reaction was completed, the mixture was cooled to rt, and diluted with EtOAc (100 mL). The resulting mixture was washed with water (50 mL x 3) and brine, dried over anhydrous Na₂SO₄ and concentrated *in vacuo.* The residue was purified by silica gel column chromatography (DCM/MeOH (v/v) = 150/1) to give the title compound as a white solid (1.01 g, 60%). The compound was characterized by the following spectroscopic data:
MS (ESI, pos.ion) *m*/*z*: 646.3 [M+H]⁺;
¹H NMR (400 MHz, CDCl₃) *δ* (ppm): 7.56 (s, 1H), 7.54-7.51 (m, 2H), 7.45-7.42 (m, 2H), 7.27, 7.25 (s, s, 1 H), 6.97, 6.95 (dd, dd, 1H), 4.78-4.73 (m, 1H), 3.80-3.73 (m, 1H), 3.15-3.05 (m, 1H), 3.00-2.96 (m, 2H), 2.42-2.34 (m, 2H), 2.29-2.20 (m, 4H), 2.11-2.03 (m, 1H), 1.86-1.51 (m, 7H), 1.39 (s, 9H), 1.25-1.02 (m, 2H).

### Step 10) the preparation of compound 26-11

A mixture of compound **26-10** (1.04 g, 1.61 mmol), compound **1-12-2** (0.45 g, 1.77 mmol), Pd(dppf)Cl₂CH₂Cl₂ (80 mg, 0.096 mmol) and KOAc (0.4 g, 4.02 mmol) in DMF (10 mL) was stirred at 90 °C under N₂ for 4.0 hrs. After the reaction was completed, the mixture was cooled to rt, diluted with EtOAc (100 mL) and filtered through a celite pad. The filtrate was washed with water (20 mL x 3) and brine, dried over anhydrous Na₂SO₄ and concentrated *in vacuo.* The residue was purified by silica gel column chromatography (PE/EtOAc (v/v) = 3/1) to give the title compound as a white solid (703 mg, 70%). The compound was characterized by the following spectroscopic data:
MS (ESI, pos.ion) *m*/*z*: 624.5 [M+H]⁺;
¹H NMR (400 MHz, CDCl₃) *δ* (ppm): 7.59, 7.57 (dd, dd, 1H), 7.56 (s, 1H), 7.54-7.51 (m, 2H), 7.50-7.46 (m, 2H), 7.45, 7.44 (s, s, 1H), 4.78-4.73 (m, 1H), 3.80-3.73 (m, 1H), 3.15-3.05 (m, 1H), 2.94-2.90 (m, 2H), 2.41-2.33 (m, 2H), 2.30-2.24 (m, 2H), 2.11-2.03 (m, 1H), 1.99-1.90 (m, 2H), 1.86-1.51 (m, 7H), 1.39 (s, 9H), 1.32 (q, 6H), 1.29 (q, 6H), 1.25-1.02 (m, 2H).

### Step 11) the preparation of compound 26-12

A suspension of compound **26-6** (168.17 mg, 0.446 mmol), compound **26-11** (261.82 g, 0.42 mmol), Pd(PPh₃)₄ (25 mg, 0.22 mmol) and K₂CO₃ (153.9 mg, 1.12 mmol) in mixed solvents of EtOH and H₂O (8.0 mL, v/v = 3/1) was stirred at 90 °C under N₂ for 2.0 hrs. After the reaction was completed, the mixture was diluted with EtOAc (50 mL). The resulting mixture was washed with brine, dried over anhydrous Na₂SO₄ and concentrated *in vacuo.* The residue was purified by silica gel column chromatography (DCM/EtOH (v/v) = 100/1) to give the title compound as a pale yellow solid (203.78 mg, 65%). The compound was characterized by the following spectroscopic data:
MS (ESI, pos.ion) *m*/*z*: 747.5 [M+H]⁺;
¹H NMR (400 MHz, CDCl₃) *δ* (ppm): 7.79 (s, 1H), 7.56 (s, 1H), 7.54-7.51 (m, 3H), 7.49-7.46 (m, 2H), 7.18, 7.16 (s, s, 1H), 4.83-4.73 (m, 2H), 4.28-4.20 (m, 1H), 3.80-3.73 (m, 1H), 3.15-3.05 (m, 1H), 2.97-2.87 (m, 1H), 2.85-2.81 (m, 2H), 2.37-2.23 (m, 4H), 2.12-2.03 (m, 2H), 1.94-1.74 (m, 4H), 1.73-1.54 (m, 8H), 1.50 (s, 9H), 1.39 (s, 9H), 1.25-1.02 (m, 2H).

### Step 12) the preparation of compound 26-13

To a solution of compound **26-12** (380.7 mg, 0.51 mmol) in EtOAc (4.0 mL) was added a solution of HCl in EtOAc (3.0 mL, 4 M) dropwise. At the end of the addition, the mixture was stirred at rt for 8.0 hrs. After the reaction was completed, the mixture was concentrated *in vacuo.* The residue was purified by beating in EtOAc (10 mL) and filtered to give the title compound as a pale yellow solid (317.8 mg, 90%), which was used for the next step without further purification. The compound was characterized by the following spectroscopic data:
MS (ESI, pos.ion) *m*/*z*: 547.3 [M+H]⁺.

### Step 13) the preparation of compound 26-14

To a suspension of compound **26-13** (200.8 mg, 0.29 mmol), compound **1-11-2** (110 mg, 0.65 mmol), EDCI (120 mg, 0.65 mmol) and HOAT (80 mg, 0.59 mmol) in DCM (5.0 mL) was added DIPEA (0.41 mL, 2.32 mmol) dropwise at 0 °C. At the end of the addition, the mixture was stirred at rt for 3.0 hrs. After the reaction was completed, the mixture was diluted with DCM (20 mL), washed with saturated NH₄Cl aqueous solution and brine, dried over anhydrous Na₂SO₄ and concentrated *in vacuo.* The residue was purified by silica gel column chromatography (DCM/MeOH (v/v) = 60/1) to give the title compound as a white solid (139.7 mg, 56%). The compound was characterized by the following spectroscopic data:
MS (ESI, pos.ion) *m*/*z*: 861.5 [M+H]⁺;
¹H NMR (400 MHz, CDCl₃) *δ* (ppm): 7.72 (s, 1H), 7.57 (s, 1H), 7.54-7.51 (m, 3H), 7.49-7.46 (m, 2H), 7.18, 7.16 (s, s, 1H), 5.32, 5.29 (d, d, 2H), 5.03-4.97 (m, 1H), 4.78-4.72 (m, 1H), 4.40-4.35 (m, 2H), 3.77-3.69 (m, 2H), 3.63 (s, 6H), 2.86-2.76 (m, 4H), 2.37-2.23 (m, 4H), 2.22-2.13 (m, 2H), 2.02-1.86 (m, 4H), 1.82-1.49 (m, 10H), 1.21-1.00 (m, 4H), 0.97, 0.95 (m, m, 6H), 0.91, 0.89 (m, m, 6H).

### Example 27

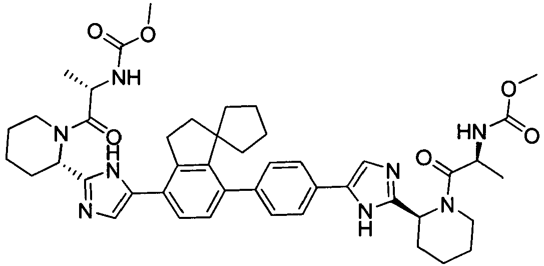

### Synthetic route:

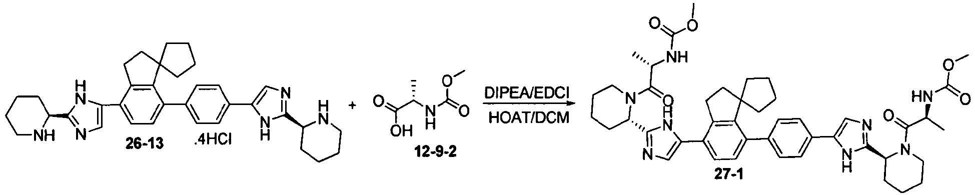

### Step 1) the preparation of compound 27-1

To a suspension of compound **26-13** (200.8 mg, 0.29 mmol), compound **12-9-2** (95.58 mg, 0.65 mmol), EDCI (120 mg, 0.65 mmol) and HOAT (80 mg, 0.59 mmol) in DCM (5.0 mL) was added DIPEA (0.41 mL, 2.32 mmol) dropwise at 0 °C. At the end of the addition, the mixture was stirred at rt for 3.0 hrs. After the reaction was completed, the mixture was diluted with DCM (20 mL), washed with saturated NH₄Cl aqueous solution and brine, dried over anhydrous Na₂SO₄ and concentrated *in vacuo.* The residue was purified by silica gel column chromatography (DCM/MeOH (v/v) = 60/1) to give the title compound as a white solid (105 mg, 45%). The compound was characterized by the following spectroscopic data:
MS (ESI, pos.ion) *m*/*z*: 805.5 [M+H]⁺;
¹H NMR (400 MHz, CDCl₃) *δ* (ppm): 7.72 (s, 1H), 7.57 (s, 1H), 7.54-7.51 (m, 3H), 7.49-7.46 (m, 2H), 7.18, 7.16 (s, s, 1H), 5.32, 5.29 (d, d, 2H), 5.03-4.97 (m, 1H), 4.78-4.72 (m, 1H), 4.40-4.35 (m, 2H), 3.77-3.69 (m, 2H), 3.63 (s, 6H), 2.86-2.76 (m, 4H), 2.27-2.23 (m, 4H), 2.22-2.13 (m, 2H), 2.02-1.86 (m, 4H), 1.82-1.49 (m, 10H), 1.21-1.00 (m, 4H), 0.97, 0.95 (m, m, 6H), 0.91, 0.89 (m, m, 6H).

### Example 28

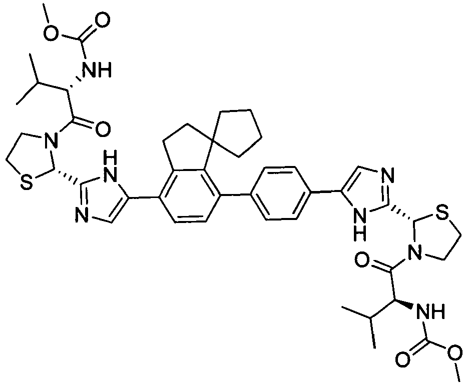

### Synthetic route:

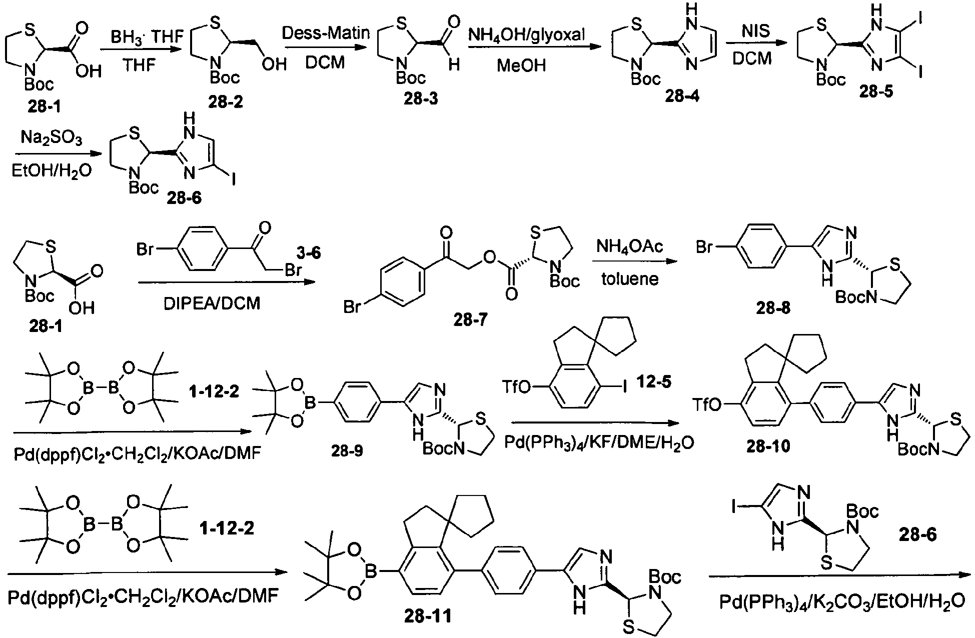

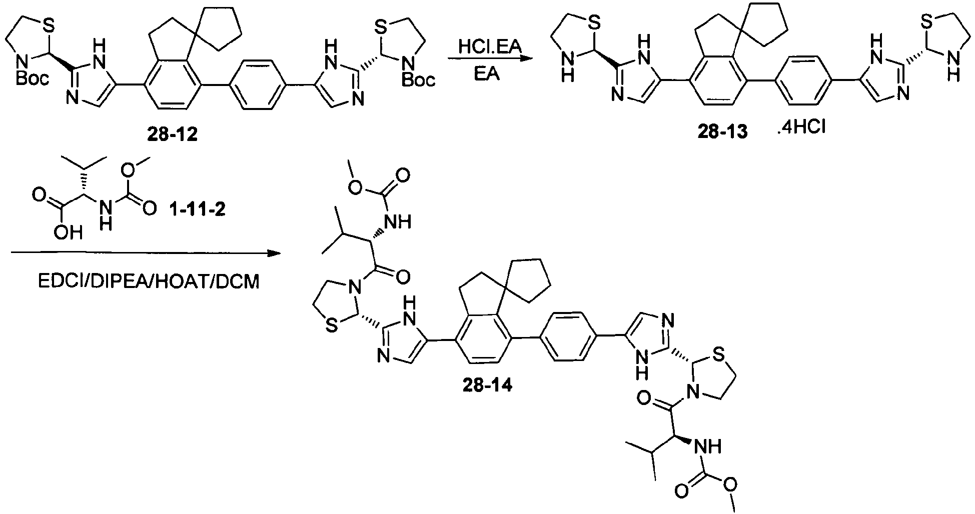

### Step 1) the preparation of compound 28-2

To a solution of compound **28-1** (10.86 g, 46.6 mmol) in THF (100 mL) was added diborane (100 mL, 1M in THF) dropwise at 0 °C under N₂. At the end of the addition, the mixture was stirred at 0 °C for 3.0 hrs. After the reaction was completed, the mixture was quenched with MeOH (80 mL) and *concentrated in vacuo.* The residue was purified by silica gel column chromatography (PE/EtOAc (v/v) = 3/2) to give the title compound as colorless oil (7.65 g, 75%). The compound was characterized by the following spectroscopic data:
¹H NMR (400 MHz, CDCl₃) *δ* (ppm): 5.16-5.12 (m, 1H), 3.91-3.84 (m, 1H), 3.76-3.72 (m, 1H), 3.63-3.58 (m, 1H), 3.56-3.48 (m, 1H), 3.17-3.10 (m, 1H), 3.00 (br, 1H), 2.90-2.83 (m, 1H), 1.43 (s, 9H).

### Step 2) the preparation of compound 28-3

To a solution of compound **28-2** (7.62 g, 34.8 mmol) in DCM (250 mL) was added Dess-Martin periodinane (20.7 g, 48.8 mmol) in a portionwise manner at 0 °C. At the end of the addition, the mixture was stirred at rt for 2.0 hrs. After the reaction was completed, to the mixture was added water (150 mL), and the mixture was filtered. After the filtrate was partitioned, the organic layer was washed with brine, dried over anhydrous Na₂SO₄ and concentrated *in vacuo.* The residue was purified by silica gel column chromatography (PE/EtOAc (v/v) = 3/2) to give the title compound as colorless oil (3.83 g, 50.7%). The compound was characterized by the following spectroscopic data:
¹H NMR (400 MHz, CDCl₃) *δ* (ppm): 8.67-8.65 (m, 1H), 5.01-4.97 (m, 1H), 4.01-3.94 (m, 1H), 3.49-3.41 (m, 1H), 3.11-3.03 (m, 1H), 2.77-2.69 (m, 1H), 1.42 (s, 9H).

### Step 3) the preparation of compound 28-4

To a solution of compound **28-3** (3.82 g, 17.6 mmol) and ammonia (13.0 mL) in MeOH (30.0 mL) was added glyoxal (8.0 mL, 40% in water) dropwise at 0 °C. At the end of the addition, the mixture was stirred at rt overnight. After the reaction was completed, the mixture was concentrated *in vacuo.* The residue was purified by silica gel column chromatography (PE/EtOAc (v/v) = 3/2) to give the title compound as a white solid (2.14 g, 47.6%). The compound was characterized by the following spectroscopic data:
MS (ESI, pos.ion) *m*/*z*: 256.5 [M+H]⁺;
¹H NMR (400 MHz, CDCl₃) *δ* (ppm): 7.16 (s, 2H), 6.46-6.43 (m, 1H), 4.05-3.99 (m, 1H), 3.47-3.40 (m, 1H), 3.31-3.23 (m, 1H), 2.92-2.85 (m, 1H), 1.41 (s, 9H).

### Step 4) the preparation of compound 28-5

To a solution of compound **28-4** (2.14 g, 8.4 mmol) in DCM (60.0 mL) was added *N*-iodosuccinimide (3.8 g, 16.8 mmol) at 0 °C in a portionwise manner. At the end of the addition, the mixture was stirred at 0 °C for 1.5 hrs. After the reaction was completed, the mixture was washed with brine, dried over anhydrous Na₂SO₄ and concentrated *in vacuo.* The residue was purified by silica gel column chromatography (PE/EtOAc (v/v) = 3/2) to give the title compound as a white solid (2.68 g, 63.1%). The compound was characterized by the following spectroscopic data:
MS (ESI, pos.ion) *m*/*z*: 507.5 [M+H]⁺;
¹H NMR (400 MHz, CDCl₃) *δ* (ppm): 5.97-5.95 (m, 1H), 4.15-4.08 (m, 1H), 3.46-3.39 (m, 1H), 3.38-3.31 (m, 1H), 3.00-2.92 (m, 1H), 1.41 (s, 9H).

### Step 5) the preparation of compound 28-6

To a suspension of compound **28-5** (1.66 g, 3.27 mmol) in mixed solvents of ethanol and water (50 mL, v/v = 3/7) was added Na₂SO₃ (3.7 g, 29.0 mmol). At the end of the addition, the mixture was refluxed for 17.0 hrs. After the reaction was completed, the ethanol solvent was removed, and to the residue was added water (50 mL). The resulting mixture was extracted with EtOAc (50 mL x 3). The combined organic layers were washed with brine, dried over anhydrous Na₂SO₄ and concentrated *in vacuo.* The residue was purified by silica gel column chromatography (PE/EtOAc (v/v) = 3/2) to give the title compound as a white solid (1.04 g, 84%). The compound was characterized by the following spectroscopic data:
MS (ESI, pos.ion) *m*/*z*: 382.5 [M+H]⁺;
¹H NMR (400 MHz, CDCl₃) *δ* (ppm): 7.19 (s, 1H), 6.71-6.68 (m, 1H), 4.12-4.05 (m, 1H), 3.49-3.42 (m, 1H), 3.34-3.27 (m, 1H), 2.96-2.88 (m, 1H), 1.41 (s, 9H).

### Step 6) the preparation of compound 28-7

To a solution of compound **3-6** (29.99 g, 107.9 mmol) and compound **28-1** (27.66 g, 118.7 mmol) in DCM (250 mL) was added DIPEA (21.4 mL, 129.48 mmol) dropwise at 0 °C. At the end of the addition, the mixture was stirred at rt for 3.0 hrs. After the reaction was completed, the DCM solvent was removed, and to the residue was added water (100 mL). The aqueous layer was extracted with EtOAc (100 mL x 3). The combined organic layers were washed with brine, dried over anhydrous Na₂SO₄ and concentrated *in vacuo.* The residue was purified by silica gel column chromatography (PE/EtOAc (v/v) = 10/1) to give the title compound as a white solid (42.12 g, 91%). The compound was characterized by the following spectroscopic data:
MS (ESI, pos.ion) *m*/*z*: 430.5 [M+H]⁺;
¹H NMR (400 MHz, CDCl₃) *δ* (ppm): 7.82-7.78 (m, 2H), 7.67-7.64 (m, 2H), 5.55-5.52 (m, 1H), 5.29 (s, 2H), 4.11-4.04 (m, 1H), 3.73-3.66 (m, 1H), 3.19-3.12 (m, 1H), 2.93-2.86 (m, 1H), 1.43 (s, 9H).

### Step 7) the preparation of compound 28-8

A suspension of compound **28-7** (15.62 g, 36.4 mmol) and ammonium acetate (42.0 g, 54.6 mmol) in toluene (150 mL) was stirred at 110 °C for 5.0 hrs. After the reaction was completed, the mixture was cooled to rt and quenched with water (100 mL). The aqueous layer was extracted with EtOAc (100 mL x 3). The combined organic layers were washed with brine, dried over anhydrous Na₂SO₄ and concentrated *in vacuo.* The residue was purified by silica gel column chromatography (PE/EtOAc (v/v) = 6/1) to give the title compound as a pale yellow solid (12.65 g, 85%). The compound was characterized by the following spectroscopic data:
MS (ESI, pos.ion) *m*/*z*: 410.5 [M+H]⁺;
¹H NMR (400 MHz, CDCl₃) *δ* (ppm): 7.64 (s, 1H), 7.45-7.41 (m, 2H), 7.29-7.26 (m, 2H), 6.54-6.51 (m, 1H), 4.08-4.01 (m, 1H), 3.44-3.37 (m, 1H), 3.30-3.22 (m, 1H), 2.91-2.84 (m, 1H), 1.42 (s, 9H).

### Step 8) the preparation of compound 28-9

A mixture of compound **28-8** (4.18 g, 10.23 mmol), compound **1-12-2** (2.86 g, 11.25 mmol), Pd(dppf)Cl₂·CH₂Cl₂ (418 mg, 0.51 mmol) and KOAc (2.51 g, 25.57 mmol) in DMF (40.0 mL) was stirred at 90 °C under N₂ for 3.0 hrs. After the reaction was completed, the mixture was cooled to rt, diluted with EtOAc (250 mL) and filtered through a celite pad. The filtrate was washed with water (100 mL x 3) and brine, dried over anhydrous Na₂SO₄ and concentrated *in vacuo.* The residue was purified by silica gel column chromatography (PE/EtOAc (v/v) = 2/1) to give the title compound (3.74 g, 80%). The compound was characterized by the following spectroscopic data:
¹H NMR (400 MHz, CDCl₃) *δ* (ppm): 7.63-7.57 (m, 4H), 7.32 (s, 1H), 6.54-6.51 (m, 1H), 4.08-4.01 (m, 1H), 3.44-3.37 (m, 1H), 3.30-3.22 (m, 1H), 2.91-2.84 (m, 1H), 1.42 (s, 9H), 1.35 (m, 6H), 1.32 (m, 6H).

### Step 9) the preparation of compound 28-10

To a mixture of compound **28-9** (1.198 g, 2.62 mmol), compound **12-5** (1.17 g, 2.62 mmol), Pd(PPh₃)₄ (120 mg, 0.1 mmol) and KF (0.30 g, 5.24 mmol) were added DME (12.0 mL) and pure water (3.0 mL) via syringe under N₂, and the mixture was stirred at 90 °C for 2.0 hrs. After the reaction was completed, the mixture was cooled to rt and diluted with EtOAc (60.0 mL). The resulting mixture was washed with water (20 mL x 3) and brine, dried over anhydrous Na₂SO₄ and concentrated *in vacuo.* The residue was purified by silica gel column chromatography (DCM/MeOH (v/v) = 100/1) to give the title compound as a white solid (1.02 g, 60%). The compound was characterized by the following spectroscopic data:
MS (ESI, pos.ion) *m*/*z*: 650.3 [M+H]⁺;
¹H NMR (400 MHz, CDCl₃) *δ* (ppm): 7.63 (s, 1H), 7.56-7.53 (m, 2H), 7.45-7.42 (m, 2H), 7.27, 7.25 (s, s, 1H), 6.97, 6.95 (dd, dd, 1H), 6.21-6.19 (m, 1H), 3.90-3.83 (m, 1H), 3.64-3.57 (m, 1H), 3.30-3.22 (m, 1H), 3.00-2.96 (m, 2H), 2.91-2.84 (m, 1H), 2.42-2.34 (m, 2H), 2.29-2.20 (m, 4H), 1.76-1.59 (m, 4H), 1.43 (s, 9H).

### Step 10) the preparation of compound 28-11

A mixture of compound **28-10** (1.04 g, 1.61 mmol), compound **1-12-2** (0.45 g, 10.7 mmol), Pd(dppf)Cl₂·CH₂Cl₂ (80 mg, 0.096 mmol) and KOAc (0.4 g, 4.02 mmol) in DMF (10.0 mL) was stirred at 120 °C under N₂ for 4.0 hrs. After the reaction was completed, the mixture was cooled to rt, diluted with EtOAc (40.0 mL) and filtered through a celite pad. The filtrate was washed with water (30 mL x 3) and brine, dried over anhydrous Na₂SO₄ and concentrated *in vacuo.* The residue was purified by silica gel column chromatography (PE/EtOAc (v/v) = 1/2) to give the title compound as a white solid (707 mg, 70%). The compound was characterized by the following spectroscopic data:
MS (ESI, pos.ion) *m*/*z*: 628.3 [M+H]⁺;
¹H NMR (400 MHz, CDCl₃) *δ* (ppm): 7.63 (s, 1H), 7.59-7.53 (m, 3H), 7.50-7.47 (m, 2H), 7.46, 7.44 (s, s, 1H), 6.54-6.51 (m, 1H), 4.08-4.01 (m, 1H), 3.44-3.37 (m, 1H), 3.30-3.22 (m, 1H), 2.94-2.84 (m, 3H), 2.41-2.33 (m, 2H), 2.30-2.24 (m, 2H), 1.99-1.90 (m, 2H), 1.77-1.60 (m, 4H), 1.41 (s, 9H), 1.32 (m, 6H), 1.29 (m, 6H).

### Step 11) the preparation of compound 28-12

To a suspension of compound **28-6** (170 mg, 0.446 mmol), compound **28-11** (263.5 mg, 0.42 mmol), Pd(PPh₃)₄ (25 mg, 0.02 mmol) and K₂CO₃ (0.17 g, 1.27 mmol) were added EtOH (6.0 mL) and pure water (1.0 mL) via syringe under N₂, and the mixture was stirred at 90 °C for 2.0 hrs. After the reaction was completed, the mixture was concentrated *in vacuo.* The residue was dissolved in EtOAc (20.0 mL). The resulting mixture was washed with brine, dried over anhydrous Na₂SO₄ and concentrated *in vacuo.* The residue was purified by silica gel column chromatography (DCM/EtOH (v/v) = 100/1) to give the title compound as a pale yellow solid (269.3 mg, 85%). The compound was characterized by the following spectroscopic data:
MS (ESI, pos.ion) *m*/*z*: 755.3 [M+H]⁺;
¹H NMR (400 MHz, CDCl₃) *δ* (ppm): 7.63 (s, 1H), 7.59 (s, 1H), 7.56-7.51 (m, 3H), 7.49-7.46 (m, 2H), 7.20, 7.18 (s, s, 1H), 6.68-6.65 (m, 1H), 6.54-6.51 (m, 1H), 4.08-4.01 (m, 2H), 3.44-3.37 (m, 2H), 3.30-3.22 (m, 2H), 2.91-2.81 (m, 4H), 2.37-2.23 (m, 4H), 1.94-1.86 (m, 2H), 1.76-1.58 (m, 4H), 1.42 (s, 9H), 1.41 (s, 9H).

### Step 12) the preparation of compound 28-13

To a solution of compound **28-12** (384.7 mg, 0.51 mmol) in EtOAc (4.0 mL) was added a solution of HCl in EtOAc (3.0 mL, 4 M) dropwise. At the end of the addition, the mixture was stirred at rt for 8.0 hrs. After the reaction was completed, the mixture was concentrated *in vacuo.* The residue was purified by beating in EtOAc (10 mL) and filtered to give the title compound as pale yellow powder (285.7 mg, 80%). The compound was characterized by the following spectroscopic data:
MS (ESI, pos.ion) *m*/*z*: 555.5 [M+H]⁺.

### Step 13) the preparation of compound 28-14

To a suspension of compound **28-13** (203.1 mg, 0.29 mmol), compound **1-11-2** (110 mg, 0.65 mmol), EDCI (120 mg, 0.65 mmol) and HOAT (80 mg, 0.59 mmol) in DCM (5.0 mL) was added DIPEA (0.6 mL, 3.63 mmol) dropwise at 0 °C. At the end of the addition, the mixture was stirred at rt for 3.0 hrs. After the reaction was completed, the mixture was diluted with DCM (20.0 mL), washed with NH₄Cl aqueous solution and brine, dried over anhydrous Na₂SO₄ and concentrated *in vacuo.* The residue was purified by silica gel column chromatography (DCM/MeOH (v/v) = 40/1) to give the title compound as a white solid (188.87 mg, 75%). The compound was characterized by the following spectroscopic data:
MS (ESI, pos.ion) *m*/*z*: 869.5 [M+H]⁺;
¹H NMR (400 MHz, CDCl₃) *δ* (ppm): 7.64 (s, 1H), 7.56-7.51 (m, 4H), 7.49-7.46 (m, 2H), 7.20, 7.18 (s, s, 1H), 6.29-6.27 (m, 1H), 6.25-6.23 (m, 1H), 5.56, 5.55 (d, d, 1H), 5.32, 5.29 (d, d, 1H), 4.43-4.30 (m, 4H), 3.66 (s, 3H), 3.63 (s, 3H), 3.59-3.53 (m, 2H), 3.25-3.17 (m, 2H), 2.86-2.79 (m, 4H), 2.37-2.14 (m, 6H), 1.94-1.86 (m, 2H), 1.76-1.58 (m, 4H), 1.02-0.89 (m, 12H).

### Example 29

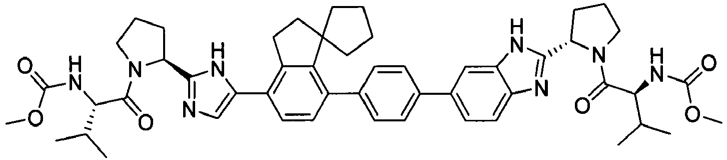

### Synthetic route:

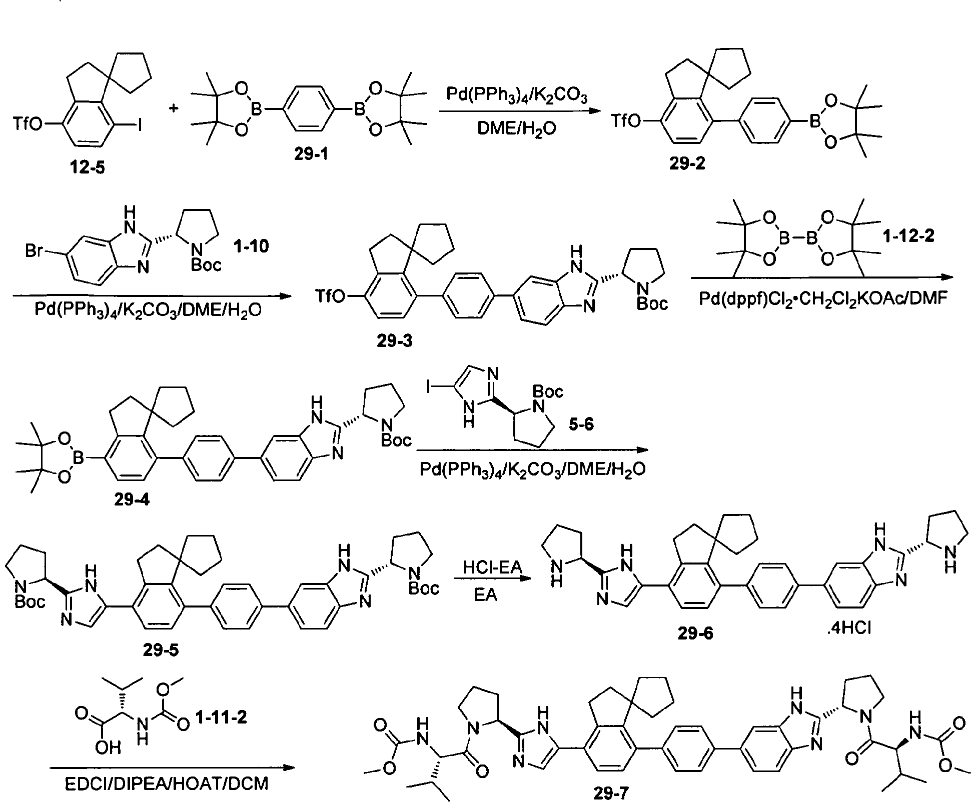

### Step 1) the preparation of compound 29-2

A suspension of compound **12-5** (1.516 g, 3.4 mmol), compound **29-1** (1.12 g, 3.4 mmol), Pd(PPh₃)₄ (196.7 mg, 0.17 mmol) and K₂CO₃ (1.412 g, 10.22 mmol) in mixed solvents of DME and H₂O (15 mL, v/v = 4/1) was stirred at 90 °C under N₂ for 3.0 hrs. After the reaction was completed, the mixture was cooled to rt and diluted with EtOAc (60.0 mL). The resulting mixture was washed with water (20 mL x 3) and brine, dried over anhydrous Na₂SO₄ and concentrated *in vacuo.* The residue was purified by silica gel column chromatography (PE/EtOAc (v/v) = 4/1) to give the title compound as a pale yellow solid (533 mg, 30%). The compound was characterized by the following spectroscopic data:
MS (ESI, pos.ion) *m*/*z*: 523.3 [M+H]⁺;
¹H NMR (400 MHz, CDCl₃) *δ* (ppm): 7.67-7.64 (m, 2H), 7.55-7.52 (m, 2H), 7.27, 7.25 (s, s, 1H), 6.97, 6.94 (dd, dd, 1H), 3.00-2.96 (m, 2H), 2.42-2.34 (m, 2H), 2.29-2.20 (m, 4H), 1.76-1.59 (m, 4H), 1.35 (m, 6H), 1.32 (m, 6H).

### Step 2) the preparation of compound 29-3

A suspension of compound **29-2** (108.09 mg, 0.207 mmol), compound **1-10** (75.57 mg, 0.207 mmol), Pd(PPh₃)₄ (23.97 mg, 0.0207 mmol) and K₂CO₃ (85.93 mg, 0.6227 mmol) in mixed solvents of DME and H₂O (5.0 mL, v/v = 4/1) was stirred at 90 °C under N₂ for 3.0 hrs. After the reaction was completed, the mixture was cooled to rt and diluted with EtOAc (20.0 mL). The resulting mixture was washed with water (10 mL x 3) and brine, dried over anhydrous Na₂SO₄ and concentrated *in vacuo.* The residue was purified by silica gel column chromatography (DCM/EtOH (v/v) = 100/1) to give the title compound as a yellow solid (119.8 mg, 85%). The compound was characterized by the following spectroscopic data:
MS (ESI, pos.ion) *m*/*z*: 682.3 [M+H]⁺;
¹H NMR (400 MHz, CDCl₃) *δ* (ppm): 7.69-7.68 (m, 1H), 7.62-7.56 (m, 5H), 7.45, 7.43 (d, d, 1H), 7.27, 7.25 (s, s, 1H), 6.97, 6.95 (dd, dd, 1H), 5.04-4.99 (m, 1H), 3.82-3.76 (m, 1H), 3.64-3.56 (m, 1H), 3.00-2.96 (m, 2H), 2.62-2.54 (m, 1H), 2.46-2.34 (m, 3H), 2.29-2.16 (m, 7H), 2.04-1.93 (m, 1H), 1.76-1.59 (m, 2H), 1.41 (s, 9H).

### Step 3) the preparation of compound 29-4

A mixture of compound **29-3** (7.677 g, 11.27 mmol), compound **1-12-2** (4.29 g, 16.9 mmol), Pd(dppf)Cl₂·CH₂Cl₂ (653 mg, 0.80 mmol) and KOAc (2.09 g, 21.3 mmol) in DMF (30.0 mL) was stirred at 90 °C under N₂ for 3.0 hrs. After the reaction was completed, the mixture was cooled to rt, diluted with EtOAc (200 mL) and filtered through a celite pad. The filtrate was washed with water (60 mL x 3) and brine, dried over anhydrous Na₂SO₄ and concentrated *in vacuo.* The residue was purified by silica gel column chromatography (PE/EtOAc (v/v) = 1/1) to give the title compound as a yellow solid (3.72 g, 50%). The compound was characterized by the following spectroscopic data:
MS (ESI, pos.ion) *m*/*z*: 660.3 [M+H]⁺;
¹H NMR (400 MHz, CDCl₃) *δ* (ppm): 7.76-7.73 (m, 2H), 7.69-7.68 (m, 1H), 7.62-7.57 (m, 4H), 7.46-7.43 (m, 2H), 5.04-4.99 (m, 1H), 3.82-3.76 (m, 1H), 3.64-3.56 (m, 1H), 2.94-2.90 (m, 2H), 2.62-2.54 (m, 1H), 2.46-2.33 (m, 3H), 2.30-2.16 (m, 4H), 2.04-1.90 (m, 2H), 1.77-1.60 (m, 4H), 1.41 (s, 9H), 1.32, 1.29 (m, m, 12H).

### Step 4) the preparation of compound 29-5

A mixture of compound **29-4** (2.24 g, 3.4 mmol), compound **5-6** (1.23 g, 3.4 mmol), Pd(PPh₃)₄ (196.7 mg, 0.17 mmol) and K₂CO₃ (1.412 g, 10.22 mmol) in mixed solvents of DME and H₂O (15 mL, v/v = 4/1) was stirred at 90 °C under N₂ for 3.0 hrs. After the reaction was completed, the mixture was cooled to rt and diluted with EtOAc (100 mL). The resulting mixture was washed with water (30 mL x 3) and brine, dried over anhydrous Na₂SO₄ and concentrated *in vacuo.* The residue was purified by silica gel column chromatography (PE/EtOAc (v/v) = 4/1) to give the title compound as a pale yellow solid (1.567 g, 60%). The compound was characterized by the following spectroscopic data:
MS (ESI, pos.ion) *m*/*z*: 769.5 [M+H]⁺;
¹H NMR (400 MHz, CDCl₃) *δ* (ppm): 7.78 (s, 1H), 7.71-7.67 (m, 3H), 7.62-7.58 (m, 3H), 7.53, 7.51 (dd, dd, 1H), 7.45, 7.43 (d, d, 1H), 7.18, 7.16 (s, s, 1H), 5.14-5.08 (m, 1H), 5.04-4.99 (m, 1H), 3.82-3.76 (m, 1H), 3.64-3.56 (m, 1H), 3.31-3.23 (m, 1H), 2.85-2.81 (m, 1H), 2.62-2.54 (m, 1H), 2.47-2.16 (m, 9H), 2.10-1.86 (m, 5H), 1.76-1.58 (m, 4H), 1.53 (s, 9H), 1.41 (s, 9H).

### Step 5) the preparation of compound 29-6

To a solution of compound **29-5** (152.15 mg, 0.198 mmol) in EtOAc (4.0 mL) was added a solution of HCl in EtOAc (3.0 mL, 4 M) dropwise. At the end of the addition, the mixture was stirred at rt for 8.0 hrs. After the reaction was completed, the mixture was concentrated *in vacuo.* The residue was purified by beating in EtOAc (4.0 mL) and filtered to give the title compound as pale yellow powder (127.3 mg, 90%). The compound was characterized by the following spectroscopic data:
MS (ESI, pos.ion) *m*/*z*: 569.3 [M+H]⁺.

### Step 6) the preparation of compound 29-7

A suspension of compound **29-6** (481.46 mg, 0.674 mmol), compound **1-11-2** (235.8 mg, 1.35 mmol), EDCI (271.3 mg, 1.415 mmol) and HOAT (137.58 mg, 1.01 mmol) in DCM (20.0 mL) was added DIPEA (0.93 mL, 5.63 mmol) dropwise at 0 °C. At the end of the addition, the mixture was stirred at rt for 3.0 hrs. After the reaction was completed, the mixture was diluted with DCM (30.0 mL), washed with NH₄Cl aqueous solution and brine, dried over anhydrous Na₂SO₄ and concentrated *in vacuo.* The residue was purified by silica gel column chromatography (DCM/MeOH (v/v) = 60/1) to give the title compound as a white solid (356.87 mg, 60%). The compound was characterized by the following spectroscopic data:
MS (ESI, pos.ion) *m*/*z*: 442.5 [M+2H]²⁺;
¹H NMR (400 MHz, CDCl₃) *δ* (ppm): 7.71 (s, 1H), 7.70-7.67 (m, 3H), 7.62-7.58 (m, 3H), 7.53, 7.51 (dd, dd, 1H), 7.45, 7.43 (dd, dd, 1H), 7.18, 7.16 (s, s, 1H), 5.56, 5.55 (d, d, 1H), 5.46, 5.44 (d, d, 1H), 5.32-5.28 (m, 1H), 5.24-5.20 (m, 1H), 4.40-4.30 (m, 2H), 3.85-3.77 (m, 2H), 3.69-3.67 (m, 2H), 3.66 (s, 6H), 2.85-2.81 (m, 2H), 2.37-1.86 (m, 16H), 1.76-1.58 (m, 4H), 1.02-0.89 (m, 12H).

### Example 30

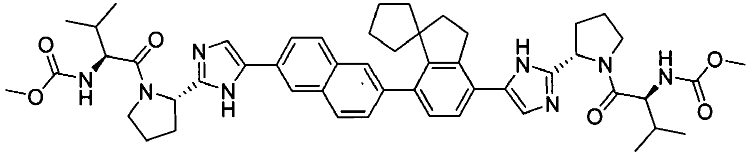

### Synthetic route:

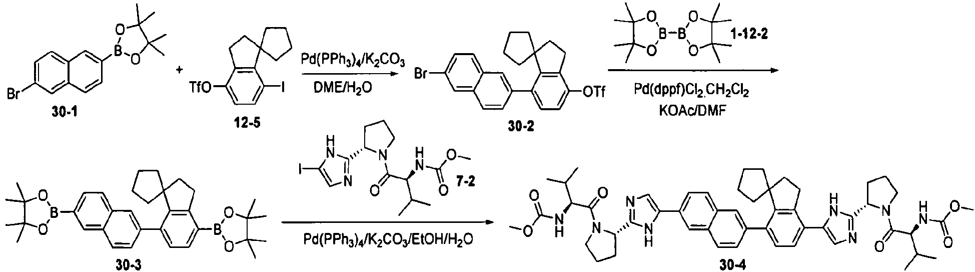

### Step 1) the preparation of compound 30-2

A suspension of compound **12-5** (8.38 g, 18.8 mmol), compound **30-1** (6.24 g, 18.8 mmol), Pd(PPh₃)₄ (1.10 g, 0.94 mmol) and K₂CO₃ (10.4 g, 75.4 mmol) in mixed solvents of DME and H₂O (80 mL, v/v = 3/1) was stirred at 90 °C under N₂ for 3.0 hrs. After the reaction was completed, the mixture was diluted with EtOAc (200 mL), washed with water (50.0 mL x 3) and brine, dried over anhydrous Na₂SO₄ and concentrated *in vacuo.* The residue was purified by silica gel column chromatography (PE/EtOAc (v/v) = 1/2) to give the title compound as a pale yellow solid (4.925 g, 50%). The compound was characterized by the following spectroscopic data:
¹H NMR (400 MHz, CDCl₃) *δ* (ppm): 8.04-8.03 (m, 1H), 8.00-7.99 (m, 1H), 7.83-7.80 (m, 1H), 7.72-7.69 (m, 1H), 7.65-7.62 (m, 1H), 7.57-7.56 (m, 0.5H), 7.55-7.54 (m, 0.5H), 7.29, 7.26 (s, s, 1H), 7.10, 7.08 (dd, dd, 1H), 2.99-2.95 (m, 2H), 2.42-2.33 (m, 2H), 2.27-2.21 (m, 2H), 1.99-1.91 (m, 2H), 1.76-1.59 (m, 4H).

### Step 2) the preparation of compound 30-3

A mixture of compound **30-2** (5.36 g, 10.23 mmol), compound **1-12-2** (5.46 g, 21.48 mmol), Pd(dppf)Cl₂·CH₂Cl₂ (418 mg, 0.51 mmol) and KOAc (2.51 g, 25.57 mmol) in DMF (60.0 mL) was stirred at 90 °C under N₂ for 3.0 hrs. After the reaction was completed, the mixture was cooled to rt, diluted with EtOAc (300 mL) and filtered through a celite pad. The filtrate was washed with water (100 mL x 3) and brine, dried over anhydrous Na₂SO₄ and concentrated *in vacuo.* The residue was purified by silica gel column chromatography (PE/EtOAc (v/v) = 6/1) to give the title compound (2.81 g, 50%). The compound was characterized by the following spectroscopic data:
MS (ESI, pos.ion) *m*/*z*: 551.5 [M+H]⁺;
¹H NMR (400 MHz, CDCl₃) *δ* (ppm): 8.24-8.23 (m, 1H), 8.07-8.06, 8.05-8.04 (m, m, 1H), 8.02, 8.00 (d, d, 1H), 7.94, 7.92 (m, m, 1H), 7.81-7.80 (m, 1H), 7.49, 7.47 (d, d, 1H), 7.46, 7.43 (s, s, 1H), 7.41, 7.39 (m, m, 1H), 2.95-2.91 (m, 2H), 2.38-2.24 (m, 4H), 1.95-1.87 (m, 2H), 1.77-1.60 (m, 4H), 1.33-1.32, 1.30-1.29 (m, m, 24H).

### Step 3) the preparation of compound 30-4

A suspension of compound **30-3** (5.5 g, 10.0 mmol), compound **7-2** (8.82 g, 21.0 mmol), Pd(PPh₃)₄ (1.156 g, 1.0 mmol) and K₂CO₃ (3.45 g, 25.0 mmol) in mixed solvents of EtOH and H₂O (80 mL, v/v = 3/1) was stirred at 90 °C under N₂ for 3.0 hrs. After the reaction was completed, the mixture was diluted with EtOAc (200 mL), washed with brine, dried over anhydrous Na₂SO₄ and concentrated *in vacuo.* The residue was purified by silica gel column chromatography (DCM/EtOH (v/v) = 50/1) to give the title compound as a pale yellow solid (3.53 g, 40%). The compound was characterized by the following spectroscopic data:
MS (ESI, pos.ion) *m*/*z*: 442.3 [M+2H]²⁺;
¹H NMR (400 MHz, CDCl₃) *δ* (ppm): 8.18-8.17 (m, 1H), 8.15-8.14 (m, 1H), 7.89, 7.87 (m, m, 1H), 7.85 (m, 1H), 7.78, 7.76 (m, m, 2H), 7.71 (s, 1H), 7.63, 7.61 (d, d, 1H), 7.60 (s, 1H), 7.16, 7.14 (s, s, 1H), 5.40-5.36 (m, 1H), 5.32-5.28 (m, 3H), 4.41-4.36 (m, 2H), 3.85-3.78 (m, 2H), 3.69-3.65 (m, 2H), 3.63 (s, 6H), 2.82-2.78 (m, 2H), 2.35-1.84 (m, 16H), 1.76-1.58 (m, 4H), 0.97, 0.95 (m, m, 6H), 0.91, 0.89 (m, m, 6H).

### Example 31

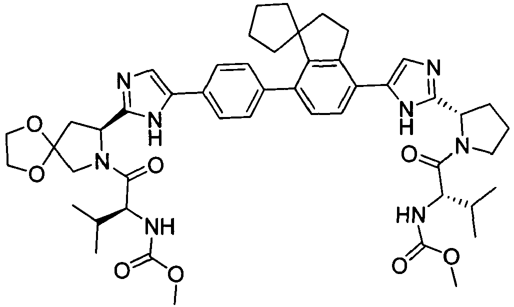

### Synthetic route:

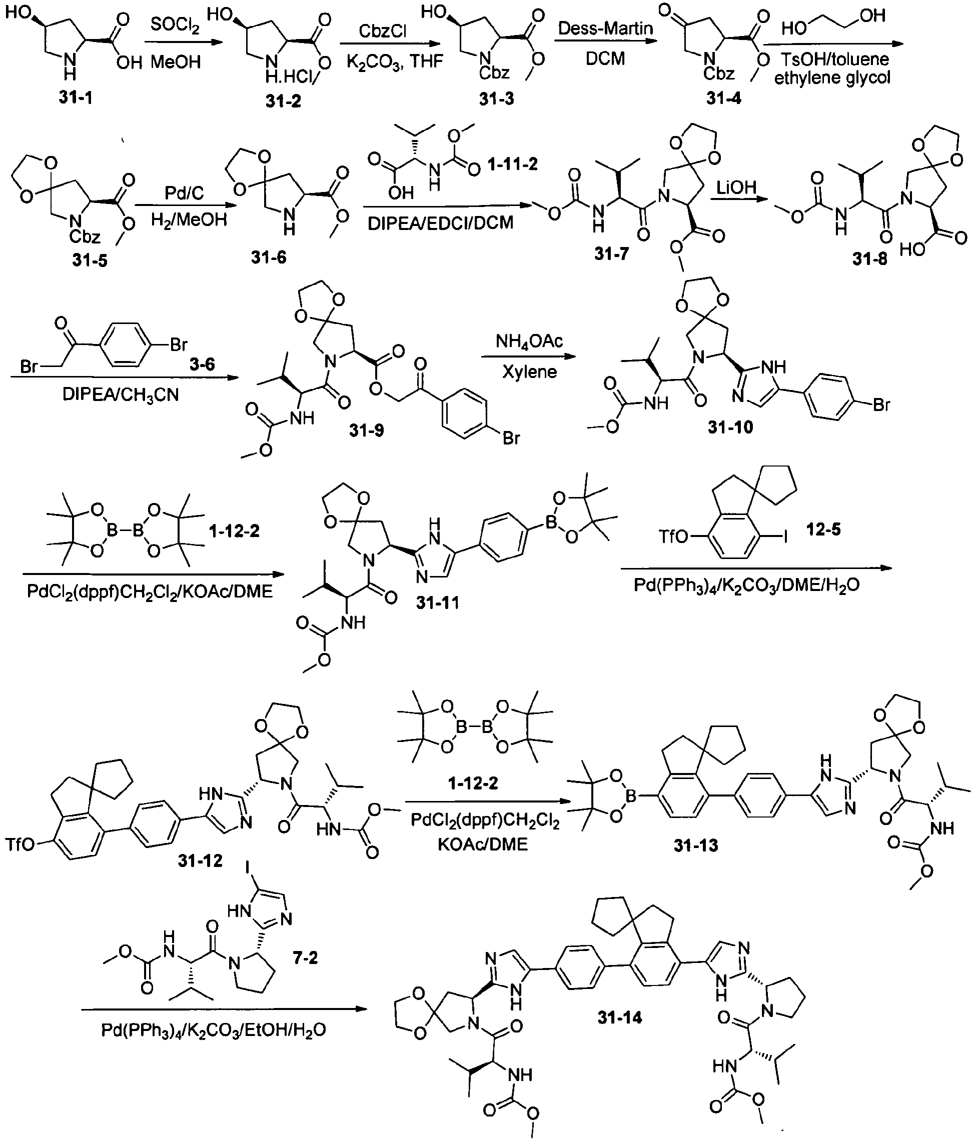

### Step 1) the preparation of compound 31-2

To a solution of compound **31-1** (2.0 g, 15.3 mmol) in MeOH (20 mL) was added thionyl chloride (3.4 mL, 46.9 mmol) dropwise at 0 °C. At the end of the addition, the mixture was stirred at 80 °C for 3.5 hrs. After the reaction was completed, the mixture was concentrated *in vacuo* to give the title compound as a white solid (2.76 g, 99.5%), which was used for the next step without further purification. The compound was characterized by the following spectroscopic data:
¹H NMR (400 Hz, CDCl₃) *δ* (ppm): 3.68 (s, 3H), 3.58 (t, 1H), 3.56 (s, 1H), 3.32 (m, 1H), 3.02 (m, 1H), 2.77 (m, 1H), 2.52 (s, 1H), 2.21 (m, 1H), 1.96 (m, 1H).

### Step 2) the preparation of compound 31-3

To a stirred vigorously solution of benzyl chloroformate (3.7 mL, 26.3 mmol) and K₂CO₃ (10.6 g, 76.7 mmol) in a mixed solvent of THF (20 mL) and H₂O (10 mL) was added compound **31-2** (3.1 g, 17.1 mmol) in one portion. The reaction mixture was stirred at rt overnight. After the reaction was completed, the mixture was adjusted to pH 3 with diluted hydrochloric acid (1 M) and extracted with EtOAc (50 mL x 3). The combined organic layers were washed with brine, dried over anhydrous Na₂SO₄ and concentrated *in vacuo.* The residue was purified by silica gel column chromatography (PE/EtOAc (v/v) = 4/1) to give the title compound as pale yellow oil (3.0 g, 62.8%). The compound was characterized by the following spectroscopic data:
¹H NMR (400 Hz, CDCl₃) *δ* (ppm): 7.47 (d, 2H, *J* = 8.24 Hz), 7.38 (d, 2H, *J* = 8.24 Hz), 7.24 (m, 1H), 5.09 (s, 2H), 4.18 (t, 1H), 3.68 (s, 3H), 3.63 (m, 1H), 3.58 (s, 1H), 3.38 (m, 1H), 3.32 (m, 1H), 2.21 (m, 1H), 1.96 (m, 1H).

### Step 3) the preparation of compound 31-4

To a solution of compound **31-3** (1.0 g, 3.6 mmol) in DCM (20 mL) was added Dess-Martin periodinane (3.0 g, 7.1 mmol) in a portionwise manner at 0 °C. At the end of the addition, the mixture was stirred at rt for 1.0 hr. After the reaction was completed, the mixture was concentrated *in vacuo.* The residue was purified by silica gel column chromatography (PE/EtOAc (v/v) = 5/1) to give the title compound as yellow oil (0.79 g, 79.5%). The compound was characterized by the following spectroscopic data:
¹H NMR (400 Hz, CDCl₃) *δ* (ppm): 7.47 (d, 2H, *J* = 8.24 Hz), 7.38 (d, 2H, *J* = 8.24 Hz), 7.24 (m, 1H), 5.09 (s, 2H), 4.18 (t, 1H), 3.68 (s, 3H), 3.38 (m, 1H), 3.32 (m, 1H), 2.21 (m, 1H), 1.96 (m, 1H).

### Step 4) the preparation of compound 31-5

To a solution of compound **31-4** (998 mg, 3.6 mmol) in toluene (20 mL) were added ethylene glycol (0.8 mL, 15.7 mmol) and TsOH (0.14 g, 0.8 mmol) separately. At the end of the addition, the mixture was refluxed o vernight. After the reaction was completed, the mixture was diluted with EtOAc (50 mL), washed with saturated NaHCO₃ aqueous solution (30 mL) and brine (30 mL), dried over anhydrous Na₂SO₄ and concentrated *in vacuo.* The residue was purified by silica gel column chromatography (PE/EtOAc (v/v) = 6/1) to give the title compound as colorless liquid (0.54 g, 46.7%). The compound was characterized by the following spectroscopic data:
¹H NMR (400 Hz, CDCl₃) *δ* (ppm): 7.47 (d, 2H, *J* = 8.24 Hz), 7.38 (d, 2H, *J* = 8.24 Hz), 7.24 (m, 1H), 5.09 (s, 2H), 4.18 (t, 1H), 4.05 (m, 2H), 3.95 (m, 2H), 3.68 (s, 3H), 3.38 (m, 1H), 3.32 (m, 1H), 2.21 (m, 1H), 1.96 (m, 1H).

### Step 5) the preparation of compound 31-6

To a solution of compound **31-5** (0.59 g, 1.8 mmol) in MeOH (150 mL) was added a catalytic amount of Pd/C (0.5 g). At the end of the addition, the mixture was stirred at rt under H₂ overnight. After the reaction was completed, the mixture was filtered. The filtrate was concentrated *in vacuo* to give the title compound (0.34 g, 98.9%), which was used for the next step without further purification. The compound was characterized by the following spectroscopic data:
¹H NMR (400 Hz, CDCl₃) *δ* (ppm): 4.18 (t, 1H), 4.05 (m, 2H), 3.95 (m, 2H), 3.68 (s, 3H), 3.38 (m, 1H), 3.32 (m, 1H), 2.21 (m, 1H), 1.96 (m, 1H).

### Step 6) the preparation of compound 31-7

To a suspension of compound **31-6** (3.48 g, 18.6 mmol), compound **1-11-2** (3.26 g, 18.6 mmol) and EDCI (7.1 g, 37 mmol) in DCM (50 mL) was added DIPEA (12.3 mL, 74.4 mmol) dropwise at 0 °C. At the end of the addition, the mixture was stirred at rt overnight. After the reaction was completed, the mixture was washed with brine, dried over anhydrous Na₂SO₄ and concentrated *in vacuo.* The residue was purified by silica gel column chromatography (PE/EtOAc (v/v) = 3/1) to give the title compound as pale yellow oil (2.5 g, 39.1%). The compound was characterized by the following spectroscopic data:
¹H NMR (400 Hz, CDCl₃) *δ* (ppm): 9.80 (s, 1H), 4.54 (d, 1H, *J* = 7.25 Hz), 4.28 (m, 1H), 4.06 (m, 4H), 3.76 (m, 2H), 3.50 (s, 3H), 3.45 (s, 3H), 2.71 (m, 2H), 2.65 (m, 1H), 0.87 (m, 3H), 0.81 (m, 3H).

### Step 7) the preparation of compound 31-8

To a solution of compound **31-7** (0.9 g, 2.6 mmol) in THF (5.0 mL) was added a solution of LiOH (0.12 g, 5.0 mmol) in water (5.0 mL). At the end of the addition, the mixture was stirred at rt overnight. After the reaction was completed, the mixture was adjusted to pH 2 with diluted hydrochloric acid (1 M), and extracted with EtOAc (50 mL x 3). The combined organic layers were washed with brine, dried over anhydrous Na₂SO₄ and concentrated *in vacuo* to give the title compound as a white solid (0.85 g, 99.0%), which was used for the next step without further purification. The compound was characterized by the following spectroscopic data:
¹H NMR (400Hz, CD₃Cl) *δ* (ppm): 9.80 (s, 1H), 4.54 (d, 1H, *J* = 7.25 Hz), 4.28 (m, 1H), 4.06 (m, 4H), 3.76 (m, 2H), 3.50 (s, 3H), 2.71 (m, 2H), 2.65 (m, 1H), 0.87 (m, 3H), 0.81 (m, 3H).

### Step 8) the preparation of compound 31-9

To a mixture of compound **3-6** (1.64 g, 5.9 mmol) and compound **31-8** (1.78 g, 5.4 mmol) in MeCN (30.0 mL) was added DIPEA (1.1 mL, 6.7 mmol) dropwise at 0 °C. At the end of the addition, the mixture was stirred at rt for 3.0 hrs. After the reaction was completed, the mixture was concentrated *in vacuo.* The residue was purified by silica gel column chromatography (PE/EtOAc (v/v) = 3/1) to give the title compound as a pale yellow solid (2.76 g, 97.3%). The compound was characterized by the following spectroscopic data:
¹H NMR (400 MHz, CDCl₃) *δ* (ppm): 7.82-7.78 (m, 2H), 7.67-7.64 (m, 2H), 5.32, 5.29 (d, d, 1H), 5.22 (s, 2H), 5.06-5.02 (m, 1H), 4.36-4.31 (m, 1H), 4.02-4.00 (m, 4H), 3.81-3.77 (m, 1H), 3.63 (s, 3H), 3.56-3.51 (m, 1H), 2.79-2.73 (m, 1H), 2.41-2.34 (m, 1H), 2.18-2.06 (m, 1H), 0.97-0.95 (m, 3H), 0.91-0.89 (m, 3H).

### Step 9) the preparation of compound 31-10

A suspension of compound **31-9** (3.0 g, 5.7 mmol) and NH₄OAc (4.4 g, 57.1 mmol) in xylene (20 mL) was stirred at 130 °C overnight. After the reaction was completed, the mixture was cooled to rt and diluted with EtOAc (40 mL). The resulting mixture was washed with brine, dried over anhydrous Na₂SO₄ and concentrated *in vacuo.* The residue was purified by silica gel column chromatography (PE/EtOAc (v/v) = 3/1) to give the title compound as a yellow solid (2.6 g, 89.9%). The compound was characterized by the following spectroscopic data:
¹H NMR (400 MHz, CDCl₃) *δ* (ppm): 7.45-7.41 (m, 2H), 7.29-7.26 (m, 3H), 5.40-5.36 (m, 1H), 5.32, 5.29 (d, d, 1H), 4.42-4.38 (m, 1H), 3.98-3.92 (m, 5H), 3.71-3.69 (m, 1H), 3.63 (s, 3H), 2.83-2.77 (m, 1H), 2.45-2.39 (m, 1H), 2.24-2.11 (m, 1H), 0.97-0.95 (m, 3H), 0.91-0.89 (m, 3H).

### Step 10) the preparation of compound 31-11

A suspension of compound **31-10** (4.0 g, 7.9 mmol), compound **1-12-2** (3.11 g, 12.2 mmol), Pd(dppf)Cl₂·CH₂Cl₂ (0.64 g, 0.8 mmol) and KOAc (1.94 g, 19.8 mmol) in DME (50 mL) was stirred at 90 °C under N₂ for 4.0 hrs. After the reaction was completed, the mixture was cooled to rt, diluted with EtOAc (200 mL) and filtered through a celite pad. The filtrate was washed with water (100 mL x 3) and brine, dried over anhydrous Na₂SO₄ and concentrated *in vacuo.* The residue was purified by silica gel column chromatography (PE/DCM (v/v) = 4/1) to give the title compound as a white solid (4.15 g, 94.7%). The compound was characterized by the following spectroscopic data:
¹H NMR (400 MHz, CDCl₃) *δ* (ppm): 7.64-7.57 (m, 4H), 7.22 (s, 1H), 5.40-5.36 (m, 1H), 5.32, 5.29 (d, d, 1H), 4.42-4.38 (m, 1H), 3.98-3.96 (m, 5H), 3.71-3.69, 3.67-3.66 (m, 1H), 3.63 (s, 3H), 2.83-2.77 (m, 1H), 2.45-2.39 (m, 1H), 2.24-2.11 (m, 1H), 1.35, 1.32 (m, m, 12H), 0.97-0.95 (m, 3H), 0.91-0.89 (m, 3H).

### Step 11) the preparation of compound 31-12

A suspension of compound **31-11** (1.3 g, 2.34 mmol), compound **12-5** (1.07 g, 2.4 mmol), Pd(PPh₃)₄ (0.14 g, 0.12 mmol) and K₂CO₃ (0.8 g, 5.85 mmol) in mixed solvents of DME and H₂O (16 mL, v/v = 3/1) was stirred at 90 °C under N₂ for 3.0 hrs. After the reaction was completed, the mixture was diluted with EtOAc (30 mL), washed with brine, dried over anhydrous Na₂SO₄ and concentrated *in vacuo.* The residue was purified by recrystallization to give the title compound as a white solid (1.4 g, 80%). The compound was characterized by the following spectroscopic data:
MS (ESI, pos.ion) m/z: 747.3 [M+H]⁺;
¹H NMR (400 MHz, CDCl₃) *δ* (ppm): 7.54-7.51 (m, 2H), 7.45-7.42 (m, 2H), 7.35 (s, 1H), 7.27, 7.25 (s, s, 1H), 6.97, 6.95 (dd, dd, 1H), 5.56, 5.55 (d, d, 1H), 5.40-5.36 (m, 1H), 4.35-4.31 (m, 1H), 3.99-3.96, 3.94-3.92 (m, m, 5H), 3.71-3.69 (m, 1H), 3.66 (s, 3H), 3.00-2.96 (m, 2H), 2.83-2.77 (m, 1H), 2.44-2.34 (m, 3H), 2.29-2.18 (m, 5H), 1.76-1.59 (m, 4H), 1.02, 1.01 (m, m, 3H), 0.94, 0.92 (m, 3H).

### Step 12) the preparation of compound 31-13

A suspension of compound **31-12** (1.343 g, 1.8 mmol), compound **1-12-2** (0.92 g, 3.6 mmol), Pd(dppf)Cl₂·CH₂Cl₂ (0.29 g, 0.36 mmol) and KOAc (0.53 g, 5.4 mmol) in DME (15 mL) was stirred at 90 °C under N₂ for 3.0 hrs. After the reaction was completed, the mixture was diluted with EtOAc (100 mL) and filtered through a celite pad. The filtrate was washed with water and brine, dried over anhydrous Na₂SO₄ and concentrated *in vacuo.* The residue was purified by recrystallization to give the title compound as a white solid (1.11 g, 85%). The compound was characterized by the following spectroscopic data:
MS (ESI, pos.ion) *m*/*z*: 725.4 [M+H]⁺;
¹H NMR (400 MHz, CDCl₃) *δ* (ppm): 7.59, 7.57 (dd, dd, 1H), 7.54-7.51 (m, 2H), 7.50-7.46 (m, 2H), 7.45, 7.44 (s, s, 1H), 7.35 (s, 1H), 5.40-5.36 (m, 1H), 5.32, 5.29 (d, d, 1H), 4.43-4.38 (m, 1H), 3.99-3.96, 3.94-3.92 (m, m, 5H), 3.71-3.69 (m, 0.5H), 3.67-3.66 (m, 0.5H), 3.63 (s, 3H), 2.94-2.90 (m, 2H), 2.83-2.77 (m, 1H), 2.45-2.33 (m, 3H), 2.30-2.23 (m, 2H), 2.22-2.11 (m, 1H), 1.99-1.90 (m, 2H), 1.77-1.60 (m, 4H), 1.32 (m, 6H), 1.29 (m, 6H), 0.97, 0.95 (m, m, 3H), 0.91, 0.89 (m, m, 3H).

### Step 13) the preparation of compound 31-14

A suspension of compound **31-13** (1.014 g, 1.4 mmol), compound **7-2** (0.72 g, 1.7 mmol), Pd(PPh₃)₄ (0.16 g, 0.14 mmol) and K₂CO₃ (0.58 g, 4.2 mmol) in mixed solvents of EtOH and H₂O (16 mL, v/v = 3/1) was stirred at 90 °C under N₂ for 3.0 hrs. After the reaction was completed, the mixture was diluted with EtOAc (100 mL). The resulting mixture was washed with brine, dried over anhydrous Na₂SO₄ and concentrated *in vacuo.* The residue was purified by recrystallization to give the title compound as a white solid (436.3 mg, 35%). The compound was characterized by the following spectroscopic data:
MS (ESI, pos.ion) *m*/*z*: 891.5 [M+H]⁺;
¹H NMR (400 MHz, CDCl₃) *δ* (ppm): 7.71 (s, 1H), 7.54-7.51 (m, 3H), 7.49-7.46 (m, 2H), 7.35 (s, 1H), 7.18, 7.16 (s, s, 1H), 5.56, 5.55 (d, d, 1H), 5.40-5.36 (m, 1H), 5.32-5.28 (m, 2H), 4.43-4.38 (m, 1H), 4.34-4.30 (m, 1H), 3.98-3.96, 3.94-3.92 (m, m, 5H), 3.71-3.69 (m, 1H), 3.68-3.67 (m, 1H), 3.66 (s, 3H), 3.63 (s, 3H), 2.85-2.77 (m, 3H), 2.45-2.39 (m, 1H), 2.37-1.86 (m, 12H), 1.76-1.58 (m, 4H), 1.02-0.89 (m, 12H).

### Example 32

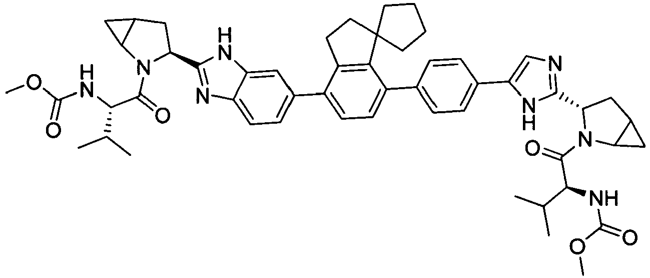

### Synthetic route:

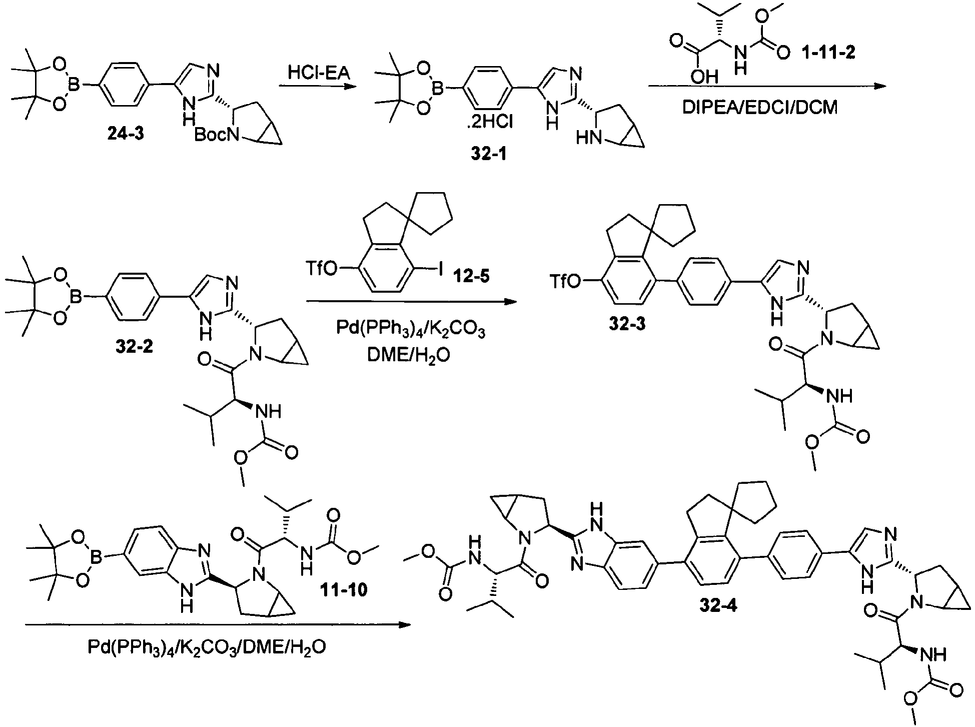

### Step 1) the preparation of compound 32-1

To a solution of compound **24-3** (4.51 g, 10 mmol) in EtOAc (40 mL) was added a solution of HCl in EtOAc (40 mL, 4 M) dropwise. At the end of the addition, the mixture was stirred at rt for 8.0 hrs. After the reaction was completed, the mixture was concentrated *in vacuo.* The residue was purified by beating in EtOAc (20 mL) and filtered to give the title compound as a pale yellow solid (3.82 g, 90%). The compound was characterized by the following spectroscopic data:
MS (ESI, pos.ion) *m*/*z*: 352.5 [M+H]⁺.

### Step 2) the preparation of compound 32-2

A suspension of compound **32-1** (2.86 g, 6.74 mmol), compound **1-11-2** (2.36 g, 13.5 mmol) and EDCI (2.71 g, 14.15 mmol) in DCM (40 mL) was added DIPEA (9.3 mL, 56.3 mmol) dropwise at 0 °C. At the end of the addition, the mixture was stirred at rt for 3.0 hrs. After the reaction was completed, the mixture was diluted with DCM (50.0 mL), washed with NH₄Cl aqueous solution and brine, dried over anhydrous Na₂SO₄ and concentrated *in vacuo.* The residue was purified by silica gel column chromatography (PE/EtOAc (v/v) = 1/2) to give the title compound as a white solid (2.055 g, 60%). The compound was characterized by the following spectroscopic data:
MS (ESI, pos.ion) *m*/*z*: 509.3 [M+H]⁺;
¹H NMR (400 MHz, CDCl₃) *δ* (ppm): 7.64-7.61 (m, 2H), 7.60-7.57 (m, 2H), 7.29 (s, 1H), 5.46, 5.44 (d, d, 1H), 4.89-4.85 (m, 1H), 4.09-4.04 (m, 1H), 3.66 (s, 3H), 3.45-3.38 (m, 1H), 2.46-2.39 (m, 1H), 2.22-2.09 (m, 1H), 2.00-1.94 (m, 1H), 1.43-1.36 (m, 1H), 1.35 (m, 6H), 1.32 (m, 6H), 0.97, 0.95 (m, m, 3H), 0.94-0.92 (m, 1H), 0.91, 0.89 (m, m, 3H), 0.50-0.46 (m, 1H).

### Step 3) the preparation of compound 32-3

A suspension of compound **32-2** (1.189 g, 2.34 mmol), compound **12-5** (1.07 g, 2.4 mmol), Pd(PPh₃)₄ (0.14 g, 0.12 mmol) and K₂CO₃ (0.8 g, 5.85 mmol) in mixed solvents of DME and H₂O (16 mL, v/v = 3/1) was stirred at 90 °C under N₂ for 3.0 hrs. After the reaction was completed, the mixture was diluted with EtOAc (30 mL), washed with water and brine, dried over anhydrous Na₂SO₄ and concentrated *in vacuo.* The residue was purified by silica gel column chromatography (PE/EtOAc (v/v) = 1/2) to give the title compound as a white solid (1.229 g, 75%). The compound was characterized by the following spectroscopic data:
MS (ESI, pos.ion) *m*/*z*: 701.3 [M+H]⁺;
¹H NMR (400 MHz, CDCl₃) *δ* (ppm): 7.62 (s, 1H), 7.54-7.51 (m, 2H), 7.45-7.42 (m, 2H), 7.27, 7.25 (s, s, 1H), 6.97, 6.95 (dd, dd, 1H), 5.46, 5.44 (d, d, 1H), 4.89-4.85 (m, 1H), 4.09-4.04 (m, 1H), 3.66 (s, 3H), 3.45-3.38 (m, 1H), 3.00-2.96 (m, 2H), 2.46-2.34 (m, 3H), 2.29-2.20 (m, 4H), 2.19-2.09 (m, 1H), 2.00-1.94 (m, 1H), 1.76-1.59 (m, 4H), 1.43-1.36 (m, 1H), 0.97, 0.95 (m, m, 3H), 0.94-0.92 (m, 1H), 0.91, 0.89 (m, m, 3H), 0.50-0.46 (m, 1H).

### Step 4) the preparation of compound 32-4

A suspension of compound **32-3** (1.638 g, 2.34 mmol), compound **11-10** (1.157 g, 2.4 mmol), Pd(PPh₃)₄ (0.14 g, 0.12 mmol) and K₂CO₃ (0.8 g, 5.85 mmol) in mixed solvents of DME and H₂O (16 mL, v/v = 3/1) was stirred at 90 °C under N₂ for 3.0 hrs. After the reaction was completed, the mixture was diluted with EtOAc (30 mL), washed with water and brine, dried over anhydrous Na₂SO₄ and concentrated *in vacuo.* The residue was purified by silica gel column chromatography (DCM/MeOH (v/v) = 50/1) to give the title compound as a white solid (1.06 g, 50%). The compound was characterized by the following spectroscopic data:
MS (ESI, pos.ion) *m*/*z*: 455.3 [M+2H]²⁺;
¹H NMR (400 MHz, CDCl₃) *δ* (ppm): 7.62 (s, 1H), 7.60, 7.58 (s, s, 1H), 7.56-7.51 (m, 6H), 7.49-7.46 (m, 2H), 5.46, 5.44 (d, d, 1H), 5.32, 5.29 (d, d, 1H), 5.14-5.10 (m, 1H), 4.89-4.85 (m, 1H), 4.13-4.04 (m, 2H), 3.66 (s, 3H), 3.63 (s, 3H), 3.45-3.36 (m, 2H), 3.10-3.06 (m, 2H), 2.62-2.55 (m, 1H), 2.46-2.35 (m, 3H), 2.30-2.20 (m, 4H), 2.18-2.11 (m, 3H), 2.00-1.94 (m, 1H), 1.76-1.58 (m, 4H), 1.53-1.45 (m, 1H), 1.43-1.36 (m, 1H), 0.97, 0.95 (m, m, 6H), 0.95-0.92 (m, 2H), 0.91, 0.89 (m, m, 6H), 0.50-0.46 (m, 2H).

### Example 33

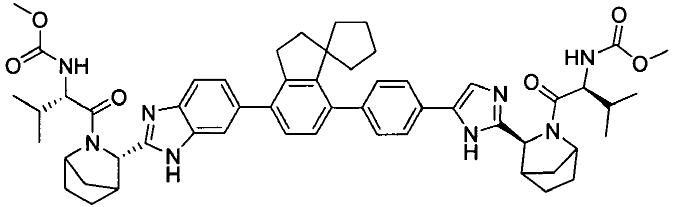

### Synthetic route:

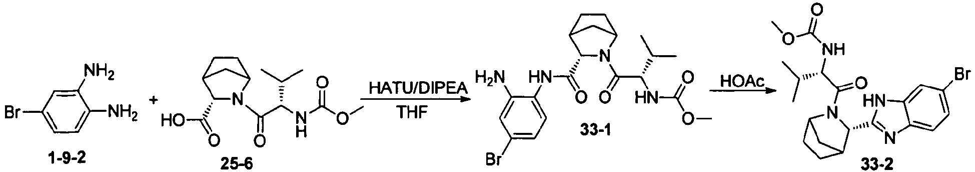

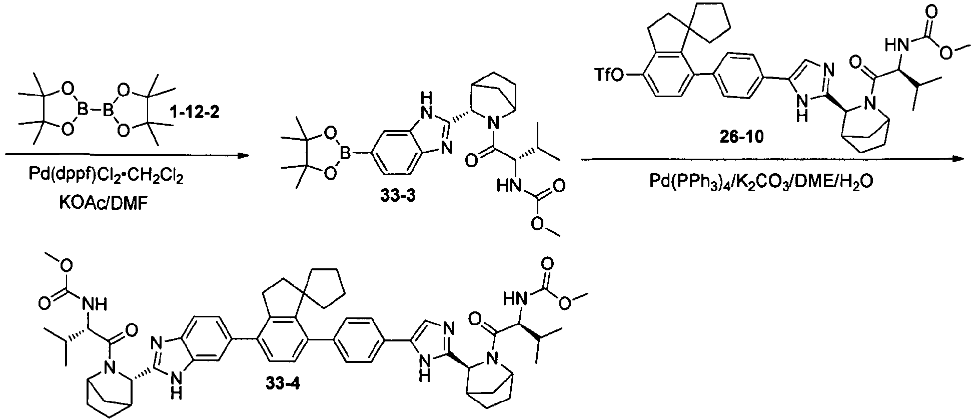

### Step 1) the preparation of compound 33-1

To a suspension of compound **25-6** (582.6 mg, 1.9542 mmol) and HATU (0.782 g, 2.0566 mmol) in THF (20 mL) was added DIPEA (0.41 mL, 2.481 mmol) dropwise at 0 °C. After stirring at 0 °C for 0.5 hr, a solution of compound **1-9-2** (0.4024 g, 2.152 mmol) in THF (10 mL) was added to the mixture. At the end of the addition, the mixture was stirred at rt for 2.0 hrs. After the reaction was completed, the THF solvent was removed and 20 mL of water was added. The resulting mixture was extracted with EtOAc (30 mL x 3). The combined organic layers were washed with brine, dried over anhydrous Na₂SO₄ and concentrated *in vacuo.* The residue was purified by silica gel column chromatography (PE/EtOAc (v/v) = 4/1) to give the title compound as brown oil (774 mg, 85%).

### Step 2) the preparation of compound 33-2

A solution of compound **33-1** (1.34 g, 2.87 mmol) in glacial acetic acid (40 mL) was stirred at 40 °C overnight. After the reaction was completed, HOAc was removed. The residue was dissolved in EtOAc (150 mL). The resulting mixture was washed with Na₂CO₃ aqueous solution (100 mL x 3), dried over anhydrous Na₂SO₄ and concentrated *in vacuo.* The residue was purified by silica gel column chromatography (PE/EtOAc (v/v) = 1/1) to give the title compound as a brown solid (874.5 mg, 68%). The compound was characterized by the following spectroscopic data:
MS (ESI, pos.ion) *m*/*z*: 450.3 [M+H]⁺;
¹H NMR (400 MHz, CDCl₃) *δ* (ppm): 7.59-7.52 (m, 1H), 7.32-7.21 (m, 2H), 5.41-5.38 (m, 2H), 4.35-4.32 (m, 1H), 3.87-3.76 (m, 1H), 3.70 (s, 3H), 3.66-3.62 (m, 1H), 2.67-2.65 (m, 1H), 2.20-2.13 (m, 1H), 1.85-1.73 (m, 4H), 1.46-1.43 (m, 2H), 0.88-0.84 (m, 6H).

### Step 3) the preparation of compound 33-3

To a mixture of compound **33-2** (0.147 g, 0.327 mmol), compound **1-12-2** (0.125 g, 0.4922 mmol), Pd(dppf)Cl₂·CH₂Cl₂ (0.027 g, 0.0327 mmol) and KOAc (0.097 g, 0.9884 mmol) was added DMF (5.0 mL) via syringe under N₂, and the mixture was stirred at 90 °C for 4.0 hrs. After the reaction was completed, the mixture was cooled to rt, diluted with EtOAc (40 mL) and filtered through a celite pad. The filtrate was washed with water (30 mL x 3) and brine, dried over anhydrous Na₂SO₄ and concentrated *in vacuo.* The residue was purified by silica gel column chromatography (PE/EtOAc (v/v) = 1/2) to give the title compound as a beige solid (0.09 g, 55.5%). The compound was characterized by the following spectroscopic data:
MS (ESI, pos.ion) *m*/*z*: 497.3 [M+H]⁺;
¹H NMR (400 MHz, CDCl₃) *δ* (ppm): 7.85-7.80 (m, 1H), 7.72-7.68 (m, 2H), 5.45-5.41 (m, 2H), 4.56-4.48 (m, 1H), 4.33-4.30 (m, 1H), 3.86-3.84 (m, 1H), 3.70 (s, 3H), 3.64-3.62 (m, 1H), 3.04-2.98 (m, 1H), 2.25-2.20 (m, 1H), 2.20-2.13 (m, 2H), 1.87-1.76 (m, 1H), 1.46-1.49 (m, 2H), 1.35 (s, 12H), 0.88-0.84 (m, 6H).

### Step 4) the preparation of compound 33-4

A suspension of compound **26-10** (49.96 mg, 0.06995 mmol), compound **33-3** (39 mg, 0.0786 mmol), Pd(PPh₃)₄ (8.0 mg, 0.007 mmol) and K₂CO₃ (30 mg, 0.21 mmol) in mixed solvents of DME and H₂O (6.0 mL, v/v = 5/1) was stirred at 90 °C under N₂ for 3.0 hrs. After the reaction was completed, the mixture was cooled to rt and diluted with EtOAc (25.0 mL). The resulting mixture was washed with water (20.0 mL x 3) and brine, dried over anhydrous Na₂SO₄ and concentrated *in vacuo.* The residue was purified by silica gel column chromatography (DCM/MeOH (v/v) = 50/1) to give the title compound as a beige solid (32.68 mg, 50%). The compound was characterized by the following spectroscopic data:
MS (ESI, pos.ion) *m*/*z*: 468.3 [M+2H]²⁺;
¹H NMR (400 MHz, CDCl₃) *δ* (ppm): 7.60 (s, 1H), 7.59, 7.58 (s, s, 1H), 7.57-7.52 (m, 5H), 7.49-7.46 (m, 2H), 7.38, 7.36 (d, d, 1H), 5.56, 5.55 (d, d, 1H), 5.39-5.35 (m, 1H), 5.32, 5.29 (d, d, 1H), 5.06-5.02 (m, 1H), 4.89-4.75 (m, 1H), 4.73-4.69 (m, 1H), 4.44-4.40 (m, 1H), 4.21-4.17 (m, 1H), 3.66 (s, 3H), 3.63 (s, 3H), 3.10-3.06 (m, 2H), 2.58-2.50 (m, 2H), 2.43-2.35 (m, 2H), 2.30-2.10 (m, 6H), 2.08-2.00 (m, 1H), 1.84-1.55 (m, 12H), 1.47-1.35 (m, 2H), 1.31-1.24 (m, 1H), 1.02-0.89 (m, 12H).

### Example 34

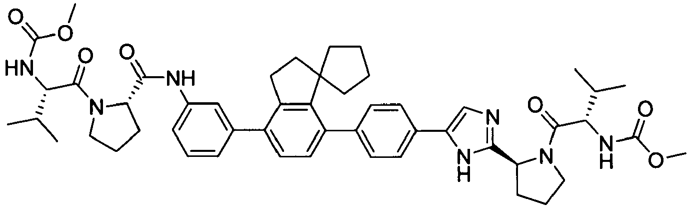

### Synthetic route:

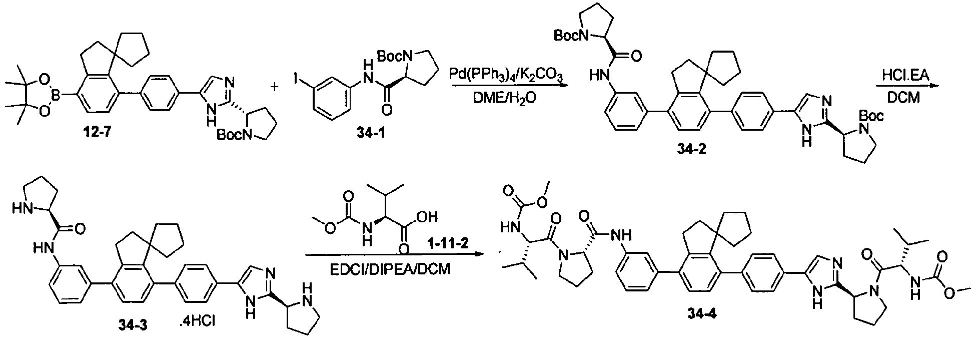

### Step 1) the preparation of compound 34-2

A suspension of compound **12-7** (2.096 g, 3.44 mmol), compound **34-1** (1.58 g, 3.78 mmol), Pd(PPh₃)₄ (397 mg, 0.34 mmol) and K₂CO₃ (1.19 g, 8.62 mmol) in mixed solvents of DME and H₂O (16 mL, v/v = 3/1) was stirred at 90 °C under N₂ for 4.0 hrs. After the reaction was completed, the mixture was cooled to rt and diluted with EtOAc (100 mL). The resulting mixture was washed with water (30 mL x 3) and brine, dried over anhydrous Na₂SO₄ and concentrated *in vacuo.* The residue was purified by silica gel column chromatography (PE/EtOAc (v/v) = 4/1) to give the title compound as a yellow solid (2.12 g, 80%). The compound was characterized by the following spectroscopic data:
MS (ESI, pos.ion) *m*/*z*: 772.3 [M+H]⁺;
¹H NMR (400 MHz, CDCl₃) *δ* (ppm): 8.96 (brs, 1H), 7.81-7.80 (m, 1H), 7.59 (s, 1H), 7.55-7.51 (m, 3H), 7.49-7.43 (m, 3H), 7.39-7.36 (m, 1H), 7.32-7.27 (m, 2H), 4.98-4.92 (m, 1H), 4.50-4.46 (m, 1H), 3.64-3.57 (m, 1H), 3.53-3.39 (m, 2H), 3.31-3.24 (m, 1H), 2.84-2.80 (m, 2H), 2.47-2.35 (m, 3H), 2.31-1.88 (m, 11H), 1.76-1.58 (m, 4H), 1.53 (s, 9H), 1.43 (s, 9H).

### Step 2) the preparation of compound 34-3

To a solution of compound **34-2** (2.175 g, 2.82 mmol) in DCM (15 mL) was added a solution of HCl in EtOAc (20 mL, 4 M) dropwise. At the end of the addition, the mixture was stirred at rt for 8.0 hrs. After the reaction was completed, the mixture was concentrated *in vacuo.* The residue was purified by beating in EtOAc (20.0 mL) and filtered to give the title compound as a yellow solid (1.92 g, 95%). The compound was characterized by the following spectroscopic data:
MS (ESI, pos.ion) *m*/*z*: 572.5 [M+H]⁺.

### Step 3) the preparation of compound 34-4

To a suspension of compound **34-3** (717.3 mg, 1.0 mmol), compound **1-11-2** (525 mg, 3.0 mmol) and EDCI (958 mg, 5.0 mmol) in DCM (20.0 mL) was added DIPEA (1.65 mL, 10 mmol) dropwise at 0 °C. At the end of the addition, the mixture was stirred at rt for 3.0 hrs. After the reaction was completed, the mixture was diluted with DCM (30.0 mL), washed with NH₄Cl aqueous solution and brine, dried over anhydrous Na₂SO₄ and concentrated *in vacuo.* The residue was purified by silica gel column chromatography (DCM/MeOH (v/v) = 80/1) to give the title compound as a pale yellow solid (442.7 mg, 50%). The compound was characterized by the following spectroscopic data:
MS (ESI, pos.ion) *m*/*z*: 886.5 [M+H]⁺;
¹H NMR (400 MHz, CDCl₃) *δ* (ppm): 8.90 (brs, 1H), 7.83-7.82 (m, 1H), 7.59 (s, 1H), 7.55-7.51 (m, 3H), 7.49-7.43 (m, 3H), 7.39-7.36 (m, 1H), 7.32, 7.30 (s, s, 1H), 7.22-7.18 (m, 1H), 5.56, 5.55 (d, d, 1H), 5.32-5.30 (d, d, 1H), 5.23-5.19 (m, 1H), 4.41-4.36 (m, 1H), 4.33-4.29 (m, 1H), 4.28-4.23 (m, 1H), 3.85-3.78 (m, 1H), 3.68-3.67 (m, 1H), 3.66 (s, 3H), 3.63 (s, 3H), 3.61-3.55 (m, 1H), 3.44-3.36 (m, 1H), 2.84-2.80 (m, 2H), 2.43-2.35 (m, 2H), 2.30-1.92 (m, 14H), 1.76-1.58 (m, 4H), 1.02-0.89 (m, 12H).

### Example 35

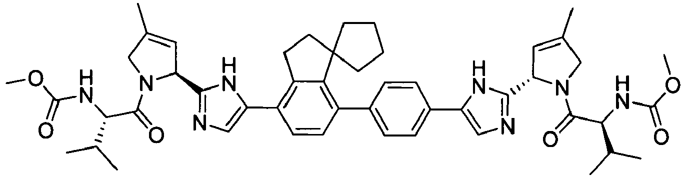

### Synthetic route:

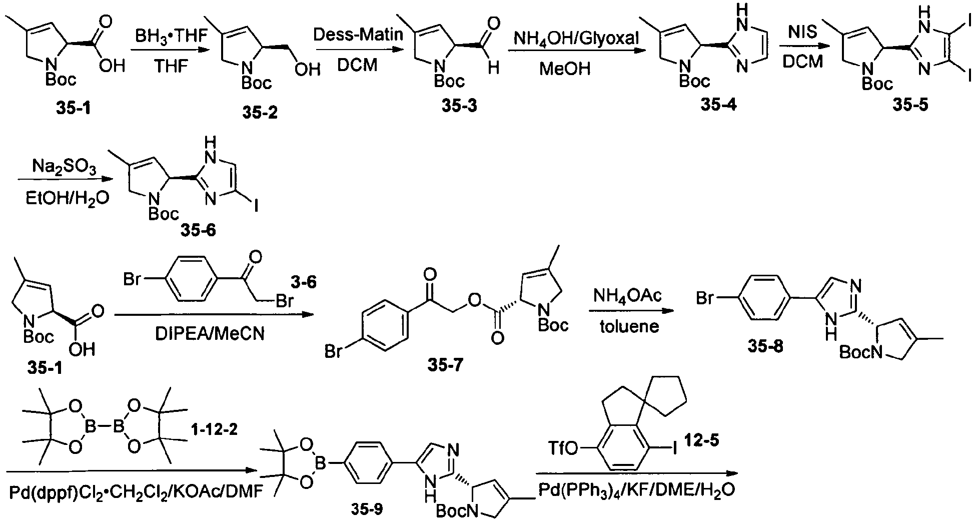

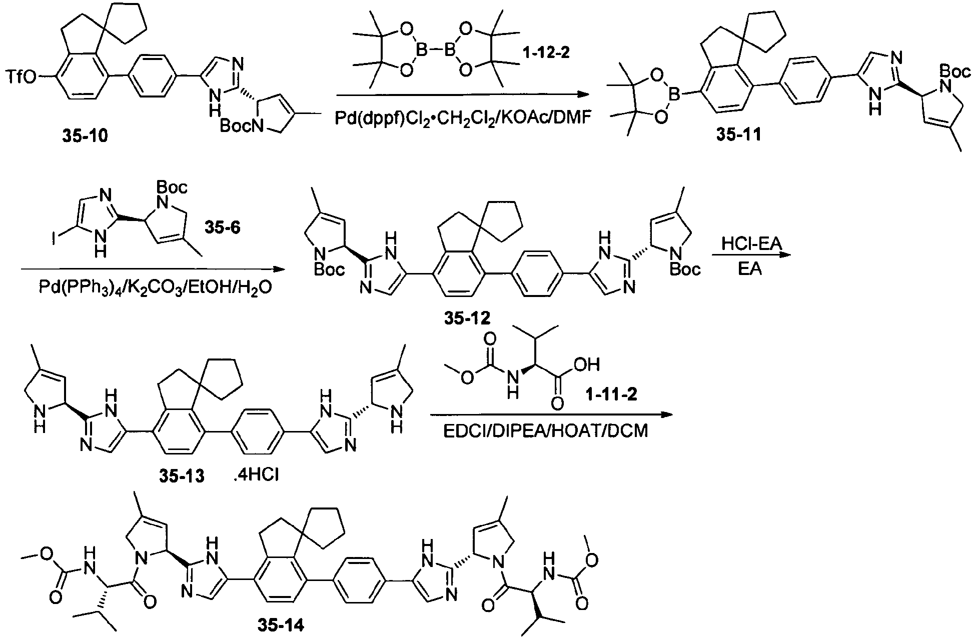

### Step 1) the preparation of compound 35-2

To a solution of compound **35-1** (10.58 g, 46.6 mmol) in THF (100 mL) was added diborane (100 mL, 1M in THF) dropwise at 0 °C under N₂. At the end of the addition, the mixture was stirred at 0 °C for 3.0 hrs. After the reaction was completed, the mixture was quenched with MeOH (80 mL) and concentrated *in vacuo.* The residue was purified by silica gel column chromatography (PE/EtOAc (v/v) = 3/2) to give the title compound as colorless oil (7.45 g, 75%). The compound was characterized by the following spectroscopic data:
¹H NMR (400 MHz, CDCl₃) *δ* (ppm): 5.32-5.29 (m, 1H), 4.63-4.54 (m, 1H), 4.16-4.09 (m, 1H), 3.97-3.92 (m, 1H), 3.88-3.82 (m, 1H), 3.80-3.79 (m, 1H), 3.22 (br, 1H), 1.62-1.61 (m, 3H), 1.43 (s, 9H).

### Step 2) the preparation of compound 35-3

To a solution of compound **35-2** (7.42 g, 34.8 mmol) in DCM (250 mL) was added Dess-Martin periodinane (20.7 g, 48.8 mmol) in a portionwise manner at 0 °C. At the end of the addition, the mixture was stirred at rt for 2.0 hrs. After the reaction was completed, the mixture was quenched with water (150 mL) and filtered. The filtrate was washed with brine, dried over anhydrous Na₂SO₄ and concentrated *in vacuo.* The residue was purified by silica gel column chromatography (PE/EtOAc (v/v) = 3/1) to give the title compound as colorless oil (3.72 g, 50.7%). The compound was characterized by the following spectroscopic data:
¹H NMR (400 MHz, CDCl₃) *δ* (ppm): 9.77-9.75 (m, 1H), 5.41-5.38 (m, 1H), 4.64-4.59 (m, 1H), 4.24-4.17 (m, 1H), 3.96-3.89 (m, 1H), 1.65-1.64 (m, 3H), 1.44 (s, 9H).

### Step 3) the preparation of compound 35-4

To a solution of compound **35-3** (3.71 g, 17.6 mmol) and ammonia (13.0 mL) in MeOH (30.0 mL) was added glyoxal (8.0 mL, 40% in water) dropwise at 0 °C. At the end of the addition, the mixture was stirred at rt overnight. After the reaction was completed, the mixture was concentrated *in vacuo.* The residue was purified by silica gel column chromatography (PE/EtOAc (v/v) = 4/1) to give the title compound as a white solid (2.08 g, 47.6%). The compound was characterized by the following spectroscopic data:
MS (ESI, pos.ion) *m*/*z*: 250.5 [M+H]⁺;
¹H NMR (400 MHz, CDCl₃) *δ* (ppm) : 7.05 (s, 2H), 6.32-6.28 (m, 1H), 5.38-5.35 (m, 1H), 4.23-4.17 (m, 1H), 3.86-3.80 (m, 1H), 1.68-1.67 (m, 3H), 1.40 (s, 9H).

### Step 4) the preparation of compound 35-5

To a solution of compound **35-4** (2.09 g, 8.4 mmol) in DCM (60.0 mL) was added *N*-iodosuccinimide (3.8 g, 16.8 mmol) at 0 °C in a portionwise manner. At the end of the addition, the mixture was stirred at 0 °C for 1.5 hrs. After the reaction was completed, the mixture was washed with brine, dried over anhydrous Na₂SO₄ and concentrated *in vacuo.* The residue was purified by silica gel column chromatography (PE/EtOAc (v/v) = 3/2) to give the title compound as a white solid (2.65 g, 63%). The compound was characterized by the following spectroscopic data:
MS (ESI, pos.ion) *m*/*z*: 501.5 [M+H]⁺;
¹H NMR (400 MHz, CDCl₃) *δ* (ppm): 5.52-5.45 (m, 2H), 4.35-4.29 (m, 1H), 3.94-3.88 (m, 1H), 1.67-1.66 (m, 3H), 1.40 (s, 9H).

### Step 5) the preparation of compound 35-6

To a suspension of compound **35-5** (1.638 g, 3.27 mmol) in mixed solvents of ethanol and water (50.0 mL, v/v = 3/7) was added Na₂SO₃ (3.7 g, 29 mmol), and the mixture was refluxed for 17.0 hrs. After the reaction was completed, the ethanol solvent was removed, and to the mixture was added water (50.0 mL). The resulting mixture was extracted with EtOAc (50.0 mL x 3). The combined organic layers were washed with brine, dried over anhydrous Na₂SO₄ and concentrated *in vacuo.* The residue was purified by silica gel column chromatography (PE/EtOAc (v/v) = 3/2) to give the title compound as a white solid (1.03 g, 84%). The compound was characterized by the following spectroscopic data:
MS (ESI, pos.ion) *m*/*z*: 376.5 [M+H]⁺;
¹H NMR (400 MHz, CDCl₃) *δ* (ppm): 7.18 (s, 1H), 5.35-5.32 (m, 1H), 5.28-5.24 (m, 1H), 4.29-4.23 (m, 1H), 3.91-3.85 (m, 1H), 1.67-1.66 (m, 3H), 1.40 (s, 9H).

### Step 6) the preparation of compound 35-7

To a solution of compound **3-6** (2.977 g, 10.79 mmol) and compound **35-1** (2.69 g, 11.87 mmol) in MeCN (250 mL) was added DIPEA (2.14 mL, 12.95 mmol) dropwise at 0 °C. At the end of the addition, the mixture was stirred at rt for 3.0 hrs. After the reaction was completed, the MeCN solvent was removed, and to the mixture was added water (100 mL). The resulting mixture was extracted with EtOAc (100 mL x 3). The combined organic layers were washed with brine, dried over anhydrous Na₂SO₄ and concentrated *in vacuo.* The residue was purified by silica gel column chromatography (PE/EtOAc (v/v) = 10/1) to give the title compound as a white solid (4.1 g, 90%). The compound was characterized by the following spectroscopic data:
MS (ESI, pos.ion) *m*/*z*: 424.5 [M+H]⁺;
¹H NMR (400 MHz, CDCl₃) *δ* (ppm): 7.82-7.78 (m, 2H), 7.67-7.64 (m, 2H), 5.61-5.59 (m, 1H), 5.33 (s, 2H), 4.73-4.69 (m, 1H), 4.35-4.28 (m, 1H), 3.99-3.92 (m, 1H), 1.76-1.74 (m, 3H), 1.42 (s, 9H).

### Step 7) the preparation of compound 35-8

A mixture of compound **35-7** (1.54 g, 3.64 mmol) and ammonium acetate (4.2 g, 5.46 mmol) in toluene (30.0 mL) was stirred at 110 °C for 5.0 hrs. After the reaction was completed, the mixture was cooled to rt and quenched with water (50.0 mL). The resulting mixture was extracted with EtOAc (50.0 mL x 3). The combined organic layers were washed with brine, dried over anhydrous Na₂SO₄ and concentrated *in vacuo.* The residue was purified by silica gel column chromatography (PE/EtOAc (v/v) = 6/1) to give the title compound as a pale yellow solid (1.25 g, 85%). The compound was characterized by the following spectroscopic data:
MS (ESI, pos.ion) *m*/*z*: 404.5 [M+H]⁺;
¹H NMR (400 MHz, CDCl₃) *δ* (ppm): 7.64 (s, 1H), 7.45-7.41 (m, 2H), 7.35-7.32 (m, 2H), 5.78-5.75 (m, 1H), 5.55-5.52 (m, 1H), 4.24-4.17 (m, 1H), 3.77-3.69 (m, 1H), 1.78-1.77 (m, 3H), 1.39 (s, 9H).

### Step 8) the preparation of compound 35-9

A mixture of compound **35-8** (4.12 g, 10.23 mmol), compound **1-12-2** (2.86 g, 11.25 mmol), Pd(dppf)Cl₂·CH₂Cl₂ (418 mg, 0.51 mmol) and KOAc (2.51 g, 25.57 mmol) in DMF (40.0 mL) was stirred at 90 °C under N₂ for 3.0 hrs. After the reaction was completed, the mixture was cooled to rt, diluted with EtOAc (100 mL) and filtered through a celite pad. The filtrate was washed with water (80 mL x 3) and brine, dried over anhydrous Na₂SO₄ and concentrated *in vacuo.* The residue was purified by silica gel column chromatography (PE/EtOAc (v/v) = 2/1) to give the title compound (3.69 g, 80%). The compound was characterized by the following spectroscopic data:
¹H NMR (400 MHz, CDCl₃) *δ* (ppm): 7.75 -7.72 (m, 2H), 7.61-7.58 (m, 2H), 7.28 (s, 1H), 5.78-5.75 (m, 1H), 5.55-5.52 (m, 1H), 4.24-4.17 (m, 1H), 3.77-3.69 (m, 1H), 1.78-1.77 (m, 3H), 1.39 (s, 9H).

### Step 9) the preparation of compound 35-10

To a mixture of compound **35-9** (1.18 g, 2.62 mmol), compound **12-5** (1.168 g, 2.62 mmol), Pd(PPh₃)₄ (120 mg, 0.10 mmol) and KF (0.30 g, 5.24 mmol) were added DME (12.0 mL) and pure water (3.0 mL) via syringe under N₂, and the mixture was stirred at 90 °C for 2.0 hrs. After the reaction was completed, the mixture was cooled to rt and diluted with EtOAc (80.0 mL). The resulting mixture was washed with water (20.0 mL x 3) and brine, dried over anhydrous Na₂SO₄ and concentrated *in vacuo.* The residue was purified by silica gel column chromatography (DCM/MeOH (v/v) = 100/1) to give the title compound as a white solid (1.011 g, 60%). The compound was characterized by the following spectroscopic data:
MS (ESI, pos.ion) *m*/*z*: 644.3 [M+H]⁺;
¹H NMR (400 MHz, CDCl₃) *δ* (ppm): 7.55-7.52 (m, 2H), 7.45-7.42 (m, 2H), 7.39 (s, 1H), 7.27, 7.25 (s, s, 1H), 6.97, 6.95 (d, d, 1H), 5.61-5.57 (m, 1H), 5.36-5.34 (m, 1H), 4.30-4.28 (m, 0.5H), 4.26-4.24 (m, 0.5H), 3.91-3.88 (m, 0.5H), 3.87-3.84 (m, 0.5H), 3.00-2.96 (m, 2H), 2.42-2.34 (m, 2H), 2.29-2.20 (m, 4H), 1.76-1.59 (m, 7H), 1.40 (s, 9H).

### Step 10) the preparation of compound 35-11

A mixture of compound **35-10** (1.036 g, 1.61 mmol), compound **1-12-2** (450 mg, 10.7 mmol), Pd(dppf)Cl₂·CH₂Cl₂ (80 mg, 0.096 mmol) and KOAc (400 mg, 4.02 mmol) in DMF (10.0 mL) was stirred at 120 °C under N₂ for 4.0 hrs. After the reaction was completed, the mixture was cooled to rt, diluted with EtOAc (100.0 mL) and filtered through a celite pad. The filtrate was washed with water (20.0 mL x 3) and brine, dried over anhydrous Na₂SO₄ and concentrated *in vacuo.* The residue was purified by silica gel column chromatography (DCM/MeOH (v/v) = 100/1) to give the title compound as a white solid (700.25 mg, 70%). The compound was characterized by the following spectroscopic data:
MS (ESI, pos.ion) *m*/*z*: 622.5 [M+H]⁺;
¹H NMR (400 MHz, CDCl₃) *δ* (ppm): 7.59, 7.57 (m, m, 1H), 7.55-7.52 (m, 2H), 7.50-7.46 (m, 2H), 7.46, 7.44 (s, s, 1H), 7.39 (s, 1H), 5.61-5.57 (m, 1H), 5.36-5.34 (m, 1H), 4.30-4.28 (m, 0.5H), 4.26-4.24 (m, 0.5H), 3.91-3.88 (m, 0.5H), 3.87-3.84 (m, 0.5H), 2.94-2.90 (m, 2H), 2.41-2.33 (m, 2H), 2.30-2.24 (m, 2H), 1.99-1.90 (m, 2H), 1.77-1.60 (m, 7H), 1.40 (s, 9H), 1.32 (m, 6H), 1.29 (m, 6H).

### Step 11) the preparation of compound 35-12

A suspension of compound **35-6** (167 mg, 0.446 mmol), compound **35-11** (260.97 mg, 0.42 mmol), Pd(PPh₃)₄ (25 mg, 0.02 mmol) and K₂CO₃ (170 mg, 1.27 mmol) in mixed solvents of ethanol and water (8.0 mL, v/v = 3/1) was stirred at 90 °C under N₂ for 2.0 hrs. After the reaction was completed, the mixture was concentrated *in vacuo.* The residue was dissolved in EtOAc (50.0 mL). The resulting mixture was washed with brine, dried over anhydrous Na₂SO₄ and concentrated *in vacuo.* The residue was purified by silica gel column chromatography (DCM/EtOH (v/v) = 50/1) to give the title compound as a pale yellow solid (265 mg, 85%). The compound was characterized by the following spectroscopic data:
MS (ESI, pos.ion) *m*/*z*: 743.5 [M+H]⁺;
¹H NMR (400 MHz, CDCl₃) *δ* (ppm): 7.80 (s, 1H), 7.55-7.46 (m, 5H), 7.39 (s, 1H), 7.19, 7.17 (s, s, 1H), 5.80-5.75 (m, 1H), 5.61-5.57 (m, 1H), 5.38-5.34 (m, 2H), 4.30-4.28 (m, 0.5H), 4.26-4.24 (m, 0.5H), 4.13-4.11 (m, 0.5H), 4.07-4.05 (m, 0.5H), 3.91-3.88 (m, 0.5H), 3.87-3.85 (m, 0.5H), 3.74-3.72 (m, 0.5H), 3.68-3.66 (m, 0.5H), 2.85-2.81 (m, 2H), 2.37-2.23 (m, 4H), 1.94-1.86 (m, 2H), 1.76-1.58 (m, 10H), 1.40 (s, 9H), 1.39 (s, 9H).

### Step 12) the preparation of compound 35-13

To a solution of compound **35-12** (378.63 mg, 0.51 mmol) in EtOAc (4.0 mL) was added a solution of HCl in EtOAc (3.0 mL, 4 M) dropwise. At the end of the addition, the mixture was stirred at rt for 8.0 hrs. After the reaction was completed, the mixture was concentrated *in vacuo.* The residue was purified by beating in EtOAc (20.0 mL) and filtered to give the title compound as a pale yellow solid (280.8 mg, 80%). The compound was characterized by the following spectroscopic data:
MS (ESI, pos.ion) *m*/*z*: 543.5 [M+H]⁺.

### Step 13) the preparation of compound 35-14

To a suspension of compound **35-13** (199.6 mg, 0.29 mmol), compound **1-11-2** (110 mg, 0.65 mmol), EDCI (120 mg, 0.65 mmol) and HOAT (80 mg, 0.59 mmol) in DCM (5.0 mL) was added DIPEA (0.6 mL, 3.63 mmol) dropwise at 0 °C. At the end of the addition, the mixture was stirred at rt for 3.0 hrs. After the reaction was completed, the mixture was diluted with DCM (20.0 mL). The resulting mixture was washed with NH₄Cl aqueous solution and brine, dried over Na₂SO₄ and concentrated *in vacuo.* The residue was purified by silica gel column chromatography (DCM/MeOH (v/v) = 40/1) to give the title compound as a white solid (198.7 mg, 80%). The compound was characterized by the following spectroscopic data:
MS (ESI, pos.ion) *m*/*z*: 857.3 [M+H]⁺;
¹H NMR (400 MHz, CDCl₃) *δ* (ppm): 7.71 (s, 1H), 7.55-7.46 (m, 5H), 7.34 (s, 1H), 7.19, 7.17 (s, s, 1H), 5.84-5.80 (m, 1 H), 5.74-5.70 (m, 1H), 5.45-5.42 (m, 2H), 5.32, 5.29 (d, d, 2H), 4.70-4.67 (m, 1H), 4.66-4.63 (m, 1H), 4.44-4.40 (m, 2H), 4.16-4.13 (m, 1H), 4.12-4.09 (m, 1H), 3.63 (s, 6H), 2.85-2.81 (m, 2H), 2.37-2.17 (m, 6H), 1.94-1.86 (m, 2H), 1.76-1.58 (m, 10H), 0.97, 0.96 (m, m, 6H), 0.91, 0.89 (m, m, 6H).

### Example 36

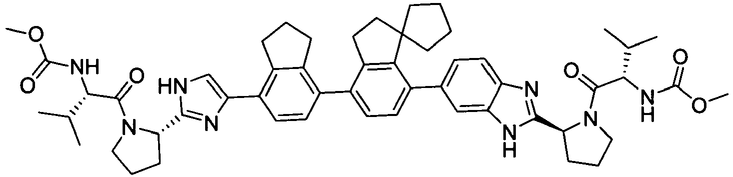

### Synthetic route:

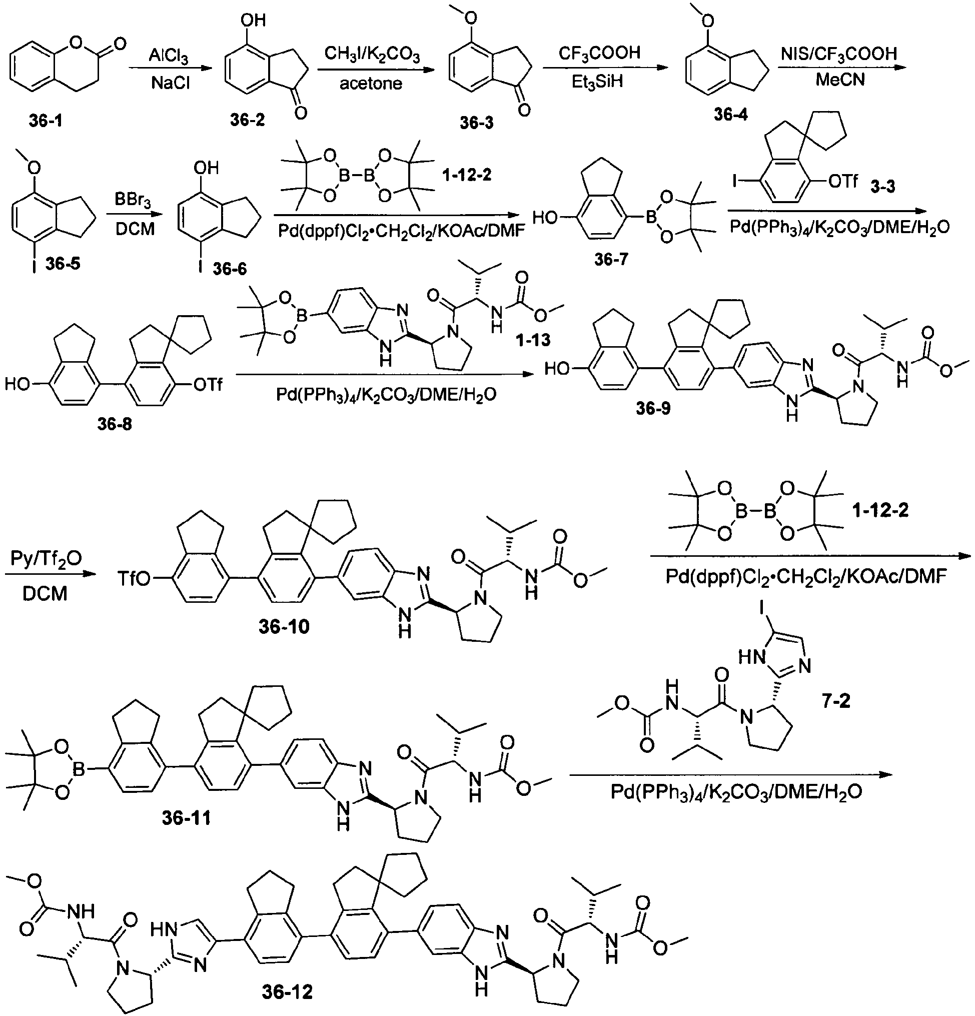

### Step1) the preparation of compound 36-2

A mixture of aluminium chloride (90.0 g, 676 mmol) and sodium chloride was stirred at 150 °C until the mixture was in molten state, and then compound **36-1** (20.0 g, 135 mmol) was added dropwise. At the end of the addition, the mixture was stirred at 200 °C for 1.0 hr. After the reaction was completed, the mixture was cooled to rt, poured slowly into ice water (500 mL) and filtered to give the crude product. The crude product was purified by beating in MeOH to give the title compound as a gray solid (19 g, 95%). The compound was characterized by the following spectroscopic data:
MS (ESI, pos.ion) *m*/*z*: 149.5 [M+H]⁺;
¹H NMR (400 MHz, CDCl₃) *δ* (ppm): 7.41-7.38 (m, 1H), 7.24-7.19 (m, 1H), 6.80-6.79, 6.78-6.77 (d, d, 1H, *J* = 4.0 Hz), 5.46 (br, 1H), 3.06-3.03 (m, 2H), 2.69-2.66 (m, 2H).

### Step 2) the preparation of compound 36-3

To a suspension of compound **36-2** (5.0 g, 33.7 mmol) and K₂CO₃ (23.4 g, 168.5 mmol) in acetone (50.0 mL) was added iodomethane (3.15 mL, 50.55 mmol) dropwise. At the end of the addition, the mixture was stirred at 60 °C for 5.0 hrs. After the reaction was completed, the mixture was concentrated *in vacuo.* The residue was dissolved in EtOAc (150 mL) and water (150 mL), and then filtered through a celite pad. After the layers were partitioned, the aqueous layer was extracted with EtOAc (150 mL x 2). The combine organic layers were washed with brine, dried over anhydrous Na₂SO₄ and concentrated *in vacuo.* The residue was purified by silica gel column chromatography (PE/EtOAc (v/v) = 10/1) to give the title compound as a pale yellow solid (2.5 g, 45%). The compound was characterized by the following spectroscopic data:
MS (ESI, pos.ion) *m*/*z*: 163.5 [M+H]⁺;
¹H NMR (400 MHz, CDCl₃) *δ* (ppm): 7.51-7.48 (m, 1H), 7.30-7.26 (m, 1H), 6.91-6.87 (m, 1H), 3.90 (s, 3H), 3.08-3.05 (m, 2H), 2.70-2.67 (m, 2H).

### Step 3) the preparation of compound 36-4

To a mixture of compound **36-3** (7.29 g, 45.0 mmol) and triethylsilane (20.98 g, 180 mmol) was added TFA (30.0 mL) dropwise at 0 °C. At the end of the addition, the mixture was stirred at 40 °C overnight. After the reaction was completed, the mixture was concentrated *in vacuo.* The residue was dissolved in EtOAc (150 mL). The resulting mixture was washed with brine (50 mL x 2), dried over anhydrous Na₂SO₄ and concentrated *in vacuo.* The residue was purified by silica gel column chromatography (PE) to give the title compound (5.2 g, 78%). The compound was characterized by the following spectroscopic data:
MS (ESI, pos.ion) *m*/*z*: 149.5 [M+H]⁺;
¹H NMR (400 MHz, CDCl₃) *δ* (ppm): 7.03 -6.96 (m, 2H), 6.68-6.66 (m, 1H), 3.86 (s, 3H), 2.99-2.81 (m, 4H), 2.24-2.05 (m, 2H).

### Step 4) the preparation of compound 36-5

To a suspension of compound **36-4** (10.34 g, 69.8 mmol) and NIS (17.2 g, 76.8 mmol) in MeCN (200 mL) was added TFA (0.52 mL, 6.98 mmol) dropwise at 0 °C. At the end of the addition, the mixture was stirred at rt overnight. After the reaction was completed, the mixture was quenched with saturated NaHCO₃ aqueous solution (100 mL), and the MeCN solvent was removed. The aqueous layer was extracted with EtOAc (100 mL x 3). The combined organic layers were washed with brine, dried over anhydrous Na₂SO₄ and concentrated *in vacuo.* The residue was purified by silica gel column chromatography (PE) to give the title compound (16.44 g, 86%). The compound was characterized by the following spectroscopic data:
MS (ESI, pos.ion) *m*/*z*: 275.5 [M+H]⁺;
¹H NMR (400 MHz, CDCl₃) *δ* (ppm): 7.42, 7.40 (t, t, 1H), 6.41-6.40, 6.39-6.38 (m, m, 1H), 3.87 (s, 3H), 2.96-2.76 (m, 4H), 2.37-2.18 (m, 2H).

### Step 5) the preparation of compound 36-6

To a solution of compound **36-5** (16.35 g, 59.7 mmol) in DCM (150.0 mL) was added boron tribromide (74.7 g, 298.8 mmol) dropwise at -78 °C. At the end of the addition, the reaction mixture was stirred at rt for 1.0 hr. After the reaction was completed, the mixture was quenched with ice water (200 mL) and the organic phase was separated. The aqueous layer was extracted with EtOAc (100 mL x 3). The combined organic layers were washed with brine, dried over anhydrous Na₂SO₄ and concentrated *in vacuo.* The residue was purified by silica gel column chromatography (PE/EtOAc (v/v) = 40/1) to give the title compound (14.28 g, 92%). The compound was characterized by the following spectroscopic data:
MS (ESI, pos.ion) *m*/*z*: 261.3 [M+H]⁺;
¹H NMR (400 MHz, CDCl₃) *δ* (ppm): 7.32, 7.30 (t, t, 1H), 6.32, 6.30 (t, t, 1H), 4.81 (br, 1H), 2.90-2.74 (m, 4H), 2.36-2.18 (m, 2H).

### Step 6) the preparation of compound 36-7

A mixture of compound **36-6** (421 mg, 1.62 mmol), compound **1-12-2** (420 mg, 1.7 mmol), Pd(dppf)Cl₂·CH₂Cl₂ (67 mg, 0.08 mmol) and KOAc (400 mg, 4.05 mmol) in DMF (5.0 mL) was stirred at 90 °C under N₂ for 3.0 hrs. After the reaction was completed, the mixture was cooled to rt, diluted with EtOAc (50.0 mL) and filtered through a celite pad. The filtrate was washed with water (20.0 mL x 3) and brine, dried over anhydrous Na₂SO₄ and concentrated *in vacuo.* The residue was purified by silica gel column chromatography (PE/EtOAc (v/v) = 10/1) to give the title compound as a pale yellow solid (295 mg, 70%). The compound was characterized by the following spectroscopic data:
MS (ESI, pos.ion) *m*/*z*: 261.5 [M+H]⁺;
¹H NMR (400 MHz, CDCl₃) *δ* (ppm): 7.94, 7.92 (t, t, 1H), 6.71, 6.69 (t, t, 1H), 4.81 (br, 1H), 2.97-2.92 (m, 2H), 2.87-2.70 (m, 2H), 2.29-2.10 (m, 2H), 1.32, 1.29 (m, m, 12H).

### Step 7) the preparation of compound 36-8

A mixture of compound **36-7** (884.5 mg, 3.40 mmol), compound **3-3** (1.52 g, 3.40 mmol), Pd(PPh₃)₄ (196.7 mg, 0.17 mmol) and K₂CO₃ (1.412 g, 10.22 mmol) in mixed solvents of DME/H₂O (15 mL, v/v = 4/1) was stirred at 90 °C under N₂ for 4.0 hrs. After the reaction was completed, the mixture was cooled to rt and diluted with EtOAc (80 mL). The resulting mixture was washed with water (20 mL x 3) and brine, dried over anhydrous Na₂SO₄ and concentrated *in vacuo.* The residue was purified by silica gel column chromatography (PE/EtOAc (v/v) = 20/1) to give the title compound as a pale yellow solid (768.6 mg, 50%). The compound was characterized by the following spectroscopic data:
MS (ESI, pos.ion) *m*/*z*: 453.5 [M+H]⁺;
¹H NMR (400 MHz, CDCl₃) *δ* (ppm): 7.52, 7.50 (d, d, 1H), 7.09, 7.07 (s, s, 1H), 6.82, 6.80 (t, t, 1H), 6.63, 6.61 (t, t, 1H), 4.81 (brs, 1H), 3.04-2.87 (m, 2H), 2.79-2.74 (m, 2H), 2.70-2.66 (m, 2H), 2.36-2.13 (m, 6H), 1.94-1.85 (m, 2H), 1.73-1.57 (m, 4H).

### Step 8) the preparation of compound 36-9

A mixture of compound **36-8** (201.65 mg, 0.446 mmol), compound **1-13** (197.51 mg, 0.42 mmol), Pd(PPh₃)₄ (25 mg, 0.02 mmol) and K₂CO₃ (0.17 g, 1.27 mmol) in mixed solvents of DME/H₂O (8.0 mL, v/v = 3/1) was stirred at 90 °C under N₂ for 3.0 hrs. After the reaction was completed, the mixture was cooled to rt and diluted with EtOAc (50 mL). The resulting mixture was washed with water (20 mL x 3) and brine, dried over anhydrous Na₂SO₄ and concentrated *in vacuo.* The residue was purified by silica gel column chromatography (DCM/EtOH (v/v) = 100/1) to give the title compound as a pale yellow solid (230.75 mg, 85%). The compound was characterized by the following spectroscopic data:
MS (ESI, pos.ion) *m*/*z*: 647.5 [M+H]⁺;
¹H NMR (400 MHz, CDCl₃) *δ* (ppm): 7.90, 7.88 (d, d, 1H), 7.65-7.63 (m, 2H), 7.57 (brs, 2H), 7.53, 7.51 (dd, dd, 1H), 7.50, 7.48 (d, d, 1H), 6.86, 6.84 (m, m, 1H), 6.63, 6.61 (m, m, 1H), 5.32, 5.29 (d, d, 1H), 5.25-5.20 (m, 1H), 4.40-4.35 (m, 1H), 3.84-3.78 (m, 1H), 3.68-3.64 (m, 1H), 3.63 (s, 3H), 3.04-2.87 (m, 2H), 2.79-2.74 (m, 2H), 2.67-2.63 (m, 2H), 2.44-2.10 (m, 12H), 2.01-1.86 (m, 1H), 1.76-1.58 (m, 4H), 0.97, 0.95 (m, m, 3H), 0.91, 0.89 (m, m, 3H).

### Step 9) the preparation of compound 36-10

To a solution of compound **36-9** (3.23 g, 5.0 mmol) in DCM (20.0 mL) was added pyridine (2.4 mL, 30.0 mmol) dropwise at 0 °C. After the mixture was stirred for 10 mins, trifluoromethanesulfonic anhydride (3.37 mL, 20.0 mmol) was added. At the end of the addition, the mixture was stirred at rt for 1.0 hr. After the reaction was completed, the mixture was quenched with ice water (25.0 mL). The aqueous layer was extracted with DCM (30 mL x 3). The combined organic layers were washed with brine, dried over anhydrous Na₂SO₄ and concentrated *in vacuo.* The residue was purified by silica gel column chromatography (PE/EtOAc (v/v) = 5/1) to give the title compound as white oil (3.5 g, 90%). The compound was characterized by the following spectroscopic data:
¹H NMR (400 MHz, CDCl₃) *δ* (ppm): 7.90, 7.88 (d, d, 1H), 7.65 (m, 1H), 7.63, 7.61 (s, s, 1H), 7.53, 7.51 (d, d, 1H), 7.50, 7.48 (d, d, 1 H), 7.32, 7.30 (t, t, 1H), 7.10-7.08 (t, t, 1H), 5.32, 5.30 (d, d, 1H), 5.25-5.18 (m, 1H), 4.40-4.35 (m, 1H), 3.84-3.78 (m, 1H), 3.68-3.65 (m, 1H), 3.63 (s, 3H), 3.06-2.89 (m, 2H), 2.77-2.73 (m, 2H), 2.67-2.63 (m, 2H), 2.44-2.10 (m, 12H), 2.00-1.87 (m, 1H), 1.75-1.58 (m, 4H), 0.97, 0.95 (m, m, 3H), 0.91, 0.89 (m, m, 3H).

### Step 10) the preparation of compound 36-11

A mixture of compound **36-10** (1.26 g, 1.62 mmol), compound **1-12-2** (420 mg, 1.7 mmol), Pd(dppf)Cl₂·CH₂Cl₂ (67 mg, 0.08 mmol) and KOAc (400 mg, 4.05 mmol) in DMF (5.0 mL) was stirred at 90 °C under N₂ for 3.0 hrs. After the reaction was completed, the mixture was cooled to rt, diluted with EtOAc (50 mL) and filtered through a celite pad. The filtrate was washed with water (20 mL x 3) and brine, dried over anhydrous Na₂SO₄ and concentrated *in vacuo.* The residue was purified by silica gel column chromatography (PE/EtOAc (v/v) = 1/3) to give the title compound as a pale yellow solid (857.8 mg, 70%). The compound was characterized by the following spectroscopic data:
MS (ESI, pos.ion) *m*/*z*: 757.5 [M+H]⁺;
¹H NMR (400 MHz, CDCl₃) *δ* (ppm): 7.90, 7.88 (d, d, 1H), 7.85-7.83 (t, t, 1H), 7.75, 7.73 (s, s, 1H), 7.65 (m, 1H), 7.53, 7.51 (dd, dd, 1H), 7.50, 7.48 (d, d, 1H), 7.25, 7.23 (t, t, 1H), 5.32, 5.29 (d, d, 1H), 5.25-5.18 (m, 1H), 4.40-4.35 (m, 1H), 3.84-3.78 (m, 1H), 3.68-3.64 (m, 1H), 3.63 (s, 3H), 3.13-3.09 (m, 2H), 2.80-2.76 (m, 2H), 2.67-2.63 (m, 2H), 2.44-2.04 (m, 12H), 2.01-1.86 (m, 1H), 1.76-1.58 (m, 4H), 1.32, 1.29 (m, m, 12H), 0.97, 0.95 (m, m, 3H), 0.91, 0.89 (m, m, 3H).

### Step 11) the preparation of compound 36-12

A mixture of compound **36-11** (2.57 g, 3.40 mmol), compound **7-2** (1.428 g, 3.4 mmol), Pd(PPh₃)₄ (196.7 mg, 0.17 mmol) and K₂CO₃ (1.412 g, 10.22 mmol) in mixed solvents of DME/H₂O (30.0 mL, v/v = 4/1) was stirred at 90 °C under N₂ for 4.0 hrs. After the reaction was completed, the mixture was cooled to rt and diluted with EtOAc (100 mL). The resulting mixture was washed with water (50 mL x 3) and brine, dried over anhydrous Na₂SO₄ and concentrated *in vacuo.* The residue was purified by silica gel column chromatography (DCM/MeOH (v/v) = 60/1) to give the title compound as a pale yellow solid (1.568 g, 50%). The compound was characterized by the following spectroscopic data:
MS (ESI, pos.ion) *m*/*z*: 463.5 [M+2H]²⁺;
¹H NMR (400 MHz, CDCl₃) *δ* (ppm): 7.90, 7.88 (d, d, 1H), 7.71, 7.69 (s, s, 1H), 7.65 (m, 1H), 7.55 (s, 1H), 7.53, 7.51 (dd, dd, 1H), 7.50, 7.48 (d, d, 1H), 7.37, 7.34 (t, t, 1H), 7.24, 7.22 (t, t, 1H), 5.32, 5.30 (d, d, 2H), 5.25-5.20 (m, 1H), 5.19-5.13 (m, 1H), 4.41-4.35 (m, 2H), 3.85-3.78 (m, 2H), 3.68-3.64 (m, 2H), 3.63 (s, 6H), 2.89-2.80 (m, 4H), 2.67-2.63 (m, 2H), 2.44-1.87 (m, 18H), 1.76-1.58 (m, 4H), 0.97, 0.95 (m, m, 6H), 0.91- 0.89 (m, m, 6H).

### Example 37

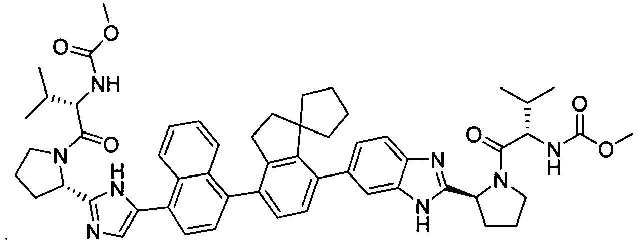

### Synthetic route:

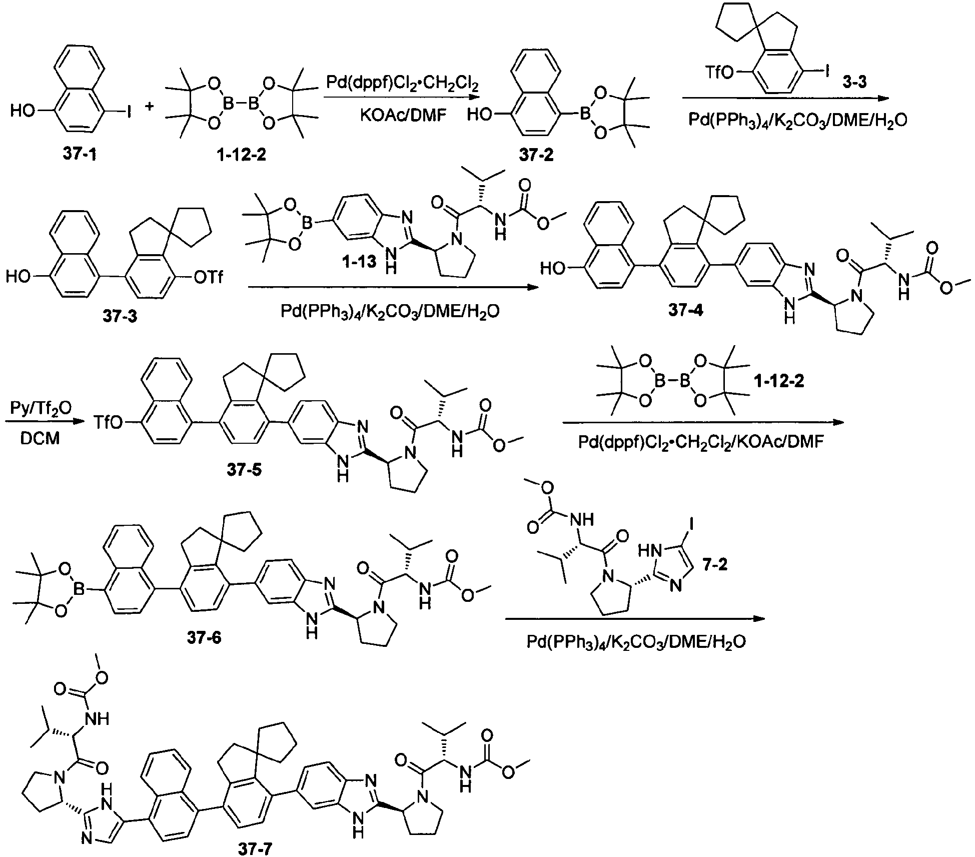

### Step 1) the preparation of compound 37-2

A mixture of compound **37-1** (437 mg, 1.62 mmol), compound **1-12-2** (420 mg, 1.7 mmol), Pd(dppf)Cl₂·CH₂Cl₂ (67 mg, 0.08 mmol) and KOAc (400 mg, 4.05 mmol) in DMF (5.0 mL) was stirred at 90 °C under N₂ for 3.0 hrs. After the reaction was completed, the mixture was cooled to rt, diluted with EtOAc (50.0 mL) and filtered through a celite pad. The filtrate was washed with water (20.0 mL x 3) and brine, dried over anhydrous Na₂SO₄ and concentrated *in vacuo.* The residue was purified by silica gel column chromatography (PE/EtOAc (v/v) = 15/1) to give the title compound as a pale yellow solid (306 mg, 70%). The compound was characterized by the following spectroscopic data:
MS (ESI, pos.ion) *m*/*z*: 271.5 [M+H]⁺;
¹H NMR (400 MHz, CDCl₃) *δ* (ppm): 8.53-8.50 (m, 1H), 8.45-8.43 (m, 1H), 7.83, 7.81 (m, m, 1H), 7.62-7.58 (m, 1H), 7.52-7.48 (m, 1H), 7.06, 7.04 (br, br, 1H), 6.17 (br, 1H), 1.57 (m, 6H), 1.54 (m, 6H).

### Step 2) the preparation of compound 37-3

A mixture of compound **37-2** (918 mg, 3.4 mmol), compound **3-3** (1.516 g, 3.4 mmol), Pd(PPh₃)₄ (196.7 mg, 0.17 mmol) and K₂CO₃ (1.412 g, 10.22 mmol) in mixed solvents of DME/H₂O (15.0 mL, v/v = 4/1) was stirred at 90 °C under N₂ for 4.0 hrs. After the reaction was completed, the mixture was cooled to rt and diluted with EtOAc (60 mL). The resulting mixture was washed with water (20.0 mL x 3) and brine, dried over anhydrous Na₂SO₄ and concentrated *in vacuo.* The residue was purified by silica gel column chromatography (PE/EtOAc (v/v) = 20/1) to give the title compound as a pale yellow solid (707 mg, 45%). The compound was characterized by the following spectroscopic data:
¹H NMR (400 MHz, CDCl₃) *δ* (ppm): 8.25-8.24, 8.23-8.22 (m, m, 1H), 7.55-7.54, 7.53-7.52 (m, m, 1H), 7.48-7.44 (m, 1H), 7.40, 7.38 (m, m, 1H), 7.29, 7.27 (dd, dd, 1H), 7.22-7.17 (m, 1H), 7.15 (s, 1H), 7.09, 7.07 (br, br, 1H), 6.17 (brs, 1H), 2.84-2.80 (m, 2H), 2.36-2.28 (m, 2H), 2.26-2.20 (m, 2H), 1.94-1.85 (m, 2H), 1.75-1.57 (m, 4H).

### Step 3) the preparation of compound 37-4

A mixture of compound **37-3** (206.1 mg, 0.446 mmol), compound 1-13 (197.51 mg, 0.42 mmol), Pd(PPh₃)₄ (25 mg, 0.02 mmol) and K₂CO₃ (0.17 g, 1.27 mmol) in mixed solvents of DME/H₂O (8.0 mL, v/v = 3/1) was stirred at 90 °C under N₂ for 3.0 hrs. After the reaction was completed, the mixture was concentrated *in vacuo* and the residue was dissolved in EtOAc (50 mL). The resulting mixture was washed with water (20.0 mL x 3) and brine, dried over anhydrous Na₂SO₄ and concentrated *in vacuo.* The residue was purified by silica gel column chromatography (DCM/EtOH (v/v) = 100/1) to give the title compound as a pale yellow solid (220.5 mg, 80%). The compound was characterized by the following spectroscopic data:
MS (ESI, pos.ion) *m*/*z*: 657.5 [M+H]⁺;
¹H NMR (400 MHz, CDCl₃) *δ* (ppm): 8.25-8.22 (m, 2H), 7.90, 7.88 (d, d, 1H), 7.74, 7.72 (s, s, 1H), 7.65 (m, 1H), 7.58, 7.56 (br, br, 1H), 7.54, 7.52 (d, d, 1H), 7.50, 7.48 (d, d, 1H), 7.46, 7.44 (m, m, 1H), 7.40, 7.38 (m, m, 1H), 7.22-7.17 (m, 1H), 7.09, 7.07 (s, s, 1H), 5.32, 5.29 (d, d, 1H), 5.25-5.20 (m, 1H), 4.40-4.35 (m, 1H), 3.84-3.78 (m, 1H), 3.68-3.64 (m, 1H), 3.63 (s, 3H), 2.79-2.75 (m, 2H), 2.44-2.13 (m, 10H), 2.01-1.89 (m, 1H), 1.75-1.61 (m, 4H), 0.97, 0.96 (m, m, 3H), 0.91, 0.89 (m, m, 3H).

### Step 4) the preparation of compound 37-5

To a solution of compound **37-4** (3.28 g, 5.0 mmol) in DCM (20.0 mL) was added pyridine (2.4 mL, 30.0 mmol) dropwise at 0 °C. After the mixture was stirred for 10 mins, trifluoromethanesulfonic anhydride (3.37 mL, 20.0 mmol) was added. At the end of the addition, the mixture was stirred at RT for 1.0 hr. After the reaction was completed, the mixture was quenched with ice water (25.0 mL). The aqueous layer was extracted with DCM (30 mL x 3). The combined organic layers were washed with brine, dried over anhydrous Na₂SO₄ and concentrated *in vacuo.* The residue was purified by silica gel column chromatography (PE/EtOAc (v/v) = 5/1) to give the title compound as white oil (3.547 g, 90%). The compound was characterized by the following spectroscopic data:
¹H NMR (400 MHz, CDCl₃) *δ* (ppm): 8.37, 8.36 (m, m, 1H), 7.90, 7.88 (d, d, 1H), 7.71, 7.69 (m, m, 2H), 7.65 (m, 1H), 7.63-7.59 (m, 1H), 7.54, 7.52 (d, d, 1H), 7.50, 7.48 (d, d, 1H), 7.39-7.38, 7.37-7.36 (m, m, 1H), 7.26-7.22 (m, 1H), 7.21, 7.20 (br, br, 1H), 5.32, 5.29 (d, d, 1H), 5.24-5.20 (m, 1H), 4.40-4.35 (m, 1H), 3.84-3.78 (m, 1H), 3.68-3.64 (m, 1H), 3.63 (s, 3H), 2.79-2.75 (m, 2H), 2.44-2.10 (m, 10H), 2.01-1.87 (m, 1H), 1.76-1.58 (m, 4H), 0.97, 0.95 (m, m, 3H), 0.91, 0.89 (m, m, 3H).

### Step 5) the preparation of compound 37-6

A mixture of compound **37-5** (1.277 g, 1.62 mmol), compound **1-12-2** (420 mg, 1.7 mmol), Pd(dppf)Cl₂·CH₂Cl₂ (67 mg, 0.08 mmol) and KOAc (400 mg, 4.05 mmol) in DMF (5.0 mL) was stirred at 90 °C under N₂ for 3.0 hrs. After the reaction was completed, the mixture was cooled to RT, diluted with EtOAc (50.0 mL) and filtered through a celite pad. The filtrate was washed with water (20.0 mL x 3) and brine, dried over anhydrous Na₂SO₄ and concentrated *in vacuo.* The residue was purified by silica gel column chromatography (PE/EtOAc (v/v) = 1/3) to give the title compound as a pale yellow solid (837.5 mg, 70%). The compound was characterized by the following spectroscopic data:
MS (ESI, pos.ion) *m*/*z*: 767.5 [M+H]⁺;
¹H NMR (400 MHz, CDCl₃) *δ* (ppm): 8.81, 8.79 (m, m, 1H), 8.01, 7.99 (br, br, 1H), 7.90, 7.88 (d, d, 1H), 7.87, 7.85 (m, m, 1H), 7.84, 7.82 (s, s, 1H), 7.71-7.70, 7.69-7.68 (m, m, 1H), 7.65 (m, 1H), 7.60-7.56 (m, 1H), 7.54, 7.52 (d, d, 1H), 7.50, 7.48 (d, d, 1H), 7.22-7.18 (m, 1H), 5.32, 5.29 (d, d, 1H), 5.24-5.20 (m, 1H), 4.40-4.35 (m, 1H), 3.84-3.78 (m, 1H), 3.68-3.64 (m, 1H), 3.63 (s, 3H), 2.79-2.75 (m, 2H), 2.44-2.10 (m, 10H), 2.01-1.86 (m, 1H), 1.76-1.59 (m, 4H), 1.57, 1.53 (m, m, 12H), 0.97, 0.96 (m, m, 3H), 0.91, 0.89 (m, m, 3H).

### Step 6) the preparation of compound 37-7

A mixture of compound **37-6** (2.60 g, 3.4 mmol), compound **7-2** (1.428 g, 3.4 mmol), Pd(PPh₃)₄ (196.7 mg, 0.17 mmol) and K₂CO₃ (1.412 g, 10.22 mmol) in mixed solvents of DME/H₂O (30 mL, v/v = 4/1) was stirred at 90 °C under N₂ for 4.0 hrs. After the reaction was completed, the mixture was cooled to RT and diluted with EtOAc (100 mL). The resulting mixture was washed with water (50 mL x 3) and brine, dried over anhydrous Na₂SO₄ and concentrated *in vacuo.* The residue was purified by silica gel column chromatography (DCM/MeOH (v/v) = 80/1) to give the title compound as a pale yellow solid (1.585 g, 50%). The compound was characterized by the following spectroscopic data:
MS (ESI, pos.ion) *m*/*z*: 467.5 [M+2H]²⁺;
¹H NMR (400 MHz, CDCl₃) *δ* (ppm): 7.90, 7.88 (d, d, 1H), 7.87-7.85 (m, 2H), 7.79, 7.77 (s, s, 1H), 7.74 (s, 1H), 7.65 (m, 1H), 7.56-7.51 (m, 3H), 7.50, 7.48 (d, d, 1H), 7.43-7.38 (m, 1H), 7.10-7.06 (m, 1H), 5.36-5.32 (m, 1H), 5.31, 5.29 (d, d, 2H), 5.25-5.20 (m, 1H), 4.41-4.35 (m, 2H), 3.85-3.78 (m, 2H), 3.68-3.64 (m, 2H), 3.63 (s, 6H), 2.79-2.75 (m, 2H), 2.44-1.86 (m, 16H), 1.76-1.58 (m, 4H), 0.97, 0.95 (m, m, 6H), 0.91, 0.89 (m, m, 6H).

### Example 38

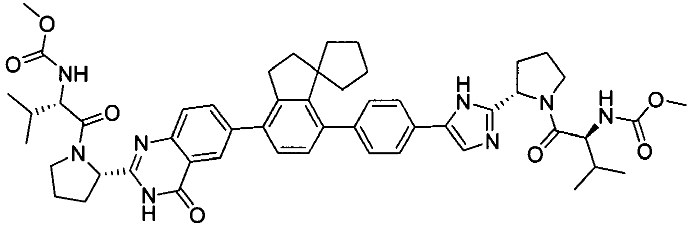

### Synthetic route:

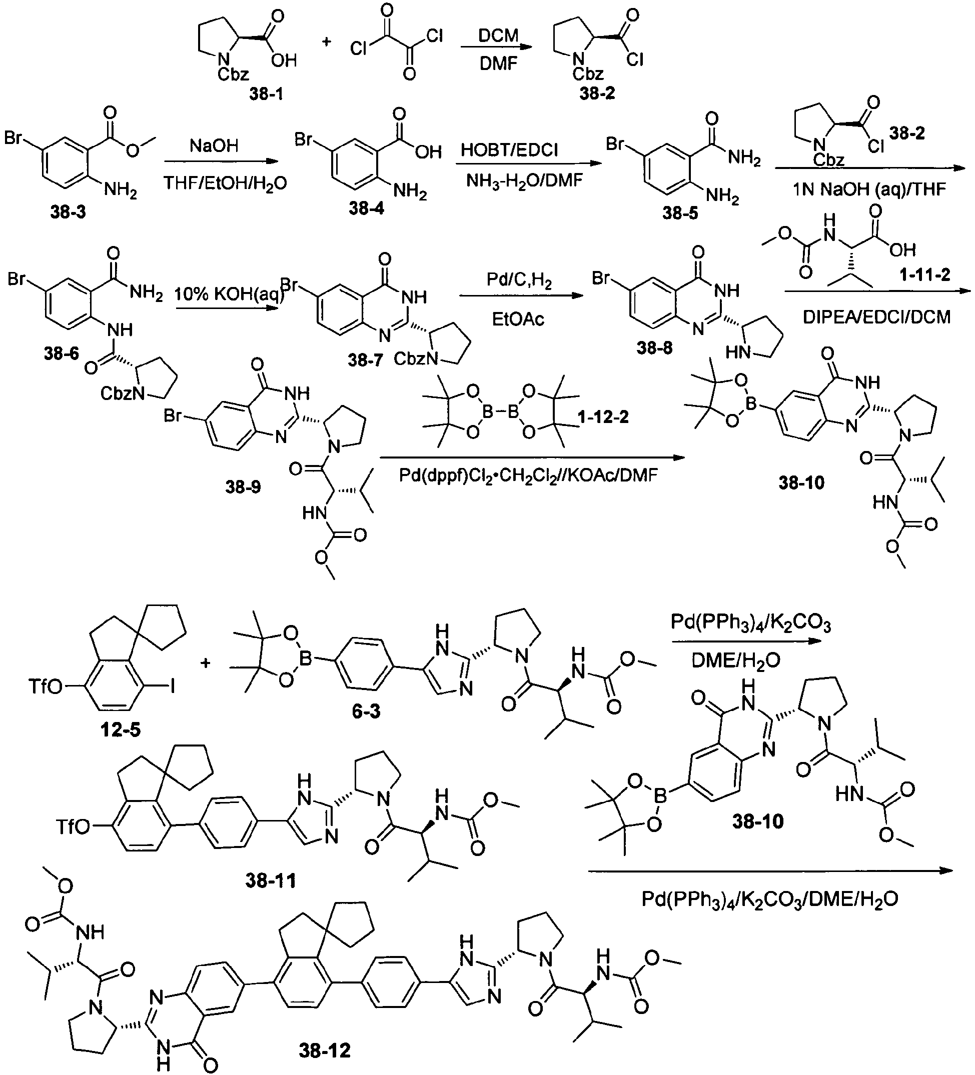

### Step 1) the preparation of compound 38-2

To a solution of compound **38-1** (1.57 g, 6.31 mmol) and DMF (0.15 mL) in DCM (20.0 mL) was added oxalyl chloride (1.35 mL, 15.75 mmol) dropwise at RT. At the end of the addition, the mixture was stirred at rt for 0.5 hr. After the reaction was completed, the mixture was concentrated *in vacuo* and the residue (2.2 g) was used for the next step without further purification. The compound was characterized by the following spectroscopic data:
MS (ESI, pos.ion) *m*/*z*: 268.5 [M+H]⁺;
¹H NMR (400 MHz, CDCl₃) *δ* (ppm): 7.28-7.22 (m, 5H), 5.14-5.13 (m, 2H), 4.47-4.42 (m, 1H), 3.66-3.60 (m, 1H), 3.46-3.38 (m, 1H), 2.23-2.09 (m, 2H), 2.03-1.96 (m, 1H), 1.83-1.73 (m, 1H).

### Step 2) the preparation of compound 38-4

A solution of compound **38-3** (10.0 g, 43.5 mmol) and NaOH (5.20 g, 130.4 mmol) in mixed solvents of H₂O (60.0 mL), EtOH (60.0 mL) and THF (180 mL) was stirred at RT for 12.0 hrs. After the reaction was completed, the solvents were removed. To the residue was added water (50 mL). The mixture was extracted with EtOAc (100 mL x 2). The aqueous layer was adjusted to pH 4 with hydrochloric acid (1M) and extracted with EtOAc (100 mL x 3). The combined organic layers were washed with brine, dried over anhydrous Na₂SO₄ and concentrated *in vacuo* to give the title compound (9.1 g, 97%). The compound was characterized by the following spectroscopic data:
MS (ESI, pos.ion) *m*/*z*: 216.5 [M+H]⁺;
¹H NMR (400 MHz, CDCl₃) *δ* (ppm): 8.03 (d, 1H), 7.34, 7.31 (d, d, 1H), 6.97 (brs, 3H), 6.86, 6.84 (d, d, 1H).

### Step 3) the preparation of compound 38-5

To a solution of compound **38-4** (9.1 g, 42.1 mmol), HOBT (13.6 g, 101.1 mmol) and EDC.HCl (19.4 g, 101.1 mmol) in DMF (60.0 mL) was added NH₃.H₂O (30.0 mL) dropwise. At the end of the addition, the mixture was stirred at rt for 15.0 hrs. After the reaction was completed, the mixture was concentrated *in vacuo.* The residue was dissolved in EtOAc (100 mL). The resulting mixture was washed with NaOH aqueous solution (20 mL,1M) and brine, dried over anhydrous Na₂SO₄ and concentrated *in vacuo* to give the title compound (7.6 g, 84%). The compound was characterized by the following spectroscopic data:
MS (ESI, pos.ion) *m*/*z*: 216.5 [M+H]⁺;
¹H NMR (400 MHz, CDCl₃) *δ* (ppm): 7.57 (d, 1H), 7.22, 7.19 (d, d, 1H), 6.58, 6.56 (d, d, 1H), 6.40 (s, 2H), 5.98 (brs, 2H).

### Step 4) the preparation of compound 38-6

To a solution of compound **38-2** (22.0 g, crude product) in dry THF (250 mL) were added compound **38-5** (7.6 g, 35.5 mmol) and NaOH (1 M, 85 mL) aqueous solution separately. At the end of the addition, the mixture was stirred at rt for 1.0 hr. After the reaction was completed, the mixture was extracted with EtOAc (100 mL x 3). The combined organic layers were washed with NaOH aqueous solution (1 M, 15.0 mL) and brine, dried over Na₂SO₄ and concentrated *in vacuo* to give the title compound. The compound was characterized by the following spectroscopic data:
MS (ESI, pos.ion) *m*/*z*: 446.5 [M+H]⁺;
¹H NMR (400 MHz, CDCl₃) *δ* (ppm): 9.06 (br, 1H), 7.75 (d, 1H), 7.35, 7.33 (d, d, 1H), 7.28-7.22 (m, 5H), 6.69, 6.67 (d, d, 1H), 5.68 (brs, 2H), 5.14-5.13 (m, 2H), 4.29-4.25 (m, 1H), 3.66-3.60 (m, 1H), 3.45-3.37 (m, 1H), 2.39-2.32 (m, 1H), 2.09-2.00 (m, 1H), 1.96-1.77 (m, 2H).

### Step 5) the preparation of compound 38-7

To a solution of compound **38-6** (17.0 g, 38.1 mmol) in EtOH (200 mL) was added KOH aqueous solution (34.0 mL, 10%). The mixture was stirred at 80 °C for 3.0 hrs. After the reaction was completed, the mixture was cooled to 0 °C, neutralized to pH 7 with concentrated HCl and filtered. The filter cake was washed with the mixed solvents of EtOAc/hexane (v/v = 5/1) to give the title compound (12.6 g, 77%). The compound was characterized by the following spectroscopic data:
MS (ESI, pos.ion) *m*/*z*: 428.5 [M+H]⁺;
¹H NMR (400 MHz, CDCl₃) *δ* (ppm): 8.02, 8.01 (d, d, 1H), 7.65, 7.62 (d, d, 1H), 7.28-7.22 (m, 5H), 7.20, 7.18 (d, d, 1H), 5.14-5.13 (m, 2H), 5.00-4.95 (m, 1H), 3.64-3.57 (m, 1H), 3.44-3.37 (m, 1H), 2.49-2.41 (m, 1H), 2.36-2.26 (m, 1H), 2.02-1.93 (m, 1H), 1.91-1.81 (m, 1H).

### Step 6) the preparation of compound 38-8

To a solution of compound **38-7** (3.43 g, 8.03 mmol) in EtOAc (40.0 mL) was added a catalytic amount of Pd/C (350 mg). The mixture was stirred at 40 °C under 10 atm of H₂ gas for 5.0 hrs. After the reaction was completed, the mixture was filtered. The filtrate was concentrated *in vacuo* to give the title compound (2.02 g, 86%). The compound was characterized by the following spectroscopic data:
MS (ESI, pos.ion) *m*/*z*: 294.5 [M+H]⁺.

### Step 7) the preparation of compound 38-9

To a suspension of compound **38-8** (2.93 g, 10.0 mmol), compound **1-11-2** (1.93 g, 11.0 mmol) and EDCI (2.10 g, 11.0 mmol) in DCM (30.0 mL) was added DIPEA (6.6 mL, 39.9 mmol) dropwise at 0 °C. At the end of the addition, the mixture was stirred at rt for 3.0 hrs. After the reaction was completed, the mixture was diluted with DCM (50.0 mL). The resulting mixture was washed with NH₄Cl aqueous solution and brine, dried over Na₂SO₄ and concentrated *in vacuo*. The residue was purified by silica gel column chromatography (PE/EtOAc (v/v) = 4/1) to give the title compound (2.25 g, 50%). The compound was characterized by the following spectroscopic data:
MS (ESI, pos.ion) *m*/*z*: 451.5 [M+H]⁺;
¹H NMR (400 MHz, CDCl₃) *δ* (ppm): 8.02, 8.01 (m, 1H), 7.65, 7.63 (d, d, 1H), 7.22, 7.20 (d, d, 1H), 5.32, 5.29 (br, br, 1H), 5.21-5.16 (m, 1H), 4.30-4.25 (m, 1H), 3.63 (s, 3H), 3.60-3.54 (m, 1H), 3.24-3.16 (m, 1H), 2.44-2.37 (m, 1H), 2.11-1.98 (m, 2H), 1.93-1.85 (m, 1H), 1.83-1.73 (m, 1H), 0.97-0.89 (m, 6H).

### Step 8) the preparation of compound 38-10

A suspension of compound **38-9** (409 mg, 0.91 mmol), compound **1-12-2** (463 mg, 1.82 mmol), Pd(dppf)Cl₂·CH₂Cl₂ (71.0 mg, 0.09 mmol) and KOAc (268 mg, 2.73 mmol) in DMF (5.0 mL) was stirred at 90 °C under N₂ for 3.0 hrs. After the reaction was completed, the mixture was cooled to rt, diluted with EtOAc (60.0 mL) and filtered through a celite pad. The filtrate was washed with water (20 mL x 3) and brine, dried over anhydrous Na₂SO₄ and concentrated *in vacuo.* The residue was purified by silica gel column chromatography (PE/EtOAc (v/v) = 2/1) to give the title compound (399 mg, 88%). The compound was characterized by the following spectroscopic data:
MS (ESI, pos.ion) *m*/*z*: 499.5 [M+H]⁺;
¹H NMR (400 MHz, CDCl₃) *δ* (ppm): 8.50 (d, 1H), 7.78, 7.76 (d, d, 1H), 7.63, 7.61 (d, d, 1H), 5.32, 5.30 (d, d, 1H), 5.21-5.16 (m, 1H), 4.30-4.25 (m, 1H), 3.63 (s, 3H), 3.60-3.54 (m, 1H), 3.24-3.16 (m, 1H), 2.44-2.37 (m, 1H), 2.11-1.98 (m, 2H), 1.93-1.85 (m, 1H), 1.83-1.73 (m, 1H), 1.24, 1.20 (m, 12H), 0.97, 0.95 (m, m, 3H), 0.90, 0.89 (m, m, 3H).

### Step 9) the preparation of compound 38-11

A mixture of compound **12-5** (1.52 g, 3.4 mmol), compound **6-3** (1.687 g, 3.4 mmol), Pd(PPh₃)₄ (196.7 mg, 0.17 mmol) and K₂CO₃ (1.412 g, 10.22 mmol) in mixed solvents of DME/H₂O (30.0 mL, v/v = 4/1) was stirred at 90 °C under N₂ for 4.0 hrs. After the reaction was completed, the mixture was cooled to rt and diluted with EtOAc (80 mL). The resulting mixture was washed with water (20.0 mL x 3) and brine, dried over anhydrous Na₂SO₄ and concentrated *in vacuo.* The residue was purified by silica gel column chromatography (DCM/MeOH (v/v) = 100/1) to give the title compound as a pale yellow solid (1.17 g, 50%). The compound was characterized by the following spectroscopic data:
MS (ESI, pos.ion) *m*/*z*: 689.5 [M+H]⁺;
¹H NMR (400 MHz, CDCl₃) *δ* (ppm): 7.59 (s, 1H), 7.54-7.51 (m, 2H), 7.45-7.42 (m, 2H), 7.27, 7.25 (s, s, 1H), 6.97, 6.95 (dd, dd, 1H), 5.32, 5.30 (d, d, 1H), 5.23-5.19 (m, 1H), 4.41-4.36 (m, 1H), 3.85-3.78 (m, 1H), 3.69-3.64 (m, 1H), 3.63 (s, 3H), 3.00-2.96 (m, 2H), 2.42-2.34 (m, 2H), 2.30-1.92 (m, 9H), 1.76-1.59 (m, 4H), 0.97, 0.95 (m, m, 3H), 0.91, 0.89 (m, m, 3H).

### Step 10) the preparation of compound 38-12

A mixture of compound **38-10** (304 mg, 0.61 mmol), compound **38-11** (419.8 mg, 0.61 mmol), Pd(PPh₃)₄ (70 mg, 0.05 mmol) and K₂CO₃ (254 mg, 1.83 mmol) in mixed solvents of DME/H₂O (6.0 mL, v/v = 5/1) was stirred at 90 °C under N₂ for 4.0 hrs. After the reaction was completed, the mixture was cooled to rt and diluted with EtOAc (50 mL). The resulting mixture was washed with water (20.0 mL x 3) and brine, dried over anhydrous Na₂SO₄ and concentrated *in vacuo.* The residue was purified by silica gel column chromatography (EtOAc) to give the title compound (333.23 mg, 60%). The compound was characterized by the following spectroscopic data:
MS (ESI, pos.ion) *m*/*z*: 456.3 [M+2H]²⁺;
¹H NMR (400 MHz, CDCl₃) *δ* (ppm): 7.93-7.91 (m, 2H), 7.66, 7.64 (dd, dd, 1H), 7.59 (s, 1H), 7.54-7.46 (m, 5H), 5.32, 5.30 (d, d, 2H), 5.23-5.16 (m, 2H), 4.41-4.36 (m, 1H), 4.30-4.25 (m, 1H), 3.85-3.78 (m, 1H), 3.69-3.65 (m, 1H), 3.63 (s, 6H), 3.62-3.54 (m, 1H), 3.24-3.16 (m, 1H), 2.81-2.77 (m, 2H), 2.44-2.35 (m, 3H), 2.30-1.58 (m, 18H), 0.97, 0.95 (m, m, 6H), 0.91, 0.89 (m, m, 6H).

### Example 39

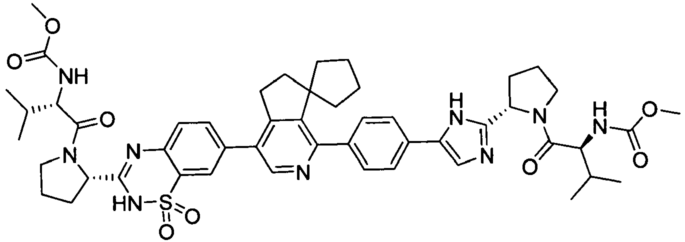

### Synthetic route:

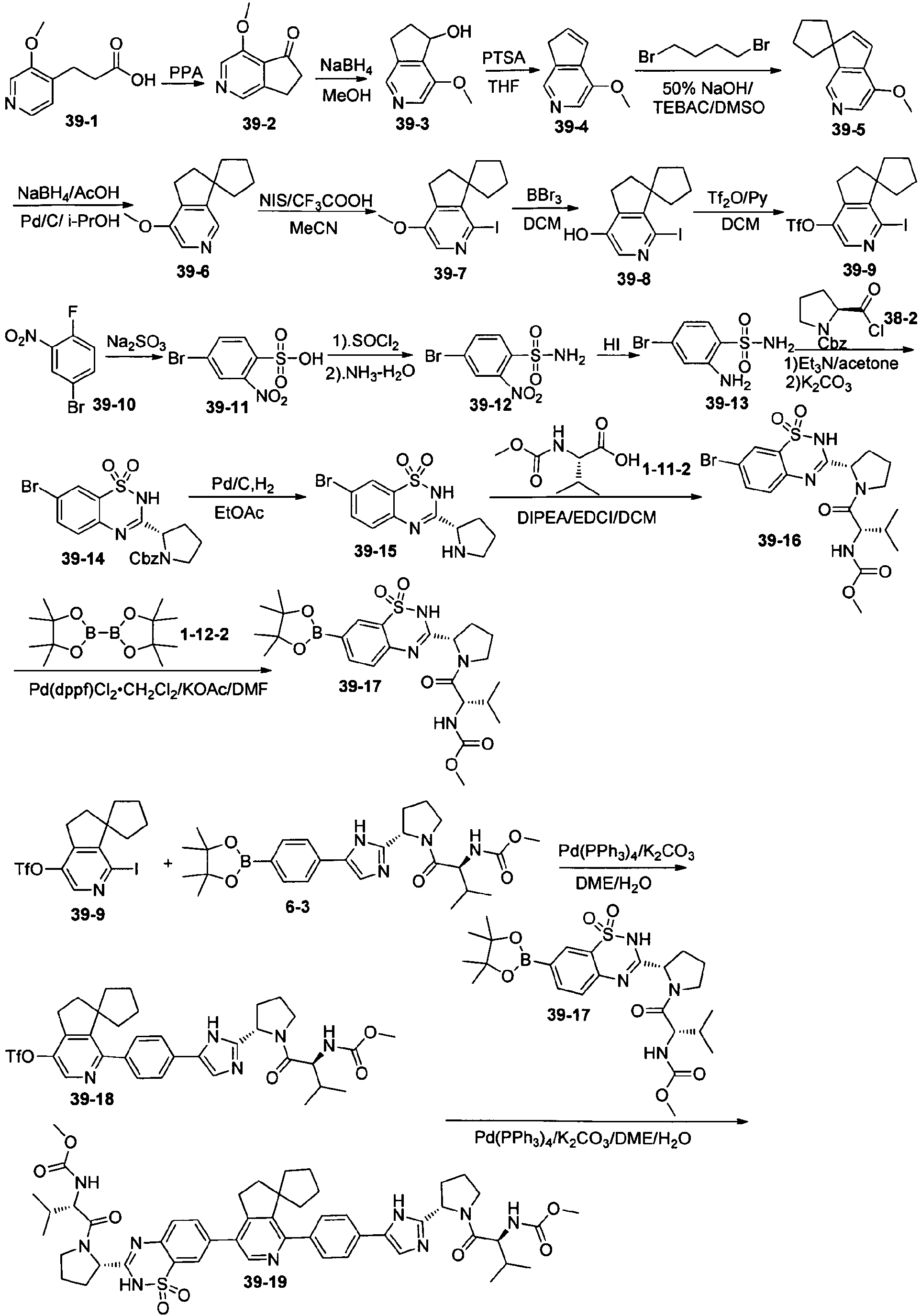

### Step 1) the preparation of compound 39-2

A mixture of compound **39-1** (4.037 g, 22.3 mmol) and PPA (50.87 g, 24.8 mmol) was stirred at 80 °C for 4.0 hrs. After the reaction was completed, the mixture was quenched with ice water (250 mL). The aqueous layer was extracted with EtOAc (100 mL x 5). The combined organic layers were washed with NaHCO₃ aqueous solution and brine, dried over anhydrous Na₂SO₄ and concentrated *in vacuo.* The residue was purified by silica gel column chromatography (PE/EtOAc (v/v) = 3/1) to give the title compound as a pale yellow solid (2.18 g, 60.0%). The compound was characterized by the following spectroscopic data:
MS (ESI, pos.ion) *m*/*z*: 164.5 [M+H]⁺;
¹H NMR (400 MHz, CDCl₃) *δ* (ppm): 8.48-8.46 (m, 2H), 3.87 (s, 3H), 3.13-3.09 (m, 2H), 2.78-2.74 (m, 2H).

### Step 2) the preparation of compound 39-3

To a solution of compound **39-2** (9.78 g, 60 mmol) in methanol (150 mL) was added NaBH₄ (1.3 g, 40 mmol) at °C. At the end of the addition, the mixture was stirred at rt for 1.0 hr. After the reaction was completed, the mixture was quenched with water (50 mL), and the MeOH solvent was removed. The resulting mixture was extracted with EtOAc (50 mL x 3). The combined organic layers were washed with brine, dried over anhydrous Na₂SO₄ and concentrated *in vacuo.* The residue was purified by silica gel column chromatography (PE/EtOAc (v/v) = 4/1) to give the title compound (7.92 g, 80%). The compound was characterized by the following spectroscopic data:
MS (ESI, pos.ion) *m*/*z*: 166.5 [M+H]⁺;
¹H NMR (400 MHz, CDCl₃) *δ* (ppm): 8.27-8.25 (m, 1H), 8.09 (m, 1H), 5.26-5.22 (m, 1H), 3.86 (s, 3H), 2.98-2.94 (m, 2H), 2.70 (brs, 1H), 2.57-2.48 (m, 1H), 2.03-1.96 (m, 1H).

### Step 3) the preparation of compound 39-4

To a solution of compound **39-3** (2.7 g, 16.4 mmol) in THF (100 mL) was added *p*-TSA (1.4 g, 8.2 mmol) at 0 °C. At the end of the addition, the mixture was refluxed for 2.0 hrs. After the reaction was completed, the mixture was quenched with saturated NaHCO₃ aqueous solution (50 mL), and the THF solvent was removed. The resulting mixture was extracted with EtOAc (50 mL x 3). The combined organic layers were washed with brine, dried over anhydrous Na₂SO₄ and concentrated *in vacuo.* The residue was purified by silica gel column chromatography (PE) to give the title compound as a white solid (964.78 mg, 40%). The compound was characterized by the following spectroscopic data:
MS (ESI, pos.ion) *m*/*z*: 148.5 [M+H]⁺;
¹H NMR (400 MHz, CDCl₃) *δ* (ppm): 8.18-8.17 (m, 1H), 7.32-7.31 (m, 1H), 6.98-6.95 (m, 1H), 6.66-6.64 (m, 1H), 3.88 (s, 3H), 3.39-3.38 (m, 2H).

### Step 4) the preparation of compound 39-5

To a suspension of compound **39-4** (1.003 g, 6.82 mmol), 1,4-dibromobutane (1.54 g, 7.15 mmol) and TEBAC (0.3 g, 1.36 mmol) in DMSO (30 mL) was added sodium hydroxide aqueous solution (50%, 2 mL) dropwise at 0 °C. At the end of the addition, the mixture was stirred at 50 °C for 2.0 hrs. After the reaction was completed, the mixture was quenched with water (50 mL). The aqueous layer was extracted with EtOAc (50 mL x 3). The combined organic layers were washed with brine, dried over anhydrous Na₂SO₄ and concentrated *in vacuo.* The residue was purified by silica gel column chromatography (PE) to give the title compound as yellow oil (1.08 g, 69%). The compound was characterized by the following spectroscopic data:
MS (ESI, pos.ion) *m*/*z*: 202.5 [M+H]⁺;
¹H NMR (400 MHz, CDCl₃) *δ* (ppm): 7.89 (m, 1H), 7.33-7.32 (m, 1H), 6.75, 6.74 (d, d, 1H), 6.64-6.61 (m, 1H), 3.87 (s, 3H), 2.21-2.11 (m, 2H), 1.85-1.66 (m, 4H), 1.55-1.45 (m, 2H).

### Step 5) the preparation of compound 39-6

To a solution of compound **39-5** (943.25 mg, 4.69 mmol) in isopropanol (15 mL) were added a catalytic amount of Pd/C (100 mg), acetic acid (0.56 g, 9.38 mmol) and NaB H₄ (0.7 g, 18.76 mmol) separately. At the end of the addition, the mixture was stirred at rt for 1.0 hr. After the reaction was completed, the mixture was filtered through a celite pad. The filtrate was concentrated *in vacuo.* The residue was purified by silica gel column chromatography (PE) to give the title compound as yellow oil (781.2 mg, 82%). The compound was characterized by the following spectroscopic data:
MS (ESI, pos.ion) *m*/*z*: 202.3 [M+H]⁺;
¹H NMR (400 MHz, CDCl₃) *δ* (ppm): 8.01 (m, 1H), 7.88 (m, 1H), 3.85 (s, 3H), 2.83-2.79 (m, 2H), 2.30-2.15 (m, 4H), 1.87-1.61 (m, 6H).

### Step 6) the preparation of compound 39-7

To a mixture of compound **39-6** (5.322 g, 26.2 mmol) and NIS (6.5 g, 28.8 mmol) in MeCN (60 mL) was added CF₃COOH (0.59 mL, 7.9 mmol) dropwise at 0 °C. At the end of the addition, the mixture was stirred at 45 °C overnight. After the reaction was completed, the mixture was concentrated *in vacuo*, and to the residue was added water (50 mL). The mixture was extracted with EtOAc (60 mL x 3). The combined organic layers were washed with saturated sodium sulfite aqueous solution (30 mL x 2), dried over anhydrous Na₂SO₄ and concentrated *in vacuo*. The residue was purified by silica gel column chromatography (PE) to give the title compound as colorless oil (7.758 g, 90%). The compound was characterized by the following spectroscopic data:
MS (ESI, pos.ion) *m*/*z*: 330.5 [M+H]⁺;
¹H NMR (400 MHz, CDCl₃) *δ* (ppm): 7.73 (s, 1H), 3.89 (s, 3H), 2.85-2.82 (m, 2H), 2.37-2.28 (m, 4H), 1.95-1.86 (m, 2H), 1.78-1.61 (m, 4H).

### Step 7) the preparation of compound 39-8

To a solution of compound **39-7** (1.553 g, 4.72 mmol) in DCM (10 mL) was added boron tribromide (1.79 mL, 18.89 mmol) dropwise at -78 °C. The mixture was stirred at -78 °C for 10 mins and at rt for another 1.0 hr. After the reaction was completed, the mixture was quenched with ice water (50 mL) and the organic phase was separated. The aqueous layer was extracted with DCM (30 mL x 3). The combined organic layers were washed with brine, dried over anhydrous Na₂SO₄ and concentrated *in vacuo.* The residue was purified by silica gel column chromatography (PE/EtOAc (v/v) = 10/1) to give the title compound as colorless oil (1.338 g, 90%). The compound was characterized by the following spectroscopic data:
¹H NMR (400 MHz, CDCl₃) *δ* (ppm): 8.00 (brs, 1H), 7.86 (s, 1H), 2.81-2.78 (m, 2H), 2.37-2.29 (m, 4H), 1.95-1.86 (m, 2H), 1.79-1.61 (m, 4H).

### Step 8) the preparation of compound 39-9

To a solution of compound **39-8** (1.3356 g, 4.24 mmol) in DCM (10.0 mL) was added pyridine (0.85 mL, 10.58 mmol) dropwise at 0 °C. After the mixture was stirred for 10 mins, trifluoromethanesulfonic anhydride (1.07 mL, 6.36 mmol) was added, and the mixture was stirred at rt for 1.0 hr. After the reaction was completed, the mixture was quenched with ice water (25 mL). The aqueous layer was extracted with DCM (30 mL x 3). The combined organic layers were washed with brine, dried over anhydrous Na₂SO₄ and concentrated *in vacuo.* The residue was purified by silica gel column chromatography (PE) to give the title compound as white oil (1.705 g, 90%). The compound was characterized by the following spectroscopic data:
¹H NMR (400 MHz, CDCl₃) *δ* (ppm): 8.09 (s, 1H), 2.99-2.95 (m, 2H), 2.37-2.29 (m, 4H), 1.95-1.87 (m, 2H), 1.77-1.61 (m, 4H).

### Step 9) the preparation of compound 39-11

To a solution of compound **39-10** (5.0 g, 22.7 mmol) in EtOH (60.0 mL) was added a mixture of Na₂SO₃ (7.16 g, 56.8 mmol) in EtOH (100 mL) and water (125 mL). At the end of the addition, the mixture was stirred at 70 °C for 15.0 hrs. After the reaction was completed, the mixture was cooled to rt, adjusted to pH 2 with HCl (2 M) and concentrated *in vacuo.* To the residue was added brine (100 mL). The mixture was refluxed until all solid dissolved. Subsequently, water (10.0 mL) was added and the mixture was cooled to 0 °C with solid precipitated out. The precipitate was collected by filtration to give the title compound (5.675 g, 89%). The compound was characterized by the following spectroscopic data:
MS (ESI, pos.ion) *m*/*z:* 281.5 [M+H]⁺;
¹H NMR (400 MHz, CDCl₃) *δ* (ppm): 8.75 (br, 1H), 8.31 (m, 1H), 8.07 (m, 2H).

### Step 10) the preparation of compound 39-12

To a solution of compound **39-11** (3.0 g, 10.6 mmol) and DMF (1 drop) in toluene (50.0 mL) was added thionyl chloride (5.0 mL). At the end of the addition, the reaction was refluxed for 4.0 hrs. After the reaction was completed, the mixture was cooled and concentrated *in vacuo.* The residue was dissolved in toluene (4.0 mL), and ammonium hydroxide solution (1.0 mL) and THF (10.0 mL) were added to the mixture at -10 °C. After stirring for 2.0 hrs, the reaction mixture was adjusted pH 4 with hydrochloric acid (6 M). The organic layer was separated, dried over anhydrous Na₂SO₄ and concentrated *in vacuo.* To the residue was added PE (15.0 mL) and solid precipitated out. The mixture was filtered to give the title compound. The compound was characterized by the following spectroscopic data:
MS (ESI, pos.ion) *m*/*z:* 280.5 [M+H]⁺;
¹H NMR (400 MHz, CDCl₃) *δ* (ppm): 8.18, 8.17 (d, d, 1H), 8.03, 8.00 (d, d, 1H), 7.84, 7.81 (d, d, 1H), 5.47 (br, 2H).

### Step 11) the preparation of compound 39-13

A solution of compound **39-12** (2.12 g, 7.5 mmol) in HI (25.0 mL, 57%) was stirred at 90 °C for 4.0 hrs. After the reaction was completed, the mixture was cooled to rt and diluted with EtOAc (50.0 mL). Then the mixture was washed with saturated Na₂S₂O₃ aqueous solution and saturated NaHCO₃ aqueous solution followed by brine, dried over anhydrous Na₂SO₄ and concentrated *in vacuo.* The residue was purified by high-performance liquid chromatography (eluent: CH₃CN/H₂O from 22/78 to 52/48 with 0.01% NH₃.H₂O as buffer) to give the title compound (1.86 g). The compound was characterized by the following spectroscopic data:
MS (ESI, pos.ion) *m*/*z:* 251.5 [M+H]⁺;
¹H NMR (400 MHz, CDCl₃) *δ* (ppm): 7.62-7.60 (m, 1H), 7.18-7.15 (m, 2H), 4.85 (brs, 4H).

### Step 12) the preparation of compound 39-14

To a solution of compound **39-13** (1.86 g, 7.4 mmol) in acetone (20.0 mL) was added triethylamine (4.05 mL, 29.6 mmol) dropwise. Then compound **38-2** (1.28 g, 4.8 mmol) was added to the mixture at 0 °C. After stirring for 5.0 hrs, the mixture was diluted with water (20.0 mL) and adjusted to pH 4 with hydrochloric acid (2 M) with solid precipitated out. The precipitate was collected by filtration and transferred to another flask. A solution of K₂CO₃ (1.5 g, 10.87 mmol) in water (10.0 mL) was added and the mixture was refluxed for 2.0 hrs. After the reaction was completed, the mixture was adjusted to pH 4 with hydrochloric acid (2 M) with solid precipitated out. The precipitate was filtered off, washed with water and purified by high-performance liquid chromatography (eluent: CH₃CN/H₂O from 35/65 to 65/35 with 0.75% CF₃COOH as buffer) to give the title compound (0.83 g, 45%). The compound was characterized by the following spectroscopic data:
MS (ESI, pos.ion) *m*/*z*: 464.5 [M+H]⁺;
¹H NMR (400 MHz, CDCl₃) *δ* (ppm): 7.93, 7.90 (d, d, 1H), 7.77-7.76 (m, 1H), 7.43, 7.41 (d, d, 1H), 7.28-7.22 (m, 5H), 6.30 (brs, 1H), 5.14-5.13 (m, 2H), 4.86-4.80 (m, 1H), 3.68-3.62 (m, 1H), 3.50-3.43 (m, 1H), 2.22-1.98 (m, 4H).

### Step 13) the preparation of compound 39-15

To a solution of compound **39-14** (3.72 g, 8.03 mmol) in EtOAc (40.0 mL) was added a catalytic amount of Pd/C (350 mg). The mixture was stirred at 40 °C under 10 atm of H₂ gas for 5.0 hrs. After the reaction was completed, the mixture was filtered. The filtrate was concentrated *in vacuo* to give the title compound (2.27 g, 86%). The compound was characterized by the following spectroscopic data:
MS (ESI, pos.ion) *m*/*z*: 330.5 [M+H]⁺.

### Step 14) the preparation of compound 39-16

To a solution of compound **39-15** (3.29 g, 10.0 mmol), compound **1-11-2** (1.93 g, 11.0 mmol) and EDCI (2.10 g, 11.0 mmol) in DCM (30.0 mL) was added DIPEA (6.6 mL, 39.9 mmol) dropwise at 0 °C. At the end of the addition, the mixture was stirred at rt for 3.0 hrs. After the reaction was completed, the mixture was diluted with DCM (100.0 mL). Then the mixture was washed with water (30 mL x 3), NH₄Cl aqueous solution and brine, dried over Na₂SO₄ and *concentrated in vacuo.* The residue was purified by silica gel column chromatography (PE/EtOAc (v/v) = 2/1) to give the title compound (2.43 g, 50%). The compound was characterized by the following spectroscopic data:
MS (ESI, pos.ion) *m*/*z:* 487.5 [M+H]⁺;
¹H NMR (400 MHz, CDCl₃) *δ* (ppm): 7.93, 7.90 (d, d, 1H), 7.77-7.76 (m, 1H), 7.43, 7.41 (d, d, 1H), 6.30 (brs, 1H), 5.32, 5.29 (d, d, 1H), 5.08-5.04 (m, 1H), 4.31-4.26 (m, 1H), 3.63 (s, 3H), 3.62-3.57 (m, 1H), 3.26-3.18 (m, 1H), 2.38-2.31 (m, 1H), 2.10-1.97 (m, 2H), 1.91-1.71 (m, 2H), 0.97, 0.95 (m, m, 3H), 0.91, 0.89 (m, m, 3H).

### Step 15) the preparation of compound 39-17

A mixture of compound **39-16** (442.3 mg, 0.91 mmol), compound **1-12-2** (463 mg, 1.82 mmol), Pd(dppf)Cl₂ CH₂Cl₂ (71.0 mg, 0.09 mmol) and KOAc (268 mg, 2.73 mmol) in DMF (5.0 mL) was stirred at 90 °C under N₂ for 3.0 hrs. After the reaction was completed, the mixture was diluted with EtOAc (50.0 mL) and filtered through a celite pad. The filtration was washed with water (20.0 mL x 3) and brine, dried over anhydrous Na₂SO₄ and concentrated *in vacuo.* The residue was purified by silica gel column chromatography (PE/EtOAc (v/v) = 2/1) to give the title compound (427.8 mg, 88%). The compound was characterized by the following spectroscopic data:
MS (ESI, pos.ion) *m*/*z:* 535.3 [M+H]⁺;
¹H NMR (400 MHz, CDCl₃) *δ* (ppm): 8.02 (t, 1H), 7.80, 7.78 (d, d, 1H), 7.46, 7.44 (d, d, 1H), 6.30 (brs, 1H), 5.32, 5.29 (d, d, 1H), 5.08-5.04 (m, 1H), 4.31-4.26 (m, 1 H), 3.63 (s, 3H), 3.62-3.57 (m, 1H), 3.26-3.18 (m, 1H), 2.38-2.31 (m, 1H), 2.10-1.97 (m, 2H), 1.91-1.71 (m, 2H), 1.32, 1.29 (m, 12H), 0.97, 0.95 (m, m, 3H), 0.91, 0.89 (m, m, 3H).

### Step 16) the preparation of compound 39-18

A mixture of compound **39-9** (1.5196 g, 3.4 mmol), compound **6-3** (1.687 g, 3.4 mmol), Pd(PPh₃)₄ (196.7 mg, 0.17 mmol) and K₂CO₃ (1.412 g, 10.22 mmol) in mixed solvents of DME/H₂O (30 mL, v/v = 4/1) was stirred at 90 °C under N₂ for 4.0 hrs. After the reaction was completed, the mixture was diluted with EtOAc (80.0 mL). The resulting mixture was washed with water (20.0 mL x 3) and brine, dried over anhydrous Na₂SO₄ and *concentrated in vacuo.* The residue was purified by silica gel column chromatography (DCM/MeOH (v/v) = 100/1) to give the title compound as a pale yellow solid (1.17 g, 50%). The compound was characterized by the following spectroscopic data:
MS (ESI, pos.ion) *m*/*z:* 690.5 [M+H]⁺;
¹H NMR (400 MHz, CDCl₃) *δ* (ppm): 8.53 (s, 1H), 7.84-7.80 (m, 2H), 7.69-7.68 (m, 2H), 7.59 (s, 1H), 5.32, 5.29 (d, d, 1H), 5.23-5.19 (m, 1H), 4.41-4.36 (m, 1H), 3.85-3.78 (m, 1H), 3.69-3.64 (m, 1H), 3.63 (s, 3H), 2.98-2.94 (m, 2H), 2.42-2.34 (m, 2H), 2.31-2.15 (m, 5H), 2.13-1.91 (m, 4H), 1.79-1.62 (m, 4H), 0.97, 0.95 (m, m, 3H), 0.91, 0.89 (m, m, 3H).

### Step 17) the preparation of compound 39-19

A mixture of compound **39-18** (420.44 mg, 0.61 mmol), compound **39-17** (325.88 mg, 0.61 mmol), Pd(PPh₃)₄ (70 mg, 0.05 mmol) and K₂CO₃ (254 mg, 1.83 mmol) in mixed solvents of DME/H₂O (6.0 mL, v/v = 5/1) was stirred at 90 °C under N₂ for 4.0 hrs. After the reaction was completed, the mixture was diluted with EtOAc (50.0 mL). The resulting mixture was washed with water (20.0 mL x 3) and brine, dried over anhydrous Na₂SO₄ and concentrated *in vacuo.* The residue was purified by silica gel column chromatography (EtOAc) to give the title compound (346.76 mg, 60%). The compound was characterized by the following spectroscopic data:
MS (ESI, pos.ion) *m*/*z:* 475.3 [M+2H]²⁺;
¹H NMR (400 MHz, CDCl₃) *δ* (ppm): 8.65 (s, 1H), 7.94, 7.92 (d, d, 1H), 7.88-7.84 (m, 3H), 7.69-7.66 (m, 2H), 7.59 (s, 1H), 7.52, 7.50 (d, d, 1H), 5.32, 5.29 (d, d, 2H), 5.23-5.19 (m, 1H), 5.08-5.04 (m, 1H), 4.41-4.37 (m, 1H), 4.31-4.26 (m, 1H), 3.85-3.78 (m, 1H), 3.69-3.65 (m, 1H), 3.63 (s, 6H), 3.62-3.57 (m, 1H), 3.26-3.18 (m, 1H), 2.91-2.87 (m, 2H), 2.44-1.61 (m, 20H), 0.97, 0.95 (m, m, 6H), 0.91, 0.89 (m, m, 6H).

### Example 40

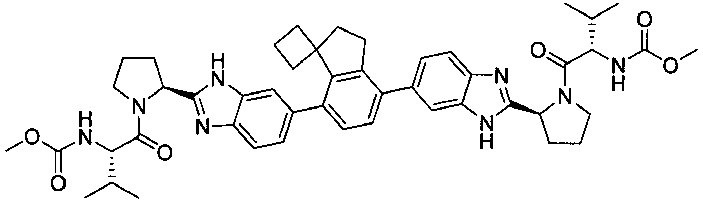

### Synthetic route:

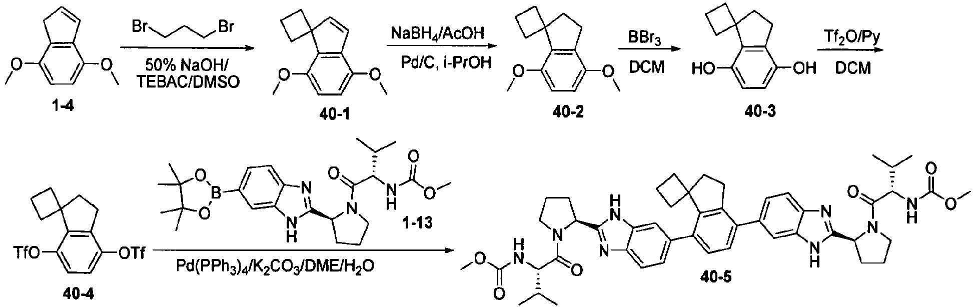

### Step 1) the preparation of compound 40-1

To a solution of compound **1-4** (1.20 g, 6.82 mmol), 1,3-dibromopropane (1.43 g, 7.15 mmol) and TEBAC (0.3 g, 1.36 mmol) in DMSO (30 mL) was added sodium hydroxide aqueous solution (50%, 2.0 mL) at 0 °C. At the end of the addition, the mixture was stirred at 50 °C for 2.0 hrs. After the reaction was completed, the mixture was quenched with water (50 mL). The aqueous layer was extracted with EtOAc (50 mL x 3). The combined organic layers were washed with brine, dried over anhydrous Na₂SO₄ and concentrated *in vacuo.* The residue was purified by silica gel column chromatography (PE) to give the title compound as yellow oil (958 mg, 65%). The compound was characterized by the following spectroscopic data:
MS (ESI, pos.ion) *m*/*z*: 217.5 [M+H]⁺;
¹H NMR (400 MHz,CDCl₃) *δ* (ppm): 6.99, 6.97 (d, d, 1H), 6.66, 6.64 (m, m, 1H), 6.63, 6.62 (m, m, 1H), 6.44, 6.42 (m, m, 1H), 3.82 (s, 3H), 3.76 (s, 3H), 2.40-2.31 (m, 2H), 2.07-1.97 (m, 2H), 1.92-1.71 (m, 2H).

### Step 2) the preparation of compound 40-2

To a solution of compound **40-1** (1.013 g, 4.69 mmol) in isopropanol (15 mL) were added a catalytic amount of Pd/C (100 mg), acetic acid (0.56 g, 9.38 mmol) and NaB H₄ (0.7 g, 18.76 mmol) separately. At the end of the addition, the mixture was stirred at rt for 1.0 hr. After the reaction was completed, the mixture was filtered through a celite pad. The filtrate was concentrated *in vacuo.* The residue was purified by silica gel column chromatography (PE) to give the title compound as yellow oil (818.4 mg, 80%). The compound was characterized by the following spectroscopic data:
MS (ESI, pos.ion) *m*/*z:* 219.1 [M+H]⁺;
¹H NMR (400 MHz, CDCl₃) *δ* (ppm): 6.71, 6.69 (m, m, 1H), 6.63, 6.61 (m, m, 1H), 3.87 (s, 3H), 3.77 (s, 3H), 2.83-2.79 (m, 2H), 2.36-2.18 (m, 4H), 2.15-1.99 (m, 3H), 1.82-1.70 (m, 1H).

### Step 3) the preparation of compound 40-3

To a solution of compound **40-2** (837.6 mg, 3.84 mmol) in DCM (15 mL) was added boron tribromide (2.2 mL, 23.04 mmol) dropwise at -78 °C. At the end of the addition, the mixture was stirred at rt for 1.0 hr. After the reaction was completed, the mixture was quenched with ice water (20 mL) and the organic phase was separated. The aqueous layer was extracted with DCM (20 mL x 3). The combined organic layers were washed with brine, dried over anhydrous Na₂SO₄ and *concentrated in vacuo.* The residue was purified by silica gel column chromatography (PE/EtOAc (v/v) = 4/1) to give the title compound as a white solid (583.99 mg, 80%). The compound was characterized by the following spectroscopic data:
MS (ESI, pos.ion) *m*/*z:* 191.5 [M+H]⁺;
¹H NMR (400 MHz, CDCl₃) *δ* (ppm): 6.58, 6.55 (m, m, 1H), 6.50, 6.48 (s, s, 1H), 5.24 (brs, 2H), 2.74-2.71 (m, 2H), 2.40-2.23 (m, 4H), 2.21-2.03 (m, 3H), 1.86-1.74 (m, 1H).

### Step 4) the preparation of compound 40-4

To a solution of compound **40-3** (577.9 mg, 3.04 mmol) in DCM (15 mL) was added pyridine (1.95 mL, 24.32 mmol) dropwise at 0 °C. After the mixture was stirred for 10 mins, trifluoromethanesulfonic anhydride (2.04 mL, 12.16 mmol) was added, and the mixture was stirred at rt for 2.0 hrs. After the reaction was completed, the mixture was quenched with ice water (50 mL). The resulting mixture was extracted with DCM (30 mL x 3). The combined organic layers were washed with brine, dried over anhydrous Na₂SO₄ and concentrated *in vacuo.* The residue was purified by silica gel column chromatography (PE) to give the title compound as yellow oil (1.28 g, 93.0%). The compound was characterized by the following spectroscopic data:
¹H NMR (400 MHz, CDCl₃) *δ* (ppm): 7.31, 7.29 (s, s, 1H), 7.15, 7.12 (m, m, 1H), 3.08-3.04 (m, 2H), 2.40-2.23 (m, 4H), 2.19-2.15 (m, 2H), 2.10-1.99 (m, 1H), 1.82-1.70 (m, 1H).

### Step 5) the preparation of compound 40-5

A mixture of compound **40-4** (49.94 mg, 0.11 mmol), compound **1-13** (131.68 mg, 0.28 mmol), Pd(PPh₃)₄ (25 mg, 0.02 mmol) and K₂CO₃ (91 mg, 0.66 mmol) in mixed solvents of DME/H₂O (2.4 mL, v/v = 5/1) was stirred at 90 °C under N₂ for 4.0 hrs. After the reaction was completed, the mixture was diluted with EtOAc (20.0 mL). The resulting mixture was washed with brine, dried over anhydrous Na₂SO₄ and concentrated *in vacuo.* The residue was purified by silica gel column chromatography (DCM/MeOH (v/v) = 50/1) to give the title compound as a pale yellow solid (74.14 mg, 80%). The compound was characterized by the following spectroscopic data:
MS (ESI, pos.ion) *m*/*z*: 843.5 [M+H]⁺;
¹H NMR (400 MHz, CDCl₃) *δ* (ppm): 7.71-7.70 (m, 1H), 7.63, 7.61 (s, s, 1H), 7.59-7.54 (m, 3H), 7.50, 7.48 (d, d, 1H), 7.44-7.39 (m, 2H), 5.32, 5.29 (d, d, 2H), 5.24-5.20 (m, 2H), 4.40-4.35 (m, 2H), 3.84-3.78 (m, 2H), 3.68-3.64 (m, 2H), 3.63 (s, 6H), 2.79-2.75 (m, 2H), 2.51-1.70 (m, 18H), 0.97, 0.95 (m, m, 6H), 0.91, 0.89 (m, m, 6H).

### Example 41

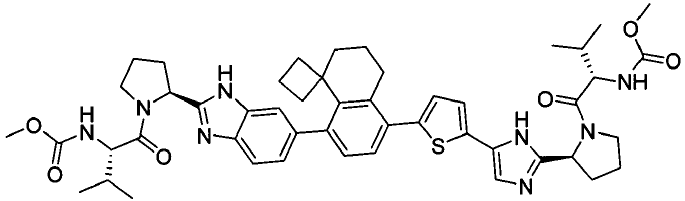

### Synthetic route:

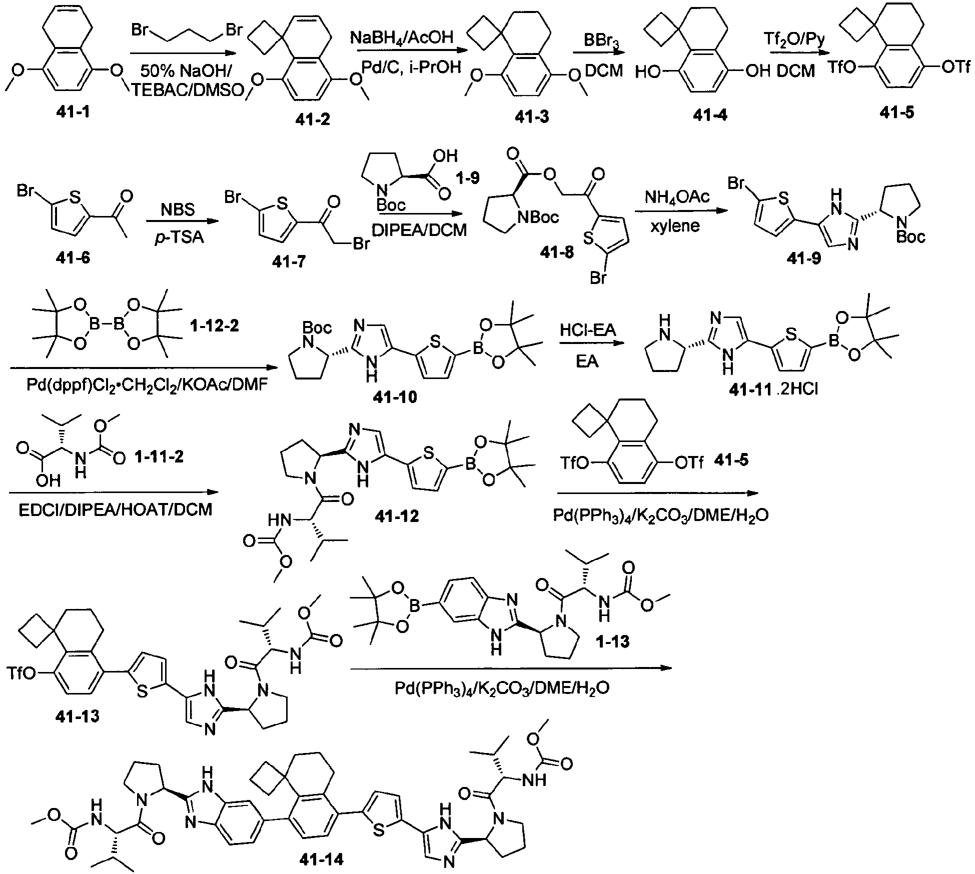

### Step 1) the preparation of compound 41-2

To a suspension of compound **41-1** (1.296 g, 6.82 mmol), 1,3-dibromopropane (1.363 g, 6.82 mmol) and TEBAC (0.3 g, 1.36 mmol) in DMSO (20 mL) was added sodium hydroxide aqueous solution (50%, 2.0 mL) dropwise at 0 °C. At the end of the addition, the mixture was stirred at 50 °C for 2.0 hrs. After the reaction was completed, the mixture was quenched with water (50 mL). The aqueous layer was extracted with EtOAc (50 mL x 3). The combined organic layers were washed with brine, dried over anhydrous Na₂SO₄ and *concentrated in vacuo.* The residue was purified by silica gel column chromatography (PE) to give the title compound as yellow oil (1.02 g, 65%). The compound was characterized by the following spectroscopic data:
MS (ESI, pos.ion) *m*/*z:* 231.5 [M+H]⁺;
¹H NMR (400 MHz, CDCl₃) *δ* (ppm): 6.62, 6.60 (m, m, 1H), 6.46, 6.44 (m, m, 1H), 6.17-6.13 (m, 1H), 5.82-5.77 (m, 1H), 3.81 (s, 3H), 3.74 (s, 3H), 3.41-3.40 (m, 2H), 2.42-2.33 (m, 2H), 2.08-1.99 (m, 2H), 1.90-1.69 (m, 2H).

### Step 2) the preparation of compound 41-3

To a solution of compound **41-2** (1.079 g, 4.69 mmol) in isopropanol (15 mL) were added a catalytic amount of Pd/C (100 mg), acetic acid (0.56 g, 9.38 mmol) and NaB H₄ (0.7 g, 18.76 mmol) separately. At the end of the addition, the mixture was stirred at rt for 1.0 hr. After the reaction was completed, the mixture was filtered through a celite pad. The filtrate was concentrated *in vacuo.* The residue was purified by silica gel column chromatography (PE) to give the title compound as yellow oil (925.4 mg, 85%). The compound was characterized by the following spectroscopic data:
MS (ESI, pos.ion) *m*/*z*: 233.5 [M+H]⁺;
¹H NMR (400 MHz, CDCl₃) *δ* (ppm): 6.83, 6.81 (m, m, 1H), 6.67, 6.65 (m, m, 1H), 3.85 (s, 3H), 3.76 (s, 3H), 2.78-2.48 (m, 6H), 2.13-1.98 (m, 2H), 1.92-1.69 (m, 3H), 1.52-1.44 (m, 1H).

### Step 3) the preparation of compound 41-4

To a solution of compound **41-3** (891.45 mg, 3.84 mmol) in DCM (15 mL) was added boron tribromide (2.2 mL, 23.04 mmol) dropwise at -78 °C. At the end of the addition, the mixture was stirred at -78 °C for 10 mins and at rt for another 1.0 hr. After the reaction was completed, the mixture was quenched with ice water (20 mL) and the organic phase was separated. The aqueous layer was extracted with EtOAc (20 mL x 3). The combined organic layers were washed with brine, dried over anhydrous Na₂SO₄ and concentrated *in vacuo.* The residue was purified by silica gel column chromatography (PE/EtOAc (v/v) = 4/1) to give the title compound as a white solid (627 mg, 80%). The compound was characterized by the following spectroscopic data:
MS (ESI, pos.ion) *m*/*z:* 205.5 [M+H]⁺;
¹H NMR (400 MHz, CDCl₃) *δ* (ppm): 6.50, 6.47 (t, t, 1H), 6.34, 6.32 (t, t, 1H), 5.24 (brs, 2H), 2.76-2.47 (m, 6H), 2.17-2.02 (m, 2H), 1.88-1.69 (m, 3H), 1.56 (m, 1H).

### Step 4) the preparation of compound 41-5

To a solution of compound **41-4** (620.52 mg, 3.04 mmol) in DCM (15 mL) was added pyridine (1.95 mL, 24.32 mmol) dropwise at 0 °C. After the mixture was stirred for 10 mins, trifluoromethanesulfonic anhydride (2.04 mL, 12.16 mmol) was added, and the mixture was stirred at rt for 1.0 hr. After the reaction was completed, the mixture was quenched with ice water (20 mL). The aqueous layer was extracted with EtOAc (20 mL x 3). The combined organic layers were washed with brine, dried over anhydrous Na₂SO₄ and concentrated *in vacuo.* The residue was purified by silica gel column chromatography (PE) to give the title compound as yellow oil (1.28 g, 90%). The compound was characterized by the following spectroscopic data:
¹H NMR (400 MHz, CDCl₃) *δ* (ppm): 7.33, 7.31 (s, s, 1H), 7.20, 7.18 (t, t, 1H), 2.79-2.53 (m, 6H), 2.35-2.26 (m, 1H), 2.09-1.66 (m, 5H).

### Step 5) the preparation of compound 41-7

A mixture of compound **41-6** (5.947 g, 29 mmol), NBS (5.76 g, 32 mmol) and *p*-TSA (1.0 g, 5.2 mmol) was stirred at 100 °C for 0.5 hr. After the reaction was completed, the mixture was cooled to rt, and DCM (100 mL) and water (50 mL) were added. After the layers were partitioned, the aqueous layer was extracted with DCM (50 mL x 3). The combined organic layers were washed with brine, dried over anhydrous Na₂SO₄ and concentrated *in vacuo.* The residue was purified by silica gel column chromatography (PE/DCM/ (v/v) = 5/1) to give the title compound as yellow slurry (5.72 g, 70%). The compound was characterized by the following spectroscopic data:
MS (ESI, pos.ion) *m*/*z*: 284.97 [M+H]⁺;
¹NMR(400 MHz, CDCl₃) *δ* (ppm): 7.55 (d, 1H*, J =* 4.0 Hz), 7.14 (d, 1H, *J =* 4.0 Hz), 4.29 (s, 2H).

### Step 6) the preparation of compound 41-8

To a mixture of compound **41-7** (5.64 g, 19.8 mmol) and compound **1-9** (4.7 g, 21.8 mmol) in DCM (100 mL) was added DIPEA (3.62 mL, 21.9 mmol) dropwise at 0 °C. At the end of the addition, the mixture was stirred at rt for 3.0 hrs. After the reaction was completed, the mixture was quenched with water (50 mL), and the DCM solvent was removed. The resulting mixture was extracted with EtOAc (50 mL x 3). The combined organic layers were washed with brine, dried over anhydrous Na₂SO₄ and *concentrated in vacuo.* The residue was purified by silica gel column chromatography (PE/EtOAc (v/v) = 2/1) to give the title compound as a yellow solid (5.8 g, 70%). The compound was characterized by the following spectroscopic data:
MS (ESI, pos.ion) *m*/*z*: 418.3 [M+H]⁺;
¹H NMR (400 MHz, CDCl₃) *δ* (ppm): 7.49 (d, 1H, *J =* 4.0 Hz), 7.13 (t, 1H, *J =* 4.0 Hz), 5.23-5.02 (m, 2H), 4.48-4.37 (m, 1H), 3.60-3.38 (m, 2H), 2.29-2.26 (m, 2H), 2.11-1.92 (m, 2H), 1.44 (s, 9H).

### Step 7) the preparation of compound 41-9

A mixture of compound **41-8** (7.948 g, 19 mmol) and NH₄OAc (22.2 g, 288 mmol) in xylene (100 mL) was stirred at 140 °C for 5.0 hrs. After the reaction was completed, the mixture was cooled to rt and quenched with water (100 mL). The resulting mixture was extracted with EtOAc (100 mL x 3). The combined organic layers were washed with brine, dried over anhydrous Na₂SO₄ and concentrated *in vacuo.* The residue was purified by silica gel column chromatography (PE/EtOAc (v/v) = 4/1) to give the title compound as a yellow solid (6.94 g, 92%). The compound was characterized by the following spectroscopic data:
MS (ESI, pos.ion) *m*/*z*: 398.32 [M+H]⁺;
¹H NMR (400 MHz, CDCl₃) *δ* (ppm): 10.51 (br, 1H), 7.07 (s, 1H), 6.94 (s, 2H), 4.91-4.90 (m, 1H), 3.39 (s, 2H), 2.98 (s, 1H), 2.12 (s, 2H), 1.95 (s, 1H), 1.48 (s, 9H).

### Step 8) the preparation of compound 41-10

A mixture of compound **41-9** (995.8 mg, 2.5 mmol), compound **1-12-2** (0.96 g, 3.8 mmol), Pd(dppf)Cl₂CH₂Cl₂ (0.11 g, 0.13 mmo) and KOAc (0.74 g, 7.5 mmol) in DMF (12 mL) was stirred at 90 °C under N₂ for 4.0 hrs. After the reaction was completed, the mixture was cooled to rt, diluted with EtOAc (60 mL) and filtered through a celite pad. The filtrate was washed with water (30 mL x 3) and brine, dried over anhydrous Na₂SO₄ and concentrated *in vacuo.* The residue was purified by silica gel column chromatography (PE/EtOAc (v/v) = 3/1) to give the title compound as a white solid (0.89 g, 80%). The compound was characterized by the following spectroscopic data:
¹H NMR (400 MHz, CDCl₃) *δ* (ppm): 10.51 (br, 1H), 7.53 (s, 1H), 7.27 (s, 1H), 7.15 (s, 1H), 4.94-4.93 (m, 1H), 3.39 (s, 2H), 2.99 (s, 1H), 2.12-1.94 (m, 4H), 1.49 (s, 9H), 1.34 (s, 12H), 1.24 (m, 8H).

### Step 9) the preparation of compound 41-11

To a solution of compound **41-10** (227.06 mg, 0.51 mmol) in EtOAc (4.0 mL) was added a solution of HCl in EtOAc (3.0 mL, 4 M) dropwise, and the mixture was stirred at rt for 8.0 hrs. After the reaction was completed, the mixture was concentrated *in vacuo.* The residue was purified by beating in EtOAc (5.0 mL) and filtered to give the title compound as a pale yellow solid (213.26 mg, 100%). The compound was characterized by the following spectroscopic data:
MS (ESI, pos.ion) *m*/*z:* 346.5 [M+H]⁺.

### Step 10) the preparation of compound 41-12

To a mixture of compound **41-11** (121.27 mg, 0.29 mmol), compound **1-11-2** (0.11 g, 0.65 mmol), EDCI (0.12 g, 0.65 mmol) and HOAT (0.08 g, 0.59 mmol) in DCM (5.0 mL) was added DIPEA (0.6 mL, 3.63 mmol) dropwise at 0 °C. At the end of the addition, the mixture was stirred at rt for 3.0 hrs. After the reaction was completed, the mixture was diluted with DCM (20 mL). The resulting mixture was washed with NH₄Cl aqueous solution and brine, dried over anhydrous Na₂SO₄ and concentrated *in vacuo.* The residue was purified by silica gel column chromatography (PE/EtOAc (v/v) = 2/1) to give the title compound as a white solid (116.52 mg, 80%). The compound was characterized by the following spectroscopic data:
MS (ESI, pos.ion) *m*/*z:* 503.5 [M+H]⁺;
¹H NMR (400 MHz, CDCl₃) *δ* (ppm): 7.68, 7.67 (s, s, 1H), 7.27 (d, 1H), 7.11 (dd, 1H), 5.56, 5.55 (d, d, 1H), 5.42-5.37 (m, 1H), 4.34-4.30 (m, 1H), 3.85-3.78 (m, 1H), 3.66 (s, 3H), 3.65-3.61 (m, 1H), 2.32-1.92 (m, 5H), 1.33, 1.30 (m, m, 12H), 1.02, 1.00 (m, m, 3H), 0.93, 0.91 (m, m, 3H).

### Step 11) the preparation of compound 41-13

To a mixture of compound **41-12** (1.707 g, 3.4 mmol), compound **41-5** (1.59 g, 3.4 mmol), Pd(PPh₃)₄ (196.7 mg, 0.17 mmol) and K₂CO₃ (1.412 g, 10.22 mmol) were added DME (24.0 mL) and H₂O (6.0 mL) via syringe, and the mixture was stirred at 90 °C under N₂ for 4.0 hrs. After the reaction was completed, the mixture was cooled to rt and diluted with EtOAc (100 mL). The resulting mixture was washed with water (50 mL x 3) and brine, dried over anhydrous Na₂SO₄ and concentrated *in vacuo.* The residue was purified by silica gel column chromatography (DCM/MeOH (v/v) = 100/1) to give the title compound as a pale yellow solid (1.18 g, 50%). The compound was characterized by the following spectroscopic data:
MS (ESI, pos.ion) *m*/*z*: 695.5 [M+H]⁺;
¹H NMR (400 MHz, CDCl₃) *δ* (ppm): 7.45, 7.43 (t, t, 1H), 7.41 (d, 1H), 7.26, 7.24 (m, m, 1H), 7.15, 7.13 (d, d, 1H), 6.89, 6.88 (s, s, 1H), 5.56, 5.55 (d, d, 1H), 5.42-5.37 (m, 1H), 4.34-4.30 (m, 1H), 3.85-3.78 (m, 1H), 3.66 (s, 3H), 3.65-3.61 (m, 1H), 2.42-1.92 (m, 15H), 1.86-1.68 (m, 1H), 1.60-1.52 (m, 1H), 1.02, 1.00 (m, m, 3H), 0.93, 0.91 (m, m, 3H).

### Step 12) the preparation of compound 41-14

To a mixture of compound **41-13** (423.46 mg, 0.61 mmol), compound **1-13** (286.86 mg, 0.61 mmol), Pd(PPh₃)₄ (70 mg, 0.05 mmol) and K₂CO₃ (254 mg, 1.83 mmol) were added DME (5.0 mL) and H₂O (1.0 mL) via syringe under N₂, and the mixture was stirred at 90 °C for 4.0 hrs. After the reaction was completed, the mixture was cooled to rt and diluted with EtOAc (50 mL). The resulting mixture was washed with water (20 mL x 3) and brine, dried over anhydrous Na₂SO₄ and concentrated *in vacuo.* The residue was purified by silica gel column chromatography (EtOAc) to give the title compound (325.17 mg, 60%). The compound was characterized by the following spectroscopic data:
MS (ESI, pos.ion) *m*/*z:* 445.3 [M+2H]²⁺;
¹H NMR (400 MHz, CDCl₃) *δ* (ppm): 7.65 (m, 1H), 7.58, 7.56 (m, m, 1H), 7.55, 7.53 (d, d, 1H), 7.52, 7.50 (d, d, 1H), 7.48, 7.46 (m, m, 1H), 7.41 (s, 1H), 7.15, 7.13 (d, d, 1H), 6.93, 6.92 (s, s, 1H), 5.56, 5.55 (d, d, 1H), 5.42-5.37 (m, 1H), 5.32, 5.29 (d, d, 1H), 5.25-5.20 (m, 1H), 4.40-4.30 (m, 2H), 3.85-3.78 (m, 2H), 3.68-3.67 (m, 2H), 3.66 (s, 3H), 3.63 (s, 3H), 2.74-2.58 (m, 2H), 2.42-1.92 (m, 15H), 2.53-1.69 (m, 19H), 1.52-1.44 (m, 1H), 1.02-0.89 (m, 12H).

### Example 42

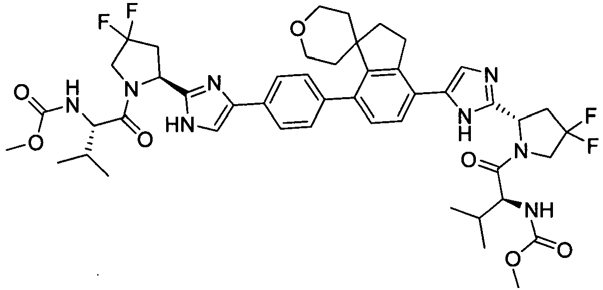

### Synthetic route:

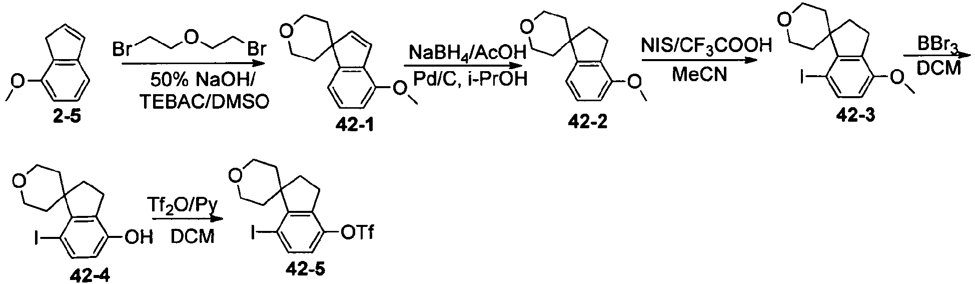

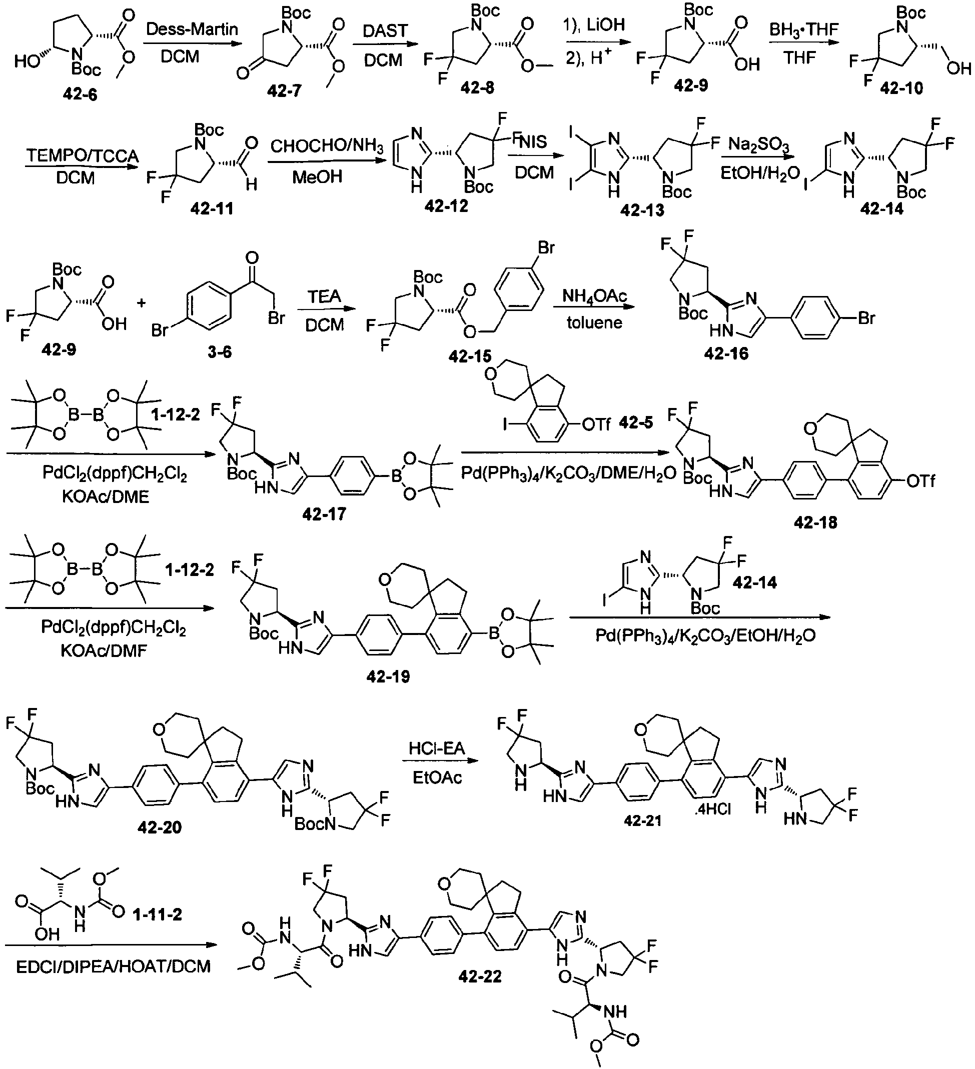

### Step 1) the preparation of compound 42-1

To a suspension of 1-bromo-2-(2-bromoethoxy)ethane (1.64 g, 7.15 mmol), compound **2-5** (996.2 mg, 6.82 mmol) and TEBAC (0.3 g, 1.36 mmol) in DMSO (20 mL) was added sodium hydroxide aqueous solution (50%, 2.0 mL) dropwise at 0 °C. At the end of the addition, the mixture was stirred at 50 °C for 2.0 hrs. After the reaction was completed, the mixture was quenched with water (100 mL). The aqueous layer was extracted with EtOAc (50 mL x 3). The combined organic layers were washed with brine, dried over anhydrous Na₂SO₄ and concentrated *in vacuo.* The residue was purified by silica gel column chromatography (PE) to give the title compound as yellow oil (884.36 mg, 60%). The compound was characterized by the following spectroscopic data:
MS (ESI, pos.ion) *m*/*z*: 217.5 [M+H]⁺;
¹H NMR (400 MHz, CDCl₃) δ (ppm): 6.82-6.78 (m, 1H), 6.77, 6.75 (m, m, 1H), 6.65-6.62 (m, 2H), 6.53, 6.51 (m, m, 1H), 3.87-3.81 (m, 2H), 3.80 (s, 3H), 3.63-3.55 (m, 2H), 2.03-1.94 (m, 2H), 1.81-1.71 (m, 2H).

### Step 2) the preparation of compound 42-2

To a solution of compound **42-1** (1.014 g, 4.69 mmol) in isopropanol (15 mL) were added a catalytic amount of Pd/C (100 mg), acetic acid (0.56 g, 9.38 mmol) and NaB H₄ (0.7 g, 18.76 mmol) separately. At the end of the addition, the mixture was stirred at rt for 1.0 hr. After the reaction was completed, the mixture was filtered through a celite pad. The filtrate was concentrated *in vacuo.* The residue was purified by silica gel column chromatography (PE) to give the title compound as yellow oil (767.3 mg, 75%). The compound was characterized by the following spectroscopic data:
MS (ESI, pos.ion) *m*/*z:* 219.5 [M+H]⁺;
¹H NMR (400 MHz, C DCl₃) *δ* (ppm): 6. 85-6.78 (m, 2H), 6.61-6.59 (m, 1H), 3.82 (s, 3H), 3.81-3.77 (m, 2H), 3.55-3.48 (m, 2H), 2.84-2.80 (m, 2H), 2.11-2.06 (m, 2H), 1.97-1.93 (m, 4H).

### Step 3) the preparation of compound 42-3

To a solution of compound **42-2** (5.715 g, 26.2 mmol) and NIS (6.5 g, 28.8 mmol) in MeCN (60 mL) was added CF₃COOH (0.59 mL, 7.9 mmol) dropwise at 0 °C. At the end of the addition, the mixture was stirred at 45 °C overnight. After the reaction was completed, the mixture was concentrated *in vacuo,* and to the residue was added water (50 mL). The resulting mixture was extracted with EtOAc (60 mL x 3). The combined organic layers were washed with saturated sodium sulfite aqueous solution (30 mL x 2), dried over anhydrous Na₂SO₄ and concentrated *in vacuo.* The residue was purified by silica gel column chromatography (PE) to give the title compound as colorless liquid (7.66 g, 85%). The compound was characterized by the following spectroscopic data:
MS (ESI, pos.ion) *m*/*z:* 345.5 [M+H]⁺;
¹H NMR (400 MHz, CDCl₃) *δ* (ppm): 7.48, 7.46 (dd, dd, 1H), 6.48, 6.46 (m, m, 1H), 3.83 (d, 3H), 3.79-3.73 (m, 2H), 3.57-3.50 (m, 2H), 2.87-2.83 (m, 2H), 2.24-2.18 (m, 2H), 2.04-2.00 (m, 4H).

### Step 4) the preparation of compound 42-4

To a solution of compound **42-3** (1.32 g, 3.84 mmol) in DCM (15 mL) was added boron tribromide (2.2 mL, 23.04 mmol) dropwise at -78 °C. At the end of the addition, the mixture was stirred at rt for 1.0 hr. After the reaction was completed, the mixture was quenched with ice water (50 mL) and the organic phase was separated. The aqueous layer was extracted with EtOAc (20 mL x 3). The combined organic layers were washed with brine, dried over anhydrous Na₂SO₄ and *concentrated in vacuo.* The residue was purified by silica gel column chromatography (PE/EtOAc (v/v) = 4/1) to give the title compound as a white solid (1.014 g, 80%). The compound was characterized by the following spectroscopic data:
MS (ESI, pos.ion) *m*/*z:* 331.5 [M+H]⁺;
¹H NMR (400 MHz, CDCl₃) *δ* (ppm): 7.39, 7.37 (dd, dd, 1H), 6.39, 6.37 (s, s, 1H), 4.81 (brs, 1H), 3.82-3.76 (m, 2H), 3.59-3.52 (m, 2H), 2.83-2.79 (m, 2H), 2.27-2.22 (m, 2H), 2.04-2.00 (m, 4H).

### Step 5) the preparation of compound 42-5

To a solution of compound **42-4** (1.003 g, 3.04 mmol) in DCM (15 mL) was added pyridine (1.95 mL, 24.32 mmol) dropwise at 0 °C. After the mixture was stirred for 10 mins, trifluoromethanesulfonic anhydride (2.04 mL, 12.16 mmol) was added, and the mixture was stirred at rt for 1.0 hr. After the reaction was completed, the mixture was quenched with ice water (30 mL). The aqueous layer was extracted with EtOAc (30 mL x 3). The combined organic layers were washed with brine, dried over anhydrous Na₂SO₄ and concentrated *in vacuo.* The residue was purified by silica gel column chromatography (PE) to give the title compound as yellow oil (1.263 g, 90%). The compound was characterized by the following spectroscopic data:
¹H NMR (400 MHz, CDCl₃) *δ* (ppm): 7.60, 7.58 (dd, dd, 1H), 6.86, 6.84 (s, s, 1H), 3.79-3.73 (m, 2H), 3.57-3.50 (m, 2H), 3.00-2.96 (m, 2H), 2.26-2.20 (m, 2H), 2.03-1.99 (m, 4H).

### Step 6) the preparation of compound 42-7

To a solution of compound **42-6** (6.856 g, 27.97 mmol) in DCM (70 mL) was added Dess-Martin periodinane (23.7 g, 56 mmol) in a portionwise manner at 0 °C. At the end of the addition, the mixture was stirred at rt for 7.0 hrs. After the mixture was completed, the mixture was quenched with Na₂S₂O₃ aqueous solution (100 mL) and filtered through a celite pad. The filtrate was extracted with DCM (100 mL x 3). The combined organic layers were dried over anhydrous Na₂SO₄ and concentrated *in vacuo.* The residue was purified by silica gel column chromatography (PE/EtOAc (v/v) = 6/1) to give the title compound as pale yellow liquid (5.78 g, 85%).

### Step 7) the preparation of compound 42-8

To a solution of compound **42-7** (5.81 g, 23.9 mmol) in DCM (70 mL) was added Et₂NF₃ (4.85 mL, 35.9 mmol) dropwise at -78 °C. At the end of the addition, the mixture was stirred at -78 °C for 2.0 hrs and at rt for another 19 hrs. After the reaction was completed, the mixture was quenched with NH₄Cl aqueous solution (50 mL). The aqueous layer was extracted with DCM (100 mL x 3). The combined organic layers were dried over anhydrous Na₂SO₄ and concentrated *in vacuo.* The residue was purified by silica gel column chromatography (PE/EtOAc (v/v) = 20/1) to give the title compound as pale yellow liquid (5.0 g, 79%). The compound was characterized by the following spectroscopic data:
MS (ESI, pos.ion) *m*/*z*: 266.25 [M+H]⁺;
¹H NMR (400 MHz, CDCl₃) *δ* (ppm): 9.60 (brs, 1H), 4.60-4.57, 4.94-4.72 (m, m, 1H), 3.93-3.84 (m, 2H), 3.77 (s, 3H), 2.78-2.48 (m, 2H), 1.44 (d, 9H, *J* = 16 Hz).

### Step 8) the preparation of compound 42-9

To a solution of compound **42-8** (5.0 g, 18.86 mmol) in THF (40 mL) was added LiOH aqueous solution (1.5 g, 20 mL) at 0 °C, and the mixture was stirred at rt for 2.0 hrs. After the reaction was completed, the mixture was adjusted to pH 5 with diluted hydrochloric acid (1 M), and then the THF solvent was removed *in vacuo.* The aqueous layer was adjusted to pH 2 with diluted hydrochloric acid (1 M) and extracted with EtOAc (80 mL x 3). The combined organic layers were washed with brine, dried over anhydrous Na₂SO₄ and concentrated *in vacuo* to give the title compound as a white solid (4.54 g, 94%). The compound was characterized by the following spectroscopic data:
MS (ESI, pos.ion) *m*/*z*: 252.23 [M+H]⁺;
¹H NMR (400 MHz, CDCl₃) *δ* (ppm): 9.60 (brs, 1H), 4.94-4.72, 4.60-4.57 (m, m, 1H), 3.89-3.74 (m, 2H), 2.78-2.48 (m, 2H), 1.44 (d, 9H, *J* = 16 Hz).

### Step 9) the preparation of compound 42-10

To a solution of compound **42-9** (2.37 g, 9.43 mmol) in THF (30 mL) was added borane (14.2 mL, 1 M in THF) at 0 °C. The mixture was stirred at rt for 2.0 hrs. After the reaction was completed, the mixture was quenched with MeOH (4.0 mL), and the THF solvent was removed *in vacuo.* The residue was dissolved in DCM (100 mL). The solution was washed with water (40 mL x 3) and brine, dried over anhydrous Na₂SO₄ and concentrated *in vacuo* to give the title compound as colorless slurry (1.79 g, 80%). The compound was characterized by the following spectroscopic data:
¹H NMR (400 MHz, CDCl₃) *δ* (ppm): 4.43-4.27 (m, 1H), 3.59-3.34 (m, 2H), 3.60-3.46 (m, 2H), 2.48-2.18 (m, 2H), 1.44 (d, 9H, *J* = 16 Hz).

### Step 10) the preparation of compound 42-12

To a solution of compound **42-10** (1.8 g, 7.59 mmol) in DCM (20 mL) was added TCCA (1.77 g, 7.59 mmol) dropwise at 0 °C followed by a solution of TEMPO (120 mg, 0.76 mmol) in DCM (5.0 mL). The mixture was stirred at 0 °C for 1.0 hr and at rt for another 1.0 hr. After the reaction was completed, the mixture was filtered. The filtrate was washed with saturated Na₂SO₃ aqueous solution (40 mL x 3), dried over anhydrous Na₂SO₄ and concentrated *in vacuo.* The residue was dissolved in a solution of NH₃ in MeOH (20 mL, 7 M). The solution was stirred at 0 °C for 0.5 hr and at rt for another 1.0 hr. A solution of glyoxal in water (2.0 mL, 40%) was added to the mixture dropwise at 0 °C. At the end of the addition, the mixture was stirred at rt for 24 hrs. After the reaction was completed, the mixture was *concentrated in vacuo.* The residue was dissolved in DCM (150 mL). The mixture was washed with water (50 mL x 3) and brine, dried over anhydrous Na₂SO₄ and concentrated *in vacuo.* The residue was purified by silica gel column chromatography (DCM/MeOH (v/v) = 60/1) to give the title compound as a pale yellow solid (1.036 g, 50%). The compound was characterized by the following spectroscopic data:
MS (ESI, pos.ion) *m*/*z*: 274.28 [M+H]⁺;
¹H NMR (400 MHz, CDCl₃) *δ* (ppm): 7.00 (s, 2H), 5.83-5.80 (m, 1H), 4.05-3.79 (m, 1H), 3.74-3.52 (m, 1H), 3.11-2.33 (m, 2H), 1.51 (s, 9H).

### Step 11) the preparation of compound 42-13

To a solution of compound **42-12** (0.93 g, 3.4 mmol) in DCM (30 mL) was added NIS (1.7 g, 7.5 mmol) at 0 °C, and the mixture was stirred at 0 °C for 2.0 hrs. After the reaction was completed, the mixture was filtered. The filtrate was washed with saturated Na₂SO₃ aqueous solution (50 mL x 3), dried over anhydrous Na₂SO₄ and concentrated *in vacuo* to give the title compound as a yellow solid (1.07 g, 60%), which was used for the next step without further purification. The compound was characterized by the following spectroscopic data:
MS (ESI, pos.ion) *m*/*z*: 525.08 [M+H]⁺;
¹H NMR (400 MHz, CDCl₃) *δ* (ppm): 5.13-5.08 (m, 1H), 3.91-3.87 (m, 1H), 3.58-3.46 (m, 2H), 2.74-2.72 (m, 1H), 1.51 (s, 9H).

### Step 12) the preparation of compound 42-14

To a solution of compound **42-13** (1.03 g, 1.96 mmol) in ethanol (10 mL) were added Na₂SO₃ (2.47 g, 19.6 mmol) and water (10 mL), and the mixture was stirred at 90 °C for 30 hrs. After the reaction was completed, the mixture was filtered, and the filtrate was concentrated *in vacuo.* The residue was dissolved in DCM (80 mL). The resulting mixture was washed with water and brine, dried over anhydrous Na₂SO₄ and concentrated *in vacuo.* The residue was purified by silica gel column chromatography (PE/EtOAc (v/v) = 6/1) to give the title compound as a white solid (0.258 g, 33%). The compound was characterized by the following spectroscopic data:
MS (ESI, pos.ion) *m*/*z:* 400.18 [M+H]⁺;
¹H NMR (400 MHz, CDCl₃) *δ* (ppm): 7.08 (s, 1H), 5.33-4.95 (m, 1H), 3.91-3.87 (m, 1H), 3.78-3.36 (m, 2H), 2.96-2.55 (m, 1H), 1.49 (s, 9H).

### Step 13) the preparation of compound 42-15

To a solution of compound **3-6** (2.41 g, 8.66 mmol) and compound **42-9** (2.17 g, 8.66 mmol) in DCM (30 mL) was added TEA (2.5 mL, 17.32 mmol) dropwise at 0 °C. At the end of the addition, the mixture was stirred at rt for 3.0 hrs. After the reaction was completed, the mixture was quenched with water (50 mL). The resulting mixture was extracted with DCM (30 mL x 3). The combined organic layers were dried over anhydrous Na₂SO₄ and concentrated *in vacuo* to give the crude product (3.6 g), which was used for the next step without further purification. The compound was characterized by the following spectroscopic data:
MS (ESI, pos.ion) *m*/*z*: 421.25 [M+H]⁺.

### Step 14) the preparation of compound 42-16

A suspension of compound **42-15** (3.6 g, 8.6 mmol) and ammonium acetate (7.0 g, 86 mmol) in toluene (30 mL) was stirred at 110 °C for 5.0 hrs. After the reaction was completed, the mixture was cooled to rt and quenched with water (60 mL). The resulting mixture was extracted with EtOAc (80 mL x 3). The combined organic layers were dried over anhydrous Na₂SO₄ and concentrated *in vacuo.* The residue was purified by silica gel column chromatography (PE/EtOAc (v/v) = 6/1) to give the title compound (1.47 g, 40%). The compound was characterized by the following spectroscopic data:
MS (ESI, pos.ion) *m*/*z*: 429.27 [M+H]⁺;
¹H NMR (400 MHz, CDCl₃) *δ* (ppm): 7.54-7.52 (m, 2H), 7.48-7.46 (m, 2H), 7.26-7.25 (m, 1H), 5.19-5.18 (m, 1H), 3.70-3.52 (m, 2H), 2.78-2.65 (m, 2H), 1.48 (s, 9H).

### Step 15) the preparation of compound 42-17

A mixture of compound **42-16** (1.4 g, 3.27 mmol), compound **1-12-2** (0.92 g, 3.6 mmol), Pd(dppf)Cl₂CH₂Cl₂ (0.13 g, 1.16 mmol) and KOAc (0.81 g, 8.17 mmol) in DME (25 mL) was stirred at 90 °C under N₂ for 2.0 hrs. After the reaction was completed, the mixture was diluted with EtOAc (80 mL) and filtered through a celite pad. The filtrate was washed with water (30 mL x 3) and brine, dried over anhydrous Na₂SO₄ and concentrated *in vacuo.* The residue was purified by silica gel column chromatography (PE/EtOAc (v/v) = 1/2) to give the title compound as a solid (1.5 g, 96%). The compound was characterized by the following spectroscopic data:
MS (ESI, pos.ion) *m*/*z:* 476.34 [M+H]⁺;
¹H NMR (400 MHz, CDCl₃) *δ* (ppm): 7.54-7.52 (m, 2H), 7.48-7.46 (m, 2H), 7.26-7.25 (m, 1H), 5.19-5.18 (m, 1H), 3.70-3.52 (m, 2H), 2.78-2.65 (m, 2H), 1.48 (s, 9H), 1.35 (s, 12H).

### Step 16) the preparation of compound 42-18

To a mixture of compound **42-17** (2.139 g, 4.5 mmol), compound **42-5** (2.079 g, 4.5 mmol), Pd(PPh₃)₄ (260 mg, 0.225 mmol) and K₂CO₃ (1.24 g, 9.0 mmol) were added DME (20 mL) and pure water (4.0 mL) via syringe under N₂. The mixture was stirred at 90 °C for 2.0 hrs. After the reaction was completed, the mixture was diluted with EtOAc (100 mL). The resulting mixture was washed with water (30 mL x 3) and brine, dried over anhydrous Na₂SO₄ and concentrated *in vacuo.* The residue was purified by silica gel column chromatography (PE/EtOAc (v/v) = 6/1) to give the title compound as a white solid (2.152 g, 70%). The compound was characterized by the following spectroscopic data:
MS (ESI, pos.ion) *m*/*z:* 684.5 [M+H]⁺;
¹H NMR (400 MHz, CDCl₃) *δ* (ppm): 7.66-7.61 (m, 4H), 7.46 (s, 1 H), 7.28, 7.26 (s, s, 1H), 6.93, 6.91 (dd, dd, 1H), 4.93-4.88 (m, 1H), 4.18-4.05 (m, 1H), 3.92-3.82 (m, 1H), 3.80-3.73 (m, 2H), 3.55-3.48 (m, 2H), 2.99-2.95 (m, 2H), 2.86-2.68 (m, 1H), 2.47-2.26 (m, 1H), 2.21-2.16 (m, 2H), 2.11-2.07 (m, 4H), 1.41 ( s, 9H).

### Step 17) the preparation of compound 42-19

A mixture of compound **42-18** (1.57 g, 2.3 mmol), compound **1-12-2** (0.64 g, 2.53 mmol), Pd(dppf)Cl₂·CH₂Cl₂ (90 mg, 0.115 mmol) and KOAc (0.6 g, 5.75 mmol) in DMF (15 mL) was stirred at 120 °C under N₂ for 4.0 hrs. After the reaction was completed, the mixture was diluted with EtOAc (100 mL) and filtered through a celite pad. The filtrate was washed with water (50 mL x 3) and brine, dried over anhydrous Na₂SO₄ and concentrated *in vacuo.* The residue was purified by silica gel column chromatography (PE/EtOAc (v/v) = 4/1) to give the title compound as a white solid (1.065 g, 70%). The compound was characterized by the following spectroscopic data:
MS (ESI, pos.ion) *m*/*z*: 662.5 [M+H]⁺;
¹H NMR (400 MHz, CDCl₃) *δ* (ppm): 7.71-7.67 (m, 2H), 7.65-7.61 (m, 2H), 7.57, 7.54 (dd, dd, 1H), 7.46 (s, 1H), 7.45, 7.44 (s, s, 1H), 4.93-4.88 (m, 1H), 4.18-4.05 (m, 1H), 3.92-3.79 (m, 1H), 3.67-3.62 (m, 2H), 3.47-3.41 (m, 2H), 2.94-2.90 (m, 2H), 2.86-2.68 (m, 1H), 2.47-2.27 (m, 1H), 2.25-2.19 (m, 2H), 2.09-2.05 (m, 4H), 1.41 (s, 9H), 1.32, 1.29 (m, m, 12H).

### Step 18) the preparation of compound 42-20

A suspension of compound **42-14** (219.47 mg, 0.55 mmol), compound **42-19** (363.74 mg, 0.55 mmol), Pd(PPh₃)₄ (32 mg, 0.027 mmol) and K₂CO₃ (190 mg, 1.37 mmol) in mixed solvents of EtOH and H₂O (7.5 mL, v/v = 4/1) was stirred at 90 °C under N₂ for 2.0 hrs. After the reaction was completed, the mixture was concentrated *in vacuo.* The residue was dissolved in EtOAc (50 mL). The solution was washed with brine, dried over anhydrous Na₂SO₄ and *concentrated in vacuo.* The residue was purified by silica gel column chromatography (DCM/MeOH (v/v) = 100/1) to give the title compound as a white solid (257.23 mg, 58%). The compound was characterized by the following spectroscopic data:
MS (ESI, pos.ion) *m*/*z*: 807.5 [M+H]⁺;
¹H NMR (400 MHz, CDCl₃) *δ* (ppm): 7.96 (s, 1H), 7.70-7.67 (m, 2H), 7.65-7.61 (m, 2H), 7.49-7.46 (m, 2H), 7.17, 7.15 (s, s, 1H), 5.14-5.09 (m, 1H), 4.93-4.88 (m, 1H), 4.18-4.05 (m, 2H), 3.92-3.79 (m, 2H), 3.77-3.71 (m, 2H), 3.54-3.47 (m, 2H), 2.88-2.68 (m, 4H), 2.49-2.26 (m, 2H), 2.24-2.18 (m, 2H), 2.04-2.00 (m, 4H), 1.53 (s, 9H), 1.41 (s, 9H).

### Step 19) the preparation of compound 42-21

To a solution of compound **42-20** (249.97 mg, 0.31 mmol) in EtOAc (4.0 mL) was added a solution of HCl in EtOAc (3.0 mL, 4 M) dropwise. At the end of the addition, the mixture was stirred at rt for 8.0 hrs. After the reaction was completed, the mixture was concentrated *in vacuo.* The residue was purified by beating in EtOAc (4.0 mL) and filtered to give the title compound as a pale yellow solid (186.56 mg, 80%), which was used for the next step without further purification.

### Step 20) the preparation of compound 42-22

To a suspension of compound **42-21** (195.6 mg, 0.26 mmol), compound **1-11-2** (100 mg, 0.57 mmol), EDCI (110 mg, 0.57 mmol) and HOAT (70 mg, 0.52 mmol) in DCM (6.0 mL) was added DIPEA (0.43 mL, 2.6 mmol) dropwise at 0 °C. At the end of the addition, the mixture was stirred at rt for 3.0 hrs. After the reaction was completed, the mixture was diluted with DCM (20 mL), washed with NH₄Cl aqueous solution and brine, dried over anhydrous Na₂SO₄ and concentrated *in vacuo.* The residue was purified by silica gel column chromatography (DCM/MeOH (v/v) = 40/1) to give the title compound as a white solid (191.44 mg, 80%). The compound was characterized by the following spectroscopic data:
MS (ESI, pos.ion) *m*/*z*: 461.3 [M+2H]²⁺;
¹H NMR (400 MHz, CDCl₃) *δ* (ppm): 7.85 (s, 1H), 7.70-7.67 (m, 2H), 7.65-7.61 (m, 2H), 7.49-7.48 (d, d, 0.5H), 7.46 (dd, 0.5H), 7.43 (s, 1H), 7.17, 7.15 (s, s, 1H), 5.56, 5.55 (d, d, 1H), 5.46, 5.44 (d, d, 1H), 5.24-5.19 (m, 1H), 5.10-5.05 (m, 1H), 4.48-4.43 (m, 1H), 4.32-4.28 (m, 1H), 4.21-4.07 (m, 2H), 3.94-3.81 (m, 2H), 3.77-3.71 (m, 2H), 3.66 (s, 6H), 3.54-3.47 (m, 2H), 2.92-2.72 (m, 4H), 2.52-2.12 (m, 6H), 2.04-2.00 (m, 4H), 1.02-0.89 (m, 12H).

### Example 43

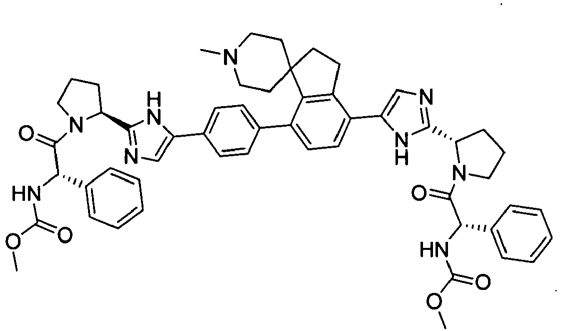

### Synthetic route:

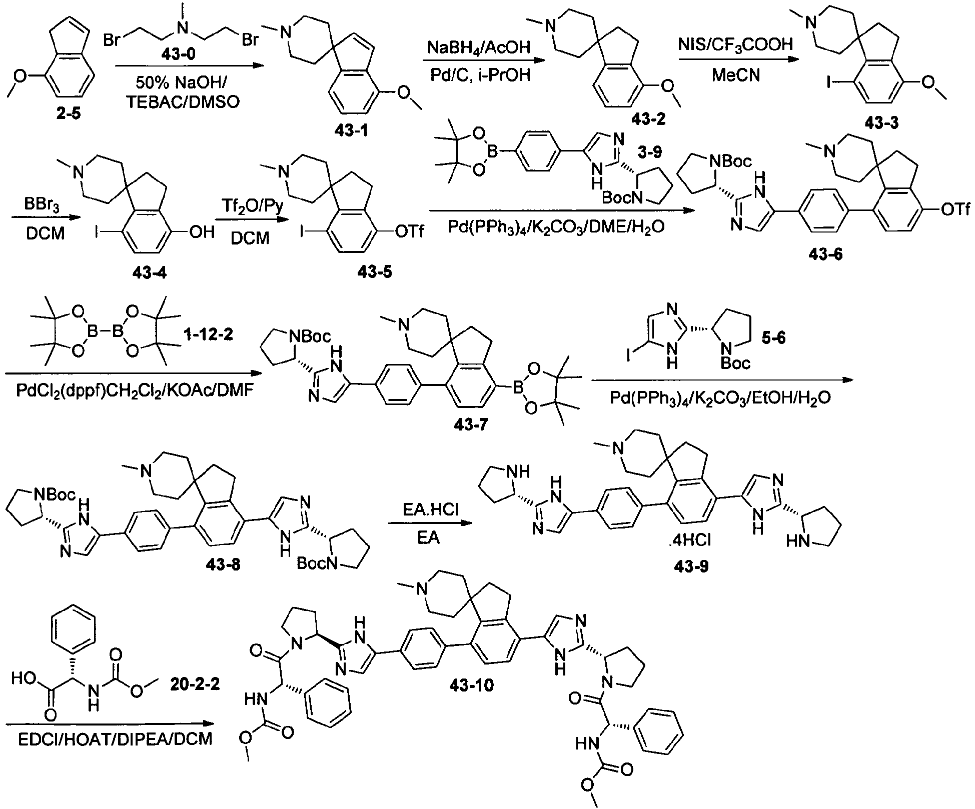

### Step 1) the preparation of compound 43-1

To a suspension of compound **2-5** (996.2 mg, 6.82 mmol), compound **43-0** (1.737 g, 7.15 mmol) and TEBAC (0.3 g, 1.36 mmol) in DMSO (30 mL) was added sodium hydroxide aqueous solution (50%, 2.0 mL) at 0 °C. At the end of the addition, the mixture was stirred at 50 °C for 2.0 hrs. After the reaction was completed, the mixture was quenched with water (50 mL). The aqueous layer was extracted with EtOAc (50 mL x 3). The combined organic layers were washed with brine, dried over anhydrous Na₂SO₄ and concentrated *in vacuo.* The residue was purified by silica gel column chromatography (PE) to give the title compound as yellow oil (937.68 mg, 60%). The compound was characterized by the following spectroscopic data:
MS (ESI, pos.ion) *m*/*z*: 230.5 [M+H]⁺;
¹H NMR (400 MHz, CDCl₃) *δ* (ppm): 6.87-6.83 (m, 1H), 6.78-6.77 (m, 2H), 6.65-6.62 (m, 1H), 6.58, 6.56 (m, m, 1H), 3.80 (s, 3H), 2.89-2.81 (m, 2H), 2.55-2.48 (m, 2H), 2.45 (m, 3H), 1.81-1.75 (m, 4H).

### Step 2) the preparation of compound 43-2

To a solution of compound **43-1** (1.075 g, 4.69 mmol) in isopropanol (15 mL) were added a catalytic amount of Pd/C (100 mg), acetic acid (0.56 g, 9.38 mmol) and NaB H₄ (0.7 g, 18.76 mmol) separately. At the end of the addition, the mixture was stirred at rt for 1.0 hr. After the reaction was completed, the mixture was filtered through a celite pad. The filtrate was concentrated *in vacuo.* The residue was purified by silica gel column chromatography (PE) to give the title compound as yellow oil (813.1 mg, 75%). The compound was characterized by the following spectroscopic data:
MS (ESI, pos.ion) *m*/*z*: 232.5 [M+H]⁺;
¹H NMR (400 MHz, CDCl₃) *δ* ( ppm): 6.89 -6.82 (m, 2H), 6.61-6.59 (m, 1H), 3.82 (s, 3H), 2.90-2.86 (m, 2H), 2.57-2.49 (m, 2H), 2.32-2.31 (m, 2H), 2.20-2.28 (m, 3H), 2.09-2.04 (m, 2H), 2.00-1.90 (m, 2H), 1.53-1.45 (m, 2H).

### Step 3) the preparation of compound 43-3

To a mixture of compound **43-2** (605.6 mg, 2.62 mmol) and NIS (650 mg, 2.88 mmol) in MeCN (6.0 mL) was added CF₃COOH (0.059 mL, 0.79 mmol) dropwise at 0 °C. At the end of the addition, the mixture was stirred at 45 °C overnight. After the reaction was completed, the mixture was concentrated *in vacuo,* and to the residue was added water (50 mL). The mixture was extracted with EtOAc (20 mL x 3). The combined organic layers were washed with saturated sodium sulfite aqueous solution (10 mL x 2), dried over anhydrous Na₂SO₄ and *concentrated in vacuo.* The residue was purified by silica gel column chromatography (PE) to give the title compound as colorless oil (759 mg, 85%). The compound was characterized by the following spectroscopic data:
MS (ESI, pos.ion) *m*/*z:* 358.5 [M+H]⁺;
¹H NMR (400 MHz, CDCl₃) *δ* (ppm): 7.44, 7.42 (dd, dd, 1H), 6.48, 6.46 (m, m, 1H), 3.83 (s, 3H), 2.90-2.86 (m, 2H), 2.59-2.51 (m, 2H), 2.34-2.33 (m, 3H), 2.32-2.26 (m, 2H), 2.22-2.16 (m, 2H), 2.11-2.01 (m, 2H), 1.55-1.47 (m, 2H).

### Step 4) the preparation of compound 43-4

To a solution of compound **43-3** (1.37 g, 3.84 mmol) in DCM (15 mL) was added boron tribromide (2.2 mL, 23.04 mmol) dropwise at -78 °C. The mixture was stirred at -78 °C for 10 mins and at rt for 1.0 hr. After the reaction was completed, the mixture was quenched with ice water (20 mL) and the organic phase was separated. The aqueous layer was extracted with EtOAc (20 mL x 3). The combined organic layers were washed with brine, dried over anhydrous Na₂SO₄ and concentrated *in vacuo.* The residue was purified by silica gel column chromatography (PE/EtOAc (v/v) = 4/1) to give the title compound as a white solid (1.054 g, 80%). The compound was characterized by the following spectroscopic data:
MS (ESI, pos.ion) *m*/*z:* 344.5 [M+H]⁺;
¹H NMR (400 MHz, CDCl₃) *δ* (ppm): 7.35, 7.33 (dd, dd, 1H), 6.39, 6.37 (s, s, 1H), 4.81 (brs, 1H), 2.82-2.78 (m, 2H), 2.60-2.53 (m, 2H), 2.37-2.28 (m, 5H), 2.24-2.19 (m, 2H), 2.14-2.04 (m, 2H), 1.59-1.51 (m, 2H).

### Step 5) the preparation of compound 43-5

To a solution of compound **43-4** (1.043 g, 3.04 mmol) in DCM (15 mL) was added pyridine (1.95 mL, 24.32 mmol) dropwise at 0 °C. After the mixture was stirred for 10 mins, trifluoromethanesulfonic anhydride (2.04 mL, 12.16 mmol) was added, and the mixture was stirred at rt for 1.0 hr. After the reaction was completed, the mixture was quenched with ice water (20 mL). The aqueous layer was extracted with EtOAc (20 mL x 3). The combined organic layers were washed with brine, dried over anhydrous Na₂SO₄ and *concentrated in vacuo.* The residue was purified by silica gel column chromatography (PE) to give the title compound as yellow oil (1.3 g, 90%). The compound was characterized by the following spectroscopic data:
¹H NMR (400 MHz, CDCl₃) δ (ppm): 7.56, 7.54 (dd, dd, 1H), 6.86, 6.84 (s, s, 1H), 3.07-3.03 (m, 2H), 2.59-2.51 (m, 2H), 2.35-2.26 (m, 5H), 2.24-2.18 (m, 2H), 2.11-2.00 (m, 2H), 1.54-1.46 (m, 2H).

### Step 6) the preparation of compound 43-6

A suspension of compound **43-5** (2.137 g, 4.5 mmol), compound **3-9** (1.976 g, 4.5 mmol), Pd(PPh₃)₄ (260 mg, 0.225 mmol) and K₂CO₃ (1.24 g, 9.0 mmol) in mixed solvents of DME and H₂O (24 mL, v/v = 5/1) was stirred at 90 °C under N₂ for 2.0 hrs. After the reaction was completed, the mixture was diluted with EtOAc (50 mL). The resulting mixture was washed with brine, dried over anhydrous Na₂SO₄ and concentrated *in vacuo.* The residue was purified by silica gel column chromatography (PE/EtOAc (v/v) = 6/1) to give the title compound as a white solid (2.08 g, 70%). The compound was characterized by the following spectroscopic data:
MS (ESI, pos.ion) *m*/*z*: 661.5 [M+H]⁺;
¹H NMR (400 MHz, CDCl₃) *δ* (ppm): 7.59 (s, 1H), 7.54-7.51 (m, 2H), 7.45-7.41 (m, 2H), 7.28, 7.26 (s, s, 1H), 6.98, 6.96 (dd, dd, 1H), 4.98-4.92 (m, 1H), 3.65-3.58 (m, 1H), 3.31-3.23 (m, 1H), 3.06-3.02 (m, 2H), 2.56-2.49 (m, 2H), 2.47-2.38 (m, 1H), 2.34-2.33 (m, 3H), 2.32-2.14 (m, 5H), 2.10-1.97 (m, 4H), 1.56-1.54 (m, 2H), 1.53 (s, 9H).

### Step 7) the preparation of compound 43-7

A suspension of compound **43-6** (1.518 g, 2.3 mmol), compound **1-12-2** (640 mg, 2.53 mmol), Pd(dppf)Cl₂·CH₂Cl₂ (90 mg, 0.115 mmol) and KOAc (600 mg, 5.75 mmol) in DMF (15 mL) was stirred at 120 °C under N₂ for 4.0 hrs. After the reaction was completed, the mixture was diluted with EtOAc (50 mL) and filtered through a celite pad. The filtrate was washed with water (30 mL x 3) and brine, dried over anhydrous Na₂SO₄ and concentrated *in vacuo.* The residue was purified by silica gel column chromatography (PE/EtOAc (v/v) = 1/1) to give the title compound as a white solid (1.027 g, 70%). The compound was characterized by the following spectroscopic data:
MS (ESI, pos.ion) *m*/*z:* 639.5 [M+H]⁺;
¹H NMR (400 MHz, CDCl₃) *δ* (ppm): 7.59 (s, 1H), 7.58, 7.55 (dd, dd, 1H), 7.54-7.51 (m, 2H), 7.49-7.46 (m, 2H), 7.44 (s, 1H), 4.97-4.93 (m, 1H), 3.65-3.58 (m, 1H), 3.31-3.24 (m, 1H), 2.95-2.91 (m, 2H), 2.49-2.38 (m, 3H), 2.34-2.33 (m, 3H), 2.28-2.16 (m, 5H), 2.10-1.96 (m, 4H), 1.57-1.54 (m, 2H), 1.53 (s, 9H), 1.32, 1.29 (m, m, 12H).

### Step 8) the preparation of compound 43-8

A suspension of compound **43-7** (351.12 mg, 0.55 mmol), compound **5-6** (199.67 mg, 0.55 mmol), Pd(PPh₃)₄ (32 mg, 0.027 mmol) and K₂CO₃ (190 mg, 1.37 mmol) in mixed solvents of EtOH and H₂O (7.5 mL, v/v = 4/1) was stirred at 90 °C under N₂ for 2.0 hrs. After the reaction was completed, the mixture was diluted with EtOAc (50 mL). The organic layers were washed with brine, dried over anhydrous Na₂SO₄ and concentrated *in vacuo.* The residue was purified by silica gel column chromatography (DCM/MeOH (v/v) = 100/1) to give the title compound as a white solid (205.54 mg, 50%). The compound was characterized by the following spectroscopic data:
MS (ESI, pos.ion) *m*/*z:* 748.5 [M+H]⁺;
¹H NMR (400 MHz, CDCl₃) *δ* (ppm): 7.78 (s, 1H), 7.59 (s, 1H), 7.54-7.51 (m, 2H), 7.48-7.46 (m, 2H), 7.45, 7.42 (dd, dd, 1H), 7.17, 7.15 (s, s, 1H), 5.14-5.08 (m, 1H), 4.97-4.93 (m, 1H), 3.65-3.58 (m, 2H), 3.31-3.23 (m, 2H), 2.85-2.81 (m, 2H), 2.55-2.48 (m, 2H), 2.47-2.38 (m, 2H), 2.34-2.33 (m, 3H), 2.32-2.16 (m, 6H), 2.10-1.97 (m, 6H), 1.53 (s, 9H), 1.52-1.45 (m, 2H), 1.41 (s, 9H).

### Step 9) the preparation of compound 43-9

To a solution of compound **43-8** (231.7 mg, 0.31 mmol) in EtOAc (4.0 mL) was added a solution of HCl in EtOAc (3.0 mL, 4 M) dropwise. At the end of the addition, the mixture was stirred at rt for 8.0 hrs. After the reaction was completed, the mixture was concentrated *in vacuo.* The residue was purified by beating in EtOAc (4.0 mL) and filtered to give the title compound as a pale yellow solid (171.95 mg, 80%), which was used for the next step without further purification.

### Step 10) the preparation of compound 43-10

To a suspension of compound **43-9** (180.27 mg, 0.26 mmol), compound **20-2-2** (119.16 mg, 0.57 mmol), EDCI (110 mg, 0.57 mmol) and HOAT (70 mg, 0.52 mmol) in DCM (6.0 mL) was added DIPEA (0.43 mL, 2.6 mmol) dropwise at 0 °C. At the end of the addition, the mixture was stirred at rt for 3.0 hrs. After the reaction was completed, the mixture was diluted with DCM (20 mL), washed with NH₄Cl aqueous solution and brine, dried over anhydrous Na₂SO₄ and *concentrated in vacuo.* The residue was purified by silica gel column chromatography (DCM/MeOH (v/v) = 60/1) to give the title compound as a white solid (193.32 mg, 80%). The compound was characterized by the following spectroscopic data:
MS (ESI, pos.ion) *m*/*z*: 465.73 [M+2H]²⁺;
¹H NMR (400 MHz, CDCl₃) *δ* (ppm): 7.71 (s, 1H), 7.59 (s, 1H), 7.54-7.51 (m, 2H), 7.48-7.46 (m, 2H), 7.45, 7.42 (dd, dd, 1H), 7.35-7.27 (m, 6H), 7.20-7.15 (m, 5H), 5.91, 5.89 (s, s, 2H), 5.35-5.34 (m, 1H), 5.33-5.32 (m, 1H), 5.25-5.20 (m, 1H), 5.18-5.13 (m, 1H), 3.91-3.84 (m, 1H), 3.75-3.67 (m, 2H), 3.64 (s, 6H), 3.41-3.33 (m, 1H), 2.85-2.81 (m, 2H), 2.55-2.48 (m, 2H), 2.34-2.33 (m, 3H), 2.31-1.93 (m, 14H), 1.52-1.44 (m, 2H).

### Example 44

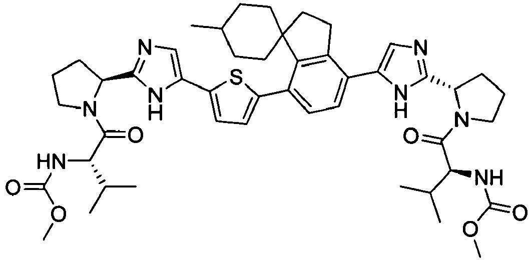

### Synthetic route:

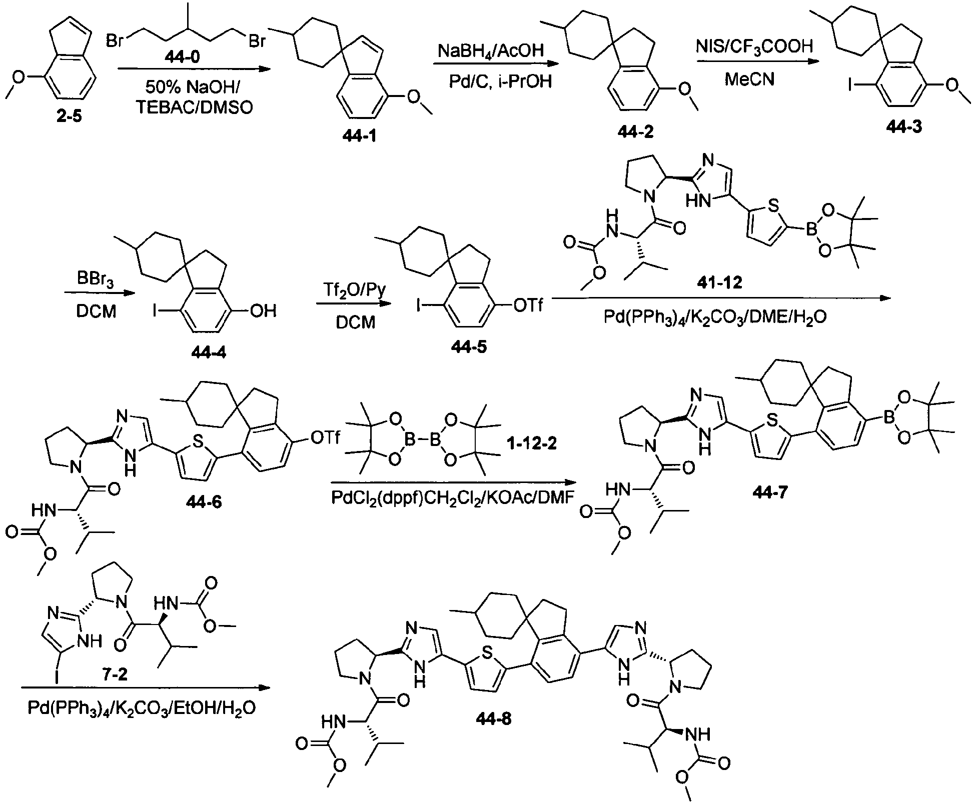

### Step 1) the preparation of compound 44-1

To a solution of compound **2-5** (996.2 mg, 6.82 mmol), compound **44-0** (1.73 g, 7.15 mmol) and TEBAC (0.3 g, 1.36 mmol) in DMSO (30 mL) was added sodium hydroxide aqueous solution (50%, 2.0 mL) dropwise at 0 °C. At the end of the addition, the mixture was stirred at 50 °C for 2.0 hrs. After the reaction was completed, the mixture was quenched with water (100 mL). The aqueous layer was extracted with EtOAc (50 mL x 3). The combined organic layers were washed with brine, dried over anhydrous Na₂SO₄ and *concentrated in vacuo.* The residue was purified by silica gel column chromatography (PE) to give the title compound as yellow oil (1.089 g, 70%). The compound was characterized by the following spectroscopic data:
MS (ESI, pos.ion) *m*/*z*: 229.5 [M+H]⁺;
¹H NMR (400 MHz, CDCl₃) *δ* (ppm): 6.86, 6.84, 6.82 (d, d, d, 1H), 6.71, 6.69 (m, m, 1H), 6.65-6.64, 6.63-6.62 (m, m, 1H), 6.61-6.58 (m, 1H), 6.50-6.49, 6.48-6.47 (m, m, 1H), 3.80 (s, 3H), 1.90-1.85 (m, 4H), 1.64-1.58 (m, 2H), 1.48-1.38 (m, 2H), 1.34-1.21 (m, 1H), 0.94-0.92 (m, 3H).

### Step 2) the preparation of compound 44-2

To a solution of compound **44-1** (1.07 g, 4.69 mmol) in isopropanol (15 mL) were added a catalytic amount of Pd/C (100 mg), acetic acid (0.56 g, 9.38 mmol) and NaB H₄ (0.7 g, 18.76 mmol) separately. At the end of the addition, the mixture was stirred at rt for 1.0 hr. After the reaction was completed, the mixture was diluted with EtOAc (50 mL) and filtered through a celite pad. The filtrate was concentrated *in vacuo.* The residue was purified by silica gel column chromatography (PE) to give the title compound as yellow oil (809.62 mg, 75%). The compound was characterized by the following spectroscopic data:
MS (ESI, pos.ion) *m*/*z:* 231.5 [M+H]⁺;
¹H NMR (400 MHz, CDCl₃) *δ* (ppm): 6.91-6.87 (m, 1H), 6.79-6.76 (m, 1H), 6.61-6.59 (m, 1H), 3.82 (s, 3H), 2.86-2.82 (m, 2H), 2.16-2.08 (m, 2H), 2.07-1.96 (m, 4H), 1.37-1.25 (m, 3H), 1.13-1.04 (m, 2H), 0.94-0.91 (m, 3H).

### Step 3) the preparation of compound 44-3

To a solution of compound **44-2** (603 mg, 2.62 mmol) and NIS (650 mg, 2.88 mmol) in MeCN (6.0 mL) was added CF₃COOH (0.059 mL, 0.79 mmol) dropwise at 0 °C. At the end of the addition, the mixture was stirred at 45 °C overnight. After the reaction was completed, the mixture was concentrated *in vacuo.* To the residue was added water (20 mL), and the mixture was extracted with EtOAc (20 mL x 3). The combined organic layers were washed with saturated sodium sulfite aqueous solution (20 mL x 2), dried over anhydrous Na₂SO₄ and concentrated *in vacuo.* The residue was purified by silica gel column chromatography (PE) to give the title compound as colorless liquid (746.3 mg, 80%). The compound was characterized by the following spectroscopic data:
MS (ESI, pos.ion) *m*/*z:* 357.5 [M+H]⁺;
¹H NMR (400 MHz, CDCl₃) *δ* (ppm): 7.42, 7.40 (dd, dd, 1H), 6.48, 6.46 (m, m, 1H), 3.83 (s, 3H), 2.87-2.83 (m, 2H), 2.25-2.14 (m, 4H), 1.91-1.82 (m, 2H), 1.38-1.20 (m, 3H), 1.18-1.09 (m, 2H), 0.91-0.88 (m, 3H).

### Step 4) the preparation of compound 44-4

To a solution of compound **44-3** (1.367 g, 3.84 mmol) in DCM (15 mL) was added boron tribromide (2.2 mL, 23.04 mmol) dropwise at -78 °C. At the end of the addition, the mixture was stirred at rt for 1.0 hr. After the reaction was completed, the mixture was quenched with ice water (50 mL) and the organic phase was separated. The aqueous layer was extracted with EtOAc (30 mL x 3). The combined organic layers were washed with brine, dried over anhydrous Na₂SO₄ and concentrated *in vacuo.* The residue was purified by silica gel column chromatography (PE/EtOAc (v/v) = 8/1) to give the title compound (1.05 g, 80%). The compound was characterized by the following spectroscopic data:
MS (ESI, pos.ion) *m*/*z:* 343.5 [M+H]⁺;
¹H NMR (400 MHz, CDCl₃) *δ* (ppm): 7.33, 7.31 (dd, dd, 1H), 6.39, 6.37 (s, s, 1H), 4.81 (brs, 1H), 2.83-2.79 (m, 2H), 2.26-2.17 (m, 4H), 1.94-1.86 (m, 2H), 1.38-1.21 (m, 3H), 1.18-1.09 (m, 2H), 0.91-0.88 (m, 3H).

### Step 5) the preparation of compound 44-5

To a solution of compound **44-4** (1.04 g, 3.04 mmol) in DCM (15 mL) was added pyridine (1.95 mL, 24.32 mmol) dropwise at 0 °C. After the mixture was stirred for 10 mins, trifluoromethanesulfonic anhydride (2.04 mL, 12.16 mmol) was added, and the mixture was stirred at rt for 1.0 hr. After the reaction was completed, the mixture was quenched with ice water (50 mL). The aqueous layer was extracted with EtOAc (30 mL x 3). The combined organic layers were washed with brine, dried over anhydrous Na₂SO₄ and concentrated *in vacuo.* The residue was purified by silica gel column chromatography (PE) to give the title compound as yellow oil (1.296 g, 90%). The compound was characterized by the following spectroscopic data:
¹H NMR (400 MHz, CDCl₃) *δ* (ppm): 7.54, 7.52 (d, d, 1H), 6.86, 6.84 (s, s, 1H), 3.02-2.98 (m, 2H), 2.32-2.24 (m, 2H), 2.21-2.16 (m, 2H), 1.90-1.82 (m, 2H), 1.38-1.20 (m, 3H), 1.18-1.09 (m, 2H), 0.91-0.88 (m, 3H).

### Step 6) the preparation of compound 44-6

A suspension of compound **44-5** (2.133 g, 4.5 mmol), compound **41-12** (2.26 g, 4.5 mmol), Pd(PPh₃)₄ (260 mg, 0.225 mmol) and K₂CO₃ (1.24 g, 9.0 mmol) in mixed solvents of DME and H₂O (24 mL, v/v = 5/1) was stirred at 90 °C under N₂ for 2.0 hrs. After the reaction was completed, the mixture was diluted with EtOAc (50 mL). The organic layers were washed with water (20 mL x 3) and brine, dried over anhydrous Na₂SO₄ and concentrated *in vacuo.* The residue was purified by silica gel column chromatography (PE/EtOAc (v/v) = 1/2) to give the title compound as a white solid (2.275 g, 70%). The compound was characterized by the following spectroscopic data:
MS (ESI, pos.ion) *m*/*z:* 723.5 [M+H]⁺;
¹H NMR (400 MHz, CDCl₃) *δ* (ppm): 7.46, 7.43 (d, d, 1H), 7.41 (s, 1H), 7.29, 7.27 (s, s, 1H), 7.12, 7.11 (d, d, 1H), 6.89, 6.88 (s, s, 1H), 5.56, 5.55 (d, d, 1H), 5.42-5.37 (m, 1H), 4.34-4.30 (m, 1H), 3.85-3.78 (m, 1H), 3.66 (s, 3H), 3.65-3.61 (m, 1H), 2.95-2.91 (m, 2H), 2.39-2.31 (m, 2H), 2.30-1.92 (m, 7H), 1.85-1.76 (m, 2H), 1.44-1.29 (m, 1H), 1.29-1.19 (m, 2H), 1.05-0.96 (m, 5H), 0.94, 0.91 (m, m, 3H), 0.90, 0.89 (m, m, 3H).

### Step 7) the preparation of compound 44-7

A mixture of compound **44-6** (1.66 g, 2.3 mmol), compound **1-12-2** (0.64 g, 2.53 mmol), Pd(dppf)Cl₂CH₂Cl₂ (90 mg, 0.115 mmol) and KOAc (0.6 g, 5.75 mmol) in DMF (15 mL) was stirred at 120 °C under N₂ for 4.0 hrs. After the reaction was completed, the mixture was diluted with EtOAc (100 mL) and filtered through a celite pad. The filtrate was washed with water (50 mL x 3) and brine, dried over anhydrous Na₂SO₄ and concentrated *in vacuo.* The residue was purified by silica gel column chromatography (PE/EtOAc (v/v) = 1/2) to give the title compound as a white solid (1.127 g, 70%). The compound was characterized by the following spectroscopic data:
MS (ESI, pos.ion) *m*/*z:* 701.5 [M+H]⁺;
¹H NMR (400 MHz, CDCl₃) *δ* (ppm): 7.66, 7.64 (dd, dd, 1H), 7.51, 7.49 (s, s, 1H), 7.41 (s, 1H), 7.13, 7.11 (s, s, 1H), 6.94, 6.93 (s, s, 1H), 5.56, 5.55 (d, d, 1H), 5.42-5.37 (m, 1H), 4.34-4.30 (m, 1H), 3.85-3.78 (m, 1H), 3.66 (s, 3H), 3.65-3.61 (m, 1H), 2.96-2.92 (m, 2H), 2.34-1.92 (m, 9H), 1.85-1.77 (m, 2H), 1.42-1.33 (m, 1H), 1.32 (t, 6H), 1.29 (t, 6H), 1.26-1.20 (m, 5H), 1.06-0.97 (m, 2H), 0.94, 0.91 (m, m, 3H), 0.90, 0.89 (m, m, 3H).

### Step 8) the preparation of compound 44-8

A suspension of compound **44-7** (385.2 mg, 0.55 mmol), compound **7-2** (231 mg, 0.55 mmol), Pd(PPh₃)₄ (32 mg, 0.027 mmol) and K₂CO₃ (190 mg, 1.37 mmol) in mixed solvents of EtOH and H₂O (7.5 mL, v/v = 4/1) was stirred at 90 °C under N₂ for 2.0 hrs. After the reaction was completed, the mixture was diluted with EtOAc (50 mL). The organic layer was washed with brine, dried over anhydrous Na₂SO₄ and concentrated *in vacuo.* The residue was purified by silica gel column chromatography (DCM/MeOH (v/v) = 50/1) to give the title compound as a white solid (238.27 mg, 50%). The compound was characterized by the following spectroscopic data:
MS (ESI, pos.ion) *m*/*z*: 434.5 [M+2H]²⁺;
¹H NMR (400 MHz, CDCl₃) *δ* (ppm): 7.71 (s, 1H), 7.62, 7.60 (s, s, 1H), 7.41 (s, 1H), 7.30, 7.28 (dd, dd, 1H), 7.12, 7.11 (d, d, 1H), 6.93, 6.92 (s, s, 1H), 5.56, 5.55 (d, d, 1H), 5.46, 5.44 (d, d, 1H), 5.42-5.37 (m, 1H), 5.32-5.28 (m, 1H), 4.41-4.30 (m, 2H), 3.85-3.78 (m, 2H), 3.66 (s, 6H), 3.65-3.61 (m, 2H), 2.76-2.72 (m, 2H), 2.39-1.92 (m, 16H), 1.44-1.29 (m, 1H), 1.25-1.19 (m, 2H), 1.05-0.88 (m, 17H).

### Example 45

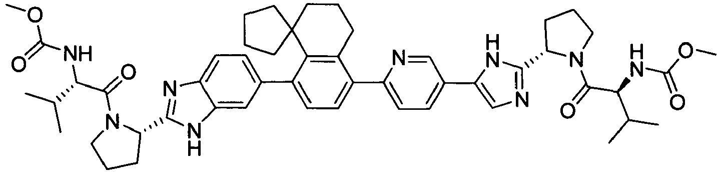

### Synthetic route:

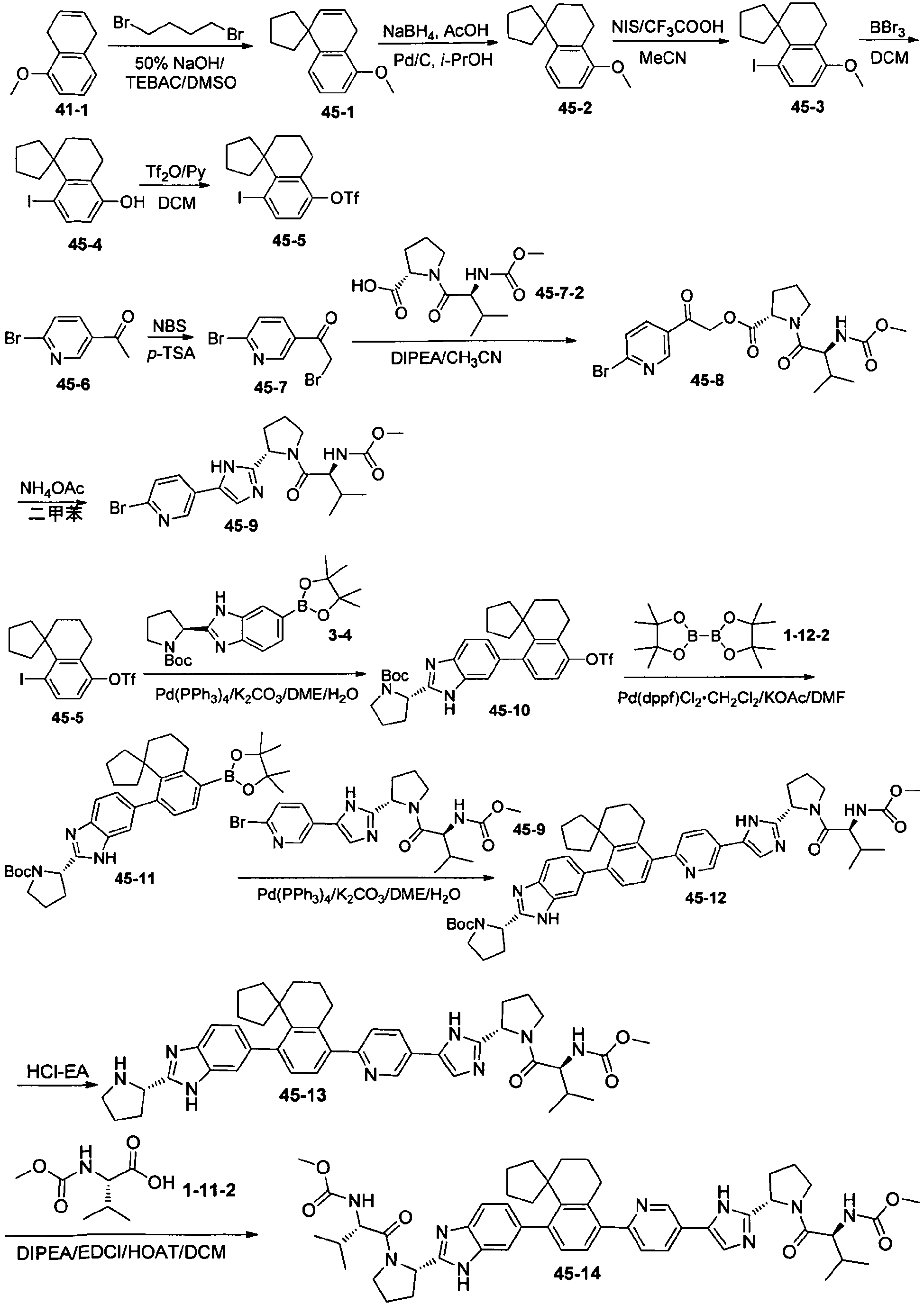

### Step 1) the preparation of compound 45-1

To a solution of compound **41-1** (1.091 g, 6.82 mmol), 1,4-dibromobutane (1.529 g, 7.15 mmol) and TEBAC (0.3 g, 1.36 mmol) in DMSO (20 mL) was added sodium hydroxide aqueous solution (50%, 2.0 mL) at 0 °C. At the end of the addition, the mixture was stirred at 50 °C for 2.0 hrs. After the reaction was completed, the mixture was quenched with water (80 mL). The aqueous layer was extracted with EtOAc (50 mL x 3). The combined organic layers were washed with brine, dried over anhydrous Na₂SO₄ and concentrated *in vacuo.* The residue was purified by silica gel column chromatography (PE) to give the title compound as yellow oil (1.095 g, 75%). The compound was characterized by the following spectroscopic data:
MS (ESI, pos.ion) *m*/*z:* 215.5 [M+H]⁺;
¹H NMR (400 MHz, CDCl₃) *δ* (ppm): 6.97-6.94 (m, 1H), 6.64-6.59 (m, 2H), 6.13-6.09 (m, 1H), 5.72-5.67 (m, 1H), 3.73 (s, 3H), 3.47-3.45 (m, 2H), 2.15-2.05 (m, 2H), 1.82-1.62 (m, 4H), 1.49-1.39 (m, 2H).

### Step 2) the preparation of compound 45-2

To a solution of compound **45-1** (1.0 g, 4.69 mmol) in isopropanol (15 mL) were added a catalytic amount of Pd/C (0.1 g), acetic acid (0.56 g, 9.38 mmol) and Na BH₄ (0.7 g, 18.76 mmol) separately. At the end of the addition, the mixture was stirred at rt for 1.0 hr. After the reaction was completed, the mixture was filtered through a celite pad. The filtrate was concentrated *in vacuo.* The residue was purified by silica gel column chromatography (PE) to give the title compound as yellow oil (760.3 mg, 75%). The compound was characterized by the following spectroscopic data:
MS (ESI, pos.ion) *m*/*z*: 217.5 [M+H]⁺;
¹H NMR (400 MHz, CDCl₃) *δ* (ppm): 6.95-6.91 (m, 1H), 6.84-6.81 (m, 1H), 6.61-6.59 (m, 1H), 3.81 (s, 3H), 2.79-2.69 (m, 2H), 2.29-2.17 (m, 2H), 2.15-2.04 (m, 3H), 1.96-1.79 (m, 2H), 1.74-1.57 (m, 4H), 1.52-1.43 (m, 1H).

### Step 3) the preparation of compound 45-3

To a solution of compound **45-2** (566.31 mg, 2.62 mmol) and NIS (650 mg, 2.88 mmol) in MeCN (6.0 mL) was added CF₃COOH (0.059 mL, 0.79 mmol) dropwise at 0 °C. At the end of the addition, the mixture was stirred at 45 °C overnight. After the reaction was completed, the mixture was concentrated *in vacuo.* To the residue was added water (20 mL), and the mixture was extracted with EtOAc (20 mL x 3). The combined organic layers were washed with saturated sodium sulfite aqueous solution (10 mL x 2), dried over anhydrous Na₂SO₄ and concentrated *in vacuo.* The residue was purified by silica gel column chromatography (PE) to give the title compound as colorless liquid (716.9 mg, 80%). The compound was characterized by the following spectroscopic data:
MS (ESI, pos.ion) *m*/*z:* 343.5 [M+H]⁺;
¹H NMR (400 MHz, CDCl₃) *δ* (ppm): 7.44, 7.42 (m, m, 1H), 6.51, 6.49 (m, m, 1H), 3.81 (s, 3H), 2.72-2.61 (m, 2H), 2.39-2.27 (m, 2H), 2.26-2.13 (m, 3H), 1.97-1.80 (m, 2H), 1.74-1.52 (m, 5H).

### Step 4) the preparation of compound 45-4

To a solution of compound **45-3** (1.313 g, 3.84 mmol) in DCM (15 mL) was added boron tribromide (2.2 mL, 23.04 mmol) dropwise at -78 °C. At the end of the addition, the mixture was stirred at -78 °C for 10 mins and at rt for another 1.0 hr. After the reaction was completed, the mixture was quenched with ice water (20 mL) and the organic phase was separated. The aqueous layer was extracted with EtOAc (20 mL x 3). The combined organic layers were washed with brine, dried over anhydrous Na₂SO₄ and concentrated *in vacuo.* The residue was purified by silica gel column chromatography (PE/EtOAc (v/v) = 8/1) to give the title compound as a white solid (1.0 g, 80%). The compound was characterized by the following spectroscopic data:
MS (ESI, pos.ion) *m*/*z*: 329.5 [M+H]⁺;
¹H NMR (400 MHz, CDCl₃) *δ* (ppm): 7.32, 7.30 (m, m, 1H), 6.42, 6.40 (m, m, 1H), 4.81 (brs, 1H), 2.70-2.59 (m, 2H), 2.43-2.31 (m, 2H), 2.29-2.14 (m, 3H), 1.97-1.80 (m, 2H), 1.74-1.52 (m, 5H).

### Step 5) the preparation of compound 45-5

To a solution of compound **45-4** (997.21 mg, 3.04 mmol) in DCM (15 mL) was added pyridine (1.95 mL, 24.32 mmol) dropwise at 0 °C. After the mixture was stirred for 10 mins, trifluoromethanesulfonic anhydride (2.04 mL, 12.16 mmol) was added slowly, and the mixture was stirred at rt for 2.0 hrs. After the reaction was completed, the mixture was quenched with ice water (30 mL). The aqueous layer was extracted with EtOAc (30 mL x 3). The combined organic layers were washed with brine, dried over anhydrous Na₂SO₄ and concentrated *in vacuo.* The residue was purified by silica gel column chromatography (PE) to give the title compound as yellow oil (1.258 g, 90%). The compound was characterized by the following spectroscopic data:
¹H NMR (400 MHz, CDCl₃) *δ* (ppm): 7.53, 7.51 (t, t, 1H), 6.95, 6.93 (t, t, 1H), 2.72-2.61 (m, 2H), 2.39-2.14 (m, 5H), 2.08-1.91 (m, 2H), 1.74-1.56 (m, 5H).

### Step 6) the preparation of compound 45-7

A mixture of compound **45-6** (25 g, 125.6 mmol), NBS (24.5 g, 138.2 mmol) and *p*-TSA (3.4 g, 20.9 mmol) was stirred at 100 °C under N₂ for 2.0 hrs. After the reaction was completed, the mixture was cooled to rt, and diluted with DCM (200 mL). The resulting mixture was washed with water (50 mL x 3) and brine, dried over anhydrous Na₂SO₄ and concentrated *in vacuo.* The residue was purified by silica gel column chromatography (PE/EtOAc (v/v) = 5/1) to give the title compound as yellow slurry (25 g, 71%). The compound was characterized by the following spectroscopic data:
MS (ESI, pos.ion) *m*/*z*: 279.9 [M+H]⁺;
¹H NMR (400 MHz, CDCl₃) *δ* (ppm): 8.95 (d, 1H, *J* = 1.12 Hz), 8.14-8.11 (m, 1H), 7.68-7.66 (m, 1H), 4.41 (s, 2H).

### Step 7) the preparation of compound 45-8

To a solution of compound **45-7** (5.0 g, 17.9 mmol) and compound **45-7-2** (5.4 g, 19.7 mmol) in MeCN (100 mL) was added DIPEA (3.3 mL, 19.7 mmol) dropwise at 0 °C. At the end of the addition, the mixture was stirred at rt for 3.0 hrs. After the reaction was completed, the mixture was concentrated *in vacuo* and to the residue was added water (100 mL). The resulting mixture was extracted with EtOAc (100 mL x 3). The combined organic layers were dried over anhydrous Na₂SO₄ and concentrated *in vacuo.* The residue was purified by silica gel column chromatography (PE/EtOAc (v/v) = 3/1) to give the title compound (8.06 g, 96%). The compound was characterized by the following spectroscopic data:
MS (ESI, pos.ion) *m*/*z:* 470.1 [M+H]⁺;
¹H NMR (400 MHz, CDCl₃) *δ* (ppm): 8.88 (s, 1H), 8.04 (d, 1H, *J* = 3.88 Hz), 7.65 (d, 1H, *J* = 4.16 Hz), 5.61-5.59 (m, 1H), 5.48 (d, 1H, *J* = 8.32 Hz), 5.23 (d, 1H, *J* = 8.3 Hz), 4.67 (t, 1H, *J* = 5.72 Hz), 4.31 (t, 1H, *J* = 7.52 Hz), 3.86-3.84 (m, 1H), 3.73-3.71 (m, 1H), 3.66 (s, 3H), 2.34-2.15 (m, 4H), 1.01 (t, 3H), 0.94-0.93 (m, 3H), 0.88-0.85 (m, 1H).

### Step 8) the preparation of compound 45-9

A mixture of compound **45-8** (2.0 g, 4.25 mmol) and ammonium acetate (4.9 g, 83 mmol) in xylene (50 mL) was stirred at 130 °C for 5.0 hrs. After the reaction was completed, the mixture was cooled to rt and quenched with water (50 mL). The aqueous layer was extracted with EtOAc (50 mL x 3). The combined organic layers were dried over anhydrous Na₂SO₄ and concentrated *in vacuo.* The residue was purified by silica gel column chromatography (PE/EtOAc (v/v) = 2/1) to give the title compound (1.39 g, 73%). The compound was characterized by the following spectroscopic data:
MS (ESI, pos.ion) *m*/*z:* 450.1 [M+H]⁺;
¹H NMR (400 MHz, CDCl₃) *δ* (ppm): 8.70 (s, 1H), 7.93 (d, 1H, *J* = 6.92 Hz), 7.45 (d, 1H*, J =* 8.28 Hz), 5.41 (d, 1H, *J* = 4.6 Hz), 5.24-5.22 (m, 1H), 4.32 (m, 1H), 3.85-3.83 (m, 1H), 3.67 (s, 3H), 3.63-3.62 (m, 3H), 3.05-3.03 (m, 1H), 2.31-1.93 (m, 4H), 1.04-1.03 (m, 1H), 0.88 (s, 3H), 0.86 (s, 3H).

### Step 9) the preparation of compound 45-10

To a mixture of compound **45-5** (331 mg, 0.72 mmol), compound **3-4** (297.54 mg, 0.72 mmol), Pd(PPh₃)₄ (83 mg, 0.07 mmol) and K₂CO₃ (300 mg, 2.12 mmol) were added DME (4.0 mL) and water (1.0 mL) via syringe under N₂. The mixture was stirred at 90°C for 4.0 hrs. After the reaction was completed, the mixture was cooled to rt, and quenched with water (10 mL). The aqueous layer was extracted with EtOAc (20 mL x 3). The combined organic layers were washed with brine, dried over anhydrous Na₂SO₄ and concentrated *in vacuo.* The residue was purified by silica gel column chromatography (PE/EtOAc (v/v) = 2/1) to give the title compound (356.67 mg, 80%). The compound was characterized by the following spectroscopic data:
MS (ESI, pos.ion) *m*/*z*: 620.3 [M+H]⁺;
¹H NMR (400 MHz, CDCl₃) *δ* (ppm): 7.78, 7.76 (d, d, 1H), 7.58-7.57 (m, 1H), 7.55-7.50 (m, 2H), 7.46, 7.44 (m, m, 1H), 5.04-4.99 (m, 1H), 3.82-3.76 (m, 1H), 3.64-3.57 (m, 1H), 2.81-2.70 (m, 2H), 2.63-2.53 (m, 1H), 2.47-2.10 (m, 7H), 2.09-1.91 (m, 3H), 1.75-1.58 (m, 5H), 1.41 (s, 9H).

### Step 10) the preparation of compound 45-11

A mixture of compound **45-10** (563.5 mg, 0.91 mmol), compound **1-12-2** (463 mg, 1.82 mmol), Pd(dppf)Cl₂·CH₂Cl₂ (71 mg, 0.09 mmol) and KOAc (268 mg, 2.73 mmol) in DMF (10.0 mL) was stirred at 90 °C under N₂ for 3.0 hrs. After the reaction was completed, the mixture was cooled to rt, diluted with EtOAc (60 mL) and filtered through a celite pad. The filtrate was washed with water (30 mL x 3) and brine, dried over anhydrous Na₂SO₄ and concentrated *in vacuo.* The residue was purified by silica gel column chromatography (PE/EtOAc (v/v) = 1/2) to give the title compound (462 mg, 85%). The compound was characterized by the following spectroscopic data:
MS (ESI, pos.ion) *m*/*z:* 598.5 [M+H]⁺;
¹H NMR (400 MHz, CDCl₃) *δ* (ppm): 7.78, 7.76 (t, t, 1H), 7.65-7.63 (m, 2H), 7.54-7.49 (m, 2H), 5.04-4.99 (m, 1H), 3.82-3.76 (m, 1H), 3.64-3.57 (m, 1H), 2.91-2.80 (m, 2H), 2.63-2.53 (m, 1H), 2.47-2.10 (m, 7H), 2.04-1.85 (m, 3H), 1.76-1.54 (m, 5H), 1.41 (s, 9H), 1.32, 1.29 (m, m, 12H).

### Step 11) the preparation of compound 45-12

A solution of compound **45-11** (364.39 mg, 0.61 mmol), compound **45-9** (274 mg, 0.61 mmol), Pd(PPh₃)₄ (70 mg, 0.05 mmol) and K₂CO₃ (254 mg, 1.83 mmol) in mixed solvents of DME and H₂O (6.0 mL, v/v = 5/1) was stirred at 90 °C under N₂ for 4.0 hrs. After the reaction was completed, the mixture was concentrated *in vacuo,* and the residue was dissolved in EtOAc (50 mL). The resulting mixture was washed with brine, dried over anhydrous Na₂SO₄ and concentrated *in vacuo.* The residue was purified by silica gel column chromatography (PE/EtOAc (v/v) = 1/1) to give the title compound (461.42 mg, 90%). The compound was characterized by the following spectroscopic data:
MS (ESI, pos.ion) *mlz:* 841.5 [M+H]⁺;
¹H NMR (400 MHz, CDCl₃) *δ* (ppm): 8.59 (m, 1H), 8.15, 8.13 (m, m, 1H), 7.80-7.78 (d, d, 1H), 7.67 (s, 1H), 7.61-7.60 (m, 1H), 7.59, 7.57 (d, d, 1H), 7.55, 7.53 (d, d, 1H), 7.52, 7.50 (d, d, 1H), 7.33, 7.30 (t, t, 1H), 5.38-5.33 (m, 1H), 5.32, 5.29 (d, d, 1H), 5.04-4.99 (m, 1H), 4.41-4.36 (m, 1H), 3.85-3.76 (m, 2H), 3.69-3.66 (m, 1H), 3.63 (s, 3H), 3.62-3.57 (m, 1H), 2.67-2.52 (m, 3H), 2.47-1.92 (m, 15H), 1.74-1.57 (m, 5H), 1.41 (s, 9H), 0.97, 0.95 (m, m, 3H), 0.91, 0.89 (m, m, 3H).

### Step 12) the preparation of compound 45-13

To a solution of compound **45-12** (672 mg, 0.8 mmol) in EtOAc (10.0 mL) was added a solution of HCl in EtOAc (5.0 mL, 4 M) dropwise. At the end of the addition, the mixture was stirred at rt for 8.0 hrs. After the reaction was completed, the mixture was concentrated *in vacuo.* The residue was purified by beating in EtOAc (10 mL) and filtered to give the title compound as a pale yellow solid (562.72 mg, 95%). The compound was characterized by the following spectroscopic data:
MS (ESI, pos.ion) *m*/*z*: 741.5 [M+H]⁺.

### Step 13) the preparation of compound 45-14

To a suspension of compound **45-13** (49.6 mg, 0.067 mmol), compound **1-11-2** (20.3 mg, 0.116 mmol), HOAT (18.24 mg, 0.134 mmol) and EDCI (30 mg, 0.154 mmol) in DCM (1.0 mL) was added DIPEA (0.09 mL, 0.539 mmol) dropwise at 0 °C. At the end of the addition, the mixture was stirred at rt for 3.0 hrs. After the reaction was completed, the mixture was diluted with DCM (20 mL), washed with NH₄Cl aqueous solution and brine, dried over anhydrous Na₂SO₄ and concentrated *in vacuo.* The residue was purified by silica gel column chromatography (EtOAc) to give the title compound (30 mg, 50%). The compound was characterized by the following spectroscopic data:
MS (ESI, pos.ion) *m*/*z*: 449.7 [M+2H]²⁺;
¹H NMR (400 MHz, CDCl₃) *δ* (ppm): 8.59 (m, 1H), 8.15, 8.13 (m, m, 1H), 7.80-7.78 (d, d, 1H), 7.67 (s, 1H), 7.61-7.60 (m, 1H), 7.59, 7.57 (d, d, 1H), 7.55, 7.53 (d, d, 1H), 7.52, 7.50 (d, d, 1H), 7.33, 7.30 (m, m, 1H), 5.38-5.33 (m, 1H), 5.32, 5.29 (d, d, 1H), 5.24-5.20 (m, 1H), 4.41-4.35 (m, 2H), 3.85-3.78 (m, 2H), 3.69-3.64 (m, 1H), 3.63 (s, 6H), 3.62-3.60 (m, 1H), 2.67-2.51 (m, 2H), 2.39-1.89 (m, 17H), 1.74-1.57 (m, 5H), 0.97, 0.95 (m, m, 6H), 0.90, 0.89 (m, m, 6H).

### Example 46

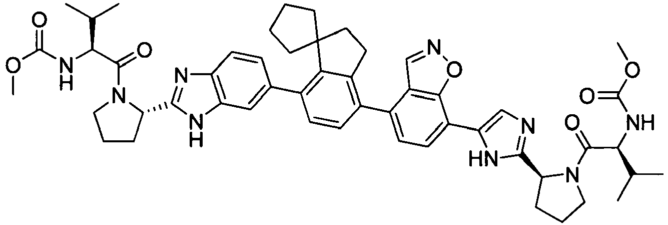

### Synthetic route:

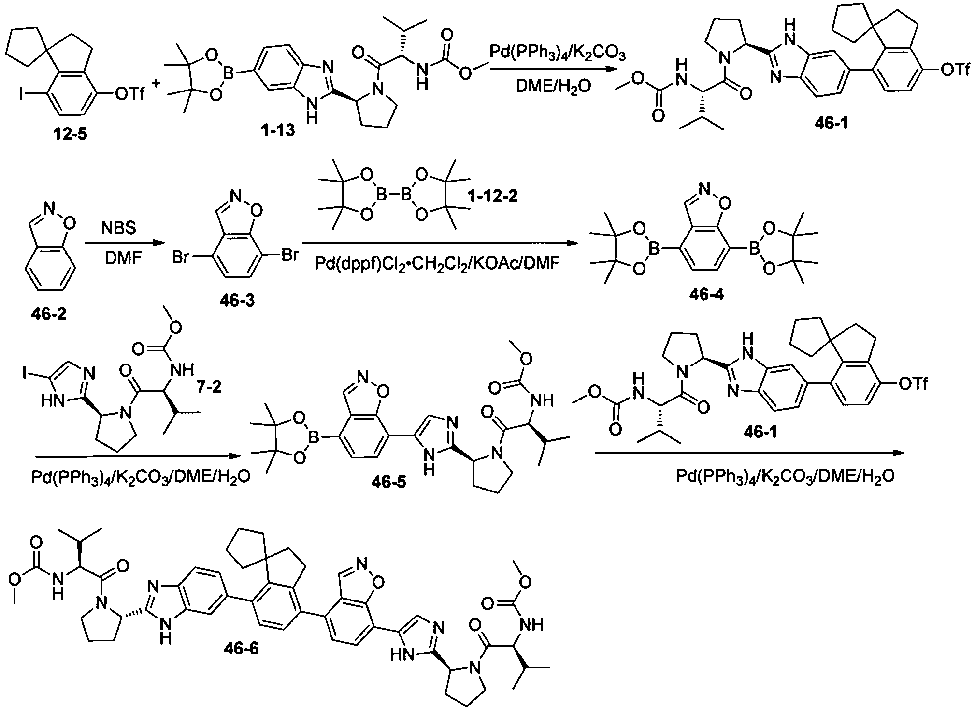

### Step 1) the preparation of compound 46-1

A mixture of compound **12-5** (321.12 mg, 0.72 mmol), compound **1-13** (338.59 mg, 0.72 mmol), Pd(PPh₃)₄ (83 mg, 0.07 mmol) and K₂CO₃ (300 mg, 2.12 mmol) in mixed solvents of DME and H₂O (5.0 mL, v/v = 4/1) was stirred at 90 °C under N₂ for 4.0 hrs. After the reaction was completed, the mixture was quenched with water (10 mL). The aqueous layer was extracted with EtOAc (20 mL x 3). The combined organic layers were washed with brine, dried over anhydrous Na₂SO₄ and concentrated *in vacuo.* The residue was purified by silica gel column chromatography (PE/EtOAc (v/v) = 1/1) to give the title compound (381.45 mg, 80%). The compound was characterized by the following spectroscopic data:
MS (ESI, pos.ion) *m*/*z*: 663.5 [M+H]⁺;
¹H NMR (400 MHz, CDCl₃) *δ* (ppm): 7.87, 7.85 (d, d, 1H), 7.67, 7.65 (dd, dd, 1H), 7.62 (m, 1H), 7.50, 7.48 (d, d, 1H), 7.39, 7.37 (s, s, 1H), 5.32, 5.29 (d, d, 1H), 5.24-5.20 (m, 1H), 4.40-4.35 (m, 1H), 3.84-3.78 (m, 1H), 3.68-3.64 (m, 1H), 3.63 (s, 3H), 2.99-2.95 (m, 2H), 2.42-2.28 (m, 3H), 2.26-2.10 (m, 5H), 2.01-1.87 (m, 3H), 1.76-1.59 (m, 4H), 0.97, 0.95 (m, 3H), 0.91, 0.89 (m, 3H).

### Step 2) the preparation of compound 46-3

To a solution of NBS (2.16 g, 12 mmol) in DMF (6.0 mL) was added a solution of compound **46-2** (714.24 mg, 6.0 mmol) in DMF (6.0 mL) dropwise in the dark at -15 °C. At the end of the addition, the mixture was stirred at rt for 1.0 hr and at 60 °C for another 5.0 hrs. After the reaction was completed, the mixture was poured into the mixed solvents of ice water (50.0 mL) and ethyl ether (60.0 mL). The organic layer was separated, washed several times with water to neutral pH, dried over anhydrous Na₂SO₄ and concentrated *in vacuo* to give the title compound (1.072 g, 65%). The compound was characterized by the following spectroscopic data:
MS (ESI, pos.ion) *m*/*z:* 275.5 [M+H]⁺;
¹H NMR (400 MHz, CDCl₃) *δ* (ppm): 9.02 (d, 1H), 7.53, 7.51 (s, s, 1H), 7.34, 7.32 (d, d, 1H).

### Step 3) the preparation of compound 46-4

A mixture of compound **46-3** (250.12 mg, 0.91 mmol), compound **1-12-2** (531.62 mg, 2.093 mmol), Pd(dppf)Cl₂CH₂Cl₂ (71 mg, 0.09 mmol) and KOAc (268 mg, 2.73 mmol) in DMF (10.0 mL) was stirred at 90 °C under N₂ for 3.0 hrs. After cooling to room temperature, the mixture was diluted with EtOAc (60.0 mL) and filtered through a celite pad. The filtration was washed with water (30 mL x 3) and brine, dried over anhydrous Na₂SO₄ and concentrated *in vacuo.* The residue was purified by silica gel column chromatography (PE/EtOAc (v/v) = 5/2) to give the title compound (270.24 mg, 80%). The compound was characterized by the following spectroscopic data:
MS (ESI, pos.ion) *m*/*z*: 372.5 [M+H]⁺;
¹H NMR (400 MHz, CDCl₃) *δ* (ppm): 9.53 (d, 1H), 8.53, 8.51 (d, d, 1H), 7.94, 7.92 (d, d, 1H), 1.56, 1.54 (m, m, 12H), 1.32, 1.29 (m, m, 12H).

### Step 4) the preparation of compound 46-5

To a mixture of compound **46-4** (267.27 mg, 0.72 mmol), compound **7-2** (302.45 mg, 0.72 mmol), Pd(PPh₃)₄ (83 mg, 0.07 mmol) and K₂CO₃ (300 mg, 2.12 mmol) were added DME (4.0 mL) and water (1.0 mL) via syringe under N₂, and the mixture was stirred at 90 °C for 4.0 hrs. After the reaction was completed, the mixture was cooled to rt and quenched with water (10.0 mL). The aqueous layer was extracted with EtOAc (20.0 mL x 3). The combined organic layers were washed with brine, dried over anhydrous Na₂SO₄ and concentrated *in vacuo.* The residue was purified by silica gel column chromatography (PE/EtOAc (v/v) = 1/1) to give the title compound (232.1 mg, 60%). The compound was characterized by the following spectroscopic data:
MS (ESI, pos.ion) *m*/*z:* 538.5 [M+H]⁺;
¹H NMR (400 MHz, CDCl₃) *δ* (ppm): 9.37 (d, 1H), 7.66 (s, 1H), 7.54, 7.52 (d, d, 1H), 7.26, 7.24 (s, s, 1H), 5.38-5.34 (m, 1H), 5.32, 5.29 (d, d, 1H), 4.41-4.36 (m, 1H), 3.85-3.78 (m, 1H), 3.68-3.65 (m, 1H), 3.63 (s, 3H), 2.30-1.92 (m, 5H), 1.56, 1.53 (m, m, 12H), 0.97-0.89 (m, 6H).

### Step 5) the preparation of compound 46-6

To a mixture of compound **46-5** (537.28 mg, 1.0 mmol), compound **46-1** (662.24 mg, 1.0 mmol), Pd(PPh₃)₄ (115.6 mg, 0.1 mmol) and K₂CO₃ (345 mg, 2.5 mmol) were added DME (4.0 mL) and water (1.0 mL) via syringe, and the mixture was stirred at 90 °C under N₂ for 4.0 hrs. After the reaction was completed, the mixture was cooled to rt and quenched with water (10.0 mL). The aqueous layer was extracted with EtOAc (20.0 mL x 3). The combined organic layers were washed with brine, dried over anhydrous Na₂SO₄ and concentrated *in vacuo.* The residue was purified by silica gel column chromatography (DCM/MeOH (v/v) = 50/1) to give the title compound (461.7 mg, 50%). The compound was characterized by the following spectroscopic data:
MS (ESI, pos.ion) *m*/*z*: 462.7 [M+2H]²⁺;
¹H NMR (400 MHz, CDCl₃) *δ* (ppm): 8.94 (d, 1H), 7.90, 7.88 (d, d, 1H), 7.87, 7.85 (s, s, 1H), 7.65 (m, 1H), 7.63 (s, 1H), 7.62, 7.59 (d, d, 1H), 7.50, 7.48 (d, d, 1H), 7.38, 7.36 (dd, dd, 1H), 7.30, 7.28 (s, s, 1H), 5.38-5.34 (m, 1H), 5.32, 5.29 (d, d, 2H), 5.25-5.20 (m, 1H), 4.41-4.35 (m, 2H), 3.85-3.77 (m, 2H), 3.69-3.65 (m, 2H), 3.63 (s, 6H), 2.82-2.78 (m, 2H), 2.44-1.87 (m, 16H), 1.76-1.58 (m, 4H), 0.97-0.95 (m, m, 6H), 0.91-0.89 (m, m, 6H).

### Example 47

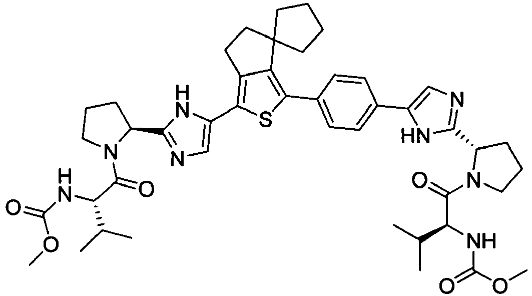

### Synthetic route:

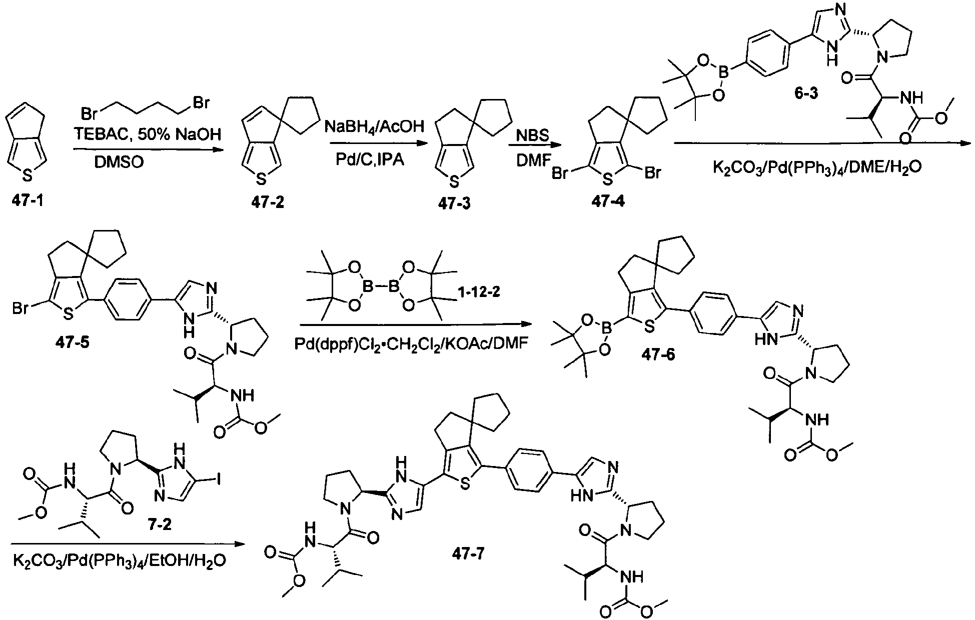

### Step 1) the preparation of compound 47-2

To a solution of compound 47-1 (832 mg, 6.82 mmol), 1,4-dibromobutane (1.529 g, 7.15 mmol) and TEBAC (0.3 g, 1.36 mmol) in DMSO (30 mL) was added sodium hydroxide aqueous solution (50%, 2.0 mL) at 0 °C. At the end of the addition, the mixture was stirred at 50 °C for 2.0 hrs. After the reaction was completed, the mixture was quenched with water (50 mL). The aqueous layer was extracted with EtOAc (50 mL x 3). The combined organic layers were washed with brine, dried over anhydrous Na₂SO₄ and concentrated *in vacuo.* The residue was purified by silica gel column chromatography (PE) to give the title compound as yellow oil (840.55 mg, 70%). The compound was characterized by the following spectroscopic data:
MS (ESI, pos.ion) *m*/*z:* 177.5 [M+H]⁺;
¹H NMR (400 MHz, CDCl₃) *δ* (ppm): 6.91-6.90 (m, 1H), 6.86-6.85 (m, 1H), 6.57-6.55 (m, 1H), 6.44-6.41 (m, 1H), 2.12-2.01 (m, 2H), 1.81-1.60 (m, 4H), 1.46-1.36 (m, 2H).

### Step 2) the preparation of compound 47-3

To a solution of compound **47-2** (825.77 mg, 4.69 mmol) in isopropanol (15 mL) were added a catalytic amount of Pd/C (100 mg), acetic acid (0.56 g, 9.38 mmol) and NaB H₄ (0.7 g, 18.76 mmol) separately. At the end of the addition, the mixture was stirred at rt for 1.0 hr. After the reaction was completed, the mixture was filtered through a celite pad. The filtrate was concentrated *in vacuo.* The residue was purified by silica gel column chromatography (PE) to give the title compound as yellow oil (626.39 mg, 75%). The compound was characterized by the following spectroscopic data:
MS (ESI, pos.ion) *mlz:* 179.5 [M+H]⁺;
¹H NMR (400 MHz, CDCl₃) *δ* (ppm): 6.81-6.79 (m, 2H), 2.84-2.79 (m, 2H), 2.23-2.15 (m, 2H), 2.07-2.01 (m, 2H), 1.80-1.72 (m, 2H), 1.71-1.56 (m, 4H).

### Step 3) the preparation of compound 47-4

To a solution of NBS (2.16 g, 12 mmol) in DMF (6.0 mL) was added a solution of compound 47-3 (1.068 g, 6.0 mmol) in DMF (6.0 mL) dropwise in the dark at -15 °C. At the end of the addition, the mixture was stirred at rt for 0.5 hr and at 60 °C for another 5.0 hrs. After the reaction was completed, the mixture was poured into the mixed solvents of ice water (50.0 mL) and ethyl ether (60.0 mL). The organic layer was separated, washed several times with water to neutral pH, and dried over anhydrous Na₂SO_{4C}oncentrated *in vacuo* to give the title compound (1.602 g, 80%). The compound was characterized by the following spectroscopic data:
MS (ESI, pos.ion) *m*/*z:* 334.9 [M+H]⁺;
¹H NMR (400 MHz, CDCl₃) *δ* (ppm): 2.79-2.75 (m, 2H), 2.32-2.23 (m, 2H), 2.22-2.16 (m, 2H), 1.89-1.81 (m, 2H), 1.79-1.62 (m, 4H).

### Step 4) the preparation of compound 47-5

A mixture of compound **47-4** (611 mg, 1.83 mmol), compound **6-3** (908.21 mg, 1.83 mmol), Pd(PPh₃)₄ (105.8 mg, 0.0915 mmol) and K₂CO₃ (632.31mg, 4.575 mmol) in mixed solvents of DME and H₂O (12 mL, v/v = 5/1) was stirred at 90 °C under N₂ for 4.0 hrs. After the reaction was completed, the mixture was quenched with water (20 mL). The aqueous layer was extracted with EtOAc (20 mL x 3). The combined organic layers were washed with brine, dried over anhydrous Na₂SO₄ and concentrated *in vacuo.* The residue was purified by silica gel column chromatography (PE/EtOAc (v/v) = 1/2) to give the title compound (799.57 mg, 70%). The compound was characterized by the following spectroscopic data:
MS (ESI, pos.ion) *m*/*z*: 625.5 [M+H]⁺;
¹H NMR (400 MHz, CDCl₃) *δ* (ppm): 7.61-7.58 (m, 2H), 7.48-7.45 (m, 2H), 5.32, 5.29 (d, d, 1H), 5.23-5.19 (m, 1H), 4.41-4.37 (m, 1H), 3.85-3.78 (m, 1H), 3.68-3.65 (m, 1H), 3.63 (s, 3H), 2.82-2.78 (m, 2H), 2.34-2.15 (m, 8H), 2.13-2.03 (m, 1H), 2.02-1.94 (m, 1H), 1.91-1.83 (m, 2H), 1.75-1.58 (m, 4H), 0.97, 0.95 (m, m, 3H), 0.91, 0.89 (m, m, 3H).

### Step 5) the preparation of compound 47-6

A mixture of compound **47-5** (792.7 mg, 1.27 mmol), compound **1-12-2** (354 mg, 1.39 mmol), Pd(dppf)Cl₂CH₂Cl₂ (103 mg, 0.13 mmol) and KOAc (311 mg, 3.17 mmol) in DMF (5.0 mL) was stirred at 90 °C under N₂ for 3.0 hrs. After cooling to rt, the mixture was diluted with EtOAc (50.0 mL) and filtered through a celite pad. The filtration was washed with water (20 mL x 3) and brine, dried over anhydrous Na₂SO₄ and concentrated *in vacuo.* The residue was purified by silica gel column chromatography (PE/EtOAc (v/v) = 1/2) to give the title compound (597.72 mg, 70%). The compound was characterized by the following spectroscopic data:
MS (ESI, pos.ion) *m*/*z*: 673.5 [M+H]⁺;
¹H NMR (400 MHz, CDCl₃) *δ* (ppm): 7.60-7.57 (m, 2H), 7.20-7.16 (m, 2H), 5.32, 5.29 (d, d, 1H), 5.23-5.19 (m, 1H), 4.41-4.36 (m, 1H), 3.85-3.78 (m, 1H), 3.68-3.65 (m, 1H), 3.63 (s, 3H), 2.78-2.74 (m, 2H), 2.30-2.15 (m, 8H), 2.13-2.03 (m, 1H), 2.02-1.92 (m, 1H), 1.87-1.78 (m, 2H), 1.75-1.58 (m, 4H), 1.33, 1.30 (m, m, 12H), 0.97, 0.95 (m, m, 3H), 0.90, 0.89 (m, m, 3H).

### Step 6) the preparation of compound 47-7

To a mixture of compound **47-6** (672.68 mg, 1.0 mmol), compound **7-2** (428.47 mg, 1.02 mmol), Pd(PPh₃)₄ (116 mg, 0.10 mmol) and K₂CO₃ (346 mg, 2.5 mmol) were added EtOH (10.0 mL) and water (2.0 mL) via syringe under N₂, and the mixture was stirred at 90 °C for 5.0 hrs. After the reaction was completed, the mixture was cooled to rt and quenched with water (20 mL). The aqueous layer was extracted with EtOAc (20.0 mL x 3). The combined organic layers were washed with brine, dried over anhydrous Na₂SO₄ and concentrated *in vacuo.* The residue was purified by silica gel column chromatography (DCM/MeOH (v/v) = 50/1) to give the title compound (503 mg, 60%). The compound was characterized by the following spectroscopic data:
MS (ESI, pos.ion) *m*/*z*: 839.5 [M+H]⁺;
¹H NMR (400 MHz, CDCl₃) *δ* (ppm): 7.60-7.54 (m, 5H), 7.48 (s, 1H), 5.56, 5.55 (d, d, 1H), 5.44-5.40 (m, 1H), 5.32, 5.29 (d, d, 1H), 5.23-5.19 (m, 1H), 4.41-4.36 (m, 2H), 3.85-3.78 (m, 2H), 3.68-3.67 (m, 1H), 3.66 (s, 3H), 3.65-3.64 (m, 1H), 3.63 (s, 3H), 2.93-2.89 (m, 2H), 2.31-2.15 (m, 10H), 2.14-2.03 (m, 2H), 2.02-1.92 (m, 2H), 1.85-1.77 (m, 2H), 1.73-1.56 (m, 4H), 1.02-0.89 (m, 12 H).

### Example 48

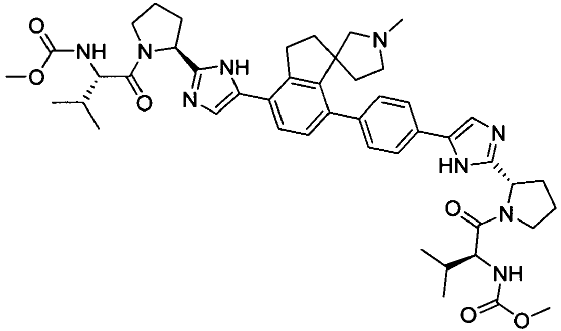

### Synthetic route:

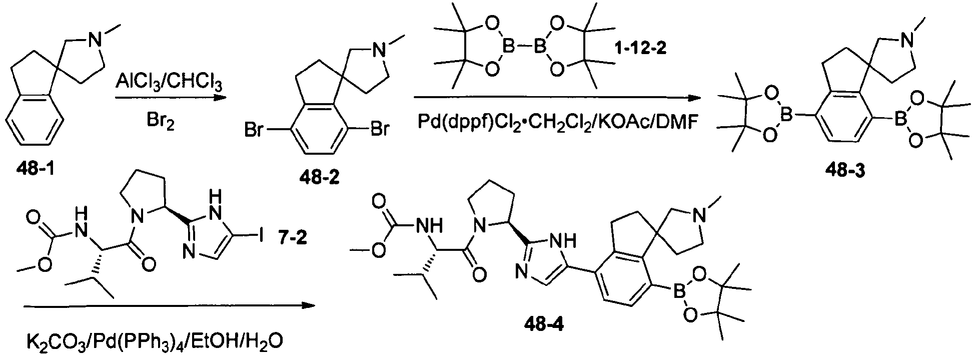

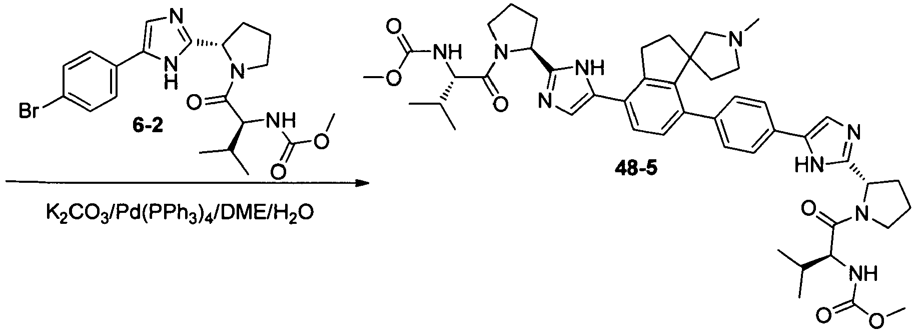

### Step 1) the preparation of compound 48-2

To a suspension of aluminium chloride (3.017 g, 22.6 mmol) in CHCl₃ (30 mL) was added a solution of compound 48-1 (1.87 g, 10 mmol) in CHCl₃ (20 mL) dropwise at 0 °C. After stirring at 0 °C for 1.0 hr, a solution of Br₂ (1.025 mL, 20 mmol) in CHCl₃ (10 mL) was added dropwise. At the end of the addition, the mixture was stirred at rt overnight. After the reaction was completed, the mixture was poured into ice water (100 mL). The aqueous layer was extracted with DCM (50 mL x 3). The combined organic layers were washed with NaHCO₃ aqueous solution and brine, dried over anhydrous Na₂SO₄ and concentrated *in vacuo.* The residue was purified by silica gel column chromatography (PE/EtOAc (v/v) = 6/1) to give the title compound (1.543 g, 45%). The compound was characterized by the following spectroscopic data:
MS (ESI, pos.ion) *mlz:* 343.5 [M+H]⁺;
¹H NMR (400 MHz, CDCl₃) *δ* (ppm): 7.25, 7.23 (s, s, 1H), 7.14, 7.12 (t, t, 1H), 3.06-2.91 (m, 3H), 2.88-2.80 (m, 2H), 2.63-2.56 (m, 1H), 2.43-2.42 (m, 3H), 2.38-2.20 (m, 3H), 1.81-1.73 (m, 1H).

### Step 2) the preparation of compound 48-3

A suspension of compound **48-2** (435.56 mg, 1.27 mmol), compound **1-12-2** (741.94 mg, 2.921 mmol), Pd(dppf)Cl₂CH₂Cl₂ (103 mg, 0.13 mmol) and KOAc (311 mg, 3.17 mmol) in DMF (10.0 mL) was stirred at 90 °C under N₂ for 3.0 hrs. After cooling to rt, the mixture was diluted with EtOAc (50.0 mL) and filtered through a celite pad. The filtration was washed with water (20 mL x 3) and brine, dried over anhydrous Na₂SO₄ and concentrated *in vacuo.* The residue was purified by silica gel column chromatography (PE/EtOAc (v/v) = 7/2) to give the title compound (362.65 mg, 65%). The compound was characterized by the following spectroscopic data:
MS (ESI, pos.ion) *mlz:* 440.5 [M+H]⁺;
¹H NMR (400 MHz, CDCl₃) *δ* (ppm): 8.00-7.98 (t, t, 1H), 7.75, 7.73 (s, s, 1H), 2.94-2.85 (m, 2H), 2.84-2.76 (m, 2H), 2.50-2.21 (m, 8H), 1.72-1.64 (m, 1H), 1.32, 1.29 (m, m, 24H).

### Step 3) the preparation of compound 48-4

A mixture of compound **48-3** (439.31 mg, 1.0 mmol), compound **7-2** (420.07 mg, 1.0 mmol), Pd(PPh₃)₄ (116 mg, 0.10 mmol) and K₂CO₃ (346 mg, 2.5 mmol) in mixed solvents of EtOH (10.0 mL) and water (2.0 mL) was stirred at 90 °C under N₂ for 5.0 hrs. After the reaction was completed, the mixture was cooled to rt and diluted with water (20.0 mL). The aqueous layer was extracted with EtOAc (20.0 mL x 3). The combined organic layers were washed with brine, dried over anhydrous Na₂SO₄ and concentrated *in vacuo.* The residue was purified by silica gel column chromatography (PE/EtOAc (v/v) = 1/2) to give the title compound (363.2 mg, 60%). The compound was characterized by the following spectroscopic data:
MS (ESI, pos.ion) *m*/*z*: 606.4 [M+H]⁺;
¹H NMR (400 MHz, CDCl₃) *δ* (ppm): 7.59, 7.56 (t, t, 1H), 7.54 (s, 1H), 7.34, 7.32 (s, s, 1H), 5.56, 5.55 (d, d, 1H), 5.32-5.28 (m, 1H), 4.34-4.30 (m, 1H), 3.85-3.78 (m, 1H), 3.66 (s, 3H), 3.65-3.61 (m, 1H), 3.01-2.94 (m, 1H), 2.84-2.75 (m, 4H), 2.57-2.50 (m, 1H), 2.42-2.16 (m, 9H), 2.13-1.92 (m, 2H), 1.73-1.65 (m, 1H), 1.32, 1.29 (m, m, 12H), 1.02, 1.00 (m, m, 3H), 0.93, 0.91 (m, m, 3H).

### Step 4) the preparation of compound 48-5

A mixture of compound **48-4** (1.1078 g, 1.83 mmol), compound **6-2** (820 mg, 1.83 mmol), Pd(PPh₃)₄ (105.8 mg, 0.0915 mmol) and K₂CO₃ (632.31mg, 4.575 mmol) in mixed solvents of DME (10.0 mL) and water (2.0 mL) was stirred at 90 °C under N₂ for 4.0 hrs. After the reaction was completed, the mixture was cooled to rt and quenched with water (20.0 mL). The aqueous layer was extracted with EtOAc (20.0 mL x 3). The combined organic layers were washed with brine, dried over anhydrous Na₂SO₄ and concentrated *in vacuo.* The residue was purified by silica gel column chromatography (PE/EtOAc (v/v) = 1/2) to give the title compound (1.086 g, 70%). The compound was characterized by the following spectroscopic data:
MS (ESI, pos.ion) *m*/*z*: 848.5 [M+H]⁺;
¹H NMR (400 MHz, CDCl₃) *δ* (ppm): 7.71 (s, 1H), 7.59 (s, 1H), 7.55-7.51 (m, 3H), 7.50-7.46 (m, 2H), 7.19, 7.17 (s, s, 1H), 5.56, 5.55 (d, d, 1H), 5.32-5.28 (m, 2H), 5.23-5.19 (m, 1H), 4.41-4.30 (m, 2H), 3.85-3.78 (m, 2H), 3.69-3.67 (m, 1H), 3.66 (s, 3H), 3.65-3.64 (m, 1H), 3.63 (s, 3H), 2.96-2.89 (m, 1H), 2.84-2.76 (m, 2H), 2.59-2.46 (m, 2H), 2.41-2.40 (m, 3H), 2.38-2.15 (m, 12H), 2.13-1.92 (m, 2H), 1.76-1.68 (m, 1H), 1.02, 0.89 (m, 12H).

### Example 49

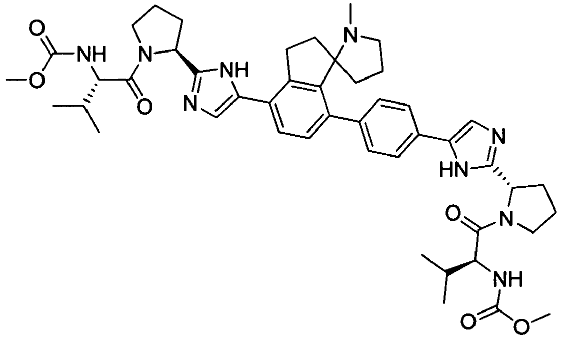

### Synthetic route:

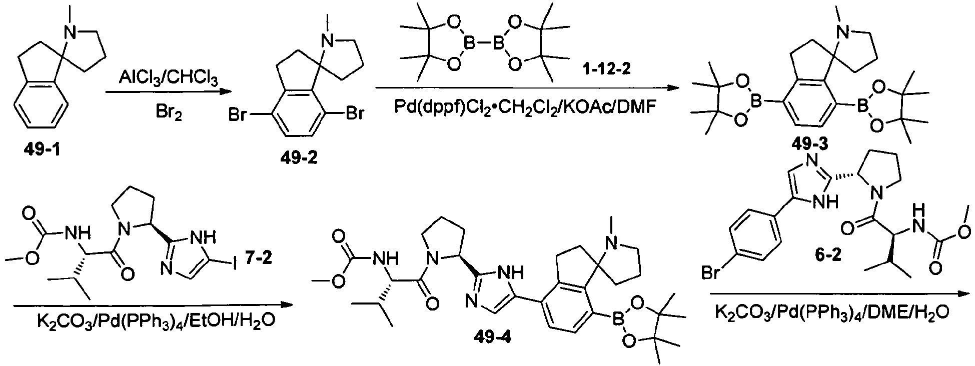

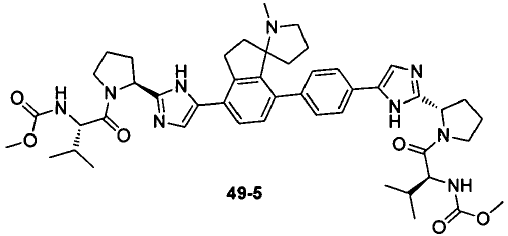

Compounds disclosed herein can be prepared by an analogous procedure to that described in Example 48:

Compound **49-2** was characterized by the following spectroscopic data:
MS (ESI, pos.ion) *mlz:* 343.5 [M+H]⁺;
¹H NMR (400 MHz, CDCl₃) *δ* (ppm): 7.27, 7.25 (m, 1H), 7.11, 7.09 (t, t, 1H), 2.97-2.82 (m, 4H), 2.77-2.70 (m, 1H), 2.47-2.39 (m, 1H), 2.37-2.29 (m, 1H), 2.26-2.25 (m, 3H), 2.06-1.88 (m, 3H).

Compound **49-3** was characterized by the following spectroscopic data:
MS (ESI, pos.ion) *m*/*z:* 440.5 [M+H]⁺;
¹H NMR (400 MHz, CDCl₃) *δ* (ppm): 7.86, 7.84 (t, t, 1H), 7.74, 7.72 (s, s, 1H), 2.92-2.74 (m, 4H), 2.55-2.47 (m, 1H), 2.25-2.15 (m, 5H), 2.01-1.84 (m, 3H), 1.32, 1.29 (m, m, 24H).

Compound **49-4** was characterized by the following spectroscopic data:
MS (ESI, pos.ion) *m*/*z:* 606.5 [M+H]⁺;
¹H NMR (400 MHz, CDCl₃) *δ* (ppm): 7.54-7.52 (m, 2H), 7.25, 7.23 (s, s, 1H), 5.56, 5.55 (d, d, 1H), 5.32-5.28 (m, 1H), 4.34-4.30 (m, 1H), 3.85-3.78 (m, 1H), 3.66 (s, 3H), 3.65-3.61 (m, 1H), 2.99-2.85 (m, 2H), 2.77-2.69 (m, 2H), 2.61-2.54 (m, 2H), 2.31-1.82 (m, 12H), 1.32, 1.29 (m, m, 12H), 1.02, 1.00 (m, m, 3H), 0.94, 0.92 (m, m, 3H).

Compound **49-5** was characterized by the following spectroscopic data:
MS (ESI, pos.ion) *m*/*z:* 848.5 [M+H]⁺;
¹H NMR (400 MHz, CDCl₃) *δ* (ppm): 7.71 (s, 1H), 7.59 (s, 1H), 7.57-7.52 (m, 4H), 7.50-7.46 (m, 2H), 5.56, 5.55 (d, d, 1H), 5.32-5.28 (m, 2H), 5.23-5.19 (m, 1H), 4.41-4.30 (m, 2H), 3.85-3.78 (m, 2H), 3.69-3.67 (m, 1H), 3.66 (s, 3H), 3.65-3.64 (m, 1H), 3.63 (s, 3H), 2.91-2.70 (m, 5H), 2.48-2.40 (m, 2H), 2.36-1.84 (m, 17H), 1.02-0.89 (m, 12H).

### Example 50

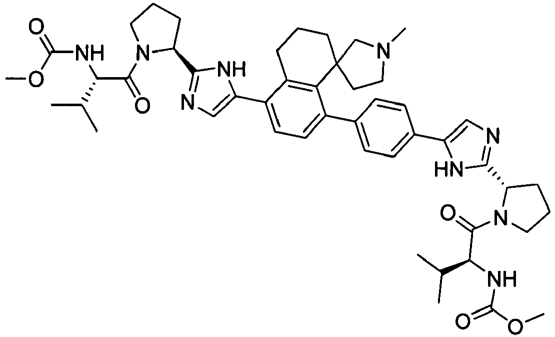

### Synthetic route:

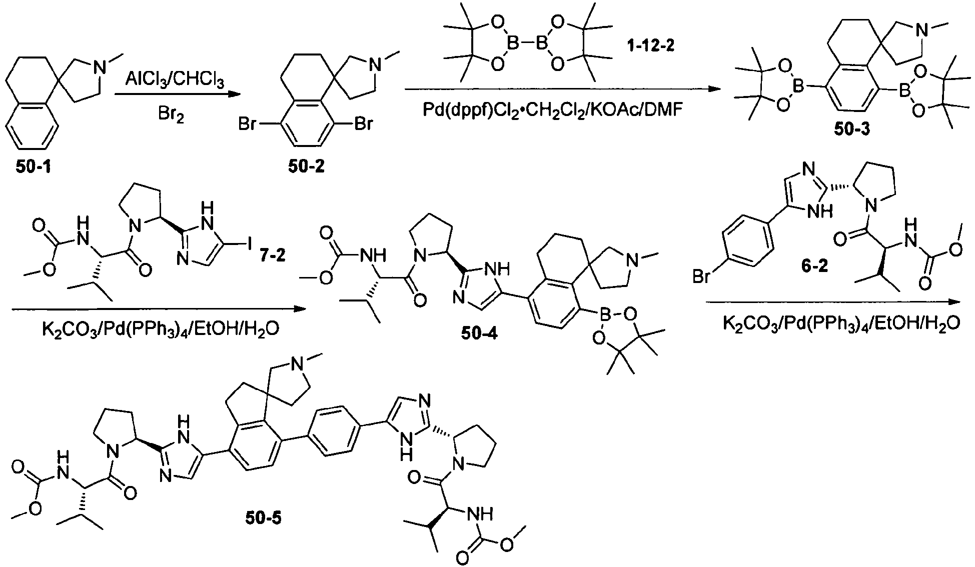

Compounds disclosed herein can be prepared by an analogous procedure to that described in Example 48:

Compound **50-2** was characterized by the following spectroscopic data:
MS (ESI, pos.ion) *mlz:* 357.5 [M+H]⁺;
¹H NMR (400 MHz, CDCl₃) *δ* (ppm): 7.32, 7.30 (m, m, 1H), 7.20, 7.17 (t, t, 1H), 3.12-3.05 (m, 1H), 2.92-2.80 (m, 2H), 2.68-2.54 (m, 3H), 2.42-2.29 (m, 5H), 1.95-1.69 (m, 4H).

Compound **50-3** was characterized by the following spectroscopic data:
MS (ESI, pos.ion) *mlz:* 454.5 [M+H]⁺;
¹H NMR (400 MHz, CDCl₃) *δ* (ppm): 7.97, 7.95 (m, m, 1H), 7.81, 7.79 (t, t, 1H), 3.00-2.93 (m, 1H), 2.83-2.66 (m, 4H), 2.57-2.50 (m, 1H), 2.40-2.39 (m, 3H), 2.36-2.28 (m, 1H), 2.22-2.14 (m, 1H), 1.90-1.71 (m, 3H), 1.61-1.54 (m, 1H), 1.32, 1.29 (m, m, 24H).

Compound **50-4** was characterized by the following spectroscopic data:
MS (ESI, pos.ion) *m*/*z*: 620.5 [M+H]⁺;
¹H NMR (400 MHz, CDCl₃ *δ* (ppm): 7.66, 7.64 (m, 1H), 7.53 (s, 1H), 7.29, 7.27 (t, t, 1H), 5.56, 5.55 (d, d, 1H), 5.32-5.28 (m, 1H), 4.34-4.30 (m, 1H), 3.85-3.78 (m, 1H), 3.66 (s, 3H), 3.65-3.61 (m, 1H), 3.07-3.00 (m, 1H), 2.63-1.59 (m, 19H), 1.32, 1.29 (m, m, 12H), 1.02, 1.00 (m, m, 3H), 0.94-0.91 (m, m, 3H).

Compound **50-5** was characterized by the following spectroscopic data:
MS (ESI, pos.ion) *m*/*z*: 862.5 [M+H]⁺;
¹H NMR (400 MHz, CDCl₃) *δ* (ppm): 7.72 (s, 1H), 7.59 (s, 1H), 7.56-7.50 (m, 3H), 7.47-7.44 (m, 2H), 5.56, 5.55 (d, d, 1H), 5.32-5.28 (m, 3H), 5.23-5.19 (m, 1H), 4.41-4.30 (m, 2H), 3.85-3.78 (m, 2H), 3.69-3.67 (m, 1H), 3.66 (s, 3H), 3.65-3.64 (m, 1H), 3.63 (s, 3H), 3.01-2.95 (m, 1H), 2.65-2.49 (m, 5H), 2.41-1.72 (m, 19H), 1.02-0.89 (m, 12H).

### Example 51

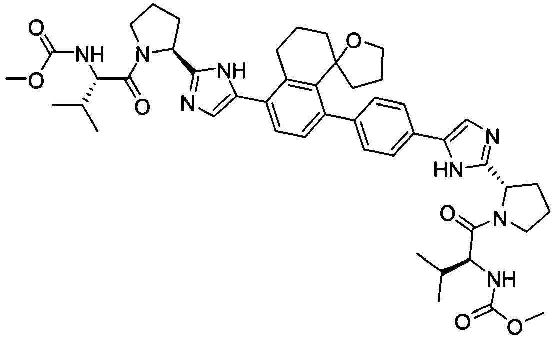

### Synthetic route:

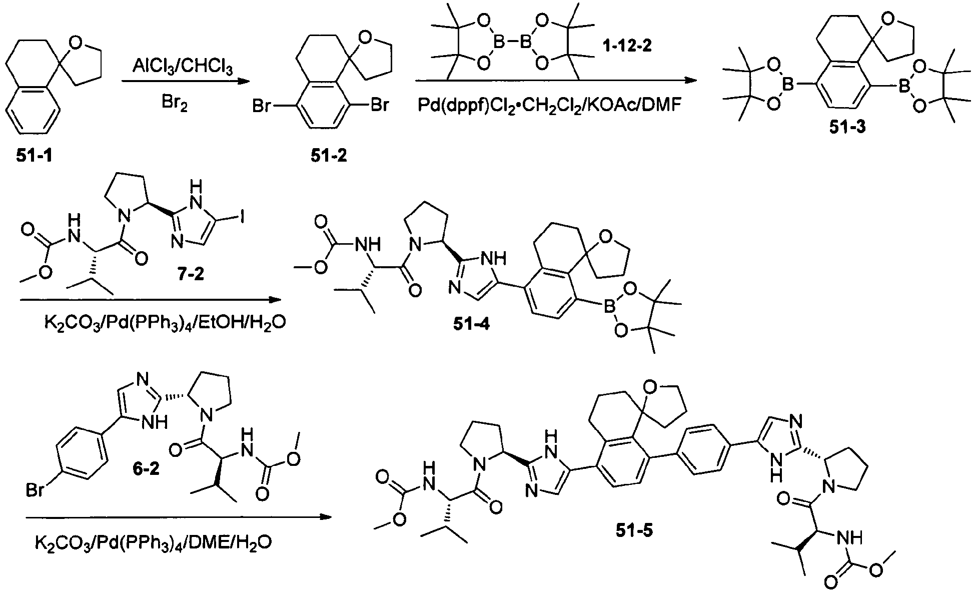

Compounds disclosed herein can be prepared by an analogous procedure to that described in Example 48:

Compound **51-2** was characterized by the following spectroscopic data:
MS (ESI, pos.ion) *m*/*z*: 344.5 [M+H]⁺;
¹H NMR (400 MHz, CDCl₃) *δ* (ppm): 7.39, 7.37 (t, t, 1H), 7.24, 7.22 (t, t, 1H), 4.06-3.89 (m, 2H), 2.96-2.83 (m, 2H), 2.79-2.63 (m, 2H), 2.23-2.13 (m, 1H), 2.02-1.75 (m, 5H).

Compound **51-3** was characterized by the following spectroscopic data:
MS (ESI, pos.ion) *m*/*z*: 441.5 [M+H]⁺;
¹H NMR (400 MHz, CDCl₃) *δ* (ppm): 8.31, 8.29 (t, t, 1H), 7.98, 7.96 (t, t, 1H), 3.99-3.82 (m, 2H), 2.82-2.70 (m, 2H), 2.59-2.44 (m, 2H), 2.08-1.79 (m, 6H), 1.32, 1.29 (m, m, 24H).

Compound **51-4** was characterized by the following spectroscopic data:
MS (ESI, pos.ion) *m*/*z*: 607.5 [M+H]⁺;
¹H NMR (400 MHz, CDCl₃) *δ* (ppm): 7.69, 7.67 (t, t, 1H), 7.56 (s, 1H), 7.28, 7.26 (t, t, 1H), 5.56, 5.55 (d, d, 1H), 5.32-5.28 (m, 1H), 4.34-4.30 (m, 1H), 4.03-3.86 (m, 2H), 3.85-3.78 (m, 1H), 3.66 (s, 3H), 3.65-3.61 (m, 1H), 2.74-2.58 (m, 2H), 2.53-2.36 (m, 2H), 2.30-2.16 (m, 3H), 2.13-2.03 (m, 2H), 2.02-1.84 (m, 4H), 1.75-1.71 (m, 2H), 1.32, 1.29 (m, m, 12H), 1.02, 1.00 (m, m, 3H), 0.93, 0.91 (m, m, 3H).

Compound **51-5** was characterized by the following spectroscopic data:
MS (ESI, pos.ion) *m*/*z*: 849.5 [M+H]⁺;
¹H NMR (400 MHz, CDCl₃) *δ* (ppm): 7.78 (s, 1H), 7.59 (s, 1H), 7.54-7.46 (m, 4H), 7.29, 7.27 (t, t, 1H), 7.20, 7.18 (t, t, 1H), 5.56, 5.55 (d, d, 1H), 5.32-5.28 (m, 2H), 5.23-5.19 (m, 1H), 4.41-4.30 (m, 2H), 4.06-4.00 (m, 1H), 3.96-3.89 (m, 1H), 3.85-3.78 (m, 2H), 3.69-3.67 (m, 1H), 3.66 (s, 3H), 3.65-3.64 (m, 1H), 3.63 (s, 3H), 2.78-2.48 (m, 4H), 2.30-1.87 (m, 16H), 1.02-0.89 (m, 12H).

### Example 52

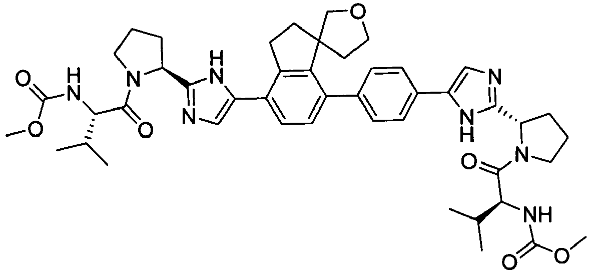

### Synthetic route:

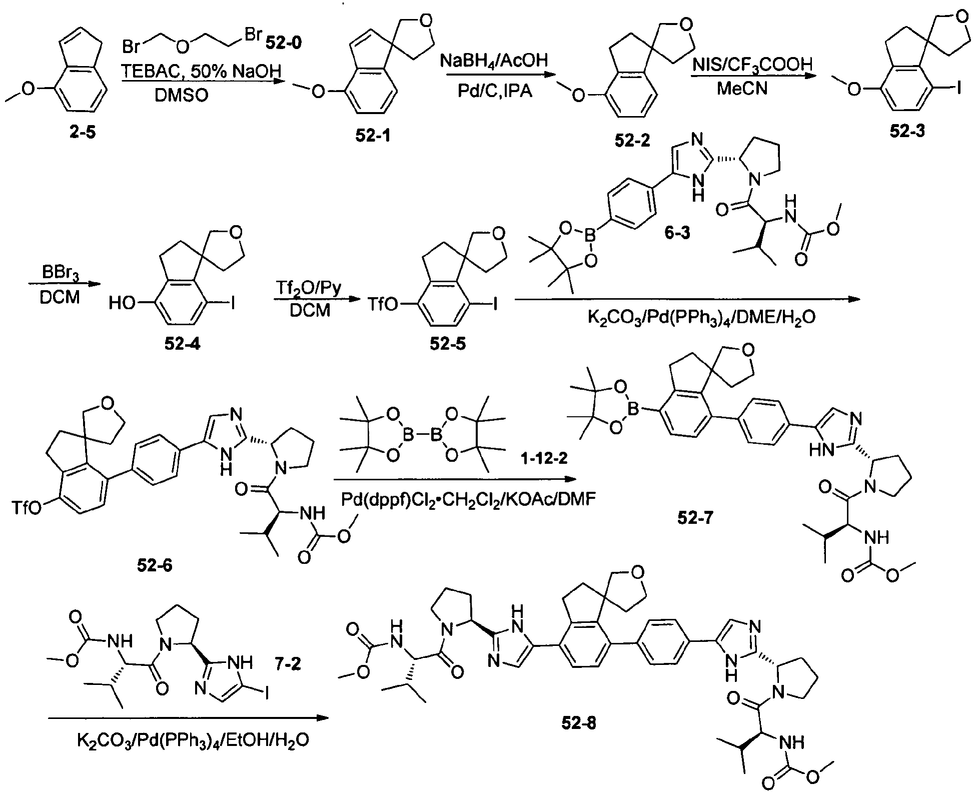

### Step 1) the preparation of compound 52-1

To a solution of compound **2-5** (996.2 mg, 6.82 mmol), compound **52-0** (1.543 g, 7.15 mmol) and TEBAC (0.3 g, 1.36 mmol) in DMSO (30 mL) was added sodium hydroxide aqueous solution (50%, 2.0 mL) dropwise at 0 °C. At the end of the addition, the mixture was stirred at 50 °C for 2.0 hrs. After the reaction was completed, the mixture was quenched with water (50 mL). The aqueous layer was extracted with EtOAc (50 mL x 3). The combined organic layers were washed with brine, dried over anhydrous Na₂SO₄ and concentrated *in vacuo.* The residue was purified by silica gel column chromatography (PE) to give the title compound as yellow oil (1.171 g, 85%). The compound was characterized by the following spectroscopic data:
MS (ESI, pos.ion) *m*/*z:* 203.5 [M+H]⁺;
¹H NMR (400 MHz, CDCl₃) *δ* (ppm): 6.89, 6.87, 6.85 (d, d, d, 1H), 6.83, 6.81 (t, t, 1H), 6.69, 6.67 (m, m, 1H), 6.63-6.60 (m, 1H), 6.46, 6.44 (m, m, 1H), 4.00-3.94 (m, 2H), 3.90-3.83 (m, 1H), 3.80 (s, 3H), 3.70-3.65 (m, 1H), 2.16-2.08 (m, 1H), 2.02-1.94 (m, 1H).

### Step 2) the preparation of compound 52-2

To a solution of compound **52-1** (947.84 mg, 4.69 mmol) in isopropanol (15 mL) were added a catalytic amount of Pd/C (100 mg), acetic acid (560 mg, 9.38 mmol) and NaBH₄ (700 mg, 18.76 mmol) in turn. At the end of the addition, the mixture was stirred at rt for 1.0 hr. After the reaction was completed, the mixture was filtered through a celite pad. The filtrate was concentrated *in vacuo.* The residue was purified by silica gel column chromatography (PE) to give the title compound as yellow oil (718 mg, 75%). The compound was characterized by the following spectroscopic data:
MS (ESI, pos.ion) *m*/*z:* 205.5 [M+H]⁺;
¹H NMR (400 MHz, CDCl₃) *δ* (ppm): 6.96, 6.93, 6.92 (m, m, m, 1H), 6.77, 6.75 (m, m, 1H), 6.62, 6.59 (m, m, 1H), 4.02-3.91 (m, 2H), 3.87-3.83 (m, 1H), 3.82 (s, 3H), 3.78-3.73 (m, 1H), 2.89-2.79 (m, 2H), 2.35-2.20 (m, 3H), 1.98-1.90 (m, 1H).

### Step 3) the preparation of compound 52-3

To a solution of compound **52-2** (534.79 mg, 2.62 mmol) and NIS (650 mg, 2.88 mmol) in MeCN (6.0 mL) was added CF₃COOH (0.059 mL, 0.79 mmol) dropwise at 0 °C. At the end of the addition, the mixture was stirred at 45 °C overnight. After the reaction was completed, the mixture was concentrated *in vacuo.* To the residue was added water (10 mL), and the mixture was extracted with EtOAc (20 mL x 3). The combined organic layers were washed with saturated sodium sulfite aqueous solution (20 mL x 2), dried over anhydrous Na₂SO₄ and concentrated *in vacuo.* The residue was purified by silica gel column chromatography (PE) to give the title compound as colorless liquid (691.7 mg, 80%). The compound was characterized by the following spectroscopic data:
MS (ESI, pos.ion) *m*/*z:* 331.5 [M+H]⁺;
¹H NMR (400 MHz, CDCl₃) *δ* (ppm): 7.49, 7.47 (t, t, 1H), 6.43, 6.41 (m, m, 1H), 4.03, 3.99 (m, 1H), 3.95-3.88 (m, 1H), 3.83 (s, 3H), 3.82-3.75 (m, 2H), 2.92-2.82 (m, 2H), 2.47-2.33 (m, 3H), 2.08-2.01 (m, 1H).

### Step 4) the preparation of compound 52-4

To a solution of compound **52-3** (1.267 g, 3.84 mmol) in DCM (15 mL) was added boron tribromide (2.2 mL, 23.04 mmol) dropwise at -78 °C. At the end of the addition, the mixture was stirred at -78 °C for 10 mins and at rt for another 1.0 hr. After the reaction was completed, the mixture was quenched with ice water (50 mL) and the organic phase was separated. The aqueous layer was extracted with EtOAc (20 mL x 3). The combined organic layers were washed with brine, dried over anhydrous Na₂SO₄ and concentrated *in vacuo.* The residue was purified by silica gel column chromatography (PE/EtOAc (v/v) = 8/1) to give the title compound as a white solid (970.75 mg, 80%). The compound was characterized by the following spectroscopic data:
MS (ESI, pos.ion) *m*/*z*: 317.5 [M+H]⁺;
¹H NMR (400 MHz, CDCl₃) *δ* (ppm): 7.42, 7.40 (t, t, 1H), 6.33, 6.31 (s, s, 1H), 4.81 (brs, 1H), 4.07-4.02 (m, 1H), 3.96-3.90 (m, 1H), 3.85-3.78 (m, 2H), 2.87-2.78 (m, 2H), 2.51-2.35 (m, 3H), 2.12-2.04 (m, 1H).

### Step 5) the preparation of compound 52-5

To a solution of compound **52-4** (960.64 mg, 3.04 mmol) in DCM (15 mL) was added pyridine (1.95 mL, 24.32 mmol) dropwise at 0 °C. After the mixture was stirred for 10 mins, trifluoromethanesulfonic anhydride (2.04 mL, 12.16 mmol) was added, and the mixture was stirred at rt for 1.0 hr. After the reaction was completed, the mixture was quenched with ice water (20 mL). The aqueous layer was extracted with EtOAc (20 mL x 3). The combined organic layers were washed with brine, dried over anhydrous Na₂SO₄ and concentrated *in vacuo.* The residue was purified by silica gel column chromatography (PE) to give the title compound as yellow oil (1.225 g, 90%). The compound was characterized by the following spectroscopic data:
¹H NMR (400 MHz, CDCl₃) *δ* (ppm): 7.63, 7.61 (t, t, 1H), 6.87, 6.85 (s, s, 1H), 4.03-3.98 (m, 1H), 3.95-3.88 (m, 1H), 3.84-3.74 (m, 2H), 3.05-2.96 (m, 2H), 2.50-2.34 (m, 3H), 2.08-2.00 (m, 1H).

### Step 6) the preparation of compound 52-6

A solution of compound **52-5** (819.75 mg, 1.83 mmol), compound **6-3** (908.21 mg, 1.83 mmol), Pd(PPh₃)₄ (105.8 mg, 0.0915 mmol) and K₂CO₃ (632.31 mg, 4.575 mmol) in mixed solvents of DME and H₂O (12 mL, v/v = 5/1) was stirred at 90 °C under N₂ for 4.0 hrs. After the reaction was completed, the mixture was diluted with EtOAc (50 mL), washed with water (20 mL x 3) and brine, dried over anhydrous Na₂SO₄ and concentrated *in vacuo.* The residue was purified by silica gel column chromatography (PE/EtOAc (v/v) = 1/2) to give the title compound (884.18 mg, 70%). The compound was characterized by the following spectroscopic data:
MS (ESI, pos.ion) *m*/*z:* 691.5 [M+H]⁺;
¹H NMR (400 MHz, CDCl₃) *δ* (ppm): 7.59 (s, 1H), 7.54-7.51 (m, 2H), 7.45-7.42 (m, 2H), 7.29, 7.27 (s, s, 1H), 6.98, 6.96 (t, t, 1H), 5.32, 5.30 (d, d, 1H), 5.23-5.19 (m, 1H), 4.41-4.36 (m, 1H), 3.99-3.92 (m, 2H), 3.87-3.78 (m, 2H), 3.75-3.71 (m, 1H), 3.68-3.65 (m, 1H), 3.63 (s, 3H), 3.03-2.94 (m, 2H), 2.45-1.92 (m, 9H), 0.97-0.89 (m, 6H).

### Step 7) the preparation of compound 52-7

A suspension of compound **52-6** (876.59 mg, 1.27 mmol), compound **1-12-2** (354 mg, 1.39 mmol), Pd(dppf)Cl₂CH₂Cl₂ (103 mg, 0.13 mmol) and KOAc (311 mg, 3.17 mmol) in DMF (5.0 mL) was stirred at 90 °C under N₂ for 3.0 hrs. After the reaction was completed, the mixture was cooled to rt, diluted with EtOAc (50 mL) and filtered through a celite pad. The filtrate was washed with water (20 mL x 3) and brine, dried over anhydrous Na₂SO₄ and concentrated *in vacuo.* The residue was purified by silica gel column chromatography (PE/EtOAc (v/v) = 1/2) to give the title compound (594.18 mg, 70%). The compound was characterized by the following spectroscopic data:
MS (ESI, pos.ion) *m*/*z*: 669.5 [M+H]⁺;
¹H NMR (400 MHz, CDCl₃) *δ* (ppm): 7.62, 7.60 (t, t, 1H), 7.59 (s, 1H), 7.54-7.51 (m, 2H), 7.50-7.46 (m, 2H), 7.47-7.45 (s, s, 1H), 5.32, 5.30 (d, d, 1H), 5.23-5.19 (m, 1H), 4.41-4.36 (m, 1H), 4.07-4.02 (m, 1H), 3.91-3.85 (m, 1H), 3.84-3.75 (m, 3H), 3.68-3.65 (m, 1H), 3.63 (s, 3H), 2.98-2.90 (m, 2H), 2.49-2.32 (m, 3H), 2.30-1.92 (m, 6H), 1.32, 1.29 (m, m, 12H), 0.97, 0.95 (m, m, 3H), 0.90, 0.89 (m, m, 3H).

### Step 8) the preparation of compound 52-8

A solution of compound **52-7** (668.37 mg, 1.0 mmol), compound **7-2** (428.47 mg, 1.02 mmol), Pd(PPh₃)₄ (116 mg, 0.10 mmol) and K₂CO₃ (346 mg, 2.5 mmol) in mixed solvents of EtOH and H₂O (12 mL, v/v = 5/1) was stirred at 90 °C under N₂ for 5.0 hrs. After the reaction was completed, the mixture was quenched with water (20 mL). The aqueous layer was extracted with EtOAc (20 mL x 3). The combined organic layers were washed with brine, dried over anhydrous Na₂SO₄ and concentrated *in vacuo.* The residue was purified by silica gel column chromatography (DCM/MeOH (v/v) = 50/1) to give the title compound (500.66 mg, 60%). The compound was characterized by the following spectroscopic data:
MS (ESI, pos.ion) *m*/*z:* 835.5 [M+H]⁺;
¹H NMR (400 MHz, CDCl₃) *δ* (ppm): 7.71 (s, 1H), 7.59 (s, 1H), 7.54-7.46 (m, 5H), 7.18, 7.16 (s, s, 1H), 5.56, 5.55 (d, d, 1H), 5.32-5.28 (m, 3H), 5.23-5.19 (m, 1H), 4.41-4.30 (m, 2H), 4.00-3.92 (m, 2H), 3.88-3.78 (m, 3H), 3.76-3.71 (m, 1H), 3.68-3.67 (m, 1H), 3.66 (s, 3H), 3.65-3.64 (m, 1H), 3.63 (s, 3H), 2.89-2.81 (m, 2H), 2.47-2.32 (m, 3H), 2.30-2.16 (m, 6H), 2.13-1.92 (m, 5H), 1.02-0.89 (m, 12H).

### Example 53

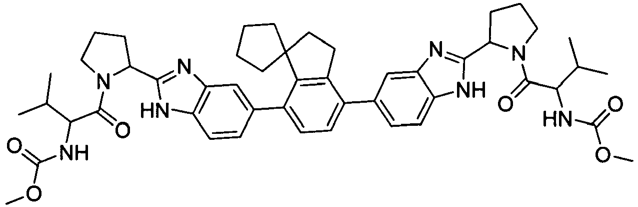

### Synthetic route:

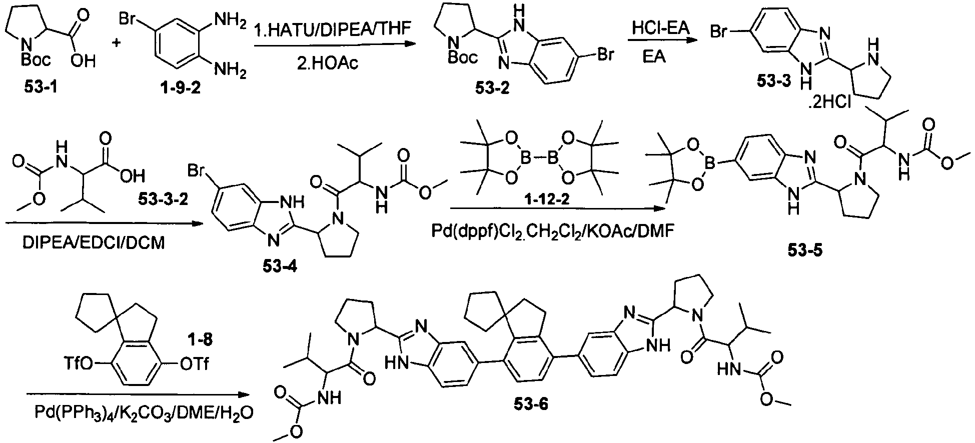

Compounds disclosed herein can be prepared by an analogous procedure to that described in Example 1:
Compound **53-2** was characterized by the following spectroscopic data:
   MS (ESI, pos.ion) *m*/*z*: 367.3 [M+H]⁺;
   ¹H NMR (400 MHz, CDCl₃) *δ* (ppm): 7.68 (s, 1H), 7.42-7.40 (m, 1H), 7.30-7.28 (m, 1H), 5.11-5.09 (m, 1H), 3.45-3.43 (m, 2H), 2.94-2.93 (m, 1H), 2.21-2.18 (m, 2H), 2.01-1.91 (m, 1H), 1.49 (s, 9H).
Compound **53-3** was characterized by the following spectroscopic data:
   MS (ESI, pos.ion) *mlz:* 313.2 [M+H]⁺;
   ¹H NMR (400 MHz, CDCl₃) *δ* (ppm): 8.01 (s, 1H), 7.70-7.76 (m, 2 H), 5.27-5.25 (m, 1H), 3.31-3.30 (m, 2H), 2.77-2.74 (m, 1H), 2.54-2.52 (m, 1H), 2.40-2.37 (m, 1H), 2.30-2.10 (m, 1H).
Compound **53-4** was characterized by the following spectroscopic data:
   MS (ESI, pos.ion) *m*/*z*: 423.3 [M+H]⁺;
   ¹H NMR (400 MHz, CDCl₃) *δ* (ppm): 7.59-7.51 (m, 1H), 7.34-7.21 (m, 2H), 5.42-5.38 (m, 2H), 4.34-4.30 (m, 1H), 3.87-3.76 (m, 1H), 3.70 (s, 3H), 3.66-3.62 (m, 1H), 3.04-2.98 (m, 1H), 2.25-2.21 (m, 1H), 2.20-2.13 (m, 2H), 1.96-1.94 (m, 1H), 0.88-0.84 (m, 6H).
Compound **53-5** was characterized by the following spectroscopic data:
   MS (ESI, pos.ion) *m*/*z*: 471.3 [M+H]⁺;
   ¹H NMR (400 MHz, CDCl₃) *δ* (ppm): 7.87-7.80 (m, 1H), 7.71-7.66 (m, 2H), 5.47-5.42 (m, 2H), 4.34-4.30 (m, 1H), 3.86-3.84 (m, 1H), 3.70 (s, 3H), 3.64-3.62 (m, 1H), 3.04-2.98 (m, 1H), 2.25-2.21 (m, 1H), 2.20-2.13 (m, 2H), 1.96-1.94 (m, 1H), 1.35 (s, 12H), 0.88-0.84 (m, 6H).
Compound **53-6** was characterized by the following spectroscopic data:
   MS (ESI, pos.ion) *m*/*z:* 857.5 [M+H]⁺;
   ¹H NMR (400 MHz, CDCl₃) *δ* (ppm): 7.78-7.69 (m, 2H), 7.48-7.47 (m, 1H), 7.38-7.32 (m, 2H), 7.22-7.18 (m, 2H), 7.06-7.01 (m, 1H), 5.54-5.53 (m, 2H), 5.48-5.46 (m, 2H), 4.37-4.33 (m, 2H), 3.89-3.87 (m, 2H), 3.71 (s, 6H), 3.13-3.11 (m, 2H), 2.94-2.92 (m, 2H), 2.48-2.32 (m, 2H), 2.29-2.11 (m, 4H), 2.09-1.92(m, 4H), 1.58-1.36 (m, 4H), 1.82-1.72 (m, 8H), 1.11-1.03 (m, 2H), 0.92-0.86 (m, 12H).

### Example 54

### Synthetic route:

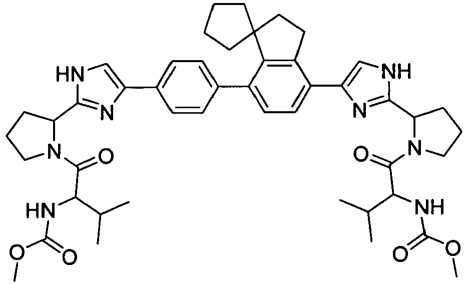

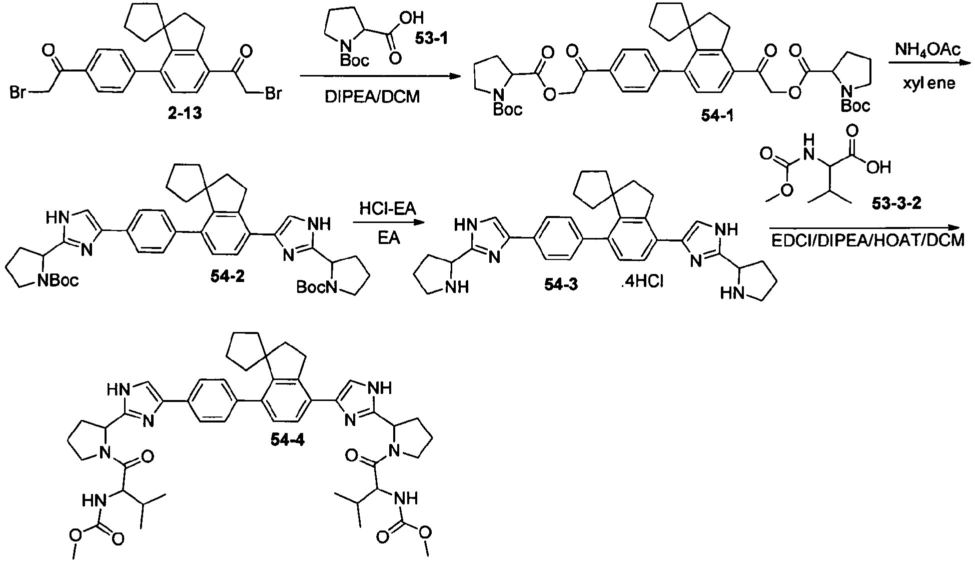

Compounds disclosed herein can be prepared by an analogous procedure to that described in Example 2:
Compound **54-1** was characterized by the following spectroscopic data:
   MS (ESI, pos.ion) *m*/*z:* 759.5 [M+H]⁺;
   ¹H NMR (400 MHz, CDCl₃) *δ* (ppm): 7.93 (t, 2H, *J* = 6.4 Hz), 7.58 (d, 1H, *J* = 7.8 Hz), 7.42-7.35 (m, 2H), 7.00 (d, 1H, *J* = 7.8 Hz), 5.66-5.10 (m, 4H), 4.53-4.38 (m, 2H), 3.65-3.51 (m, 2H), 3.51-3.36 (m, 2H), 3.21-3.14 (m, 2H), 2.40-2.25 (m, 4H), 2.15-2.05 (m, 2H), 2.00-1.85 (m, 4H), 1.60-1.40 (m, 24H), 1.36-1.20 (m, 4H).
Compound **54-2** was characterized by the following spectroscopic data:
   MS (ESI, pos.ion) *m*/*z*: 719.5 [M+H]⁺;
   ¹H NMR (400 MHz, CDCl₃) *δ* (ppm): 7.77-7.53 (br, 2H), 7.32-7.24 (m, 4H), 7.15 (s, 1H), 7.02-6.97 (m, 1H), 5.05-4.95 (m, 2H), 3.60-3.34 (m, 4H), 3.10-2.90 (m, 4H), 2.25-2.10 (m, 4H), 2.02-1.94 (m, 2H), 1.90 (t, 2H, *J* = 7.0 Hz), 1.80-1.69 (m, 2H), 1.63-1.40 (m, 22H), 1.40-1.30 (m, 2H).
Compound **54-3** was characterized by the following spectroscopic data:
   MS (ESI, pos.ion) *mlz:* 519.4 [M+H]⁺.
Compound **54-4** was characterized by the following spectroscopic data:
   MS (ESI, pos.ion) *m*/*z*: 833.5 [M+H]⁺;
   ¹H NMR (400 MHz, CDCl₃) *δ* (ppm): 10.80 (brs, 1H), 10.45 (brs, 1H), 7.80-7.67 (m, 2H), 7.45-7.10 (m, 5H), 6.96 (d, 1H, *J* = 7.7 Hz), 5.68-5.55 (m, 2H), 5.33-5.20 (m, 2H), 4.40-4.30 (m, 2H), 3.90-3.80 (m, 2H), 3.77-3.58 (m, 8H), 3.15-2.88 (m, 4H), 2.46-1.80 (m, 16H), 1.80-1.63 (m, 2H), 1.63-1.40 (m, 4H), 1.40-1.28 (m, 2H), 0.95-0.80 (m, 12H).

### Example 55

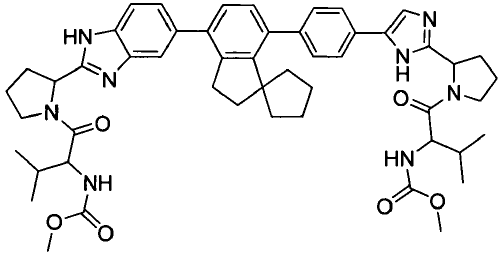

### Synthetic route:

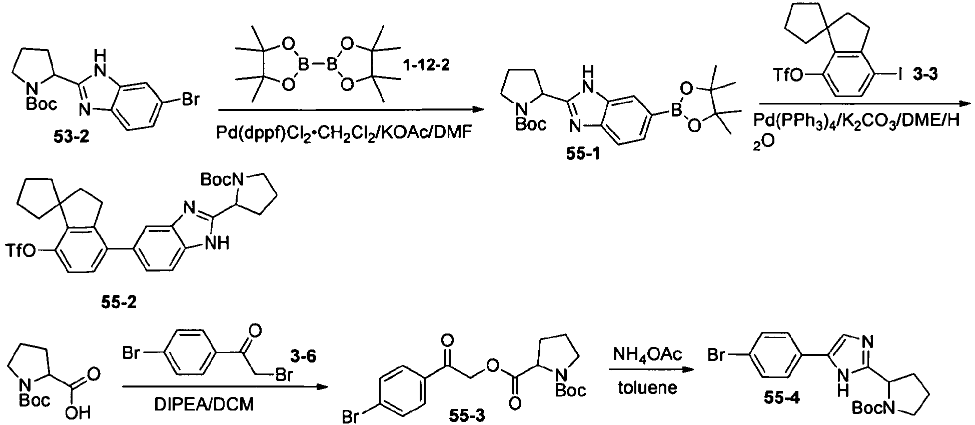

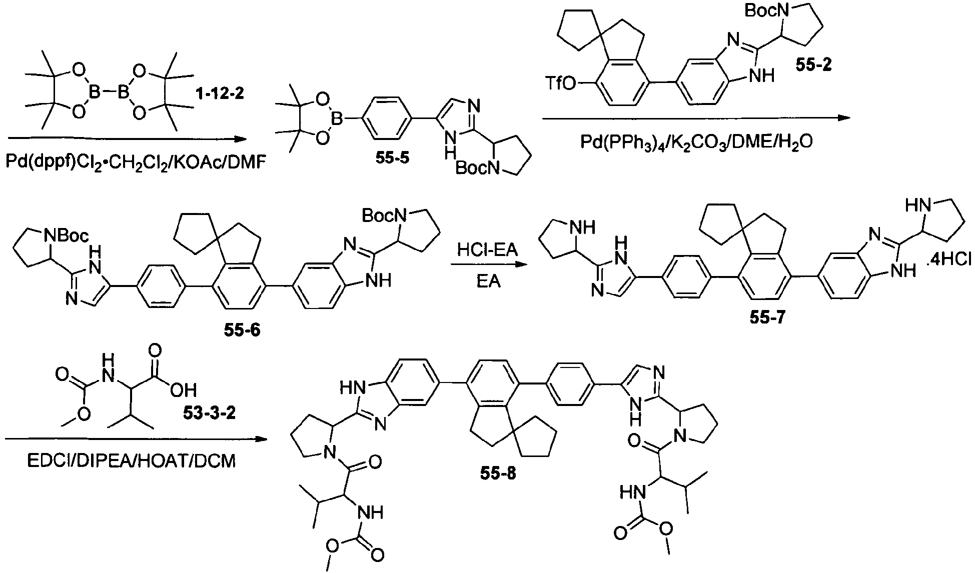

Compounds disclosed herein can be prepared by an analogous procedure to that described in Example 3:
Compound **55-1** was characterized by the following spectroscopic data:
   MS (ESI, pos.ion) *mlz:* 414.3 [M+H]⁺;
   ¹H NMR (400 MHz, CDCl₃) *δ* (ppm): 7.69 (s, 1H), 7.45-7.43 (m, 1H), 7.32-7.30 (m, 1H), 5.12-5.10 (m, 1H), 3.45-3.43 (m, 2H), 2.95-2.94 (m, 1H), 2.25-2.22 (m, 2H), 2.01-1.91 (m, 1H), 1.49 (s, 9H), 1.35 (s, 12H).
Compound **55-2** was characterized by the following spectroscopic data:
   MS (ESI, pos.ion) *m*/*z:* 606.5 [M+H]⁺;
   ¹H NMR (400 MHz, CDCl₃) *δ* (ppm): 7.61-7.51 (m, 2H), 7.25-7.16 (m, 3H), 5.15-5.13 (br, 1H), 3.44 (br, 2H), 2.93-2.89 (t, 2H, *J* = 8.0 Hz), 2.21-2.14 (m, 4H), 2.03-2.02 (m, 2H), 1.95-1.88 (m, 4H), 1.86-1.67 (m, 4H), 1.49 (s, 9H).
Compound **55-3** was characterized by the following spectroscopic data:
   MS (ESI, pos.ion) *mlz:* 412.7 [M+H]⁺;
   ¹H NMR (400 MHz, CDCl₃) *δ* (ppm): 7.78-7.75 (m, 2H), 7.65-7.63 (m, 2H), 5.53-5.15 (m, 2H), 4.49-4.39 (m, 1H), 3.59-3.54 (m, 1H), 3.48-3.38 (m, 1H), 2.31-2.21 (m, 2H), 2.12-2.01 (m, 1H), 1.98-1.85 (m, 1H), 1.45 (d, 9H).
Compound **55-4** was characterized by the following spectroscopic data:
   MS (ESI, pos.ion) *m*/*z*: 392.2 [M+H]⁺;
   ¹H NMR (400 MHz, CDCl₃) *δ* (ppm): 7.78-7.75 (m, 2H), 7.65-7.63 (m, 2H), 7.21-7.20 (m, 1H), 5.53-5.15 (m, 2H), 4.49-4.39 (m, 1H), 3.59-3.54 (m, 1H), 3.48-3.38 (m, 1H), 2.31-2.21 (m, 2H), 2.12-2.01 (m, 1H), 1.98-1.85 (m, 1H), 1.45 (d, 9H).
Compound **55-5** was characterized by the following spectroscopic data:
   ¹H NMR (400 MHz, CDCl₃) *δ* (ppm): 7.35 (m, 4H), 7.10 (s, 1H), 4.93 (t, 1H, *J* = 8.2 Hz), 3.88-3.66 (m, 2H), 2.90 (t, 1H, *J* = 8.0 Hz), 2.50-2.47 (m, 2H), 2.27-2.25 (m, 1H), 1.48 (s, 9H), 1.26 (s, 12H).
Compound **55-6** was characterized by the following spectroscopic data:
   MS (ESI, neg.ion) *m*/*z*: 769.5 [M-H]⁻;
   ¹H NMR (400 MHz, CDCl₃) *δ* (ppm): 7.73-7.68 (m, 3H), 7.40-7.35 (m, 3H), 7.29-7.27 (m, 3H), 7.07-7.05 (d, 3H, *J* = 8.0 Hz), 5.19-5.17 (br, 1H), 5.03-5.02 (br, 1H), 3.46 (br, 4H), 2.97-2.94 (m, 4H), 2.24-2.20 (m, 4H), 2.06-1.99 (m, 2H),1.88-1.79 (m, 4H), 1.64-1.61 (m, 2H), 1.53-1.51 (m, 18H), 1.41-1.36 (m, 4H).
Compound **55-7** was characterized by the following spectroscopic data:
   MS (ESI, pos.ion) *m*/*z:* 569.5 [M+H]⁺.
Compound **55-8** was characterized by the following spectroscopic data:
   MS (ESI, pos.ion) *m*/*z*: 883.5 [M+H]⁺;
   ¹H NMR (400 MHz, CDCl₃) *δ* (ppm): 7.77 (m, 1H), 7.73-7.69 (m, 1H), 7.54-7.52 (m, 1H), 7.37-7.26 (m, 4H), 7.26-7.16 (m, 2H), 6.98-6.96 (d, 1H, *J* = 8.0 Hz), 5.73-5.69 (m, 2H), 5.45-5.42 (br, 1H), 5.29-5.26 (br, 1H), 3.92-3.81 (m, 2H), 3.68 (s, 6H), 3.67-3.65 (m, 2H), 3.11-2.99 (m, 2H), 2.89-2.88 (m, 2H), 2.49-2.32 (m, 2H), 2.32-2.12 (m, 4H), 2.12-1.98 (m, 2H), 1.85-1.69 (m, 4H), 1.63-1.52 (br, 2H), 1.51-1.42 (m, 2H), 1.41-1.32 (m, 2H), 0.87-0.83 (m, 12H).

### Example 56

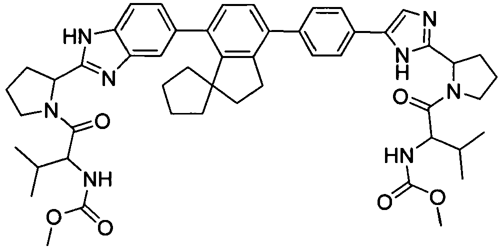

### Synthetic route:

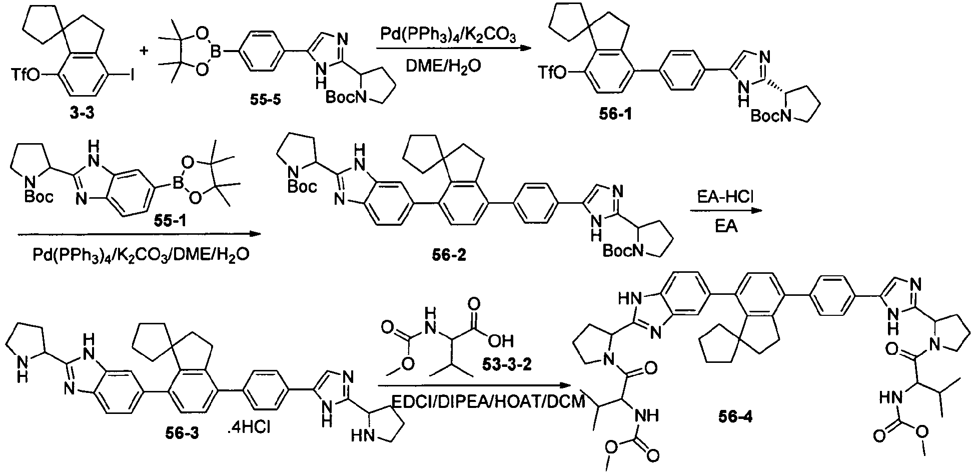

Compounds disclosed herein can be prepared by an analogous procedure to that described in Example 4:
Compound **56-1** was characterized by the following spectroscopic data:
   MS (ESI, pos.ion) *m*/*z*: 632.3 [M+H]⁺;
   ¹H NMR (400 MHz, CDCl₃) *δ* (ppm): 7.79-7.61 (m, 3H), 7.40-7.38 (d, 2H, *J* = 8.0 Hz), 7.23-7.18 (m, 2H), 5.01-4.99 (br, 1H), 3.44 (br, 2H), 2.95-2.92 (t, 2H, *J* = 8.0 Hz), 2.22-2.15 (m, 6H), 1.99-1.91 (m, 6H), 1.77-1.73 (m, 3H), 1.51 (s, 9H).
Compound **56-2** was characterized by the following spectroscopic data:
   MS (ESI, neg.ion) *m*/*z*: 767.5 [M-H]⁻;
   ¹H NMR (400 MHz, CDCl₃) *δ* (ppm): 7.71-7.70 (m, 3H), 7.53-7.51 (m, 3H), 7.28-7.27 (m, 1H), 7.22-7.20 (t, 2H, *J* = 8.0 Hz), 7.07-7.05 (m, 1H), 5.17-5.16 (br, 1H), 5.02-5.01 (br, 1H), 3.46 (br, 4H), 3.11-3.01 (br, 2H), 2.97-2.93 (t, 4H, *J* = 8.0 Hz), 2.23-2.21 (m, 4H), 2.06-1.99 (m, 2H), 1.87-1.84 (m, 2H), 1.61-1.60 (m, 2H), 1.57-1.55 (m, 2H), 1.53-1.51 (m, 18H), 1.41-1.36 (m, 4H).
Compound **56-3** was characterized by the following spectroscopic data:
   MS (ESI, pos.ion) *m*/*z*: 569.3 [M+H]⁺.
Compound **56-4** was characterized by the following spectroscopic data:
   MS (ESI, pos.ion) *m*/*z*: 883.5 [M+H]⁺;
   ¹H NMR (400 MHz, CDCl₃) *δ* (ppm): 7.80-7.68 (m, 2H), 7.45-7.43 (m, 3H), 7.19-7.17 (m, 1H), 7.16-7.15 (m, 3H), 7.00-6.98 (d, 1H, *J* = 8.0 Hz), 5.86-5.82 (m, 2H), 5.45-5.43 (br, 1H), 5.28-5.27 (br, 1H), 3.95-3.82 (m, 2H), 3.68 (s, 6H), 3.67-3.65 (m, 2H), 3.11-2.99 (m, 2H), 2.91-2.88 (m, 2H), 2.49-2.32 (m, 2H), 2.31-2.12 (m, 4H), 2.11-1.98 (m, 2H), 1.85-1.69 (m, 4H), 1.63-1.52 (br, 2H), 1.51-1.42 (m, 2H), 1.41-1.32 (m, 2H), 0.88-0.84 (m, 12H).

### BIOLOGICAL ACTIVITY

An HCV Replicon assay was utilized in the present invention, and was prepared, developed and validated as described in Science, 1999, 285 (5424), 110-3 and J. Virol., 2003, 77 (5), 3007-19.

HCV GT1a, GT1b and GT2a replicon cells were used to test the currently described compound series as well as wild-type cells HCV 1b and resistant cells Y93H, L31F, P32L and I302V. GT1a, GT1b and GT2a are HCV Replicon System which is transfected HCV 1a, HCV 1b and HCV 2b genotype respectively. The system containing G418 resistance gene NEO and Luciferase Reporter Gene can be used to determine the level of HCV replication, and evaluate the effects of the compounds inhibit HCV replication, by using a real-time quantitative polymerase chain reaction (qPCR) method to detect NEO, and chemiluminescence method to test Luciferase Reporter Gene.

### Operating procedure:

### 1. Test method for measuring EC₅₀ of the compounds by luciferase assay.

The GT1a cells and GT1b cells were seeded into 96-well plates (8,000 cells in 125 µL / well) respectively; each compound was diluted to desired concentration using 5-fold serial dilutions protocol, 10 doses in duplicate, and added to wells with POD™ 810 Plate Assembler. The final concentration of DMSO was 0.5%; the plates were incubated in a CO₂ incubator for 72 hours; after that, 40 µL of Luciferase assay substrate (Promega Bright-Glo) was added to each well, and detected by a chemiluminescence detection system (Topcount Microplate Scintillation and Luminescence Counter) 5 minutes later; data analysis.

### 2. Test method for measuring EC₅₀ of the compounds by detecting antibiotic G418 resistance gene NEO gene.

The GT1a cells and GT1b cells were seeded into 96-well plates (8,000 cells in 125 µL / well) respectively; each compound was diluted to desired concentration using 5-fold serial dilutions protocol, 10 doses in duplicate, and added to wells with POD™ 810 Plate Assembler., the final concentration of DMSO was 0.5%; the cells were incubated in a CO₂ incubator for 72 hours; quantitative PCR.
- Sample preparation: the supernatant was removed, 100 µL of FCW buffer solution was added to each well, washed carefully and discarded the solution; 50 µL of lysate FCP was added to each well, the cells was lysed as PCR template and PCR template was diluted with DEPC water as sample template.
- Quantitative PCR: preparation of reaction mixture according to PCR system; the reaction mixture was dispensed into a 384-well PCR reaction plate (specially for quantitative); and a standard template which was diluted in proportion was distributed into the plate; and the sample template was distributed into the plate; then the 384-well plate was sealed with closure plate membrane; the qualitative PCR machine was operated by procedures; data analysis.

### 3. Data processing: the EC₅₀ values of compounds were analyzed by GraphPad Prism software.

The compounds of the present disclosure can be effective against the HCV 1b genotype according to the experiment data, and EC₅₀ ranges of compounds which have different groups against HCV 1b are 1-999 pM, 1-99 nM; the compounds of the present disclosure can inhibit multiple genotypes of HCV (such as HCV 1a or HCV 2a). Table 2 shows the EC₅₀ values of representative compounds of the present disclosure against the HCV 1a and HCV 1b genotypes. In one embodiment compounds of the present disclosure are active against the 1a, 1b, 2a, 2b, 3a, 3b, 4a, and 5a genotypes.

The experimental results of wild-type and resistance cells and the simulation results of molecular modeling and docking show that compounds disclosed herein play an excellent anti-HCV role, which suggest a novel anti-HCV mechanism by interfering with HCV NS5A protein.

**Table 2**

| Example | 1a (nM) | 1b (nM) | Example | 1a (nM) | 1b (nM) | Example | 1a (nM) | 1b (nM) |
|---|---|---|---|---|---|---|---|---|
| **1** | 0.102 | 0.041 | **20** | 5.896 | 1.034 | **39** | 0.027 | 0.020 |
| **2** | 0.325 | 0.009 | **21** | 4.875 | 0.980 | **40** | 0.043 | 0.017 |
| **3** | 0.097 | 0.032 | **22** | 3.652 | 0.876 | **41** | 0.876 | 0.043 |
| **4** | 0.085 | 0.029 | **23** | 0.334 | 0.012 | **42** | 6.890 | 0.109 |
| **5** | 2.489 | 0.086 | **24** | 2.687 | 0.055 | **43** | 7.276 | 0.148 |
| **6** | 1.372 | 0.208 | **25** | 10.675 | 0.032 | **44** | 13.684 | 0.937 |
| **7** | 0.955 | 0.106 | **26** | 13.675 | 0.088 | **45** | 0.107 | 0.034 |
| **8** | 5.436 | 1.328 | **27** | 12.675 | 0.655 | **46** | 0.388 | 0.118 |
| **9** | 0.097 | 0.032 | **28** | 4.566 | 0.667 | **47** | 3.568 | 0.687 |
| **10** | 9.864 | 3.462 | **29** | 8.675 | 0.218 | **48** | 3.083 | 0.240 |
| **11** | 0.447 | 0.096 | **30** | 3.203 | 0.074 | **49** | 23.587 | 0.357 |
| **12** | 0.856 | 0.018 | **31** | 4.678 | 0.048 | **50** | 0.875 | 0.096 |
| **13** | 0.438 | 0.032 | **32** | 0.064 | 0.077 | **51** | 4.573 | 0.086 |
| **14** | 0.785 | 0.011 | **33** | 0.090 | 0.039 | **52** | 3.671 | 0.033 |
| **15** | 0.886 | 0.029 | **34** | 0.837 | 0.017 | **53** | 0.263 | 0.074 |
| **16** | 0.008 | 0.005 | **35** | 0.518 | 0.086 | **54** | 3.572 | 0.044 |
| **17** | 0.475 | 0.072 | **36** | 1.085 | 0.089 | **55** | 0.073 | 0.028 |
| **18** | 0.458 | 0.203 | **37** | 3.779 | 0.099 | **56** | 0.138 | 0.036 |
| **19** | 0.376 | 0.011 | **38** | 0.066 | 0.009 | | | |

It will be evident to one skilled in the art that the present disclosure is not limited to the foregoing illustrative examples, and that it can be embodied in other specific forms without departing from the essential attributes thereof. It is therefore desired that the examples be considered in all respects as illustrative and not restrictive, reference being made to the appended claims, rather than to the foregoing examples, and all changes which come within the meaning are therefore intended to be embraced therein.

The compounds of the present disclosure may inhibit HCV by mechanisms in addition to or other than NS5A inhibition. In one embodiment the compounds of the present disclosure inhibit HCV replicon and in another embodiment the compounds of the present disclosure inhibit NS5A. The compounds of the present disclosure may inhibit multiple genotypes of HCV.

Reference throughout this specification to "an embodiment," "some embodiments," "one embodiment", "another example," "an example," "a specific example," or "some examples," means that a particular feature, structure, material, or characteristic described in connection with the embodiment or example is included in at least one embodiment or example of the present disclosure. Thus, the appearances of the phrases such as "in some embodiments," "in one embodiment", "in an embodiment", "in another example," "in an example," "in a specific example," or "in some examples," in various places throughout this specification are not necessarily referring to the same embodiment or example of the present disclosure. Furthermore, the particular features, structures, materials, or characteristics may be combined in any suitable manner in one or more embodiments or examples.

Although explanatory embodiments have been shown and described, it would be appreciated by those skilled in the art that the above embodiments can not be construed to limit the present disclosure.

## Claims

1. A compound of formula (I): 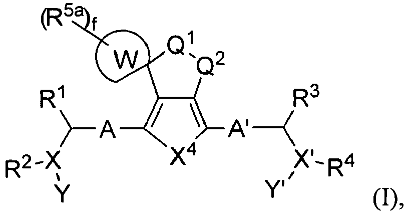 or a stereoisomer, a geometric isomer, a tautomer, an N-oxide, a hydrate, a solvate, or a pharmaceutically acceptable salt thereof, wherein
each of A and A' is independently a bond, alkylene, alkenylene, cycloalkylene, heterocyclylalkylene, -(CR⁸R^{8a})ₙ-O-(CR⁸R^{8a})ₚ-, -(CR⁸R^{8a})ₙ-N(R⁵)-(CR⁸R^{8a})ₚ-, -(CR⁸R^{8a})ₙ-S(=O)ᵣ-N(R⁵)-(CR⁸R^{8a})ₚ-, -(CR⁸R^{8a})ₙ-C(=O)-N(R⁵)-(CR⁸R^{8a})ₚ-, -(CR⁸R^{8a})ₙ-N(R⁵)-C(=O)-N(R⁵)-(CR⁸R^{8a})ₚ-, -(CR⁸R^{8a})ₙ-C(=O)-O-(CR⁸R^{8a})ₚ-, -(CR⁸R^{8a})ₙ-N(R⁵)-S(=O)ᵣ-N(R⁵)-(CR⁸R^{8a})ₚ- or -(CR⁸R^{8a})ₙ-N(R⁵)-C(=O)-O-(CR⁸R^{8a})ₚ-; or each of A and A' is independently 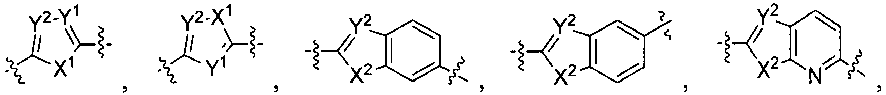 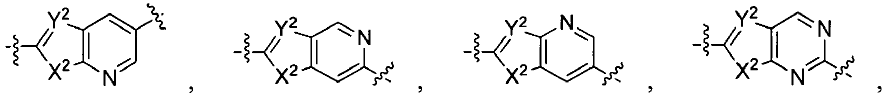 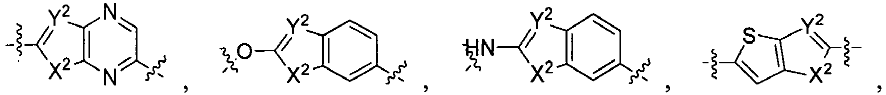 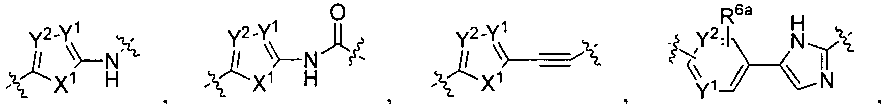 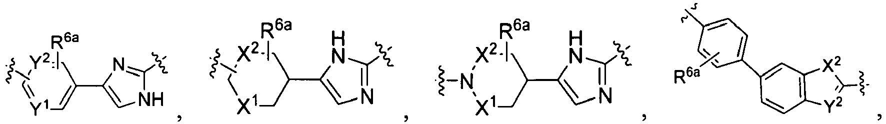 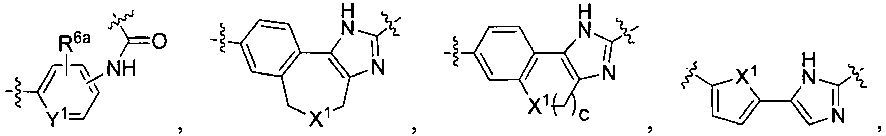 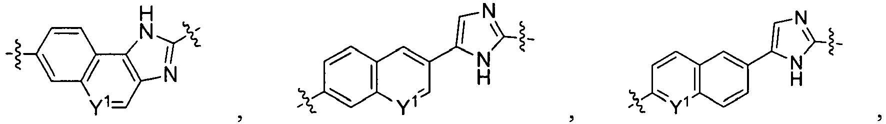 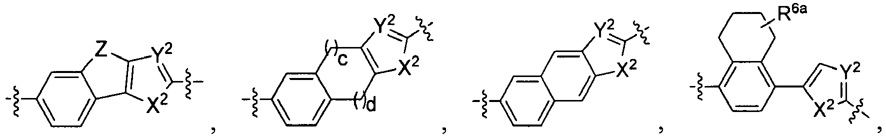 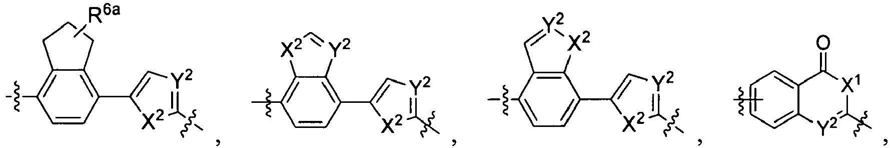 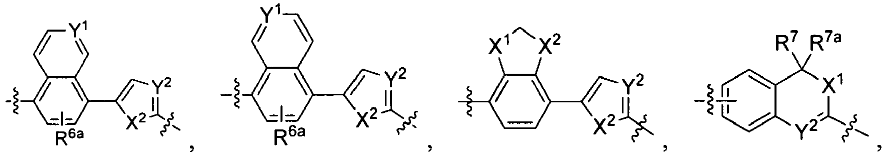 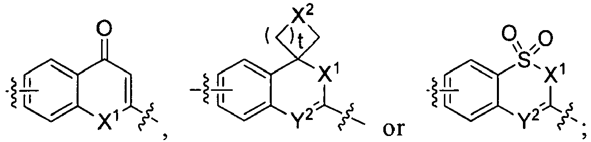
wherein each X¹ is independently O, S, NR⁶ or CR⁷R^{7a};
each X² is independently NR⁶, O or S;
X⁴ is (CR⁷R^{7a})ₙ, -Y¹=Y²-, O, S or NR⁶;
W is carbocyclyl or heterocyclyl;
each Y¹ and Y² is independently N or CR⁷;
each Z is independently -(CH₂)ₐ-, -CH=CH-, -N=CH-, -(CH₂)ₐ-N(R⁵)-(CH₂)_{b}- or -(CH₂)ₐ-O-(CH₂)_{b}-, wherein each a and b is independently 0, 1, 2 or 3;
each c is independently 1 or 2;
each d is independently 1 or 2;
each n is independently 0, 1, 2 or 3;
each p is independently 0, 1, 2 or 3;
each r is independently 0, 1 or 2;
f is 0, 1, 2, 3 or 4;
each of Q¹ and Q² is independently NR⁶, O, S, C(=O) or (CR⁷R^{7a})ₑ;
e is 0, 1, 2, 3 or 4;
each of X and X' is independently N or CR⁷;
each of Y and Y' is independently H, deuterium, alkyl, heteroalkyl, cycloalkyl, heterocyclyl, aryl, heteroaryl, aralkyl, a group derived from α-amino acid or an optically isomer thereof; or each of Y and Y' is independently -[U-(CR⁹R^{9a})ₜ-N(R¹⁰)-(CR⁹R^{9a})ₜ]ₖ-U-(CR⁹R^{9a})ₜ-N(R¹¹)-(CR⁹R^{9a})ₜ-R¹², -U-(CR⁹R^{9a})ₜ-R¹² or -[U-(CR⁹R^{9a})ₜ-N(R¹⁰)-(CR⁹R^{9a})ₜ]ₖ-U-(CR⁹R^{9a})ₜ-O-(CR⁹R^{9a})ₜ-R¹²;
each U is independently -C(=O)-, -C(=S)-, -S(=O)- or -S(=O)₂-;
each t is independently 0, 1, 2, 3 or 4;
each k is independently 0, 1 or 2;
R¹ and R², together with X-CH they are attached to, optionally form a 3-8 membered heterocycle or carbocycle, C₅₋₁₂ fused bicycle, C₅₋₁₂ fused heterobicycle, C₅₋₁₂ spiro bicycle or C₅₋₁₂ spiro heterobicycle; and R³ and R⁴, together with X'-CH they are attached to, optionally form a 3-8 membered heterocycle or carbocycle, C₅₋₁₂ fused bicycle, C₅₋₁₂ fused heterobicycle, C₅₋₁₂ spiro bicycle or C₅₋₁₂ spiro heterobicycle;
each R⁵ is independently H, deuterium, hydroxy, alkyl, heteroalkyl, cycloalkyl, heterocyclyl, aryl, heteroaryl, aralkyl, alkoxy, alkyl-OC(=O)-, alkyl-C(=O)-, carbamoyl, alkyl-OS(=O)ᵣ-, alkyl-S(=O)ᵣO-, alkyl-S(=O)ᵣ- or aminosulfonyl;
each R^{5a} is independently H, deuterium, oxo (=O), hydroxy, amino, F, Cl, Br, I, cyano, R^{13a}R¹³N-, -C(=O)NR¹³R^{13a}, -OC(=O)NR¹³R^{13a}, -OC(=O)OR¹³, -N(R¹³)C(=O)NR¹³R^{13a}, -N(R¹³)C(=O)OR¹³, -N(R¹³)C(=O)-R¹³, R¹³R¹³-S(=O)₂-, R¹³S(=O)₂-, R¹³S(=O)₂N(R^{13a})-, R^{13a}R¹³N-alkyl, R¹³S(=O)-alkyl, R¹³R^{13a}N-C(=O)-alkyl, R^{13a}R¹³N-alkoxy, R¹³S(=O)-alkoxy, R¹³R^{13a}N-C(=O)-alkoxy, aryl, heteroaryl, alkoxy, alkylamino, alkyl, haloalkyl, alkenyl, alkynyl, heterocyclyl, cycloalkyl, mercapto, nitro, aralkyl, arylamino, heteroarylamino, arylalkylamino, heteroarylalkylamino, heteroaryloxy, heteroarylalkyl, arylalkoxy, heteroarylalkoxy, heterocyclyloxy, heterocyclylalkoxy, heterocyclylamino, heterocyclylalkylamino or aryloxy;
each R⁶ is independently H, deuterium, R¹³R¹³NC(=O)-, R¹³OC(=O)-, R¹³C(=O)-, R¹³R^{13a}NS(=O)-, R¹³OS(=O)-, R¹³S(=O)-, R¹³R^{13a}NS(=O)₂-, R¹³OS(=O)₂-, R¹³S(=O)₂-, aliphatic, haloaliphatic, hydroxyaliphatic, aminoaliphatic, alkoxyaliphatic, alkylaminoaliphatic, alkylthioaliphatic, arylaliphatic, heteroarylaliphatic, heterocyclylaliphatic, cycloalkylaliphatic, aryloxyaliphatic, heterocyclyloxyaliphatic, cycloalkyloxyaliphatic, arylaminoaliphatic, heterocyclylaminoaliphatic, cycloalkylaminoaliphatic, aryl, heteroaryl, heterocyclyl or carbocyclyl;
each R^{6a} is independently H, deuterium, hydroxy, amino, F, Cl, Br, I, cyano, oxo (=O), R^{13a}R¹³N-, -C(=O)NR¹³R^{13a}, -OC(=O)NR¹³R^{13a}, -OC(=O)OR¹³, -N(R¹³)C(=O)NR¹³R^{13a}, -N(R¹³)C(=O)OR^{13a}, -N(R¹³)C(=O)-R^{13a}, R¹³R^{13a}N-S(=O)₂-, R¹³S(=O)₂-, R¹³S(=O)₂N(R^{13a})-, R^{13a}R¹³N-alkyl, R¹³S(=O)-alkyl, R¹³R^{13a}N-C(=O)-alkyl, R¹³R¹³N-alkoxy, R¹³S(=O)-alkoxy, R¹³R^{13a}N-C(=O)-alkoxy, aryl, heteroaryl, alkoxy, alkylamino, alkyl, haloalkyl, alkenyl, alkynyl, heterocyclyl, cycloalkyl, mercapto, nitro, aralkyl, arylamino, heteroarylamino, arylalkylamino, heteroarylalkylamino, heteroaryloxy, heteroarylalkyl, arylalkoxy, heteroarylalkoxy, heterocyclyloxy, heterocyclylalkoxy, heterocyclylamino, heterocyclylalkylamino or aryloxy;
each R⁷ and R^{7a} is independently H, deuterium, F, Cl, Br, I, aliphatic, heteroalkyl, haloaliphatic, hydroxyaliphatic, aminoaliphatic, alkoxyaliphatic, alkylaminoaliphatic, alkylthioaliphatic, arylaliphatic, heteroarylaliphatic, heterocyclylaliphatic, cycloalkylaliphatic, aryloxyaliphatic, heterocyclyloxyaliphatic, cycloalkyloxyaliphatic, arylaminoaliphatic, heterocyclylaminoaliphatic, cycloalkylaminoaliphatic, aryl, heteroaryl, heterocyclyl or carbocyclyl;
each R⁸ and R^{8a} is independently H, deuterium, hydroxy, cyano, nitro, F, Cl, Br, I, alkyl, heteroalkyl, cycloalkyl, heterocyclyl, aryl, heteroaryl, aralkyl, alkoxy, alkyl-OC(=O)-, alkyl-C(=O)-, carbamoyl, alkyl-OS(=O)ᵣ-, alkyl-S(=O)ᵣO-, alkyl-S(=O)ᵣ- or aminosulfonyl;
each R⁹, R^{9a}, R¹⁰ and R¹¹ is independently H, deuterium, alkyl, heteroalkyl, cycloalkyl, heterocyclyl, aryl, heteroaryl, aralkyl, haloalkyl, hydroxyalkyl, heteroarylalkyl, heterocyclylalkyl or cycloalkylalkyl;
each R¹² is independently R^{13a}R¹³N-, -C(=O)R¹³, -C(=S)R¹³, -C(=O)OR¹³, -C(=O)NR¹³R^{13a}, -OC(=O)NR¹³R^{13a}, -OC(=O)OR¹³, -N(R¹³)C(=O)NR¹³R^{13a}, -N(R¹³)C(=O)OR^{13a}, -N(R¹³)C(=O)-R^{13a}, R¹³R^{13a}N-S(=O)₂-, R¹³S(=O)₂-, R¹³S(=O)₂N(R^{13a})-, R¹³OS(=O)₂-, alkyl, heteroalkyl, cycloalkyl, heterocyclyl, aryl, heteroaryl or aralkyl;
or R¹¹ and R¹² are optionally joined to form a 4-7 membered ring; and
each R¹³ and R^{13a} is independently H, deuterium, alkyl, heteroalkyl, cycloalkyl, heterocyclyl, aryl, heteroaryl or aralkyl; with the proviso that where R¹³ and R^{13a} are bonded to the same nitrogen atom, R¹³ and R^{13a}, together with the nitrogen atom they are attached to, optionally form a substituted or unsubstituted 3-8 membered ring or a substituted or unsubstituted spiro or fused bicyclic ring;
wherein each of alkylene, alkenylene, cycloalkylene, heterocyclylalkylene, -(CR⁸R^{8a})ₙ-O-(CR⁸R^{8a})ₚ-, -(CR⁸R^{8a})ₙ-N(R⁵)-(CR⁸R^{8a})ₚ-, -(CR⁸R^{8a})ₙ-S(=O)ᵣ-N(R⁵)-(CR⁸R^{8a})ₚ-, -(CR⁸R^{8a})ₙ-C(=O)-N(R⁵)-(CR⁸R^{8a})ₚ-, -(CR⁸R^{8a})ₙ-N(R⁵)-C(=O)-N(R⁵)-(CR⁸R^{8a})ₚ-, -(CR⁸R^{8a})ₙ-C(=O)-O-(CR⁸R^{8a})ₚ-, -(CR⁸R^{8a})ₙ-N(R⁵)-S(=O)ᵣ-N(R⁵)-(CR⁸R^{8a})ₚ-, -(CR⁸R^{8a})ₙ-N(R⁵)-C(=O)-O-(CR⁸R^{8a})ₚ-, -[U-(CR⁹R^{9a})ₜ-N(R¹⁰)-(CR⁹R^{9a})ₜ]ₖ-U-(CR⁹R^{9a}]ₜ-N(R¹¹)-(CR⁹R^{9a})ₜ-R¹², -U-(CR⁹R^{9a})ₜ-R¹², -[U-(CR⁹R^{9a})ₜ -N(R¹⁰)-(Ck⁹R^{9a})ₜ]ₖ-U-(CR⁹R^{9a})ₜ-O-(CR⁹R^{9a})ₜ-R¹², NR⁶, CR⁷R^{7a}, CR⁷, -(CH₂)ₐ-, -CH=CH-, -N=CH-, -(CH₂)ₐ-N(R⁵)-(CH₂)_{b}-, -(CH₂)ₐ-O-(CH₂)_{b}-, R^{13a}R¹³N-, -C(=O)R¹³, -C(=S)R¹³, -C(=O)OR¹³, -C(=O)NR¹³R^{13a}, -OC(=O)NR¹³R^{13a}, -OC(=O)OR¹³, -N(R¹³)C(=O)NR¹³R^{13a}, -N(R¹³)C(=O)OR^{13a}, -N(R¹³)C(=O)-R^{13a}, R¹³R^{13a}N-S(=O)₂-, R¹³S(=O)₂-, R¹³S(=O)₂N(R^{13a})-, R¹³OS(=O)₂-, alkyl-OC(=O)-, alkyl-C(=O)-, alkyl-OS(=O),-, alkyl-S(=O)ᵣO-, alkyl-S(=O)ᵣ-, R¹³R^{13a}NC(=O)-, R¹³OC(=O)-, R¹³C(=O)-, R¹³R^{13a}NS(=O)-, R¹³OS(=O)-, R¹³S(=O)-, R^{13a}R¹³N-alkyl, R¹³S(=O)-alkyl, R¹³R^{13a}N-C(=O)-alkyl, R^{13a}R¹³N-alkoxy, R¹³S(=O)-alkoxy, R¹³R^{13a}-C(=O)-alkylamino, alkyl, heteroalkyl, carbocyclyl, cycloalkyl, heterocyclyl, aryl, heteroaryl, aralkyl, a group derived from α-amino acid, C₅₋₁₂ fused bicycle, C₅₋₁₂ fused heterobicycle, C₅₋₁₂ spiro bicycle, C₅₋₁₂ spiro heterobicycle, alkoxy, aliphatic, haloaliphatic, hydroxyaliphatic, aminoaliphatic, alkoxyaliphatic, alkylaminoaliphatic, alkylthioaliphatic, arylaliphatic, heteroarylaliphatic, heterocyclylaliphatic, cycloalkylaliphatic, aryloxyaliphatic, heterocyclyloxyaliphatic, cycloalkyloxyaliphatic, arylaminoaliphatic, heterocyclylaminoaliphatic, cycloalkylaminoaliphatic, haloalkyl, alkenyl, alkynyl, arylamino, heteroarylamino, arylalkylamino, heteroarylalkylamino, heteroaryloxy, heteroarylalkyl, arylalkoxy, heteroarylalkoxy, heterocyclyloxy, heterocyclylalkoxy, heterocyclylamino, heterocyclylalkylamino and aryloxy is optionally substituted with one or more substituents, wherein the substituent is deuterium, hydroxy, amino, halo, cyano, aryl, heteroaryl, alkoxy, alkylamino, alkylthio, alkyl, alkenyl, alkynyl, heterocyclyl, mercapto, nitro, aryloxy, heteroaryloxy, oxo (=O), carboxy, hydroxy-substituted alkoxy, hydroxy-substituted alkyl-C(=O)-, alkyl-C(=O)-, alkyl-S(=O)-, alkyl-S(=O)₂ hydroxy-substituted alkyl-S(=O)-, hydroxy-substituted alkyl-S(=O)₂- or carboxy-substituted alkoxy; or wherein W is C₃₋₈ carbocyclyl or C₂₋₁₀ heterocyclyl.

2. The compound according to claim 1, wherein 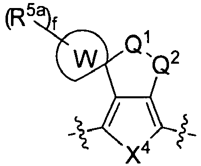 is 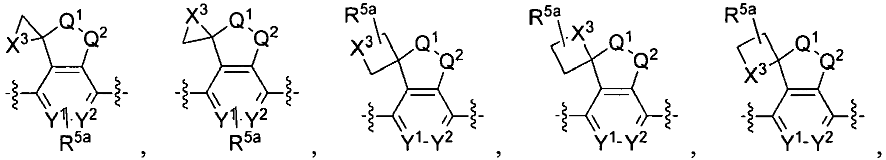 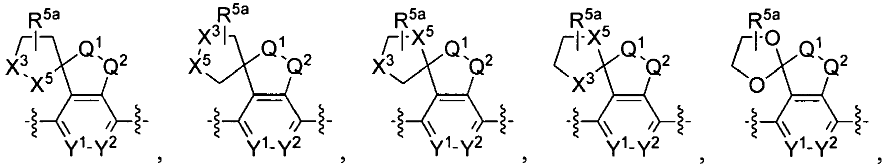 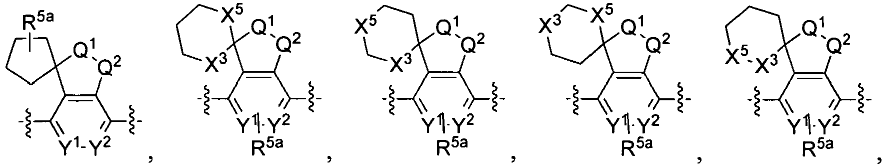 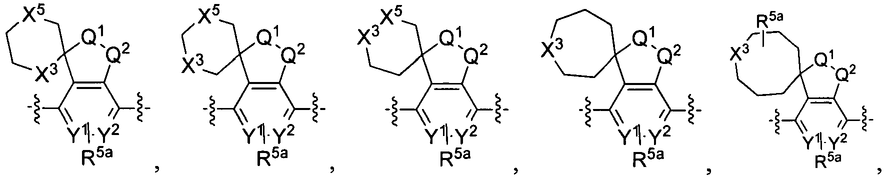 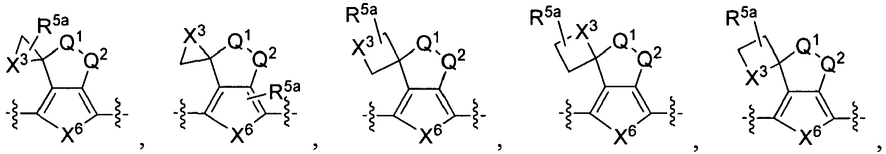 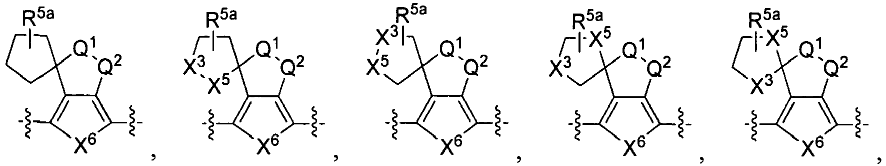 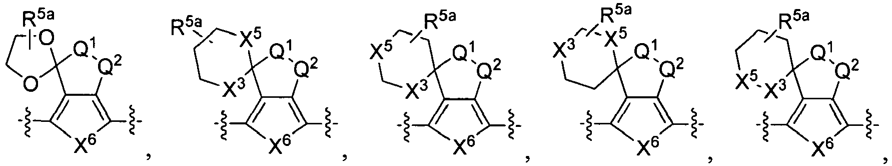 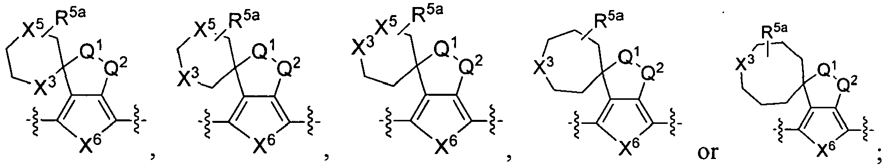
wherein each X³ and X⁵ is independently O, S, NR⁶ or CR⁷R^{7a};
each X⁶ is independently CR⁷R^{7a}, O, S or NR⁶;
each Y¹ and Y² is independently N or CR⁷;
f is 0, 1, 2, 3 or 4;
each Q¹ and Q² is independently NR⁶, O, S, C(=O) or (CR⁷R^{7a})ₑ;
e is 0, 1,2,3 3 or 4;
each R^{5a} is independently H, deuterium, oxo (=O), hydroxy, amino, F, Cl, Br, I, cyano, C₁₋₆ alkylacyl, C₁₋₆ alkylacyloxy, C₁₋₆ alkoxyacyl, C₁₋₆ alkylsulfonyl, C₁₋₆ alkoxysulfonyl, C₁₋₆ alkylsulfinyl, C₁₋₆ alkylsulfonyloxy, C₁₋₆ alkylsulfinyloxy, C₁₋₆ alkoxy, C₁₋₆ alkyl, C₆₋₁₀ aryl, -CF₃, -OCF₃, mercapto, nitro, C₁₋₆ alkylamino, C₃₋₁₀ cycloalkyl or C₆₋₁₀ aryloxy;
each R⁶ is independently H, deuterium, R¹³R^{13a}NC(=O)-, R¹³OC(=O)-, R¹³C(=O)-, R¹³R^{13a}NS(=O)-, R¹³OS(=O)-, R¹³S(=O)-, R¹³R^{13a}NS(=O)₂-, R¹³OS(=)₂-, R¹³S(=O)2-, C₁₋₆ alkyl, C₁₋₆ haloalkyl, C₁₋₆ hydroxyalkyl, C₁₋₆ aminoalkyl, C₁₋₆ alkoxy-C₁₋₆-alkyl, C₁₋₆ alkylamino-C₁₋₆-alkyl, C₁₋₆ alkylthio-C₁₋₆-alkyl, C₆₋₁₀ aryl-C₁₋₆-alkyl, C₁₋₉ heteroaryl-C₁₋₆-alkyl, C₂₋₁₀ heterocyclyl-C₁₋₆-alkyl, C₃₋₁₀ cycloalkyl-C₁₋₆-alkyl, C₆₋₁₀ aryloxy-C₁₋₆-alkyl, C₂₋₁₀ heterocyclyloxy-C₁₋₆-alkyl, C₃₋₁₀ cycloalkyloxy-C₁₋₆-alkyl, C₆₋₁₀ arylamino-C₁₋₆-alkyl, C₂₋₁₀ heterocyclylamino-C₁₋₆-alkyl, C₃₋₁₀ cycloalkylamino-C₁₋₆-alkyl, C₆₋₁₀ aryl, C₁₋₉ heteroaryl, C₂₋₁₀ heterocyclyl or C₃₋₁₀ carbocyclyl;
each R⁷ and R^{7a} is independently H, deuterium, F, Cl, Br, I, C₁₋₆ alkyl, C₂₋₆ heteroalkyl, C₁₋₆ haloalkyl, C₁₋₆ hydroxyalkyl, C₁₋₆ aminoalkyl, C₁₋₆ alkoxy-C₁₋₆-alkyl, C₁₋₆ alkylamino-C₁₋₆-alkyl, C₁₋₆ alkylthio-C₁₋₆-alkyl, C₆₋₁₀ aryl-C₁₋₆-alkyl, C₁₋₉ heteroaryl-C₁₋₆-alkyl, C₂₋₁₀ heterocyclyl-C₁₋₆-alkyl, C₃₋₁₀ cycloalkyl-C₁₋₆-alkyl, C₆₋₁₀ aryloxy-C₁₋₆-alkyl, C₂₋₁₀ heterocyclyloxy-C₁₋₆-alkyl, C₃₋₁₀ cycloalkyloxy-C₁₋₆-alkyl, C₆₋₁₀ arylamino-C₁₋₆-alkyl, C₂₋₁₀ heterocyclylamino-C₁₋₆-alkyl, C₃₋₁₀ cycloalkylamino-C₁₋₆-alkyl, C₆₋₁₀ aryl, C₁₋₉ heteroaryl, C₂₋₁₀ heterocyclyl or C₃-₁₀ carbocyclyl; and
each R¹³ and R^{13a} is independently H, deuterium, C₁₋₆ alkyl, C₂₋₆ heteroalkyl, C₃₋₁₀ cycloalkyl, C₂₋₁₀ heterocyclyl, C₆₋₁₀ aryl, C₁₋₉ heteroaryl or C₆₋₁₀ aryl-C₁₋₆-alkyl; with the proviso that where R¹³ and R^{13a} are bonded to the same nitrogen atom, R¹³ and R^{13a}, together with the nitrogen atom they are attached to, optionally form a substituted or unsubstituted 3-8 membered ring or a substituted or unsubstituted spiro or fused bicyclic ring; or wherein 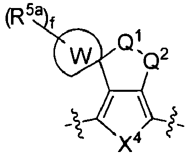 is 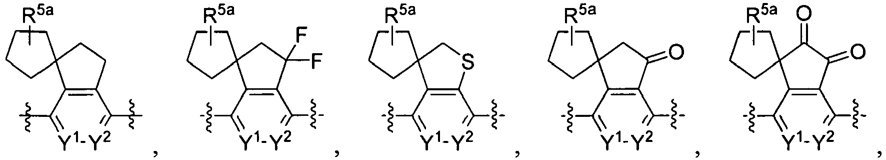 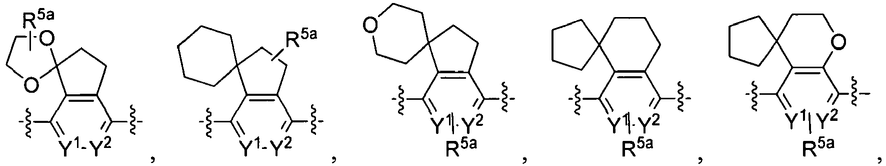 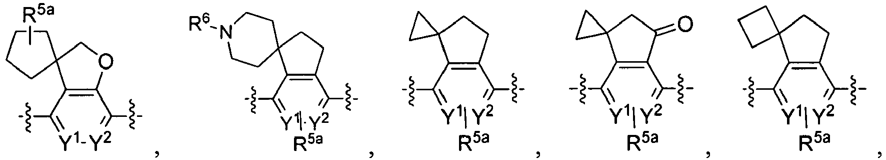 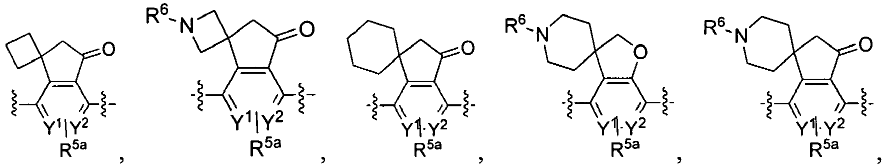 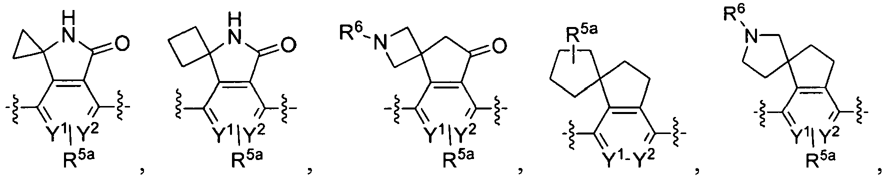 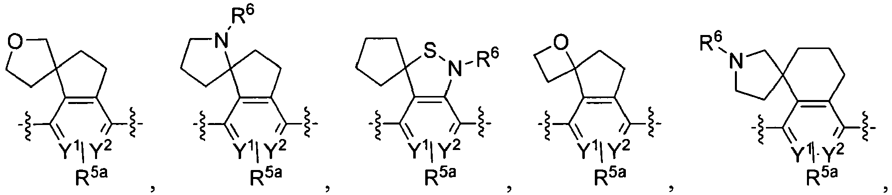 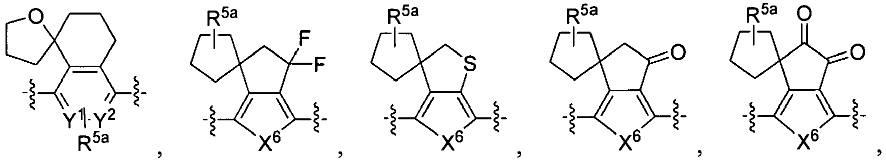 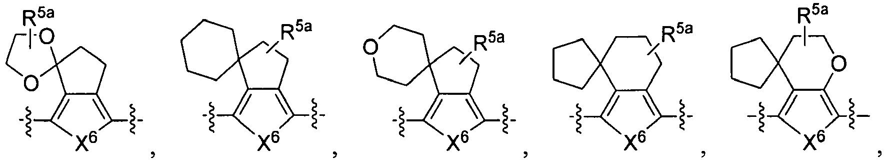 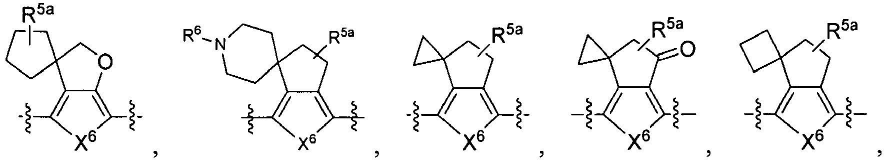 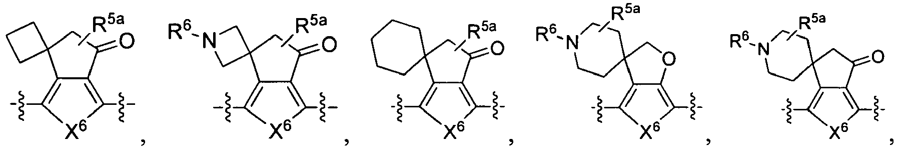 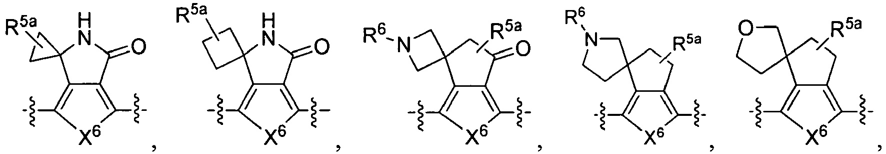 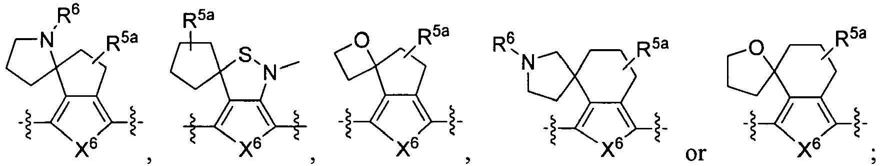
wherein each Y¹ and Y² is independently N or CR⁷;
each X⁶ is independently CR⁷R^{7a}, O, S or NR⁶_{;}
each R^{5a} is independently H, deuterium, oxo (=O), hydroxy, amino, F, Cl, Br, I, cyano, C₁₋₆ alkylacyl, C₁₋₆ alkylacyloxy, C₁₋₆ alkoxyacyl, C₁₋₆ alkylsulfonyl, C₁₋₆ alkoxysulfonyl, C₁₋₆ alkylsulfinyl, C₁₋₆ alkylsulfonyloxy, C₁₋₆ alkylsulfinyloxy, C₁₋₆ alkoxy, C₁₋₆ alkyl, C₆₋₁₀ aryl, -CF₃, -OCF₃, mercapto, nitro or C₁₋₆ alkylamino;
each R⁶ is independently H, deuterium, C₁₋₆ alkyl, C₁₋₆ haloalkyl, C₁₋₆ hydroxyalkyl, C₁₋₆ aminoalkyl, C₁₋₆ alkoxy-C₁₋₆-alkyl, C₁₋₆ alkylamino-C₁₋₆-alkyl, C₁₋₆ alkylthio-C₁₋₆-alkyl, C₆₋₁₀ aryl-C₁₋₆-alkyl, C₁₋₉ heteroaryl-C₁₋₆-alkyl, C₂₋₁₀ heterocyclyl-C₁₋₆**-**alkyl or C₃₋₈ cycloalkyl-C₁₋₆-alkyl; and
each R⁷ and R^{7a} is independently H, deuterium, F, Cl, Br, I, C₁₋₆ alkyl, C₂₋₆ heteroalkyl, C₁₋₆ haloalkyl, C₁₋₆ hydroxyalkyl, C₁₋₆ aminoalkyl, C₁₋₆ alkoxy-C₁₋₆-alkyl, C₁₋₆ alkylamino-C₁₋₆-alkyl, C₁₋₆ alkylthio-C₁₋₆-alkyl, C₆₋₁₀ aryl-C₁₋₆-alkyl, C₂₋₁₀ heterocyclyl-C₁₋₆-alkyl, C₃₋₁₀ cycloalkyl-C₁-₆-alkyl, C₆₋₁₀ aryloxy-C₁₋₆-alkyl, C₂₋₁₀ heterocyclyloxy-C₁₋₆-alkyl, C₃₋₁₀ cycloalkyloxy-C₁₋₆-alkyl, C₆₋₁₀ arylamino-C₁₋₆-alkyl, C₂₋₁₀ heterocyclylamino-C₁₋₆-alkyl, C₃₋₁₀ cycloalkylamino-C₁₋₆-alkyl, C₆₋₁₀ aryl, C₁₋₉ heteroaryl, C₂₋₁₀ heterocyclyl or C₃₋₈ carbocyclyl.

3. The compound according to claim 1, wherein each of A and A' is independently a bond, C₁₋₆ alkylene, C₂₋₆ alkenylene, C₃₋₈ cycloalkylene, C₂₋₁₀ heterocyclylalkylene, -(CR⁸R^{8a})ₙ-O-(CR⁸R^{8a})ₚ-, -(CR⁸R^{8a})ₙ-N(R⁵)-(CR⁸R^{8a})p-, -(CR⁸R^{8a})ₙ-S(=O)ᵣN(R⁵)-(CR⁸R^{8a})ₚ-, -(CR⁸R^{8a})ₙ-C(=O)-N(R⁵)-(CR⁸R^{8a})ₚ-, -(CR⁸R^{8a})ₙ-N(R⁵)-C(=O)-N(R⁵)-(CR⁸R^{8a})ₚ-, -(CR⁸R^{8a})ₙ-C(=O)-O-(CR⁸R^{8a})ₚ-, -(CR⁸R^{8a})ₙ-N(R⁵)-S(=O)ᵣ-N(R⁵)-(CR⁸R^{8a})ₚ- or -(CR⁸R^{8a})ₙ-N(R⁵)-C(=O)-O-(CR⁸R^{8a})ₚ-; or each of A and A' is independently 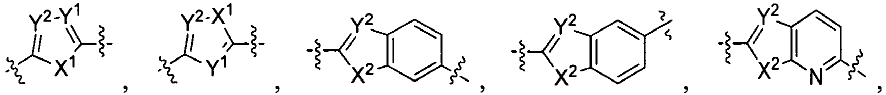 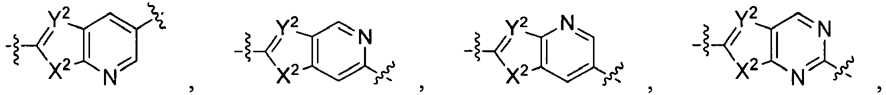 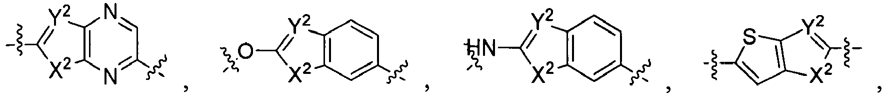 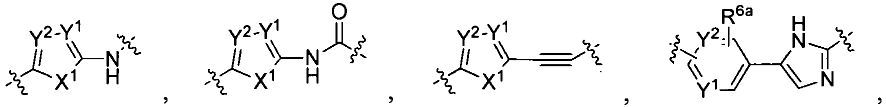 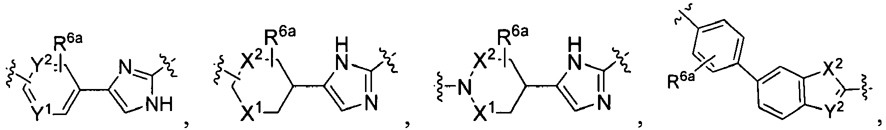 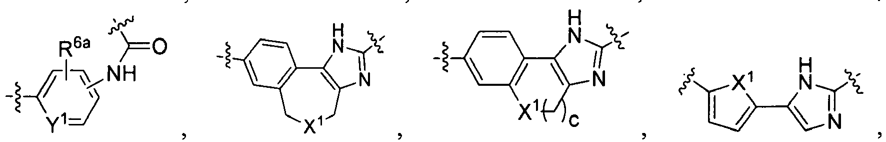 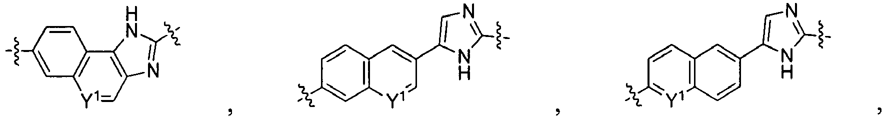 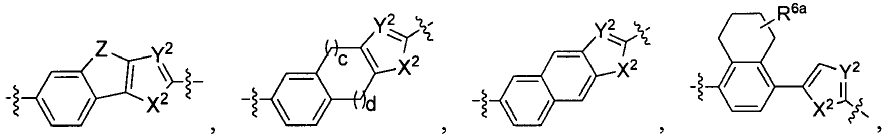 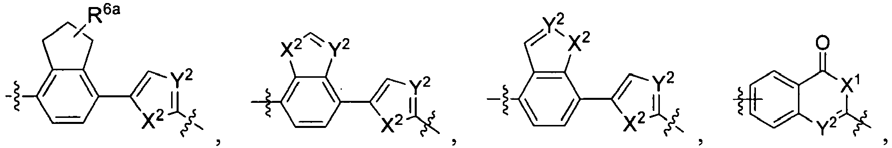 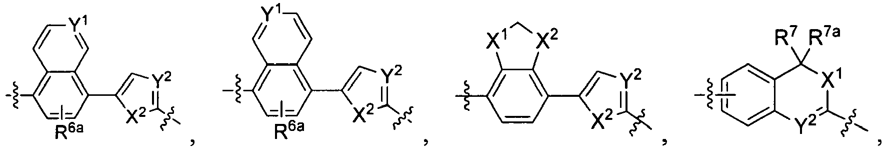 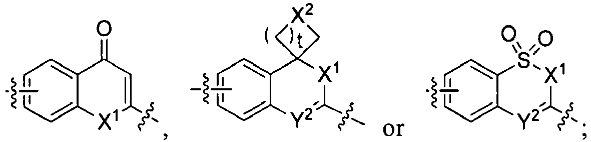
wherein each X¹ is independently O, S, NR⁶ or CR⁷R^{7a};
each X² is independently NR⁶, O or S;
each Y¹ and Y² is independently N or CR⁷;
each c is independently 1 or 2;
each d is independently 1 or 2;
each n is independently 0, 1, 2 or 3;
each p is independently 0, 1, 2 or 3;
each r is independently 0, 1 or 2;
each R⁵ is independently H, deuterium, hydroxy, C₁₋₆ alkyl, C₂₋₆ heteroalkyl, C₃₋₈ cycloalkyl, C₂₋₁₀ heterocyclyl, C₆₋₁₀ aryl, C₁₋₉ heteroaryl, C₆₋₁₀ aryl-C₁₋₆-alkyl, C₁₋₆ alkoxy, C₁₋₆ alkyl-OC(=O)-, C₁₋₆ alkyl-C(=O)-, carbamoyl, C₁₋₆ alkyl-OS(=O)ᵣ-, C₁₋₆ alkyl-S(=O)ᵣO-, C₁₋₆ alkyl-S(=O)ᵣ- or aminosulfonyl;
each R⁶ is independently H, deuterium, R¹³R^{13a}NC(=O)-, R¹³OC(=O)-, R¹³C(=O)-, R¹³R^{13a}NS(=O)-, R¹³OS(=O)-, R¹³S(=O)-, R¹³R^{13a}NS(=O)₂-, R¹³OS(=O)₂-, R¹³S(=O)₂-, C₁₋₆ alkyl, C₁₋₆ haloalkyl, C₁₋₆ hydroxyalkyl, C₁₋₆ aminoalkyl, C₁₋₆ alkoxy-C₁₋₆-alkyl, C₁₋₆ alkylamino-C₁₋₆-alkyl, C₁₋₆ alkylthio-C₁₋₆-alkyl, C₆₋₁₀ aryl-C₁₋₆-alkyl, C₁₋₆ heteroaryl-C₁₋₆-alkyl, C₂₋₁₀ heterocyclyl-C₁₋₆-alkyl, C₃₋₁₀ cycloalkyl-C₁₋₆-alkyl, C₆₋₁₀ aryloxy-C₁₋₆-alkyl, C₂₋₁₀ heterocyclyloxy-C₁₋₆-alkyl, C₃₋₁₀ cycloalkyloxy-C₁₋₆-alkyl, C₆₋₁₀ arylamino-C₁₋₆-alkyl, C₂₋₁₀ heterocyclylamino-C₁₋₆-alkyl, C₃₋₁₀ cycloalkylamino-C₁₋₆-alkyl, C₆₋₁₀ aryl, C₁₋₉ heteroaryl, C₂₋₁₀ heterocyclyl or C₃₋₁₀ carbocyclyl;
each R^{6a} is independently H, deuterium, hydroxy, amino, F, Cl, Br, I, cyano, oxo (=O), R^{13a}R¹³N-, -C(=O)NR¹³R^{13a}, -OC(=O)NR¹³R^{13a}, -OC(=O)OR¹³, -N(R¹³)C(=O)NR¹³R^{13a}, -N(R¹³)C(=O)OR^{13a}, -N(R¹³)C(=O)-R^{13a}, R¹³R^{13a}N-S(=O)₂-, R¹³S(=O)₂-, R¹³S(=O)₂N(R^{13a})-, R^{13a}R¹³N-C₁₋₆ alkyl, R¹³S(=O)-C₁₋₆ alkyl, R¹³R^{13a}-C(=O)-C₁₋₆ alkyl, R^{13a}R¹³N-C₁₋₆ alkoxy, R¹³S(=O)-C₁₋₆ alkoxy, R¹³R^{13a}N-C(=O)-C₁₋₆ alkoxy, C₆₋₁₀ aryl, C₁₋₉ heteroaryl, C₁₋₆ alkoxy, C₁₋₆ alkylamino, C₁₋₆ alkyl, C₁₋₆ haloalkyl, C₂₋₆ alkenyl, C₂₋₆ alkynyl, C₂₋₁₀ heterocyclyl, C₃₋₈ cycloalkyl, mercapto, nitro, C₆₋₁₀ aryl-C₁₋₆-alkyl, C₆₋₁₀ arylamino, C₁₋₉ heteroarylamino or C₆₋₁₀ aryloxy;
each R⁷ and R^{7a} is independently H, deuterium, F, Cl, Br, I, C₁₋₆ alkyl, C₂₋₆ heteroalkyl, C₁₋₆ haloalkyl, C₁₋₆ hydroxyalkyl, C₁₋₆ aminoalkyl, C₁₋₆ alkoxy-C₁₋₆-alkyl, C₁₋₆ alkylamino-C₁₋₆-alkyl, C₁₋₆ alkylthio-C₁₋₆-alkyl, C₆₋₁₀ aryl-C₁₋₆-alkyl, C₂₋₁₀ heterocyclyl-C₁₋₆-alkyl, C₃₋₁₀ cycloalkyl-C₁₋₆-alkyl, C₆₋₁₀ aryloxy-C₁₋₆-alkyl, C₂₋₁₀ heterocyclyloxy-C₁₋₆-alkyl, C₃₋₁₀ cycloalkyloxy-C₁₋₆-alkyl, C₆₋₁₀ arylamino-C₁₋₆-alkyl, C₂₋₁₀ heterocyclylamino-C₁₋₆-alkyl, C₃₋₁₀ cycloalkylamino-C₁₋₆-alkyl, C₆₋₁₀ aryl, C₁₋₉ heteroaryl, C₂₋₁₀ heterocyclyl or C₃₋₈ carbocyclyl;
each R¹³ and R^{13a} is independently H, deuterium, C₁₋₆ alkyl, C₂₋₆ heteroalkyl, C₃₋₁₀ cycloalkyl, C₂₋₁₀ heterocyclyl, C₆₋₁₀ aryl, C₁₋₉ heteroaryl or C₆₋₁₀ aryl-C₁₋₆-alkyl; with the proviso that where R¹³ and R^{13a} are bonded to the same nitrogen atom, R¹³ and R^{13a}, together with the nitrogen atom they are attached to, optionally form a substituted or unsubstituted 3-8 membered ring or a substituted or unsubstituted spiro or fused bicyclic ring; and
each R⁸ and R^{8a} is independently H, deuterium, hydroxy, cyano, nitro, F, Cl, Br, I, C₁₋₆ alkyl, C₂₋₆ heteroalkyl, C₃₋₁₀ cycloalkyl, C₂₋₁₀ heterocyclyl, C₆₋₁₀ aryl, C₁₋₉ heteroaryl, C₆₋₁₀ aryl-C₁₋₆-alkyl, C₁₋₆ alkoxy, C₁₋₆ alkyl-OC(=O)-, C₁₋₆ alkyl-C(=O)-, carbamoyl, C₁₋₆ alkyl-OS(=O)ᵣ-, C₁₋₆ alkyl-S(=O)ᵣO-, C₁₋₆ alkyl-S(=O)ᵣ- or aminosulfonyl; or wherein each of A and A' is independently a bond, -CH₂-, -(CH₂)₂-, -CH=CH-, -CH=CH-CH₂-, -N(R⁶)-, -C(=O)-, -C(=S)-, -C(=O)-O-, -C(=O)N(R⁶)-, -OC(=O)N(R⁶)-, -OC(=O)O-, -N(R⁶)C(=O)N(R⁶)-, -(R⁶)N-S(=O)₂-, -S(=O)₂-, -OS(=O)₂-, -(R⁶)N-S(=O)-, -S(=O)-, -OS(=O)-; or each of A and A' is independently 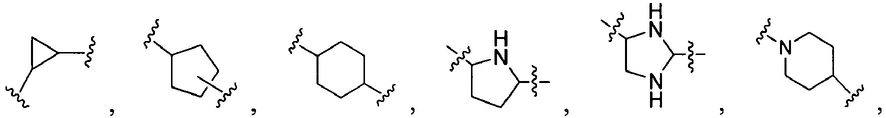 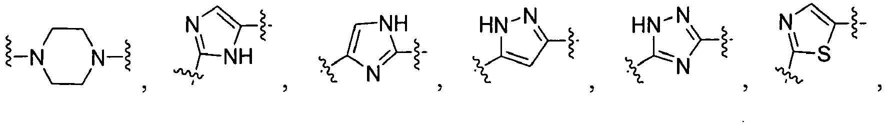 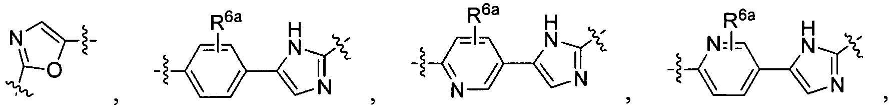 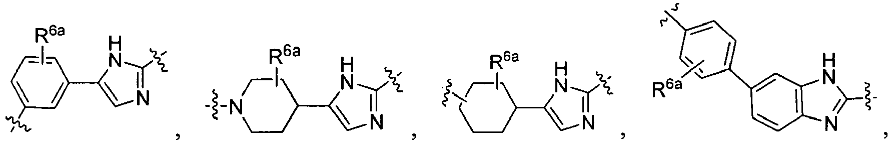 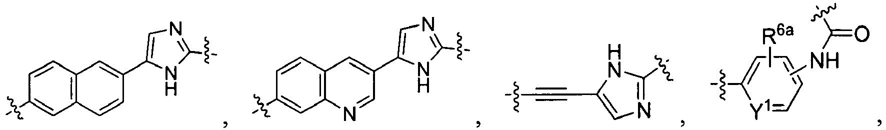 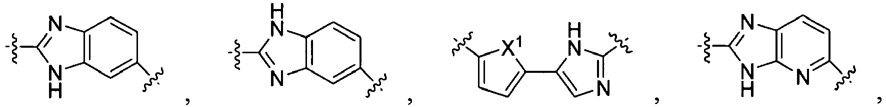 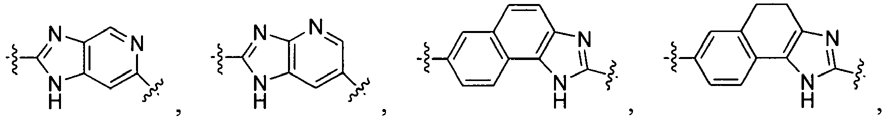 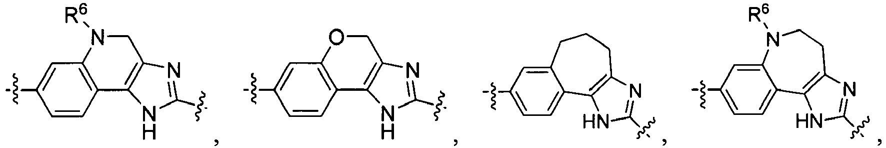 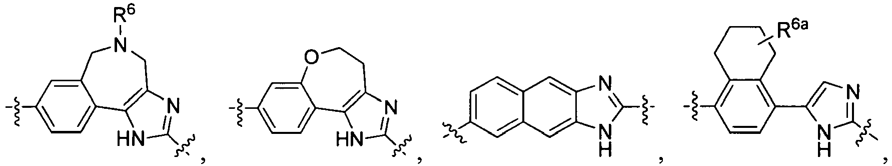 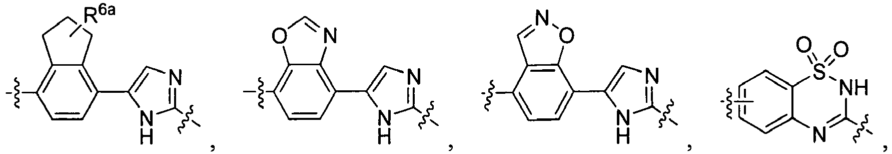 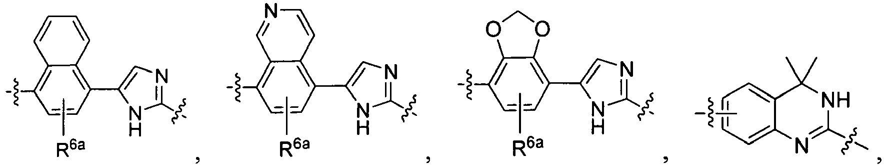 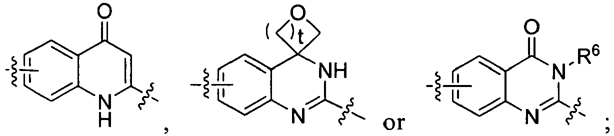
wherein X¹ is O or S;
each Y¹ independently is N or CR⁷;
each R⁶ is independently H, deuterium, C₁₋₄ alkyl, C₁₋₆ haloalkyl, C₁₋₆ hydroxyalkyl, C₁₋₆ aminoalkyl, C₁₋₆ alkoxy-C₁₋₄-alkyl, C₁₋₆ alkylamino-C₁₋₄-alkyl, C₁₋₆ alkylthio-C₁₋₄-alkyl, C₆₋₁₀ aryl-C₁₋₄-alkyl, C₁₋₆ heteroaryl, C₆₋₁₀ aryl, C₂₋₁₀ heterocyclyl or C₃₋₈ carbocyclyl;
each R^{6a} and R⁷ is independently H, deuterium, oxo (=O), hydroxy, amino, F, Cl, Br, I, cyano, R^{13a}R¹³N-, C₁₋₆ alkoxy, C₁₋₆ alkylamino, C₁₋₆ alkyl, C₁₋₆ haloalkyl, C₂₋₆ alkenyl, C₂₋₆ alkynyl, mercapto or nitro; and
each of R¹³ and R^{13a} is independently H, deuterium, C₁₋₆ alkyl, C₂₋₆ heteroalkyl, C₃-₁₀ cycloalkyl, C₂₋₁₀ heterocyclyl, C₆₋₁₀ aryl, C₁₋₉ heteroaryl or C₆₋₁₀ aryl-C₁₋₆-alkyl; with the proviso that where R¹³ and R^{13a} are bonded to the same nitrogen atom, R¹³ and R^{13a}, together with the nitrogen atom they are attached to, optionally form a substituted or unsubstituted 3-8 membered ring or a substituted or unsubstituted spiro or fused bicyclic ring.

4. The compound according to claim 1,
wherein R¹, R² and X-CH together form one of the following monovalent groups: 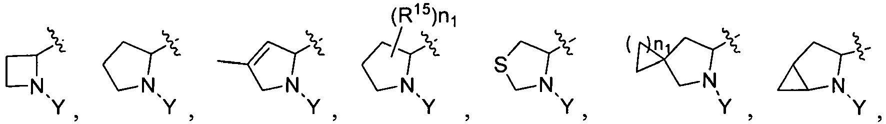 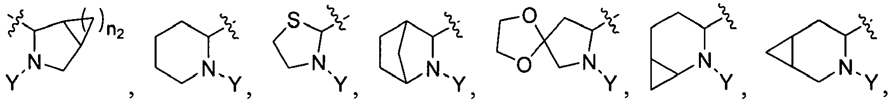 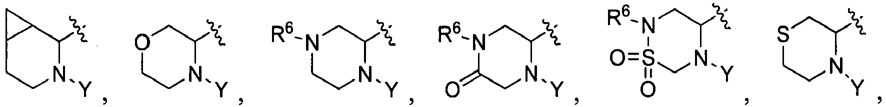 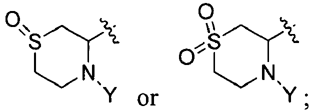 wherein each R¹⁵ is independently H, deuterium, F, Cl, Br, I, cyano, hydroxy, C_{I}-₃ alkyl, C₁₋₃ haloalkyl, C₁₋₃ alkoxy, C₁₋₃ alkylamino, C₁₋₃ alkylthio, C₆₋₁₀ arylamino, C₆₋₁₀ aryloxy, C₁₋₉ heteroaryl, C₁₋₉ heteroaryloxy, C₁₋₉ heteroaryl-C₁₋₃-alkyl or C₂₋₁₀ heterocyclyl;
each R⁶ is independently H, deuterium, C₁₋₃ alkyl, C₁₋₃ haloalkyl, C₁₋₃ hydroxyalkyl, C₁₋₃ aminoalkyl, C₁₋₃ alkoxy-C₁₋₃-alkyl, C₁₋₃ alkylamino-C₁₋₃-alkyl, C₁₋₃ alkylthio-C₁₋₃-alkyl, C₆₋₁₀ aryl-C₁₋₃-alkyl, C₁₋₉ heteroaryl, C₆₋₁₀ aryl, C₂₋₁₀ heterocyclyl or C₃₋₈ carbocyclyl; and
each n₁ and n₂ is independently 1, 2, 3 or 4;
wherein R³, R⁴ and X'-CH together form one of the following monovalent groups: 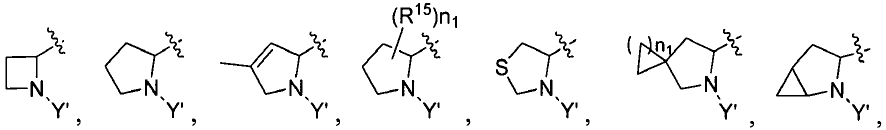 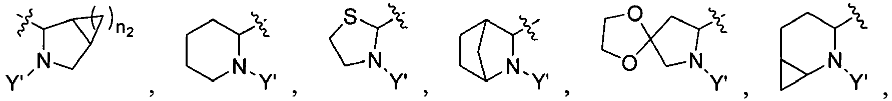 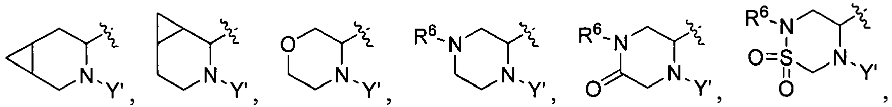 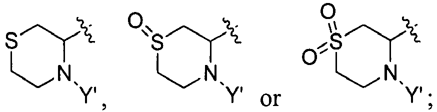 wherein each R¹⁵ is independently H, deuterium, F, Cl, Br, I, cyano, hydroxy, C₁₋₃ alkyl, C₁₋₃ haloalkyl, C₁₋₃ alkoxy, C₁₋₃ alkylamino, C₁₋₃ alkylthio, C₆₋₁₀ arylamino, C₆₋₁₀ aryloxy, C₁₋₉ heteroaryl, C₁₋₉ heteroaryloxy, C₁₋₉ heteroaryl-C₁₋₃-alkyl or C₂₋₁₀ heterocyclyl;
each R⁶ is independently H, deuterium, C₁₋₃ alkyl, C₁₋₃ haloalkyl, C₁₋₃ hydroxyalkyl, C₁₋₃ aminoalkyl, C₁₋₃ alkoxy-C₁₋₃-alkyl, C₁₋₃ alkylamino-C₁₋₃-alkyl, C₁₋₃ alkylthio-C₁₋₃-alkyl, C₆₋₁₀ aryl-C₁₋₃-alkyl, C₁₋₉ heteroaryl, C₆₋₁₀ aryl, C₂₋₁₀ heterocyclyl or C₃₋₈ carbocyclyl; and
each n₁ and n₂ is independently 1, 2, 3 or 4.

5. The compound according to claim 1 having formula (II): 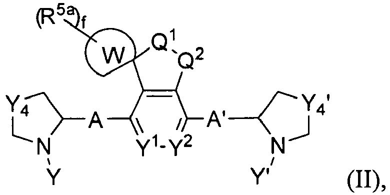 wherein 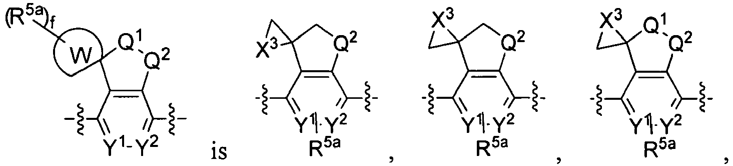 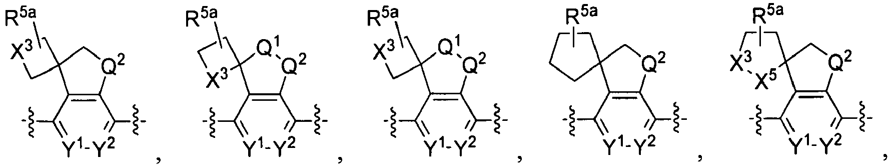 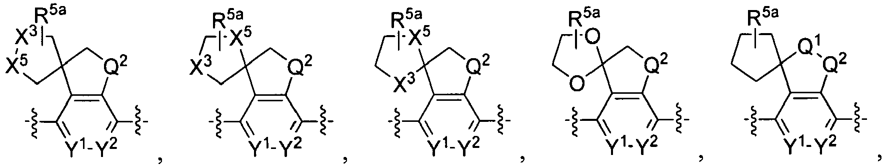 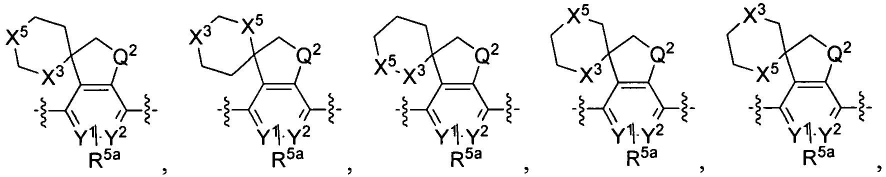 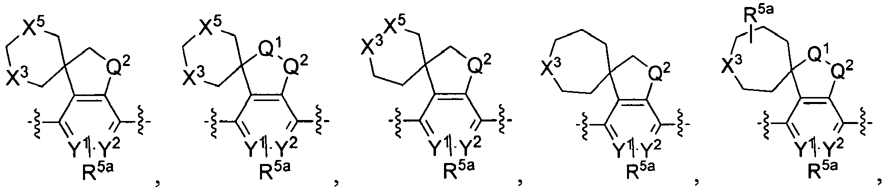 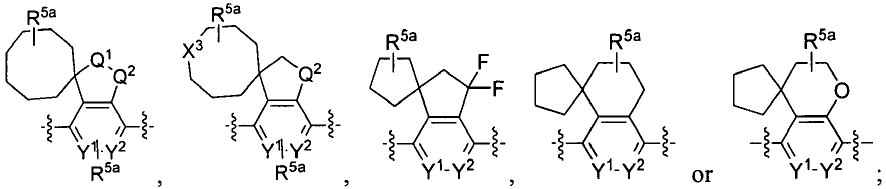
wherein each Q¹ and Q² is independently NR⁶, O, S, C(=O) or (CH₂)ₑ;
e is 0, 1, 2, 3 or 4;
each X¹, X³ and X⁵ is independently O, S, NR⁶ or CR⁷R^{7a};
each Y¹ and Y² is independently N or CR⁷;
each of A and A' is independently a bond, C₁₋₆ alkylene, C₂₋₆ alkenylene, C₃₋₈ cycloalkylene, C₂₋₁₀ heterocyclylalkylene, -(CR⁸R^{8a})ₙ-O-(CR⁸R^{8a})ₚ-, -(CR⁸R^{8a})ₙ-N(R⁵)-(CR⁸R^{8a})ₚ-, -(CR⁸R^{8a})ₙ-S(=O)ᵣ-N(R⁵)-(CR⁸R^{8a})ₚ-, -(CR⁸R^{8a})ₙC(=O)-N(R⁵)-(CR⁸R^{8a})ₚ-, -(CR⁸R^{8a})ₙ-N(R⁵)-C(=O)-N(R⁵)-(CR⁸R^{8a})ₚ-, -(CR⁸R^{8a})ₙ-C(=O)-O-(CR⁸R^{8a})ₚ-, -(CR⁸R^{8a})ₙ-N(R⁵)-S(=O)ᵣ-N(R⁵)-(CR⁸R^{8a})ₚ-, -(CR⁸R^{8a})ₙ-N(R⁵)-C(=O)-O-(CR⁸R^{8a})ₚ-; or each of A and A' is independently 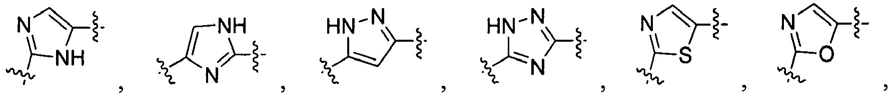 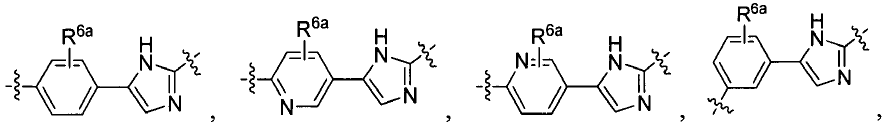 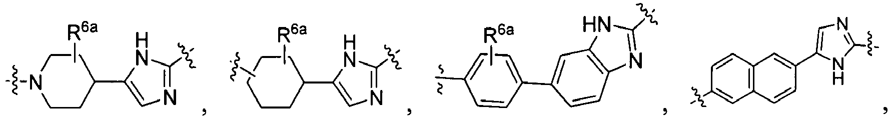 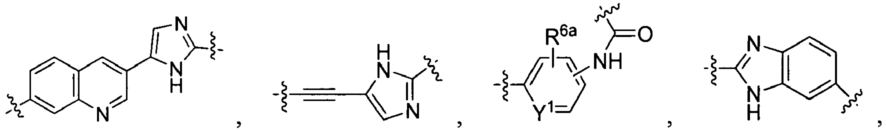 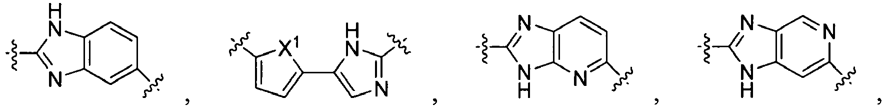 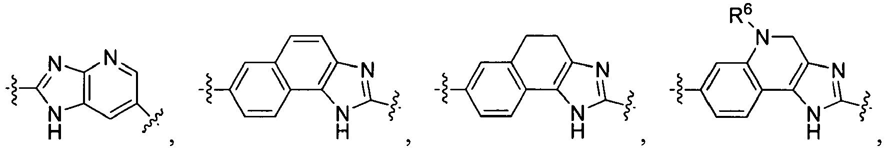 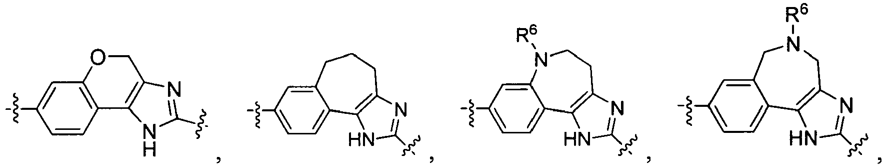 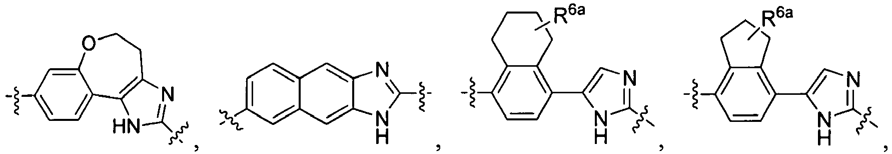 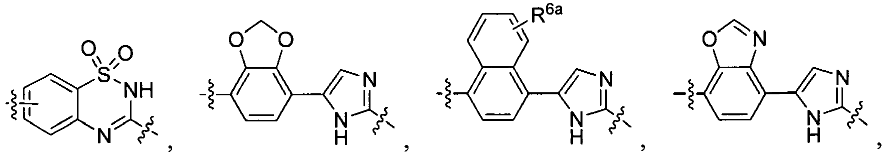 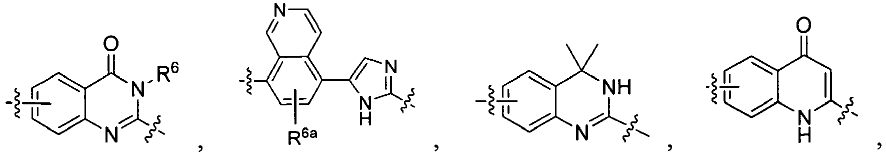 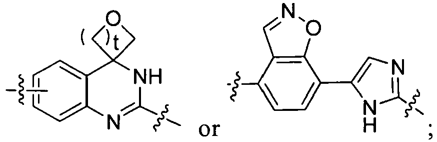
each R⁵ is independently H, deuterium, hydroxy, C₁₋₆ alkyl, C₂₋₆ heteroalkyl, C₃-₁₀ cycloalkyl, C₂₋₁₀ heterocyclyl, C₆₋₁₀ aryl, C₁₋₉ heteroaryl, C₆₋₁₀ aryl-C₁₋₆alkyl, C₁₋₆ alkoxy, C₁₋₆ alkyl-OC(=O)-, C₁₋₆ alkyl-C(=O)-, carbamoyl, C₁₋₆ alkyl-OS(=O)ᵣ-, C₁₋₆ alkyl-S(=O),O-, C₁₋₆ alkyl-S(=O)ᵣ- or aminosulfonyl;
each R^{5a} and R^{6a} is independently H, deuterium, oxo (=O), hydroxy, amino, F, Cl, Br, I, cyano, C₁₋₆ alkylacyl, C₁₋₆ alkylacyloxy, C₁₋₆ alkoxyacyl, C₁₋₆ alkylsulfonyl, C₁₋₆ alkoxysulfonyl, C₁₋₆ alkylsulfinyl, C₁₋₆ alkylsulfonyloxy, C₁₋₆ alkylsulfinyloxy, C₁₋₆ alkoxy, C₁₋₆ alkyl, C₆₋₁₀ aryl, -CF₃, -OCF₃, mercapto, nitro, C₁₋₆ alkylamino, C₃₋₁₀ cycloalkyl or C₆₋₁₀ aryloxy;
each R⁶ is independently H, deuterium, R¹³R^{13a}NC(=O)-, R¹³OC(=O)-, R¹³C(=O)-, R¹³R^{13a}NS(=O)-, R¹³OS(=O)-, R¹³S(=O)-, R¹³R^{13a}NS(=O)₂-, R¹³OS(=O)₂-, R¹³S(=O)₂-, C₁₋₆ aliphatic, C₁₋₆-alkoxy-C₁₋₆-aliphatic, C₁₋₆ alkylamino-C₁₋₆-aliphatic, C₆₋₁₀ aryl-C₁₋₆-aliphatic, C₁₋₉ heteroaryl-C₁₋₆-aliphatic, C₂₋₁₀ heterocyclyl-C₁₋₆-aliphatic, C₃₋₁₀ cycloalkyl-C₁₋₆-aliphatic, C₆₋₁₀ aryl, C₁₋₉ heteroaryl, C₂₋₁₀ heterocyclyl or C₃₋₁₀ carbocyclyl;
each R⁷ and R^{7a} is independently H, deuterium, F, Cl, Br, I, C₁₋₆ aliphatic, C₂₋₆ heteroalkyl, C₁₋₆ alkoxy-C₁₋₆-aliphatic, C₁₋₆ alkylamino-C₁₋₆-aliphatic, C₆₋₁₀ aryl-C₁₋₆-aliphatic, C₂₋₁₀ heterocyclyl-C₁₋₆-aliphatic, C₃₋₁₀ cycloalkyl-C₁₋₆ aliphatic, C₆₋₁₀ aryl, C₁₋₉ heteroaryl, C₂₋₁₀ heterocyclyl or C₃₋₁₀ carbocyclyl;
each R⁸ and R^{8a} is independently H, deuterium, hydroxy, cyano, nitro, F, Cl, Br, I, C₁₋₆ alkyl, C₂₋₆ heteroalkyl, C₃₋₁₀ cycloalkyl, C₂₋₁₀ heterocyclyl, C₆₋₁₀ aryl, C₁₋₉ heteroaryl, C₆₋₁₀ aryl-C₁₋₆-alkyl, C₁₋₆ alkoxy, C₁₋₆ alkyl-OC(=O)-, C₁₋₆ alkyl-C(=O)-, carbamoyl, C₁₋₆ alkyl-OS(=O)ᵣ-, C₁₋₆ alkyl-S(=O)ᵣO-, C₁₋₆ alkyl-S(=O)ᵣ- or aminosulfonyl;
each R¹³ and R^{13a} is independently H, deuterium, C₁₋₆ alkyl, C₂₋₆ heteroalkyl, C₃₋₁₀ cycloalkyl, C₂₋₁₀ heterocyclyl, C₆₋₁₀ aryl, C₁₋₉ heteroaryl or C₆₋₁₀ aryl-C₁₋₆-alkyl; with the proviso that where R¹³ and R^{13a} are bonded to the same nitrogen atom, R¹³ and R^{13a}, together with the nitrogen atom they are attached to, optionally form a substituted or unsubstituted 3-8 membered ring or a substituted or unsubstituted spiro or fused bicyclic ring;
each n is independently 0, 1, 2 or 3;
each p is independently 0, 1, 2 or 3;
each r is independently 0, 1 or 2; and
each of Y₄ and Y₄' is independently a bond, O, S, -(CH₂)ₙ-, -CH=CH-, -S(=O)r-, -CH₂O-, -CH₂S-, -CH₂F, -CHR^{5a}-, -CR^{5a}R^{6a}-, -CH₂S(=O)ᵣ- or -CH₂N(R⁶)-.

6. The compound according to claim 1 having formula (II'): 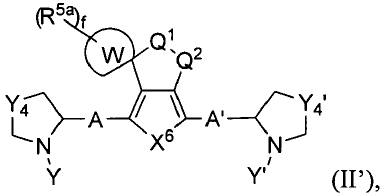 wherein 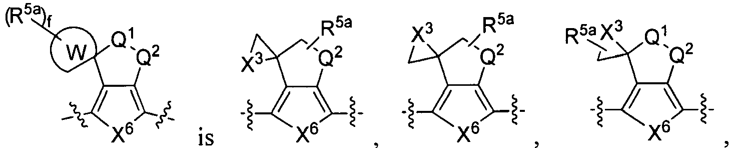 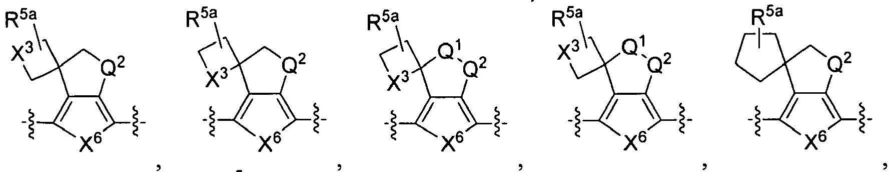 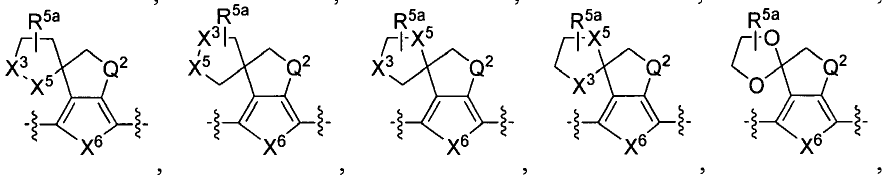 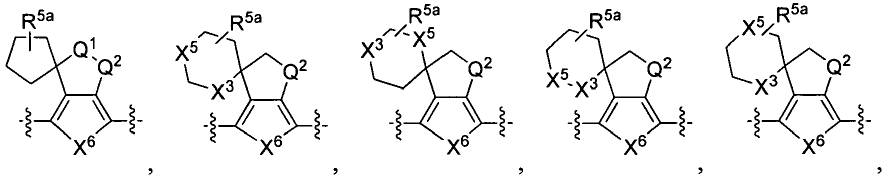 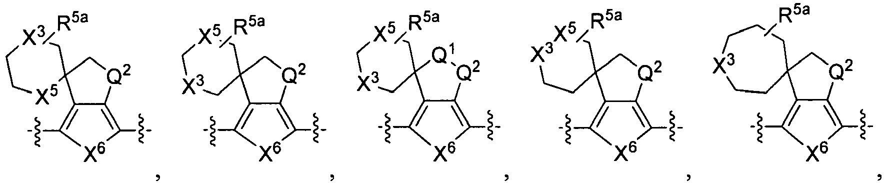 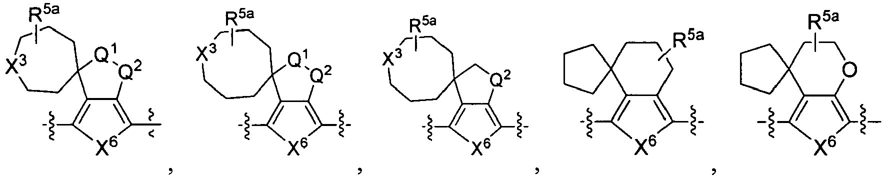 or 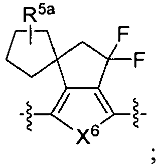
wherein each Q¹ and Q² is independently NR⁶,O, S, C(=O) or (CH₂₎ₑ;
e is 0, 1, 2, 3 or 4;
each X¹, X³ and X⁵ is independently O, S, NR⁶ or CR⁷R^{7a};
each X⁶ is independently CR⁷R^{7a}, O, S or NR⁶_{;}
f is O, 1, 2, 3 or 4;
each of A and A' is independently a bond, C₁₋₆ alkylene, C₂₋₆ alkenylene, C₃₋₈ cycloalkylene, C₂₋₁₀ heterocyclylalkylene, -(CR⁸R^{8a})ₙ-O-(CR⁸R^{8a})ₚ-_{,} -(CR⁸R^{8a})ₙ-N(R⁵)-(CR⁸R^{8a})ₚ-, -(CR⁸R^{8a})ₙ-S(=O)ᵣ-N(R⁵)-(CR⁸R^{8a})ₚ-, -(CR⁸R^{8a})ₙ-C(=O)-N(R⁵)-(CR⁸R^{8a})ₚ-, -(CR⁸R^{8a})ₙ-N(R⁵)-C(=O)-N(R⁵)-(CR⁸R^{8a})ₚ-, -(CR⁸R^{8a})ₙ-C(=O)-O-(CR⁸R^{8a})ₚ-, -(CR⁸R^{8a})ₙ-N(R⁵)-S(=O)ᵣ-N(R⁵)-(CR⁸R^{8a})ₚ-, -(CR⁸R^{8a})ₙ-N(R⁵)-C(=O)-O-(CR⁸R^{8a})ₚ-; or each of A and A' is independently 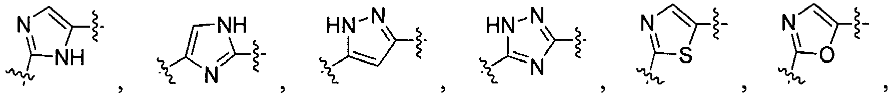 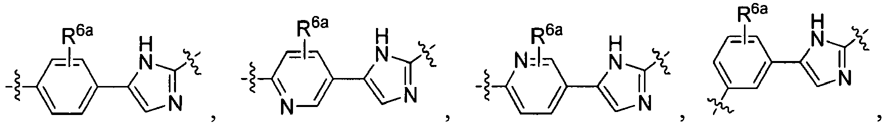 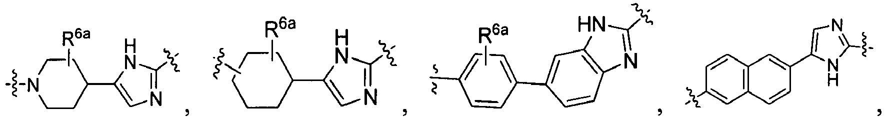 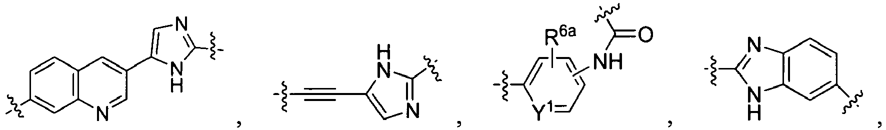 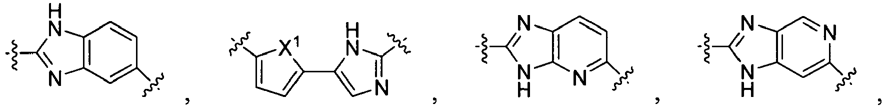 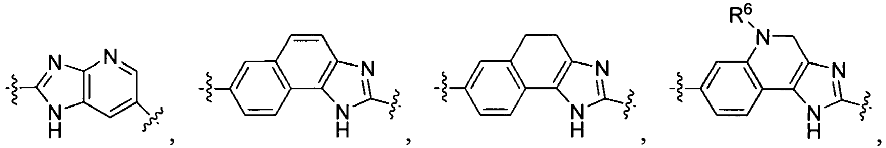 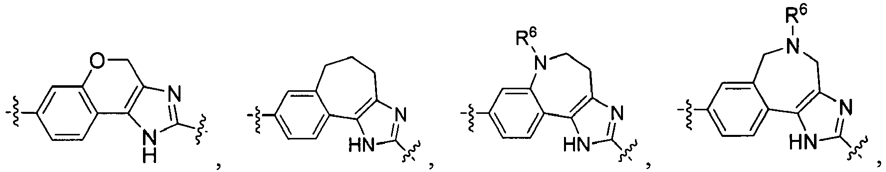 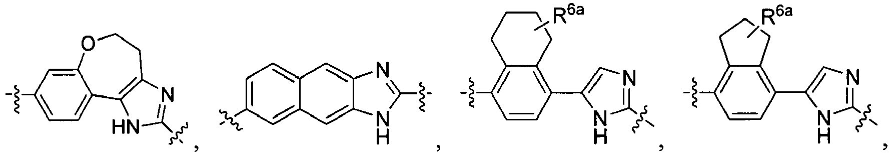 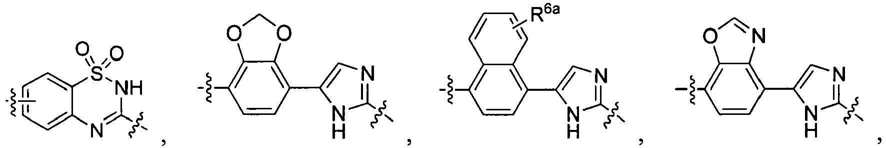 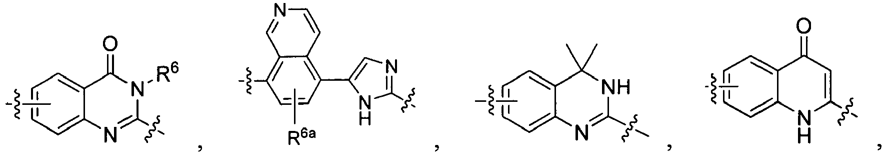 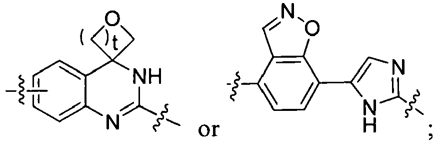
each Y¹ is independently N or CR⁷;
each R⁵ is independently H, deuterium, hydroxy, C₁₋₆ alkyl, C₂₋₆ heteroalkyl, C₃₋₁₀ cycloalkyl, C₂₋₁₀ heterocyclyl, C₆₋₁₀ aryl, C₁₋₉ heteroaryl, C₆₋₁₀ aryl-C₁₋₆-alkyl, C₁₋₆ alkoxy, C₁₋₆ alkyl-OC(=O)-, C₁₋₆ alkyl-C(=O)-, carbamoyl, C₁₋₆ alkyl-OS(=O)ᵣ-, C₁₋₆ alkyl-S(=O)ᵣO-, C₁₋₆ alkyl-S(=O)ᵣ or aminosulfonyl;
each R^{5a} and R^{6a} is independently H, deuterium, oxo (=O), hydroxy, amino, F, Cl, Br, I, cyano, C₁₋₆ alkylacyl, C₁₋₆ alkylacyloxy, C₁₋₆ alkoxyacyl, C₁₋₆ alkylsulfonyl, C₁₋₆ alkoxysulfonyl, C₁₋₆ alkylsulfinyl, C₁₋₆ alkylsulfonyloxy, C₁₋₆ alkylsulfinyloxy, C₁₋₆ alkoxy, C₁₋₆ alkyl, C₆₋₁₀ aryl, -CF₃, -OCF₃, mercapto, nitro, C₁₋₆ alkylamino, C₃-₁₀ cycloalkyl or C₆₋₁₀ aryloxy;
each R⁶ is independently H, deuterium, R¹³R^{13a}NC(=O)-, R¹³OC(=O)-, R¹³C(=O)-, R¹³R^{13a}NS(=O)-, R¹³OS(=O)-, R¹³S(=O)-, R¹³R^{13a}NS(=O)₂-, R¹³OS(=O)₂-, R¹³S(=O)₂-, C₁₋₆ aliphatic, C₁₋₆ alkoxy-C₁₋₆-aliphatic, C₁₋₆ alkylamino-C₁₋₆-aliphatic, C₆₋₁₀ aryl-C₁₋₆-aliphatic, C₁₋₉ heteroaryl-C₁₋₆-aliphatic, C₂₋₁₀ heterocyclyl-C₁₋₆-aliphatic, C₃₋₁₀ cycloalkyl-C₁₋₆-aliphatic, C₆₋₁₀ aryl, C₁₋₉ heteroaryl, C₂₋₁₀ heterocyclyl or C₃₋₁₀ carbocyclyl;
each R⁷ and R^{7a} is independently H, deuterium, F, Cl, Br, I, C₁₋₆ aliphatic, C₂₋₆ heteroalkyl, C₁₋₆ alkoxy-C₁₋₆-aliphatic, C₁₋₆ alkylamino-C₁₋₆-aliphatic, C₆₋₁₀ aryl-C₁₋₆-aliphatic, C₂₋₁₀ heterocyclyl-C₁₋₆-aliphatic, C₃₋₁₀ cycloalkyl-C₁₋₆-aliphatic, C₆₋₁₀ aryl, C₁₋₉ heteroaryl, C₂₋₁₀ heterocyclyl or C₃₋₁₀ carbocyclyl;
each R⁸ and R^{8a} is independently H, deuterium, hydroxy, cyano, nitro, F, Cl, Br, I, C₁₋₆ alkyl, C₂₋₆ heteroalkyl, C₃₋₁₀ cycloalkyl, C₂₋₁₀ heterocyclyl, C₆₋₁₀ aryl, C₁₋₉ heteroaryl, C₆₋₁₀ aryl-C₁₋₆-alkyl, C_{I}-₆ alkoxy, C₁₋₆ alkyl-OC(=O)-, C₁₋₆ alkyl-C(=O)-, carbamoyl, C₁₋₆ alkyl-OS(=O)ᵣ-, C₁₋₆ alkyl-S(-O)ᵣO-, C₁₋₆ alkyl-S(=O)ᵣ- or aminosulfonyl;
each R¹³ and R^{13a} is independently H, deuterium, C₁₋₆ alkyl, C₂₋₆ heteroalkyl, C₃₋₁₀ cycloalkyl, C₂₋₁₀ heterocyclyl, C₆₋₁₀ aryl, C₁₋₉ heteroaryl or C₆₋₁₀ aryl-C₁₋₆-alkyl; with the proviso that where R¹³ and R^{13a} are bonded to the same nitrogen atom, R¹³ and R^{13a}, together with the nitrogen atom they are attached to, optionally form a substituted or unsubstituted 3-8 membered ring or a substituted or unsubstituted spiro or fused bicyclic ring;
each n is independently 0, 1, 2 or 3;
each p is independently 0, 1, 2 or 3;
each r is independently 0, 1 or 2; and
each of Y₄ and Y₄' is independently a bond, O, S, -(CH₂)ₙ₋, -CH=CH-, -S(=O)ᵣ-_{,} -CH₂O-, -CH₂S-, -CH₂F-, -CHR^{5a}-, -CR^{5a}R^{6a}-, -CH₂S(=O)ᵣ- or -CH₂N(R⁶)-.

7. The compound according to claim 5 having formula (III), or Formula (IV), or Formula (V): 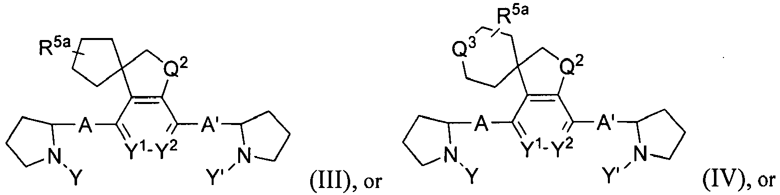 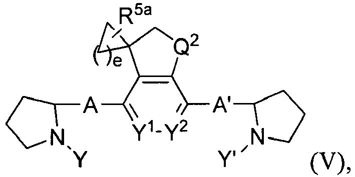
wherein each of Q² and Q³ is independently O, S, C(=O), NR⁶ or CH₂; and
wherein e is 1, 2, 3 or 4.

8. The compound according to claim 6 having formula (III'), or Formula (IV'), or Formula (V'): 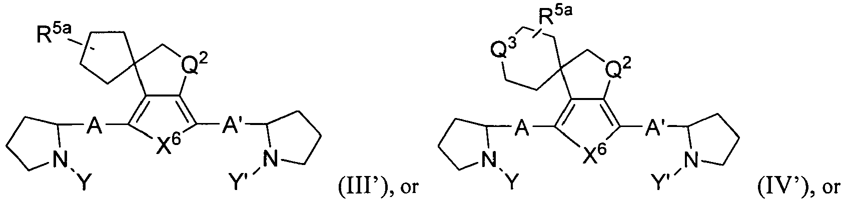 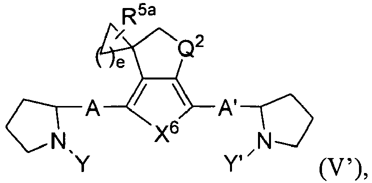
wherein each of Q² and Q³ is independently O, S, C(=O), NR⁶ or CH₂; and
wherein e is 1, 2, 3 or 4.

9. The compound according to claim 1, wherein each of Y and Y' is independently a group derived from an α-amino acid and the group derived from an α-amino acid is optionally substituted with one or more substituents, wherein the substituent is deuterium, F, Cl, Br, I, hydroxy, cyano, alkoxycarbonyl, aryloxycarbonyl, heteroaryloxycarbonyl or heterocyclylcarbonyl;
wherein the α-amino acid is isoleucine, leucine, lysine, methionine, phenylalanine, threonine, tryptophane, valine, alanine, asparagine, aspartic acid, glutamic acid, glutamine, proline, serine, p-tyrosine, arginine, histidine, cysteine, glycine, sarcosine, *N*,*N*-dimethylglycine, homoserine, norvaline, norleucine, ornithine, homocysteine, homophenylalanine, phenylglycine, o-tyrosine, m-tyrosine or hydroxyproline; or
wherein the α-amino acid is in the D configuration; or
wherein the α-amino acid is in the L configuration.

10. The compound according to any one of claims 1-8, wherein each of Y and Y' is independently -[U-(CR⁹R^{9a})ₜ-N(R¹⁰)-(CR⁹R^{9a})ₜ]ₖ-U-(CR⁹R^{9a})ₜ-N(R¹¹)-(CR⁹R^{9a})ₜ-R¹², -U-(CR⁹R^{9a})=ₜ-R¹² or -[U-(CR⁹R^{9a})ₜ-N(R¹⁰)-(CR⁹R^{9a})ₜ]ₖ-U-(CR⁹R^{9a})ₜ-O-(CR⁹R^{9a})ₜ-R¹²; or
wherein each of Y and Y' is independently -[U-(CR⁹R^{9a})ₜ-N(R¹⁰)-(CR⁹R^{9a})ₜ]ₖ-U-(CR⁹R^{9a})ₜ-N(R¹¹)-(CR⁹R^{9a})ₜ-R¹²; or
wherein each of Y and Y' is independently -U-(CR⁹R^{9a})ₜ-N(R¹⁰)-(CR⁹R^{9a})ₜ-U-(CR⁹R^{9a})ₜ-N(R¹¹)-(CR⁹R^{9a})ₜ-R¹²; or
wherein each of Y and Y' is independently -U-(CR⁹R^{9a})ₜ-N(R¹¹)-(CR⁹R^{9a})ₜ-R¹²; or
wherein each of Y and Y' is independently -[C(=O)-(CR⁹R^{9a})ₜ-N(R¹⁰)-(CR⁹R^{9a})ₜ]ₖ-U-(CR⁹R^{9a})ₜ-N(R¹¹)-(CR⁹R^{9a})ₜ-R¹²; or
wherein each of Y and Y' is independently -C(=O)-(CR⁹R^{9a})ₜ-N(R¹⁰)-(CR⁹R^{9a})ₜ]ₖ-U-(CR⁹R^{9a}),-N(R¹¹)-(CR⁹R^{9a})ₜ-R¹²; or
wherein each of Y and Y' is independently -[C(=O)-(CR⁹R^{9a})ₜ-N(R¹⁰)-(CR⁹R^{9a})ₜ]ₖ-C(=O)-(CR⁹R^{9a})ₜ-N(R¹¹)-(CR⁹R^{9a})ₜ-R¹²; or
wherein each of Y and Y' is independently -C(=O)-(CR⁹R^{9a})ₜ₋N(R¹⁰)-(CR⁹R^{9a})ₜ-C(=O)-(CR⁹R^{9a})ₜ-N(R¹¹)₋(CR⁹R^{9a}₎ₜ₋R¹²_{;} or
wherein each of Y and Y' is independently -C(=O)-(CR⁹R^{9a})ₜ-N(R¹¹)-(CR⁹R^{9a})ₜ-R¹²; or
wherein each of Y and Y' is independently -C(=O)-(CR⁹R^{9a})ₙ-N(R¹¹)-(CR⁹R^{9a})ₙ-C(=O)-R¹³; or
wherein each of Y and Y' is independently -C(=O)-(CR⁹R^{9a})ₙ-N(R¹¹)-C(=O)-R¹³; or
wherein each of Y and Y' is independently -C(=O)-(CR⁹R^{9a})ₙ-N(R¹¹)-(CR⁹R^{9a})ₙ-C(=O)-O-R¹³; or
wherein each of Y and Y' is independently -C(=O)-(CR⁹R^{9a})n-N(R¹¹)-C(=O)-O-R¹³; or
wherein each of Y and Y' is independently -U-(CR⁹R^{9a})ₜ-R¹²; or
wherein each of Y and Y' is independently -C(=O)-(CR⁹R^{9a})ₜ-R¹²; or wherein each of Y and Y' is independently -[U-(CR⁹R^{9a})ₜ-N(R¹⁰)-(CR⁹R^{9a})ₜ]ₖ-U-(CR⁹R^{9a})ₜ-O-(CR⁹R^{9a})ₜ-R¹²; or
wherein each of Y and Y' is independently -U-(CR⁹R^{9a})ₜ-N(R¹⁰)-(CR⁹R^{9a})ₜ-U-(CR⁹R^{9a})ₜ-O-(CR⁹R^{9a})ₜ-R¹²; or
wherein each of Y and Y' is independently -C(=O)-(CR⁹R^{9a})ₜ-N(R¹⁰)-(CR⁹R^{9a})ₜ-C(=O)-(CR⁹R^{9a})ₜ-O-(CR⁹R^{9a})ₜ-R¹²; or
wherein each of Y and Y' is independently -U-(CR⁹R^{9a})ₜ-O-(CR⁹R^{9a})ₜ-R¹²_{;} or
wherein each of Y and Y' is independently -C(=O)-(CR⁹R^{9a})ₜ-O-(CR⁹R^{9a})ₜ-R¹²_{;} or
wherein each of Y and Y' is independently -C(=O)-(CR⁹R^{9a})ₙ-N(R¹¹)-R¹², wherein R¹¹ and R¹², together with the atom they are attached to, form a 4-7 membered ring.

11. The compound according to claim 10, wherein each R⁹, R^{9a}, R¹⁰ and R¹¹ is independently H, deuterium, C₁₋₆ alkyl, C₂₋₆ heteroalkyl, C₃₋₁₀ cycloalkyl, C₂₋₁₀ heterocyclyl, C₆₋₁₀ aryl, C₁₋₉ heteroaryl, C₁₋₆ haloalkyl, C₁₋₆ hydroxyalkyl, C₆₋₁₀ aryl-C₁₋₆-alkyl, C₁₋₉ heteroaryl-C₁₋₆-alkyl, C₂₋₁₀ heterocyclyl-C₁₋₆-alkyl or C₃₋₈ cycloalkyl-C₁₋₆-alkyl;
each R¹² is independently R^{13a}R¹³N-, -C(=O)R¹³, -C(=S)R¹³, -C(=O)-O-R¹³, -C(=O)NR¹³R^{13a}, -OC(=O)NR¹³R^{13a}, -OC(=O)OR¹³, -N(R¹³)C(=O)NR¹³R^{13a}, -N(R¹³)C(=O)OR^{13a}, -N(R¹³)C(=O)-R^{13a}, R¹³R^{13a}N-S(=O)₂-, R¹³S(=O)₂-, R¹³S(=O)₂N(R^{13a})-, R¹³OS(=O)₂-, C₁₋₆ alkyl, C₂₋₆ heteroalkyl, C₃₋₁₀ cycloalkyl, C₂₋₁₀ heterocyclyl, C₆₋₁₀ aryl, C₁₋₉ heteroaryl or C₆₋₁₀ aryl-C₁₋₆-alkyl; or R¹¹ and R¹², together with the atom they are attached to, form a 4-7 membered ring;
each R¹³ and R^{13a} is independently H, deuterium, C₁₋₆ alkyl, C₂₋₆ heteroalkyl, C₃₋₁₀ cycloalkyl, C₂₋₁₀ heterocyclyl, C₆₋₁₀ aryl, C₁₋₆ heteroaryl or C₆₋₁₀ aryl-C₁₋₆-alkyl; with the proviso that where R¹³ and R^{13a} are bonded to the same nitrogen atom, R¹³ and R^{13a}, together with the nitrogen atom they are attached to, optionally form a substituted or unsubstituted 3-8 membered ring or a substituted or unsubstituted spiro or fused bicyclic ring;
each t is independently 0, 1, 2, 3 or 4; and
each k is independently 0, 1 or 2; or
wherein each R⁹, R^{9a}, R¹⁰ and R¹¹ is independently H, deuterium, methyl, ethyl, isopropyl, cyclohexyl, isobutyl or phenyl;
each R¹² is independently -C(=O)R¹³, -C(=O)-O-R¹³, -C(=O)NR¹³R^{13a}, methyl, ethyl, propyl, phenyl, cyclohexyl, morpholinyl or piperidinyl;
or R¹¹ and R¹², together with the atom they are attached to, form a 4-7 membered ring; and
each R¹³ and R^{13a} is independently H, deuterium, methyl, ethyl, propyl, phenyl, cyclohexyl, morpholinyl or piperidinyl.

12. The compound according to claim 5 having formula (VI): 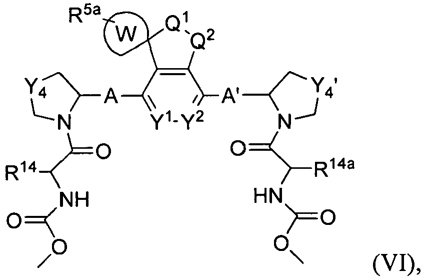
wherein each of R¹⁴ and R^{14a} is independently H, deuterium, C₁₋₆ alkyl, C₁₋₆ haloalkyl, C₁₋₆ hydroxyalkyl, C₂₋₆ heteroalkyl, C₆₋₁₀ aryl, C₁₋₉ heteroaryl, C₂₋₁₀ heterocyclyl, C₃₋₈ cycloalkyl, C₆₋₁₀ aryl-C₁₋₆-alkyl, C₁₋₉ heteroaryl-C₁₋₆-alkyl, C₂₋₁₀ eterocyclyl-C₁₋₆-alkyl or C₃₋₈ cycloalkyl-C₁₋₆-alkyl; and
wherein each of C₁₋₆ alkyl, C₁₋₆ haloalkyl, C₁₋₆ hydroxyalkyl, C₂₋₆ heteroalkyl, C₆₋₁₀ aryl, C₁₋₉ heteroaryl, C₂₋₁₀ heterocyclyl, C₃₋₈ cycloalkyl, C₆₋₁₀ aryl-C₁₋₆-alkyl, C₁₋₉ heteroaryl-C₁₋₆-alkyl, C₂₋₁₀ heterocyclyl-C₁₋₆-alkyl and C₃₋₈ cycloalkyl-C₁₋₆-alkyl is optionally substituted with one or more substituents, wherein the substituent is deuterium, F, Cl, Br, hydroxy or cyano.

13. The compound according to claim 12 having formula (VII): 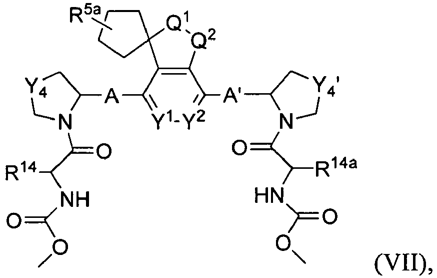
wherein each of R¹⁴ and R^{14a} is independently H, deuterium, C₁₋₃ hydroxyalkyl, methyl, ethyl, isopropyl, isobutyl, *tert*-butyl*,* allyl, propargyl, trifluoroethyl, phenyl, pyranyl, morpholinyl, -NR¹³R^{13a}, benzyl, piperazinyl, cyclopentyl, cyclopropyl, cyclohexyl or C₁₋₉ heteroaryl; and
wherein each of methyl, ethyl, isopropyl, isobutyl, *tert*-butyl, allyl, propargyl, trifluoroethyl, phenyl, pyranyl, morpholinyl, benzyl, piperazinyl, cyclopentyl, cyclopropyl and cyclohexyl is optionally substituted with one or more substituents, wherein the substituent is deuterium, F, Cl, Br, hydroxy or cyano.

14. The compound according to claim 1 having formula (VIII) or formula (IX): 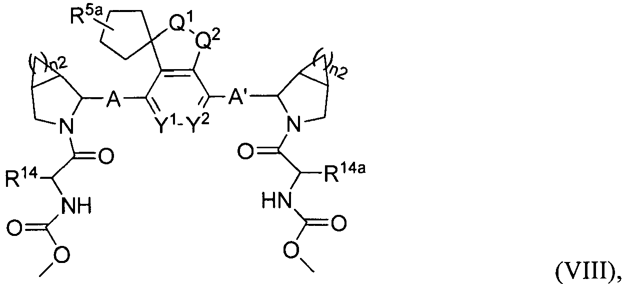 or 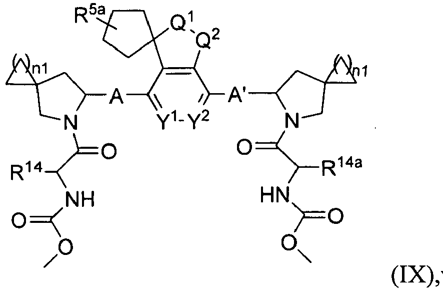
wherein each of R¹⁴ and R^{14a} is independently H, deuterium, C₁₋₆ alkyl, C₁₋₆ haloalkyl, C₁₋₆ hydroxyalkyl, C₂₋₆ heteroalkyl, C₆₋₁₀ aryl, C₁₋₉ heteroaryl, C₂₋₁₀ heterocyclyl, C₃₋₈ cycloalkyl, C₆₋₁₀ aryl-C₁₋₆-alkyl, C₁₋₉ heteroaryl-C₁₋₆-alkyl, C₂₋₁₀ heterocyclyl-C₁₋₆-alkyl or C₃₋₈ cycloalkyl-C₁₋₆-alkyl;
wherein each of ₁₋₆ alkyl, C₁₋₆ haloalkyl, C₁₋₆ hydroxyalkyl, C₂₋₆ heteroalkyl, C₆₋₁₀ aryl, C₁₋₉ heteroaryl, C₂₋₁₀ heterocyclyl, C₃₋₈ cycloalkyl, C₆₋₁₀ aryl-C₁₋₆-alkyl, C₁₋₉ heteroaryl-C₁₋₆-alkyl, C₂₋₁₀ heterocyclyl-C₁₋₆-alkyl and C₃₋₈ cycloalkyl-C₁₋₆-alkyl is optionally substituted with one or more substituents, wherein the substituent is deuterium, F, Cl, Br, hydroxy or cyano; and
each n₁ and n₂ is independently 1, 2, 3 or 4.

15. The compound according to claim 1 having formula (X): 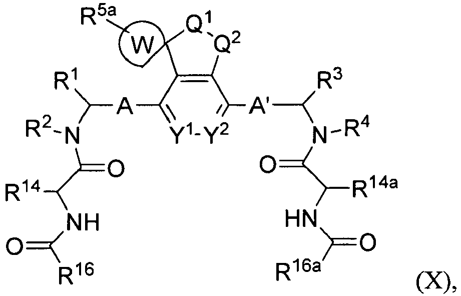
wherein R^{5a} is H, deuterium, methyl, ethyl, F, Cl, Br or I;
Q¹ is CH₂, C(=O), O, S or NR⁶;
Q² is CH₂, C(=O), CF₂, O, NR⁶ or S;
each of Y¹ and Y² is independently N or CR⁷;
each R⁶ and R⁷ is independently H, deuterium, methyl, ethyl, isopropyl, phenyl or cyclohexyl;
each of R¹⁴ and R^{14a} is independently H, deuterium, methyl, ethyl, phenyl, cyclohexyl, 1-methylpropyl, isopropyl or *tert*-butyl;
each of R¹⁶ and R^{16a} is independently hydroxy, methoxy, ethoxy, phenoxy, 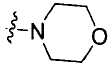 or *tert*-butoxy;
wherein each of methyl, ethyl, phenyl, cyclohexyl, 1-methylpropyl, isopropyl, methoxy, ethoxy, *tert*-butoxy and *tert-*butyl is optionally substituted with one or more substituents, wherein the substituent is deuterium, F, Cl, Br, hydroxy or cyano;
wherein 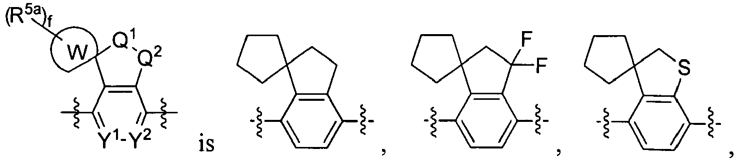 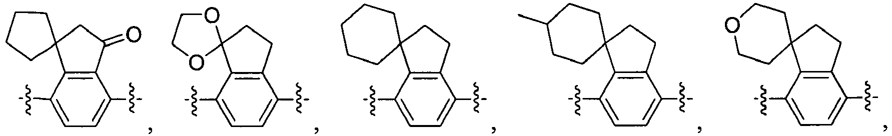 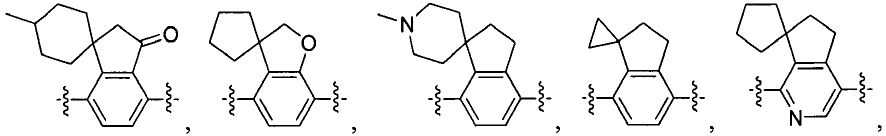 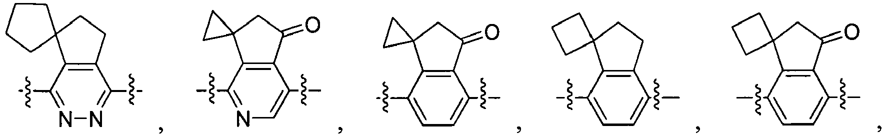 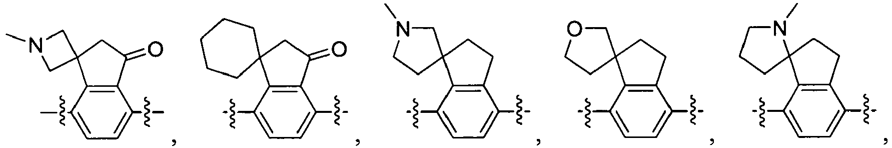 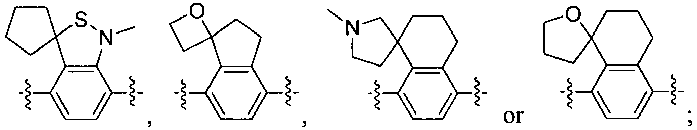
each of A and A' is independently 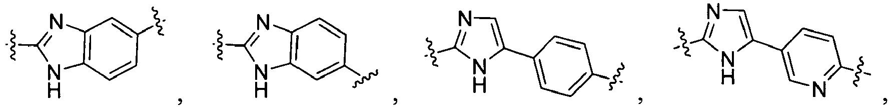 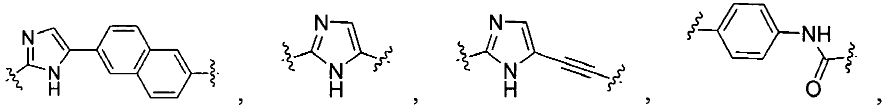 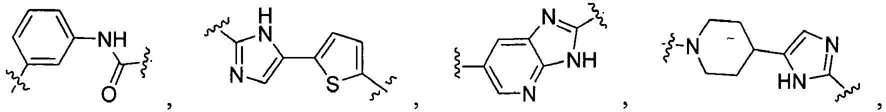 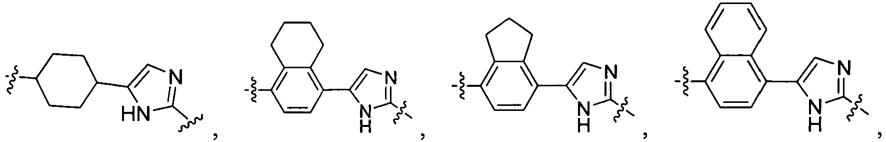 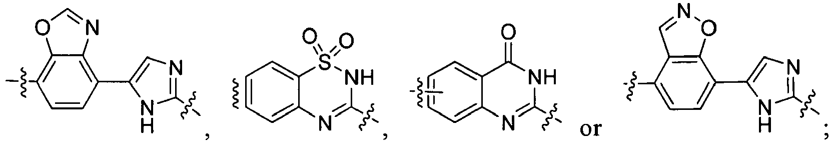
wherein R¹, R² and N-CH together form one of the following divalent groups: 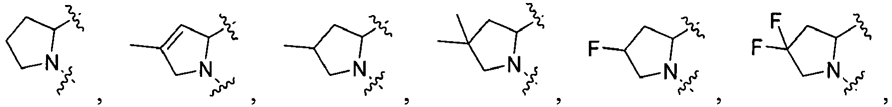 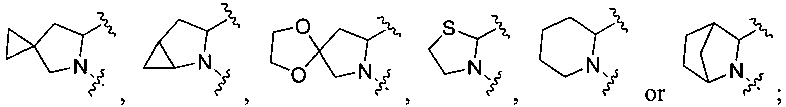 and
wherein R³, R⁴ and N-CH together form one of the following divalent groups:

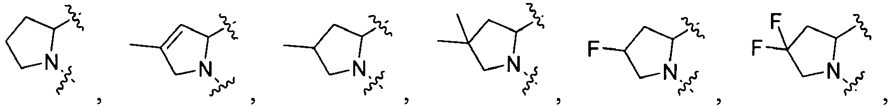

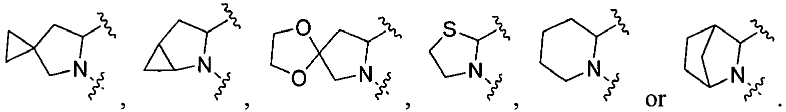

16. The compound according to claim 15 having formula (XI): 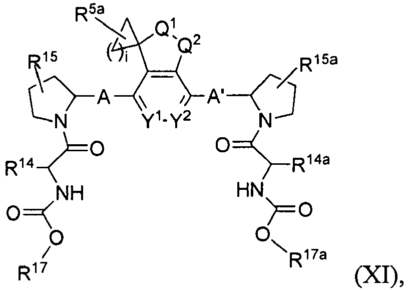
wherein i is 1, 2, 3 or 4;
R^{5a} is H, deuterium or methyl;
each of Q¹ and Q² is independently (CH₂)ₑ, CF₂, S, NR⁶, O or C(=O);
e is 0, 1, 2, 3 or 4;
R⁶ is H, deuterium, methyl, ethyl, isobutyl, cyclohexyl, phenyl or isopropyl;
each of R¹⁴ and R^{14a} is independently H, deuterium, methyl, ethyl, isobutyl, cyclohexyl, phenyl or isopropyl;
each of R¹⁵ and R^{15a} is independently H, deuterium, F, Cl, Br, methyl, ethyl, isopropyl or *tert*-butyl; and
each of R¹⁷ and R^{17a} is independently methyl, phenyl or ethyl;
wherein each of methyl, ethyl, phenyl, cyclohexyl, isopropyl, isobutyl and *tert*-butyl is optionally substituted with one or more substituents, wherein the substituent is deuterium, F, Cl, Br, hydroxy or cyano.

17. The compound according to claim 1 having formula (XI'): 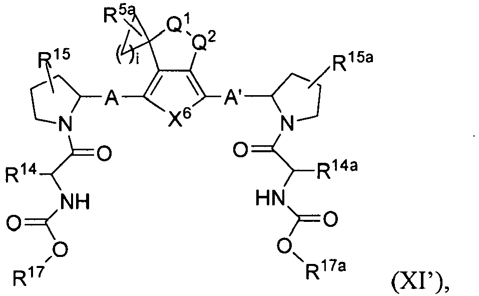
wherein i is 1, 2, 3 or 4;
R^{5a} is H, deuterium or methyl;
each of Q¹ and Q² is independently (CH₂)ₑ, CF₂, S, NR⁶, O or C(=O);
e is 0, 1, 2, 3 or 4;
each R⁶ is independently H, deuterium, methyl, ethyl, isobutyl, cyclohexyl, phenyl or isopropyl;
each of R¹⁴ and R^{14a} is independently H, deuterium, methyl, ethyl, isobutyl, cyclohexyl, phenyl or isopropyl;
each of R¹⁵ and R^{15a} is independently H, deuterium, F, Cl, Br, methyl, ethyl, isopropyl or *tert*-butyl;
each of R¹⁷ and R^{17a} is independently methyl, phenyl or ethyl;
wherein each of methyl, ethyl, phenyl, cyclohexyl, isopropyl, isobutyl and *tert*-butyl is optionally substituted with one or more substituents, wherein the substituent is deuterium, F, Cl, Br, hydroxy or cyano;
X⁶ is independently CH₂, O, S or NR⁶; and
each of A and A' is independently

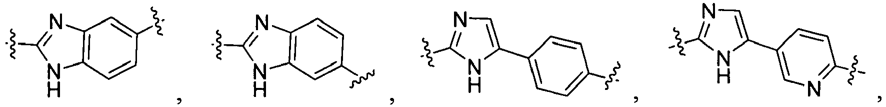

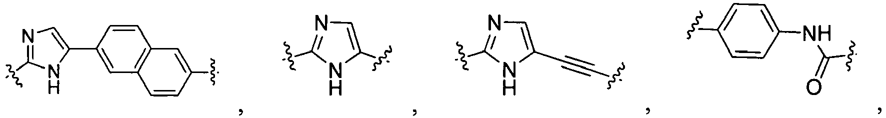

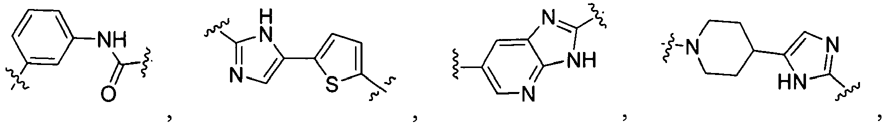

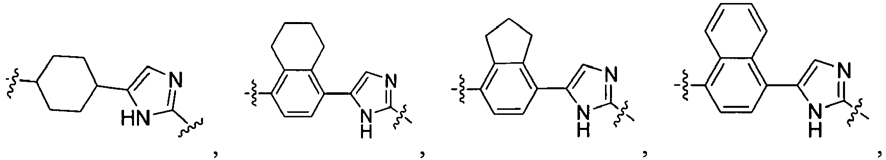

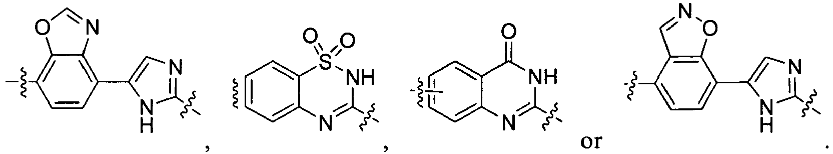

18. The compound of claim 1 having one of the following structures: 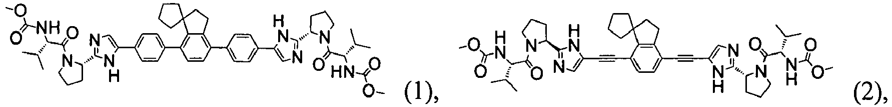 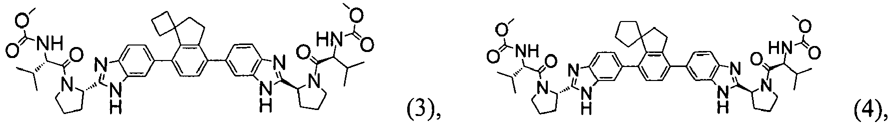 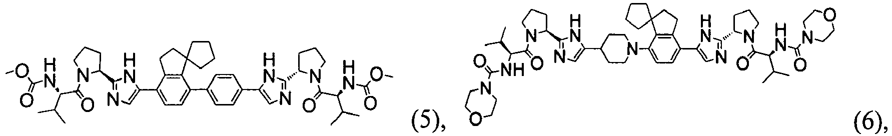 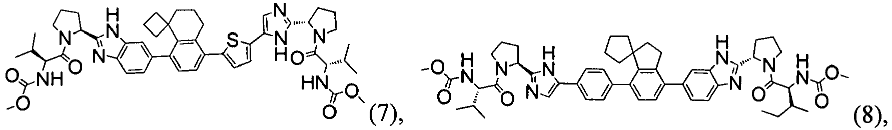 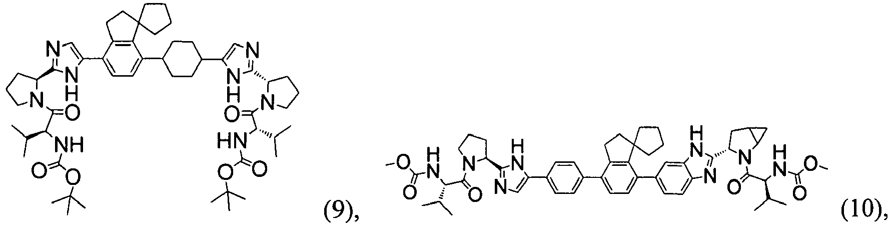 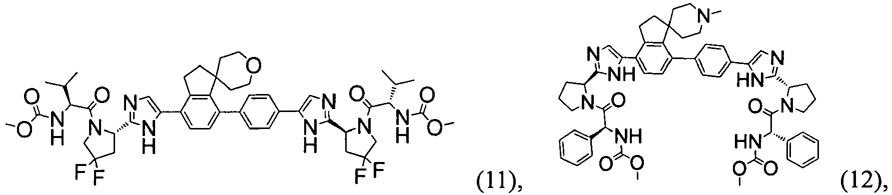 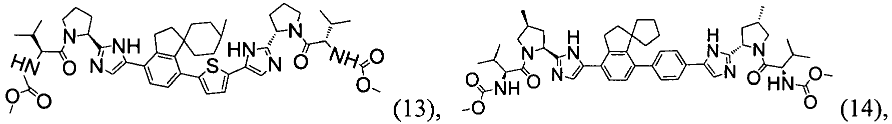 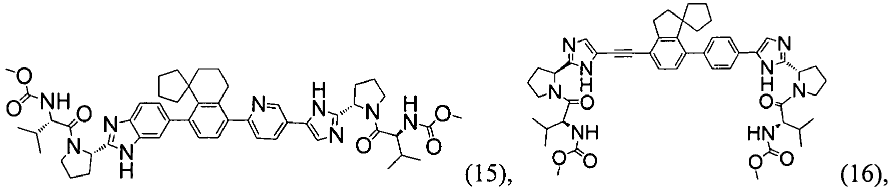 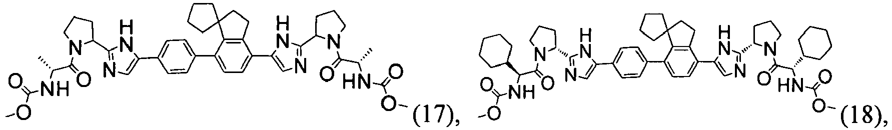 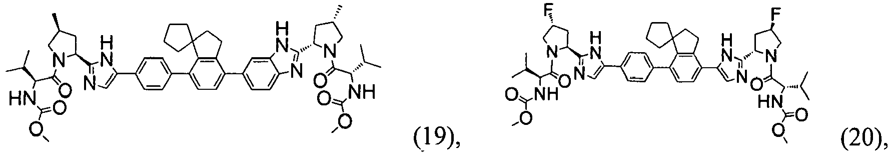 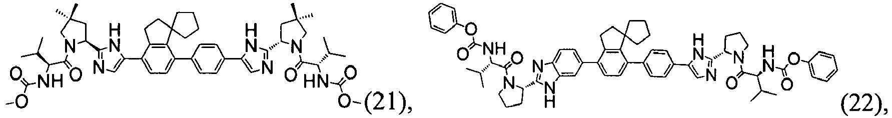 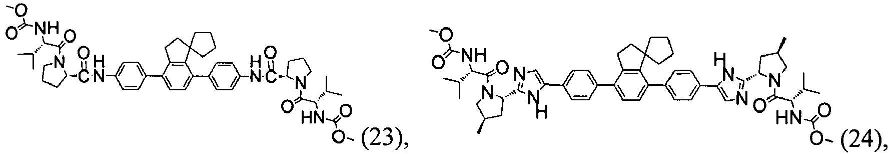 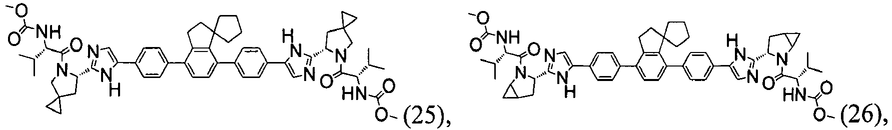 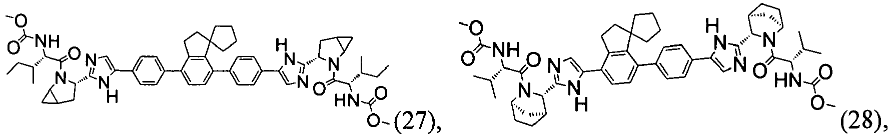 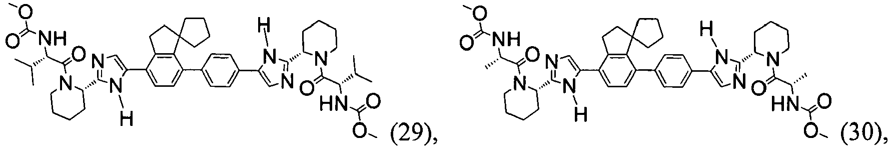 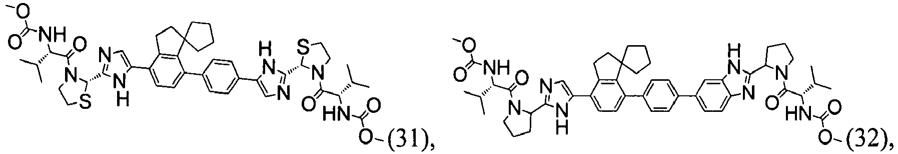 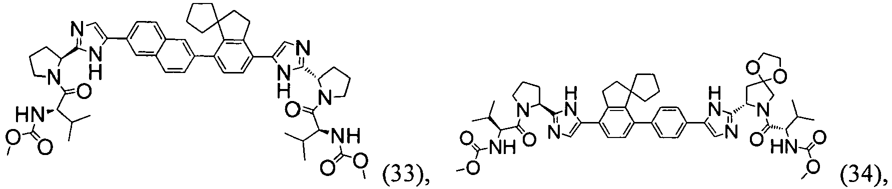 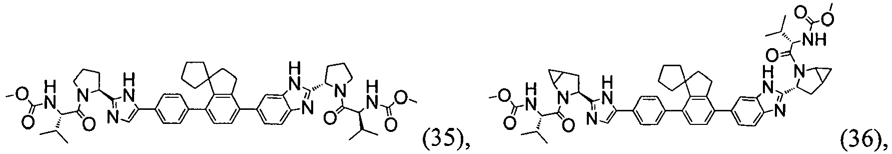 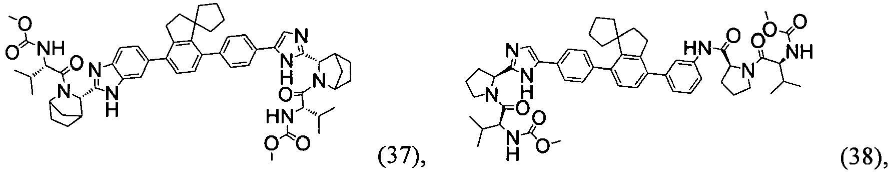 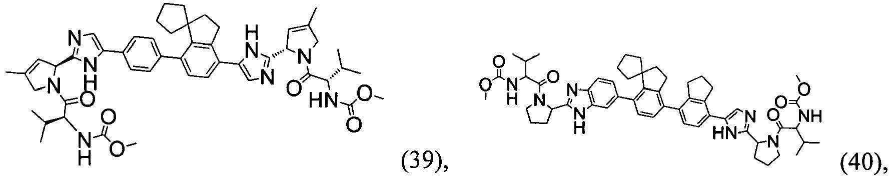 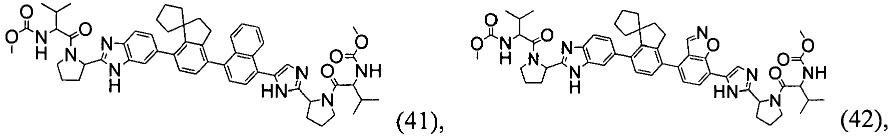 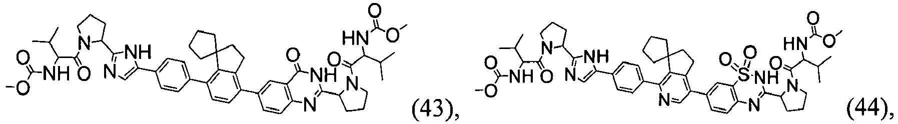 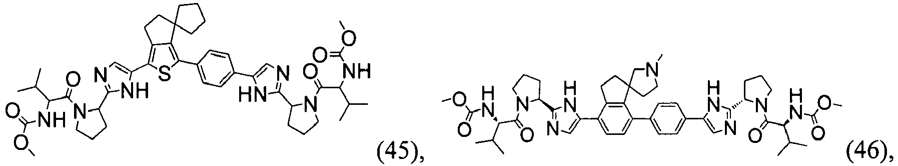 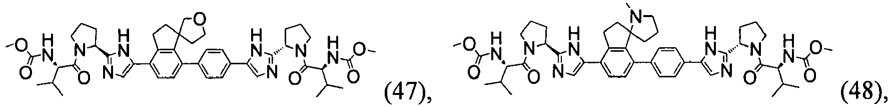 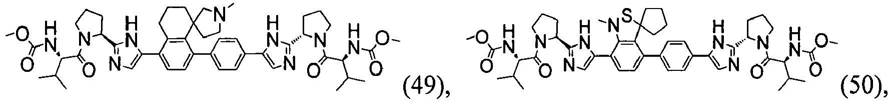 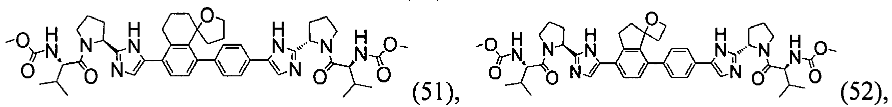 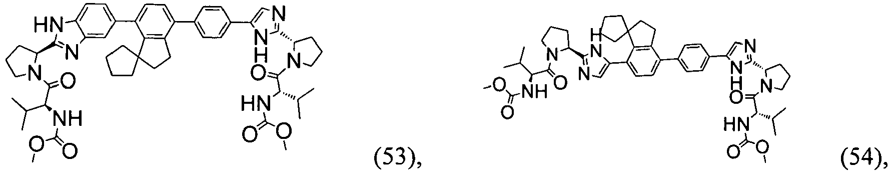 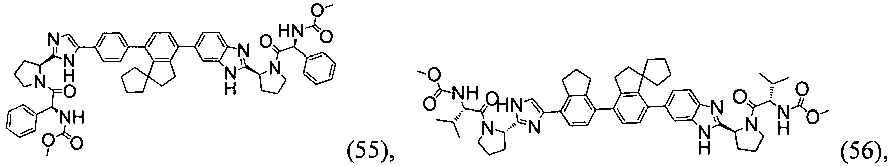 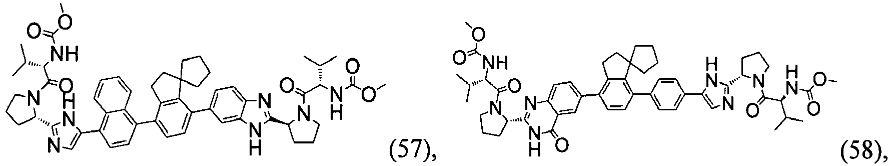 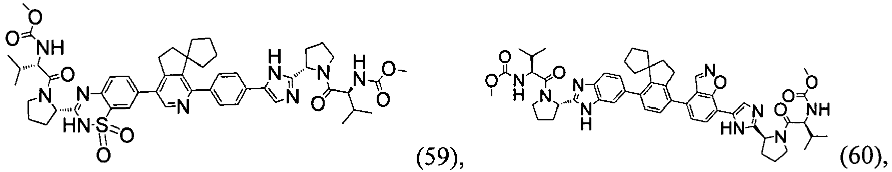 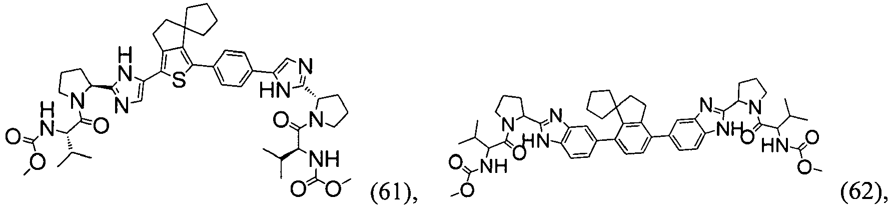 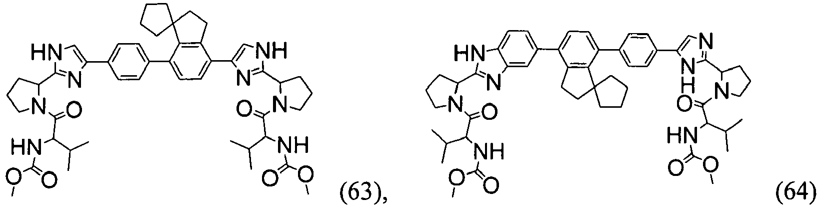 and 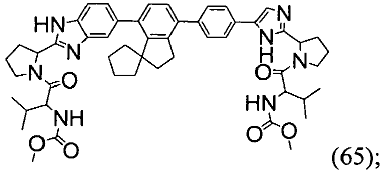 or a stereoisomer, a geometric isomer, a tautomer, an N-oxide, a hydrate, a solvate, or a pharmaceutically acceptable salt thereof.

19. A pharmaceutical composition comprising the compound according to any one of claims 1 to 18; and a pharmaceutically acceptable carrier, excipient, diluent, adjuvant, vehicle or a combination thereof; or
wherein the pharmaceutical composition further comprising an anti-HCV agent;
wherein the anti-HCV agent is interferon, ribavirin, IL-2, IL-6, IL-12, a compound that enhances the development of a type 1 helper T cell response, interfering RNA, anti-sense RNA, imiquimod, an inosine5'-monophosphate dehydrogenase inhibitor, amantadine, rimantadine, ribavirin, bavituximab, human hepatitis C immune globulin (CIVACIR™), boceprevir, telaprevir, erlotinib, daclatasvir, simeprevir, asunaprevir, vaniprevir, faldaprevir, ABT-450, danoprevir, sovaprevir, MK-5172, vedroprevir, BZF-961, GS-9256, narlaprevir, ANA975, ABT-267, EDP239, PPI-668, GS-5816, samatasvir (IDX-719), MK-8742, MK-8325, GSK-2336805, PPI-461, TMC-435, MK-7009, BI-2013335, ciluprevir, BMS-650032, ACH-1625, ACH-1095, VX-985, IDX-375, VX-500, VX-813, PHX-1766, PHX-2054, IDX-136, IDX-316, EP-013420, VBY-376, TMC-649128, R-7128, PSI-7977, INX-189, IDX-184, IDX102, R1479, UNX-08189, PSI-6130, PSI-938, PSI-879, HCV-796, HCV-371, VCH-916, VCH-222, ANA-598, MK-3281, ABT-333, ABT-072, PF-00868554, BI-207127, GS-9190, A-837093, JKT-109, Gl-59728, GL-60667, AZD-2795, TMC647055 or a combination thereof;
wherein the interferon is interferon α-2b, pegylated interferon α, interferon α-2a, pegylated interferon α-2a, consensus interferon-α, interferon γ or a combination thereof; or
wherein the pharmaceutical composition further comprising at least one HCV inhibitor; and
wherein the HCV inhibitor inhibits at least one of HCV replication process and HCV viral protein function, wherein the HCV replication process is a whole viral cycle consisting of HCV entry, uncoating, translation, replication, assembly and egress; and wherein the HCV viral protein is helicase, proteinase, metalloproteinase, serine proteinase, non-structural protein NS4A, non-structural protein NS4B, RNA polymerase of non-structural protein NS5A or non-structural protein NS5B, or an internal ribosome entry site (IRES) and inosine-5'-monophosphate dehydrogenase (IMPDH) required in HCV viral replication.

20. The compound according to any one of claims 1 to 18 or the pharmaceutical composition according to claim 19 for use in inhibiting at least one of HCV replication process and HCV viral protein function, wherein the HCV replication process is a whole viral cycle consisting of HCV entry, uncoating, translation, replication, assembly and egress; and wherein the HCV viral protein is helicase, proteinase, metalloproteinase, serine proteinase, non-structural protein NS4A, non-structural protein NS4B, RNA polymerase of non-structural protein NS5A or non-structural protein NS5B, or an internal ribosome entry site (IRES) and inosine-5'-monophosphate dehydrogenase (IMPDH) required in HCV viral replication.

21. The compound according to any one of claims 1 to 18 or the pharmaceutical composition according to claim 19 for use in preventing, managing, treating or lessening the severity of HCV infection or a HCV disorder in a patient.

## Patentansprüche

1. Verbindung der Formel (I): 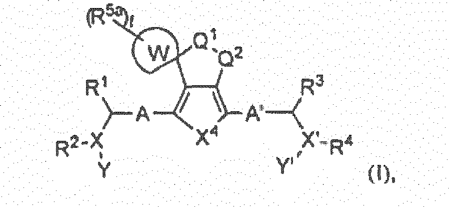 oder ein Stereoisomer, ein geometrisches Isomer, ein Tautomer, ein N-Oxid, ein Hydrat, ein Solvat oder ein pharmazeutisch akzeptables Salz davon, wobei
A und A' jeweils unabhängig für eine Bindung, Alkylen, Alkenylen, Cycloalkylen, Heterocycloalkylen, - (CR⁸R^{8a})ₙ-O-CR⁸R^{8a})ₚ-, -(CR^{8R}R^{8a})ₙ-N(R⁵)-(CR⁸R^{8a})ₚ-, -(CR⁸R^{8a})ₙ-S(=O)ᵣ-N(R⁵)-(CR⁸R^{8a})ₚ-, -(CR⁸R^{8a})ₙ-C(=O)-N(R⁵)-(CR⁸R^{8a})ₚ-, -(CR⁸R^{8a})ₙ-N(R⁵)-C(=O)-N(R⁵)-(CR⁸R^{8a})ₚ-, -(CR⁸R^{8a})ₙ-C(=O)-O-(CR⁸R^{8a})ₚ-, -(CR⁸R^{8a})ₙ-N(R⁵)-S(=O)ᵣ-N(R⁵)-(CR⁸R^{8a})ₚ- oder -(CR⁸R^{8a})ₙ-N(R⁵)-C(=O)-O-(CR⁸R^{8a})ₚ- stehen oder A und A' jeweils unabhängig für 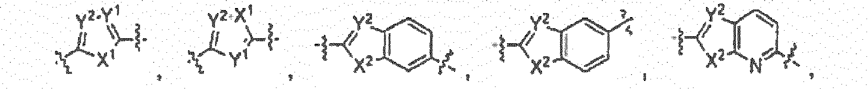 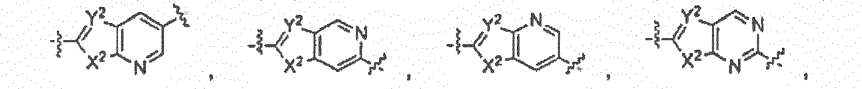 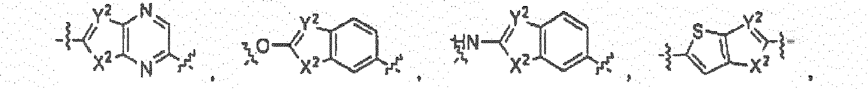 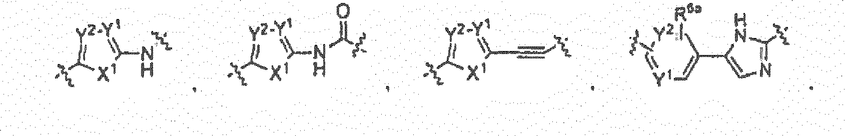 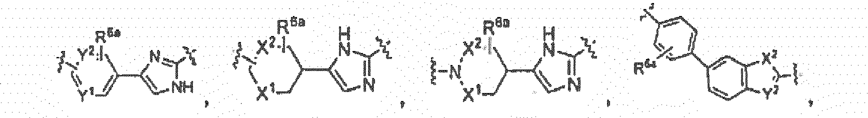 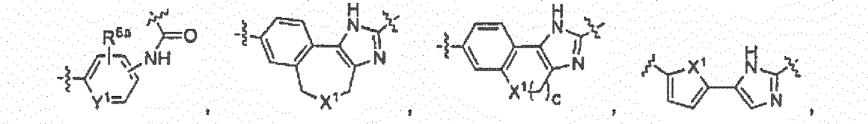 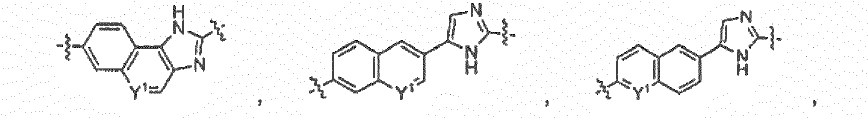 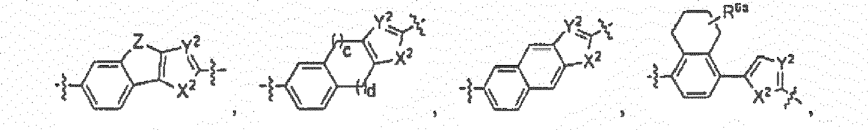 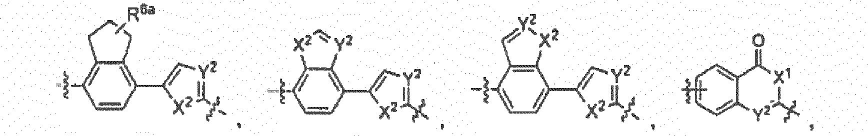 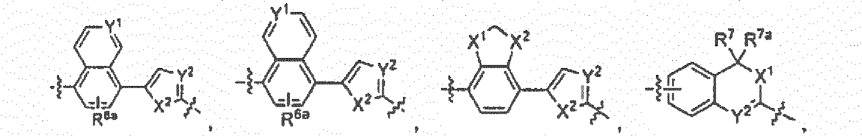 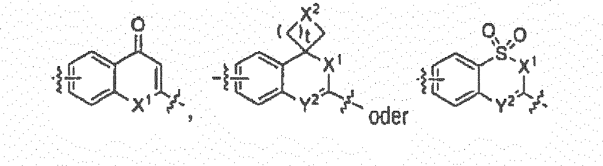 stehen,
wobei X¹ jeweils unabhängig für O, S, NR⁶ oder CR⁷R^{7a} steht;
X² jeweils unabhängig für NR⁶, O oder S steht;
X⁴ für (CR⁷R^{7a})ₙ, -Y¹=Y²-, O, S oder NR⁶ steht;
W für eine Carbocyclyl- oder Heterocyclyl-Gruppe steht;
Y¹ und Y² jeweils unabhängig für N oder CR⁷ stehen;
Z jeweils unabhängig für -(CH₂)ₐ-, -CH=CH-, -N=CH-, - (CH₂)ₐ-N(R⁵)-(CH₂)_{b}- oder -(CH₂)ₐ-O-(CH₂)b- steht, wobei a und b jeweils unabhängig 0, 1, 2 oder 3 sind;
c jeweils unabhängig 1 oder 2 ist;
d jeweils unabhängig 1 oder 2 ist;
n jeweils unabhängig 0, 1, 2 oder 3 ist;
p jeweils unabhängig 0, 1, 2 oder 3 ist;
r jeweils unabhängig 0, 1 oder 2 ist;
f 0, 1, 2, 3 oder 4 ist;
Q¹ und Q² jeweils unabhängig für NR⁶, O, S, C(=O) oder (CR⁷R^{7a})ₑ stehen;
e 0, 1, 2, 3 oder 4 ist;
X und X' jeweils unabhängig für N oder CR⁷ stehen;
Y und Y' jeweils unabhängig für H, Deuterium, Alkyl, Heteroalkyl, Cycloalkyl, Heterocyclyl, Aryl, Heteroaryl, Aralkyl, eine aus einer α-Aminosäure oder einem optischen Isomer davon abgeleitete Gruppe stehen oder Y und Y' jeweils unabhängig für -[U-(CR⁹R^{9a})ₜ-N(R¹⁰)-(CR⁹R^{9a})ₜ]ₖ-U-(CR⁹R^{9a})ₜ-N(R¹¹)-(CR⁹R^{9a})ₜ-R¹², -U-(CR⁹R^{9a})ₜ-R¹² oder -[U-(CR⁹R^{9a})ₜ-N(R¹⁰)-(CR⁹R^{9a})ₜ]ₖ-U-(CR⁹R^{9a})ₜ-O-(CR⁹R^{9a})ₜ-R¹² stehen;
u jeweils unabhängig für -C(=O)-, -C(=S)-, -S(=O)- oder -S(=O)₂- steht;
t jeweils unabhängig 0, 1, 2, 3 oder 4 ist;
k jeweils unabhängig 0, 1 oder 2 ist;
R¹ und R² zusammen mit X-CH, woran sie gebunden sind, gegebenenfalls einen 3- bis 8-gliedrigen Heterocyclus oder Kohlenstoffring, einen annellierten C₅₋₁₂-Bicyclus, einen annellierten C₅₋₁₂-Heterobicyclus, einen C₅-₁₂-Spiro-Bicyclus oder einen C₅₋₁₂-Spiro-Heterobicyclus bilden und R³ und R⁴ zusammen mit X'-CH, woran sie gebunden sind, gegebenenfalls einen 3-bis 8-gliedrigen Heterocyclus oder Kohlenstoffring, einen annellierten C₅₋₁₂-Bicyclus, einen annellierten C₅-₁₂-Heterobicyclus, einen C₅₋₁₂-Spiro-Bicyclus oder einen C₅₋₁₂-Spiro-Heterobicyclus bilden;
R⁵ jeweils unabhängig für H, Deuterium, Alkyl, Heteroalkyl, Cycloalkyl, Heterocyclyl, Aryl, Heteroaryl, Aralkyl, Alkoxy, Alkyl-OC(=O)-, Alkyl-C(=O)-, Carbamoyl, Alkyl-OS(=O)ᵣ, Alkyl-S(=O)ᵣO-, Alkyl-S(=O)ᵣ- oder Aminosulfonyl steht;
R^{5a} jeweils unabhängig für H, Deuterium, Oxo (=O), Hydroxy, Amino, F, Cl, Br, I, Cyano, R^{13a}R¹³N-,-C(=O)NR¹³R^{13a}, -OC(=O)NR¹³R^{13a}, -OC(=O)OR¹³, N(R¹³)C(=O)NR¹³R^{13a}, -N(R¹³)C(=O)OR¹³, -N(R¹³)C(=O)-R¹³, R¹³R^{13a}N-S(=O)₂-, R¹³S(=O)₂-, R¹³S(=O)₂N(R^{13a})-, R^{13a}R¹³N-alkyl, R¹³S(=O)-alkyl, R¹³R^{13a}N-C(=O)-alkyl, R^{13a}R¹³N-alkoxy, R¹³S(=O)-alkoxy, R¹³R^{13a}N-C(=O)-alkoxy, Aryl, Heteroaryl, Alkoxy, Alkylamino, Alkyl, Haloalkyl, Alkenyl, Alkinyl, Heterocyclyl, Cycloalkyl, Mercapto, Nitro, Aralkyl, Arylamino, Heteroarylamino, Arylalkylamino, Heteroarylalkylamino, Heteroaryloxy, Heteroarylalkyl, Arylalkoxy, Heteroarylalkoxy, Heterocyclyloxy, Heterocyclylalkoxy, Heterocyclylamino, Heterocyclylalkylamino oder Aryloxy steht;
R⁶ jeweils unabhängig für H, Deuterium, R¹³R^{13a}NC(=O)-, R¹³OC(=O)-, R¹³C(=O)-, R¹³R^{13a}NS(=O)-, R¹³OS(-O)-, R¹³S(=O)-, R¹³R^{13a}NS(=O)₂-, R¹³OS(=O)₂-, R¹³S(-O)₂-, eine aliphatische, haloaliphatische, hydroxyaliphatische, aminoaliphatische, alkoxyaliphatische, alkylaminoaliphatische, alkylthioaliphatische, arylaliphatische, heteroarylaliphatische, heterocyclylaliphatische, cycloalkylaliphatische, aryloxyaliphatische, heterocyclyloxyaliphatische, cycloalkyloxyaliphatische, arylaminoaliphatische, heterocyclylaminoaliphatische, cycloalkylaminoaliphatische, Aryl-, Heteroaryl-, Heterocyclyl- oder Carbocyclyl-Gruppe steht;
R^{6a} jeweils unabhängig für H, Deuterium, Hydroxy, Amino, F, Cl, Br, I, Cyano, Oxo (=O), R^{13a}R¹³N-,-C(=O)NR¹³R^{13a}, -OC(=O)NR¹³R^{13a}, -OC(=O)OR¹³,-N(R¹³)C(=O)NR¹³R^{13a}, -N(R¹³)C(=O)OR^{13a}, -N(R¹³)C(=O)-R^{13a}, R¹³R^{13a}N-S(=O)₂-, R¹³S(=O)₂-, R¹³S(=O)₂N(R^{13a})-, R^{13a}R¹⁹N-alkyl, R¹³S(=O)-alkyl, R¹³R^{13a}N-C(=O)-alkyl, R^{13a}R¹³N-alkoxy, R¹³S(=O)-alkoxy, R¹³R^{13a}N-C(=O)-alkoxy, Aryl, Heteroaryl, Alkoxy, Alkylamino, Alkyl, Haloalkyl, Alkenyl, Alkynyl, Heterocyclyl, Cycloalkyl, Mercapto, Nitro, Aralkyl, Arylamino, Heteroarylamino, Arylalkylamino, Heteroarylalkylamino, Heteroaryloxy, Heteroarylalkyl, Arylalkoxy, Heteroarylalkoxy, Heterocyclyloxy, Heterocyclylalkoxy, Heterocyclylamino, Heterocyclylalkylamino oder Aryloxy steht;
R⁷ und R^{7a} jeweils unabhängig für H, Deuterium, F, Cl, Br, I, eine aliphatische, Heteroalkyl-, haloaliphatische, hydroxyaliphatische, aminoaliphatische, alkoxyaliphatische, alkylaminoaliphatische, alkylthioaliphatische, arylaliphatische, heteroarylaliphatische, heterocyclylaliphatische, cycloalkylaliphatische, aryloxyaliphatische, heterocyclyloxyaliphatische, cycloalkyloxyaliphatische, arylaminoaliphatische, heterocyclylaminoaliphatische, cycloalkylaminoaliphatische, Aryl-, Heteroaryl-, Heterocyclyl- oder Carbocyclyl-Gruppe stehen;
R⁸ und R^{8a} jeweils unabhängig für H, Deuterium, Hydroxy, Cyano, Nitro, F, Cl, Br, I, Alkyl, Heteroalkyl, Cycloalkyl, Heterocyclyl, Aryl, Heteroaryl, Aralkyl, Alkoxy, Alkyl-OC(=O)-, Alkyl-C(=O)-, Carbamoyl, Alkyl-OS(=O)ᵣ-, Alkyl-S(=O)ᵣO-, Alkyl-S(=O)ᵣ- oder Aminosulfonyl stehen;
R⁹, R^{9a}, R¹⁰ und R¹¹ jeweils unabhängig für H, Deuterium, Alkyl, Heteroalkyl, Cycloalkyl, Heterocyclyl, Aryl, Heteroaryl, Aralkyl, Haloalkyl, Hydroxyalkyl, Heteroarylalkyl, Heterocyclylalkyl oder Cycloalkylalkyl stehen;
R¹² jeweils unabhängig für R^{13a}R¹³N-, -C(=O)R¹³,-C(=S)R¹³, -C(=O)OR¹³, -C(=O)NR¹³R^{13a}, -OC(=O)NR¹³R^{13a},-OC(=O)OR¹³, -N(R¹³)C(=O)NR¹³R^{13a}, -N(R¹³)C(=O)OR^{13a},-N(R¹³)C(=O)-R^{13a}, R¹³R^{13a}N-S(=O)₂-, R¹³S(=O)₂-, R¹³S(=O)₂N(R^{13a})-, R¹³OS(=O)₂-, Alkyl, Heteroalkyl, Cycloalkyl, Heterocyclyl, Aryl, Heteroaryl oder Aralkyl steht;
Oder R¹¹ und R¹² gegebenenfalls verbunden sind, so dass sie einen 4- bis 7-gliedrigen Ring bilden; und
R¹³ und R^{13a} jeweils unabhängig für H, Deuterium, Alkyl, Heteroalkyl, Cycloalkyl, Heterocyclyl, Aryl, Heteroaryl oder Aralkyl stehen; mit der Vorgabe, dass, wenn R¹³ und R^{13a} an dasselbe Stickstoffatom gebunden sind, R¹³ und R^{13a} zusammen mit dem Stickstoffatom, woran sie gebunden sind, gegebenenfalls einen substituierten oder unsubstituierten 3- bis 8-gliedrigen Ring oder einen substituierten oder unsubstituierten Spiro-Ring oder annellierten bicyclischen Ring bilden;
wobei jedes aus Alkylen, Alkenylen, Cycloalkylen, Heterocyclylalkylen, -(CR⁸R^{8a})ₙ-O-(CR⁸R^{8a})ₚ-, (CR⁸R^{8a})ₙ-N(R⁵)-(CR⁸R^{8a})ₚ-, -(CR⁸R^{8a})ₙ-S(=O)ᵣ-N(R⁵)-(CR⁸R^{8a})ₚ-, (CR⁸R^{8a})ₙ-C(=O)-N(R⁵)-(CR⁸R^{8a})ₚ-, -(CR⁸R^{8a})ₙ-N(R⁵)-C(=O)-N(R⁵)-(CR⁸R^{8a})ₚ-, -(CR⁸R^{8a})ₙ-C(=O)-O-(CR⁸R^{8a})ₚ-, (CR⁸R^{8a})ₙ-N(R⁵)-S(=O)ᵣ-N(R⁵)-(CR⁸R^{8a})ₚ-, -(CR⁸R^{8a})ₙ-N(R⁵)-C(=O)-O-(CR⁸R^{8a})ₚ-, -[U-(CR⁹R^{9a})ₜ-N(R¹⁰)-(CR⁹R^{9a})ₜ]ₖ-U-(CR⁹R^{9a})ₜ-N(R¹¹)-(CR⁹R^{9a})ₜ-R¹², -U-(CR⁹R^{9a})ₜ-R¹², -[U-(CR⁹R^{9a})ₜ-N(R¹⁰)-(CR⁹R^{9a})ₜ]ₖ-U-(CR⁹R^{9a})ₜ-O-(CR⁹R^{9a})ₜ-R¹², NR⁶, CR⁷R^{7a}, CR⁷, -(CH₂)ₐ-, -CH=CH-, -N=CH-, -(CH₂)ₐ-N(R⁵)-(CH₂)_{b}-, -(CH₂)ₐ-O-(CH₂)_{b}-, R^{13a}R¹³N-, -C(=O)R¹³, - C(=S)R¹³, -C(=O)OR¹³, -C(=O)NR¹³R^{13a}, -OC(=O)NR¹³R^{13a},-OC(=O)OR¹³, -N(R¹³)C(=O)NR¹³R^{13a}, -N(R¹³)C(=O)OR^{13a},-N(R¹³)C(=O)-R^{13a}, R¹³R^{13a}N-S(=O)₂-, R¹³S(=O)₂-, R¹³S(=O)₂N(R^{13a})-, R¹³OS(=O)₂-, Alkyl-OC(=O)-, Alkyl-C(=O)-, Alkyl-OS(=O)ᵣ, Alkyl-S(=O)rO-, Alkyl-S(=O)ᵣ-, R¹³R^{13a}NC(=O)-, R¹³OC(=O)-, R¹³C(=O)-, R¹³R^{13a}NS(=O)-, R¹³OS(=O)-, R¹³S(=O)-, R^{13a}R¹³N-alkyl, R¹³S(=O)-alkyl, R¹³R^{13a}N-C(=O)-alkyl, R^{13a}R¹³N-alkoxy, R¹³S(=O)-alkoxy, R¹³R^{13a}N-C(=O)-alkylamino, Alkyl, Heteroalkyl, Carbocyclyl, Cycloalkyl, Heterocyclyl, Aryl, Heteroaryl, Aralkyl, einer aus α-Aminosäure abgeleiteten Gruppe, annelliertem C₅₋₁₂-Bicyclus, annelliertem C₅₋₁₂₋Heterobicyclus, C₅₋₁₂₋Spiro-Bicyclus, C₅₋₁₂₋Spiro-Heterobicyclus, Alkoxy, aliphatischer, haloaliphatischer, hydroxyaliphatischer, aminoaliphatischer, alkoxyaliphatischer, alkylaminoaliphatischer, alkylthioaliphatischer, arylaliphatischer, heteroarylaliphatischer, heterocyclylaliphatischer, cycloalkylaliphatischer, aryloxyaliphatischer, heterocyclyloxyaliphatischer, cycloalkyloxyaliphatischer, arylaminoaliphatischer, heterocyclylaminoaliphatischer, cycloalkylaminoaliphatischer Gruppe, Haloalkyl, Alkenyl, Alkynyl, Arylamino, Heteroarylamino, Arylalkylamino, Heteroarylalkylamino, Heteroaryloxy, Heteroarylalkyl, Arylalkoxy, Heteroarylalkoxy, Heterocyclyloxy, Heterocyclylalkoxy, Heterocyclylamino, Heterocyclylalkylamino und Aryloxy gegebenenfalls mit einem oder mehreren Substituenten substituiert ist, wobei der Substituent Deuterium, Hydroxy, Amino, Halogen, Cyano, Aryl, Heteroaryl, Alkoxy, Alkylamino, Alkylthio, Alkyl, Alkenyl, Alkynyl, Heterocyclyl, Mercapto, Nitro, Aryloxy, Heteroaryloxy, Oxo (=O), Carboxy, Hydroxy-substituiertes Alkoxy, Hydroxy-substituiertes Alkyl-C(=O)-, Alkyl-C(=O)-, Alkyl-S(=O)-, Alkyl-S(=O)₂-, Hydroxy-substituiertes Alkyl-S(=O)-, Hydroxy-substituiertes Alkyl-S(=O)₂- oder Carboxysubstituiertes Alkoxy ist; oder wobei W für C₃₋₈-Carbocyclyl oder C₂₋₁₀-Heterocyclyl steht.

2. Verbindung nach Anspruch 1, wobei 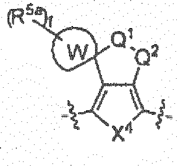 für 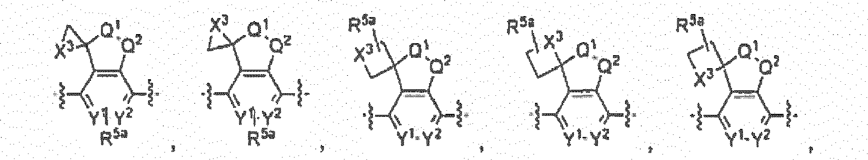 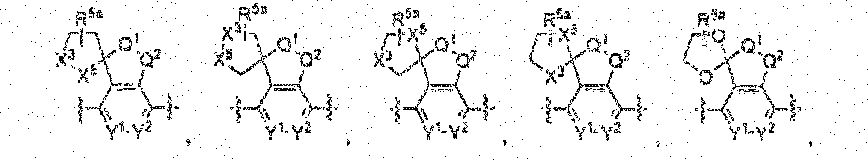 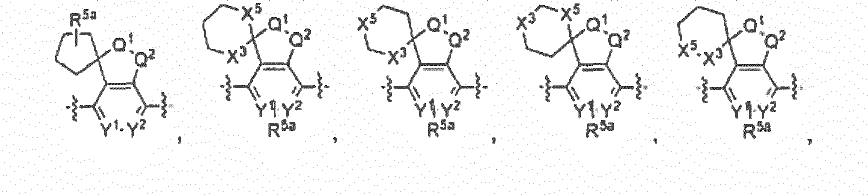 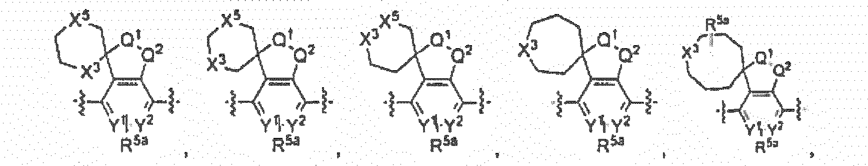 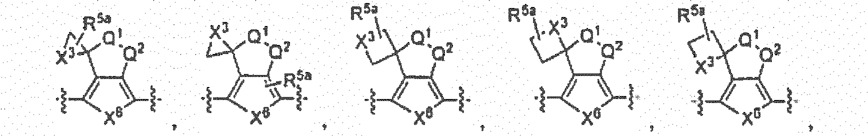 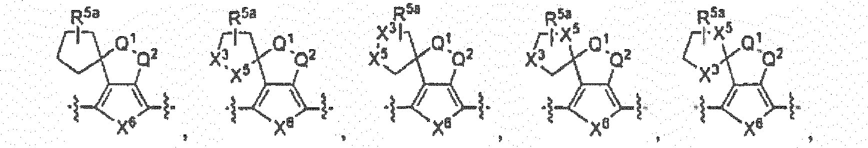 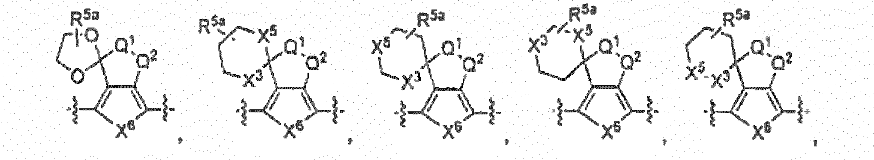 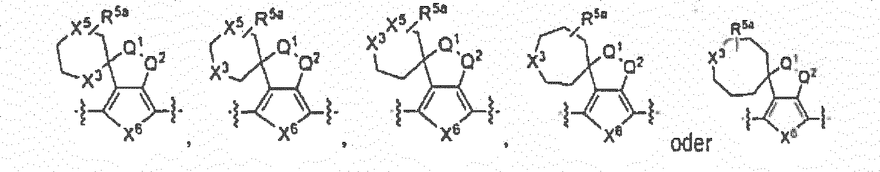 steht,
wobei X³ und X⁵ jeweils unabhängig für O, S, NR⁶ oder CR⁷R^{7a} stehen;
X⁶ jeweils unabhängig für CR⁷R⁷⁴, O, S oder NR⁶ steht;
Y¹ und Y² jeweils unabhängig für N oder CR⁷ stehen;
f 0, 1, 2, 3 oder 4 ist;
Q¹ und Q² jeweils unabhängig für NR⁶, O, S, C(=O) oder (CR⁷R^{7a})ₑ stehen;
e 0, 1, 2, 3 oder 4 ist;
R^{5a} jeweils unabhängig für H, Deuterium, Oxo (=O), Hydroxy, Amino, F, Cl, Br, I, Cyano, C₁₋₆-Alkylacyl, C₁-6₋Alkylacyloxy, C₁₋₆₋Alkoxyacyl, C₁₋₆₋Alkylsulfonyl, C₁₋₆₋Alkoxysulfonyl, C₁₋₆₋Alkylsulfinyl, C₁₋₆₋Alkylsulfonyloxy, C₁₋₆₋Alkylsulfinyloxy, C₁₋₆₋Alkoxy, C₁₋₆-Alkyl, C₆₋₁₀-Aryl, -CF₃, -OCF₃, Mercapto, Nitro, C₁₋₆₋Alkylamino, C₃₋₁₀₋Cycloalkyl oder C₆₋₁₀₋Aryloxy steht;
R⁶ jeweils unabhängig für H, Deuterium, R¹³R^{13a}NC(=O)-, R¹³OC(=O)-, R¹³C(=O)-, R¹³R^{13a}NS(=O)-, R¹³OS(=O)-, R¹³S(=O)-, R¹³R^{13a}NS(=O)₂-, R¹³OS(=O)₂-, R¹³S(=O)₂-, C₁₋₆₋Alkyl, C₁₋₆₋Haloalkyl, C₁₋₆₋Hydroxyalkyl, C₁₋₆₋Aminoalkyl, C₁₋₆₋Alkoxy-C₁₋₆-alkyl, C₁₋₆₋Alkylamino-C₁₋₆-alkyl, C₁₋₆₋Alkylthio-C₁₋₆-alkyl, C₆₋₁₀₋Aryl-C₁₋₆-alkyl, C₁₋₉₋Heteroaryl-C₁₋₆-alkyl, C₂₋₁₀₋Heterocyclyl-C₁₋₆-alkyl, C₃₋₁₀₋Cycloalkyl-C₁₋₆-alkyl, C₆₋₁₀₋Aryloxy-C₁₋₆-alkyl, C₂₋₁₀₋Heterocyclyloxy-C₁₋₆-alkyl, C₃₋₁₀₋Cycloalky-C₁₋₆-alkyl, C₆₋₁₀₋Arylamino-C₁₋₆-alkyl, C₂₋₁₀₋Heterocyclylamino-C₁₋₆-alkyl, C₃₋₁₀₋Cycloalkylamino-C₁₋₆-alkyl, C₆₋₁₀₋Aryl, C₁₋₉₋Heteroaryl, C₂₋₁₀₋Heterocyclyl oder C₃₋₁₀₋Carbocyclyl steht;
R⁷ und R^{7a} jeweils unabhängig für H, Deuterium, F, Cl, Br, I, C₁₋₆-Alkyl, C₂₋₆₋Heteroalkyl, C₁₋₆₋Haloalkyl, C₁₋₆₋Hydroxyalkyl, C₁₋₆₋Aminoalkyl, C₁₋₆₋Alkoxy-C₁₋₆-alkyl, C₁₋₆-Alkylamino-C₁₋₆-alkyl, C₁₋₆₋Alkylthio-C₁₋₆-alkyl, C₆₋₁₀₋Aryl-C₁₋₆-alkyl, C₁₋₉-Heteroaryl-C₁₋₆-alkyl, C₂₋₁₀₋Heterocyclyl-C₁₋₆-alkyl, C₃₋₁₀-Cycloalkyl-C₁₋₆-alkyl, C₆₋₁₀-Aryloxy-C₁₋₆-alkyl, C₂₋₁₀-Heterocycloxy-C₁₋₆-alkyl, C₃₋₁₀-Cycloalkyloxy-C₁₋₆-alkyl, C₆₋₁₀-Arylamino-C₁₋₆-alkyl, C₂₋₁₀-Heterocyclylamino-C₁₋₆-alkyl, C₃₋₁₀-Cycloalkylamino-C₁₋₆-alkyl, C₆₋₁₀-Aryl, C₁₋₉-Heteroaryl, C₂₋₁₀-Heterocyclyl oder C₃₋₁₀-Carbocyclyl stehen und
R¹³ und R^{13a} jeweils unabhängig für H, Deuterium, C₁₋₆-Alkyl, C₂₋₆-Heteroalkyl, C₃₋₁₀-Cycloalkyl, C₂₋₁₀-Heterocyclyl, C₆₋₁₀-Aryl, C₁₋₉-Heteroaryl oder C₆₋₁₀-Aryl-C₁₋₆-alkyl stehen, mit der Vorgabe, dass, wenn R¹³ und R^{13a} an dasselbe Stickstoffatom gebunden sind, R¹³ und R^{13a} zusammen mit dem Stickstoffatom, woran sie gebunden sind, gegebenenfalls einen substituierten oder unsubstituierten 3- bis 8-gliedrigen Ring oder einen substituierten oder unsubstituierten Spiro-Ring oder annellierten bicyclischen Ring bilden; oder wobei 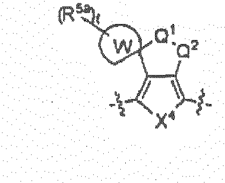 für 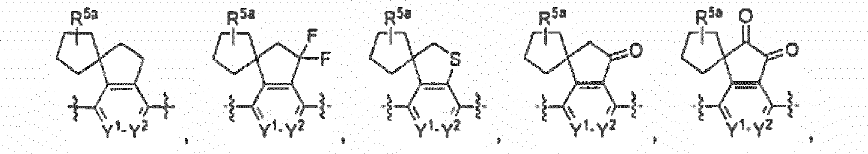 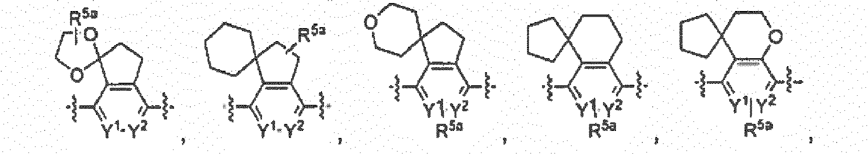 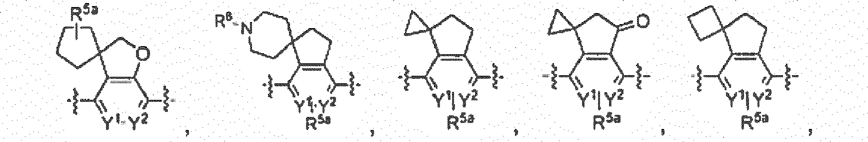 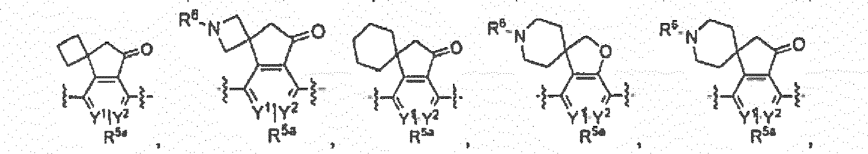 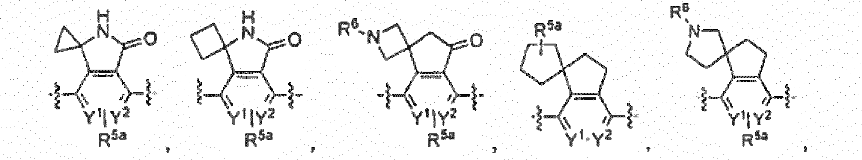 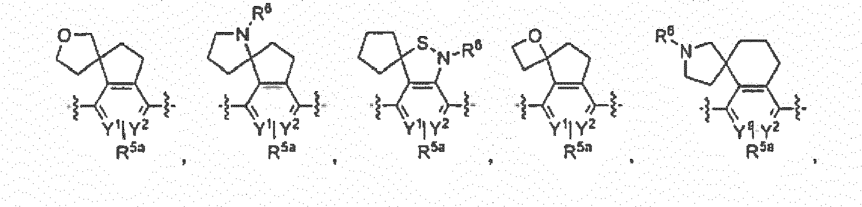 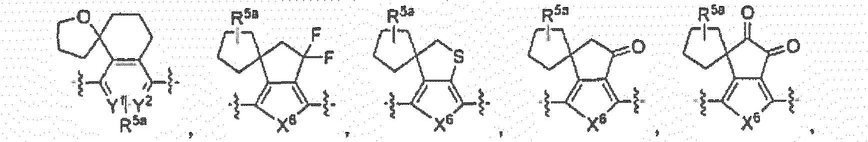 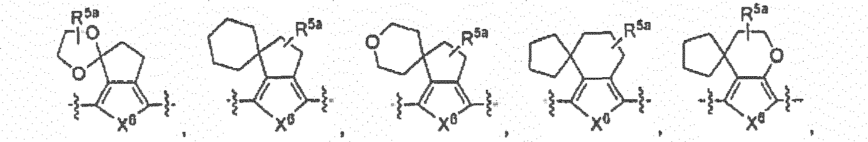 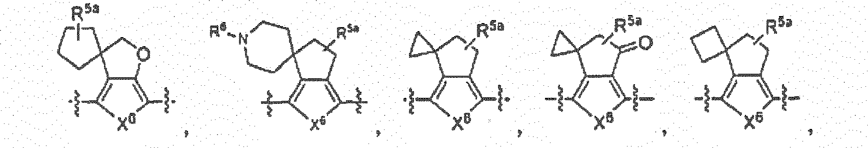 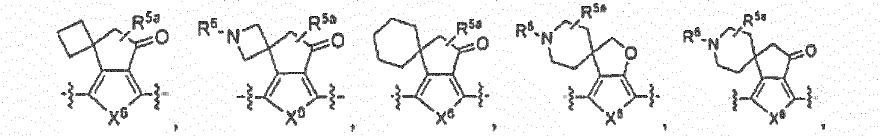 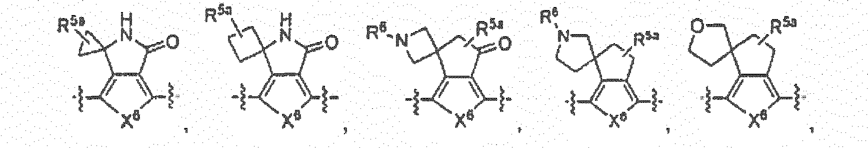 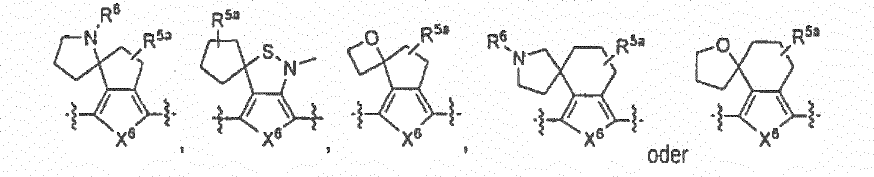 steht,
wobei Y¹ und Y² jeweils unabhängig für N oder CR⁷ stehen;
X⁶ jeweils unabhängig für CR⁷R^{7a}, O, S oder NR⁶ steht;
R^{5a} jeweils unabhängig für H, Deuterium, Oxo (=O), Hydroxy, Amino, F, Cl, Br, I, Cyano, C₁₋₆₋Alkylacyl, C₁₋₆₋Alkylacyloxy, C₁₋₆₋Alkoxyacyl, C₁₋₆₋Alkylsulfonyl, C₁₋₆₋Alkoxysulfonyl, C₁₋₆₋Alkylsulfinyl, C₁₋₆₋Alkylsulfonyloxy, C₁₋₆₋Alkylsulfinyloxy, C₁₋₆₋Alkoxy, C₁₋₆₋Alkyl, C₆₋₁₀₋Aryl, -CF₃, -OCF₃, Mercapto, Nitro oder C₁₋₆-Alkylamino steht;
R⁶ jeweils unabhängig für H, Deuterium, C₁₋₆₋Alkyl, C₁₋₆-Haloalkyl, C₁₋₆-Hydroxyalkyl, C₁₋₆-Aminoalkyl, C₁₋₆-Alkoxy-C₁₋₆-alkyl, C₁₋₆-Alkylamino-C₁₋₆-alkyl, C₁₋₆-Alkylthio-C₁₋₆-alkyl, C₆₋₁₀-Aryl-C₁₋₆-alkyl, C₁₋₉-Heteroaryl-C₁₋₆-alkyl, C₂₋₁₀-Heterocyclyl-C₁₋₆-alkyl oder C₃₋₈-Cycloalkyl-C₁₋₆-alkyl steht und
R⁷ und R^{7a} jeweils unabhängig für H, Deuterium, F, Cl, Br, I, C₁₋₆-Alkyl, C₂₋₆-Heteroalkyl, C₁₋₆-Haloalkyl, C₁₋₆-Hydroxyalkyl, C₁₋₆-Aminoalkyl, C₁₋₆-Alkoxy-C₁₋₆-alkyl, C₁₋₆-Alkylamino-C₁₋₆-alkyl, C₁₋₆-Alkylthio-C₁₋₆-alkyl, C₆₋₁₀-Aryl-C₁₋₆-alkyl, C₂₋₁₀-Heterocyclyl-C₁₋₆-alkyl, C₃₋₁₀-Cycloalkyl-C₁₋₆-alkyl, C₆₋₁₀-Aryloxy-C₁₋₆-alkyl, C₂₋₁₀-Heterocyclyloxy-C₁₋₆-alkyl, C₃₋₁₀-Cycloalkyloxy-C₁₋₆-alkyl, C₆₋₁₀-Arylamino-C₁₋₆-alkyl, C₂₋₁₀-Heterocyclylamino-C₁₋₆-alkyl, C₃₋₁₀-Cycloalkylamino-C₁₋₆-alkyl, C₆₋₁₀-Aryl, C₁₋₉-Heteroaryl, C₂₋₁₀-Heterocyclyl oder C₃₋₈-Carbocyclyl stehen.

3. Verbindung nach Anspruch 1, wobei A und A' jeweils unabhängig für eine Bindung, C₁₋₆-Alkylen, C₂₋₆-Alkenylen, C₃₋₈-Cycloalkylen, C₂₋₁₀-Heterocyclylalkylen, -(CR⁸R^{8a})ⁿ-O-(CR⁸R^{8a})ₚ-, -(CR⁸R^{8a})ₙ-N(R⁵)-(CR⁸R^{8a})ₚ-, (CR⁸R^{8a})ₙ-S(=O)ᵣ-N(R⁵)-(CR⁸R^{8a})ₚ-, (CR⁸R^{8a})ₙ-C(=O)-N(R⁵)-(CR⁸R^{8a})ₚ-, -(CR⁸R^{8a})ₙ-N(R⁵)-C(=O)-N(R⁵)-(CR⁸R^{8a})ₚ-,-(CR⁸R^{8a})ₙ-C(=O)-O-(CR⁸R^{8a})ₚ-, -(CR⁸R^{8a})ₙ-N(R⁵)-S(=O)ᵣ-N(R⁵)-(CR⁸R^{8a})ₚ- oder -(CR⁸R^{8a})ₙ-N(R⁵)-C(=O)-O-(CR⁸R^{8a})ₚ-stehen oder A und A' jeweils unabhängig für 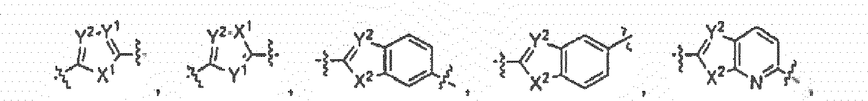 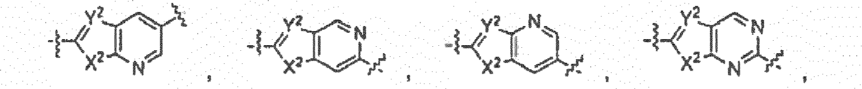 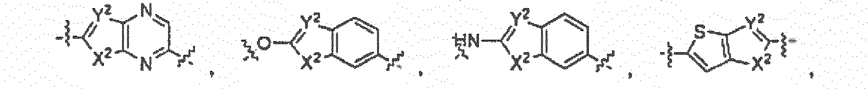 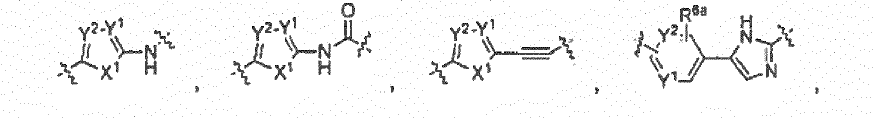 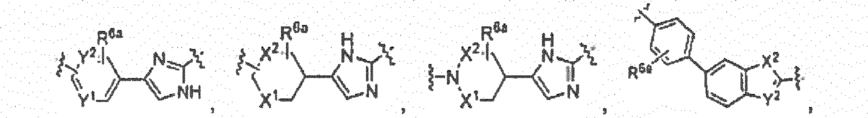 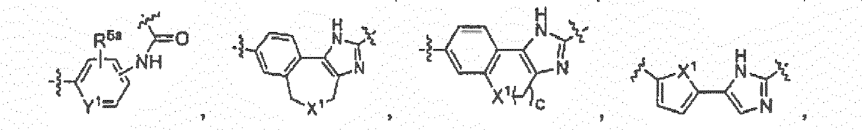 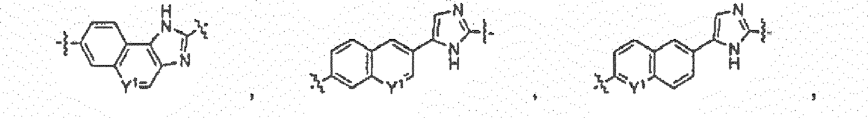 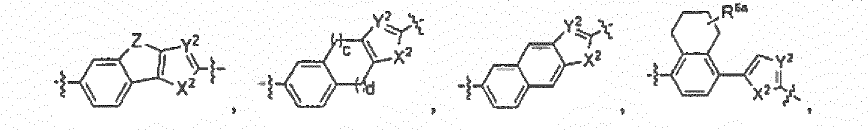 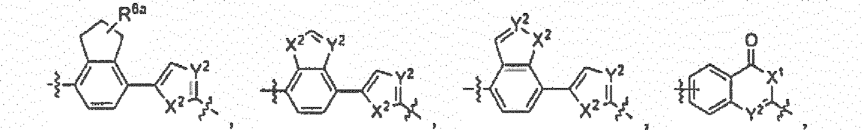 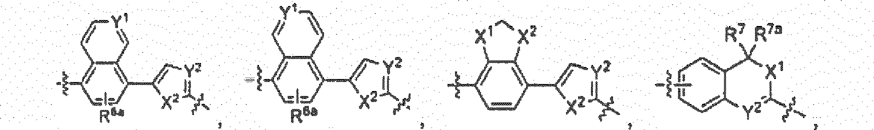 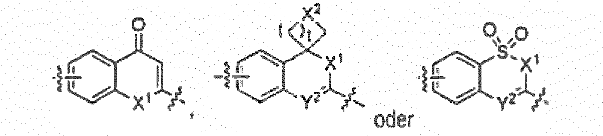 stehen,
wobei X¹ jeweils unabhängig für 0, S, NR⁶ oder CR⁷R^{7a} steht;
X² jeweils unabhängig für NR⁶, O oder S steht;
Y¹ und Y² jeweils unabhängig für N oder CR⁷ stehen;
c jeweils unabhängig 1 oder 2 ist;
d jeweils unabhängig 1 oder 2 ist;
n jeweils unabhängig 0, 1, 2, oder 3 ist;
p jeweils unabhängig 0, 1, 2, oder 3 ist;
r jeweils unabhängig 0, 1 oder 2 ist;
R⁵ jeweils unabhängig für H, Deuterium, Hydroxy, C₁₋₆-Alkyl, C₂₋₆-Heteroalkyl, C₃₋₈-Cycloalkyl, C₂₋₁₀-Heterocyclyl, C₆₋₁₀-Aryl, C₁₋₉-Heteroaryl, C₆₋₁₀-Aryl-C₁₋₆-alkyl, C₁₋₆-Alkoxy, C₁₋₆-Alkyl-OC(=O)-, C₁₋₆-Alkyl-C(=O)-, Carbamoyl, C₁₋₆-Alkyl-OS(=O)ᵣ-, C₁₋₆-Alkyl-S(=O)ᵣ-, C₁₋₆-Alkyl-S(=O)ᵣ- oder Aminosulfonyl steht;
R⁶ jeweils unabhängig für H, Deuterium, R¹³R^{13a}NC(=O)-, R¹³OC(=O)-, R¹³C(=O)-, R¹³R^{13a}NS(=O)-, R¹³OS(=O)-, R¹³S(=O)-, R¹³R^{13a}NS(=O)₂-, R¹³OS(=O)₂-, R¹³S(=O)₂-, C₁₋₆-Alkyl, C₁₋₆-Haloalkyl, C₁₋₆-Hydroxyalkyl, C₁₋₆-Aminoalkyl, C₁₋₆-Alkoxy-C₁₋₆-alkyl, C₁₋₆-Alkylamino-C₁₋₆-alkyl, C₁₋₆-Alkylthio-C₁₋₆-alkyl, C₆₋₁₀-Aryl-C₁₋₆-alkyl, C₁₋₉-Heteroaryl-C₁₋₆-alkyl, C₂₋₁₀-Heterocyclyl-C₁₋₆-alkyl, C₃₋₁₀-Cycloalkyl-C₁₋₆-alkyl, C₆₋₁₀-Aryloxy-C₁₋₆-alkyl, C₂₋₁₀-Heterocyclyloxy-C₁₋₆-alkyl, C₃₋₁₀-Cycloalkyloxy-C₁₋₆-alkyl, C₆₋₁₀-Arylamino-C₁₋₆-alkyl, C₂₋₁₀-Heterocyclylamino-C₁₋₆-alkyl, C₃₋₁₀-Cycloalkylamino-C₁₋₆-alkyl, C₆₋₁₀-Aryl, C₁₋₉-Heteroaryl, C₂₋₁₀-Heterocyclyl oder C₃₋₁₀-Carbocyclyl steht;
R^{6a} jeweils unabhängig für H, Deuterium, Hydroxy, Amino, F, Cl, Br, I, Cyano, Oxo (=O), R^{13a}R¹³N-,-C(=O)NR¹³R^{13a}, -OC(=O)NR¹³R^{13a}, -OC(=O)OR¹³, N(R¹³)C(=O)NR¹³R^{13a}, -N(R¹³)C(=O)OR^{13a}, -N(R¹³)C(=O)-R^{13a}, R¹³R^{13a}N-S(=O)₂-, R¹³S(=O)₂-, R¹³S(=O)₂N(R^{13a})-, R^{13a}R¹³N-C₁₋₆-Alkyl, R¹³S(=O)-C₁₋₆-Alkyl, R¹³R^{13a}N-C(=O)-C₁₋₆-Alkyl, R^{13a}R¹³N-C₁₋₆-Alkoxy, R¹³S(=O)-C₁₋₆-Alkoxy, R¹³R^{13a}N-C(=O)-C₁₋₆-Alkoxy, C₆₋₁₀-Aryl, C₁₋₉-Heteroaryl, C₂₋₆-Alkoxy, C₁₋₆-Alkylamino, C₁₋₆-Alkyl, C₁₋₆-Haloalkyl, C₂₋₆-Alkenyl, C₂₋₆-Alkinyl, C₂₋₁₀-Heterocyclyl, C₃₋₈-Cycloalkyl, Mercapto, Nitro, C₆₋₁₀-Aryl-C₁₋₆-alkyl, C₆₋₁₀-Arylamino, C₁₋₉-Heteroarylamino oder C₆₋₁₀-Aryloxy steht;
R⁷ und R^{7a} jeweils unabhängig für H, Deuterium, F, Cl, Br, I, C₁₋₆-Alkyl, C₂₋₆-Heteroalkyl, C₁₋₆-Haloalkyl, C₁₋₆-Hydroxyalkyl, C₁₋₆-Aminoalkyl, C₁₋₆-Alkoxy-C₁₋₆-alkyl, C₁₋₆-Alkylamino-C₁₋₆-alkyl, C₁₋₆-Alkylthio-C₁₋₆-alkyl, C₆₋₁₀₋Aryl-C₁₋₆-alkyl, C₂₋₁₀-Heterocyclyl-C₁₋₆-alkyl, C₃₋₁₀-Cycloalkyl-C₁₋₆-alkyl, C₆₋₁₀-Aryloxy-C₁₋₆-alkyl, C₂₋₁₀-Heterocyclyloxy-C₁₋₆-alkyl, C₃₋₁₀-Cycloalkyloxy-C₁₋₆-alkyl, C₆₋₁₀-Arylamino-C₁₋₆-alkyl, C₂₋₁₀-Heterocyclylamino-C₁₋₆-alkyl, C₃₋₁₀-Cycloalkylamino-C₁₋₆-alkyl, C₆₋₁₀-Aryl, C₁₋₉-Heteroaryl, C₂₋₁₀-Heterocyclyl oder C₃₋₈-Carbocyclyl stehen;
R¹³ und R^{13a} jeweils unabhängig für H, Deuterium, C₁₋₆-Alkyl, C₂₋₆-Heteroalkyl, C₃₋₁₀-Cycloalkyl, C₂₋₁₀-Heterocyclyl, C₆₋₁₀-Aryl, C₁₋₉-Heteroaryl oder C₆₋₁₀-Aryl-C₁₋₆-alkyl stehen, mit der Vorgabe, dass, wenn R¹³ und R^{13a} an dasselbe Stickstoffatom gebunden sind, R¹³ und R^{13a} zusammen mit dem Stickstoffatom, woran sie gebunden sind, gegebenenfalls einen substituierten oder unsubstituierten 3- bis 8-gliedrigen Ring oder einen substituierten oder unsubstituierten Spiro-Ring oder annellierten bicyclischen Ring bilden; und
R⁸ und R^{8a} jeweils unabhängig für H, Deuterium, Hydroxy, Cyano, Nitro, F, Cl, Br, I, C₁₋₆-Alkyl, C₂₋₆-Heteroalkyl, C₃₋₁₀-Cycloalkyl, C₂₋₁₀-Heterocyclyl, C₆₋₁₀-Aryl, C₁₋₉-Heteroaryl, C₆₋₁₀-Aryl-C₁₋₆-alkyl, C₁₋₆-Alkoxy, C₁₋₆-Alkyl-OC(=O)-, C₁₋₆-Alkyl-C(=O)-, Carbamoyl, C₁₋₆-Alkyl-OS(=O)ᵣ-, C₁₋₆-Alkyl-S(=O)ᵣO-, C₁₋₆-Alkyl-S(=O)ᵣ- oder Aminosulfonyl stehen oder wobei A und A' jeweils unabhängig für eine Bindung, -CH₂-, -(CH₂)₂-, -CH=CH-, -CH=CH-CH₂-, -N(R⁶)-, -C(=O)-, -C(=S)-, -C(=O)-O-,-C(=O)N(R⁶)-, -OC(=O)N(R⁶)-, -OC(=O)O-, N(R⁶)C(=O)N(R⁶)-, -(R⁶)N-S(=O)₂-, -S(=O)₂-, -OS(=O)₂-,-(R⁶)N-S(=O)-, -S(=O)-, -OS(=O)- stehen oder A und A' jeweils unabhängig für 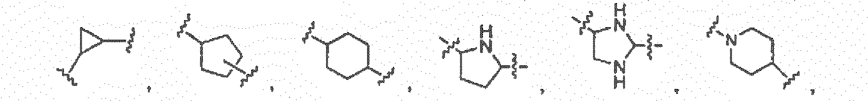 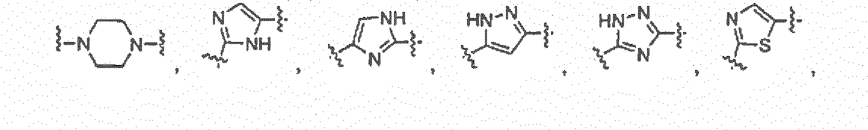 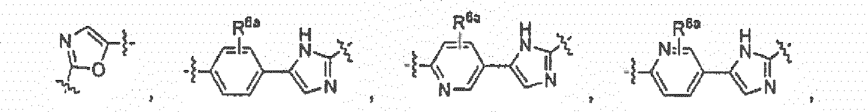 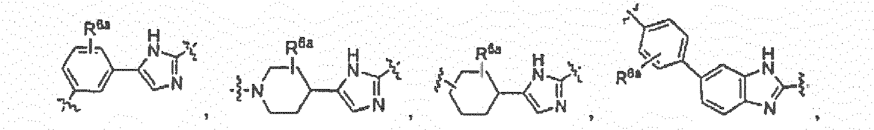 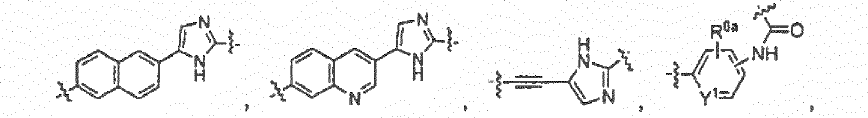 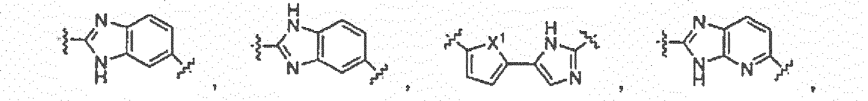 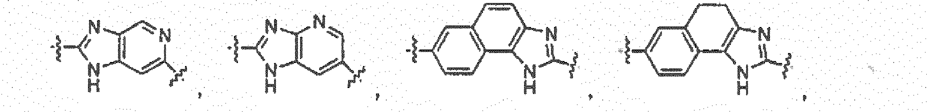 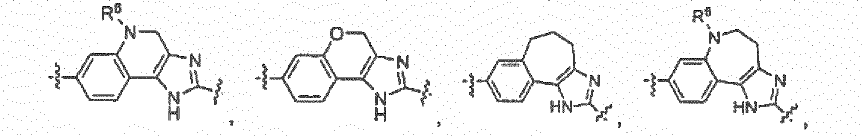 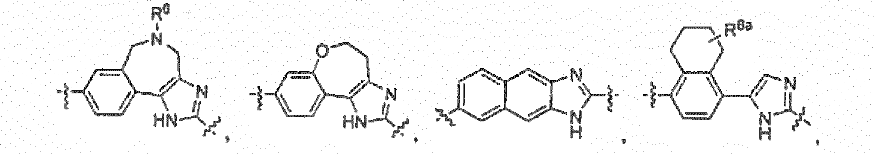 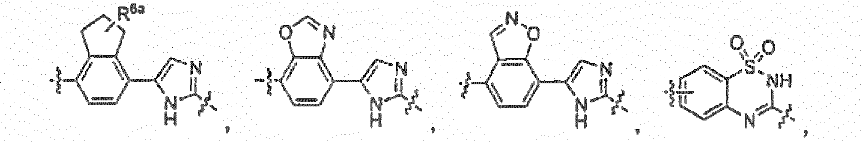 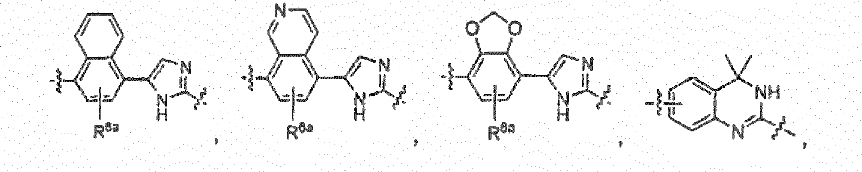 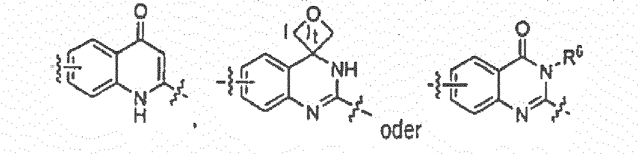 stehen,
wobei X¹ für O oder S steht;
Y¹ jeweils unabhängig für N oder CR⁷ steht;
R⁶ jeweils unabhängig für H, Deuterium, C₁₋₄-Alkyl, C₁₋₆-Haloalkyl, C₁₋₆-Hydroxyalkyl, C₁₋₆-Aminoalkyl, C₁₋₆-Alkoxy-C₁₋₄-alkyl, C₁₋₆-Alkylamino-C₁₋₄-alkyl, C₁₋₆-Alkylthio-C₁₋₄-alkyl, C₆₋₁₀-Aryl-C₁₋₄-alkyl, C₁₋₉-Heteroaryl, C₆₋₁₀-Aryl, C₂₋₁₀-Heterocyclyl oder C₃₋₈-Carbocyclyl steht;
R^{6a} und R⁷ jeweils unabhängig für H, Deuterium, Oxo (=O) , Hydroxy, Amino, F, Cl, Br, I, Cyano, R^{13a}R¹³N-, C₁₋₆-Alkoxy, C₁₋₆-Alkylamino, C₁₋₆-Alkyl, C₁₋₆-Haloalkyl, C₂₋₆-Alkenyl, C₂₋₆-Alkinyl, Mercapto oder Nitro stehen;
R¹³ und R^{13a} jeweils unabhängig für H, Deuterium, C₁₋₆-Alkyl, C₂₋₆-Heteroalkyl, C₃₋₁₀-Cycloalkyl, C₂₋₁₀-Heterocyclyl, C₆₋₁₀-Aryl, C₁₋₉-Heteroaryl oder C₆₋₁₀-Aryl-C₁₋₆-alkyl stehen, mit der Vorgabe, dass, wenn R¹³ und R^{13a} an dasselbe Stickstoffatom gebunden sind, R¹³ und R^{13a} zusammen mit dem Stickstoffatom, woran sie gebunden sind, gegebenenfalls einen substituierten oder unsubstituierten 3- bis 8-gliedrigen Ring oder einen substituierten oder unsubstituierten Spiro-Ring oder annellierten bicyclischen Ring bilden.

4. Verbindung Anspruch 1,
wobei R¹, R² und X-CH zusammen eine der folgenden monovalenten Gruppen bilden: 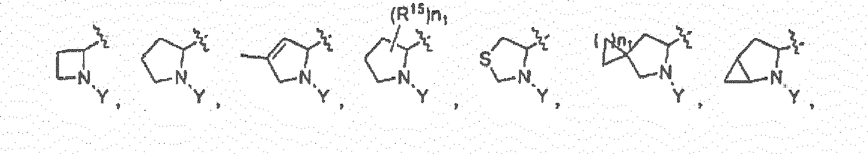 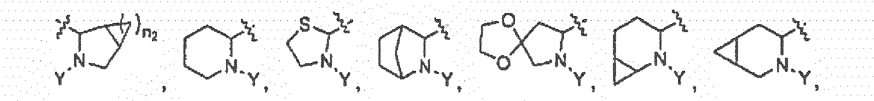 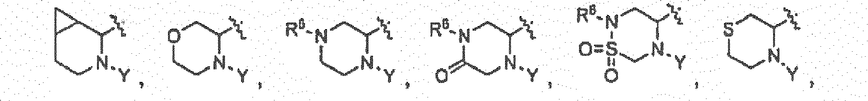 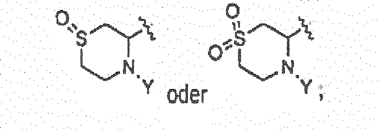 wobei R¹⁵ jeweils unabhängig für H, Deuterium, F, Cl, Br, I, Cyano, Hydroxy, C₁₋₃-Alkyl, C₁₋₃-Haloalkyl, C₁₋₃-Alkoxy, C₁₋₃-Alkylamino, C₁₋₃-Alkylthio, C₆₋₁₀-Acylamino, C₆₋₁₀-Aryloxy, C₁₋₉-Heteroaryl, C₁₋₉-Heteroaryloxy, C₁₋₉-Heteroaryl-C₁₋₃-alkyl oder C₂₋₁₀-Heterocyclyl steht;
R⁶ jeweils unabhängig für H, Deuterium, C₁₋₃-Alkyl, C₁₋₃-Haloalkyl, C₁₋₃-Hydroxyalkyl, C₁₋₃-Aminoalkyl, C₁₋₃-Alkoxy-C₁₋₃-alkyl, C₁₋₃-Alkylamino-C₁₋₃-alkyl, C₁₋₃-Alkylthio-C₁₋₃-alkyl, C₆₋₁₀-Aryl-C₁₋₃-alkyl, C₁₋₃-Heteroaryl, C₆₋₁₀-Aryl, C₂₋₁₀-Heterocyclyl oder C₃₋₈-Carbocyclyl steht und
n₁ und n₂ jeweils unabhängig 1, 2, 3 oder 4 sind;
wobei R³, R⁴ und X'-CH zusammen die folgenden monovalenten Gruppen bilden: 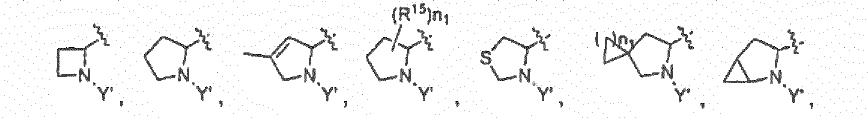 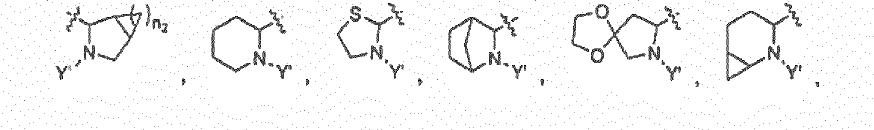 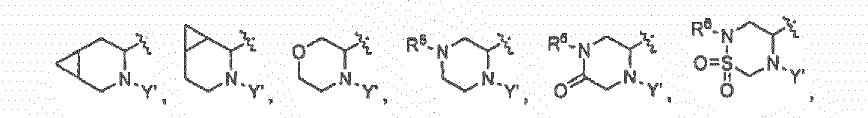 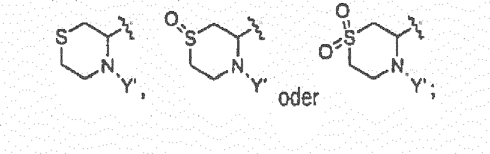 wobei R¹⁵ jeweils unabhängig für H, Deuterium, F, Cl, Br, I, Cyano, Hydroxy, C₁₋₃-Alkyl, C₁₋₃-Haloalkyl, C₁₋₃-Alkoxy, C₁₋₃-Alkylamino, C₁₋₃-Alkylthio, C₆₋₁₀-Arylamino, C₆₋₁₀)-Aryloxy, C₁₋₉-Heteroaryl, C₁₋₉-Heteroaryloxy, C₁₋₉-Heteroaryl-C₁₋₃-alkyl oder C₂₋₁₀-Heterocyclyl steht;
R⁶ jeweils unabhängig für H, Deuterium, C₁₋₃-Alkyl, C₁₋₃-Haloalkyl, C₁₋₃-Hydroxyalkyl, C₁₋₃-Aminoalkyl, C₁₋₃-Alkoxy-C₁₋₃-alkyl, C₁₋₃-Alkylamino-C₁₋₃-alkyl, C₁₋₃-Alkylthio-C₁₋₃-alkyl, C₆₋₁₀-Aryl-C₁₋₃-alkyl, C₁₋₉-Heteroaryl, C₆₋₁₀-Aryl, C₂₋₁₀-Heterocyclyl oder C₃₋₈-Carbocyclyl steht und
n₁ und n₂ jeweils unabhängig 1, 2, 3 oder 4 sind.

5. Verbindung nach Anspruch 1, welche die Formel (II) aufweist: 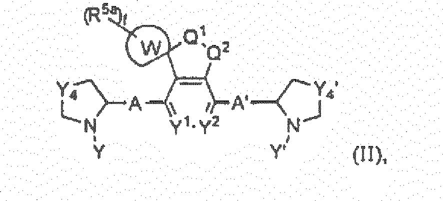 wobei 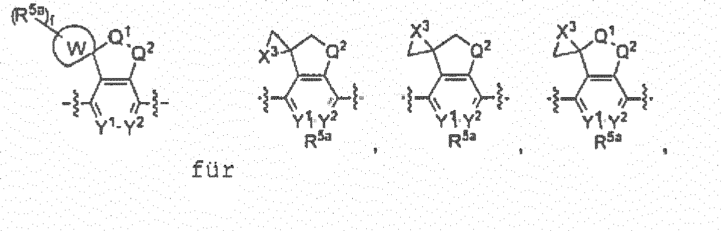 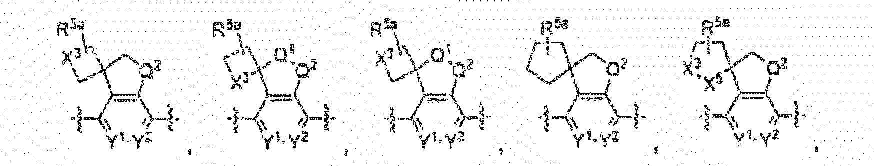 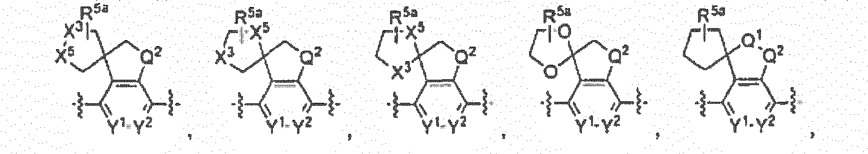 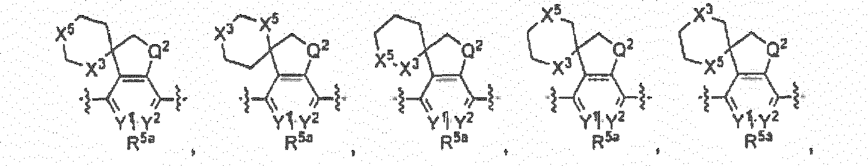 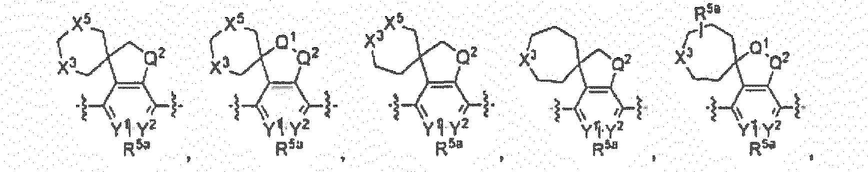 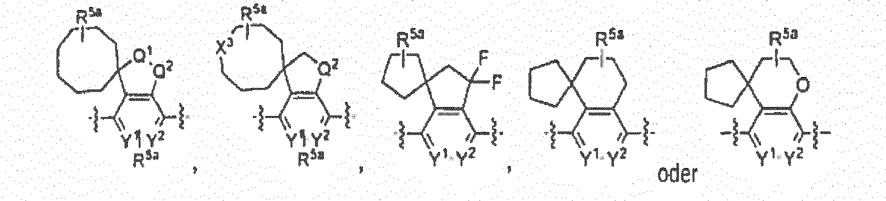 steht;
wobei Q¹ und Q² jeweils unabhängig für NR⁶, O, S, C(=O) oder (CH₂)ₑ stehen;
e 0, 1, 2, 3 oder 4 ist;
X¹, X² und X⁵ jeweils unabhängig für O, S, NR⁶ oder CR⁷R^{7a} stehen;
Y¹ und Y² jeweils unabhängig für N oder CR⁷ stehen;
A und A' jeweils unabhängig für eine Bindung, C₁₋₆-Alkylen, C₁₋₆-Alkenylen, C₃₋₈-Cycloalkylen, C₂₋₁₀-Heterocyclylalkylen, - (CR⁸R^{8a})ₙ-O-CR⁸R^{8a})ₚ-, -(CR⁸R^{8a})ₙ-N(R⁵)-(CR⁸R^{8a})ₚ-, -(CR⁸R^{8a})ₙ-S(=O)ᵣ-N(R⁵)-(CR⁸R^{8a})ₚ-, -(CR⁸R^{8a})ₙ-C(=O)-N(R⁵)-(CR⁸R^{8a})ₚ-, -(CR⁸R^{8a})ₙ-N(R⁵)-C(=O)-N(R⁵)-(CR⁸R^{8a})ₚ-, -(CR⁸R^{8a})ₙ-C(=O)-O-(CR⁸R^{8a})ₚ-, -(CR⁸R^{8a} )ₙ-N(R⁵)-S(=O)ᵣ-N(R⁵)-(CR⁸R^{8a})ₚ- oder -(CR⁸R^{8a})ₙ-N(R⁵)-C(=O)-O-(CR⁸R^{8a})ₚ- stehen oder A und A' jeweils unabhängig für 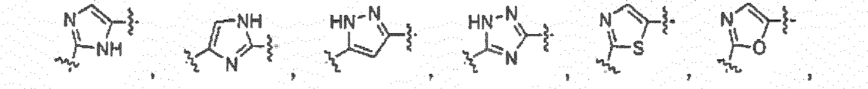 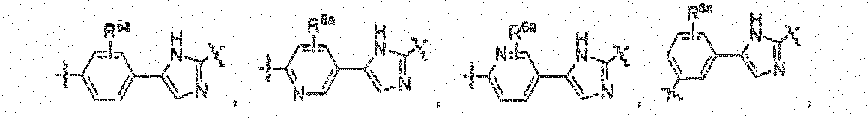 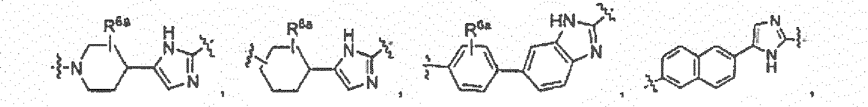 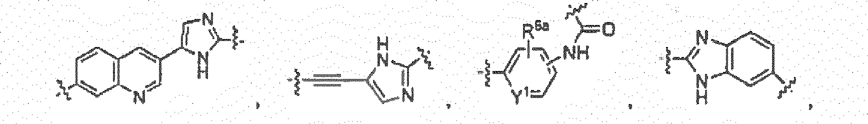 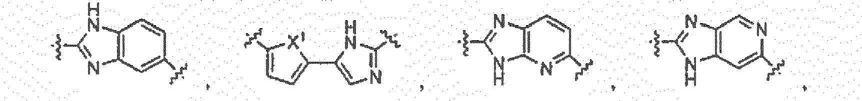 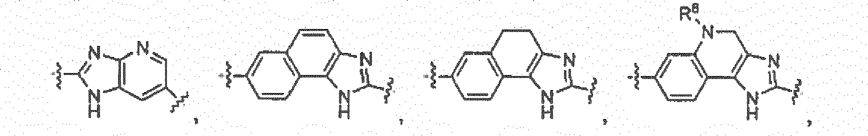 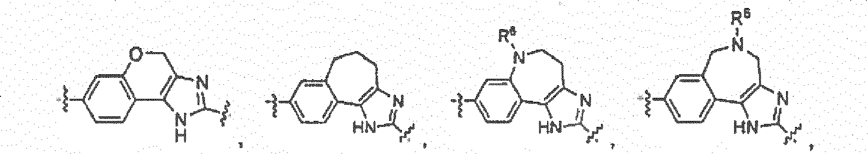 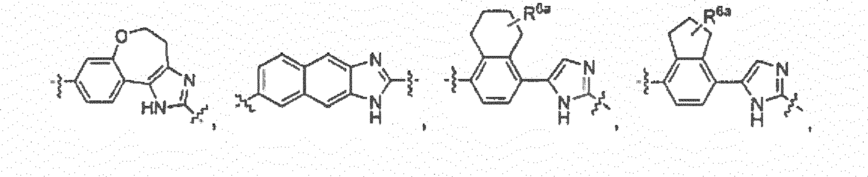 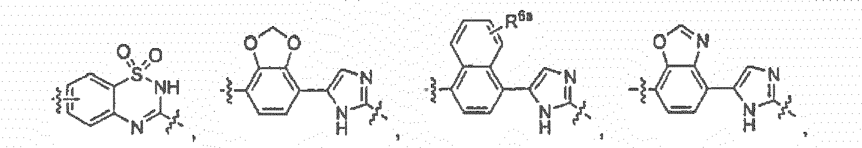 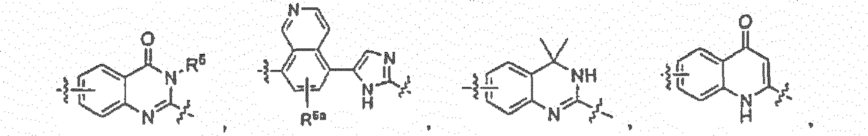 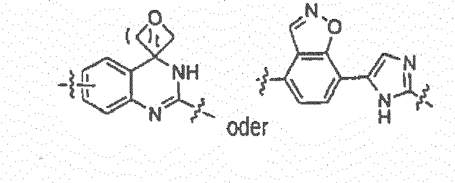 stehen;
R⁵ jeweils unabhängig für H, Deuterium, Hydroxy, C₁₋₆-Alkyl, C₂₋₆-Heteroalkyl, C₃₋₁₀-Cycloalkyl, C₂₋₁₀-Heterocyclyl, C₆₋₁₀-Aryl, C₁₋₉-Heteroaryl, C₆₋₁₀-Aryl-C₁₋₆-alkyl, C₁₋₆-Alkoxy, C₁₋₆-Alkyl-OC(=O)-, C₁₋₆-Alkyl-C(=O)-, Carbamoyl, C₁₋₆-Alkyl-OS(=O)ᵣ-, C₁₋₆-AlkylS(=O)ᵣO-, C₁₋₆-Alkyl-S(=O)ᵣ- oder Aminosulfonyl steht;
R^{5a} und R^{6a} jeweils unabhängig für H, Deuterium, Oxo (=O), Hydroxy, Amino, F, Cl, Br, I, Cyano, C₁₋₆-Alkylacyl, C₁₋₆-Alkylacyloxy, C₁₋₆-Alkoxyacyl, c₁₋₆-Alkylsulfonyl, C₁₋₆-Alkoxysulfonyl, C₁₋₆-Alkylsulfinyl, C₁₋₆-Alkylsulfonyloxy, C₁₋₆₋Alkylsulfinyloxy, C₁₋₆-Alkoxy, C₁₋₆-Alkyl, C₆₋₁₀-Aryl, -CF₃, -OCF₃, Mercapto, Nitro, C₁₋₆-Alkylamino, C₃₋₁₀-Cycloalkyl oder C₆₋₁₀-Aryloxy stehen;
R⁶ jeweils unabhängig für H, Deuterium, R¹³R^{13a}NC(=O)-, R¹³OC(=O)-, R¹³C(=O)-, R¹³R^{13a}NS(=O)-, R¹³OS(-O)-, R¹³S(=O)-, R¹³R^{13a}NS(=O)₂-, R¹³OS(=O)₂-, R¹³S(-O)₂-, eine C₁₋₆-aliphatische, C₁₋₆-alkoxy-C₁₋₆-aliphatische, C₁₋₆-alkylamino-C₁₋₆-aliphatische, C₆₋₁₀-aryl-C₁₋₆-aliphatische, C₁₋₉-heteroaryl-C₁₋₆-aliphatische, C₂₋₁₀-heterocyclyl-C₁₋₆-aliphatische, C₃₋₁₀-cycloalkyl-C₁₋₆-aliphatische, C₆₋₁₀-Aryl-, C₁₋₉-Heteroaryl-, C₂₋₁₀-Heterocycly- oder C₃₋₁₀-Carbocyclyl-Gruppe steht;
R⁷ und R^{7a} jeweils unabhängig für H, Deuterium, F, Cl, Br, I, eine C₁₋₆-aliphatische, C₂₋₆-Heteroalkyl-, C₁₋₆-Alkoxy-C₁₋₆-aliphatische, C₁₋₆-Alkylamino-C₁₋₆-aliphatische, C₆₋₁₀-Aryl-C₁₋₆-aliphatische, C₂₋₁₀-Heterocyclyl-C₁₋₆-aliphatische, C₃₋₁₀-Cycloalkyl-C₁₋₆-aliphatische, C₆₋₁₀-Aryl-, C₁₋₉-Heteroaryl-, C₂₋₁₀-Heterocyclyl- oder C₃₋₁₀-Carbocyclyl-Gruppe stehen;
R⁸ und R^{8a} jeweils unabhängig für H, Deuterium, Hydroxy, Cyano, Nitro, F, Cl, Br, I, C₁₋₆-Alkyl, C₂₋₆-Heteroalkyl, C₃₋₁₀-Cycloalkyl, C₂₋₁₀-Heterocyclyl, C₆₋₁₀-Aryl, C₁₋₉-Heteroaryl, C₆₋₁₀-Aryl-C₁₋₆-alkyl, C₁₋₆-Alkoxy, C₁₋₆-Alkyl-OC(=O)-, C₁₋₆-Alkyl-C(=O)-, Carbamoyl, C₁₋₆-Alkyl-OS(=O)ᵣ-, C₁₋₆-Alkyl-S(=O)ᵣO-, C₁₋₆-Alkyl-S(=O)ᵣ- oder Aminosulfonyl stehen;
R¹³ und R^{13a} jeweils unabhängig für H, Deuterium, C₁₋₆-Alkyl, C₂₋₆-Heteroalkyl, C₃₋₁₀-Cycloalkyl, C₂₋₁₀-Heterocyclyl, C₆₋₁₀-Aryl, C₁₋₉-Heteroaryl oder C₆₋₁₀-Aryl-C₁₋₆-alkyl stehen, mit der Vorgabe, dass, wenn R¹³ und R^{13a} an dasselbe Stickstoffatom gebunden sind, R¹³ und R^{13a} zusammen mit dem Stickstoffatom, woran sie gebunden sind, gegebenenfalls einen substituierten oder unsubstituierten 3- bis 8-gliedrigen Ring oder einen substituierten oder unsubstituierten Spiro-Ring oder annellierten bicyclischen Ring bilden;
n jeweils unabhängig 0, 1, 2 oder 3 ist;
p jeweils unabhängig 0, 1, 2 oder 3 ist;
r jeweils unabhängig 0, 1 oder 2 ist und
Y₄ und Y₄' jeweils unabhängig für eine Bindung, 0, S, -(CH₂)ₙ-, -CH=CH-, -S(=O)ᵣ-, -CH₂O)-, -CH₂S-, -CH₂F,-CHR^{5a}-, -CR^{5a}R^{6a}-, -CH₂S(=O)ᵣ- oder -CH₂N(R⁶)- stehen.

6. Verbindung nach Anspruch 1, welche die Formel (II') aufweist: 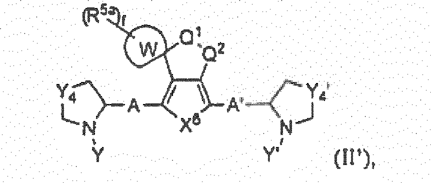 wobei 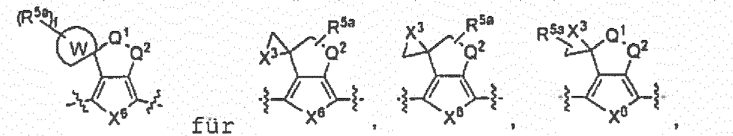 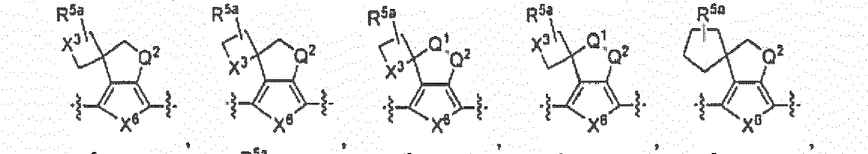 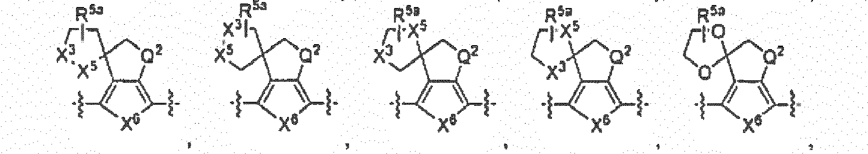 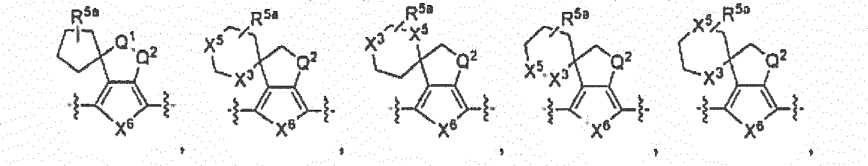 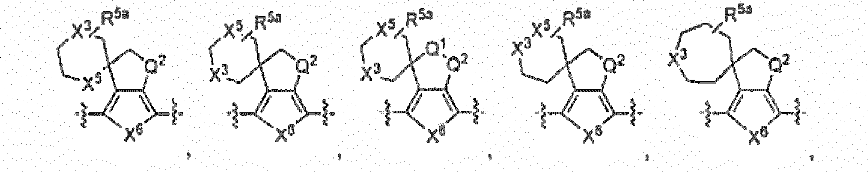 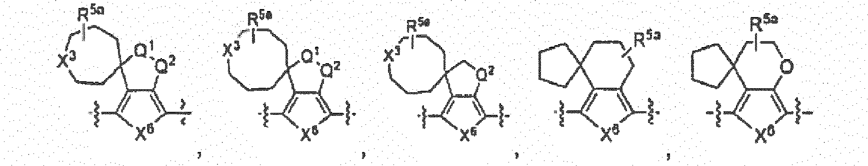 oder 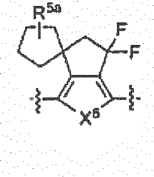 steht,
wobei Q¹ und Q² jeweils unabhängig für NR⁶, O, S, C(=O) oder (CH₂)ₑ stehen;
e 0, 1, 2, 3 oder 4 ist;
X¹, X³ und X⁵ jeweils unabhängig für O, S, NR⁶ oder CR⁷R^{7a} stehen;
X⁶ jeweils unabhängig für CR⁷R^{7a}, O, S oder NR⁶ stehen;
f 0, 1, 2, 3 oder 4 ist;
A und A' jeweils unabhängig für eine Bindung, C₁₋₆-Alkylen, C₂₋₆-Alkenylen, C₃₋₈-Cycloalkylen, C₂₋₁₀-Heterocyclylalkylen, -(CR⁸R^{8a})ₙ-O-(CR⁸R^{8a})ₚ-, -(CR⁸R^{8a})ₙ-N(R⁵)-(CR⁸R^{8a})ₚ-, -(CR⁸R^{8a})ₙ-S(=O)ᵣ-N(R⁵)-(CR⁸R^{8a})ₚ-,-(CR⁸R^{8a})ₙ-C(=O)-N(R⁵)-(CR⁸R^{8a})ₚ-, -(CR⁸R^{8a})ₙ-N(R⁵)-C(=O)-N(R⁵)-(CR⁸R^{8a})ₚ-, -(CR⁸R^{8a})ₙ-C(=O)-O-(CR⁸R^{8a})ₚ-,-(CR⁸R^{8a})ₙ-N(R⁵)-S(=O)ᵣ-N(R⁵)-(CR⁸R^{8a})ₚ-, -(CR⁸R^{8a})ₙ-N(R⁵)-C(=O)-O-(CR⁸R^{8a})ₚ- stehen oder A und A' jeweils unabhängig für 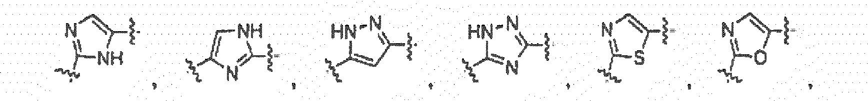 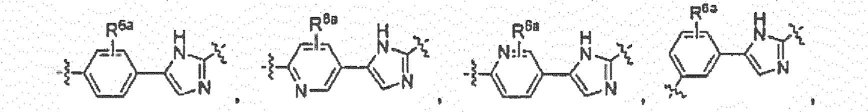 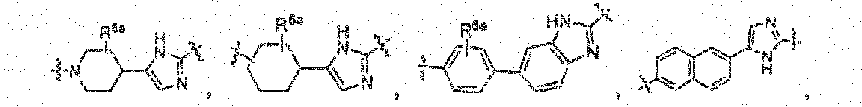 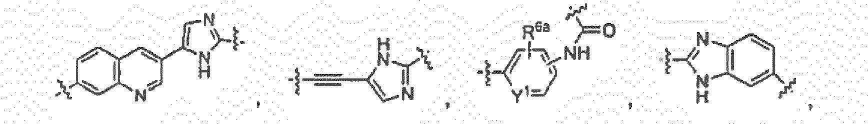 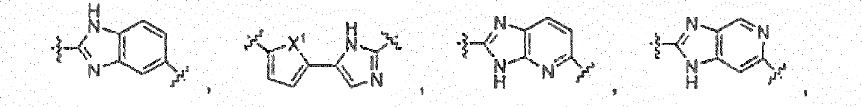 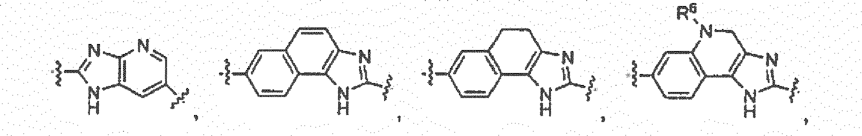 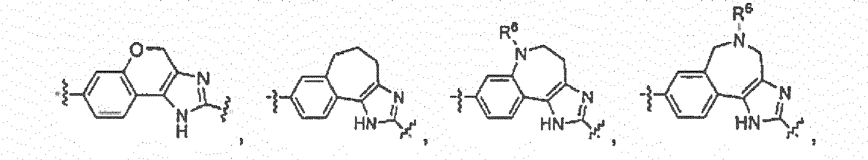 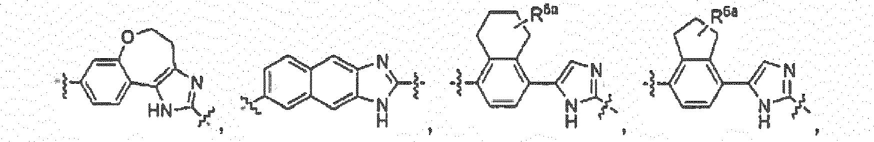 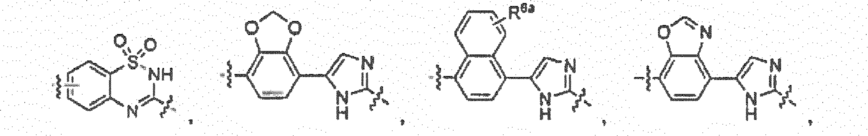 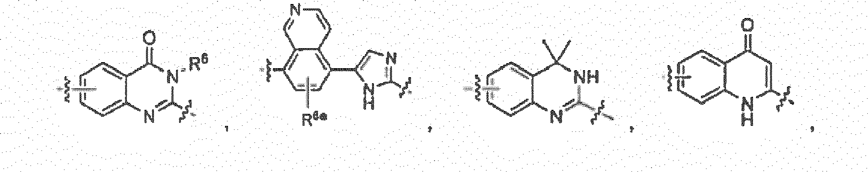 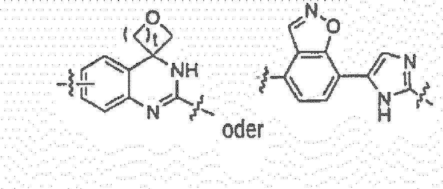 stehen;
Y¹ jeweils unabhängig für N oder CR⁷ steht;
R⁵ jeweils unabhängig für H, Deuterium, Hydroxy, C₁₋₆-Alkyl, C₂₋₆-Heteroalkyl, C₃₋₁₀-Cycloalkyl, C₂₋₁₀-Heterocyclyl, C₆₋₁₀-Aryl, C₁₋₉-Heteroaryl, C₆₋₁₀-Aryl-C₁₋₆-alkyl, C₁₋₆-Alkoxy, C₁₋₆-Alkyl-OC(=O)-, C₁₋₆-Alkyl-C(=O)-, Carbamoyl, C₁₋₆-Alkyl-OS(=O)ᵣ-, C₁₋₆-AlkylS(=O)ᵣO-, C₁₋₆-Alkyl-S(=O)ᵣ- oder Aminosulfonyl steht;
R^{5a} und R^{6a} jeweils unabhängig für H, Deuterium, Oxo (=O), Hydroxy, Amino, F, Cl, Br, I, Cyano, C₁₋₆-Alkylacyl, C₁₋₆-Alkylacyloxy, C₁₋₆-Alkoxyacyl, C₁₋₆-Alkylsulfonyl, C₁₋₆-Alkoxysulfonyl, C₁₋₆-Alkylsulfinyl, C₁₋₆-Alkylsulfonyloxy, C₁₋₆-Alkylsulfinyloxy, C₁₋₆-Alkoxy, C₁₋₆-Alkyl, C₆₋₁₀-Aryl, -CF₃, -OCF₃, Mercapto, Nitro, C₁₋₆-Alkylamino, C₃₋₁₀-Cycloalkyl oder C₆₋₁₀-Aryloxy stehen;
R⁶ jeweils unabhängig für H, Deuterium, R¹³R^{13a}NC(=O)-, R¹³OC(=O)-, R¹³C(=O)-, R¹³R^{13a}NS(=O)-, R¹³OS(-O)-, R¹³S(=O)-, R¹³R^{13a}NS(=O)₂-, R¹³OS(=O)₂-, R¹³S(-O)₂-, eine C₁₋₆-aliphatische, C₁₋₆-alkoxy-C₁₋₆-aliphatische, C₁₋₆-alkylamino-C₁₋₆-aliphatische, C₆₋₁₀-aryl-C₁₋₆-aliphatische, C₁₋₉-heteroaryl-C₁₋₆-aliphatische, C₂₋₁₀-heterocyclyl-C₁₋₆-aliphatische, C₃₋₁₀-cycloalkyl-C₁₋₆-aliphatische, C₆₋₁₀-Aryl-, C₁₋₉-Heteroaryl-, C₂₋₁₀-Heterocyclyl- oder C₃₋₁₀-Carbocyclyl-Gruppe steht;
R⁷ und R^{7a} jeweils unabhängig für H, Deuterium, F, Cl, Br, I, eine C₁₋₆-aliphatische, C₂₋₆-Heteroalkyl-, C₁₋₆-Alkoxy-C₁₋₆-aliphatische, C₁₋₆-Alkylamino-C₁₋₆-aliphatische, C₆₋₁₀-Aryl-C₁₋₆-aliphatische, C₂₋₁₀-Heterocyclyl-C₁₋₆₋aliphatische, C₃₋₁₀-Cycloalkyl-C₁₋₆-aliphatische, C₆₋₁₀-Aryl-, C₁₋₉-Heteroaryl-, C₂₋₁₀-Heterocyclyl- oder C₃₋₁₀-Carbocyclyl-Gruppe stehen;
R⁸ und R^{8a} jeweils unabhängig für H, Deuterium, Hydroxy, Cyano, Nitro, F, Cl, Br, I, C₁₋₆-Alkyl, C₂₋₆-Heteroalkyl, C₃₋₁₀-Cycloalkyl, C₂₋₁₀-Heterocyclyl, C₆₋₁₀-Aryl, C₁₋₉-Heteroaryl, C₆₋₁₀-Aryl-C₁₋₆-alkyl, C₁₋₆-Alkoxy, C₁₋₆-Alkyl-OC(=O)-, C₁₋₆-Alkyl-C(=O)-, Carbamoyl, C₁₋₆-Alkyl-OS(=O)ᵣ-, C₁₋₆-Alkyl-S(=O)ᵣO-, C₁₋₆-Alkyl-S(=O)ᵣ- oder Aminosulfonyl stehen;
R¹³ und R^{13a} jeweils unabhängig für H, Deuterium, C₁₋₆-Alkyl, C₂₋₆-Heteroalkyl, C₃₋₁₀-Cycloalkyl, C₂₋₁₀-Heterocyclyl, C₆₋₁₀-Aryl, C₁₋₉-Heteroaryl oder C₆₋₁₀-Aryl-C₁₋₆-alkyl stehen, mit der Vorgabe, dass, wenn R¹³ und R^{13a} an dasselbe Stickstoffatom gebunden sind, R¹³ und R^{13a} zusammen mit dem Stickstoffatom, woran sie gebunden sind, gegebenenfalls einen substituierten oder unsubstituierten 3- bis 8-gliedrigen Ring oder einen substituierten oder unsubstituierten Spiro-Ring oder annellierten bicyclischen Ring bilden;
n jeweils unabhängig 0, 1, 2 oder 3 ist;
p jeweils unabhängig 0, 1, 2 oder 3 ist;
r jeweils unabhängig 0, 1 ader 2 ist und
Y₄ und Y₄' jeweils unabhängig für eine Bindung, O, S, -(CH₂)ₙ-, -CH=CH-, -S(=O)ᵣ-, -CH₂O-, -CH₂S-, -CH₂F,-CHR^{5a}-, -CR^{5a}R^{6a}-, -CH₂S(=O)ᵣ- oder -CH₂N(R⁶)- stehen.

7. Verbindung nach Anspruch 5, welche die Formel (III) oder die Formel (IV) oder die Formel (V) aufweist: 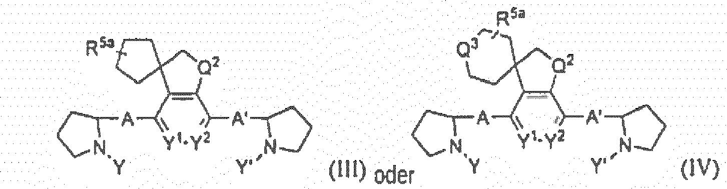 oder 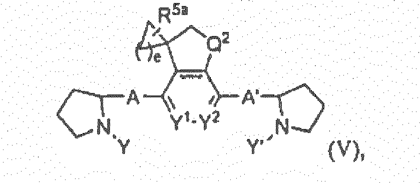
wobei Q² und Q³ jeweils unabhängig für O, S, C(=O), NR⁶ oder CH₂ stehen und
wobei e 1, 2, 3 oder 4 ist.

8. Verbindung nach Anspruch 6, welche die Formel (III') oder die Formel (IV') oder die Formel (V') aufweist: 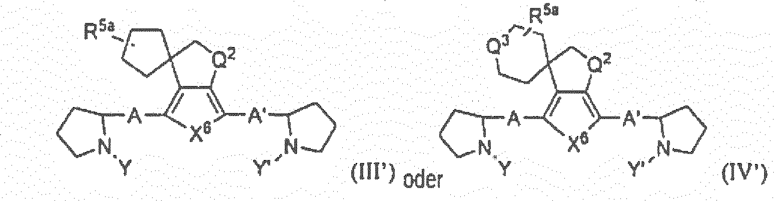 oder 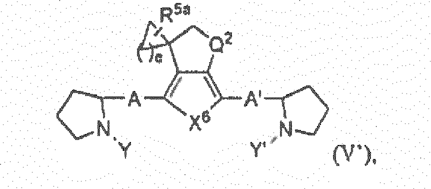
wobei Q² und Q³ jeweils unabhängig für O, S, C(=O), NR⁶ oder CH₂ stehen und
wobei e 1, 2, 3 oder 4 ist.

9. Verbindung nach Anspruch 1, wobei Y und Y' jeweils unabhängig für eine Gruppe stehen, die aus einer α-Aminosäure abgeleitet ist, und die Gruppe, die aus einer α-Aminosäure abgeleitet ist, gegebenenfalls mit einem oder mehreren Substituenten substituiert ist, wobei der Substituent Deuterium, F, Cl, Br, I, Hydroxy, Cyano, Alkoxycarbonyl, Aryloxycarbonyl, Heteroaryloxycarbonyl oder Heterocyclylcarbonyl ist; oder
wobei die α-Aminosäure Isoleucin, Leucin, Lysin, Methionin, Phenylalanin, Threonin, Tryptophan, Valin, Alanin, Asparagin, Aspartinsäure, Glutaminsäure Glutamin, Prolin, Serin, p-Tyrosin, Arginin, Histidin, Cystein, Glycin, Sarcosin, N,N-Dimethylglycin, Homoserin, Norvalin, Norleucin, Ornithin, Homocystein, Homophenylalanin, Phenylglycin, o-Tyrosin, m-Tyrosin oder Hydroxyprolin ist oder
wobei die α-Aminosäure in der D-Konfiguration vorliegt oder
wobei die α-Aminosäure in der L-Konfiguration vorliegt.

10. Verbindung nach einem der Ansprüche 1 bis 8, wobei Y und Y' jeweils unabhängig für -[U-(CR⁹R^{9a})ₜ-N(R¹⁰)-(CR⁹R^{9a})ₜ]ₖ-U-(CR⁹R^{9a})ₜ-N(R¹¹)-(CR⁹R^{9a})ₜ-R¹², -U-(CR⁹R^{9a})ₜ-R¹² oder -[U-(CR⁹R^{9a})ₜ-N(R¹⁰)-(CR⁹R^{9a})ₜ]ₖ-U-(CR⁹R^{9a})ₜ-O-(CR⁹R^{9a})ₜ-R¹² stehen oder
wobei Y und Y' jeweils unabhängig für -[U-(CR⁹R^{9a})ₜ-N (R¹⁰)-(CR⁹R^{9a})ₜ]ₖ-U-(CR⁹R^{9a})ₜ-N(R¹¹)-(CR⁹R^{9a})ₜ-R¹² stehen oder
wobei Y und Y' jeweils unabhängig für -U-(CR⁹R^{9a})ₜ-N(R¹⁰)-(CR⁹R^{3a})ₜ-U-(CR⁹R^{9a})ₜ-N(R¹¹)-(CR⁹R^{9a})ₜ-R¹² stehen oder
wobei Y und Y' jeweils unabhängig für -U-(CR⁹R^{9a})ₜ-N(R¹¹)-(CR⁹R^{9a})ₜ-R¹² stehen oder
wobei Y und Y' jeweils unabhängig für -[C(=O)-(CR⁹R^{9a})ₜ-N(R¹⁰)-(CR⁹R^{9a})ₜ]ₖ-C(=O)-(CR⁹R^{9a})ₜ-N(R¹¹)-(CR⁹R^{9a})ₜ-R¹² stehen oder
wobei Y und Y' jeweils unabhängig für -C(=O)-(CR⁹R^{9a})ₜ-N(R¹¹)-(CR⁹R^{9a})ₜ-U-(CR⁹R^{9a})ₜ-N(R¹¹)-(CR⁹R^{9a})ₜ-R¹² stehen oder
wobei Y und Y' jeweils unabhängig für -[C(=O)-(CR⁹R^{9a})ₜ-N(R¹⁰)-(CR⁹R^{9a})ₜ]ₖ-C(=O)-(CR⁹R^{9a})ₜ-N(R¹¹)-(CR⁹R^{9a})ₜ-R¹² stehen oder
wobei Y und Y' jeweils unabhängig für -C(=O)-(CR⁹R^{9a})ₜ-N(R¹⁰)-(CR⁹R^{9a})ₜ-C(=O)-(CR⁹R^{9a})ₜ-N(R¹¹)-(CR⁹R^{9a})ₜ-R¹² stehen oder
wobei Y und Y' jeweils unabhängig für -C(=O)-(CR⁹R^{9a})ₜ-N(R¹¹)-(CR⁹R^{9a})ₜ-R¹² stehen oder
wobei Y und Y' jeweils unabhängig für -C(=O)-(CR⁹R^{9a})ₙ-N(R¹¹)-(CR⁹R^{9a})ₙ-C(=O)-R¹³ stehen oder
wobei Y und Y' jeweils unabhängig für -C(=O)-(CR⁹R^{9a})ₙ-N(R¹¹)-C(=O)-R¹³ stehen oder
wobei Y und Y' jeweils unabhängig für -C(=O)-(CR⁹R^{9a})ₙ-N(R¹¹)-(CR⁹R^{9a})ₙ-C(=O)-R¹³ stehen oder
wobei Y und Y' jeweils unabhängig für -C(=O)-(CR⁹R^{9a})ₙ-N(R¹¹)-C(=O) -O-R¹³ stehen oder
wobei Y und Y' jeweils unabhängig für -U-(CR⁹R^{9a})ₜ-R¹² stehen oder
wobei Y und Y' jeweils unabhängig für -C(=O)-(CR⁹R^{9a})ᵣR¹² stehen oder
wobei Y und Y' jeweils unabhängig für -[U-(CR⁹R^{9a})ₜ-N(R¹⁰)-(CR⁹R^{9a})ₜ]ₖ-U-(CR⁹R^{9a})ₜ-O-(CR⁹R^{9a})ₜ-R¹² stehen oder wobei Y und Y' jeweils unabhängig für -U-(CR⁹R^{9a})ₜ-N(R¹⁰)-(CR⁹R^{9a})ₜ-U-(CR⁹R^{9a})ₜ-O-(CR⁹R^{9a})ₜ-R¹² stehen oder wobei Y und Y' jeweils unabhängig für -C(=O)-(CR⁹R^{9a})ₜ-N(R¹⁰)-(CR⁹R^{9a})ₜ-C(=O)-(CR⁹R^{9a})ₜ-O-(CR⁹R^{9a})ₜ-R¹² stehen oder
wobei Y und Y' jeweils unabhängig für -U-(CR⁹R^{9a})ₜ-O-(CR⁹R^{9a})ₜ-R¹² stehen oder
wobei Y und Y' jeweils unabhängig für -C(=O)-(CR⁹R^{9a})ₜ-O-(CR⁹R^{9a})ₜR¹² stehen oder
wobei Y und Y' jeweils unabhängig für -C(=O)-(CR⁹R^{9a})ₙ-N(R¹¹)-R¹² stehen,
wobei R¹¹ und R¹² zusammen mit dem Atom, woran sie gebunden sind, einen 4- bis 7-gliedrigen Ring bilden.

11. Verbindung nach Anspruch 10, wobei R⁹, R^{9a}, R¹⁰ und R¹¹ jeweils unabhängig für H, Deuterium, C₁₋₆-Alkyl, C₂₋₆-Heteroalkyl, C₃₋₁₀-Cycloalkyl, C₂₋₁₀-Heterocyclyl, C₆₋₁₀-Aryl, C₁₋₉-Heteroaryl, C₁₋₆-Haloalkyl, C₁₋₆-Hydroxyalkyl, C₆₋₁₀-Aryl-C₁₋₆-alkyl, C₁₋₉-Heteroaryl-C₁₋₆-alkyl, C₂₋₁₀-Heterocyclyl-C₁₋₆-alkyl oder C₃₋₈-Cycloalkyl-C₁₋₆-alkyl stehen;
R¹² jeweils unabhängig für R^{13a}R¹³N-, -C(=O)R¹³,-C(=S)R¹³, -C(=O)-O-R¹³, -C(=O)NR¹³R^{13a}, -OC(=O)NR¹³R^{13a}, OC(=O)OR¹³, -N(R¹³)C(=O)NR¹³R^{13a}, -N(R¹³)C(=O)OR^{13a},-N(R¹³)C(=O)-R^{13a}, R¹³R^{13a}N-S(=O)₂-, R¹³S(=O)₂-, R¹³S(=O)₂N(R^{13a})-, R¹³OS(=O)₂-, C₁₋₆-Alkyl, C₂₋₆-Heteroalkyl, C₃₋₁₀-Cycloalkyl, C₂₋₁₀-Heterocyclyl, C₆₋₁₀-Aryl, C₁₋₉-Heteroaryl oder C₆₋₁₀-Aryl-C₁₋₆-alkyl steht oder R¹¹ und R¹² zusammen mit dem Atom, woran sie gebunden sind, einen 4- bis 7-gliedrigen Ring bilden;
R¹³ und R^{13a} jeweils unabhängig für H, Deuterium, C₁₋₆-Alkyl, C₂₋₆-Heteroalkyl, C₃₋₁₀-Cycloalkyl, C₂₋₁₀-Heterocyclyl, C₆₋₁₀-Aryl, C₁₋₉-Heteroaryl oder C₆₋₁₀-Aryl-C₁₋₆-alkyl stehen, mit der Vorgabe, dass, wenn R¹³ und R^{13a} an dasselbe Stickstoffatom gebunden sind, R¹³ und R^{13a} zusammen mit dem Stickstoffatom, woran sie gebunden sind, gegebenenfalls einen substituierten oder unsubstituierten 3- bis 8-gliedrigen Ring oder einen substituierten oder unsubstituierten Spiro-Ring oder annellierten bicyclischen Ring bilden;
t jeweils unabhängig 0, 1, 2, 3 oder 4 ist und
k jeweils unabhängig 0, 1 oder 2 ist; oder
wobei R⁹, R^{9a}, R¹⁰ und R¹¹ jeweils unabhängig für H, Deuterium, Methyl, Ethyl, Isopropyl, Cyclohexyl, Isobutyl oder Phenyl stehen;
R¹² jeweils unabhängig für -C(=O)R¹³, -C(=O)-O-R¹³,-C(=O)NR¹³R^{13a}, Methyl, Ethyl, Propyl, Phenyl, Cyclohexyl, Morpholinyl oder Piperidinyl steht;
oder R¹¹ und R¹² zusammen mit dem Atom, woran sie gebunden sind, einen 4- bis 7-gliedrigen Ring bilden und
R¹³ und R^{13a} jeweils unabhängig für H, Deuterium, Methyl, Ethyl, Propyl, Phenyl, Cyclohexyl, Morpholinyl oder Piperidinyl stehen.

12. Verbindung nach Anspruch 5, welche die Formel (VI) aufweist: 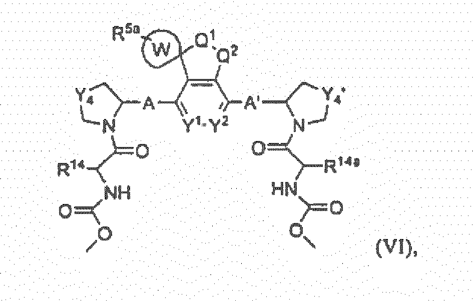
wobei R¹⁴ und R^{14a} jeweils unabhängig für H, Deuterium, C₁₋₆-Alkyl, C₁₋₆-Haloalkyl, C₁₋₆-Hydroxyalkyl, C₂₋₆-Heteroalkyl, C₆₋₁₀-Aryl, C₁₋₉-Heteroaryl, C₂₋₁₀-Heterocyclyl, C₃₋₈-Cycloalkyl, C₆₋₁₀-Aryl-C₁₋₆-alkyl, C₁₋₉-Heteroaryl-C₁₋₆-alkyl, C₂₋₁₀-Heterocyclyl-C₁₋₆-alkyl oder C₃₋₈-Cycloalkyl-C₁₋₆-alkyl stehen und
wobei jedes aus C₁₋₆-Alkyl, C₁₋₆-Haloalkyl, C₁₋₆-Hydroxyalkyl, C₂₋₆-Heteroalkyl, C₆₋₁₀-Aryl, C₁₋₉-Heteroaryl, C₂₋₁₀-Heterocyclyl, C₃₋₈-Cycloalkyl, C₆₋₁₀-Aryl-C₁₋₆-alkyl, C₁₋₉-Heteroaryl-C₁₋₆-alkyl, C₂₋₁₀-Heterocyclyl-C₁₋₆-alkyl und C₃₋₈-Cycloalkyl-C₁₋₆-alkyl gegebenenfalls mit einem oder mehreren Substituenten substituiert ist, wobei der Substituent Deuterium, F, Cl, Br, Hydroxy oder Cyano ist.

13. Verbindung nach Anspruch 12, welche die Formel (VII) aufweist: 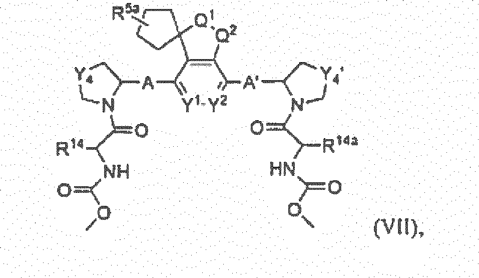
wobei R¹⁴ und R^{14a} jeweils unabhängig für H, Deuterium, C₁₋₃-Hydroxyalkyl, Methyl, Ethyl, Isopropyl, Isobutyl, tert.-Butyl, Allyl, Propargyl, Trifluoroethyl, Phenyl, Pyranyl, Morpholinyl, -NR¹³R^{13a}, Benzyl, Piperazinyl, Cyclopentyl, Cyclopropyl, Cyclohexyl oder C₁₋₉-Heteroaryl stehen und
wobei jedes aus Methyl, Ethyl, Isopropyl, Isobutyl, tert.-Butyl, Allyl, Propargyl, Trifluoroethyl, Phenyl, Pyranyl, Morpholinyl, Benzyl, Piperazinyl, Cyclopentyl, Cyclopropyl und Cyclohexyl gegebenenfalls mit einem oder mehreren Substituenten substituiert ist, wobei der Substituent Deuterium, F, Cl, Br, Hydroxy oder Cyano ist.

14. Verbindung nach Anspruch 1, welche die Formel (VIII) oder die Formel (IX) aufweist: 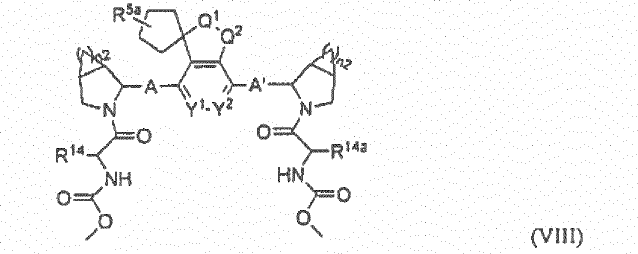 Oder 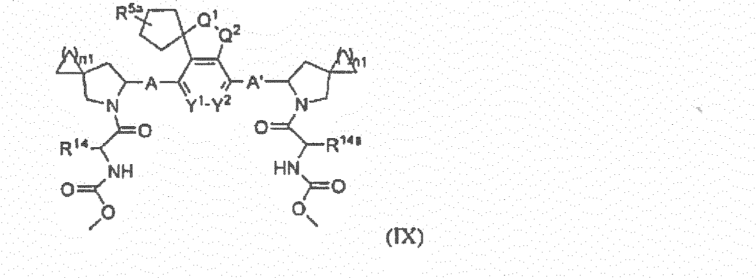
wobei R¹⁴ und R^{14a} jeweils unabhängig für H, Deuterium, C₁₋₆-Alkyl, C₁₋₆-Haloalkyl, C₁₋₆-Hydroxyalkyl, C₂₋₆-Heteroalkyl, C₆₋₁₀-Aryl, C₁₋₉-Heteroaryl, C₂₋₁₀-Heterocyclyl, C₃₋₈-Cycloalkyl, C₆₋₁₀-Aryl-C₁₋₆-alkyl, C₁₋₉-Heteroaryl-C₁₋₆-alkyl, C₂₋₁₀-Heterocyclyl-C₁₋₆-alkyl oder C₃₋₈-Cycloalkyl-C₁₋₆-alkyl stehen;
wobei jedes aus C₁₋₆-Alkyl, C₁₋₆-Haloalkyl, C₁₋₆-Hydroxyalkyl, C₂₋₆-Heteroalkyl, C₆₋₁₀-Aryl, C₁₋₉-Heteroaryl, C₂₋₁₀-Heterocyclyl, C₃₋₈-Cycloalkyl, C₆₋₁₀-Aryl-C₁₋₆-alkyl, C₁₋₉-Heteroaryl-C₁₋₆-alkyl, C₂₋₁₀-Heterocyclyl-C₁₋₆-alkyl und C₃₋₈-Cycloalkyl-C₁₋₆-alkyl gegebenenfalls mit einem oder mehreren Substituenten substituiert ist, wobei der Substituent Deuterium, F, Cl, Br, Hydroxy oder Cyano ist; und
n₁ und n₂ jeweils unabhängig 1, 2, 3 oder 4 sind.

15. Verbindung nach Anspruch 1, welche die Formel (X) aufweist: 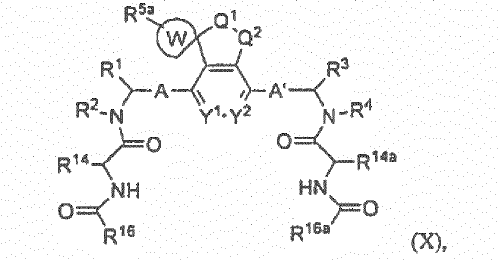
wobei R^{5a} für H, Deuterium, Methyl, Ethyl, F, Cl, Br oder I steht;
Q¹ für CH₂, C(=O), O, S oder NR⁶ steht;
Q² für CH₂, C(=O), CF₂, O, NR⁶ oder S steht;
Y¹ und Y² jeweils unabhängig für N oder CR⁷ stehen;
R⁶ und R⁷ jeweils unabhängig für H, Deuterium, Methyl, Ethyl, Isopropyl, Phenyl oder Cyclohexyl stehen;
R¹⁴ und R^{14a} jeweils unabhängig für H, Deuterium, Methyl, Ethyl, Phenyl, Cyclohexyl, 1-Methylpropyl, Isopropyl oder tert.-Butyl stehen;
R¹⁶ und R^{16a} jeweils unabhängig für Hydroxy, Methoxy, Ethoxy, Phenoxy, 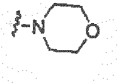 oder tert.-Butoxy stehen;
wobei jedes aus Methyl, Ethyl, Phenyl, Cyclohexyl, 1-Methylpropyl, Isopropyl, Methoxy, Ethoxy, tert.-Butoxy und tert.-Butyl gegebenenfalls mit einem oder mehreren Substituenten substituiert ist, wobei der Substituent Deuterium, F, Cl, Br, Hydroxy oder Cyano ist;
wobei 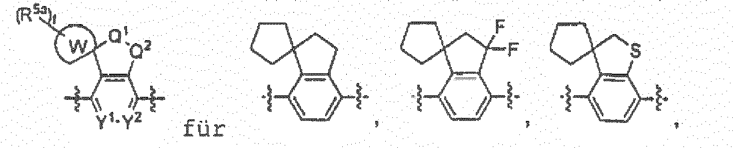 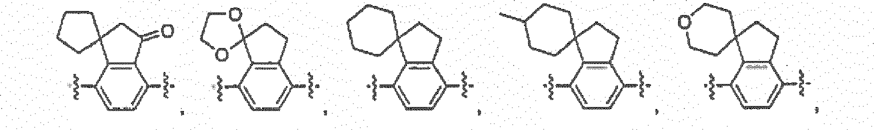 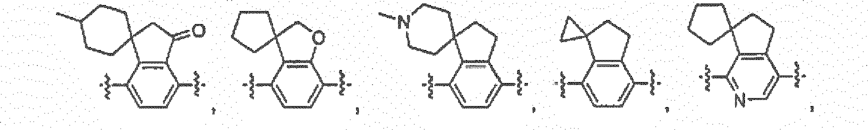 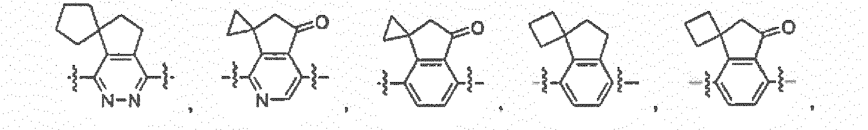 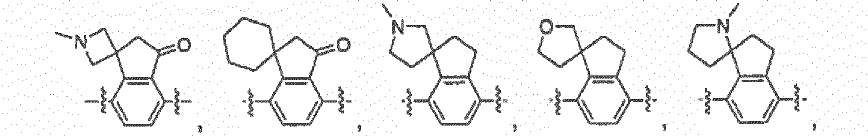 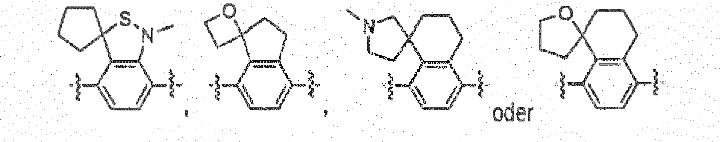 steht;
A und A' jeweils unabhängig für 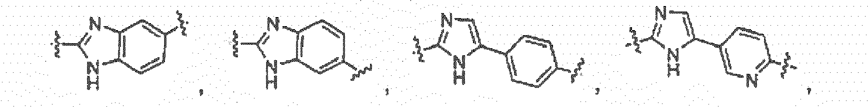 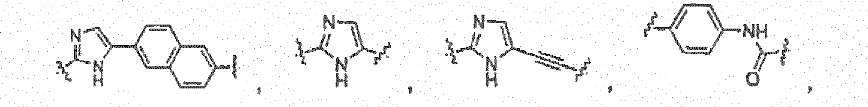 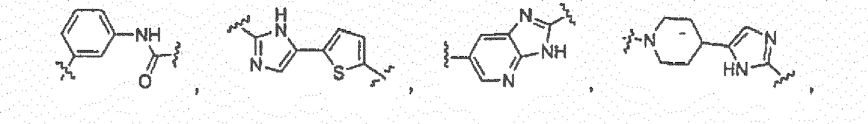 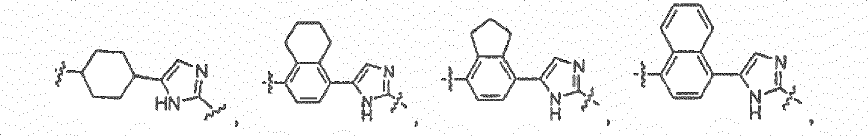 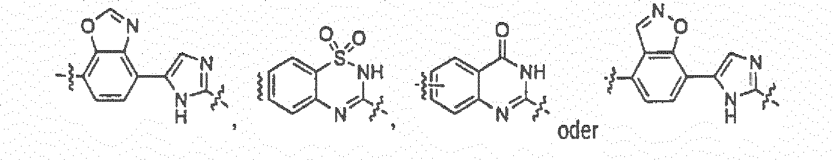 steht;
wobei R¹, R² und N-CH zusammen eine der folgenden bivalenten Gruppen bilden: 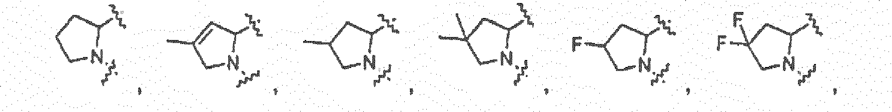 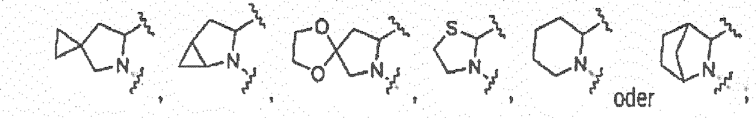 und
wobei R³, R⁴ und N-CH zusammen eine der folgenden bivalenten Gruppen bilden:

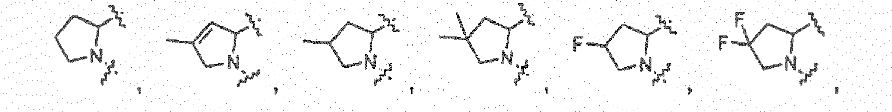

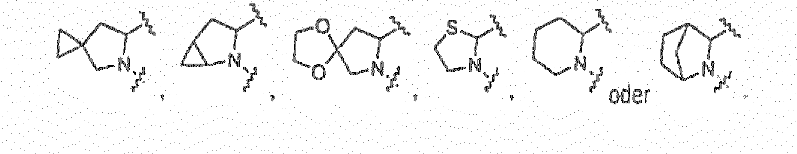

16. Verbindung nach Anspruch 15, welche die Formel (XI) aufweist: 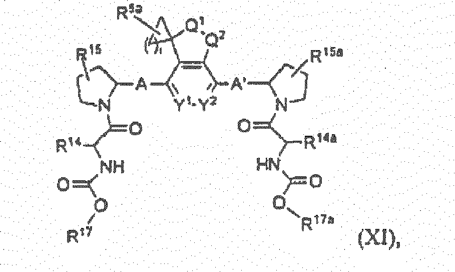
wobei i 1, 2, 3 oder 4 ist;
R^{5a} für Deuterium, H oder Methyl steht;
Q¹ und Q² jeweils unabhängig für (CH₂)ₑ, CF₂, S, NR⁶, O oder C(=O) stehen;
e 0, 1, 2, 3 oder 4 ist;
R⁶ für H, Deuterium, Methyl, Ethyl, Isobutyl, Cyclohexyl, Phenyl oder Isopropyl steht;
R¹⁴ und R^{14a} jeweils unabhängig für H, Deuterium, Methyl, Ethyl, Isobutyl, Cyclohexyl, Phenyl oder Isopropyl stehen;
R¹⁵ und R^{15a} jeweils unabhängig für H, Deuterium, F, Cl, Br, Methyl, Ethyl, Isopropyl oder tert.-Butyl stehen; und
R¹⁷ und R^{17a} jeweils unabhängig für Methyl, Phenyl oder Ethyl stehen;
wobei jedes aus Methyl, Ethyl, Phenyl, Cyclohexyl, Isopropyl, Isobutyl und tert.-Butyl gegebenenfalls mit einem oder mehreren Substituenten substituiert ist, wobei der Substituent Deuterium, F, Cl, Br, Hydroxy oder Cyano ist.

17. Verbindung nach Anspruch 1, welche die Formel (XI') aufweist: 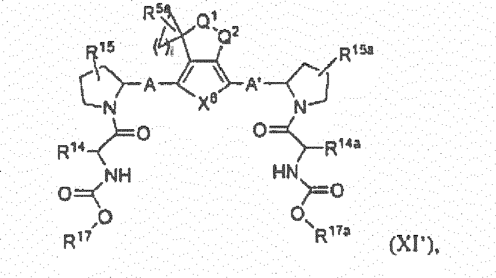
wobei i 1, 2, 3 oder 4 ist;
R^{5a} für H, Deuterium oder Methyl steht;
Q¹ und Q² jeweils unabhängig für (CH₂)ₑ, CF₂, S, NR⁶, O oder C(=O) stehen;
e 0, 1, 2, 3 oder 4 ist;
R⁶ jeweils unabhängig für H, Deuterium, Methyl, Ethyl, Isobutyl, Cyclohexyl, Phenyl oder Isopropyl steht;
R¹⁴ und R^{14a} jeweils unabhängig für H, Deuterium, Methyl, Ethyl, Isobutyl, Cyclohexyl, Phenyl oder Isopropyl stehen;
R¹⁵ und R^{15a} jeweils unabhängig für H, Deuterium, F, Cl, Br, Methyl, Ethyl, Isopropyl oder tert.-Butyl stehen;
R¹⁷ und R^{17a} jeweils unabhängig für Methyl, Phenyl oder Ethyl stehen;
wobei jedes aus Methyl, Ethyl, Phenyl, Cyclohexyl, Isopropyl, Isobutyl und tert.-Butyl gegebenenfalls mit einem oder mehreren Substituenten substituiert ist, wobei der Substituent Deuterium, F, Cl, Br, Hydroxy oder Cyano ist;
X⁶ unabhängig für CH₂, O, S oder NR⁶ steht und
A und A' jeweils unabhängig für 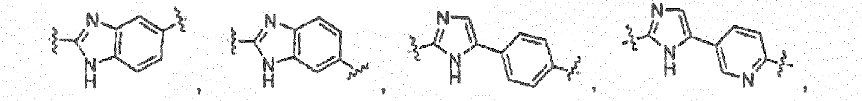 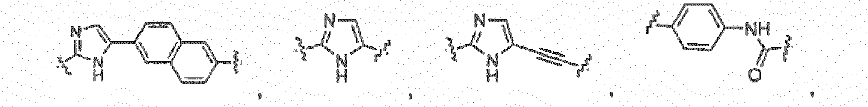 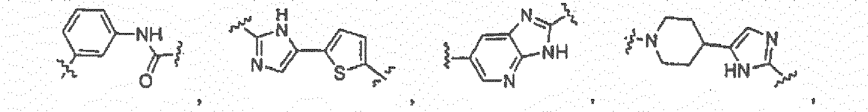 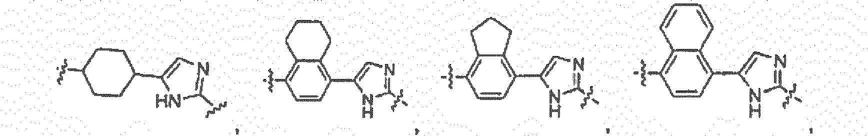 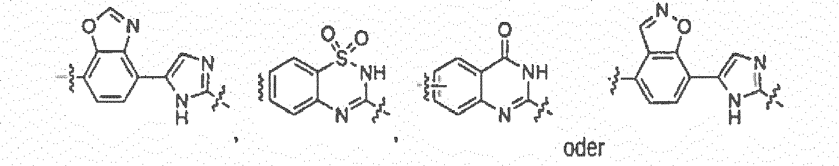 stehen.

18. Verbindung nach Anspruch 1, welche eine der folgenden Strukturen aufweist: 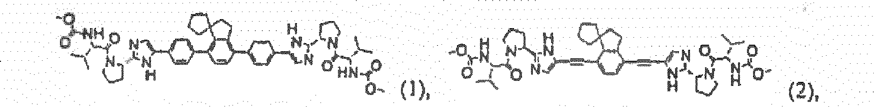 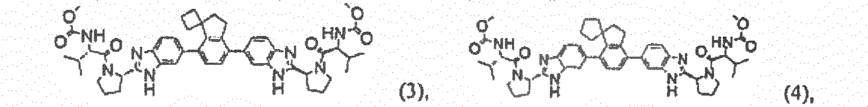 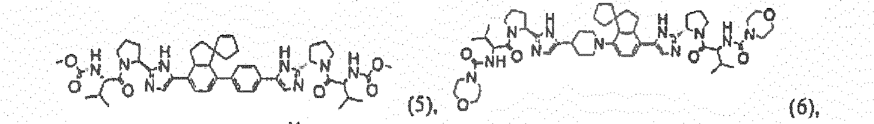 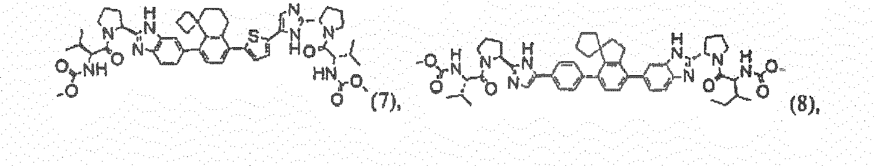 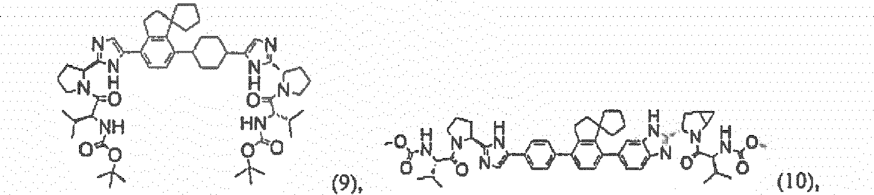 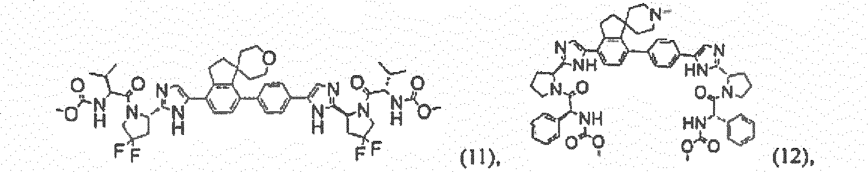 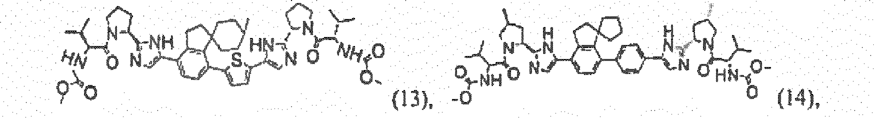 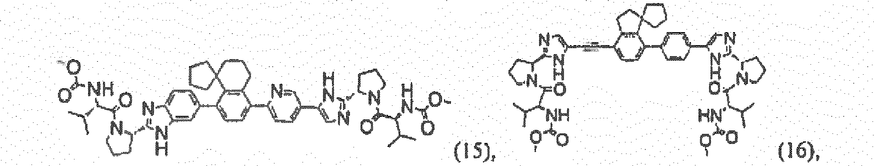 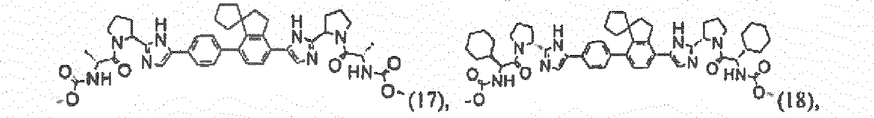 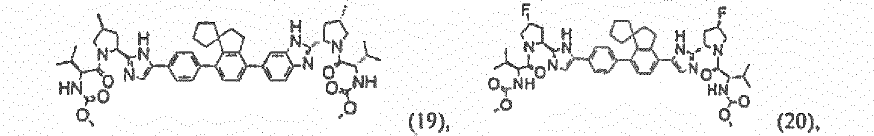 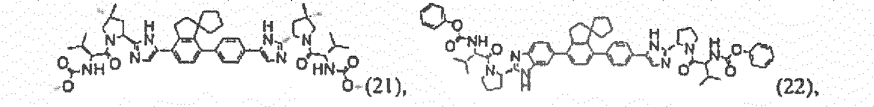 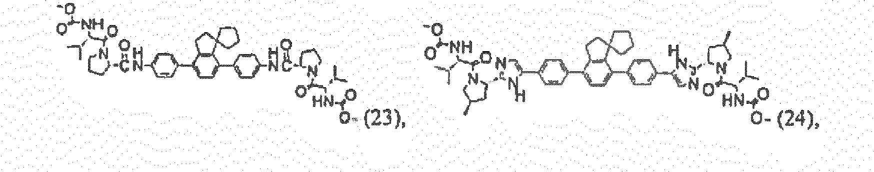 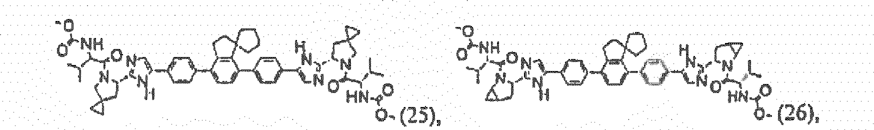 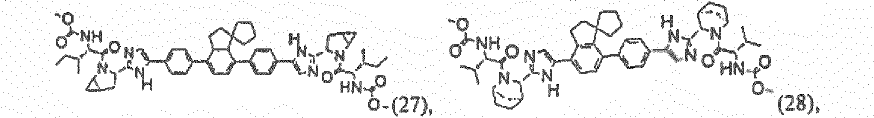 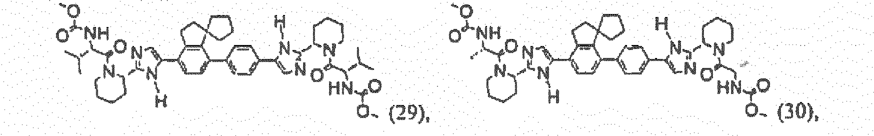 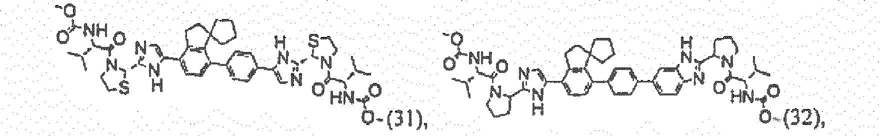 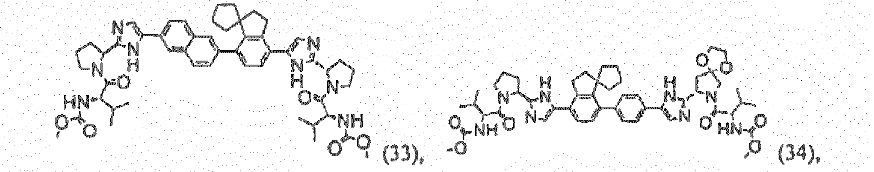 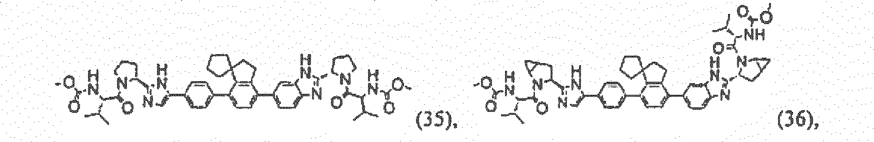 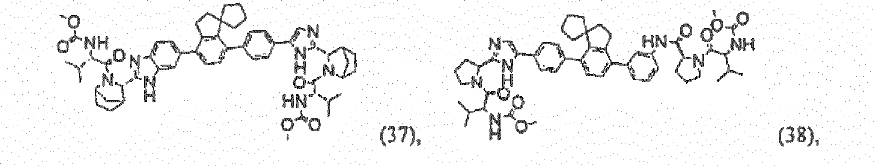 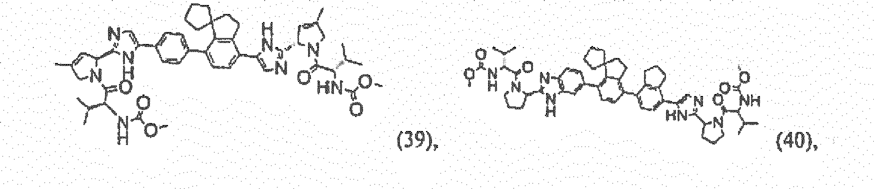 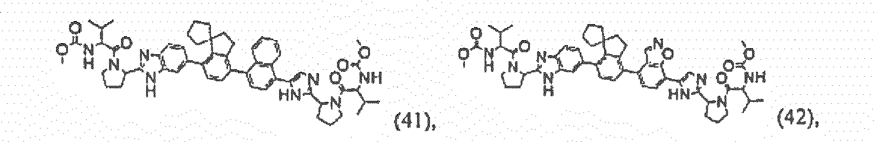 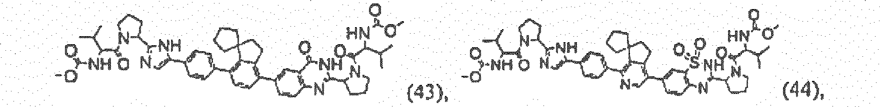 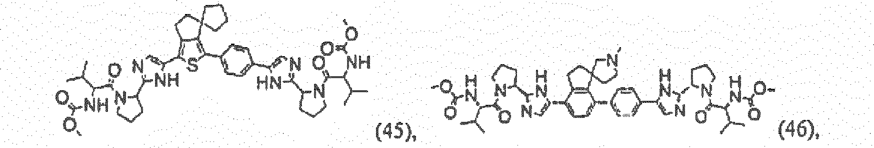 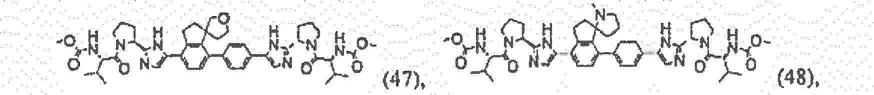 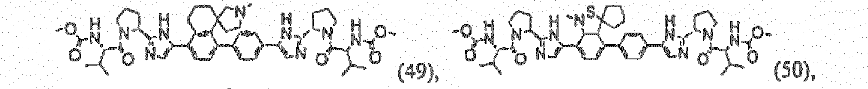 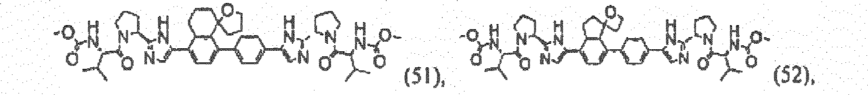 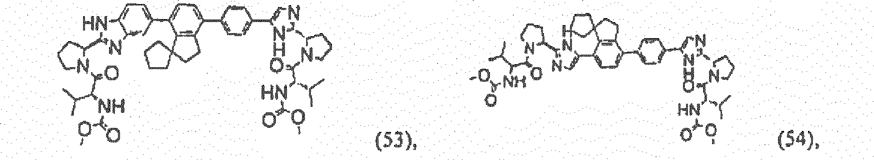 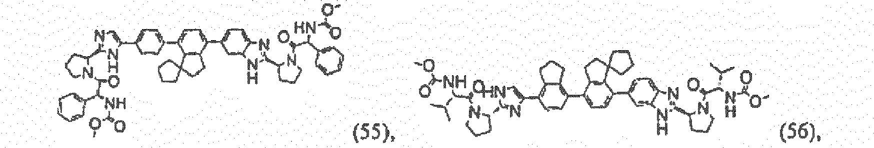 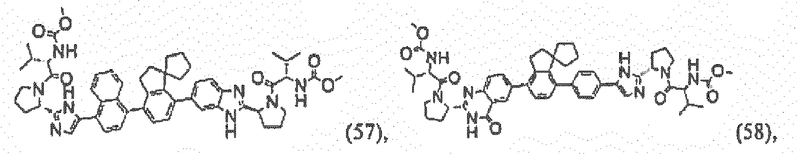 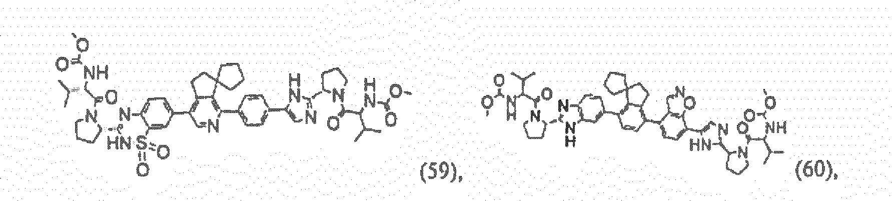 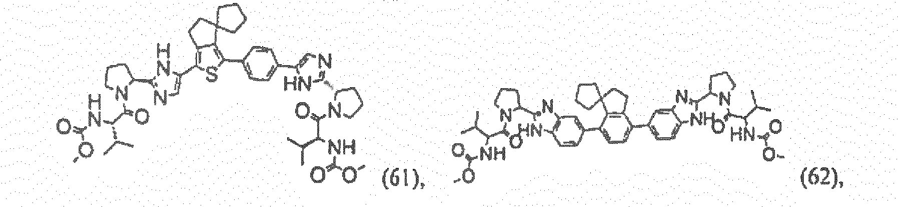 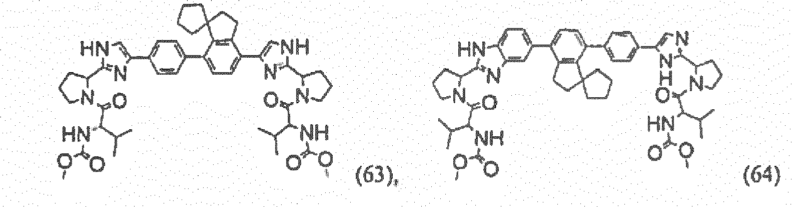 und 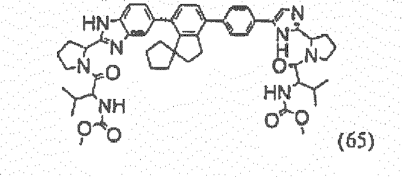 oder ein Stereoisomer, ein geometrisches Isomer, ein Tautomer, ein N-Oxid, ein Hydrat, ein Solvat oder ein pharmazeutisch akzeptables Salz davon.

19. Pharmazeutische Zusammensetzung, welche die Verbindung nach einem der Ansprüche 1 bis 18 und einen/eine/ein pharmazeutisch akzeptablen/akzeptable/akzeptables Träger, Salbengrundlage, Verdünnungsmittel, Hilfsstoff, Vehikel oder eine Kombination davon umfasst; oder
wobei die pharmazeutische Zusammensetzung ferner ein Anti-HCV-Mittel umfasst;
wobei das Anti-HCV-Mittel
Interferon, Ribavirin, IL-2, IL-6, IL-12, eine Verbindung, welche die Entwicklung einer Typ-1-T-Helfer-Zellen-Reaktion verstärkt, interferierende RNA, Antisense-RNA, Imiquimod, ein Inosin-5'-monophosphat-Dehydrogenase-Inhibitor, Amantadin, Rimantadin, Ribavirin, Bavituximab, menschliches Hepatitis-C-Immunglobulin (CIVACIR™), Boceprevir, Telaprevir, Erlotinib, Daclatasvir, Simeprevir, Asunaprevir, Vaniprevir, Faldaprevir, ABT-450, Danoprevir, Sovaprevir, MK-5172, Vedroprevir, BZF-961, GS-9256, Narlaprevir, ANA975, ABT-267, EDP239, PPI-668, GS-5816, Samatasvir (IDX-719), MK-8742, MK-8325, GSK-2336805, PPI-461, TMC-435, MK-7009, BI-2013335, Ciluprevir, BMS-650032, ACH-1625, ACH-1095, VX-985, IDX-375, VX-500, VX-813, PHX-1766, PHX-2054, IDX-136, IDX-316, EP-013420, VBY-376, TMC-649128, R-7128, PSI-7977, INX-189, IDX-184, IDX102, R1479, UNX-08189, PSI-6130, PSI-938, PSI-879, HCV-796, HCV-371, VCH-916, VCH-222, ANA-598, MK-3281, ABT-333, ABT-072, PF-00868554, BI-207127, GS-9190, A-837093, JKT-109, Gl-59728, GL-60667, AZD-2795, TMC647055 oder eine Kombination davon ist;
wobei das Interferon Interferon α-2b, pegyliertes Interferon α, Interferon α-2a, pegyliertes Interferon α-2a, Consensus-Interferon-α, Interferon Y oder eine Kombination davon ist; oder
wobei die pharmazeutische Zusammensetzung ferner mindestens einen HCV-Inhibitor umfasst; und
wobei der HCV-Inhibitor mindestens eines aus einem HCV-Replikationsverfahren und einer HCV-Virusproteinfunktion inhibiert, wobei das HCV-Replikationsverfahren ein gesamter Virenzyklus ist, bestehend aus HCV-Eintritt, Freisetzung, Translation, Replikation, Assembly und Austritt; und wobei das HCV-Virusprotein Helicase, Proteinase, Metalloproteinase, Serinproteinase, Nichtstrukturprotein NS4A, Nichtstrukturprotein NS4B, RNA-Polymerase des Nichtstrukturproteins NS5A oder Nichtstrukturproteins NS5B oder eine interne Ribosomeneintrittsstelle (Internal Ribosome Entry Site, IRES) und Inosin-5'-monophosphat-Dehydrogenase (IMPDH) ist, die in der HCV-Virusreplikation benötigt wird.

20. Verbindung nach einem der Ansprüche 1 bis 18 oder pharmazeutische Zusammensetzung nach Anspruch 19 zur Verwendung beim Inhibieren von mindestens einem aus einem HCV-Replikationsverfahren und einer HCV-Virusproteinfunktion, wobei das HCV-Replikationsverfahren ein gesamter Virenzyklus ist, bestehend aus HCV-Eintritt, Freisetzung, Translation, Replikation, Assembly und Austritt; und wobei das HCV-Virusprotein Helicase, Proteinase, Metalloproteinase, Serinproteinase, Nichtstrukturprotein NS4A, Nichtstrukturprotein NS4B, RNA-Polymerase des Nichtstrukturproteins NS5A oder Nichtstrukturproteins NS5B oder eine interne Ribosomeneintrittsstelle (IRES) und Inosin-5'-monophosphat-Dehydrogenase (IMPDH) ist, die in der HCV-Virusreplikation benötigt wird.

21. Verbindung nach einem der Ansprüche 1 bis 18 oder pharmazeutische Zusammensetzung nach Anspruch 19 zur Verwendung beim Verhindern, Handhaben, Behandeln oder Verringern der Schwere einer HCV-Infektion oder einer HCV-Erkrankung bei einem Patienten.

## Revendications

1. Composé de formule (I) : 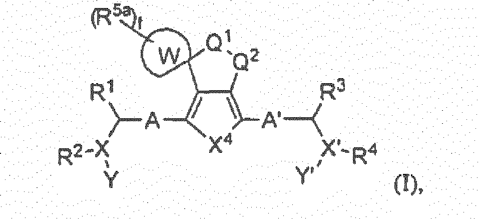 ou un stéréoisomère, un isomère géométrique, un tautomère, un N-oxyde, un hydrate, un solvate ou un sel pharmaceutiquement acceptable de celui-ci, dans laquelle
A et A' sont chacun indépendamment une liaison, un groupe alkylène, alcénylène, cycloalkylène, hétérocyclylalkylène, -(CR⁸R^{8a})ₙ-O-(CR⁸R^{8a})ₚ-, -(CR⁸R^{8a})ₙ-N(R⁵)-(CR⁸R⁸)ₚ-, -(CR⁸R^{8a})ₙ-S(=O)ᵣ-N(R⁵)-(CR⁸R^{8a})ₚ-,-(CR⁸R^{8a})ₙ-C(=O)-N(R⁵)-(CR⁸R^{8a})ₚ-, -(CR⁸R^{8a})ₙ-N(R⁵)-C(=O)-N(R⁵)-(CR⁸R^{8a})ₚ-, -(CR⁸R^{8a})ₙ-C(=O)-O-(CR⁸R^{8a})ₚ-,-(CR⁸R^{8a})ₙ-N(R⁵)-S(=O)ᵣ-N(R⁵)-(CR⁸R^{8a})ₚ- ou -(CR⁸R^{8a})ₙ-N(R⁵)-C(=O)-O-(CR⁸R^{8a})ₚ- ; ou A et A' sont chacun indépendamment 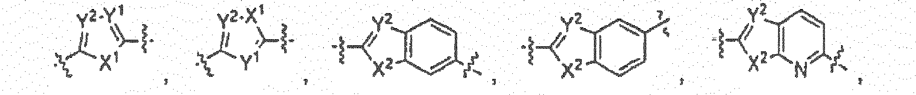 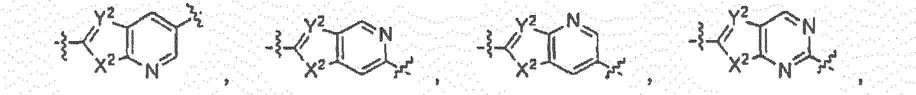 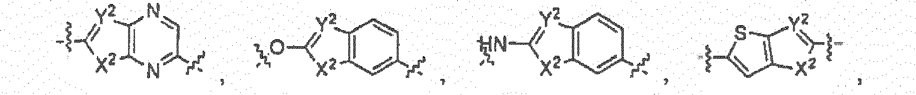 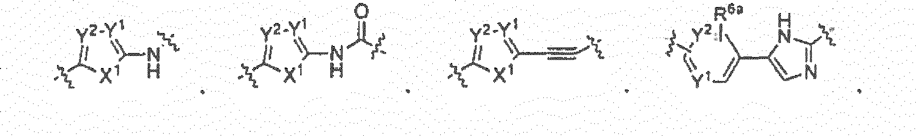 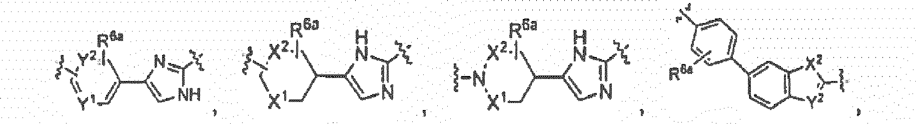 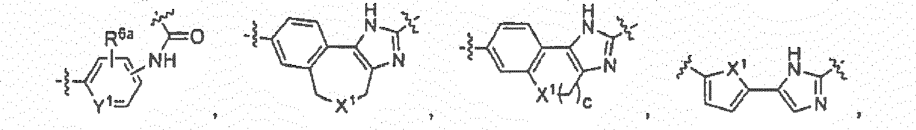 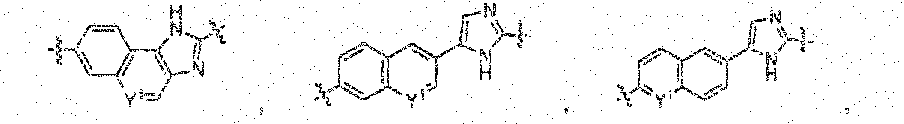 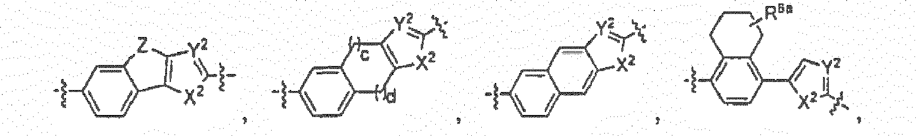 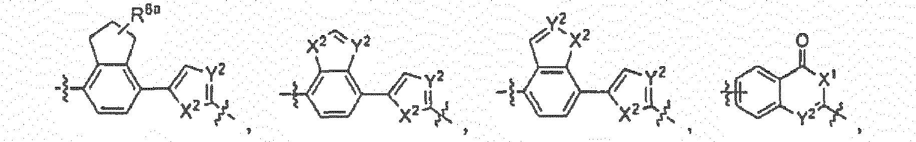 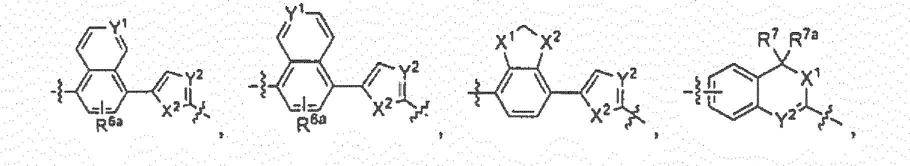 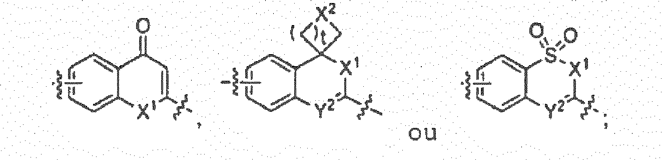
où chaque X¹ est indépendamment 0, S, NR⁶ ou CR⁷R^{7a} ;
chaque X² est indépendamment NR⁶, 0 ou S ;
X⁴ est (CR₇R₇ₐ)ₙ, -Y¹=Y²-, 0, S ou NR⁶ ;
W est un groupe carbocyclyle ou hétérocyclyle ;
Y¹ et Y² sont chacun indépendamment N ou CR⁷ ;
chaque Z est indépendamment -(CH₂)ₐ-, -CH=CH-, -N=CH-, -(CH₂)ₐ-N(R⁵)-(CH₂)_{b}- ou -(CH₂)ₐ-O-(CH₂)_{b}-, ou chaque a et b est indépendamment 0, 1, 2 ou 3 ;
chaque c est indépendamment 1 ou 2 ;
chaque d est indépendamment 1 ou 2 ;
chaque n est indépendamment 0, 1, 2 ou 3 ;
chaque p est indépendamment 0, 1, 2 ou 3 ;
chaque r est indépendamment 0, 1 ou 2 ;
f est 0, 1, 2, 3 ou 4 ;
Q¹ et Q² sont chacun indépendamment NR⁶, 0, S, C(=O) ou (CR⁷R^{7a})ₑ ;
e est 0, 1, 2, 3 ou 4 ;
X et X' sont chacun indépendamment N ou CR⁷ ;
Y et Y' sont chacun indépendamment H, un groupe deutérium, alkyle, hétéroalkyle, cycloalkyle, hétérocyclyle, aryle, hétéroaryle, aralkyle, un groupe dérivé d'un acide α-aminé ou d'un de ses isomères optiques ; ou chacun de Y et Y' est indépendamment - [U-(CR⁹R^{9a})ₜ-N(R¹⁰)-(CR⁹R^{9a})ₜ]ₖ-U-(CR⁹R^{9a})ₜ-N(R¹¹)-(CR⁹R^{9a})ₜ-R¹², -U-(CR⁹R^{9a})ₜ-R¹² ou -[U-(CR⁹R^{9a})ₜ-N(R¹⁰)-(CR⁹R^{9a})ₜ]ₖ-U-(CR⁹R^{9a})ₜ-O-(CR⁹R^{9a})ₜ-R¹² ;
chaque U est indépendamment -C(=O)-, -C(=S)-, -S(=O)- ou -S(=O)₂- ;
chaque t est indépendamment 0, 1, 2, 3 ou 4 ;
chaque k est indépendamment 0, 1 ou 2 ;
R¹ et R², ensemble avec X-CH auquel ils sont attachés, forment facultativement un hétérocycle ou un carbocycle de 3 à 8 chaînons, un bicycle condensé en C₅ à C₁₂, un hétérobicycle condensé en C₅ à C₁₂, un bicycle spiro en C₅ à C₁₂ ou un hétérobicycle spiro en C₅ à C₁₂ ; ; et R³ et R⁴, ensemble avec X'-CH auquel ils sont attachés, forment facultativement un hétérocycle ou un carbocycle de 3 à 8 chaînons, un bicycle condensé en C₅ à C₁₂, un hétérobicycle condensé en C₅ à C₁₂, un bicycle spiro en C₅ à C₁₂ ou un hétérobicycle spiro en C₅ à C₁₂ ;
chaque R⁵ est indépendamment H, un groupe deutérium, hydroxy, alkyle, hétéroalkyle, cycloalkyle, hétérocyclyle, aryle, hétéroaryle, aralkyle, alcoxy, alkyl-OC(=O)-, alkyl-C(=O)-, carbamoyle, alkyl-OS(=O)ᵣ-, alkyl-S(=O)ᵣO-, alkyl-S(=O)ᵣ- ou aminosulfonyle ;
chaque R^{5a} est indépendamment H, un groupe deutérium, oxo (=0), hydroxy, amino, F, CI, Br, I, cyano, R^{13a}R¹³N-, -C(=O)NR¹³R^{13a}, -OC(=O)NR¹³R^{13a}, -OC(=O)OR¹³,-N(R¹³)C(=O)NR¹³R^{13a}, -N(R¹³)C(=O)OR¹³, -N(R¹³)C(=O)-R¹³, R¹³R^{13a}N-S(=O)₂-, R¹³S(=O)₂-, R¹³S(=O)₂N(R^{13a})-, R^{13a}R¹³N-alkyle, R¹³S(=O)-alkyle, R¹³R^{13a}N-C(=O)-alkyle, R^{13a}R¹³N-alcoxy, R¹³S(=O)-alcoxy, R¹³R^{13a}N-C(=O)-alcoxy, aryle, hétéroaryle, alcoxy, alkylamino, alkyle, halogénoalkyle, alcényle, alcynyle, hétérocyclyle, cycloalkyle, mercapto, nitro, aralkyle, arylamino, hétéroarylamino, arylalkylamino, hétéroarylalkylamino, hétéroaryloxy, hétéroarylalkyle, arylalcoxy, hétéroarylalcoxy, hétérocyclyloxy, hétérocyclylalcoxy, hétérocyclylamino, hétérocyclylalkylamino ou aryloxy ;
chaque R⁶ est indépendamment H, un groupe deutérium, R¹³R^{13a}NC(=O)-, R¹³OC(=O)-, R¹³C(=O)-, R¹³R^{13a}NS(=O)-, R¹³OS(=O)-, R¹³S(=O)-, R¹³R^{13a}NS(=O)₂-, R¹³OS(=O)₂-, R¹³S(=O)₂-, aliphatique, halogénoaliphatique, hydroxyaliphatique, aminoaliphatique, alcoxyaliphatique, alkylaminoaliphatique, alkylthioaliphatique, arylaliphatique, hétéroarylaliphatique, hétérocyclylaliphatique, cycloalkylaliphatique, aryloxyaliphatique, hétérocyclyloxyaliphatique, cycloalkyloxyaliphatique, arylaminoaliphatique, hétérocyclylaminoaliphatique, cycloalkylaminoaliphatique, aryle, hétéroaryle, hétérocyclyle ou carbocyclyle ;
chaque R^{6a} est indépendamment H, un groupe deutérium, hydroxy, amino, F, CI, Br, I, cyano, oxo (=0), R^{13a}R¹³N-, -C(=O)NR¹³R^{13a}, -OC(=O)NR¹³R^{13a}, -OC(=O)OR¹³,-N(R¹³)C(=O)NR¹³R^{13a}, -N(R¹³)C(=O)OR^{13a}, -N(R¹³)C(=O)-R^{13a}, R¹³R^{13a}N-S(=O)₂-, R¹³S(=O)₂-, R¹³S(=O)₂N(R^{13a})-, R^{13a}R¹³N-alkyle, R¹³S(=O)-alkyle, R¹³R^{13a}N-C(=O)-alkyle, R^{13a}R¹³N-alcoxy, R¹³S(=O)-alcoxy, R¹³R^{13a}N-C(=O)-alcoxy, aryle, hétéroaryle, alcoxy, alkylamino, alkyle, halogénoalkyle, alcényle, alcynyle, hétérocyclyle, cycloalkyle, mercapto, nitro, aralkyle, arylamino, hétéroarylamino, arylalkylamino, hétéroarylalkylamino, hétéroaryloxy, hétéroarylalkyle, arylalcoxy, hétéroarylalcoxy, hétérocyclyloxy, hétérocyclylalcoxy, hétérocyclylamino, hétérocyclylalkylamino ou aryloxy ;
R⁷ et R^{7a} sont chacun indépendamment H, un groupe deutérium, F, CI, Br, I, aliphatique, hétéroalkyle, halogénoaliphatique, hydroxyaliphatique, aminoaliphatique, alcoxyaliphatique, alkylaminoaliphatique, alkylthioaliphatique, arylaliphatique, hétéroarylaliphatique, hétérocyclylaliphatique, cycloalkylaliphatique, aryloxyaliphatique, hétérocyclyloxyaliphatique, cycloalkyloxyaliphatique, arylaminoaliphatique, hétérocyclylaminoaliphatique, cycloalkylaminoaliphatique, aryle, hétéroaryle, hétérocyclyle ou carbocyclyle ;
R⁸ et R^{8a} sont chacun indépendamment H, un groupe deutérium, hydroxy, cyano, nitro, F, CI, Br, I, alkyle, hétéroalkyle, cycloalkyle, hétérocyclyle, aryle, hétéroaryle, aralkyle, alcoxy, alkyl-OC(=O)-, alkyl-C(=O)-, carbamoyle, alkyl-OS(=O)ᵣ-, alkyl-S(=O)ᵣO-, alkyl-S(=O)r- ou aminosulfonyle ;
R⁹, R^{9a}, R¹⁰ et R¹¹ sont chacun indépendamment H, un groupe deutérium, alkyle, hétéroalkyle, cycloalkyle, hétérocyclyle, aryle, hétéroaryle, aralkyle, halogénoalkyle, hydroxyalkyle, hétéroarylalkyle, hétérocyclylalkyle ou cycloalkylalkyle ;
chaque R¹² est indépendamment R^{13a}R¹³N-, -C(=O)R¹³,-C(=S)R¹³, -C(=O)OR¹³, -C(=O)NR¹³R^{13a}, -OC(=O)NR¹³R^{13a},-OC(=O)OR¹³, -N(R¹³)C(=O)NR¹³R^{13a}, -N(R¹³)C(=O)OR^{13a},-N(R¹³)C(=O)-R^{13a}, R¹³R^{13a}N-S(=O)₂-, R¹³S(=O)₂-, R¹³S(=O)₂N(R^{13a})-, R¹³OS(=O)₂-, alkyle, hétéroalkyle, cycloalkyle, hétérocyclyle, aryle, hétéroaryle ou aralkyle ;
ou R¹¹ et R¹² sont facultativement liés pour former un cycle de 4 à 7 chaînons ; et
R¹³ et R^{13a} sont chacun indépendamment H, un groupe deutérium, alkyle, hétéroalkyle, cycloalkyle, hétérocyclyle, aryle, hétéroaryle ou aralkyle ; à condition que lorsque R¹³ et R^{13a} sont liés au même atome d'azote, R¹³ et R^{13a}, ensemble avec l'atome d'azote auquel ils sont attachés, forment facultativement un cycle de 3 à 8 chaînons substitué ou non substitué ou un cycle bicyclique spiro ou condensé substitué ou non substitué ;
où les groupes alkylène, alcénylène, cycloalkylène, hétérocyclylalkylène, -(CR⁸R^{8a})ₙ-O-(CR⁸R^{8a})ₚ-, -(CR⁸R^{8a})ₙ-N(R⁵)-(CR⁸R^{8a})ₚ-, -(CR⁸R^{8a})ₙ-(S=O)ᵣ-N(R⁵)-(CR⁸R^{8a})ₚ-,-(CR⁸R^{8a})ₙ-C(=O)-N(R⁵)-(CR⁸R^{8a})ₚ-, -(CR⁸R^{8a})ₙ-N(R⁵)-C(=O)-N(R⁵)-(CR⁸R^{8a})ₚ-, -(CR⁸R^{8a})ₙ-C(=O)-O-(CR⁸R^{8a})ₚ-,-(CR⁸R^{8a})ₙ-N(R⁵)-S(=O)ᵣ-N(R⁵)-(CR⁸R^{8a})ₚ-, -(CR⁸R^{8a})ₙ-N(R⁵)-C(=O)-O-(CR⁸R^{8a})ₚ-, -[U-(CR⁹R^{9a})ₜ-N(R¹⁰)-(CR⁹R^{9a})ₜ]ₖ-U-(CR⁹R^{9a})ₜ-N(R¹¹)-(CR⁹R^{a})ₜ-R¹², -U-(CR⁹R^{9a})ₜ-R¹², -[U-(CR⁹R^{9a})ₜ-N(R¹⁰)-(CR⁹R^{9a})ₜ]ₖ-U-(CR⁹R^{9a})ₜ-O-(CR⁹R^{9a})ₜ-R¹², NR⁶, CR⁷R^{7a}, CR⁷, -(CH₂)ₐ-, -CH=CH-, -N=CH-, -(CH₂)ₐ-N(R⁵)-(CH₂)_{b}-, -(CH₂)ₐ-O-(CH₂)_{b}-, R^{13a}R¹³N-, -C(=O)R¹³,-C(=S)R¹³, -C(=O)OR¹³, -C(=O)NR¹³R^{13a}, -OC(=O)NR¹³R^{13a},-OC(=O)OR¹³, -N(R¹³)C(=O)NR¹³R^{13a}, -N(R¹³)C(=O)OR^{13a},-N(R¹³)C(=O)-R^{13a}, R¹³R^{13a}N-S(=O)₂-, R¹³S(=O)₂-, R¹³S(=O)₂N(R^{13a})-, R¹³OS(=O)₂-, alkyl-OC(=O)-, alkyl-C(=0)-, alkyl-OS(=O)ᵣ-, alkyl-S(=O)ᵣO-, alkyl-S(=O)ᵣ-, R¹³R^{13a}NC(=O)-, R¹³OC(=O)-, R¹³C(=O)-, R¹³R^{13a}NS(=O)-, R¹³OS(=O)-, R¹³S(=O)-, R^{13a}R¹³N-alkyle, R¹³S(=O)-alkyle, R¹³R^{13a}N-C(=O)-alkyle, R^{13a}R¹³N-alcoxy, R¹³S(=O)-alcoxy, R^{13a}R¹³N-C(=O)-alkylamino, alkyle, hétéroalkyle, carbocyclyle, cycloalkyle, hétérocyclyle, aryle, hétéroaryle, aralkyle, un groupe dérivé d'un acide α-aminé, un bicycle condensé en C₅ à C₁₂, un hétérobicycle condensé en C₅ à C₁₂, un bicycle spiro en C₅ à C₁₂, un hétérobicycle spiro en C₅ à C₁₂, les groupes alcoxy, aliphatique, halogénoaliphatique, hydroxyaliphatique, aminoaliphatique, alcoxyaliphatique, alkylaminoaliphatique, alkylthioaliphatique, arylaliphatique, hétéroarylaliphatique, hétérocyclylaliphatique, cycloalkylaliphatique, aryloxyaliphatique, hétérocyclyloxyaliphatique, cycloalkyloxyaliphatique, arylaminoaliphatique, hétérocyclylaminoaliphatique, cycloalkylaminoaliphatique, halogénoalkyle, alcényle, alcynyle, arylamino, hétéroarylamino, arylalkylamino, hétéroarylalkylamino, hétéroaryloxy, hétéroarylalkyle, arylalcoxy, hétéroarylalcoxy, hétérocyclyloxy, hétérocyclylalcoxy, hétérocyclylamino, hétérocyclylalkylamino et aryloxy sont chacun facultativement substitués par un ou plusieurs substituants, le substituant étant un groupe deutérium, hydroxy, amino, halogéno, cyano, aryle, hétéroaryle, alcoxy, alkylamino, alkylthio, alkyle, alcényle, alcynyle, hétérocyclyle, mercapto, nitro, aryloxy, hétéroaryloxy, oxo (=O), carboxy, alcoxy substitué par un groupe hydroxy, alkyl-C(=O)-substitué par un groupe hydroxy, alkyl-C(=O)-, alkyl-S(=O)-, alkyl-S(=O)₂-, alkyl-S(=O)- substitué par un groupe hydroxy, alkyl-S(=O)₂- substitué par un groupe hydroxy ou alcoxy substitué par un groupe carboxy ; ou dans laquelle W est un groupe carbocyclyle en C₃ à C₈ ou hétérocyclyle en C₂ à C₁₀.

2. Composé selon la revendication 1, dans lequel 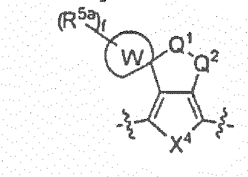 est 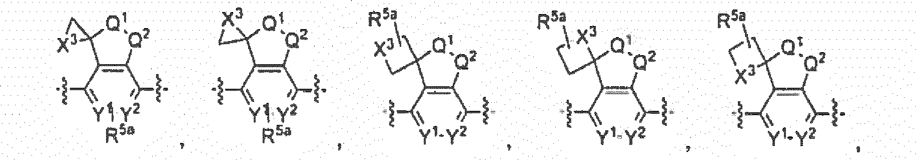 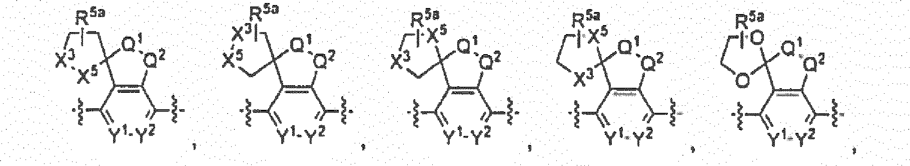 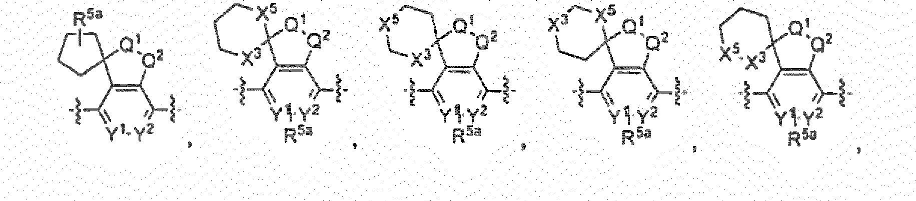 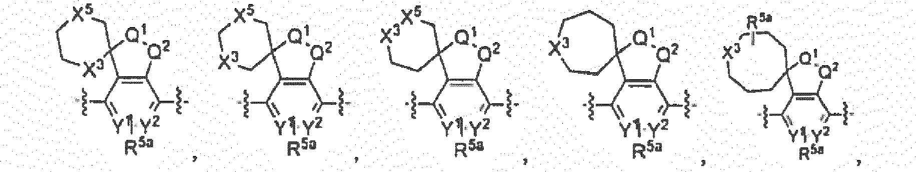 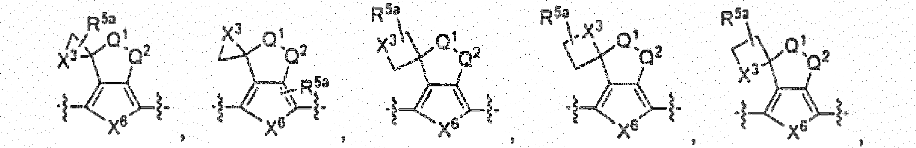 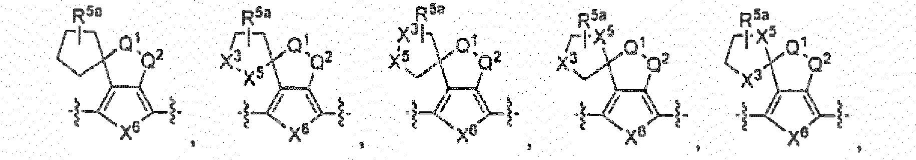 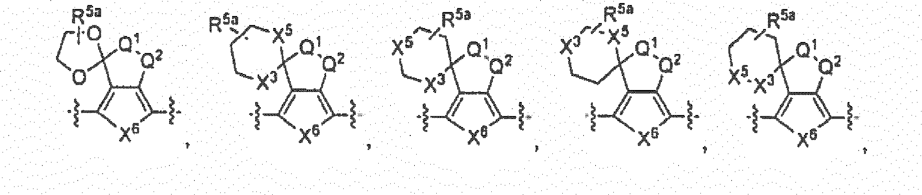 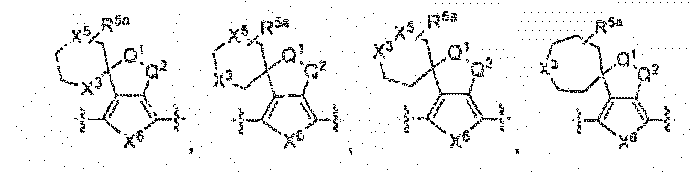 ou 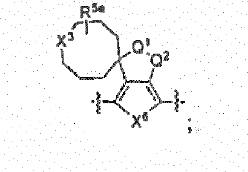
où X³ et X⁵ sont chacun indépendamment O, S, NR⁶ ou CR⁷R^{7a} ;
chaque X⁶ est indépendamment CR⁷R^{7a}, O, S ou, NR⁶ ;
Y¹ et Y² sont chacun indépendamment N ou CR⁷ ;
f est 0, 1, 2, 3 ou 4 ;
Q¹ et Q² sont chacun indépendamment NR⁶, O, S, C(=O) ou (CR⁷R^{7a})ₑ ;
e est 0, 1, 2, 3 ou 4 ;
chaque R^{5a} est indépendamment H, un groupe deutérium, oxo (=O), hydroxy, amino, F, CI, Br, I, cyano, (alkyle en C₁ à C₆)acyle, (alkyle en C₁ à C₆)acyloxy, (alcoxy en C₁ à C₆)acyle, (alkyle en C₁ à C₆)sulfonyle, (alcoxy en C₁ à C₆)sulfonyle, (alkyle en C₁ à C₆)sulfinyle, (alkyle en C₁ à C₆)sulfonyloxy, (alkyle en C₁ à C₆)sulfinyloxy, alcoxy en C₁ à C₆, alkyle en C₁ à C₆, aryle en C₆ à C₁₀, -CF₃, -OCF₃, mercapto, nitro, (alkyle en C₁ à C₆)amino, cycloalkyle en C₃ à C₁₀ ou aryloxy en C₃ à C₁₀ ;
chaque R⁶ est indépendamment H, un groupe deutérium, R¹³R^{13a}NC(=O)-, R¹³OC(=O)-, R¹³C(=O)-, R¹³R^{13a}NS(=O)-, R¹³OS(=O)-, R¹³S(=O)-, R¹³R^{13a}NS(=O)₂-, R¹³OS(=O)₂-, R³³S(=O)₂-, alkyle en C₁ à C₆, halogénoalkyle en C₁ à C₆, hydroxyalkyle en C₁ à C₆, aminoalkyle en C₁ à C₆, (alcoxy en C₁ à C₆)alkyle en C₁ à C₆, (alkyle en C₁ à C₆) aminoalkyle en C₁ à C₆, (alkyle en C₁ à C₆)thioalkyle en C₁ à C₆, (aryle en C₆ à C₁₀)alkyle en C₁ à C₆, (hétéroaryle en C₁ à C₉)alkyle en C₁ à C₆, (hétérocyclyle en C₂ à C₁₀)alkyle en C₁ à C₆, (cycloalkyle en C₃ à C₁₀)alkyle en C₁ à C₆, (aryloxy en C₆ à C₁₀)alkyle en C₁ à C₆, (hétérocyclyloxy en C₂ à C₁₀)alkyle en C₁ à C₆, (cycloalkyloxy en C₃ à C₁₀)alkyle en C₁ à C₆, (aryle en C₆ à C₁₀)aminoalkyle en C₁ à C₆, (hétérocyclyle en C₂ à C₁₀)aminoalkyle en C₁ à C₆, (cycloalkyle en C₃ à C₁₀)aminoalkyle en C₁ à C₆, aryle en C₆ à C₁₀, hétéroaryle en C₁ à C₉, hétérocyclyle en C₂ à C₁₀ ou carbocyclyle en C₃ à C₁₀ ;
R⁷ et R^{7a} sont chacun indépendamment H, un groupe deutérium, F, CI, Br, I, alkyle en C₁ à C₆, hétéroalkyle en C₂ à C₆, halogénoalkyle en C₁ à C₆, hydroxyalkyle en C₁ à C₆, aminoalkyle en C₁ à C₆, (alcoxy en C₁ à C₆)alkyle en C₁ à C₆, (alkyle en C₁ à C₆)aminoalkyle en C₁ à C₆, (alkyle en C₁ à C₆)thioalkyle en C₁ à C₆, (aryle en C₆ à C₁₀)alkyle en C₁ à C₆, (hétéroaryle en C₁ à C₉)alkyle en C₁ à C₆, (hétérocyclyle en C₂ à C₁₀)alkyle en C₁ à C₆, (cycloalkyle en C₃ à C₁₀)alkyle en C₁ à C₆, (aryloxy en C₆ à C₁₀)alkyle en C₁ à C₆, (hétérocyclyloxy en C₂ à C₁₀)alkyle en C₁ à C₆, (cycloalkyloxy en C₃ à C₁₀)alkyle en C₁ à C₆, (aryle en C₆ à C₁₀)aminoalkyle en C₁ à C₆, (hétérocyclyle en C₂ à C₁₀)aminoalkyle en C₁ à C₆, (cycloalkyle en C₃ à C₁₀)aminoalkyle en C₁ à C₆, aryle en C₆ à C₁₀, hétéroaryle en C₁ à C₉, hétérocyclyle en C₂ à C₁₀ ou carbocyclyle en C₃ à C₁₀ ; et
R¹³ et R^{13a} sont chacun indépendamment H, un groupe deutérium, alkyle en C₁ à C₆, hétéroalkyle en C₂ à C₆, cycloalkyle en C₃ à C₁₀, hétérocyclyle en C₂ à C₁₀, aryle en C₆ à C₁₀, hétéroaryle en C₁ à C₉ ou (aryle en C₆ à C₁₀) alkyle en C₁ à C₆ ; à condition que lorsque R¹³ et R^{13a} sont liés au même atome d'azote, R¹³ et R^{13a}, ensemble avec l'atome d'azote auquel ils sont attachés, forment facultativement un cycle de 3 à 8 chaînons substitué ou non substitué ou un cycle bicyclique spiro ou condensé substitué ou non substitué ; ou dans lequel 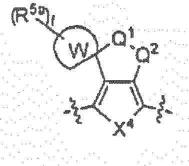 est 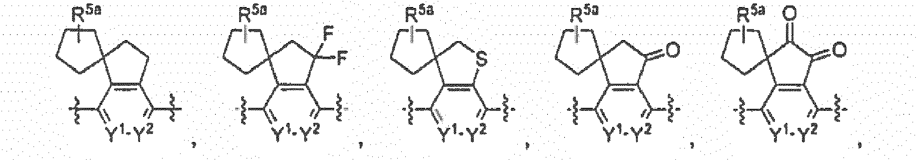 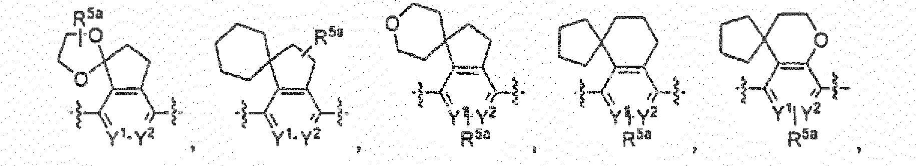 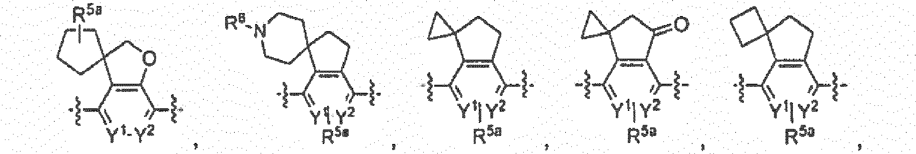 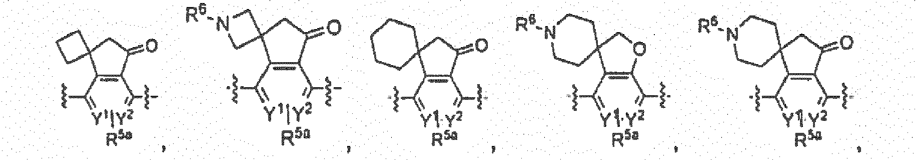 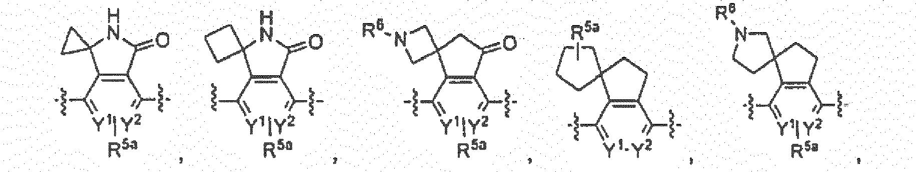 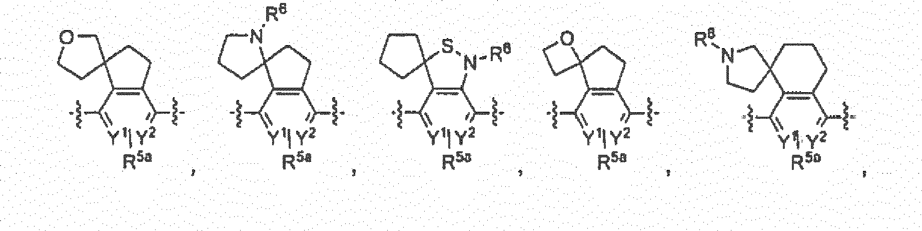 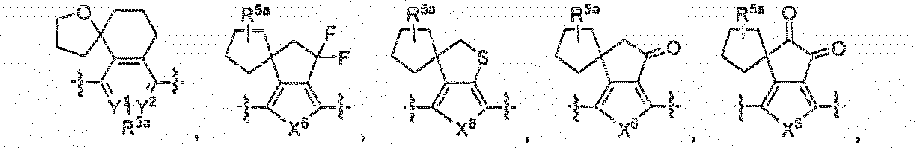 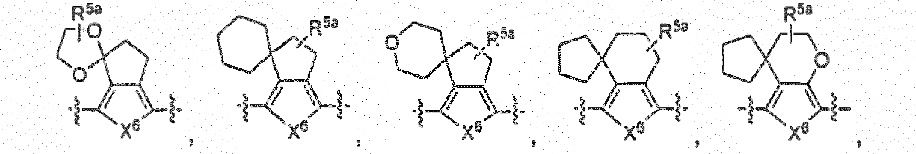 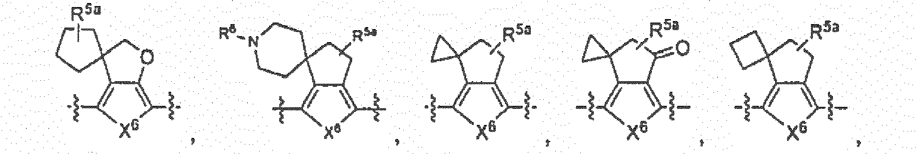 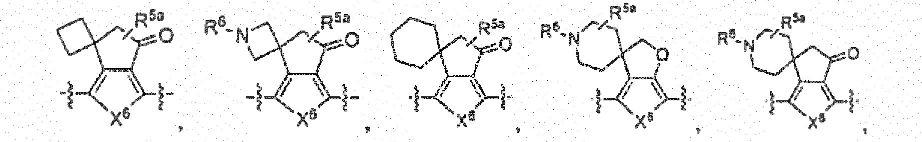 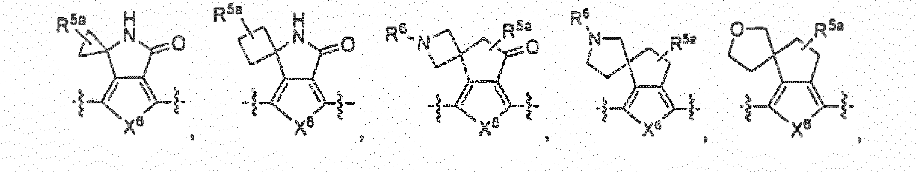 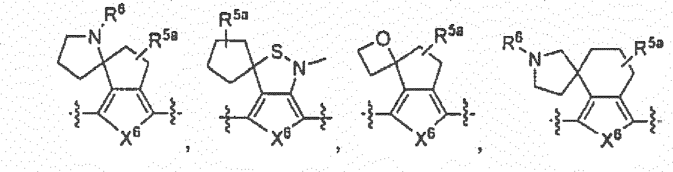 ou 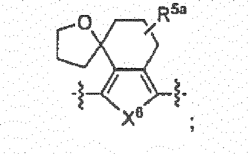
où Y¹ et Y² sont chacun indépendamment N ou CR⁷ ;
chaque X⁶ est indépendamment CR⁷R^{7a}, O, S ou NR⁶ ;
chaque R^{5a} est indépendamment H, un groupe deutérium, oxo (=O), hydroxy, amino, F, CI, Br, I, cyano, (alkyle en C₁ à C₆)acyle, (alkyle en C₁ à C₆)acyloxy, (alcoxy en C₁ à C₆)acyle, (alkyle en C₁ à C₆)sulfonyle, (alcoxy en C₁ à C₆)sulfonyle, (alkyle en C₁ à C₆)sulfinyle, (alkyle en C₁ à C₆)sulfonyloxy, (alkyle en C₁ à C₆)sulfinyloxy, alcoxy en C₁ à C₆, alkyle en C₁ à C₆, aryle en C₆ à C₁₀, -CF₃, -OCF₃, mercapto, nitro ou (alkyle en C₁ à C₆)amino ;
chaque R⁶ est indépendamment H, un groupe deutérium, alkyle en C₁ à C₆, halogénoalkyle en C₁ à C₆, hydroxyalkyle en C₁ à C₆, aminoalkyle en C₁ à C₆, (alcoxy en C₁ à C₆)alkyle en C₁ à C₆, (alkyle en C₁ à C₆)aminoalkyle en C₁ à C₆, (alkyle en C₁ à C₆)thioalkyle en C₁ à C₆, (aryle en C₆ à C₁₀)alkyle en C₁ à C₆, (hétéroaryle en C₁ à C₉)alkyle en C₁ à C₆, (hétérocyclyle en C₂ à C₁₀)alkyle en C₁ à C₆ ou (cycloalkyle en C₃ à C₈)alkyle en C₁ à C₆ ; et
R⁷ et R^{7a} sont chacun indépendamment H, un groupe deutérium, F, CI, Br, I, alkyle en C₁ à C₆, hétéroalkyle en C₂ à C₆, halogénoalkyle en C₁ à C₆, hydroxyalkyle en C₁ à C₆, aminoalkyle en C₁ à C₆, (alcoxy en C₁ à C₆)alkyle en C₁ à C₆, (alkyle en C₁ à C₆)aminoalkyle en C₁ à C₆, (alkyle en C₁ à C₆)thioalkyle en C₁ à C₆, (aryle en C₆ à C₁₀)alkyle en C₁ à C₆, (hétérocyclyle en C₂ à C₁₀)alkyle en C₁ à C₆, (cycloalkyle en C₃ à C₁₀)alkyle en C₁ à C₆, (aryloxy en C₆ à C₁₀)alkyle en C₁ à C₆, (hétérocyclyloxy en C₂ à C₁₀)alkyle en C₁ à C₆, (cycloalkyloxy en C₃ à C₁₀)alkyle en C₁ à C₆, (aryle en C₆ à C₁₀)aminoalkyle en C₁ à C₆, (hétérocyclyle en C₂ à C₁₀)aminoalkyle en C₁ à C₆, (cycloalkyle en C₃ à C₁₀)aminoalkyle en C₁ à C₆, aryle en C₆ à C₁₀, hétéroaryle en C₁ à C₉, hétérocyclyle en C₂ à C₁₀ ou carbocyclyle en C₃ à C₈.

3. Composé selon la revendication 1, dans lequel A et A' sont chacun indépendamment une liaison, un groupe alkylène en C₁ à C₆, alcénylène en C₂ à C₆, cycloalkylène en C₃ à C₈, hétérocyclylalkylène en C₂ à C₁₀, -(CR⁸R^{8a})ₙ-O-(CR⁸R^{8a})ₚ-, -(CR⁸R^{8a})ₙ-N(R⁵)-(CR⁸R^{8a})ₚ-,-(CR⁸R^{8a})ₙ-S(=O)ᵣ-N(R⁵)-(CR⁸R^{8a})ₚ-, -(CR⁸R^{8a})ₙ-C(=O)-N(R⁵)-(CR⁸R^{8a})ₚ-, -(CR⁸R^{8a})ₙ-N(R⁵)-C(=O)-(R⁵)-(CR⁸R^{8a})ₚ-,-(CR⁸R^{8a})ₙ-(=O)-O-(CR⁸R^{8a})ₚ-, -(CR⁸R^{8a})ₙ-N(R⁵)-S(=O)ᵣ-N(R⁵)-(CR⁸R^{8a})ₚ- ou -(CR⁸R^{8a})ₙ-N(R⁵)-C(=O)-O-(CR⁸R^{8a})ₚ- ; ou A et A' sont chacun indépenamment 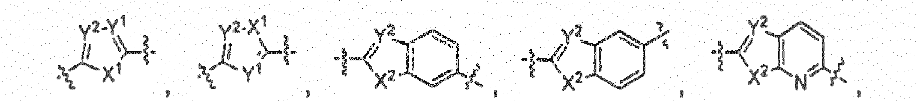 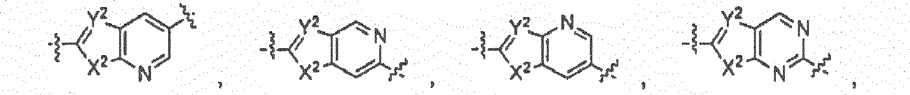 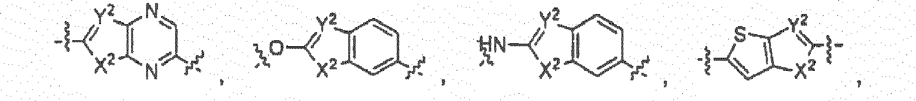 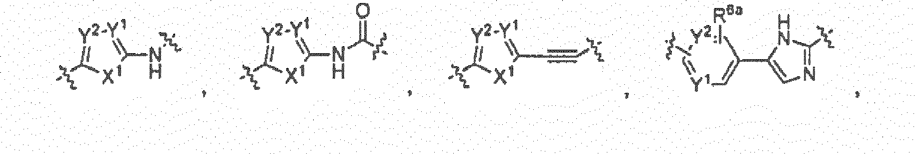 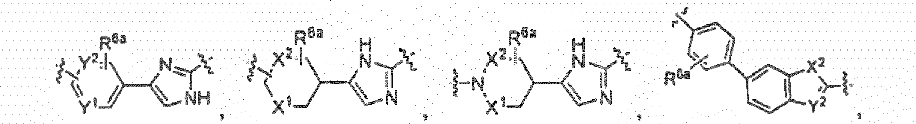 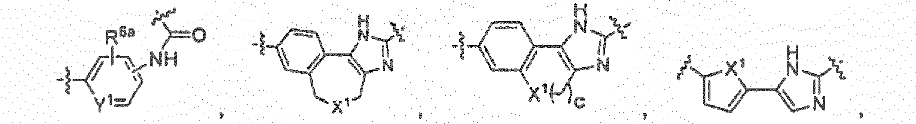 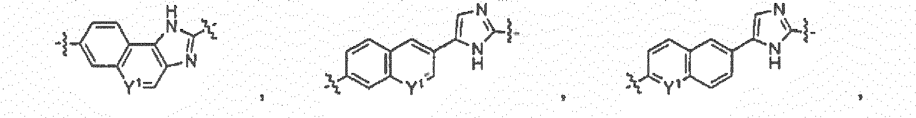 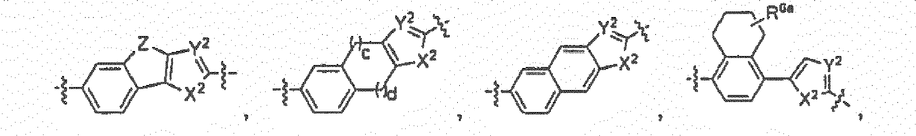 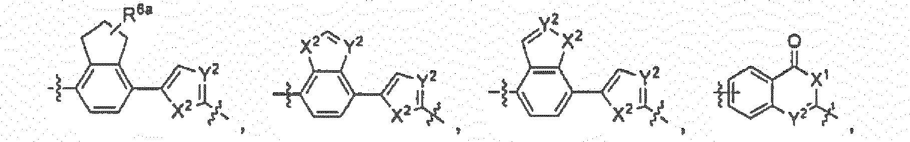 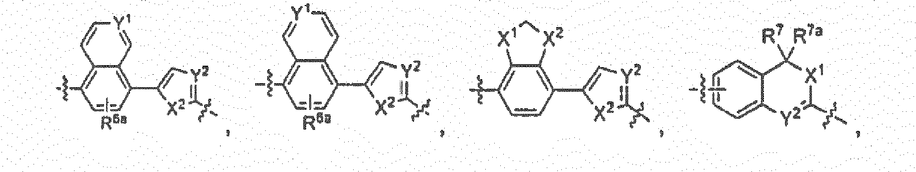 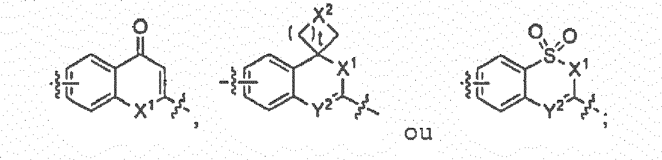
où chaque X¹ est indépendamment O, S, NR⁶ ou CR⁷R^{7a} ;
chaque X² est indépendamment NR⁶, O ou S ;
Y¹ et Y² sont chacun indépendamment N ou CR⁷ ;
chaque c est indépendamment 1 ou 2 ;
chaque d est indépendamment 1 ou 2 ;
chaque n est indépendamment 0, 1, 2 ou 3 ;
chaque p est indépendamment 0, 1, 2 ou 3 ;
chaque r est indépendamment 0, 1 ou 2 ;
chaque R⁵ est indépendamment H, un groupe deutérium, hydroxy, alkyle en C₁ à C₆, hétéroalkyle en C₂ à C₆, cycloalkyle en C₃ à C₈, hétérocyclyle en C₂ à C₁₀, aryle en C₆ à C₁₀, hétéroaryle en C₁ à C₉, (aryle en C₆ à C₁₀)alkyle en C₁ à C₆, alcoxy en C₁ à C₆, alkyle en C₁ à C₆-OC(=O)-, alkyle en C₁ à C₆-C(=O)-, carbamoyle, alkyle en C₁ à C₆-OS(=O)ᵣ-, alkyle en C₁ à C₆-S(=O)ᵣO-, alkyle en C₁ à C₆-S(=O)ᵣ- ou aminosulfonyle ;
chaque R⁶ est indépendamment H, un groupe deutérium, R¹³R^{13a}NC(=O)-, R¹³OC(=O)-, R¹³C(=O)-, R¹³R^{13a}NS(=O)-, R¹³OS(=O)-, R¹³S(=O)-, R¹³R^{13a}NS(=O)₂-, R¹³OS(=O)₂-, R¹³S(=O)₂-, alkyl en C₁ à C₆, halogénoalkyle en C₁ à C₆, hydroxyalkyle en C₁ à C₆, aminoalkyle en C₁ à C₆, (alcoxy en C₁ à C₆)alkyle en C₁ à C₆, (alkyle en C₁ à C₆)aminoalkyle en C₁ à C₆, (alkyle en C₁ à C₆)thioalkyle en C₁ à C₆, (aryle en C₆ à C₁₀)alkyle en C₁ à C₆, (hétéroaryle en C₁ à C₉)alkyle en C₁ à C₆, (hétérocyclyle en C₂ à C₁₀)alkyle en C₁ à C₆, (cycloalkyle en C₃ à C₁₀) alkyle en C₁ à C₆, (aryloxy en C₆ à C₁₀)alkyle en C₁ à C₆, (hétérocyclyloxy en C₂ à C₁₀)alkyle en C₁ à C₆, (cycloalkyloxy en C₃ à C₁₀)alkyle en C₁ à C₆, (aryle en C₆ à C₁₀)aminoalkyle en C₁ à C₆, (hétérocyclyle en C₂ à C₁₀)aminoalkyle en C₁ à C₆, (cycloalkyle en C₃ à C₁₀)aminoalkyle en C₁ à C₆, aryle en C₆ à C₁₀, hétéroaryle en C₁ à C₉, hétérocyclyle en C₂ à C₁₀ ou carbocyclyle en C₃ à C₁₀ ;
chaque R^{6a} est indépendamment H, un groupe deutérium, hydroxy, amino, F, CI, Br, I, cyano, oxo (=0), R^{13a}R¹³N-, -C(=O)NR¹³R^{13a}, -OC(=O)NR¹³R^{13a}, -OC(=O)OR¹³,-N(R¹³)C(=O)NR¹³R^{13a}, -N(R¹³)C(=O)OR^{13a}, -N(R¹³)C(=O)-R^{13a}, R¹³R^{13a}N-S(=O)₂-, R¹³S(=O)₂-, R¹³S(=O)₂N(R^{13a})-, R^{13a}R¹³N-alkyle en C₁ à C₆, R¹³S(=O)-alkyle en C₁ à C₆, R¹³R^{13a}N-C(=O)-alkyle en C₁ à C₆, R^{13a}R¹³N-alcoxy en C₁ à C₆, R¹³S(=O)-alcoxy en C₁ à C₆, R¹³R^{13a}N-C(=O)-alcoxy en C₁ à C₆, aryle en C₆ à C₁₀, hétéroaryle en C₁ à Cg, alcoxy en C₁ à C₆, (alkyle en C₁ à C₆)amino, alkyle en C₁ à C₆, halagénoalkyle en C₁ à C₆, alcényle en C₂ à C₆, alcynyle en C₂ à C₆, hétérocyclyle en C₂ à C₁₀, cycloalkyle en C₃ à C₈, mercapto, nitro, (aryle en C₆ à C₁₀) alkyle en C₁ à C₆, (aryle en C₆ à C₁₀)amino, (hétéroaryle en C₁ à C₉)amino, ou aryloxy en C₆ à C₁₀ ;
R⁷ et R^{7a} sont chacun indépendamment H, un groupe deutérium, F, CI, Br, I, alkyle en C₁ à C₆, hétéroalkyle en C₂ à C₆, halogénoalkyle en C₁ à C₆, hydroxyalkyle en C₁ à C₆, aminoalkyle en C₁ à C₆, (alcoxy en C₁ à C₆)alkyle en C₁ à C₆, (alkyle en C₁ à C₆) aminoalkyle en C₁ à C₆, (alkyle en C₁ à C₆)thioalkyle en C₁ à C₆, (aryle en C₆ à C₁₀)alkyle en C₁ à C₆, (hétérocyclyle en C₂ à C₁₀)alkyle en C₁ à C₆, (cycloalkyle en C₃ à C₁₀)alkyle en C₁ à C₆, (aryloxy en C₆ à C₁₀)alkyle en C₁ à C₆, (hétérocyclyloxy en C₂ à C₁₀)alkyle en C₁ à C₆, (cycloalkyloxy en C₃ à C₁₀)alkyle en C₁ à C₆, (aryle en C₆ à C₁₀) aminoalkyle en C₁ à C₆, (hétérocyclyle en C₂ à C₁₀)aminoalkyle en C₁ à C₆, (cycloalkyle en C₃ à C₁₀)aminoalkyle en C₁ à C₆, aryle en C₆ à C₁₀, hétéroaryle en C₁ à C₉, hétérocyclyle en C₂ à C₁₀ ou carbocyclyle en C₃ à C₈ ;
R¹³ et R^{13a} sont chacun indépendamment H, un groupe deutérium, alkyle en C₁ à C₆, hétéroalkyle en C₂ à C₆, cycloalkyle en C₃ à C₁₀, hétérocyclyle en C₂ à C₁₀, aryle en C₆ à C₁₀, hétéroaryle en C₁ à C₉ ou (aryle en C₆ à C₁₀)alkyle en C₁ à C₆ ; à condition que lorsque R¹³ et R^{13a} sont liés au même atome d'azote, R¹³ et R^{13a}, ensemble avec l'atome d'azote auquel ils sont attachés, forment facultativement un cycle de 3 à 8 chaînons substitué ou non substitué ou un cycle bicyclique spiro ou condensé substitué ou non substitué ; et
R⁸ et R^{8a} sont chacun indépendamment H, un groupe deutérium, hydroxy, cyano, nitro, F, CI, Br, I, alkyle en C₁ à C₆, hétéroalkyle en C₂ à C₆, cycloalkyle en C₃ à C₁₀, hétérocyclyle en C₂ à C₁₀, aryle en C₆ à C₁₀, hétéroaryle en C₁ à C₉, (aryle en C₆ à C₁₀) alkyle en C₁ à C₆, alcoxy en C₁ à C₆, alkyle en C₁ à C₆-OC(=O)-, alkyle en C₁ à C₆-C(=O)-, carbamoyle, alkyle en C₁ à C₆-OS(=O)ᵣ-, alkyle en C₁ à C₆-S(=O)ᵣO-, alkyle en C₁ à C₆-S(=O)ᵣ- ou aminosulfonyle ; ou dans lequel A et A' sont chacun indépendamment une liaison, -CH₂-, -(CH₂)₂-, -CH=CH-, -CH=CH-CH₂-, -N(R⁶)-, -C(=O)-, -C(=S)-,-C(=O)-O-, -C(=O)N(R⁶)-, -OC(=O)N(R⁶)-, -OC(=O)O-,-N(R⁶)C(=O)N(R⁶)-, -(R⁶)N-S(=O)₂-, -S(=O)₂-, -OS(=O)₂-,-(R⁶)N-S(=O)-, -S(=O)-, -OS(=O)- ; ou A et A' sont chacun indépendamment 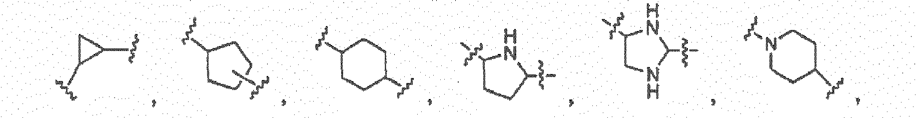 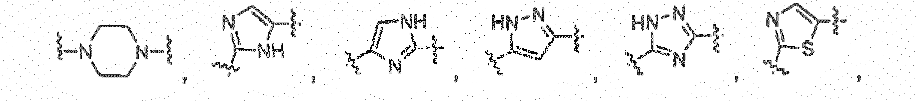 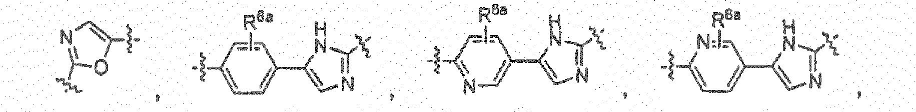 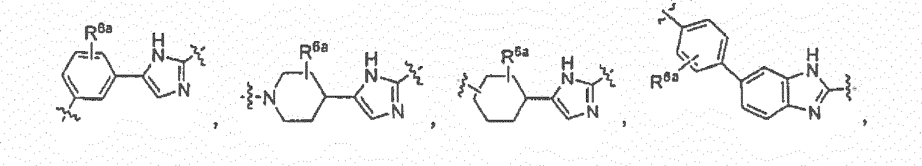 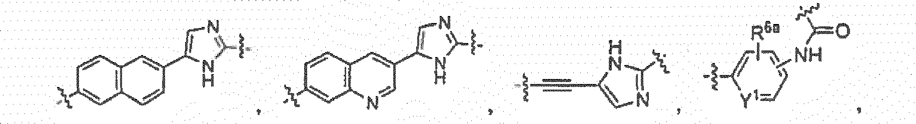 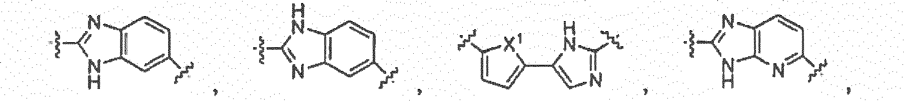 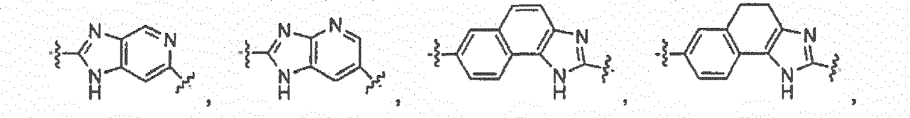 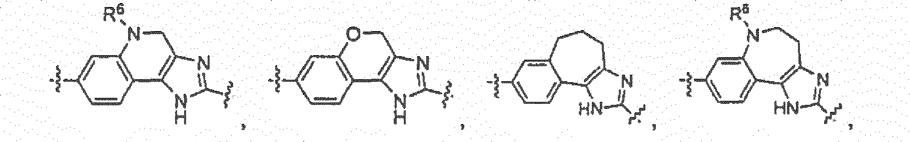 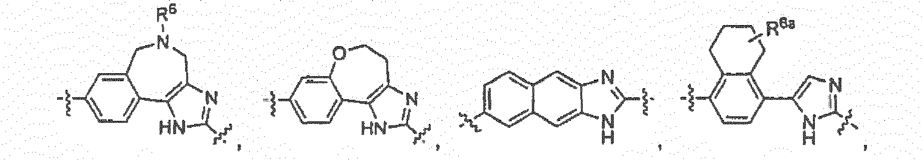 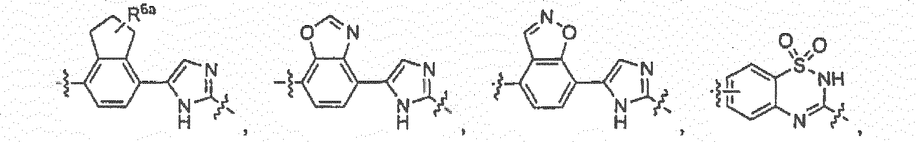 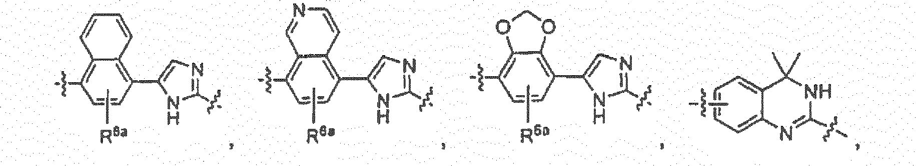 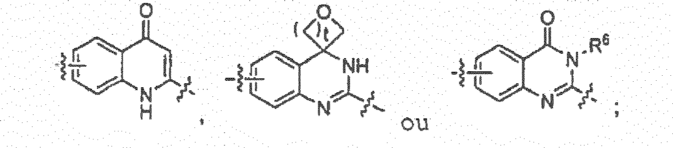
où X¹ est O ou S ;
chaque Y¹ est indépendamment N ou CR⁷ ;
chaque R⁶ est indépendamment H, un groupe deutérium, alkyle en C₁ à C₄, halogénoalkyle en C₁ à C₆, hydroxyalkyle en C₁ à C₆, aminoalkyle en C₁ à C₆, (alcoxy en C₁ à C₆)alkyle en C₁ à C₄, (alkyle en C₁ à C₆) aminoalkyle en C₁ à C₄, (alkyle en C₁ à C₆)thioalkyle en C₁ à C₄, (aryle en C₆ à C₁₀)alkyle en C₁ à C₄, hétéroaryle en C₁ à C₉, aryle en C₆ à C₁₀, hétérocyclyle en C₂ à C₁₀ ou carbocyclyle en C₃ à C₈ ;
R^{6a} et R⁷ sont chacun indépendamment H, un groupe deutérium, oxo (=O), hydroxy, amino, F, CI, Br, I, cyano, R^{13a}R¹³N-, alcoxy en C₁ à C₆, (alkyle en C₁ à C₆)amino, alkyle en C₁ à C₆, halogénoalkyle en C₁ à C₆, alcényle en C₂ à C₆, alcynyle en C₂ à C₆, mercapto ou nitro ; et
R¹³ et R^{13a} sont chacun indépendamment H, un groupe deutérium, alkyle en C₁ à C₆, hétéroalkyle en C₂ à C₆, cycloalkyle en C₃ à C₁₀, hétérocyclyle en C₂ à C₁₀, aryle en C₆ à C₁₀, hétéroaryle en C₁ à C₉ ou (aryle en C₆ à C₁₀)alkyle en C₁ à C₆ ; à condition que lorsque R¹³ et R^{13a} sont liés au même atome d'azote, R¹³ et R^{13a}, ensemble avec l'atome d'azote auquel ils sont attachés, forment facultativement un cycle de 3 à 8 chaînons substitué ou non substitué ou un cycle bicyclique spiro ou condensé substitué ou non substitué.

4. Composé selon la revendication 1,
dans lequel R¹, R² et X-CH ensemble forment l'un des groupes monovalents suivants : 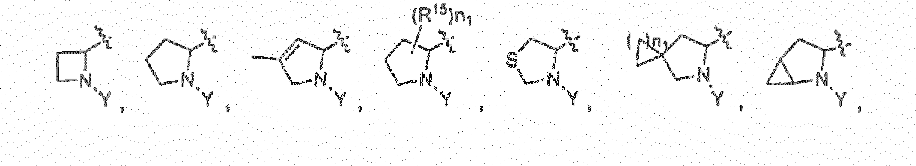 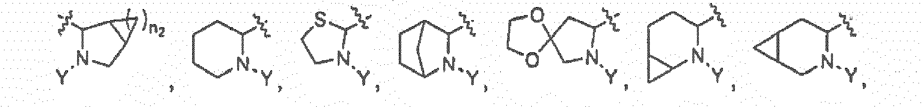 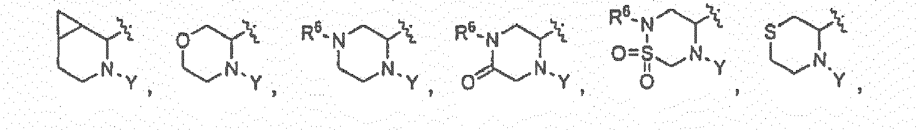 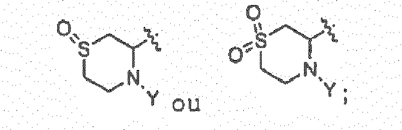 où chaque R¹⁵ est indépendamment H, un groupe deutérium, F, Cl, Br, I, un groupe cyano, hydroxy, alkyle en C₁ à C₃, halogénoalkyle en C₁ à C₃, alcoxy en C₁ à C₃, (alkyle en C₁ à C₃)amino, (alkyle en en C₁ à C₃)thio, (aryle en C₆ à C₁₀)amino, aryloxy en C₆ à C₁₀, hétéroaryle en C₁ à C₉, hétéroaryloxy en C₁ à C₉, (hétéroaryle en C₁ à C₉)alkyle en C₁ à C₃ ou hétérocyclyle en C₂ à C₁₀ ;
chaque R⁶ est indépendamment H, un groupe deutérium, alkyle en C₁ à C₃, halogénoalkyle en C₁ à C₃, hydroxyalkyle en C₁ à C₃, aminoalkyle en C₁ à C₃, (alcoxy en C₁ à C₃)alkyle en C₁ à C₃, (alkyle en C₁ à C₃)aminoalkyle en C₁ à C₃, (alkyle en C₁ à C₃)thioalkyle en C₁ à C₃, (aryle en C₆ à C₁₀)alkyle en C₁ à C₃, hétéroaryle en C₁ à C₉, aryle en C₆ à C₁₀, hétérocyclyle en C₂ à C₁₀ ou carbocyclyle en C₃ à C₈ ; et
n₁ et n₂ sont chacun indépendamment 1, 2, 3 ou 4 ;
dans lequel R³, R⁴ et X'-CH ensemble forment l'un des groupes monovalents suivants : 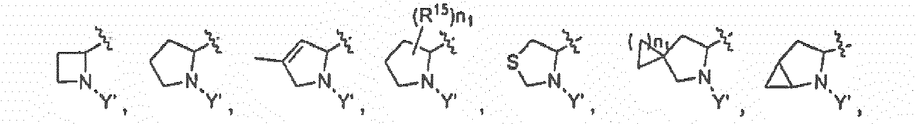 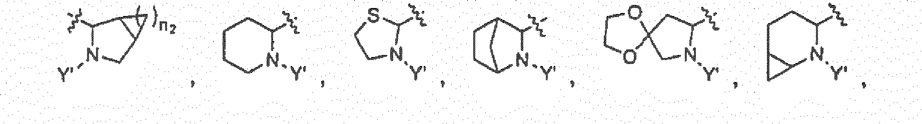 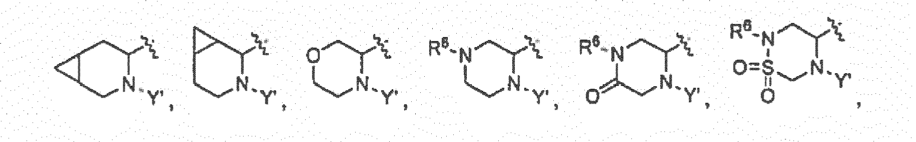 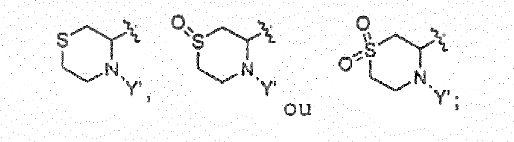 où chaque R¹⁵ est indépendamment H, un groupe deutérium, F, Cl, Br, I, cyano, hydroxy, alkyle en C₁ à C₃, halogénoalkyle en C₁ à C₃, alcoxy en C₁ à C₃, (alkyle en C₁ à C₃)amino, (alkyle en en C₁ à C₃)thio, (aryle en C₆ à C₁₀)amino, aryloxy en C₆ à C₁₀, hétéroaryle en C₁ à C₉, hétéroaryloxy en C₁ à C₉, (hétéroaryle en C₁ à C₉)alkyle en C₁ à C₃ ou hétérocyclyle en C₂ à C₁₀ ;
chaque R⁶ est indépendamment H, un groupe deutérium, alkyle en C₁ à C₃, halogénoalkyle en C₁ à C₃, hydroxyalkyle en C₁ à C₃, aminoalkyle en C₁ à C₃, (alcoxy en C₁ à C₃)alkyle en C₁ à C₃, (alkyle en C₁ à C₃)aminoalkyle en C₁ à C₃, (alkyle en C₁ à C₃)thioalkyle en C₁ à C₃, (aryle en C₆ à C₁₀)alkyle en C₁ à C₃, hétéroaryle en C₁ à C₉, aryle en C₆ à C₁₀, hétérocyclyle en C₂ à C₁₀ ou carbocyclyle en C₃ à C₈ ; et
n₁ et n₂ sont chacun indépendamment 1, 2, 3 ou 4.

5. Composé selon la revendication 1, répondant à la formule (II) : 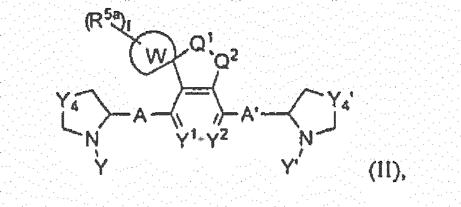 dans laquelle 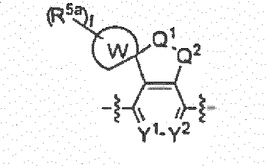 est 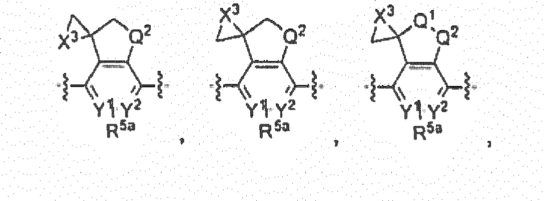 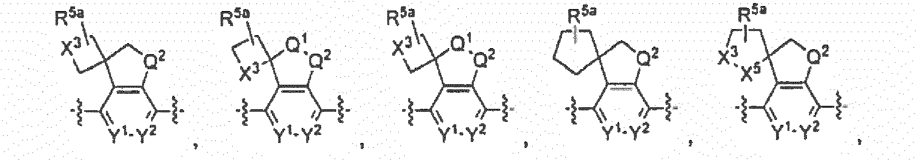 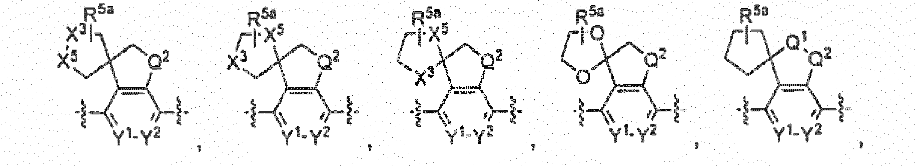 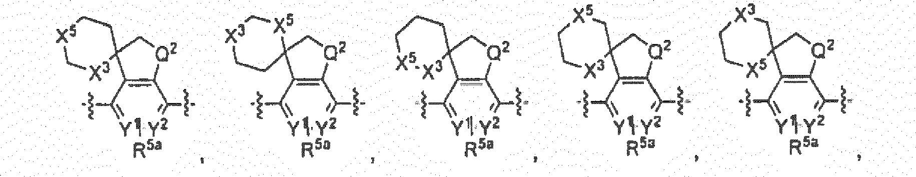 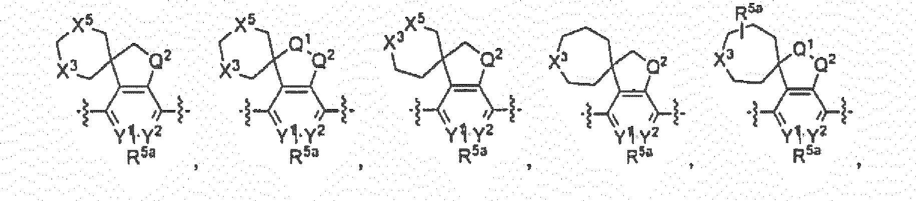 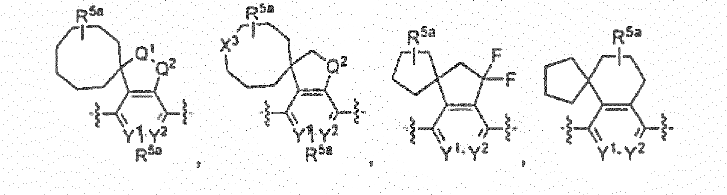 ou 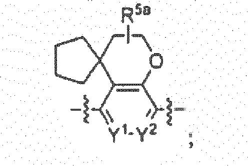
où Q¹ et Q² sont chacun indépendamment NR⁶, 0, S, C(=O) ou (CH₂)ₑ ;
e est 0, 1, 2, 3 ou 4 ;
X¹, X³ et X⁵ sont chacun indépendamment O, S, NR⁶ ou CR⁷R^{7a} ;
Y¹ et Y² sont chacun indépendamment N ou CR⁷ ;
A et A' sont chacun indépendamment une liaison, un groupe alkylène en C₁ à C₆, alcénylène en C₂ à C₆, cycloalkylène en C₃ à C₈, hétérocyclylalkylène en C₂ à C₁₀, -(CR⁸R^{8a})ₙ-O-(CR⁸R^{8a})ₚ-, -(CR⁸R^{8a})ₙ-N(R⁵)-(CR⁸R^{8a})ₚ-,-(CR⁸R^{8a})ₙ-S(=O)ᵣ-N(R⁵)-(CR⁸R^{8a})ₚ-, -(CR⁸R^{8a})ₙ-C(=O)-N(R⁵)-(CR⁸R^{8a})ₚ-, -(CR⁸R^{8a})ₙ-N(R⁵)-C(=O)-N(R⁵)-(CR⁸R^{8a})ₚ-,-(CR⁸R^{8a})ₙ-C(=O)-O-(CR⁸R^{8a})ₚ-, -(CR⁸R^{8a})ₙ-N(R⁵)-S(=O)ᵣ-N(R⁵)-(CR⁸R^{8a})ₚ- ou - (CR⁸R^{8a})ₙ-N(R⁵)-C(=O)-O-(CR⁸R^{8a})ₚ- ; ou A et A' sont chacun indépendamment 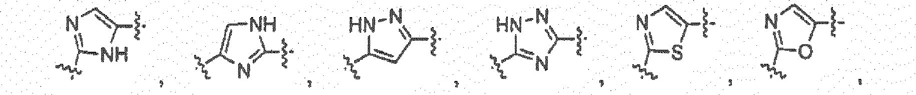 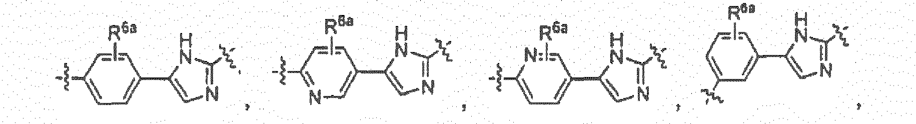 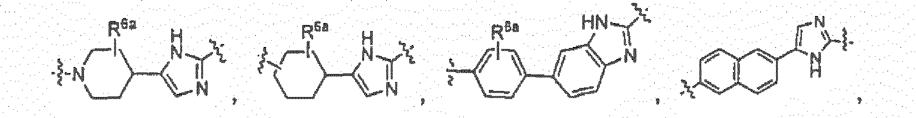 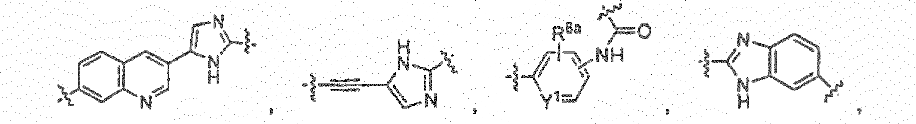 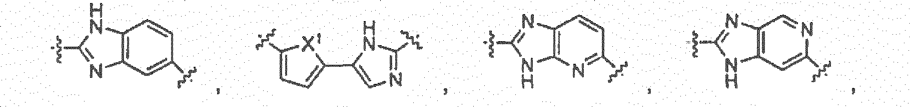 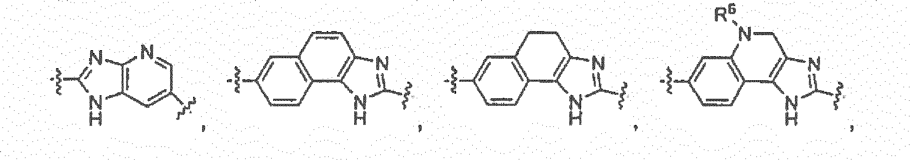 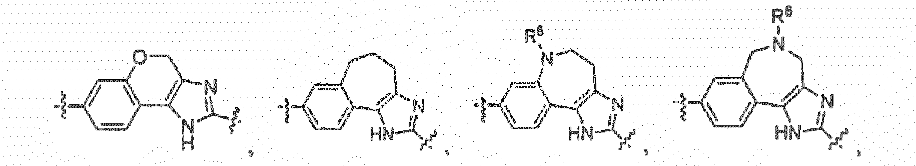 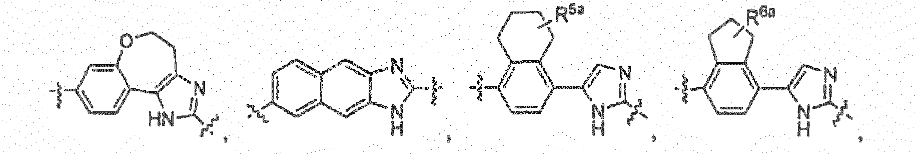 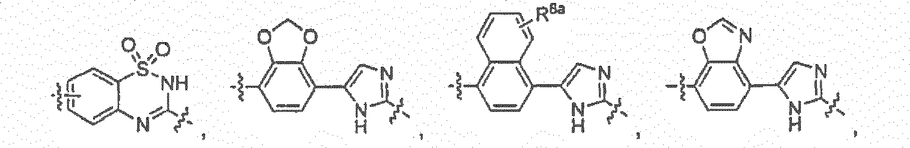 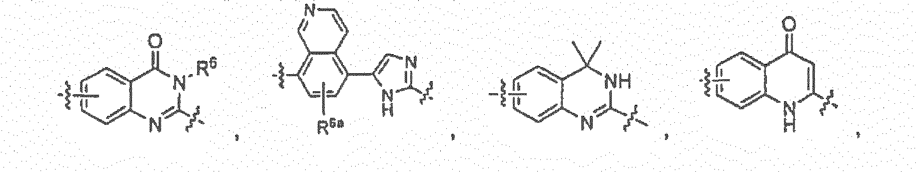 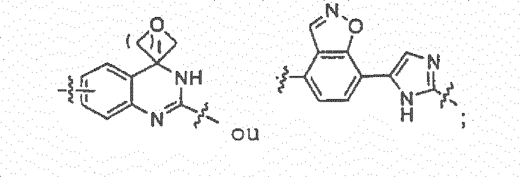
chaque R⁵ est indépendamment H, un groupe deutérium, hydroxy, alkyle en C₁ à C₆, hétéroalkyle en C₂ à C₆, cycloalkyle en C₃ à C₁₀, hétérocyclyle en C₂ à C₁₀, aryle en C₆ à C₁₀, hétéroaryle en C₁ à C₉, (aryle en C₆ à C₁₀)alkyle en C₁ à C₆, alcoxy en C₁ à C₆, alkyle en C₁ à C₆-OC(=O)-, alkyle en C₁ à C₆-C(=O)-, carbamoyle, alkyle en C₁ à C₆-OS(=O)ᵣ-, alkyle en C₁ à C₆-S(=O)ᵣO-, alkyle en C₁ à C₆-S(=O)ᵣ- ou aminosulfonyle ;
R^{5a} et R^{6a} sont chacun H, un groupe deutérium, oxo (=0), hydroxy, amino, F, CI, Br, I, cyano, (alkyle en C₁ à C₆)acyle, (alkyle en C₁ à C₆)acyloxy, (alcoxy en C₁ à C₆)acyle, (alkyle en C₁ à C₆)sulfonyle, (alcoxy en C₁ à C₆)sulfonyle, (alkyle en C₁ à C₆)sulfinyle, (alkyle en C₁ à C₆)sulfonyloxy, (alkyle en C₁ à C₆)sulfinyloxy, alcoxy en C₁ à C₆, alkyle en C₁ à C₆, aryle en C₆ à C₁₀, -CF₃, -OCF₃, mercapto, nitro, (alkyle en C₁ à C₆)amino, cycloalkyle en C₃ à C₁₀ ou aryloxy en C₆ à C₁₀ ;
chaque R⁶ est indépendamment H, un groupe deutérium, R¹³R^{13a}NC(=O)-, R¹³OC(=O)-, R¹³C(=O)-, R¹³R^{13a}NS(=O)-, R¹³OS(=O)-, R¹³S(=O)-, R¹³R^{13a}NS(=O)₂-, R¹³OS(=O)₂-, R¹³S(=O)₂-, aliphatique en C₁ à C₆, (alcoxy en C₁ à C₆)aliphatique en C₁ à C₆, (alkyle en C₁ à C₆)amino-aliphatique en C₁ à C₆, (aryle en C₆ à C₁₀) aliphatique en C₁ à C₆, (hétéroaryle en C₁ à C₉) aliphatique en C₁ à C₆, (hétérocyclyle en C₂ à C₁₀) aliphatique en C₁ à C₆, (cycloalkyle en C₃ à C₁₀) aliphatique en C₁ à C₆, aryle en C₆ à C₁₀, hétéroaryle en C₁ à C₉, hétérocyclyle en C₂ à C₁₀ ou carbocyclyle en C₃ à C₁₀ ;
R⁷ et R^{7a} sont chacun indépendamment H, un groupe deutérium, F, CI, Br, I, aliphatique en C₁ à C₆, hétéroalkyle en C₂ à C₆, (alcoxy en C₁ à C₆)aliphatique en C₁ à C₆, (alkyle en C₁ à C₆) amino-aliphatique en C₁ à C₆, (aryle en C₆ à C₁₀) aliphatique en C₁ à C₆, (hétérocyclyle en C₂ à C₁₀)aliphatique en C₁ à C₆, (cycloalkyle en C₃ à C₁₀)aliphatique en C₁ à C₆, aryle en C₆ à C₁₀, hétéroaryle, en C₁ à C₉, hétérocyclyle, en C₂ à C₁₀ ou carbocyclyle en C₃ à C₁₀ ;
R⁸ et R^{8a} sont chacun indépendamment H, un groupe deutérium, hydroxy, cyano, nitro, F, CI, Br, I, alkyle en C₁ à C₆, hétéroalkyle, en C₂ à C₆, cycloalkyle en C₃ à C₁₀, hétérocyclyle en C₂ à C₁₀, aryle en C₆ à C₁₀, hétéroaryle en C₁ à C₉, (aryle en C₆ à C₁₀)alkyle en C₁ à C₆, alcoxy en C₁ à C₆, alkyle en C₁ à C₆-OC(=O)-, alkyle en C₁ à C₆-C(=O)-, carbamoyle, alkyle en C₁ à C₆-OS(=O)ᵣ-, alkyle en C₁ à C₆-S(=O)ᵣO-, alkyle en C₁ à C₆-S(=O)ᵣ- ou aminosulfonyle ;
R¹³ et R^{13a} sont chacun indépendamment H, un groupe deutérium, alkyle en C₁ à C₆, hétéroalkyle en C₂ à C₆, cycloalkyle en C₃ à C₁₀, hétérocyclyle en C₂ à C₁₀, aryle en C₆ à C₁₀, hétéroaryle en C₁ à C₉ ou (aryle en C₆ à C₁₀)alkyle en C₁ à C₆ ; à condition que lorsque R¹³ et R^{13a} sont liés au même atome d'azote, R¹³ et R^{13a}**,** ensemble avec l'atome d'azote auquel ils sont attachés, forment facultativement un cycle de 3 à 8 chaînons substitué ou non substitué ou un cycle bicyclique spiro ou condensé substitué ou non substitué ;
chaque n est indépendamment 0, 1, 2 ou 3 ;
chaque p est indépendamment 0, 1, 2 ou 3 ;
chaque r est indépendamment 0, 1 ou 2 ;
Y₄ et Y₄' sont chacun indépendamment une liaison, 0, S, -(CH₂)ₙ-, -CH=CH-, -S(=O)ᵣ-, -CH₂O-, -CH₂S-, -CH₂F,-CHR^{5a}-, -CR^{5a}R^{6a}-, -CH₂S(=O)ᵣ- ou -CH₂N(R⁶)-.

6. Composé selon la revendication 1, répondant à la formule (II') : 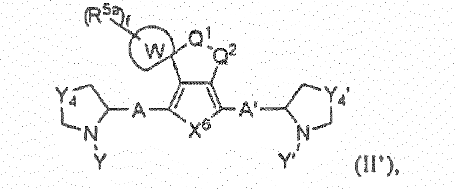 dans laquelle 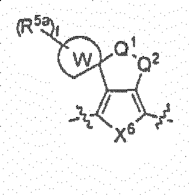 est 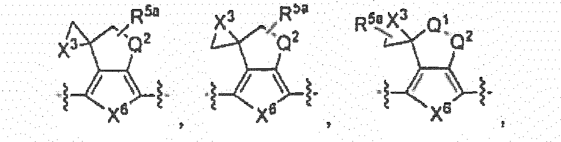 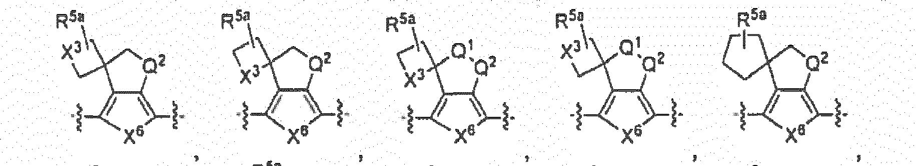 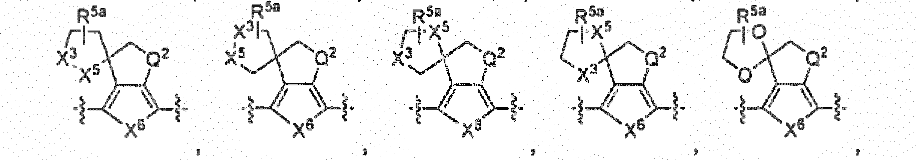 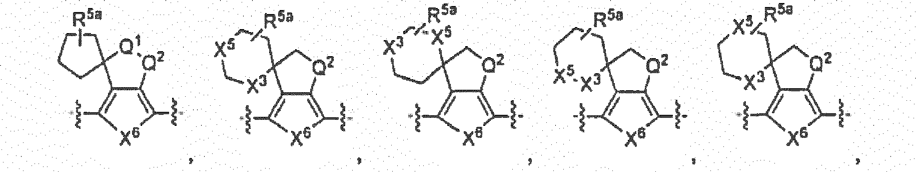 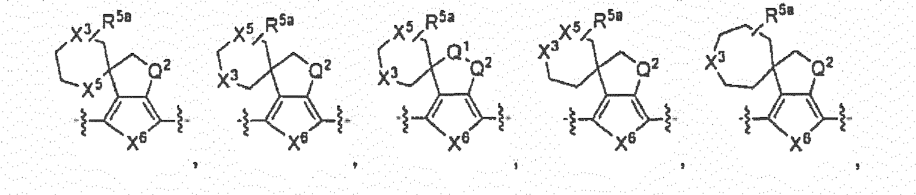 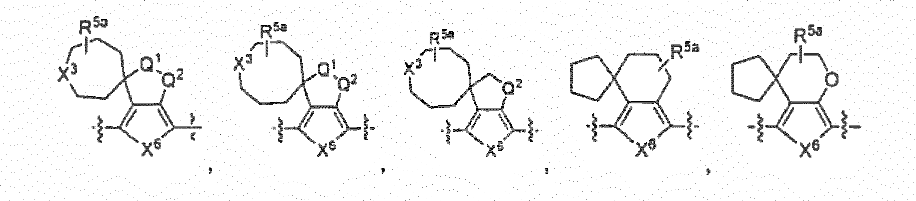 ou 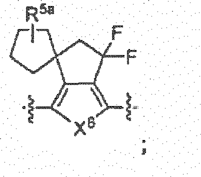
où Q¹ et Q² sont chacun indépendamment NR⁶, O, S, C(=O) ou (CH₂)ₑ ;
e est 0, 1, 2, 3 ou 4 ;
X¹, X³ et X⁵ sont chacun indépendamment O, S, NR⁶ ou CR⁷R^{7a} ;
chaque X⁶ est indépendamment CR⁷R^{7a}, O, S ou, NR⁶ ;
f est 0, 1, 2, 3 ou 4 ;
A et A' sont chacun indépendamment une liaison, un groupe alkylène en C₁ à C₆, alcénylène en C₂ à C₆, cycloalkylène en C₃ à C₈, hétérocyclylalkylène en C₂ à C₁₀, -(CR⁸R^{8a})ₙ-O-(CR⁸R^{8a})ₚ-, -(CR⁸R^{8a})ₙ-N(R⁵)-(CR⁸R^{8a})ₚ-,-(CR⁸R^{8a})ₙ-S(=O)ᵣ-N(R⁵)-(CR⁸R^{8a})ₚ-, -(CR⁸_{R}^{8a})ₙ-C(=O)-N(R⁵)-(CR⁸R^{8a})ₚ-, -(CR⁸R^{8a})ₙ-N(R⁵)-C(=O)-N(R⁵)-(CR⁸R^{8a})ₚ-,-(CR⁸R^{8a})ₙ-C(=O)-O-(CR⁸R^{8a})ₚ-, -(CR⁸R^{8a})ₙ-N(R⁵)-S(=O)ᵣ-N(R⁵)-(CR⁸R^{8a})ₚ-, -(CR⁸R^{8a})ₙ-N(R⁵)-C(=O)-O-(CR⁸R^{8a})ₚ- ; ou A et A' sont chacun indépendamment 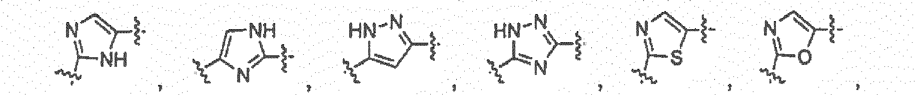 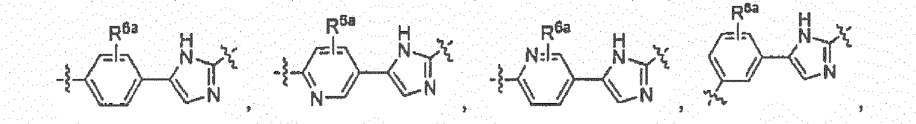 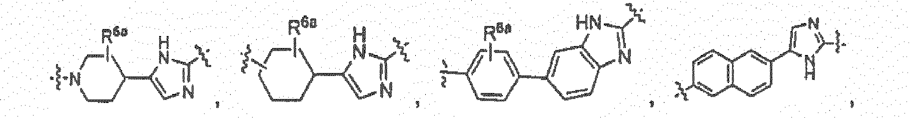 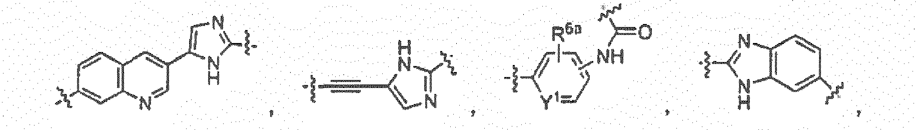 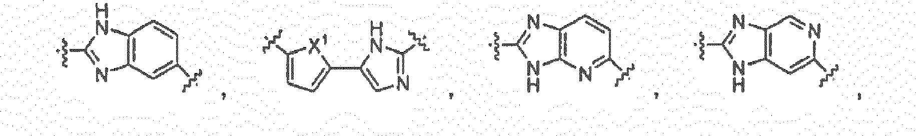 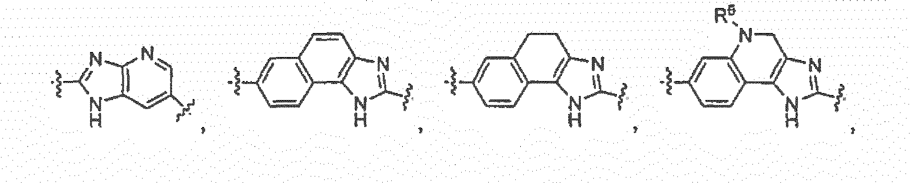 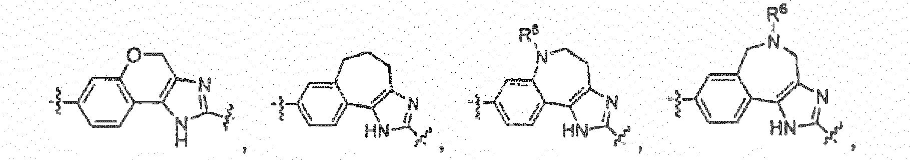 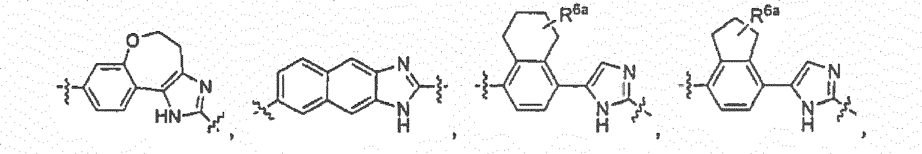 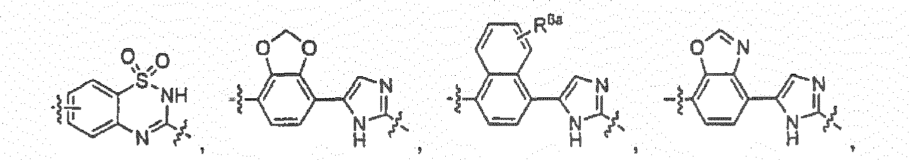 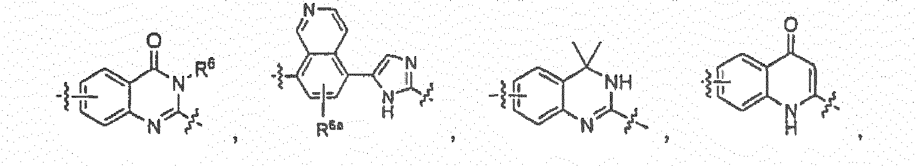 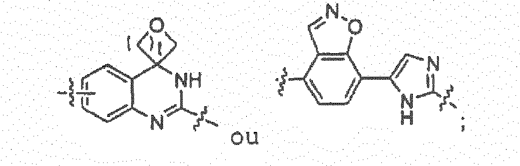
Y¹ est indépendamment N ou CR⁷ ;
chaque R⁵ est indépendamment H, un groupe deutérium, hydroxy, alkyle en C₁ à C₆, hétéroalkyle en C₂ à C₆, cycloalkyle en C₃ à C₁₀, hétérocyclyle en C₂ à C₁₀, aryle en C₆ à C₁₀, hétéroaryle en C₁ à C₉, (aryle en C₆ à C₁₀)alkyle en C₁ à C₆, alcoxy en C₁ à C₆, alkyle en C₁ à C₆-OC(=O)-, alkyle en C₁ à C₆-C(=O)-, carbamoyle, alkyle en C₁ à C₆-OS(=O)ᵣ-, alkyle en C₁ à C₆-S(=O)ᵣO-, alkyle en C₁ à C₆-S(=O)ᵣ- ou aminosulfonyle ;
R^{5a} et R^{6a} sont chacun indépendamment H, un groupe deutérium, oxo (=O), hydroxy, amino, F, CI, Br, I, cyano, (alkyle en C₁ à C₆)acyle, (alkyle en C₁ à C₆)acyloxy, (alcoxy en C₁ à C₆)acyle, (alkyle en C₁ à C₆)sulfonyle, (alcoxy en C₁ à C₆)sulfonyle, (alkyle en C₁ à C₆)sulfinyle, (alkyle en C₁ à C₆)sulfonyloxy, (alkyle en C₁ à C₆)sulfinyloxy, alcoxy en C₁ à C₆, alkyle en C₁ à C₆, aryle en C₆ à C₁₀, -CF₃, -OCF₃, mercapto, nitro, (alkyle en C₁ à C₆)amino, cycloalkyle en C₃ à C₁₀ ou aryloxy en C₆ à C₁₀ ;
chaque R⁶ est indépendamment H, un groupe deutérium, R¹³R^{13a}NC(-O)-, R¹³OC(=O)-, R¹³C(=O)-, R¹³R^{13a}NS(=O)-, R¹³OS(=O)-, R¹³S(=O)-, R¹³R^{13a}NS(=O)₂-, R¹³OS(=O)₂-, R¹³S(=O)₂-, aliphatique en C₁ à C₆, (alcoxy en C₁ à C₆)aliphatique en C₁ à C₆, (alkyle en C₁ à C₆)amino-aliphatique en C₁ à C₆, (aryle en C₆ à C₁₀) aliphatique en C₁ à C₆, (hétéroaryle en C₁ à C₉) aliphatique en C₁ à C₆, (hétérocyclyle, en C₂ à C₁₀) aliphatique en C₁ à C₆, (cycloalkyle en C₃ à C₁₀) aliphatique en C₁ à C₆, aryle en C₆ à C₁₀, hétéroaryle en C₁ à C₉, hétérocyclyle en C₂ à C₁₀ ou carbocyclyle en C₃ à C₁₀ ;
R⁷ et R^{7a} sont chacun indépendamment H, un groupe deutérium, F, CI, Br, I, aliphatique en C₁ à C₆, hétéroalkyle, en C₂ à C₆, (alcoxy en C₁ à C₆)aliphatique en C₁ à C₆, (alkyle en C₁ à C₆)amino-aliphatique en C₁ à C₆, (aryle en C₆ à C₁₀)aliphatique en C₁ à C₆, (hétérocyclyle en C₂ à C₁₀)aliphatique en C₁ à C₆, (cycloalkyle en C₃ à C₁₀)aliphatique en C₁ à C₆, aryle en C₆ à C₁₀, hétéroaryle en C₁ à C₉, hétérocyclyle en C₂ à C₁₀ ou carbocyclyle en C₃ à C₁₀ ;
R⁸ et R^{8a} sont chacun indépendamment H, un groupe deutérium, hydroxy, cyano, nitro, F, CI, Br, I, alkyle en C₁ à C₆, hétéroalkyle en C₂ à C₆, cycloalkyle en C₃ à C₁₀, hétérocyclyle en C₂ à C₁₀, aryle en C₆ à C₁₀, hétéroaryle en C₁ à C₉, (aryle en C₆ à C₁₀)alkyle en C₁ à C₆, alcoxy en C₁ à C₆, alkyle en C₁ à C₆-OC(=O)-, alkyle en C₁ à C₆-C(=O)-, carbamoyle, alkyle en C₁ à C₆-OS(=O)ᵣ-, alkyle en C₁ à C₆-S(=O)ᵣO-, alkyle en C₁ à C₆-S(=O)ᵣ- ou aminosulfonyle ;
R¹³ et R^{13a} sont chacun indépendamment H, un groupe deutérium, alkyle en C₁ à C₆, hétéroalkyle en C₂ à C₆, cycloalkyle en C₃ à C₁₀, hétérocyclyle en C₂ à C₁₀, aryle en C₆ à C₁₀, hétéroaryle en C₁ à C₉ ou (aryle en C₆ à C₁₀)alkyle en C₁ à C₆ ; à condition que lorsque R¹³ et R^{13a} sont liés au même atome d'azote, R¹³ et R^{13a}, ensemble avec l'atome d'azote auquel ils sont attachés, forment facultativement un cycle de 3 à 8 chaînons substitué ou non substitué ou un cycle bicyclique spiro ou condensé substitué ou non substitué
chaque n est indépendamment 0, 1, 2 ou 3 ;
chaque p est indépendamment 0, 1, 2 ou 3 ;
chaque r est indépendamment 0, 1 ou 2 ; et
Y₄ et Y₄' sont chacun indépendamment une liaison, 0, S, -(CH₂)ₙ-, -CH=CH-, -S(=O)ᵣ-, -CH₂O-, -CH₂S-, -CH₂F-, -CHR^{5a}-, -CR^{5a}R^{6a}-, -CH₂S(=O)ᵣ- ou -CH₂N(R⁶)-.

7. Composé selon la revendication 5 répondant à la formule (III) ou à la formule (IV) ou à la formule (V) : 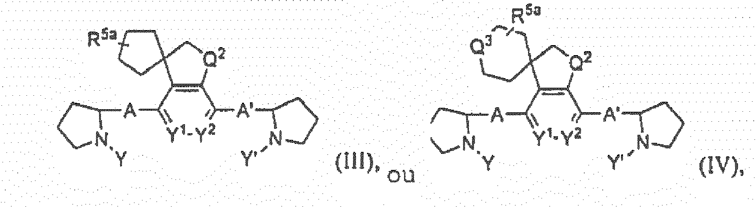 ou 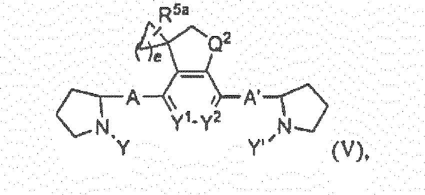
dans laquelle Q² et Q³ sont chacun indépendamment 0, S, C(=O), NR⁶ ou CH₂ ; et
dans laquelle e est 1, 2, 3 ou 4.

8. Composé selon la revendication 6 répondant à la formule (III') ou à la formule (IV') ou à la formule (V') : 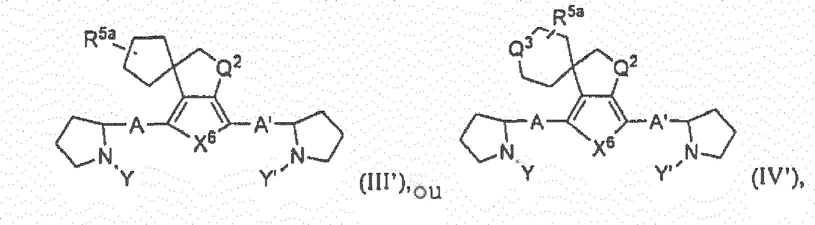 ou 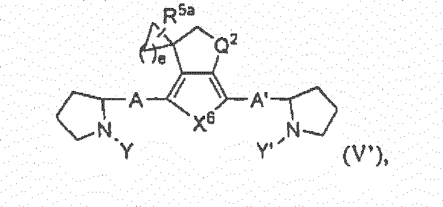
dans laquelle Q² et Q³ sont chacun indépendamment 0, S, C(=O), NR⁶ ou CH₂ ; et
dans laquelle e est 1, 2, 3 ou 4.

9. Composé selon la revendication 1, dans lequel Y et Y' sont chacun indépendamment un groupe dérivé d'un acide α-aminé et le groupe dérivé d'un acide α-aminé est facultativement substitué par un ou plusieurs substituants, le substituant étant un groupe deutérium, F, Cl, Br, I hydroxy, cyano, alcoxycarbonyle, aryloxycarbonyle, hétéroaryloxycarbonyle ou hétérocyclylcarbonyle ;
dans lequel l'acide α-aminé est l'isoleucine, la leucine, la lysine, la méthionine, la phénylalanine, la thréonine, le tryptophane, la valine, l'alanine, l'asparagine, l'acide aspartique, l'acide glutamique, la glutamine, la proline, la serine, la p-tyrosine, l'arginine, l'histidine, la cystéine, la glycine, la sarcosine, la *N,N*-diméthylglycine, l'homosérine, la norvaline, la norleucine, l'ornithine, l'homocystéine, l'homophénylalanine, la phénylglycine, l'o-tyrosine, la m-tyrosine ou l'hydroxyproline ; ou
dans lequel l'acide α-aminé est dans la configuration D ; ou
dans lequel l'acide α-aminé est dans la configuration L.

10. Composé selon l'une quelconque des revendications 1 à 8, dans lequel Y et Y' sont chacun indépendamment -[U-(CR⁹R^{9a})ₜ-N(R¹⁰)-(CR⁹R^{9a})ₜ]ₖ-U-(CR⁹R^{9a})ₜ-N(R¹¹)-(CR⁹R^{9a})ₜ-R¹², -U-(CR⁹R^{9a})ₜ-R¹² ou -[U-(CR⁹R^{9a})ₜ-N(R¹⁰)-(CR⁹R^{9a})ₜ]ₖ-U-(CR⁹R^{9a})ₜ-O-(CR⁹R^{9a})ₜ-R¹² ; ou
dans lequel Y et Y' sont chacun indépendamment -[U-(CR⁹R^{9a})ₜ-N(R¹⁰)-(CR⁹R^{9a})ₜ]ₖ-U-(CR⁹R^{9a})ₜ-N(R¹¹)-(CR⁹R^{9a})ₜ-R¹² ; ou
dans lequel Y et Y' sont chacun indépendamment -U-(CR⁹R^{9a})ₜ-N(R¹⁰)-(CR⁹R^{9a})ₜ-U-(CR⁹R^{9a})ₜ-N(R¹¹)-(CR⁹R^{9a})ₜ-R¹² ; ou
dans lequel Y et Y' sont chacun indépendamment -U-(CR⁹R^{9a})ₜ-N(R¹¹)-(CR⁹R^{9a})ₜ-R¹² ; ou
dans lequel Y et Y' sont chacun indépendamment-[C(=O)-(CR⁹R^{9a})ₜ-N(R¹⁰)-(CR⁹R^{9a})ₜ]ₖ-U-(CR⁹R^{9a})ₜ-N(R¹¹)-(CR⁹R^{9a})ₜ-R¹² ; ou
dans lequel Y et Y' sont chacun indépendamment -C(=O)-(CR⁹R^{9a})ₜ-N(R¹⁰)-(CR⁹R^{9a})ₜ-U-(CR⁹R^{9a})ₜ-N(R³¹)-(CR⁹R^{9a})ₜ-R¹² ; ou
dans lequel Y et Y' sont chacun indépendamment-[C(=O)-(CR⁹R^{9a})ₜ-N(R¹⁰)-(CR⁹R^{9a})ₜ]ₖ-C(=O)-(CR⁹R^{9a})ₜ-N(R¹¹)-(CR⁹R^{9a})ₜ-R¹² ; ou
dans lequel Y et Y' sont chacun indépendamment -C(=O)-(CR⁹R^{9a})ₜ-N(R¹⁰)-(CR⁹R^{9a})ₜ-C(=O)-(CR⁹R^{9a})ₜ-N(R¹¹)-(CR⁹R^{9a})ₜ-R¹² ; ou
dans lequel Y et Y' sont chacun indépendamment -C(=O)-(CR⁹R^{9a})ₜ-N(R¹¹)-(CR⁹R^{9a})ₜ-R¹² ; ou
dans lequel Y et Y' sont chacun indépendamment -C(=O)-(CR⁹R^{9a})ₙ-N(R¹¹)-(CR⁹R^{9a})ₙ-C(=O)-R¹³ ; ou
dans lequel Y et Y' sont chacun indépendamment -C(=O)-(CR⁹R^{9a})ₙ-N(R¹¹)-C(=O)-R¹³ ; ou
dans lequel Y et Y' sont chacun indépendamment -C(=O)-(CR⁹R^{9a})ₙ-N(R¹¹)-(CR⁹R^{9a})ₙ-C(=O)-O-R¹³ ; ou
dans lequel Y et Y' sont chacun indépendamment -C(=O)-(CR⁹R^{9a})ₙ-N(R¹¹)-C(=O)-O-R¹³ ; ou
dans lequel Y et Y' sont chacun indépendamment -U-(CR⁹R^{9a})ₜ-R¹² ; ou
dans lequel Y et Y' sont chacun indépendamment -C(=O)-(CR⁹R^{9a})ₜ-R¹² ; ou
dans lequel Y et Y' sont chacun indépendamment -[U-(CR⁹R^{9a})ₜ-N(R¹⁰)-(CR⁹R^{9a})ₜ]ₖ-U-(CR⁹R^{9a})ₜ-O-(CR⁹R^{9a})ₜ-R¹² ; ou dans lequel Y et Y' sont chacun indépendamment -U-(CR⁹R^{9a})ₜ-N(R¹⁰)-(CR⁹R^{9a})ₜ-U-(CR⁹R^{9a})ₜ-O-(CR⁹R^{9a})ₜ-R¹² ; ou dans lequel Y et Y' sont chacun indépendamment -C(=0)-(CR⁹R^{9a})ₜ-N(R¹⁰)-(CR⁹R^{9a})ₜ-C(=O)-(CR⁹R^{9a})ₜ-O-(CR⁹R^{9a})ₜ-R¹² ; ou
dans lequel Y et Y' sont chacun indépendamment -U-(CR⁹R^{9a})ₜ-O-(CR⁹R^{9a})ₜ-R¹² ; ou
dans lequel Y et Y' sont chacun indépendamment -C(=O)-(CR⁹R^{9a})ₜ-O-(CR⁹R^{9a})ₜ-R¹² ; ou
dans lequel Y et Y' sont chacun indépendamment -C(=O)-(CR⁹R^{9a})ₙ-N(R¹¹)-R¹²,
dans lequel R¹¹ et R¹², ensemble avec l'atome auquel ils sont attachés, forment un cycle de 4 à 7 éléments.

11. Composé selon la revendication 10, dans lequel R⁹, R^{9a}, R¹⁰ et R¹¹ sont chacun indépendamment H, un groupe deutérium, alkyle en C₁ à C₆, hétéroalkyle en C₂ à C₆, cycloalkyle en C₃ à C₁₀, hétérocyclyle en C₂ à C₁₀, aryle en C₆ à C₁₀, hétéroaryle en C₁ à C₉, halogénoalkyle en C₁ à C₆, hydroxyalkyle en C₁ à C₆, (aryle en C₆ à C₁₀) alkyle en C₁ à C₆, (hétéroaryle en C₁ à C₉) alkyle en C₁ à C₆, (hétérocyclyle en C₂ à C₁₀)alkyle en C₁ à C₆ ou (cycloalkyle en C₃ à C₈)alkyle, en C₁ à C₆ ;
chaque R¹² est indépendamment R^{13a}R¹³N-, -C(=O)R¹³,-C(=S)R¹³, -C(=O)OR¹³, -C(=O)NR¹³R^{13a}, -OC(=O)NR¹³R^{13a},-OC(=O)OR¹³, -N(R¹³)C(=O)NR¹³R^{13a}, -N(R¹³)C(=O)OR^{13a},-N(R¹³)C(=O)-R^{13a}, R¹³R^{13a}N-S(=O)₂-, R¹³S(=O)₂-, R¹³S(=O)₂N(R^{13a})-, R¹³OS(=O)₂-, alkyle en C₁ à C₆, hétéroalkyle en C₂ à C₆, cycloalkyle en C₃ à C₁₀, hétérocyclyle en C₂ à C₁₀, aryle en C₆ à C₁₀, hétéroaryle en C₁ à C₉ ou (aryle en C₆ à C₁₀)alkyle en C₁ à C₆ ; ou R¹¹ et R¹², ensemble avec l'atome auquel ils sont attachés, forment un cycle de 4 à 7 chaînons ;
R¹³ et R^{13a} sont chacun indépendamment H, un groupe deutérium, alkyle en C₁ à C₆, hétéroalkyle en C₂ à C₆, cycloalkyle en C₃ à C₁₀, hétérocyclyle en C₂ à C₁₀, aryle en C₆ à C₁₀, hétéroaryle en C₁ à C₉ ou (aryle en C₆ à C₁₀)alkyle en C₁ à C₆ ; à condition que lorsque R¹³ et R^{13a} sont liés au même atome d'azote, R¹³ et R^{13a}, ensemble avec l'atome d'azote auquel ils sont attachés, forment facultativement un cycle de 3 à 8 chaînons substitué ou non substitué ou un cycle bicyclique spiro ou condensé substitué ou non substitué ;
chaque t est indépendamment 0, 1, 2, 3 ou 4 ; et
chaque k est indépendamment 0, 1 ou 2 ; ou
R⁹, R^{9a}, R¹⁰ et R¹¹ sont chacun indépendamment H, un groupe deutérium, méthyle, éthyle, isopropyle, cyclohexyle, isobutyle ou phényle ;
chaque R¹² est indépendamment un groupe -C(=O)R¹³,-C(=O)OR¹³, -C(=O)NR¹³R^{13a}, méthyle, éthyle, propyle, phényle, cyclohexyle, morpholinyle ou pipéridinyle ;
ou R¹¹ et R¹², ensemble avec l'atome auquel ils sont attachés, forment un cycle de 4 à 7 chaînons ; et
R¹³ et R^{13a} sont chacun indépendamment H, un groupe deutérium, méthyle, éthyle, propyle, phényle, cyclohexyle, morpholinyle ou pipéridinyle.

12. Composé selon la revendication 5, répondant à la formule (VI) : 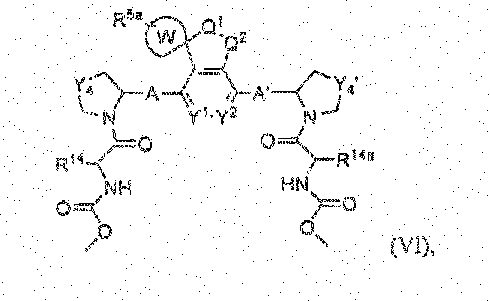
dans laquelle R¹⁴ et R^{14a} sont chacun indépendamment H, un groupe deutérium, alkyle en C₁ à C₆, halogénoalkyle en C₁ à C₆, hydroxyalkyle en C₁ à C₆, hétéroalkyle en C₂ à C₆, aryle en C₆ à C₁₀, hétéroaryle en C₁ à Cg, hétérocyclyle en C₂ à C₁₀, cycloalkyle en C₃ à C₈, (aryle en C₆ à C₁₀)alkyle en C₁ à C₆, (hétéroaryle en C₁ à C₉)alkyle en C₁ à C₆, (hétérocyclyle en C₂ à C₁₀)alkyle en C₁ à C₆ ou (cycloalkyle en C₃ à C₈)alkyle en C₁ à C₆ ; et
les groupes alkyle en C₁ à C₆, halogénoalkyle en C₁ à C₆, hydroxyalkyle en C₁ à C₆, hétéroalkyle en C₂ à C₆, aryle en C₆ à C₁₀, hétéroaryle en C₁ à C₉, hétérocyclyle en C₂ à C₁₀, cycloalkyle en C₃ à C₈, (aryle en C₆ à C₁₀) alkyle en C₁ à C₆, (hétéroaryle en C₁ à C₉)alkyle en C₁ à C₆, (hétérocyclyle en C₂ à C₁₀)alkyle en C₁ à C₆ et (cycloalkyle en C₃ à C₈)alkyle en C₁ à C₆ sont chacun facultativement substitués par un ou plusieurs substituants, le substituant étant un groupe deutérium, F, Cl, Br, hydroxy ou cyano.

13. Composé selon la revendication 12, répondant à la formule (VII) : 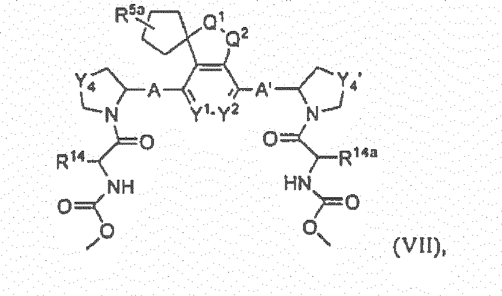
dans laquelle R¹⁴ et R^{14a} sont chacun indépendamment H, un groupe deutérium, hydroxyalkyle en C₁ à C₃, méthyle, éthyle, isopropyle, isobutyle, tert-butyle, allyle, propargyle, trifluoroéthyle, phényle, pyranyle, morpholinyle, -NR¹³R^{13a}, benzyle, pipérazinyle, cyclopentyle, cyclopropyle, cyclohexyle ou hétéroaryle en C₁ à C₉; et
dans laquelle les groupes méthyle, éthyle, isopropyle, isobutyle, *tert*-butyle, allyle, propargyle, trifluoroéthyle, phényle, pyranyle, morpholinyle, benzyle, pipérazinyle, cyclopentyle, cyclopropyle et cyclohexyle sont chacun facultativement substitués par un ou plusieurs substituants, le substituant étant un groupe deutérium, F, Cl, Br, hydroxy ou cyano.

14. Composé selon la revendication 1, répondant à la formule (VIII) ou à la formule (IX) : 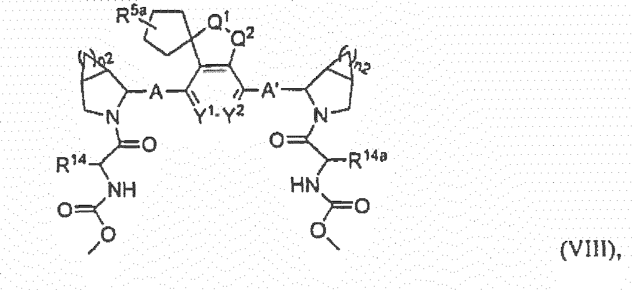 ou 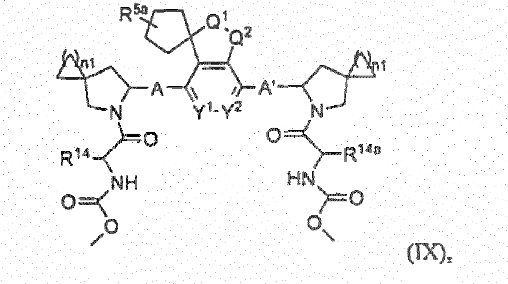
dans laquelle R¹⁴ et R^{14a} sont chacun indépendamment H, un groupe deutérium, alkyle en C₁ à C₆, halogénoalkyle en C₁ à C₆, hydroxyalkyle en C₁ à C₆, hétéroalkyle en C₂ à C₆, aryle en C₆ à C₁₀, hétéroaryle en C₁ à C₉, hétérocyclyle en C₂ à C₁₀, cycloalkyle en C₃ à C₈, (aryle en C₆ à C₁₀) alkyle en C₁ à C₆, (hétéroaryle en C₁ à C₉) alkyle en C₁ à C₆, (hétérocyclyle en C₂ à C₁₀) alkyle en C₁ à C₆ ou (cycloalkyle en C₃ à C₈) alkyle en C₁ à C₆ ; et
dans laquelle les groupes alkyle en C₁ à C₆, halogénoalkyle, en C₁ à C₆, hydroxyalkyle en C₁ à C₆, hétéroalkyle en C₂ à C₆, aryle en C₆ à C₁₀, hétéroaryle en C₂ à C₉, hétérocyclyle en C₂ à C₁₀, cycloalkyle en C₃ à Ce, (aryle en C₆ à C₁₀) alkyle en C₁ à C₆, (hétéroaryle en C₁ à C₉) alkyle en C₁ à C₆, (hétérocyclyle en C₂ à C₁₀) alkyle en C₁ à C₆ et (cycloalkyle en C₃ à C₈)alkyle en C₁ à C₆ sont chacun facultativement substitués par un ou plusieurs substituants, le substituant étant un groupe deutérium, F, Cl, Br, hydroxy ou cyano ; et
n₁ et n₂ sont chacun indépendamment 1, 2, 3 ou 4.

15. Composé selon la revendication 1, répondant à la formule (X) : 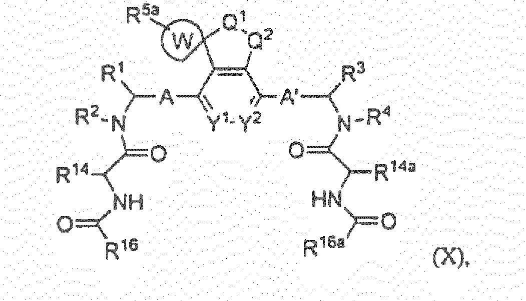
dans laquelle R⁵ est indépendamment H, un groupe deutérium, méthyle, éthyle, F, Cl, Br ou I ;
Q¹ est CH₂, C(=O), O, S ou NR⁶ ;
Q² est CH₂, C(=O), CF₂, 0, NR⁶ ou S ;
Y¹ et Y² sont chacun indépendamment N ou CR⁷ ;
R⁶ et R⁷ sont chacun indépendamment H, un groupe deutérium, méthyle, éthyle, isopropyle, phényle ou cyclohexyle ;
R¹⁴ et R^{14a} sont chacun indépendamment H, un groupe deutérium, méthyle, éthyle, phényle, cyclohexyle, 1-méthylpropyle, isopropyle ou tert-butyle ;
R¹⁶ et R^{16z} sont chacun indépendamment un groupe hydroxy, méthoxy, éthoxy, phénoxy, 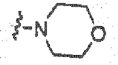 ou *tert-*butoxy ;
dans laquelle les groupes méthyle, éthyle, phényle, cyclohexyle, 1-méthylpropyle, isopropyle, méthoxy, éthoxy, *tert*-butoxy et *tert*-butyle sont chacun facultativement substitués par un ou plusieurs substituants, le substituant étant un groupe deutérium, F, Cl, Br, hydroxy ou cyano ;
dans laquelle 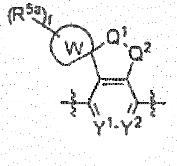 est 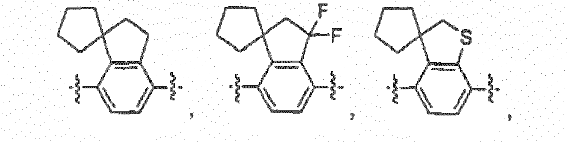 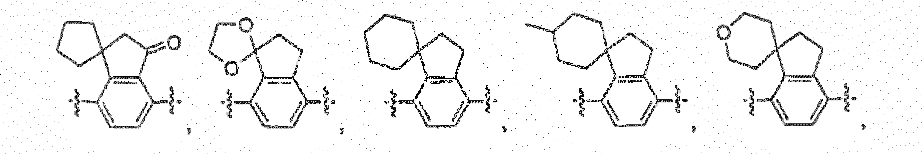 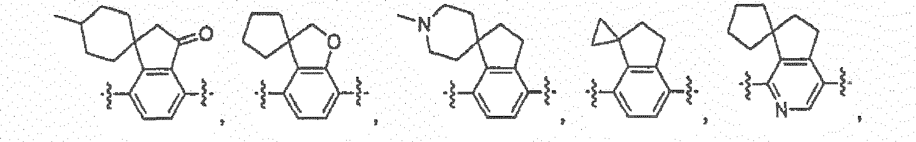 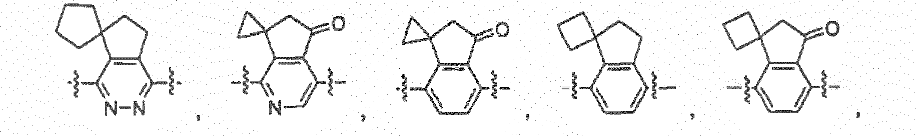 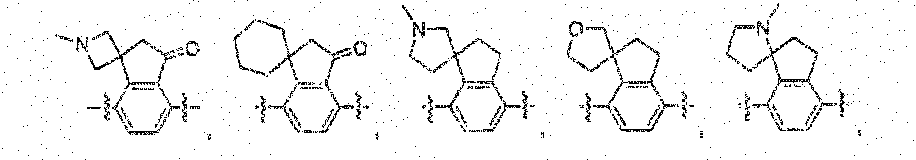 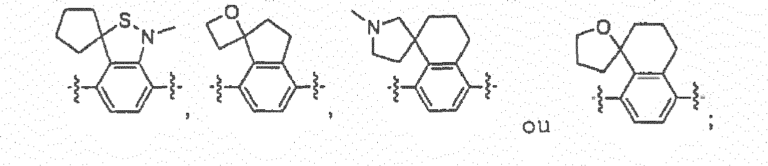
A et A' sont chacun indépendamment 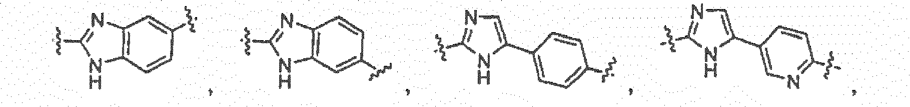 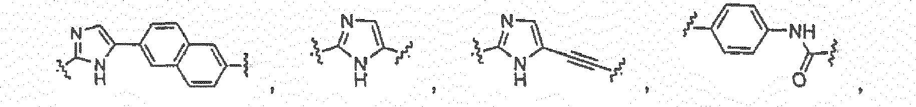 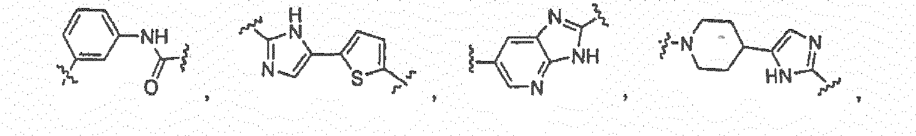 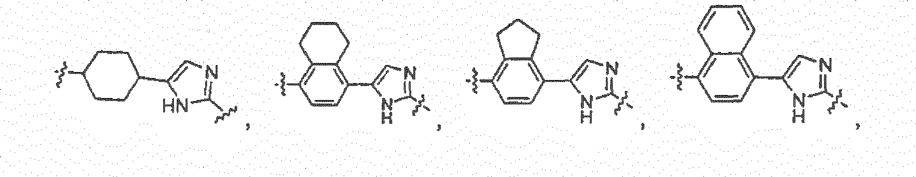 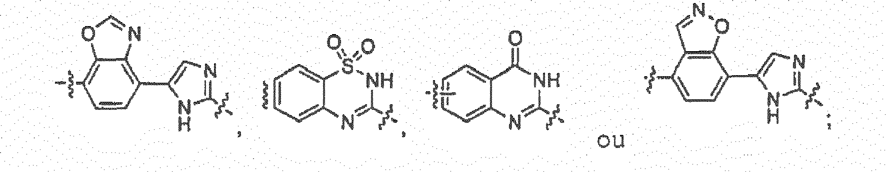
dans laquelle R¹, R² et N-CH forment ensemble l'un des groupes divalents suivants ; 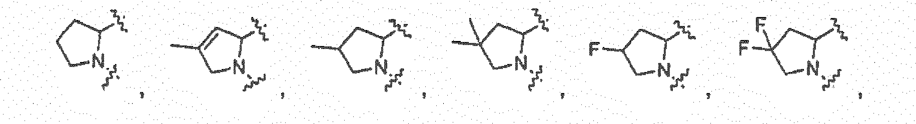 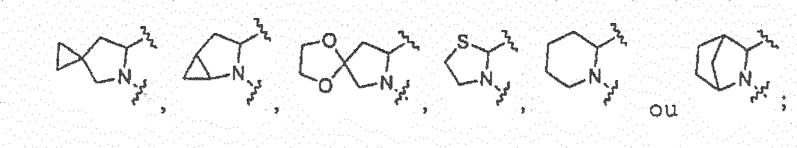 et
dans laquelle R³, R⁴ et N-CH forment ensemble l'un des groupes divalents suivants ;

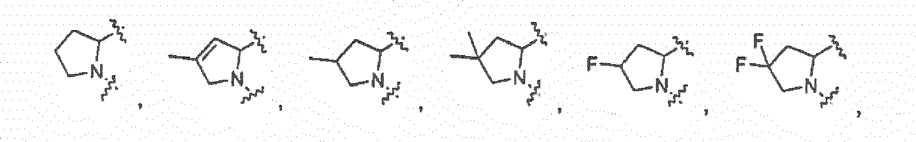

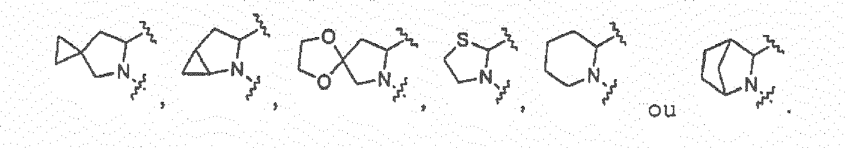

16. Composé selon la revendication 15, répondant à la formule (XI) : 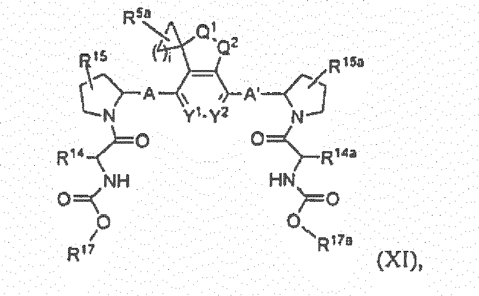
dans laquelle i est 1, 2, 3 ou 4 ;
R^{5a} est H, un groupe deutérium ou méthyle ;
Q¹ et Q² sont chacun indépendamment (CH₂)ₑ, CF₂, S, NR⁶, O ou C(=O) ;
e est 0, 1, 2, 3 ou 4 ;
R⁶ est H, un groupe deutérium, méthyle, éthyle, isobutyle, cyclohexyle, phényle ou isopropyle ;
R¹⁴ et R^{14a} sont chacun indépendamment H, un groupe deutérium, méthyle, éthyle, isobutyle, cyclohexyle, phényle ou isopropyle ;
R¹⁵ et R^{15a} sont chacun indépendamment H, un groupe deutérium, F, Cl, Br, méthyle, éthyle, isopropyle ou *tert*-butyle ; et
R¹⁷ et R^{17a} sont chacun indépendamment un groupe méthyle, phényle ou éthyle ;
dans laquelle les groupes méthyle, éthyle, phényle, cyclohexyle, isopropyle, isobutyle et *tert*-butyle sont chacun facultativement substitués par un ou plusieurs substituants, le substituant étant un groupe deutérium, F, Cl, Br, hydroxy ou cyano.

17. Composé selon la revendication 1, répondant à la formule (XI') : 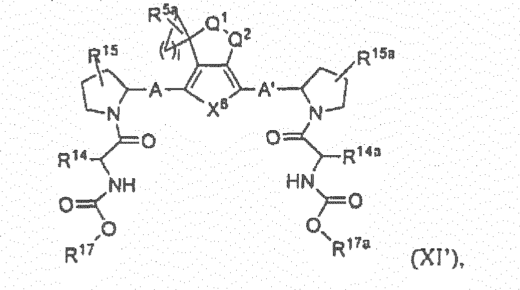
dans laquelle i est 1, 2, 3 ou 4 ;
R^{5a} est H, un groupe deutérium ou méthyle ;
Q¹ et Q² sont chacun indépendamment (CH₂)ₑ, CF₂, S, NR⁶, O ou C(=O) ;
e est 0, 1, 2, 3 ou 4 ;
R⁶ est indépendamment H, un groupe deutérium, méthyle, éthyle, isobutyle, cyclohexyle, phényle ou isopropyle ;
R¹⁴ et R^{14a} sont chacun indépendamment H, un groupe deutérium, méthyle, éthyle, isobutyle, cyclohexyle, phényle ou isopropyle ;
R¹⁵ et R^{15a} sont chacun indépendamment H, un groupe deutérium, F, Cl, Br, méthyle, éthyle, isopropyle ou *tert*-butyle ;
R¹⁷ et R^{17a} sont chacun indépendamment un groupe méthyle, phényle ou éthyle ;
dans laquelle les groupes méthyle, éthyle, phényle, cyclohexyle, isopropyle, isobutyle et *tert*-butyle sont chacun facultativement substitués par un ou plusieurs substituants, le substituant étant un groupe deutérium, F, Cl, Br, hydroxy ou cyano ;
X⁶ est indépendamment CH₂, 0, S ou NR⁶ ; et
A et A' sont chacun indépendamment

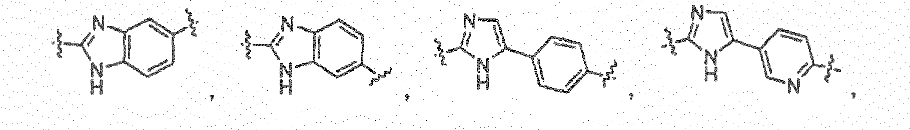

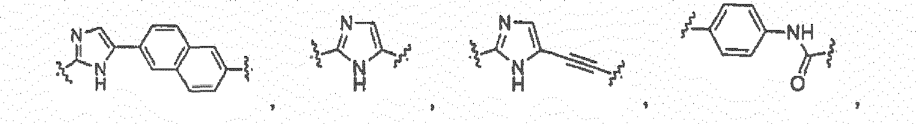

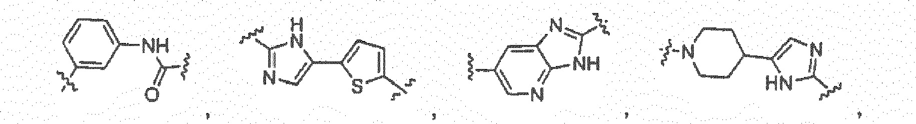

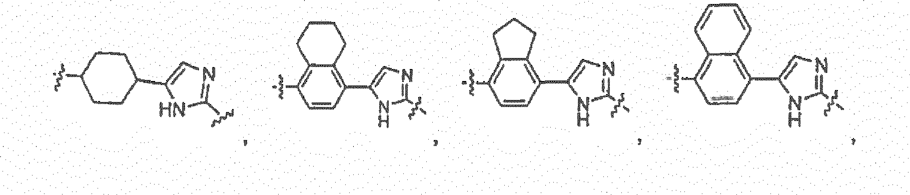

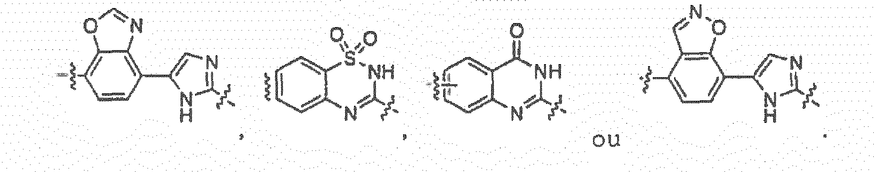

18. Composé selon la revendication 1, répondant à l'une des structures suivantes : 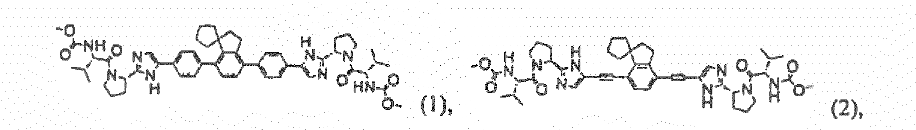 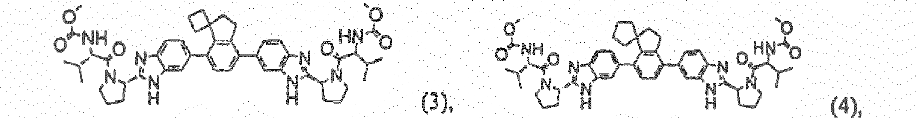 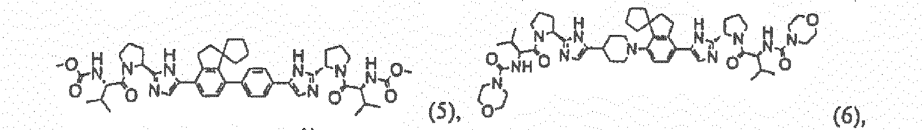 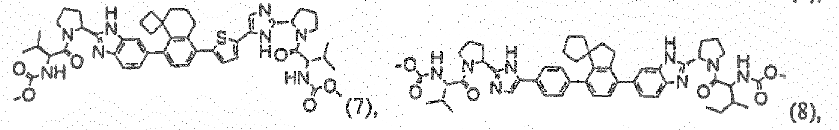 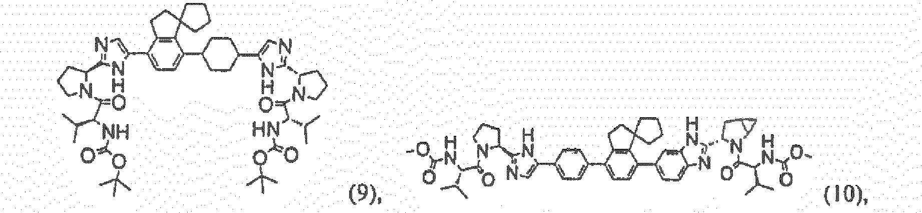 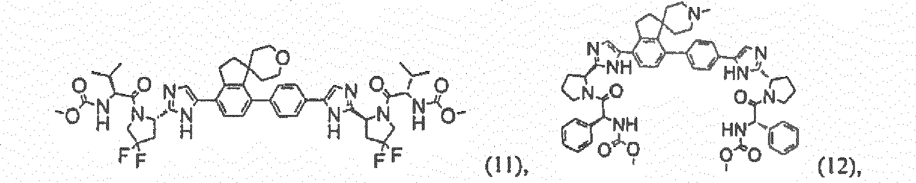 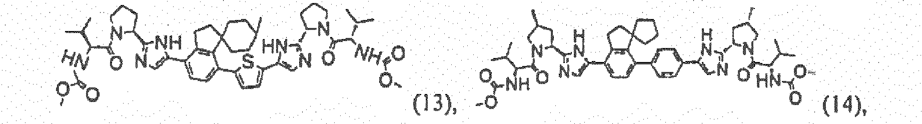 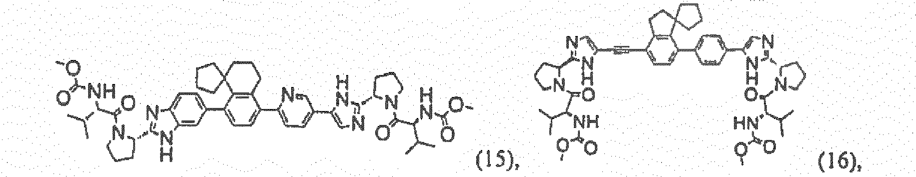 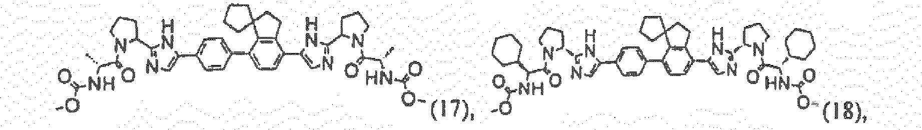 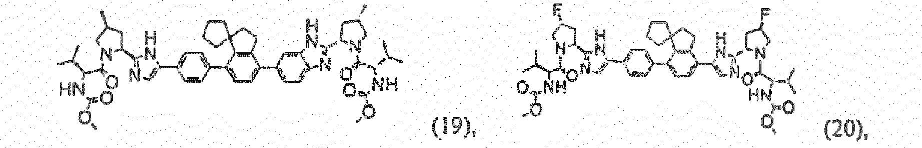 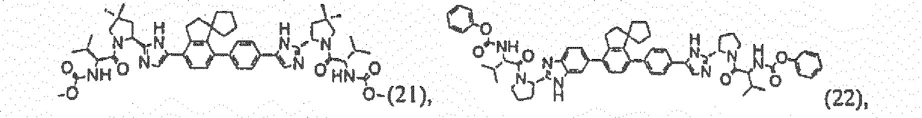 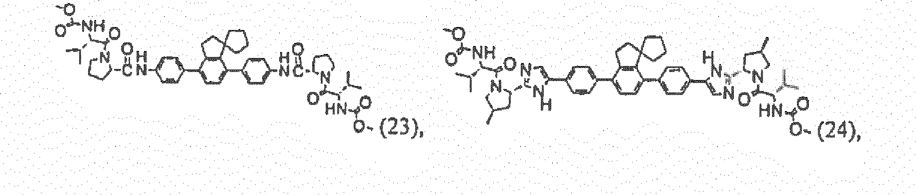 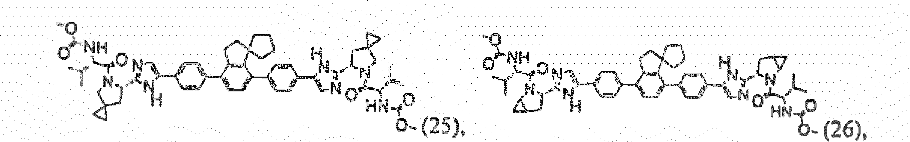 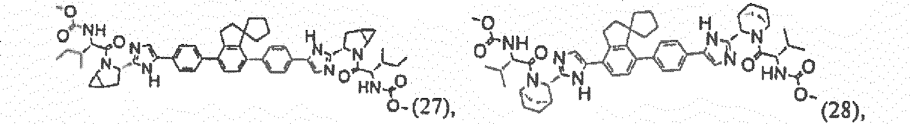 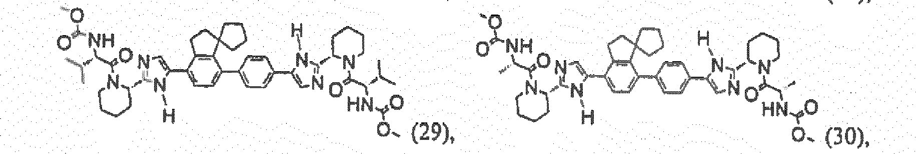 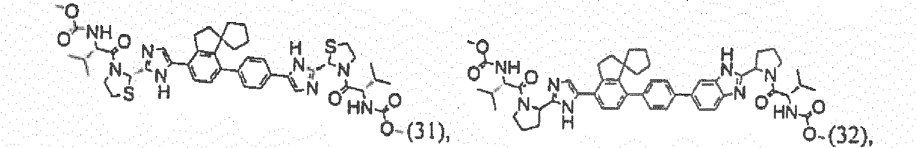 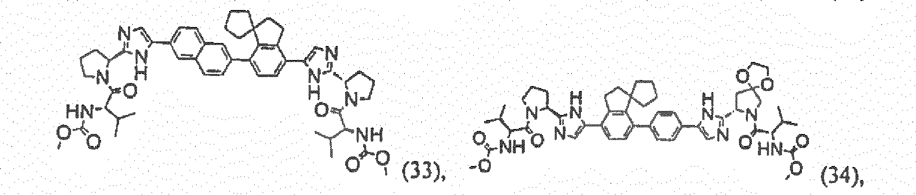 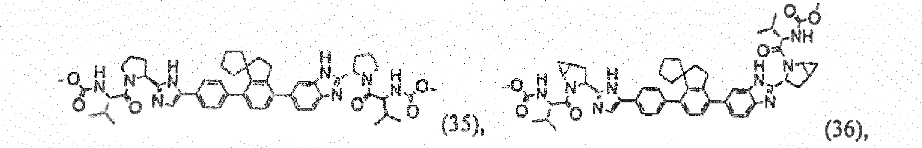 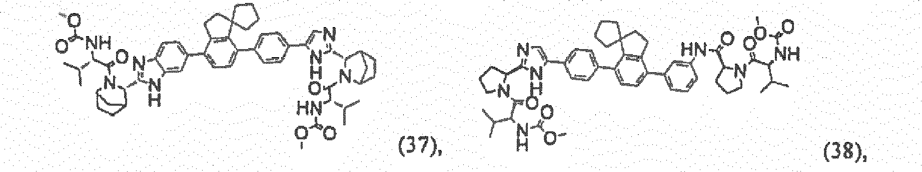 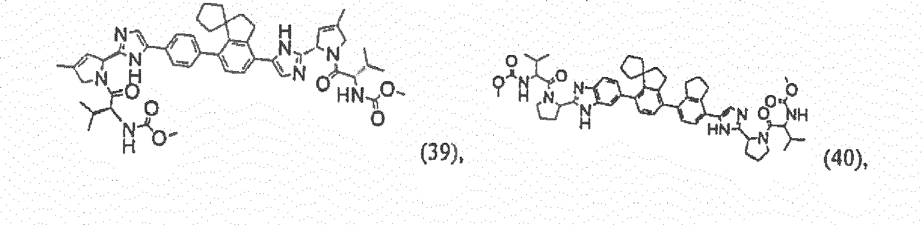 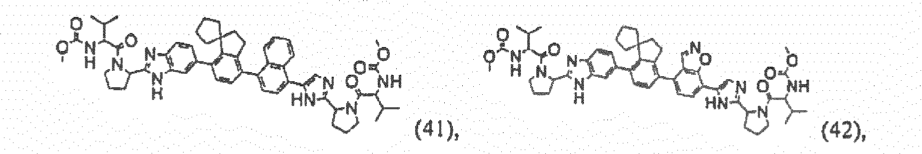 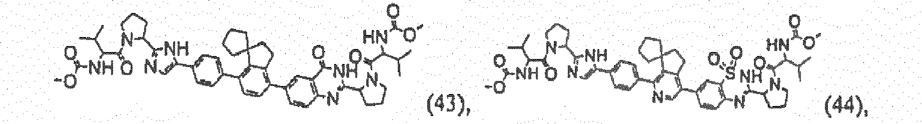 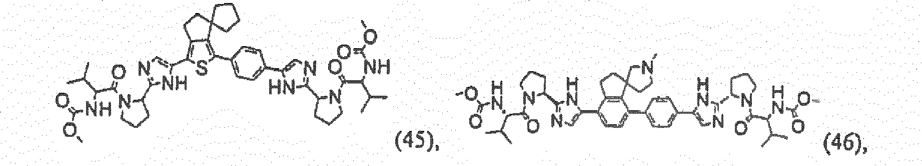 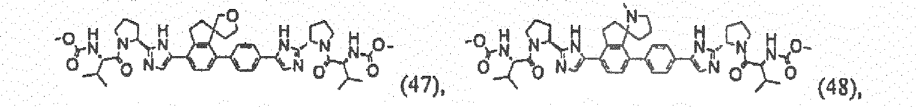 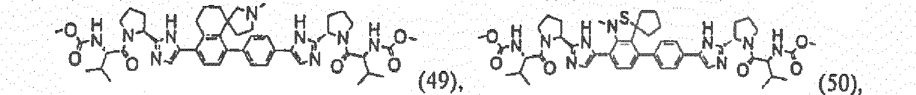 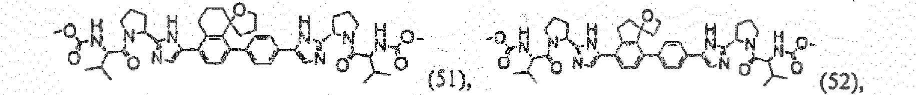 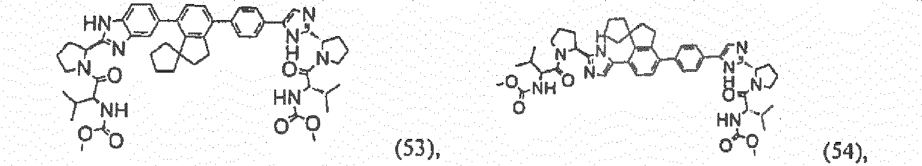 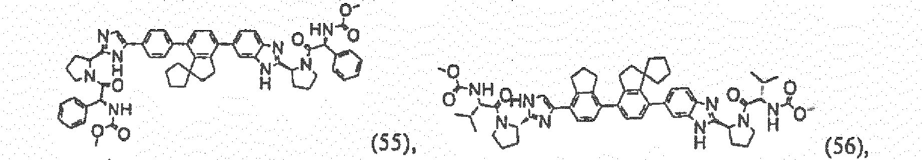 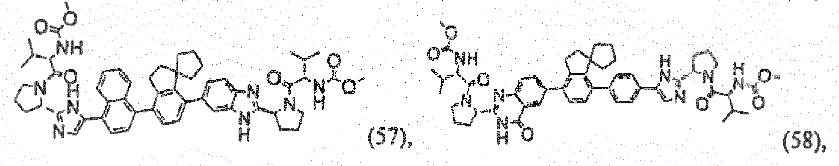 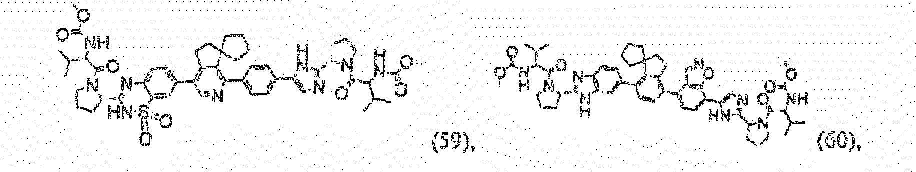 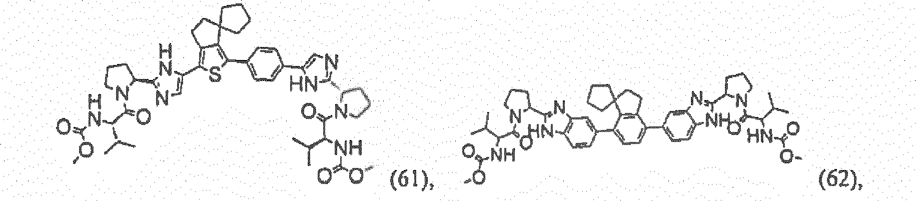 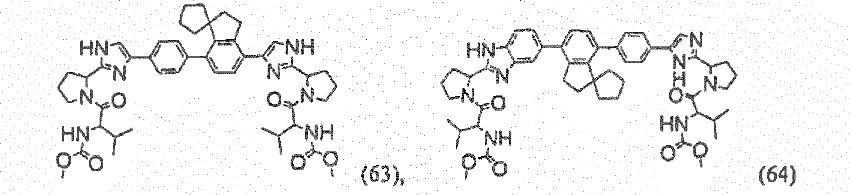 et 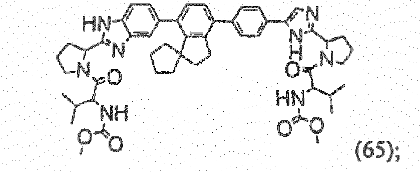 ou un stéréoisomère, un isomère géométrique, un tautomère, un N-oxyde, un hydrate, un solvate, ou un sel pharmaceutiquement acceptable de celles-ci.

19. Composition pharmaceutique comprenant le composé selon l'une quelconque des revendications 1 à 18 ; et un support, un excipient, un diluant, un adjuvant, véhicule ou une combinaison de ceux-ci pharmaceutiquement acceptable ; ou
la composition pharmaceutique comprenant en outre un agent anti-VHC ;
l'agent anti-VHC étant un interféron, la ribavirine, l'IL-2, l'IL-6, l'IL-12, un composé qui améliore le développement d'une réponse des lymphocytes T auxiliaires de type 1, un ARN interférant, un ARN anti-sens, l'imiquimod, un inhibiteur de l'inosine 5'-monophosphate déshydrogénase, l'amantadine, la rimantadine, la ribavirine, le bavituximab, l'immunoglobuline de l'hépatite C humaine (CIVACIR™), le bocéprévir, le télaprévir, l'erlotinib, le daclatasvir, le siméprévir, l'asunaprévir, le vaniprévir, le faldaprévir, l'ABT-450, le danoprévir, le sovaprévir, le MK-5172, le védroprévir, le BZF-961, le GS-9256, le narlaprévir, l'ANA975, l'ABT-267, l'EDP239, le PPI-668, le GS-5816, le samatasvir (IDX-719), le MK-8742, le MK-8325, le GSK-2336805, le PPI-461, le TMC-435, le MK-7009, le BI-2013335, le ciluprévir, le BMS-650032, l'ACH-1625, l'ACH-1095, le VX-985, l'IDX-375, le VX-500, le VX-813, le PHX-1766, le PHX-2054, l'IDX-136, l'IDX-316, l'EP-013420, le VBY-376, le TMC-649128, le R-7128, le PSI-7977, l'INX-189, l'IDX-184, l'IDX-102, le R1479, l'UNX-08189, le PSI-6130, le PSI-938, le PSI-879, l'HCV-796, l'HCV-371, le VCH-916, le VCH-222, l'ANA-598, le MK-3281, l'ABT-333, l'ABT-072, le PF-00868554, le BI-207127, le GS-9190, l'A-837093, le JKT-109, le GI-59728, le GL-60667, l'AZD-2795, le TMC647055 ou une combinaison de ceux-ci ;
l'interféron étant l'interféron α-2b, l'interféron α pégylé, l'interféron α-2a, l'interféron α-2a pégylé, l'interféron α consensus, l'interféron γ ou une combinaison de ceux-ci ; ou
la composition pharmaceutique comprenant en outre au moins un inhibiteur du VHC ; et
l'inhibiteur du VHC inhibant au moins un parmi le processus de réplication du VHC et la fonction de la protéine virale du VHC, le processus de réplication du VHC étant un cycle viral entier constitué par l'entrée, la décapsidation, la traduction, la réplication, l'assemblage et la sortie du VHC ; et la protéine virale du VHC étant l'hélicase, la protéinase, la métalloprotéinase, la sérine protéinase, la protéine non structurale NS4A, la protéine non structurale NS4B, l'ARN polymérase de la protéine non structurale NS5A ou de la protéine non structurale NS5B, ou un site d'entrée interne des ribosomes (IRES) et l'inosine-5'-monophosphate déshydrogénase (IMPDH) nécessaires à la réplication virale du VHC.

20. Composé selon l'une quelconque des revendications 1 à 18 ou composition pharmaceutique selon la revendication 19 pour une utilisation dans l'inhibition d'au moins un parmi le processus de réplication du VHC et la fonction de la protéine virale du VHC, le processus de réplication du VHC étant un cycle viral entier constitué par l'entrée, la décapsidation, la traduction, la réplication, l'assemblage et la sortie du VHC ; et la protéine virale du VHC étant l'hélicase, la protéinase, la métalloprotéinase, la sérine protéinase, la protéine non structurale NS4A, la protéine non structurale NS4B, l'ARN polymérase de la protéine non structurale NS5A ou de la protéine non structurale NS5B, ou un site d'entrée interne des ribosomes (IRES) et l'inosine-5'-monophosphate déshydrogénase (IMPDH) nécessaires à la réplication virale du VHC.

21. Composé selon l'une quelconque des revendications 1 à 18 ou composition pharmaceutique selon la revendication 19 pour une utilisation dans la prévention, la prise en charge, le traitement ou la diminution de la sévérité d'une infection par le VHC ou d'un trouble du VHC chez un patient.
